(19) Europäisches Patentamt
European Patent Office
Office européen des brevets

(11) EP 1 911 753 A1

(12) EUROPEAN PATENT APPLICATION
published in accordance with Art. 153(4) EPC

(43) Date of publication:
16.04.2008 Bulletin 2008/16

(21) Application number: 06782307.0

(22) Date of filing: 28.07.2006

(51) Int Cl.:
*C07D 401/04* (2006.01) *A61K 31/454* (2006.01)
*A61K 31/4545* (2006.01) *A61K 31/4709* (2006.01)
*A61K 31/499* (2006.01) *A61K 31/5386* (2006.01)
*A61P 3/00* (2006.01) *A61P 3/04* (2006.01)
*A61P 3/06* (2006.01) *A61P 3/10* (2006.01)
*A61P 9/04* (2006.01) *A61P 9/12* (2006.01)
*A61P 21/00* (2006.01) *A61P 43/00* (2006.01)
*C07D 471/10* (2006.01) *C07D 487/10* (2006.01)
*C07D 491/107* (2006.01) *C07D 498/10* (2006.01)
*C07D 513/10* (2006.01) *C07D 519/00* (2006.01)
*C07F 7/18* (2006.01)

(86) International application number:
PCT/JP2006/315447

(87) International publication number:
WO 2007/013691 (01.02.2007 Gazette 2007/05)

(84) Designated Contracting States:
AT BE BG CH CY CZ DE DK EE ES FI FR GB GR HU IE IS IT LI LT LU LV MC NL PL PT RO SE SI SK TR

(30) Priority: 29.07.2005 JP 2005221959
07.06.2006 JP 2006159117

(71) Applicant: Takeda Pharmaceutical Company Limited
Osaka-shi,
Osaka 541-0045 (JP)

(72) Inventors:
- KAMATA, Makoto
TAKEDA PHARMACEUTICAL COMPANY LTD.
Osaka-shi
5328686 (JP)
- FUKATSU, Kohji
TAKEDA PHARMACEUTICAL COMPANY Ltd.
Osaka-shi
5328686 (JP)
- YAMASHITA, Tohru
TAKEDA PHARMACEUTICAL COMPANYLtd
Osaka-shi
5328686 (JP)
- FURUYAMA, Naoki
TAKEDA PHARMACEUTICAL COMPANY Ltd.
Osaka-shi
5328686 (JP)
- ENDO, Satoshi
TAKEDA PHARMACEUTICAL COMPANY Ltd.
Osaka-shi
5328686 (JP)

(74) Representative: Casalonga, Axel
Bureau Casalonga & Josse
Bayerstrasse 71/73
80335 München (DE)

(54) **SPIRO-CYCLIC COMPOUND**

(57) The present invention provides a compound represented by the formula (I):

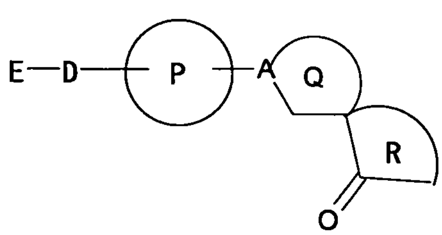

(I)

EP 1 911 753 A1

wherein
E is an optionally substituted cyclic group;
D is a carbonyl group or a sulfonyl group;
A is CH or N;ring P is an optionally further substituted 5- to 7-membered ring;
ring Q is an optionally further substituted 5- to 7-membered nonaromatic ring; and
ring R is an optionally further substituted and optionally condensed 5- to 7-membered nonaromatic ring,
or a salt thereof. The compound of the present invention has an ACC inhibitory activity, is useful for the prophylaxis or treatment of obesity, diabetes, hypertension, hyperlipidemia, cardiac failure, diabetic complications, metabolic syndrome, sarcopenia and the like, and has superior properties in the efficacy, duration of activity, specificity, low toxicity and the like.

## Description

### Technical Field

The present invention relates to a spiro ring compound having an acetyl-CoA carboxylase (sometimes to be abbreviated as ACC in the present specification) inhibitory action, which is useful for the prophylaxis or treatment of obesity, diabetes, hypertension, hyperlipidemia, cardiac failure, diabetic complications, metabolic syndrome, sarcopenia and the like.

### Background Art

ACC is an enzyme that converts acetyl-CoA to malonyl-CoA, and catalyzes a rate determining reaction in fatty acid metabolism. Malonyl-CoA, which is produced by an ACC catalyst reaction, inhibits fatty acid oxidation in mitochondria based on the feedback inhibition of carnitine palmitoyl transferase-1 (CPT-1). Accordingly, ACC plays a key role in controlling the balance between use of carbohydrate and fatty acid in the liver and skeletal muscle, and further, controlling insulin sensitivity in the liver, skeletal muscle and adipose tissue.

A reduced level of malonyl-CoA by ACC inhibition can promote an increase in fatty acid oxidation, deficient secretion of triglycelite (TG)-rich lipoprotein (VLDL) in the liver, regulation of insulin secretion in the pancreas, and further, improvement in the insulin sensitivity in the liver, skeletal muscle and adipose tissue.

In addition, a long-term administration of a compound having an ACC inhibitory action markedly decreases the TG content of the liver and adipose tissue and selectively decreases the body fat in test subjects with obesity, who are on low-fat diets, based on the promotion of fatty acid oxidation and suppression of de novo synthesis of fatty acid.

Accordingly, a compound having an ACC inhibitory action is extremely useful for the prophylaxis or treatment of metabolic syndrome, obesity, hypertension, diabetes, cardiovascular diseases associated with atherosclerosis and the like.

As a spiro ring compound, the following compound has been reported.

(1) A compound represented by the formula:

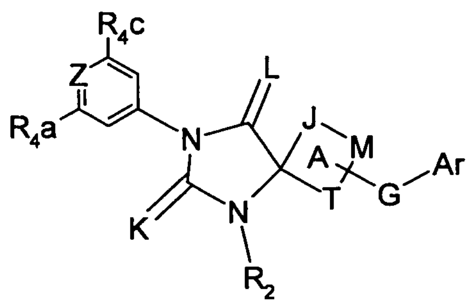

wherein L and K are each independently O or S; Z is N or $CR_{4b}$; G is a linker or bond binding to T or M; Ar is aryl or heteroaryl each of which is optionally substituted; J, M and T are each selected so that a 3- to 6-membered saturated or partially unsaturated cycloalkyl or heterocycle can be formed; and $R_2$, $R_{4a}$, $R_{4b}$ and $R_{4c}$ are each a hydrogen atom and the like, which is an LFA-1/ICAM inhibitor and useful as a therapeutic agent for inflammation (including diabetes, atherosclerosis), immune disease and the like (see WO 03/029245).

As a compound having an ACC inhibitory action, moreover, the following compound has been reported.

(2) A compound represented by the formula:

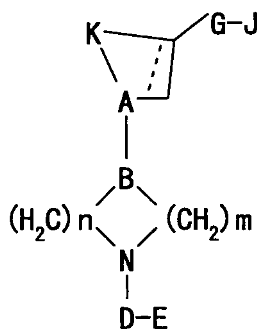

wherein A-B is N-CH, CH-N; K is $(CH_2)_r$ r (r is an integer of 2-4); m and n are each an integer of 1-3; D is CO or $SO_2$; E is an optionally substituted bi- to tetra-cyclic ring and the like; G is CO, $SO_2$ or $CR^7R^8$ ($R^7$ and $R^8$ are each a hydrogen atom and the like); and J is $OR^1$, $NR^2R^3$, $CR^4R^5R^6$ ($R^1$, $R^2$, $R^3$, $R^4$, $R^5$ and $R^6$ are each a hydrogen atom and the like), which has an ACC inhibitory action and is useful as a therapeutic agent for metabolic syndrome, arteriosclerosis, diabetes, obesity and the like (see WO 03/072197).

However, the compound of the present invention is not reported.

## Disclosure of the Invention

There is a demand for the development of a compound having an ACC inhibitory action, which is useful for the prophylaxis or treatment of obesity, diabetes, hypertension, hyperlipidemia, cardiac failure, diabetic complications, metabolic syndrome, sarcopenia and the like, and has superior properties such as efficacy, duration of activity, specificity, low toxicity and the like.

The present inventors have first found that a compound represented by the formula (I):

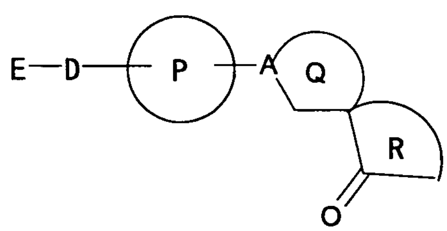

(I)

wherein

E is an optionally substituted cyclic group;
D is a carbonyl group or a sulfonyl group;
A is CH or N;
ring P is an optionally further substituted 5- to 7-membered ring;
ring Q is an optionally further substituted 5- to 7-membered nonaromatic ring; and
ring R is an optionally further substituted and optionally condensed 5- to 7-membered nonaromatic ring,
or a salt thereof (hereinafter sometimes to be referred to as compound (I)), which is characterized by a chemical structure where a spiro ring formed by two nonaromatic rings is bonded
to a 5- to 7-membered ring, has a superior ACC inhibitory action and is useful for the prophylaxis or treatment of obesity, diabetes, hypertension, hyperlipidemia, cardiac failure, diabetic complications, metabolic syndrome, sarcopenia and the like. Based on this finding, the present inventors have conducted intensive studies and completed the present invention. Accordingly, the present invention relates to

(1) compound (I);
(2) compound (I) wherein ring P is a 5- to 7-membered nitrogen-containing non-aromatic heterocycle optionally further substituted;
(3) compound (I) of the aforementioned (2), wherein the 5- to 7-membered nitrogen-containing non-aromatic heterocycle is a piperidine;

(4) compound (I) wherein E is an optionally substituted aromatic hydrocarbon group;
(5) compound (I) of the aforementioned (4), wherein the aromatic hydrocarbon group is an anthryl;
(6) compound (I) wherein E is an optionally substituted aromatic heterocyclic group;
(7) compound of the aforementioned (6), wherein the aromatic heterocyclic group is a thienyl, a benzothiophenyl, a pyridyl or a quinolyl;
(8) compound (I) wherein D is a carbonyl group;
(9) compound (I) wherein A is N;
(10) compound (I) wherein ring Q is an optionally further substituted 6-membered monocyclic non-aromatic heterocycle;
(11) compound (I) wherein ring R is an optionally further substituted 5-membered monocyclic non-aromatic heterocycle;
(12) compound (I) which is selected from N-(3-{[4-(3,3-dimethyl-1-oxo-2-oxa-7-azaspiro[4.5]dec-7-yl)piperidin-1-yl]carbonyl}-1-benzothien-2-yl)-N'-ethylurea, 3,3-dimethyl-7-{1-[(2-(pyridin-3-yl)quinolin-4-yl)carbonyl]piperidin-4-yl}-2-oxa-7-azaspiro[4.5]decan-1-one, 7-{1-[(3'-acetyl-5-(pyridin-3-yl)biphenyl-3-yl)carbonyl]piperidin-4-yl}-3,3-dimethyl-2-oxa-7-azaspiro[4.5]decan-1-one, 9-[1-(9-anthrylcarbonyl)piperidin-4-yl]-3,3-dimethyl-2-oxa-6,9-diazaspiro[4.5]decan-1-one, 1-(3-{[4-(3,3-dimethyl-1-oxo-2-oxa-7-azaspiro[4.5]dec-7-yl)piperidin-1-yl]carbonyl}-5-(pyridin-2-yl)-2-thienyl)-3-ethylurea, 4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)benzoic acid, N-ethyl-N'-(3-{[4-(3-ethyl-2,4-dioxo-1-oxa-3,7-diazaspiro[4.5]dec-7-yl)piperidin-1-yl]carbonyl}-1-benzothien-2-yl)urea, 1-(3-{[4-(3-ethyl-2,4-dioxo-1-oxa-3,7-diazaspiro[4.5]dec-7-yl)piperidin-1-yl]carbonyl}-1-benzothien-2-yl)urea, 1-ethyl-3-(3-{[4-(3-ethyl-2-methyl-4-oxo-1,3,7-triazaspiro[4.5]dec-1-en-7-yl)piperidin-1-yl]carbonyl}-1-benzothien-2-yl)urea, 1-(3-{[4-(2-ethyl-1,3-dioxo-2,7-diazaspiro[4.5]dec-7-yl)piperidin-1-yl]carbonyl}-1-benzothien-2-yl)urea, 1-ethyl-3-(3-{[4-(3-ethyl-2,4-dioxo-1-oxa-3,7-diazaspiro[4.5]dec-7-yl)piperidin-1-yl]carbonyl}-5-(pyridin-2-yl)-2-thienyl)urea, N-(4-{[4-(3,3-dimethyl-l-oxo-2-oxa-7-azaspiro[4.5]dec-7-yl)piperidin-1-yl]carbonyl}-6-pheriylpyridin-3-yl)-N'-ethylurea, N-(3-{[4-(1,3-dioxo-2,7-diazaspiro[4.5]dec-7-yl)piperidin-1-yl]carbonyl}-1-benzothien-2-yl)-N'-ethylurea, N-ethyl-N'-(3-{[4-(2-ethyl-1-oxo-2,7-diazaspiro[4.5]dec-7-yl)piperidin-1-yl]carbonyl}-1-benzothien-2-yl)urea N-(3-{[4-(3-isopropyl-2,4-dioxo-1-oxa-3,7-diazaspiro[4.5]dec 7-yl)piperidin-1-yl]carbonyl}-1-benzothien-2-yl) urea, N-(3-{[4-(2-ethyl-1-oxo-2,7-diazaspiro[4.5]dec-7-yl)piperidin-1-yl]carbonyl}-1-benzothien-2-yl)-N'-methylurea, N-(3-{[4-(2,4-dioxo-3-propyl-l-oxa-3,7-diazaspiro[4.5]dec-7-yl)piperidin-1-yl]carbonyl}-1-benzothien-2-yl)urea, N-(3-{[4-(3-ethyl-2,4-dioxo-1-oxa-3,7-diazaspiro[4.5]dec-7-yl)piperidin-1-yl]carbonyl}-1-benzothien-2-yl)-N'-methylurea, N-(3-{[4-(1-oxo-2-propyl-2,7-diazaspiro[4.5]dec-7-yl)piperidin-1-yl]carbonyl}-1-benzothien-2-yl)urea, N-(3-{[4-(2-isopropyl-1-oxo-2,7-diazaspiro[4.5]dec-7-yl)piperidin-1-yl]carbonyl}-1-benzothien-2-yl)-N'-methylurea, N-(3-{[4-(2-ethyl-1,3-dioxo-2,7-diazaspiro[4.5]dec-7-yl)piperidin-1-yl]carbonyl}-1-benzothien-2-yl)-N'-methylurea, N-(3-{[4-(2-isopropyl-1,3-dioxo-2,7-diazaspiro[4.5]dec-7-yl)piperidin-1-yl]carbonyl}-1-benzothien-2-yl)urea, N-ethyl-N'-(3-{[4-(2-methyl-1,3-dioxo-2,7-diazaspiro[4.5]dec-7-yl)piperidin-1-yl]carbonyl}-1-benzothien-2-yl)urea, N-(3-{[4-(1,3-dioxo-2,7-diazaspiro[4.5]dec-7-yl)piperidin-1-yl]carbonyl}-1-benzothien-2-yl)-N'-isopropylurea, and an optically active form thereof and a salt thereof;
(13) a prodrug of compound (I);
(14) a pharmaceutical agent comprising compound (I) or a prodrug thereof;
(15) an acetyl-CoA carboxylase inhibitor comprising compound (I) or a prodrug thereof;
(16) a pharmaceutical agent of the aforementioned (14), which is an agent for the prophylaxis or treatment of obesity, diabetes, hypertension, hyperlipidemia, cardiac failure, diabetic complications, metabolic syndrome or sarcopenia;
(17) use of compound (I) or a prodrug thereof for the production of an acetyl CoA carboxylase inhibitor;
(18) use of compound (I) or a prodrug thereof for the production of an agent for the prophylaxis or treatment of obesity, diabetes, hypertension, hyperlipidemia, cardiac failure, diabetic complications, metabolic syndrome or sarcopenia;
(19) a method for inhibiting acetyl CoA carboxylase in a mammal, which comprises administering compound (I) or a prodrug thereof to the mammal;
(20) a method for the prophylaxis or treatment of obesity, diabetes, hypertension, hyperlipidemia, cardiac failure, diabetic complications, metabolic syndrome or sarcopenia in a mammal, which comprises administering compound (I) or a prodrug thereof to the mammal; and the like.

The compound of the present invention has an ACC inhibitory action, and is useful for the prophylaxis or treatment of obesity, diabetes, hypertension, hyperlipidemia, cardiac failure, diabetic complications, metabolic syndrome, sarcopenia and the like.

## Best Mode for Embodying the Invention

The definition of each symbol in the formula (I) is described in detail in the following.

The "halogen atom" in the present specification is, unless otherwise specified, a fluorine atom, a chlorine atom, a bromine atom or an iodine atom.

The "$C_{1-3}$ alkylenedioxy group" in the present specification is, unless otherwise specified, methylenedioxy, ethylenedioxy and the like.

The "$C_{1-6}$ alkyl group" in the present specification is, unless otherwise specified, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl and the like.

The "$C_{1-6}$ alkoxy group" in the present specification is, unless otherwise specified, methoxy, ethoxy, propoxy, isopropoxy, butoxy, isobutoxy, sec-butoxy, tert-butoxy and the like.

The "$C_{1-6}$ alkoxy-carbonyl group" in the present specification is, unless otherwise specified, methoxycarbonyl, ethoxycarbonyl, propoxycarbonyl, tert-butoxycarbonyl and the like.

The "$C_{1-6}$ alkyl-carbonyl group" in the present specification is, unless otherwise specified, acetyl, propanoyl, butanoyl, isobutanoyl, pentanoyl, isopentanoyl, hexanoyl and the like.

E is an optionally substituted cyclic group. Examples of the "cyclic group" of the "optionally substituted cyclic group" for E include an aromatic hydrocarbon group, a nonaromatic cyclic hydrocarbon group, a heterocyclic group and the like.

Examples of the aromatic hydrocarbon group include a $C_{6-14}$ aromatic hydrocarbon group. Examples of the $C_{6-14}$ aromatic hydrocarbon group include phenyl, naphthyl, anthryl, fluorenyl, phenanthryl, acenaphthyl, biphenylyl and the like. Of these, phenyl, naphthyl (e.g., 1-naphthyl, 2-naphthyl), anthryl (e.g., 1-anthryl, 2-anthryl, 9-anthryl), fluorenyl (e.g., 9-fluorenyl) and the like are preferable.

Examples of the nonaromatic cyclic hydrocarbon group include a $C_{3-10}$ cycloalkyl group, a $C_{3-10}$ cycloalkenyl group, a $C_{4-10}$ cycloalkadienyl group and the like.

Examples of the $C_{3-10}$ cycloalkyl group include cyclopropyl, cyclobutyl, cyclopentyl, cyclohexyl, cycloheptyl, cyclooctyl and the like.

Examples of the $C_{3-10}$ cycloalkenyl group include 2-cyclopenten-1-yl, 1-cyclopenten-1-yl, 2-cyclohexen-1-yl, 3-cyclohexen-1-yl, 1-cyclohexen-1-yl and the like.

Examples of the $C_{4-10}$ cycloalkadienyl group include 2,4-cyclopentadien-1-yl, 2,4-cyclohexadien-1-yl, 2,5-cyclohexadien-1-yl and the like.

The above-mentioned $C_{3-10}$ cycloalkyl group, $C_{3-10}$ cycloalkenyl group and $C_{4-10}$ cycloalkadienyl group may be each condensed with a benzene ring. Examples of such fused ring group include indanyl (e.g., 1-indanyl, 2-indanyl), dihydronaphthyl (e.g., 3,4-dihydronaphthalen-1-yl), tetrahydronaphthyl (e.g., 1,2,3,4-tetrahydronaphthalen-1-yl, 1,2,3,4-tetrahydronaphthalen-2-yl), fluorenyl (e.g., 9-fluorenyl) and the like. In addition, examples of the aforementioned non-aromatic cyclic hydrocarbon group also include crosslinked hydrocarbon groups such as bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl, norbornanyl and the like, and the like.

Examples of the heterocyclic group include an aromatic heterocyclic group and a nonaromatic heterocyclic group.

Examples of the aromatic heterocyclic group include a 5-to 7-membered monocyclic aromatic heterocyclic group containing, as a ring-constituting atom besides carbon atom, 1 to 4 hetero atoms selected from an oxygen atom, a sulfur atom and a nitrogen atom and a condensed aromatic heterocyclic group. Examples of the condensed aromatic heterocyclic group include a group derived from a ring wherein a ring constituting such 5- to 7-membered monocyclic aromatic heterocyclic, and 1 or 2 rings such as a 5- or 6-membered ring containing 1 or 2 nitrogen atoms, a 5-membered ring containing one sulfur atom or a benzene ring and the like are condensed, and the like.

Preferable examples of the above-mentioned aromatic heterocyclic group include monocyclic aromatic heterocyclic group such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 4-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-3-yl, 1,2,4-oxadiazol-5-yl, 1,3,4-oxadiazol-2-yl), thiadiazolyl (e.g., 1,3,4-thiadiazol-2-yl, 1,2,4-thiadiazol-3-yl, 1,2,4-thiadiazol-5-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-1-yl, 1,2,4-triazin-3-yl, 1,3,5-triazin-2-yl) and the like; condensed aromatic heterocyclic group such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuranyl (e.g., 2-benzofuranyl, 3-benzofuranyl), benzothiophenyl (e.g., 2-benzothiophenyl, 3-benzothiophenyl), benzoxazolyl (e.g., 2-benzoxazolyl), benzisoxazolyl (e.g., 7-benzisooxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl, 1H-pyrazolo[5,4-b]pyridin-4-yl), pyrazolothienyl (e.g., 2H-pyrazolo[3,4-b]thiophen-2-yl), pyrazolotriazinyl (e.g., pyrazolo[5,1-c][1,2,4]triazin-3-yl), thiazolopyridinyl (e.g., thiazolo[4,5-b]pyridin-7-yl), pyridopyridinyl (e.g., pyrido[2,3-b]pyridin-3-yl), etc.; and the like.

Examples of the above-mentioned nonaromatic heterocyclic group include a 4- to 7-membered (preferably 5- or 6-membered) monocyclic nonaromatic heterocyclic group containing, as a ring-constituting atom besides carbon atom, 1 to 4 hetero atoms selected from an oxygen atom, a sulfur atom and a nitrogen atom and a condensed nonaromatic heterocyclic group. Examples of the condensed nonaromatic heterocyclic group include a group derived from a ring wherein a ring constituting such 4- to 7-membered monocyclic nonaromatic heterocyclic, and 1 or 2 rings such as a 5- or 6-membered ring containing 1 or 2 nitrogen atoms, a 5-membered ring containing one sulfur atom or a benzene ring and the like are condensed, and the like.

Preferable examples of the nonaromatic heterocyclic group include

monocyclic nonaromatic heterocyclic group such as pyrrolidinyl (e.g., 1-pyrrolidinyl), piperidinyl (e.g., piperidino, 2-piperidinyl, 3-piperidinyl, 4-piperidinyl), morpholinyl (e.g., morpholino), thiomorpholinyl (e.g., thiomorpholino), piperazinyl (e.g., 1-piperazinyl, 2-piperazinyl, 3-piperazinyl), hexamethyleniminyl (e.g., hexamethyleneimin-1-yl), oxazolidinyl (e.g., oxazolidin-2-yl), thiazolidinyl (e.g., thiazolidin-2-yl), imidazolidinyl (e.g., imidazolidin-2-yl, imidazolidin-3-yl), oxazolinyl (e.g., oxazolin-2-yl), thiazolinyl (e.g., thiazolin-2-yl), imidazolinyl (e.g., imidazolin-2-yl, imidazolin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, pyranyl (e.g., 4-pyranyl), tetrahydropyranyl (e.g., 4-tetrahydropyranyl), thiopyranyl (e.g., 4-thiopyranyl), tetrahydrothiopyranyl (e.g., 4-tetrahydrothiopyranyl), 1-oxidotetrahydrothiopyranyl (e.g., 1-oxidotetrahydrothiopyran-4-yl), 1,1-dioxidotetrahydrothiopyranyl (e.g., 1,1-dioxidotetrahydrothiopyran-4-yl), tetrahydrofuryl (e.g., tetrahydrofuran-3-yl), pyrazolidinyl (e.g., pyrazolidin-1-yl), pyrazolinyl (e.g., pyrazolin-1-yl), tetrahydropyrimidinyl and the like;

condensed nonaromatic heterocyclic group such as dihydroindolyl (e.g., 2,3-dihydro-1H-indol-1-yl), dihydroisoindolyl (e.g., 1,3-dihydro-2H-isoindol-2-yl), dihydrobenzofuranyl (e.g., 2,3-dihydrobenzofuran-5-yl), dihydrobenzodioxinyl (e.g., 2,3-dihydro-1,4-benzodioxinyl), dihydrobenzodioxepinyl (e.g., 3,4-dihydro-2H-1,5-benzodioxepinyl), tetrahydrobenzofuranyl (e.g., 4,5,6,7-tetrahydrobenzofuran-3-yl), tetrahydrobenzothiophenyl (e.g., 4,5,6,7-tetrahydrobenzothiophen-3-yl), chromenyl (e.g., 4H-chromen-2-yl, 2H-chromen-3-yl), dihydroquinolinyl (e.g., 1,2-dihydroquinolin-4-yl), tetrahydroquinolinyl (e.g., 1,2,3,4-tetrahydroquinolin-4-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl), tetrahydrothienopyridinyl (e.g., 4,5,6,7-tetrahydrothieno[2,3-c]pyridin-3-yl), dihydrothienopyranyl (e.g., 4,7-dihydro-5H-thieno[2,3-c]pyran-3-yl), dihydrothienothiopyranyl (e.g., 4,7-dihydro-5H-thieno[2,3-c]thiopyran-3-yl), xanthenyl (e.g., 9-xanthenyl), etc.;

and the like.

Examples of the "cyclic group" of the "optionally substituted cyclic group" for E include an aromatic hydrocarbon group (preferably phenyl, naphthyl, anthryl, fluorenyl; more preferably anthryl), an aromatic heterocyclic group (preferably pyridyl, quinolyl, benzofuranyl, benzothiophenyl, thienyl, pyrazolyl, imidazolyl, pyrimidinyl, oxazolyl, isoxazolyl, thiazolyl, triazinyl, isoquinolyl, imidazopyridinyl, pyrazolopyridinyl, thiazolopyridinyl, pyridopyridinyl; more preferably pyridyl, quinolyl, thienyl, benzothiophenyl), nonaromatic heterocyclic group (preferably tetrahydrobenzothiophenyl, tetrahydrothienopyridinyl, dihydrothienopyranyl, dihydrothienothiopyranyl, xanthenyl) and the like are preferable, and an aromatic hydrocarbon group (preferably phenyl, naphthyl, anthryl, fluorenyl), and an aromatic heterocyclic group (preferably pyridyl, quinolyl, benzofuranyl, benzothiophenyl, thienyl, pyrazolyl, imidazolyl, pyrimidinyl, oxazolyl, isoxazolyl, thiazolyl, triazinyl, isoquinolyl, imidazopyridinyl, pyrazolopyridinyl, thiazolopyridinyl, pyridopyridinyl) are more preferable.

E is preferably an optionally substituted aromatic hydrocarbon group (particularly preferably anthryl), or an optionally substituted aromatic heterocyclic group (particularly preferably pyridyl, quinolyl, thienyl, benzothiophenyl).

The "cyclic group" of the "optionally substituted cyclic group" for E optionally has 1 to 3 substituents at substitutable position(s).

Examples of such substituent include

(1) a $C_{3-10}$ cycloalkyl group (e.g., cyclopropyl, cyclobutyl, cyclohexyl);

(2) a $C_{6-14}$ aryl group (e.g., phenyl, naphthyl) optionally substituted with 1 to 3 substituents selected from

(i) a halogen atom,

(ii) a hydroxy group,

(iii) a carboxyl group,
(iv) a cyano group,
(v) a $C_{1-6}$ alkyl group optionally substituted with 1 to 3 substituents selected from

(a) a halogen atom,
(b) a carboxyl group, and
(c) a $C_{1-6}$ alkoxy-carbonyl group,

(vi) a $C_{1-6}$ alkoxy group optionally substituted with 1 to 3 substituents selected from

(a) a carboxyl group, and
(b) a $C_{1-6}$ alkoxy-carbonyl group optionally substituted with 1 to 3 $C_{6-14}$ aryl groups (e.g., phenyl),

(vii) a $C_{1-6}$ alkyl-carbonyl group,
(viii) a $C_{1-6}$ alkoxy-carbonyl group optionally substituted with 1 to 3 $C_{6-14}$ aryl groups (e.g., phenyl),
(ix) an amino group optionally mono- or di-substituted with substituent(s) selected from

(a) a $C_{1-6}$ alkyl-carbonyl group, and
(b) a $C_{1-6}$ alkylsulfonyl group (e.g., methylsulfonyl),

(x) a carbamoyl group, and
(xi) a $C_{1-6}$ alkylthio group (e.g., methylthio);

(3) an aromatic heterocyclic group (e.g., thienyl, furyl, pyridyl, oxazolyl, thiazolyl, tetrazolyl, oxadiazolyl, pyrazinyl, quinolyl, indolyl, pyrrolyl, imidazolyl, pyrazolyl, triazolyl, benzimidazolyl, indazolyl, benzotriazolyl, benzothiophenyl, pyrrolopyridinyl) optionally substituted with 1 to 3 substituents selected from

(i) a halogen atom,
(ii) a hydroxy group,
(iii) a $C_{1-6}$ alkyl group optionally substituted with 1 to 3 halogen atoms,
(iv) a $C_{1-6}$ alkoxy group, and
(v) a nonaromatic heterocyclic group (e.g., morpholinyl, pyrrolidinyl);

(4) a nonaromatic heterocyclic group (e.g., tetrahydrofuryl, morpholinyl, thiomorpholinyl, piperidinyl, pyrrolidinyl, piperazinyl, dioxolyl, dioxolanyl, 1,3-dihydro-2-benzofuranyl, thiazolidinyl, isoindolinyl) optionally substituted with 1 to 3 substituents selected from

(i) a halogen atom,
(ii) a hydroxy group,
(iii) a carboxyl group,
(iv) an oxo group,
(v) a $C_{1-6}$ alkyl group optionally substituted with 1 to 3 halogen atoms,
(iv) a $C_{1-6}$ alkoxy group,
(vii) a $C_{1-6}$ alkoxy-carbonyl group optionally substituted with 1 to 3 $C_{6-14}$ aryl groups (e.g., phenyl), and
(viii) a $C_{1-3}$ alkylenedioxy group;

(5) an amino group optionally mono- or di-substituted with substituent(s) selected from

(i) a $C_{1-6}$ alkyl-carbonyl group optionally substituted with 1 to 3 substituents selected from

(a) a $C_{1-6}$ alkoxy-carbonyl group,
(b) a $C_{1-6}$ alkoxy group, and
(c) a $C_{6-14}$ aryl group (e.g., phenyl),

(ii) a $C_{1-6}$ alkoxy-carbonyl group optionally substituted with 1 to 3 substituents selected from

(a) a $C_{6-14}$ aryl group (e.g., phenyl), and
(b) a $C_{1-6}$ alkoxy group,

(iii) a $C_{6-14}$ aryl-carbonyl group (e.g., benzoyl);
(iv) an aromatic heterocyclylcarbonyl group (e.g., thenoyl)
(v) a carbamoyl group optionally mono- or di-substituted with substituent(s) selected from

(a) a $C_{1-6}$ alkyl group optionally substituted with 1 to 3 substituents selected from

(a-1) a $C_{1-6}$ alkoxy-carbonyl group,
(a-2) a $C_{6-14}$ aryl group (e.g., phenyl), and
(a-3) a $C_{1-6}$ alkoxy group,

(b) a $C_{3-10}$ cycloalkyl group (e.g., cyclohexyl),
(c) a $C_{6-14}$ aryl group (e.g., phenyl) optionally substituted with 1 to 3 substituents selected from

(c-1) a halogen atom,
(c-2) a $C_{1-6}$ alkyl group optionally substituted with 1 to 3 halogen atoms, and
(c-3) a $C_{1-6}$ alkoxy group, and

(d) an aromatic heterocyclic group (e.g., pyridyl),

(vi) a $C_{1-6}$ alkylsulfonyl group (e.g., methylsulfonyl, ethylsulfonyl, isopropylsulfonyl) optionally substituted with 1 to 3 $C_{6-14}$ aryl groups (e.g., phenyl),
(vii) a $C_{6-14}$ arylsulfonyl group (e.g., benzenesulfonyl, toluenesulfonyl, 1-naphthalenesulfonyl, 2-naphthalenesulfonyl) optionally substituted with 1 to 3 halogen atoms,
(viii) a $C_{1-6}$ alkyl group optionally substituted with 1 to 3 substituents selected from

(a) an amino group optionally mono- or di-substituted with $C_{1-6}$ alkoxy-carbonyl group(s), and
(b) an imino group,
and

(ix) a thiocarbamoyl group optionally mono- or di-substituted with $C_{1-6}$ alkyl group(s);

(6) an amidino group;
(7) a $C_{1-6}$ alkyl-carbonyl group optionally substituted with 1 to 3 halogen atoms;
(8) a $C_{1-6}$ alkoxy-carbonyl group optionally substituted with 1 to 3 substituents selected from

(i) a halogen atom, and
(ii) a $C_{6-14}$ aryl group (e.g., phenyl);

(9) a $C_{1-6}$ alkylsulfonyl group (e.g., methylsulfonyl) optionally substituted with 1 to 3 halogen atoms;
(10) a carbamoyl group optionally mono- or di-substituted with substituent(s) selected from

(i) a $C_{1-6}$ alkyl group optionally substituted with 1 to 3 halogen atoms,
(ii) a $C_{6-14}$ aryl group (e.g., phenyl),
(iii) a $C_{7-13}$ aralkyl group (e.g., benzyl), and
(iv) an aromatic heterocyclyl-$C_{1-6}$ alkyl group (e.g., furfuryl);

(11) a thiocarbamoyl group optionally mono- or di-substituted with $C_{1-6}$ alkyl group(s) optionally substituted with 1 to 3 halogen atoms;
(12) a sulfamoyl group optionally mono- or di-substituted with $C_{1-6}$ alkyl group(s) optionally substituted with 1 to 3 substituents selected from

(i) a halogen atom, and
(ii) a nonaromatic heterocyclic group (e.g., pyrrolidinyl) optionally substituted with oxo group(s);

(13) a carboxyl group;
(14) a hydroxy group;
(15) a $C_{1-6}$ alkoxy group optionally substituted with 1 to 3 substituents selected from

(i) a halogen atom,
(ii) a carboxyl group,
(iii) a $C_{1-6}$ alkoxy group, and
(iv) a $C_{1-6}$ alkoxy-carbonyl group;

(16) a $C_{2-6}$ alkenyloxy group (e.g., ethenyloxy) optionally substituted with 1 to 3 halogen atoms;
(17) a $C_{3-10}$ cycloalkyloxy group (e.g., cyclohexyloxy);
(18) a $C_{7-13}$ aralkyloxy group (e.g., benzyloxy) optionally substituted with 1 to 3 halogen atoms;
(19) a $C_{6-14}$ aryloxy group (e.g., phenyloxy, naphthyloxy);
(20) a $C_{1-6}$ alkyl-carbonyloxy group (e.g., acetyloxy, tert-butylcarbonyloxy);
(21) a mercapto group;
(22) a $C_{1-6}$ alkylthio group (e.g., methylthio, ethylthio) optionally substituted with 1 to 3 substituents selected from

(i) a halogen atom, and
(ii) a $C_{6-14}$ aryl group;

(23) a $C_{7-20}$ aralkylthio group (e.g., benzylthio, tritylthio);
(24) a $C_{6-14}$ arylthio group (e.g., phenylthio, naphthylthio);
(25) a sulfo group;
(26) a cyano group;
(27) an azido group;
(28) a nitro group;
(29) a nitroso group;
(30) a halogen atom;
(31) a $C_{1-6}$ alkylsulfinyl group (e.g., methylsulfinyl);
(32) an oxo group;
(33) a $C_{3-10}$ cycloalkyl-$C_{1-6}$ alkyloxy group (e.g., cyclopropylmethyloxy);
(34) a $C_{1-3}$ alkylenedioxy group;
(35) a carbamoyloxy group optionally mono- or di-substituted with $C_{1-6}$ alkyl group(s);
(36) a $C_{1-6}$ alkyl group optionally substituted with 1 to 3 substituents selected from

(i) a halogen atom,
(ii) a hydroxy group,
(iii) a carboxyl group,
(iv) a cyano group,
(v) a $C_{1-6}$ alkoxy group,
(vi) a $C_{1-6}$ alkoxy-carbonyl group,
(vii) a $C_{1-6}$ alkyl-carbonyloxy group (e.g., acetyloxy, tert-butylcarbonyloxy),
(viii) a carbamoyl group,
(ix) a $C_{3-10}$ cycloalkyl group (e.g., cyclopropyl),
(x) an aromatic heterocyclic group (e.g., thiazolyl, oxazolyl, pyridyl, imidazolyl),
(xi) a nonaromatic heterocyclic group (e.g., piperidinyl, pyrrolidinyl)
(xii) an amino group optionally mono- or di-substituted with substituent(s) selected from

(a) a $C_{1-6}$ alkyl-carbonyl group,
(b) a $C_{1-6}$ alkoxy-carbonyl group,
(c) a $C_{1-6}$ alkylsulfonyl group (e.g., methylsulfonyl)
(d) a carbamoyl group optionally mono- or di-substituted with $C_{1-6}$ alkyl group(s), and
(e) an aromatic heterocyclic group (e.g., thiazolyl, oxazolyl, pyridyl, imidazolyl),

(xiii) a $C_{1-6}$ alkylthio group (e.g., methylthio, ethylthio) optionally substituted with 1 to 3 $C_{1-6}$ alkoxy-carbonyl groups, and
(xiv) a tri$C_{1-6}$ alkylsilyloxy group (e.g., tert-butyldimethylsilyloxy);

(37) a $C_{2-6}$ alkenyl group (e.g., ethenyl, 1-propenyl) optionally substituted with 1 to 3 substituents selected from

(i) a halogen atom,
(ii) a hydroxy group,

(iii) a carboxyl group,
(iv) a cyano group,
(v) a $C_{1-6}$ alkoxy group,
(vi) a $C_{1-6}$ alkoxy-carbonyl group,
(vii) a $C_{1-6}$ alkyl-carbonyloxy group (e.g., acetyloxy, tert-butylcarbonyloxy),
(viii) a carbamoyl group,
(ix) a $C_{3-10}$ cycloalkyl group (e.g., cyclopropyl),
(x) an aromatic heterocyclic group (e.g., thiazolyl, oxazolyl, pyridyl, imidazolyl),
(xi) a nonaromatic heterocyclic group (e.g., piperidinyl, pyrrolidinyl)
(xii) an amino group optionally mono- or di-substituted with substituent(s) selected from

(a) a $C_{1-6}$ alkyl-carbonyl group,
(b) a $C_{1-6}$ alkoxy-carbonyl group,
(c) a $C_{1-6}$ alkylsulfonyl group (e.g., methylsulfonyl)
(d) a carbamoyl group optionally mono- or di-substituted with $C_{1-6}$ alkyl group(s), and
(e) an aromatic heterocyclic group (e.g., thiazolyl, oxazolyl, pyridyl, imidazolyl), and

(xiii) a $C_{1-6}$ alkylthio group (e.g., methylthio, ethylthio) optionally substituted with 1 to 3 $C_{1-6}$ alkoxy-carbonyl groups;

(38) a $C_{7-13}$ aralkyl group (e.g., benzyl) optionally substituted with 1 to 3 substituents selected from

(i) a $C_{1-6}$ alkyl group optionally substituted with 1 to 3 halogen atoms,
(ii) a hydroxy group,
(iii) a $C_{1-6}$ alkoxy group, and
(iv) a halogen atom;

(39) a $C_{2-6}$ alkynyl group (e.g., ethynyl, propynyl) optionally substituted with 1 to 3 substituents selected from

(i) a halogen atom,
(ii) a hydroxy group,
(iii) a carboxyl group,
(iv) a cyano group,
(v) a $C_{1-6}$ alkoxy group,
(vi) a $C_{1-6}$ alkoxy-carbonyl group,
(vii) a $C_{1-6}$ alkyl-carbonyloxy group (e.g., acetyloxy, tert-butylcarbonyloxy),
(viii) a carbamoyl group,
(ix) a $C_{3-10}$ cycloalkyl group (e.g., cyclopropyl),
(x) an aromatic heterocyclic group (e.g., thiazolyl, oxazolyl, pyridyl, imidazolyl),
(xi) a nonaromatic heterocyclic group (e.g., piperidinyl, pyrrolidinyl)
(xii) an amino group optionally mono- or di-substituted with substituent(s) selected from

(a) a $C_{1-6}$ alkyl-carbonyl group,
(b) a $C_{1-6}$ alkoxy-carbonyl group,
(c) a $C_{1-6}$ alkylsulfonyl group (e.g., methylsulfonyl)
(d) a carbamoyl group optionally mono- or di-substituted with $C_{1-6}$ alkyl group(s), and
(e) an aromatic heterocyclic group (e.g., thiazolyl, oxazolyl, pyridyl, imidazolyl), and

(xiii) a $C_{1-6}$ alkylthio group (e.g., methylthio, ethylthio)optionally substituted with 1 to 3 $C_{1-6}$ alkoxy-carbonyl groups;
and the like.

Examples of preferable substituent for E include

(1) a halogen atom;
(2) a hydroxy group;
(3) an oxo group;
(4) a nitro group;

(5) a cyano group;
(6) a $C_{1-6}$ alkyl group optionally substituted with 1 to 3 substituents selected from

(i) a halogen atom,
(ii) a hydroxy group,
(iii) a carboxyl group,
(iv) a $C_{1-6}$ alkoxy-carbonyl group,
(v) a $C_{1-6}$ alkyl-carbonyloxy group (e.g., acetyloxy),
(vi) an amino group optionally mono- or di-substituted with substituent(s) selected from

(a) a $C_{1-6}$ alkyl-carbonyl group,
(b) a $C_{1-6}$ alkoxy-carbonyl group, and
(c) a $C_{1-6}$ alkylsulfonyl group (e.g., methylsulfonyl), and

(vii) a $C_{1-6}$ alkylthio group (e.g., methylthio, ethylthio) optionally substituted with 1 to 3 $C_{1-6}$ alkoxy-carbonyl groups;

(7) a $C_{2-6}$ alkenyl group (e.g., ethenyl) optionally substituted with 1 to 3 $C_{1-6}$ alkoxy-carbonyl groups;
(8) a $C_{1-6}$ alkoxy group optionally substituted with 1 to 3 substituents selected from

(i) a carboxyl group, and
(ii) a $C_{1-6}$ alkoxy-carbonyl group;

(9) a $C_{6-14}$ aryl group (e.g., phenyl, naphthyl) optionally substituted with 1 to 3 substituents selected from

(i) a halogen atom,
(ii) a hydroxy group,
(iii) a carboxyl group,
(iv) a cyano group,
(v) a $C_{1-6}$ alkyl group optionally substituted with 1 to 3 substituents selected from

(a) a halogen atom,
(b) a carboxyl group, and
(c) a $C_{1-6}$ alkoxy-carbonyl group,

(vi) a $C_{1-6}$ alkoxy group optionally substituted with 1 to 3 substituents selected from

(a) a carboxyl group, and
(b) a $C_{1-6}$ alkoxy-carbonyl group optionally substituted with 1 to 3 $C_{6-14}$ aryl groups (e.g., phenyl),

(vii) a $C_{1-6}$ alkyl-carbonyl group,
(viii) a $C_{1-6}$ alkoxy-carbonyl group optionally substituted with 1 to 3 $C_{6-14}$ aryl groups (e.g., phenyl),
(ix) an amino group optionally mono- or di-substituted with substituent(s) selected from

(a) a $_{1-6}$ alkyl-carbonyl group, and
(b) a $C_{1-6}$ alkylsulfonyl group (e.g., methylsulfonyl),

(x) a carbamoyl group, and
(xi) a $C_{1-6}$ alkylthio group (e.g., methylthio);

(10) a $C_{3-10}$ cycloalkyl group (e.g., cyclopropyl);
(11) a $C_{7-13}$ aralkyl group (e.g., benzyl);
(12) a $C_{6-14}$ aryloxy group (e.g., phenoxy);
(13) a $C_{7-13}$ aralkyloxy group (e.g., benzyloxy);
(14) a $C_{1-6}$ alkyl-carbonyl group;
(15) a $C_{1-6}$ alkoxy-carbonyl group;
(16) an aromatic heterocyclic group (e.g., pyridyl, pyrrolyl, imidazolyl, pyrazolyl, triazolyl, furyl, thienyl, benzimidazolyl, indazolyl, benzotriazolyl, benzothiophenyl, pyrrolopyridinyl) optionally substituted with 1 to 3 substituents selected

from

(i) a $C_{1-6}$ alkyl group,
(ii) a $C_{1-6}$ alkoxy group, and
(iii) a nonaromatic heterocyclic group (e.g., morpholinyl, pyrrolidinyl);

(17) a nonaromatic heterocyclic group (e.g., piperidinyl, morpholinyl, isoindolinyl, pyrrolidinyl) optionally substituted with 1 to 3 substituents selected from

(i) a hydroxy group,
(ii) a carboxyl group,
(iii) an oxo group,
(iv) a $C_{1-6}$ alkoxy-carbonyl group optionally substituted with 1 to 3 $C_{6-14}$ aryl groups (e.g., phenyl),
(v) a $C_{1-6}$ alkoxy group, and
(vi) a $C_{1-3}$ alkylenedioxy group;

(18) an amino group optionally mono- or di-substituted with substituent(s) selected from

(i) a $C_{1-6}$ alkyl-carbonyl group optionally substituted with 1 to 3 substituents selected from

(a) a $C_{1-6}$ alkoxy-carbonyl group,
(b) a $C_{1-6}$ alkoxy group, and
(c) a $C_{6-14}$ aryl group (e.g., phenyl),

(ii) a $C_{1-6}$ alkoxy-carbonyl group optionally substituted with 1 to 3 substituents selected from

(a) a $C_{6-14}$ aryl group (e.g., phenyl), and
(b) a $C_{1-6}$ alkoxy group,

(iii) an aromatic heterocyclylcarbonyl group (e.g., thenoyl)
(iv) a carbamoyl group optionally mono- or di-substituted with substituent(s) selected from

(a) a $C_{1-6}$ alkyl group optionally substituted with 1 to 3 substituents selected from

(a-1) a $C_{1-6}$ alkoxy-carbonyl group,
(a-2) a $C_{6-14}$ aryl group (e.g., phenyl), and
(a-3) a $C_{1-6}$ alkoxy group,

(b) a $C_{3-10}$ cycloalkyl group (e.g., cyclohexyl),
(c) a $C_{6-14}$ aryl group (e.g., phenyl) optionally substituted with 1 to 3 substituents selected from

(c-1) a halogen atom,
(c-2) a $C_{1-6}$ alkyl group optionally substituted with 1 to 3 halogen atoms, and
(c-3) a $C_{1-6}$ alkoxy group, and

(d) an aromatic heterocyclic group (e.g., pyridyl),

(v) a $C_{1-6}$ alkylsulfonyl group (e.g., methylsulfonyl, ethylsulfonyl) optionally substituted with 1 to 3 $C_{6-14}$ aryl groups (e.g., phenyl),
(vi) a $C_{6-14}$ arylsulfonyl group (e.g., benzenesulfonyl) optionally substituted with 1 to 3 halogen atoms,
(vii) a $C_{1-6}$ alkyl group optionally substituted with 1 to 3 substituents selected from

(a) an amino group optionally mono- or di-substituted with $C_{1-6}$ alkoxy-carbonyl group(s), and
(b) an imino group, and

(viii) a thiocarbamoyl group optionally mono- or di-substituted with $C_{1-6}$ alkyl group(s);

(19) a carbamoyloxy group optionally mono- or di-substituted with $C_{1-6}$ alkyl group(s);

(20) a sulfamoyl group optionally mono- or di-substituted with $C_{1-6}$ alkyl group(s) optionally substituted with nonaromatic heterocyclic group(s) (e.g., pyrrolidinyl) optionally substituted with oxo group(s);
and the like.

As E,
an aromatic hydrocarbon group (preferably phenyl, naphthyl, anthryl, fluorenyl), an aromatic heterocyclic group (preferably pyridyl, quinolyl, benzofuranyl, benzothiophenyl, thienyl, pyrazolyl, imidazolyl, pyrimidinyl, oxazolyl, isoxazolyl, thiazolyl, triazinyl, isoquinolyl, imidazopyridinyl, pyrazolopyridinyl, thiazolopyridinyl, pyridopyridinyl), a nonaromatic heterocyclic group (preferably tetrahydrobenzothiophenyl, tetrahydrothienopyridinyl, dihydrothienopyranyl, dihydrothienothiopyranyl, xanthenyl), each of which is optionally substituted with 1 to 3 substituents selected from

(1) a halogen atom;
(2) a hydroxy group;
(3) an oxo group;
(4) a nitro group;
(5) a cyano group;
(6) a $C_{1-6}$ alkyl group optionally substituted with 1 to 3 substituents selected from

(i) a halogen atom,
(ii) a hydroxy group,
(iii) a carboxyl group,
(iv) a $C_{1-6}$ alkoxy-carbonyl group,
(v) a $C_{1-6}$ alkyl-carbonyloxy group (e.g., acetyloxy),
(vi) an amino group optionally mono- or di-substituted with substituent(s) selected from

(a) a $C_{1-6}$ alkyl-carbonyl group,
(b) a $C_{1-6}$ alkoxy-carbonyl group, and
(c) a $C_{1-6}$ alkylsulfonyl group (e.g., methylsulfonyl), and

(vii) a $C_{1-6}$ alkylthio group (e.g., methylthio, ethylthio) optionally substituted with 1 to 3 $C_{1-6}$ alkoxy-carbonyl groups;

(7) a $C_{2-6}$ alkenyl group (e.g., ethenyl) optionally substituted with 1 to 3 $C_{1-6}$ alkoxy-carbonyl groups;
(8) a $C_{1-6}$ alkoxy group optionally substituted with 1 to 3 substituents selected from

(i) a carboxyl group, and
(ii) a $C_{1-6}$ alkoxy-carbonyl group;

(9) a $C_{6-14}$ aryl group (e.g., phenyl, naphthyl) optionally substituted with 1 to 3 substituents selected from

(i) a halogen atom,
(ii) a hydroxy group,
(iii) a carboxyl group,
(iv) a cyano group,
(v) a $C_{1-6}$ alkyl group optionally substituted with 1 to 3 substituents selected from

(a) a halogen atom,
(b) a carboxyl group, and
(c) a $C_{1-6}$ alkoxy-carbonyl group,

(vi) a $C_{1-6}$ alkoxy group optionally substituted with 1 to 3 substituents selected from

(a) a carboxyl group, and
(b) a $C_{1-6}$ alkoxy-carbonyl group optionally substituted with 1 to 3 $C_{6-14}$ aryl groups (e.g., phenyl),

(vii) a $C_{1-6}$ alkyl-carbonyl group,
(viii) a $C_{1-6}$ alkoxy-carbonyl group optionally substituted with 1 to 3 $C_{6-14}$ aryl groups (e.g., phenyl),
(ix) an amino group optionally mono- or di-substituted with substituent(s) selected from

(a) a $C_{1-6}$ alkyl-carbonyl group, and
(b) a $C_{1-6}$ alkylsulfonyl group (e.g., methylsulfonyl),

(x) a carbamoyl group, and
(xi) a $C_{1-6}$ alkylthio group (e.g., methylthio);

(10) a $C_{3-10}$ cycloalkyl group (e.g., cyclopropyl);
(11) a $C_{7-13}$ aralkyl group (e.g., benzyl);
(12) a $C_{6-14}$ aryloxy group (e.g., phenoxy) ;
(13) a $C_{7-13}$ aralkyloxy group (e.g., benzyloxy);
(14) a $C_{1-6}$ alkyl-carbonyl group;
(15) a $C_{1-6}$ alkoxy-carbonyl group;
(16) an aromatic heterocyclic group (e.g., pyridyl, pyrrolyl, imidazolyl, pyrazolyl, triazolyl, furyl, thienyl, benzimidazolyl, indazolyl, benzotriazolyl, benzothiophenyl, pyrrolopyridinyl) optionally substituted with 1 to 3 substituents selected from

(i) a $C_{1-6}$ alkyl group,
(ii) a $C_{1-6}$ alkoxy group, and
(iii) a nonaromatic heterocyclic group (e.g., morpholinyl, pyrrolidinyl);

(17) a nonaromatic heterocyclic group (e.g., piperidinyl, morpholinyl, isoindolinyl, pyrrolidinyl) optionally substituted with 1 to 3 substituents selected from

(i) a hydroxy group,
(ii) a carboxyl group,
(iii) an oxo group,
(iv) a $C_{1-6}$ alkoxy-carbonyl group optionally substituted with 1 to 3 $C_{6-14}$ aryl groups (e.g., phenyl),
(v) a $C_{1-6}$ alkoxy group, and
(vi) a $C_{1-3}$ alkylenedioxy group;

(18) an amino group optionally mono- or di-substituted with substituent(s) selected from

(i) a $C_{1-6}$ alkyl-carbonyl group optionally substituted with 1 to 3 substituents selected from

(a) a $C_{1-6}$ alkoxy-carbonyl group,
(b) a $C_{1-6}$ alkoxy group, and
(c) a $C_{6-14}$ aryl group (e.g., phenyl),

(ii) a $C_{1-6}$ alkoxy-carbonyl group optionally substituted with 1 to 3 substituents selected from

(a) a $C_{6-14}$ aryl group (e.g., phenyl), and
(b) a $C_{1-6}$ alkoxy group,

(iii) an aromatic heterocyclylcarbonyl group (e.g., thenoyl)
(iv) a carbamoyl group optionally mono- or di-substituted with substituent(s) selected from

(a) a $C_{1-6}$ alkyl group optionally substituted with 1 to 3 substituents selected from

(a-1) a $C_{1-6}$ alkoxy-carbonyl group,
(a-2) a $C_{6-14}$ aryl group (e.g., phenyl), and
(a-3) a $C_{1-6}$ alkoxy group,

(b) a $C_{3-10}$ cycloalkyl group (e.g., cyclohexyl),
(c) a $C_{6-14}$ aryl group (e.g., phenyl) optionally substituted with 1 to 3 substituents selected from

(c-1) a halogen atom,
(c-2) a $C_{1-6}$ alkyl group optionally substituted with 1 to 3 halogen atoms, and
(c-3) a $C_{1-6}$ alkoxy group, and

(d) an aromatic heterocyclic group (e.g., pyridyl),

(v) a $C_{1-6}$ alkylsulfonyl group (e.g., methylsulfonyl, ethylsulfonyl) optionally substituted with 1 to 3 $C_{6-14}$ aryl groups (e.g., phenyl),
(vi) a $C_{6-14}$ arylsulfonyl group (e.g., benzenesulfonyl) optionally substituted with 1 to 3 halogen atoms,
(vii) a $C_{1-6}$ alkyl group optionally substituted with 1 to 3 substituents selected from

(a) an amino group optionally mono- or di-substituted with $C_{1-6}$ alkoxy-carbonyl group(s), and
(b) an imino group, and

(viii) a thiocarbamoyl group optionally mono- or di-substituted with $C_{1-6}$ alkyl group(s);

(19) a carbamoyloxy group optionally mono- or di-substituted with $C_{1-6}$ alkyl group(s);
(20) a sulfamoyl group optionally mono- or di-substituted with $C_{1-6}$ alkyl group(s) optionally substituted with nonaromatic heterocyclic group(s) (e.g., pyrrolidinyl) optionally substituted with oxo group(s); and the like
and the like are preferable.

D is a carbonyl group or a sulfonyl group.
As D, a carbonyl group is preferable.
Ring P is an optionally further substituted 5- to 7-membered ring. Examples of the "5- to 7-membered ring" of the "optionally further substituted 5- to 7-membered ring" include, from those exemplified as the "cyclic group" of the "optionally substituted cyclic group" for E, a ring constituting the 5- to 7-membered cyclic group. specifically, benzene, cycloalkane having 5 to 7 carbon atoms, cycloalkene having 5 to 7 carbon atoms, cycloalkadiene having 5 to 7 carbon atoms, 5- to 7-membered monocyclic aromatic heterocycle, 5- to 7-membered monocyclic non-aromatic heterocycle and the like can be mentioned. Of these, a 5- to 7-membered nitrogen-containing non-aromatic heterocycle and the like are preferable. Preferable examples of the 5- to 7-membered nitrogen-containing non-aromatic heterocycle include pyrrolidine, piperidine, morpholine, thiomorpholine, piperazine, hexamethyleneimine, oxazolidine, thiazolidine, imidazolidine, pyrazolidine, oxazoline, thiazoline, imidazoline, pyrazoline, dihydrooxadiazole, tetrahydropyrimidine and the like. Of these, 6-membered nitrogen-containing non-aromatic heterocycle and the like are more preferable and piperidine, piperazine and the like are particularly preferable.
The "5- to 7-membered ring" of the "optionally further substituted 5- to 7-membered ring" for ring P is substituted by E-D-group and spiro ring constituting ring Q and ring R, and optionally further has 1 to 3 (preferably 1 or 2) substituents at substitutable position(s). Examples of the substituent include those exemplified as the substituent of the "cyclic group" of the aforementioned "optionally substituted cyclic group" for E.
As ring P, an optionally further substituted 5- to 7-membered nitrogen-containing non-aromatic heterocycle is preferable. Here, as the 5- to 7-membered nitrogen-containing non-aromatic heterocycle, a 6-membered nitrogen-containing non-aromatic heterocycle and the like are preferable, and piperidine, piperazine and the like are more preferable. Especially, piperidine is preferable.
Ring P is more preferably a 5- to 7-membered nitrogen-containing non-aromatic heterocycle, particularly preferably a 6-membered nitrogen-containing non-aromatic heterocycle (preferably piperidine, piperazine), and most preferably piperidine.
Ring Q is an optionally further substituted 5- to 7-membered nonaromatic ring. Examples of the "5- to 7-membered nonaromatic ring" of the "optionally further substituted 5- to 7-membered nonaromatic ring" include a ring constituting a 5-to 7-membered nonaromatic cyclic group from those exemplified as the "cyclic group" of the "optionally substituted cyclic group" for E. Specifically, cycloalkane having 5 to 7 carbon atoms, cycloalkene having 5 to 7 carbon atoms, cycloalkadiene having 5 to 7 carbon atoms, 5- to 7-membered monocyclic non-aromatic heterocycle and the like can be mentioned. Of these, 5- to 7-membered monocyclic non-aromatic heterocycle (preferably pyrrolidine, piperidine, piperazine, morpholine, thiomorpholine) and the like are preferable and 6-membered monocyclic non-aromatic heterocycle (preferably piperidine, piperazine, morpholine, thiomorpholine) are more preferable.
The "5- to 7-membered nonaromatic ring" of the "optionally further substituted 5- to 7-membered nonaromatic ring" for ring Q is substituted with ring P, and form a spiro ring with ring R. In addition, it may further have 1 to 3, preferably 1 or 2, substituents at substitutable position(s). Examples of the substituent include those exemplified as the substituent for the "cyclic group" of the aforementioned "optionally substituted cyclic group" for E.
Examples of preferable substituent for ring Q include

(1) a $C_{1-6}$ alkyl-carbonyl group optionally substituted with 1 to 3 halogen atoms;
(2) a $C_{1-6}$ alkyl group optionally substituted with 1 to 3 substituents selected from a carboxyl group and a $C_{1-6}$ alkoxy-carbonyl group;

(3) a $C_{1-6}$ alkoxy-carbonyl group optionally substituted with 1 to 3 $C_{6-14}$ aryl groups (e.g., phenyl); and the like.

As ring Q,
a 5- to 7-membered monocyclic non-aromatic heterocycle (preferably pyrrolidine, piperidine, piperazine, morpholine, thiomorpholine, more preferably 6-membered monocyclic non-aromatic heterocycle (preferably piperidine, piperazine, morpholine, thiomorpholine)) optionally substituted with 1 to 3 (preferably 1 or 2) substituents selected from

(1) a $C_{1-6}$ alkyl-carbonyl group optionally substituted with 1 to 3 halogen atoms;
(2) a $C_{1-6}$ alkyl group optionally substituted with 1 to 3 substituents selected from a carboxyl group and a $C_{1-6}$ alkoxy-carbonyl group;
(3) a $C_{1-6}$ alkoxy-carbonyl group optionally substituted with 1 to 3 $C_{6-14}$ aryl groups (e.g., phenyl); and the like and the like are preferable.

A is CH or N.
As A, N is preferable.
Ring R is an optionally further substituted and optionally condensed 5- to 7-membered nonaromatic ring. Examples of the "optionally condensed 5- to 7-membered nonaromatic ring" of the "optionally further substituted and optionally condensed 5- to 7-membered nonaromatic ring" include a ring constituting a 5- to 7-membered nonaromatic cyclic group from those exemplified as the "cyclic group" of the "optionally substituted cyclic group" for E. These may be condensed. Specifically, cycloalkane having 5 to 7 carbon atoms, cycloalkene having 5 to 7 carbon atoms, cycloalkadiene having 5 to 7 carbon atoms, 5- to 7-membered monocyclic non-aromatic heterocycle, each of which is optionally condensed with benzene ring and the like, and the like can be mentioned. Of these, cycloalkane having 5 to 7 carbon atoms and optionally condensed with a benzene ring (preferably tetrahydronaphthalene (e.g., 1,2,3,4-tetrahydronaphthalene), indane, benzocycloheptane, cyclopentane), 5- to 7-membered monocyclic non-aromatic heterocycle (preferably pyrrolidine, piperidine, tetrahydrofuran, imidazolidine, imidazoline, oxazolidine, oxazoline, thiazolidine, thiazoline) and the like are preferable and 5-membered monocyclic non-aromatic heterocycle (preferably pyrrolidine, tetrahydrofuran, imidazolidine, imidazoline, oxazolidine, oxazoline, thiazolidine, thiazoline) is more preferable.
Ring R is preferably an optionally further substituted 5-membered monocyclic non-aromatic heterocycle.
The "optionally condensed 5- to 7-membered nonaromatic ring" of the "optionally further substituted and optionally condensed 5- to 7-membered nonaromatic ring" for ring R is substituted with an oxo group, and form a spiro ring with ring Q. In addition, it may further have 1 to 3 (preferably 1 or 2) substituents at substitutable position(s). Examples of the substituent include those exemplified as the substituent of the "cyclic group" of the aforementioned "optionally substituted cyclic group" for E.
Examples of preferable substituent for ring R include

(1) a $C_{1-6}$ alkyl group optionally substituted with 1 to 3 substituents selected from

(i) a carboxyl group,
(ii) a hydroxy group,
(iii) a cyano group,
(iv) a $C_{1-6}$ alkoxy group,
(v) a $C_{3-10}$ cycloalkyl group (e.g., cyclopropyl),
(vi) a $C_{1-6}$ alkoxy-carbonyl group,
(vii) a $C_{1-6}$ alkyl-carbonyloxy group (e.g., acetyloxy),
(viii) an amino group optionally mono- or di-substituted with $C_{1-6}$ alkylsulfonyl group(s) (e.g., methylsulfonyl),
(ix) an aromatic heterocyclic group (e.g., thiazolyl, oxazolyl, pyridyl, imidazolyl),
(x) a nonaromatic heterocyclic group (e.g., piperidinyl, pyrrolidinyl), and
(xi) a tri$C_{1-6}$ alkylsilyloxy group (e.g., tert-butyldimethylsilyloxy);

(2) an oxo group;
(3) a cyano group;
(4) a $C_{1-6}$ alkoxy group;
(5) a $C_{3-10}$ cycloalkyl group (e.g., cyclopropyl, cyclobutyl); and the like.

Of the above-mentioned substituents,

(1) a $C_{1-6}$ alkyl group optionally substituted with 1 to 3 substituents selected from

(i) a carboxyl group,
(ii) a hydroxy group,
(iii) a cyano group,
(iv) a $C_{1-6}$ alkoxy group,
(v) a $C_{1-6}$ alkoxy-carbonyl group,
(vi) a $C_{1-6}$ alkyl-carbonyloxy group (e.g., acetyloxy), and
(vii) an amino group optionally mono- or di-substituted with $C_{1-6}$ alkylsulfonyl group(s) (e.g., methylsulfonyl);

(2) an oxo group;
(3) a cyano group;
(4) a $C_{1-6}$ alkoxy group;
and the like are preferable.

As ring R,
a cycloalkane having 5 to 7 carbon atoms and optionally condensed with a benzene ring (preferably tetrahydronaphthalene (e.g., 1,2,3,4-tetrahydronaphthalene), indane, benzocycloheptane, cyclopentane), a 5- to 7-membered monocyclic non-aromatic heterocycle (preferably pyrrolidine, piperidine, tetrahydrofuran, imidazolidine, imidazoline, oxazolidine, oxazoline, thiazolidine, thiazoline) each of 32 which is optionally substituted with 1 to 3 (preferably 1 or 2) substituents selected from

(1) a $C_{1-6}$ alkyl group optionally substituted with 1 to 3 substituents selected from

(i) a carboxyl group,
(ii) a hydroxy group,
(iii) a cyano group,
(iv) a $C_{1-6}$ alkoxy group,
(v) a $C_{3-10}$ cycloalkyl group (e.g., cyclopropyl),
(vi) a $C_{1-6}$ alkoxy-carbonyl group,
(vii) a $C_{1-6}$ alkyl-carbonyloxy group (e.g., acetyloxy),
(viii) an amino group optionally mono- or di-substituted with $C_{1-6}$ alkylsulfonyl group(s) (e.g., methylsulfonyl),
(ix) an aromatic heterocyclic group (e.g., thiazolyl, oxazolyl, pyridyl, imidazolyl),
(x) a nonaromatic heterocyclic group (e.g., piperidinyl, pyrrolidinyl), and
(xi) a tri$C_{1-6}$ alkylsilyloxy group (e.g., tert-butyldimethylsilyloxy);

(2) an oxo group;
(3) a cyano group;
(4) a $C_{1-6}$ alkoxy group; and the like and the like are preferable.

As ring R,
pyrrolidine, piperidine, tetrahydrofuran, imidazolidine, imidazoline, oxazolidine, oxazoline, thiazolidine or thiazoline, each of which is optionally substituted with 1 to 3 (preferably 1 or 2) substituents selected from

(1) a $C_{1-6}$ alkyl group optionally substituted with 1 to 3 substituents selected from

(i) a carboxyl group,
(ii) a hydroxy group,
(iii) a cyano group,
(iv) a $C_{1-6}$ alkoxy group,
(v) a $C_{1-6}$ alkoxy-carbonyl group,
(vi) a $C_{1-6}$ alkyl-carbonyloxy group (e.g., acetyloxy), and
(vii) an amino group optionally mono- or di-substituted with $C_{1-6}$ alkylsulfonyl group(s) (e.g., methylsulfonyl);

(2) an oxo group;
(3) a cyano group;
(4) a $C_{1-6}$ alkoxy group; and the like

are more preferable.
When, in the formula (I), A is N, and ring Q is pyrrolidine, piperidine or azepane, each of which is optionally

further substituted, the 5- to 7-membered nonaromatic ring for ring R is preferably other than a 5- to 7-membered nitrogen-containing nonaromatic ring having, on N atom, a substituent: $-(CR^1R^2)m-R^3$ wherein $R^1$ and $R^2$ are each independently a hydrogen atom or a $C_{1-8}$ alkyl group, m is 0, 1, 2 or 3, $R^3$ is an aryl group, an aromatic heterocyclic group, a cycloalkyl group or a nonaromatic heterocyclic group, each of which is optionally further substituted.

Here, Examples of the "5- to 7-membered nitrogen-containing nonaromatic ring" include one containing at least one nitrogen atom as a ring-constituting atom, from those exemplified as the 5- to 7-membered nonaromatic ring for the aforementioned ring R.

Preferable examples of compound (I) include the following compounds.

[Compound A]

A compound wherein E is an optionally substituted aromatic hydrocarbon group (preferably phenyl, naphthyl, anthryl) or an optionally substituted aromatic heterocyclic group (preferably pyridyl, quinolyl, benzofuranyl));
D is a carbonyl group;
ring P is a 5- to 7-membered nitrogen-containing non-aromatic heterocycle (preferably 6-membered nitrogen-containing non-aromatic heterocycle, more preferably piperidine, piperazine);
A is N;
ring Q is a 5- to 7-membered monocyclic non-aromatic heterocycle (preferably pyrrolidine, piperidine, piperazine, morpholine, thiomorpholine) optionally substituted with 1 to 3 substituents selected from (1) a $C_{1-6}$ alkyl group, and (2) a $C_{1-6}$ alkyl-carbonyl group optionally substituted with 1 to 3 halogen atoms; and
ring R is a cycloalkane having 5 to 7 carbon atoms (preferably tetrahydronaphthalene, indane) or a 5- to 7-membered monocyclic non-aromatic heterocycle (preferably pyrrolidine, piperidine, tetrahydrofuran), each of which is optionally substituted with 1 to 3 substituents selected from (1) a $C_{1-6}$ alkyl group optionally substituted with 1 to 3 substituents selected from a carboxyl group, a cyano group, a $C_{1-6}$ alkoxy group, a $C_{1-6}$ alkoxy-carbonyl group, a $C_{3-10}$ cycloalkyl group, an aromatic heterocyclic group (preferably thiazolyl, oxazolyl, pyridyl) and a nonaromatic heterocyclic group (preferably piperidinyl, pyrrolidinyl), and (2) an oxo group and is optionally condensed with a benzene ring.

[Compound B]

A compound wherein E is
an aromatic hydrocarbon group (preferably phenyl, naphthyl, anthryl, fluorenyl), an aromatic heterocyclic group (preferably pyridyl, quinolyl, benzofuranyl, benzothiophenyl, thienyl, pyrazolyl, imidazolyl, pyrimidinyl, oxazolyl, isoxazolyl, thiazolyl, triazinyl, isoquinolyl, imidazopyridinyl, pyrazolopyridinyl, thiazolopyridinyl, pyridopyridinyl), or a nonaromatic heterocyclic group (preferably tetrahydrobenzothiophenyl, tetrahydrothienopyridinyl, dihydrothienopyranyl, dihydrothienothiopyranyl, xanthenyl), each of which is optionally substituted with 1 to 3 substituents selected from

(1) a halogen atom;
(2) a hydroxy group;
(3) an oxo group;
(4) a nitro group;
(5) a cyano group;
(6) a $C_{1-6}$ alkyl group optionally substituted with 1 to 3 substituents selected from

 (i) a halogen atom,
 (ii) a hydroxy group,
 (iii) a carboxyl group,
 (iv) a $C_{1-6}$ alkoxy-carbonyl group,
 (v) a $C_{1-6}$ alkyl-carbonyloxy group (e.g., acetyloxy),
 (vi) an amino group optionally mono- or di-substituted with substituent(s) selected from

  (a) a $C_{1-6}$ alkyl-carbonyl group,
  (b) a $C_{1-6}$ alkoxy-carbonyl group, and
  (c) a $C_{1-6}$ alkylsulfonyl group (e.g., methylsulfonyl), and

 (vii) a $C_{1-6}$ alkylthio group (e.g., methylthio, ethylthio) optionally substituted with 1 to 3 $C_{1-6}$ alkoxy-carbonyl groups;

(7) a $C_{2-6}$ alkenyl group (e.g., ethenyl) optionally substituted with 1 to 3 $C_{1-6}$ alkoxy-carbonyl groups;

(8) a $C_{1-6}$ alkoxy group optionally substituted with 1 to 3 substituents selected from

(i) a carboxyl group, and
(ii) a $C_{1-6}$ alkoxy-carbonyl group;

(9) a $C_{6-14}$ aryl group (e.g., phenyl, naphthyl) optionally substituted with 1 to 3 substituents selected from

(i) a halogen atom,
(ii) a hydroxy group,
(iii) a carboxyl group,
(iv) a cyano group,
(v) a $C_{1-6}$ alkyl group optionally substituted with 1 to 3 substituents selected from

(a) a halogen atom,
(b) a carboxyl group, and
(c) a $C_{1-6}$ alkoxy-carbonyl group,

(vi) a $C_{1-6}$ alkoxy group optionally substituted with 1 to 3 substituents selected from

(a) a carboxyl group, and
(b) a $C_{1-6}$ alkoxy-carbonyl group optionally substituted with 1 to 3 $C_{6-14}$ aryl groups (e.g., phenyl),

(vii) a $C_{1-6}$ alkyl-carbonyl group,
(viii) a $C_{1-6}$ alkoxy-carbonyl group optionally substituted with 1 to 3 $C_{6-14}$ aryl groups (e.g., phenyl),
(ix) an amino group optionally mono- or di-substituted with substituent(s) selected from

(a) a $C_{1-6}$ alkyl-carbonyl group, and
(b) a $C_{1-6}$ alkylsulfonyl group (e.g., methylsulfonyl),

(x) a carbamoyl group, and
(xi) a $C_{1-6}$ alkylthio group (e.g., methylthio);

(10) a $C_{3-10}$ cycloalkyl group (e.g., cyclopropyl);
(11) a $C_{7-13}$ aralkyl group (e.g., benzyl);
(12) a $C_{6-14}$ aryloxy group (e.g., phenoxy);
(13) a $C_{7-13}$ aralkyloxy group (e.g., benzyloxy);
(14) a $C_{1-6}$ alkyl-carbonyl group;
(15) a $C_{1-6}$ alkoxy-carbonyl group;
(16) an aromatic heterocyclic group (e.g., pyridyl, pyrrolyl, imidazolyl, pyrazolyl, triazolyl, furyl, thienyl, benzimidazolyl, indazolyl, benzotriazolyl, benzothiophenyl, pyrrolopyridinyl) optionally substituted with 1 to 3 substituents selected from

(i) a $C_{1-6}$ alkyl group,
(ii) a $C_{1-6}$ alkoxy group, and
(iii) a nonaromatic heterocyclic group (e.g., morpholinyl, pyrrolidinyl);

(17) a nonaromatic heterocyclic group (e.g., piperidinyl, morpholinyl, isoindolinyl, pyrrolidinyl) optionally substituted with 1 to 3 substituents selected from

(i) a hydroxy group,
(ii) a carboxyl group,
(iii) an oxo group, and
(iv) a $C_{1-6}$ alkoxy-carbonyl group optionally substituted with 1 to 3 $C_{6-14}$ aryl groups (e.g., phenyl);

(18) an amino group optionally mono- or di-substituted with substituent(s) selected from

(i) a $C_{1-6}$ alkyl-carbonyl group optionally substituted with 1 to 3 substituents selected from

(a) a $C_{1-6}$ alkoxy-carbonyl group,
(b) a $C_{1-6}$ alkoxy group, and
(c) a $C_{6-14}$ aryl group (e.g., phenyl),

(ii) a $C_{1-6}$ alkoxy-carbonyl group optionally substituted with 1 to 3 substituents selected from

(a) a $C_{6-14}$ aryl group (e.g., phenyl), and
(b) a $C_{1-6}$ alkoxy group,

(iii) an aromatic heterocyclylcarbonyl group (e.g., thenoyl)
(iv) a carbamoyl group optionally mono- or di-substituted with substituent(s) selected from

(a) a $C_{1-6}$ alkyl group optionally substituted with 1 to 3 substituents selected from

(a-1) a $C_{1-6}$ alkoxy-carbonyl group, and
(a-2) a $C_{6-14}$ aryl group (e.g., phenyl),

(b) a $C_{3-10}$ cycloalkyl group (e.g., cyclohexyl),
(c) a $C_{6-14}$ aryl group (e.g., phenyl) optionally substituted with 1 to 3 substituents selected from

(c-1) a halogen atom,
(c-2) a $C_{1-6}$ alkyl group optionally substituted with 1 to 3 halogen atoms, and
(c-3) a $C_{1-6}$ alkoxy group, and

(d) an aromatic heterocyclic group (e.g., pyridyl),

(v) a $C_{1-6}$ alkylsulfonyl group (e.g., methylsulfonyl, ethylsulfonyl) optionally substituted with 1 to 3 $C_{6-14}$ aryl groups (e.g., phenyl),
(vi) a $C_{6-14}$ arylsulfonyl group (e.g., benzenesulfonyl) optionally substituted with 1 to 3 halogen atoms, and
(vii) a $C_{1-6}$ alkyl group optionally substituted with 1 to 3 substituents selected from

(a) an amino group optionally mono- or di-substituted with $C_{1-6}$ alkoxy-carbonyl group(s), and
(b) an imino group;

(19) a carbamoyloxy group optionally mono- or di-substituted with $C_{1-6}$ alkyl group(s); and
(20) a sulfamoyl group optionally mono- or di-substituted with $C_{1-6}$ alkyl group(s) optionally substituted with nonaromatic heterocyclic group(s) (e.g., pyrrolidinyl) optionally substituted with oxo group(s);

D is a carbonyl group;
ring P is a 5- to 7-membered nitrogen-containing non-aromatic heterocycle (preferably 6-membered nitrogen-containing non-aromatic heterocycle, more preferably piperidine, piperazine);
A is N;
ring Q is
a 5- to 7-membered monocyclic non-aromatic heterocycle (preferably pyrrolidine, piperidine, piperazine, morpholine, thiomorpholine, more preferably 6-membered monocyclic non-aromatic heterocycle (preferably piperidine, piperazine, morpholine, thiomorpholine)) optionally substituted with 1 to 3 (preferably 1 or 2) substituents selected from

(1) a $C_{1-6}$ alkyl-carbonyl group optionally substituted with 1 to 3 halogen atoms;
(2) a $C_{1-6}$ alkyl group optionally substituted with 1 to 3 substituents selected from a carboxyl group and a $C_{1-6}$ alkoxy-carbonyl group; and
(3) a $C_{1-6}$ alkoxy-carbonyl group optionally substituted with 1 to 3 $C_{6-14}$ aryl groups (e.g., phenyl); and
ring R is

a cycloalkane having 5 to 7 carbon atoms and optionally condensed with a benzene ring (preferably tetrahydronaphthalene (e.g., 1,2,3,4-tetrahydronaphthalene), indane, benzocycloheptane, cyclopentane), or a 5- to 7-membered monocyclic non-aromatic heterocycle (preferably pyrrolidine, piperidine, tetrahydrofuran, imidazolidine, imidazoline, oxazolidine), each of which is optionally substituted with 1 to 3 (preferably 1 or 2) substituents selected from

(1) a $C_{1-6}$ alkyl group optionally substituted with 1 to 3 substituents selected from

(i) a carboxyl group,
(ii) a hydroxy group,
(iii) a cyano group,
(iv) a $C_{1-6}$ alkoxy group,
(v) a $C_{3-10}$ cycloalkyl group,
(vi) a $C_{1-6}$ alkoxy-carbonyl group,
(vii) a $C_{1-6}$ alkyl-carbonyloxy group (e.g., acetyloxy),
(viii) an amino group optionally mono- or di-substituted with $C_{1-6}$ alkylsulfonyl group(s) (e.g., methylsulfonyl),
(ix) an aromatic heterocyclic group (e.g., thiazolyl, oxazolyl, pyridyl, imidazolyl),
(x) a nonaromatic heterocyclic group (e.g., piperidinyl, pyrrolidinyl), and
(xi) a tri-$C_{1-6}$ alkylsilyloxy group (e.g., tert-butyldimethylsilyloxy);

(2) an oxo group; and
(3) a cyano group.

[Compound C]

The aforementioned [compound B] wherein E is an aromatic hydrocarbon group (preferably anthryl).

[Compound D]

The aforementioned [compound B] wherein E is an aromatic heterocyclic group (preferably pyridyl, quinolyl, thienyl, benzothiophenyl) optionally substituted with 1 to 3 substituents selected from

(1) a $C_{1-6}$ alkyl group optionally substituted with 1 to 3 substituents selected from

(i) a halogen atom,
(ii) a hydroxy group,
(iii) a carboxyl group,
(iv) a $C_{1-6}$ alkoxy-carbonyl group,
(v) a $C_{1-6}$ alkyl-carbonyloxy group (e.g., acetyloxy),
(vi) an amino group optionally mono- or di-substituted with substituent(s) selected from

(a) a $C_{1-6}$ alkyl-carbonyl group,
(b) a $C_{1-6}$ alkoxy-carbonyl group, and
(c) a $C_{1-6}$ alkylsulfonyl group (e.g., methylsulfonyl), and

(vii) a $C_{1-6}$ alkylthio group (e.g., methylthio, ethylthio) optionally substituted with 1 to 3 $C_{1-6}$ alkoxy-carbonyl groups;

(2) a $C_{1-6}$ alkoxy group optionally substituted with 1 to 3 substituents selected from

(i) a carboxyl group, and
(ii) a $C_{1-6}$ alkoxy-carbonyl group;

(3) a $C_{6-14}$ aryl group (e.g., phenyl, naphthyl) optionally substituted with 1 to 3 substituents selected from

(i) a halogen atom,
(ii) a hydroxy group,
(iii) a carboxyl group,
(iv) a cyano group,
(v) a $C_{1-6}$ alkyl group optionally substituted with 1 to 3 substituents selected from

(a) a halogen atom,
(b) a carboxyl group, and

(c) a $C_{1-6}$ alkoxy-carbonyl group,

(vi) a $C_{1-6}$ alkoxy group optionally substituted with 1 to 3 substituents selected from

(a) a carboxyl group, and
(b) a $C_{1-6}$ alkoxy-carbonyl group optionally substituted with 1 to 3 $C_{6-14}$ aryl groups (e.g., phenyl),

(vii) a $C_{1-6}$ alkyl-carbonyl group,
(viii) a $C_{1-6}$ alkoxy-carbonyl group optionally substituted with 1 to 3 $C_{6-14}$ aryl groups (e.g., phenyl),
(ix) an amino group optionally mono- or di-substituted with substituent(s) selected from

(a) a $C_{1-6}$ alkyl-carbonyl group, and
(b) a $C_{1-6}$ alkylsulfonyl group (e.g., methylsulfonyl),

(x) a carbamoyl group, and
(xi) a $C_{1-6}$ alkylthio group (e.g., methylthio);

(4) an aromatic heterocyclic group (e.g., pyridyl, pyrrolyl, imidazolyl, pyrazolyl, triazolyl, furyl, thienyl, benzimidazolyl, indazolyl, benzotriazolyl, benzothiophenyl, pyrrolopyridinyl) optionally substituted with 1 to 3 substituents selected from

(i) a $C_{1-6}$ alkyl group,
(ii) a $C_{1-6}$ alkoxy group, and
(iii) a nonaromatic heterocyclic group (e.g., morpholinyl, pyrrolidinyl); and

(5) an amino group optionally mono- or di-substituted with carbamoyl group(s) optionally mono- or di-substituted with substituent(s) selected from

(a) a $C_{1-6}$ alkyl group optionally substituted with 1 to 3 substituents selected from

(a-1) a $C_{1-6}$ alkoxy-carbonyl group, and
(a-2) a $C_{6-14}$ aryl group (e.g., phenyl),

(b) a $C_{3-10}$ cycloalkyl group (e.g., cyclohexyl),
(c) a $C_{6-14}$ aryl group (e.g., phenyl) optionally substituted with 1 to 3 substituents selected from

(c-1) a halogen atom,
(c-2) a $C_{1-6}$ alkyl group optionally substituted with 1 to 3 halogen atoms, and
(c-3) a $C_{1-6}$ alkoxy group, and

(d) an aromatic heterocyclic group (e.g., pyridyl).

[Compound AA]

A compound wherein E is an aromatic hydrocarbon group (preferably phenyl, naphthyl, anthryl, fluorenyl), an aromatic heterocyclic group (preferably pyridyl, quinolyl, benzofuranyl, benzothiophenyl, thienyl, pyrazolyl, imidazolyl, pyrimidinyl, oxazolyl, isoxazolyl, thiazolyl, triazinyl, isoquinolyl, imidazopyridinyl, pyrazolopyridinyl, thiazolopyridinyl, pyridopyridinyl), or a nonaromatic heterocyclic group (preferably tetrahydrobenzothiophenyl, tetrahydrothienopyridinyl, dihydrothienopyranyl, dihydrothienothiopyranyl, xanthenyl), each of which is optionally substituted with 1 to 3 substituents selected from

(1) a halogen atom;
(2) a hydroxy group;
(3) an oxo group;
(4) a nitro group;
(5) a cyano group;
(6) a $C_{1-6}$ alkyl group optionally substituted with 1 to 3 substituents selected from

(i) a halogen atom,
(ii) a hydroxy group,
(iii) a carboxyl group,
(iv) a $C_{1-6}$ alkoxy-carbonyl group,
(v) a $C_{1-6}$ alkyl-carbonyloxy group (e.g., acetyloxy),
(vi) an amino group optionally mono- or di-substituted with substituent(s) selected from

(a) a $C_{1-6}$ alkyl-carbonyl group,
(b) a $C_{1-6}$ alkoxy-carbonyl group, and
(c) a $C_{1-6}$ alkylsulfonyl group (e.g., methylsulfonyl), and

(vii) a $C_{1-6}$ alkylthio group (e.g., methylthio, ethylthio) optionally substituted with 1 to 3 $C_{1-6}$ alkoxy-carbonyl groups;

(7) a $C_{2-6}$ alkenyl group (e.g., ethenyl) optionally substituted with 1 to 3 $C_{1-6}$ alkoxy-carbonyl groups;
(8) a $C_{1-6}$ alkoxy group optionally substituted with 1 to 3 substituents selected from

(i) a carboxyl group, and
(ii) a $C_{1-6}$ alkoxy-carbonyl group;

(9) a $C_{6-14}$ aryl group (e.g., phenyl, naphthyl) optionally substituted with 1 to 3 substituents selected from

(i) a halogen atom,
(ii) a hydroxy group,
(iii) a carboxyl group,
(iv) a cyano group,
(v) a $C_{1-6}$ alkyl group optionally substituted with 1 to 3 substituents selected from

(a) a halogen atom,
(b) a carboxyl group, and
(c) a $C_{1-6}$ alkoxy-carbonyl group,

(vi) a $C_{1-6}$ alkoxy group optionally substituted with 1 to 3 substituents selected from

(a) a carboxyl group, and
(b) a $C_{1-6}$ alkoxy-carbonyl group optionally substituted with 1 to 3 $C_{6-14}$ aryl groups (e.g., phenyl),

(vii) a $C_{1-6}$ alkyl-carbonyl group,
(viii) a $C_{1-6}$ alkoxy-carbonyl group optionally substituted with 1 to 3 $C_{6-14}$ aryl groups (e.g., phenyl),
(ix) an amino group optionally mono- or di-substituted with substituent(s) selected from

(a) a $C_{1-6}$ alkyl-carbonyl group, and
(b) a $C_{1-6}$ alkylsulfonyl group (e.g., methylsulfonyl),

(x) a carbamoyl group, and
(xi) a $C_{1-6}$ alkylthio group (e.g., methylthio);

(10) a $C_{3-10}$ cycloalkyl group (e.g., cyclopropyl);
(11) a $C_{7-13}$ aralkyl group (e.g., benzyl);
(12) a $C_{6-14}$ aryloxy group (e.g., phenoxy);
(13) a $C_{7-13}$ aralkyloxy group (e.g., benzyloxy);
(14) a $C_{1-6}$ alkyl-carbonyl group;
(15) a $C_{1-6}$ alkoxy-carbonyl group;
(16) an aromatic heterocyclic group (e.g., pyridyl, pyrrolyl, imidazolyl, pyrazolyl, triazolyl, furyl, thienyl, benzimidazolyl, indazolyl, benzotriazolyl, benzothiophenyl, pyrrolopyridinyl) optionally substituted with 1 to 3 substituents selected from

(i) a $C_{1-6}$ alkyl group,

(ii) a $C_{1-6}$ alkoxy group, and
(iii) a nonaromatic heterocyclic group (e.g., morpholinyl, pyrrolidinyl);

(17) a nonaromatic heterocyclic group (e.g., piperidinyl, morpholinyl, isoindolinyl, pyrrolidinyl) optionally substituted with 1 to 3 substituents selected from

(i) a hydroxy group,
(ii) a carboxyl group,
(iii) an oxo group,
(iv) a $C_{1-6}$ alkoxy-carbonyl group optionally substituted with 1 to 3 $C_{6-14}$ aryl groups (e.g., phenyl),
(v) a $C_{1-6}$ alkoxy group, and
(vi) a $C_{1-3}$ alkylenedioxy group;

(18) an amino group optionally mono- or di-substituted with substituent(s) selected from

(i) a $C_{1-6}$ alkyl-carbonyl group optionally substituted with 1 to 3 substituents selected from

(a) a $C_{1-6}$ alkoxy-carbonyl group,
(b) a $C_{1-6}$ alkoxy group, and
(c) a $C_{6-14}$ aryl group (e.g., phenyl),

(ii) a $C_{1-6}$ alkoxy-carbonyl group optionally substituted with 1 to 3 substituents selected from

(a) a $C_{6-14}$ aryl group (e.g., phenyl), and
(b) a $C_{1-6}$ alkoxy group,

(iii) an aromatic heterocyclylcarbonyl group (e.g., thenoyl)
(iv) a carbamoyl group optionally mono- or di-substituted with substituent(s) selected from

(a) a $C_{1-6}$ alkyl group optionally substituted with 1 to 3 substituents selected from

(a-1) a $C_{1-6}$ alkoxy-carbonyl group,
(a-2) a $C_{6-14}$ aryl group (e.g., phenyl), and
(a-3) a $C_{1-6}$ alkoxy group,

(b) a $C_{3-10}$ cycloalkyl group (e.g., cyclohexyl),
(c) a $C_{6-14}$ aryl group (e.g., phenyl) optionally substituted with 1 to 3 substituents selected from

(c-1) a halogen atom,
(c-2) a $C_{1-6}$ alkyl group optionally substituted with 1 to 3 halogen atoms, and
(c-3) a $C_{1-6}$ alkoxy group, and

(d) an aromatic heterocyclic group (e.g., pyridyl),

(v) a $C_{1-6}$ alkylsulfonyl group (e.g., methylsulfonyl, ethylsulfonyl) optionally substituted with 1 to 3 $C_{6-14}$ aryl groups (e.g., phenyl),
(vi) a $C_{6-14}$ arylsulfonyl group (e.g., benzenesulfonyl) optionally substituted with 1 to 3 halogen atoms,
(vii) a $C_{1-6}$ alkyl group optionally substituted with 1 to 3 substituents selected from

(a) an amino group optionally mono- or di-substituted with $C_{1-6}$ alkoxy-carbonyl group(s), and
(b) an imino group, and

(viii) a thiocarbamoyl group optionally mono- or di-substituted with $C_{1-6}$ alkyl group(s);

(19) a carbamoyloxy group optionally mono- or di-substituted with $C_{1-6}$ alkyl group(s); and
(20) a sulfamoyl group optionally mono- or di-substituted with $C_{1-6}$ alkyl group(s) optionally substituted with nonaromatic heterocyclic group(s) (e.g., pyrrolidinyl) optionally substituted with oxo group(s);

D is a carbonyl group or a sulfonyl group;
ring P is a 5- to 7-membered nitrogen-containing non-aromatic heterocycle (preferably 6-membered nitrogen-containing non-aromatic heterocycle, more preferably piperidine, piperazine);
A is N;
ring Q is
a 5- to 7-membered monocyclic non-aromatic heterocycle (preferably pyrrolidine, piperidine, piperazine, morpholine, thiomorpholine, more preferably 6-membered monocyclic non-aromatic heterocycle (preferably piperidine, piperazine, morpholine, thiomorpholine)) optionally substituted with 1 to 3 (preferably 1 or 2) substituents selected from

(1) a $C_{1-6}$ alkyl-carbonyl group optionally substituted with 1 to 3 halogen atoms;
(2) a $C_{1-6}$ alkyl group optionally substituted with 1 to 3 substituents selected from a carboxyl group and a $C_{1-6}$ alkoxy-carbonyl group; and
(3) a $C_{1-6}$ alkoxy-carbonyl group optionally substituted with 1 to 3 $C_{6-14}$ aryl groups (e.g., phenyl); and
ring R is a cycloalkane having 5 to 7 carbon atoms and optionally condensed with a benzene ring (preferably tetrahydronaphthalene (e.g., 1,2,3,4-tetrahydronaphthalene), indane, benzocycloheptane, cyclopentane), or a 5- to 7-membered monocyclic non-aromatic heterocycle (preferably pyrrolidine, piperidine, tetrahydrofuran, imidazolidine, imidazoline, oxazolidine, oxazoline, thiazolidine, thiazoline), each of which is optionally substituted with 1 to 3 (preferably 1 or 2) substituents selected from

(1) a $C_{1-6}$ alkyl group optionally substituted with 1 to 3 substituents selected from

(i) a carboxyl group,
(ii) a hydroxy group,
(iii) a cyano group,
(iv) a $C_{1-6}$ alkoxy group,
(v) a $C_{3-10}$ cycloalkyl group (e.g., cyclopropyl),
(vi) a $C_{1-6}$ alkoxy-carbonyl group,
(vii) a $C_{1-6}$ alkyl-carbonyloxy group (e.g., acetyloxy),
(viii) an amino group optionally mono- or di-substituted with $C_{1-6}$ alkylsulfonyl group(s) (e.g., methylsulfonyl),
(ix) an aromatic heterocyclic group (e.g., thiazolyl, oxazolyl, pyridyl, imidazolyl),
(x) a nonaromatic heterocyclic group (e.g., piperidinyl, pyrrolidinyl), and
(xi) a tri$C_{1-6}$ alkylsilyloxy group (e.g., tert-butyldimethylsilyloxy);

(2) an oxo group;
(3) a cyano group;
(4) a $C_{1-6}$ alkoxy group; and
(5) a $C_{3-10}$ cycloalkyl group (e.g., cyclopropyl, cyclobutyl).

[Compound AB]

The aforementioned [Compound AA] wherein ring R is a cycloalkane having 5 to 7 carbon atoms and optionally condensed with a benzene ring (preferably tetrahydronaphthalene (e.g., 1,2,3,4-tetrahydronaphthalene), indane, benzocycloheptane, cyclopentane), or a 5- to 7-membered monocyclic non-aromatic heterocycle (preferably pyrrolidine, piperidine, tetrahydrofuran, imidazolidine, imidazoline, oxazolidine, oxazoline, thiazolidine, thiazoline), each of which is optionally substituted with 1 to 3 (preferably 1 or 2) substituents selected from

(1) a $C_{1-6}$ alkyl group optionally substituted with 1 to 3 substituents selected from

(i) a carboxyl group,
(ii) a hydroxy group,
(iii) a cyano group,
(iv) a $C_{1-6}$ alkoxy group,
(v) a $C_{3-10}$ cycloalkyl group (e.g., cyclopropyl),
(vi) a $C_{1-6}$ alkoxy-carbonyl group,
(vii) a $C_{1-6}$ alkyl-carbonyloxy group (e.g., acetyloxy),
(viii) an amino group optionally mono- or di-substituted with $C_{1-6}$ alkylsulfonyl group(s) (e.g., methylsulfonyl),
(ix) an aromatic heterocyclic group (e.g., thiazolyl, oxazolyl, pyridyl, imidazolyl),
(x) a nonaromatic heterocyclic group (e.g., piperidinyl, pyrrolidinyl), and

(xi) a tri-$C_{1-6}$ alkylsilyloxy group (e.g., tert-butyldimethylsilyloxy);

(2) an oxo group;
(3) a cyano group; and
(4) a $C_{1-6}$ alkoxy group.

[Compound AC]

The aforementioned [Compound AA] wherein E is an aromatic hydrocarbon group (preferably anthryl).

[Compound AD]

The aforementioned [Compound AA] wherein E is an aromatic heterocyclic group (preferably pyridyl, quinolyl, thienyl, benzothiophenyl) optionally substituted with 1 to 3 substituents selected from

(1) a $C_{1-6}$ alkyl group optionally substituted with 1 to 3 substituents selected from

(i) a halogen atom,
(ii) a hydroxy group,
(iii) a carboxyl group,
(iv) a $C_{1-6}$ alkoxy-carbonyl group,
(v) a $C_{1-6}$ alkyl-carbonyloxy group (e.g., acetyloxy),
(vi) an amino group optionally mono- or di-substituted with substituent(s) selected from

(a) a $C_{1-6}$ alkyl-carbonyl group,
(b) a $C_{1-6}$ alkoxy-carbonyl group, and
(c) a $C_{1-6}$ alkylsulfonyl group (e.g., methylsulfonyl), and

(vii) a $C_{1-6}$ alkylthio group (e.g., methylthio, ethylthio) optionally substituted with 1 to 3 $C_{1-6}$ alkoxy-carbonyl group;

(2) a $C_{1-6}$ alkoxy group optionally substituted with 1 to 3 substituents selected from

(i) a carboxyl group, and
(ii) a $C_{1-6}$ alkoxy-carbonyl group;

(3) a $C_{6-14}$ aryl group (e.g., phenyl, naphthyl) optionally substituted with 1 to 3 substituents selected from

(i) a halogen atom,
(ii) a hydroxy group,
(iii) a carboxyl group,
(iv) a cyano group,
(v) a $C_{1-6}$ alkyl group optionally substituted with 1 to 3 substituents selected from

(a) a halogen atom,
(b) a carboxyl group, and
(c) a $C_{1-6}$ alkoxy-carbonyl group,

(vi) a $C_{1-6}$ alkoxy group optionally substituted with 1 to 3 substituents selected from

(a) a carboxyl group, and
(b) a $C_{1-6}$ alkoxy-carbonyl group optionally substituted with 1 to 3 $C_{6-14}$ aryl groups (e.g., phenyl),

(vii) a $C_{1-6}$ alkyl-carbonyl group,
(viii) a $C_{1-6}$ alkoxy-carbonyl group optionally substituted with 1 to 3 $C_{6-14}$ aryl groups (e.g., phenyl),
(ix) an amino group optionally mono- or di-substituted with substituent(s) selected from

(a) a $C_{1-6}$ alkyl-carbonyl group, and
(b) a $C_{1-6}$ alkylsulfonyl group (e.g., methylsulfonyl),

(x) a carbamoyl group, and
(xi) a $C_{1-6}$ alkylthio group (e.g., methylthio);

(4) an aromatic heterocyclic group (e.g., pyridyl, pyrrolyl, imidazolyl, pyrazolyl, triazolyl, furyl, thienyl, benzimidazolyl, indazolyl, benzotriazolyl, benzothiophenyl, pyrrolopyridinyl) optionally substituted with 1 to 3 substituents selected from

(i) a $C_{1-6}$ alkyl group,
(ii) a $C_{1-6}$ alkoxy group, and
(iii) a nonaromatic heterocyclic group (e.g., morpholinyl, pyrrolidinyl); and

(5) an amino group optionally mono- or di-substituted with carbamoyl group(s) optionally mono- or di-substituted with substituent(s) selected from

(a) a $C_{1-6}$ alkyl group optionally substituted with 1 to 3 substituents selected from

(a-1) a $C_{1-6}$ alkoxy-carbonyl group,
(a-2) a $C_{6-14}$ aryl group (e.g., phenyl), and
(a-3) a $C_{1-6}$ alkoxy group,

(b) a $C_{3-10}$ cycloalkyl group (e.g., cyclohexyl),
(c) a $C_{6-14}$ aryl group (e.g., phenyl) optionally substituted with 1 to 3 substituents selected from

(c-1) a halogen atom,
(c-2) a $C_{1-6}$ alkyl group optionally substituted with 1 to 3 halogen atoms, and
(c-3) a $C_{1-6}$ alkoxy group, and

(d) an aromatic heterocyclic group (e.g., pyridyl).

[Compound AE]

N-(3-{[4-(3,3-Dimethyl-1-oxo-2-oxa-7-azaspiro[4.5]dec-7-yl)piperidin-1-yl]carbonyl}-1-benzothien-2-yl)-N'-ethylurea (Examples 36, 318, 319), 3,3-dimethyl-7-{1-[(2-(pyridin-3-yl)quinolin-4-yl)carbonyl]piperidin-4-yl}-2-oxa-7-azaspiro[4.5]decan-1-one (Example 92), 7-{1-[(3'-acetyl-5-(pyridin-3-yl)biphenyl-3-yl)carbonyl]piperidin-4-yl}-3,3-dimethyl-2-oxa-7-azaspiro[4.5]decan-1-one (Example 133), 9-[1-(9-anthrylcarbonyl)piperidin-4-yl]-3,3-dimethyl-oxa-6,9-diazaspiro[4.5]decan-1-one (Example 153), 1-(3-{[4-(3,3-dimethyl-1-oxo-2-oxa-7-azaspiro[4.5]dec-7-yl)piperidin-1-yl]carbonyl}-5-(pyridin-2-yl)-2-thienyl)-3-ethylurea (Example 179), 4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)benzoic acid (Example 190), N-ethyl-N'-(3-{[4-(3-ethyl-2,4-dioxo-1-oxa-3,7-diazaspiro[4.5]dec-7-yl)piperidin-1-yl]carbonyl}-1-benzothien-2-yl)urea (Examples 228, 327), 1-(3-{[4-(3-ethyl-2,4-dioxo-1-oxa-3,7-diazaspiro[4.5]dec-7-yl)piperidin-1-yl]carbonyl}-1-benzothien-2-yl)urea (Example 229), 1-ethyl-3-(3-{[4-(3-ethyl-2-methyl-4-oxo-1,3,7-triazaspiro[4.5]dec-1-en-7-yl)piperidin-1-yl]carbonyl}-1-benzothien-2-yl) urea (Examples 233, 401), 1-(3-{[4-(2-ethyl-1,3-dioxo-2,7-diazaspiro[4.5]dec-7-yl)piperidin-1-yl]carbonyl}-1-benzothien-2-yl)urea (Examples 240, 340), 1-ethyl-3-(3-{[4-(3-ethyl-2,4-dioxo-1-oxa-3,7-diazaspiro[4.5]dec-7-yl)piperidin-1-yl]carbonyl}-5-(pyridin-2-yl)-2-thienyl)urea (Example 245), N-(4-{[4-(3,3-dimethyl-1-oxo-2-oxa-7-azaspiro[4.5]dec-7-yl)piperidin-1-yl]carbonyl}-6-phenylpyridin-3-yl)-N'-ethylurea (Example 301), N-(3-{[4-(1,3-dioxo-2,7-diazaspiro[4.5]dec-7-yl) piperidin-1-yl]carbonyl}-1-benzothien-2-yl)-N'-ethylurea (Example 311), N-ethyl-N'-(3-{[4-(2-ethyl-1-oxo-2,7-diazaspiro [4.5]dec-7-yl)piperidin-1-yl]carbonyl}-1-benzothien-2-yl)urea (Examples 321, 400), N-(3-{[4-(3-isopropyl-2,4-dioxo-1-oxa-3,7-diazaspiro[4.5]dec-7-yl)piperidin-1-yl]carbonyl}-1-benzothien-2-yl)urea (Example 335), N-(3-{[4-(2-ethyl-1-oxo-2,7-diazaspiro[4.5]dec-7-yl)piperidin-1-yl]carbonyl}-1-benzothien-2-yl)-N'-methylurea (Example 339), N-(3-{[4-(2,4-dioxo-3-propyl-1-oxa-3,7-diazaspiro[4.5]dec-7-yl)piperidin-1-yl]carbonyl}-1-benzothien-2-yl)urea (Example 343), N-(3-{[4-(3-ethyl-2,4-dioxo-1-oxa-3,7-diazaspiro[4.5]dec-7-yl)piperidin-1-yl]carbonyl}-1-benzothien-2-yl)-N'-methylurea (Example 347), N-(3-{[4-(1-oxo-2-propyl-2,7-diazaspiro[4.5]dec-7-yl)piperidin-1-yl]carbonyl}-1-benzothien-2-yl)urea (Example 352), N-(3-{[4-(2-isopropyl-1-oxo-2,7-diazaspiro[4.5]dec-7-yl)piperidin-1-yl]carbonyl}-1-benzothien-2-yl)-N'-methylurea (Example 360), N-(3-{[4-(2-ethyl-1,3-dioxo-2,7-diazaspiro[4.5]dec-7-yl)piperidin-1-yl]carbonyl}-1-benzothien-2-yl)-N'-methylurea (Example 363), N-(3-{[4-(2-isopropyl-1,3-dioxo-2,7-diazaspiro[4.5]dec-7-yl)piperidin-1-yl]carbonyl}-1-benzothien-2-yl)urea (Example 366), N-ethyl-N'-(3-{[4-(2-methyl-1,3-dioxo-2,7-diazaspiro[4.5]dec-7-yl)piperidin-1-yl]carbonyl}-1-benzothien-2-yl)urea (Example 380), N-(3-{[4-(1,3-dioxo-2,7-diazaspiro[4.5]dec-7-yl)piperidin-1-yl] carbonyl}-1-benzothien-2-yl)-N'-isopropylurea (Example 397), and an optically active form thereof and a salt thereof.

As a salt of compound (I), a pharmacologically acceptable salt is preferable. Examples of such salt include salts with inorganic base, salts with organic base, salts with inorganic acid, salts with organic acid, salts with basic or acidic amino acid and the like.

Preferable examples of salts with inorganic base include alkali metal salt such as sodium salt, potassium salt and the like; alkaline earth metal salt such as calcium salt, magnesium salt and the like; and aluminum salt: ammonium salt and the like.

Preferable examples of salts with inorganic base include salts with trimethylamine, triethylamine, pyridine, picoline, ethanolamine, diethanolamine, triethanolamine, tromethamine[tris(hydroxymethyl)methylamine], tert-butylamine, cyclohexylamine, benzylamine, dicyclohexylamine, N,N-dibenzylethylenediamine and the like.

Preferable examples of salts with inorganic acid include salts with hydrochloric acid, hydrobromic acid, nitric acid, sulfuric acid, phosphoric acid and the like.

Preferable examples of salts with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of salts with basic amino acid include salts with arginine, lysine, ornithine and the like.

Preferable examples of salts with acidic amino acid include salts with aspartic acid, glutamic acid and the like.

A prodrug of the compound (I) means a compound which is converted to the compound (I) with a reaction due to an enzyme, an gastric acid, etc. under the physiological condition in the living body, that is, a compound which is converted to the compound (I) with oxidation, reduction, hydrolysis, etc. according to an enzyme; a compound which is converted to the compound (I) by hydrolysis etc. due to gastric acid, etc.

A prodrug of compound (I) may be a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation, etc.); a compound obtained by subjecting a hydroxy group in compound (I) to an acylation, alkylation, phosphorylation or boration (e.g., a compound obtained by subjecting a hydroxy group in compound (I) to an acetylation, palmitoylation, propanoylation, pivaloylation, succinylation, fumarylation, alanylation, dimethylaminomethylcarbonylation); a compound obtained by subjecting a carboxyl group in compound (I) to an esterification or amidation (e.g., a compound obtained by subjecting a carboxyl group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methyl amidation) and the like. Any of these compounds can be produced from compound (I) by a method known per *se*.

A prodrug for compound (I) may also be one which is converted into compound (I) under a physiological condition, such as those described in IYAKUHIN no KAIHATSU, Development of Pharmaceuticals, Vol. 7, Design of Molecules, p. 163-198, Published by HIROKAWA SHOTEN, 1990.

In addition, compound (I) may be labeled with an isotope (e.g., $^{3}H$, $^{14}C$, $^{35}S$, $^{125}I$) and the like.

Moreover, compound (I) may be an anhydride or a hydrate.

Compound (I) or a prodrug thereof (hereinafter sometimes to be abbreviated simply as the compound of the present invention) has low toxicity, and can be used as an agent for the prophylaxis or treatment of various diseases mentioned below in a mammal (e.g., human, mouse, rat, rabbit, dog, cat, bovine, horse, swine, monkey) directly or in the form of a pharmaceutical composition by admixing with a pharmacologically acceptable carrier and the like.

Here, examples of the pharmacologically acceptable carrier include various organic or inorganic carrier substances conventionally used as preparation materials, which are added as excipient, lubricant, binder or disintegrant for solid dosage forms; as solvent, dissolution aids, suspending agent, isotonicity agent, buffer or soothing agent for liquid preparation, and the like. Where necessary, preparation additives such as preservative, antioxidant, colorant, sweetener and the like can also be used.

Preferable examples of the excipient include lactose, sucrose, D-mannitol, D-sorbitol, starch, pregelatinized starch, dextrin, crystalline cellulose, low-substituted hydroxypropylcellulose, sodium carboxymethylcellulose, gum arabic, pullulan, light anhydrous silicic acid, synthetic aluminum silicate, magnesium aluminometasilicate.

Preferable examples of the lubricant include magnesium stearate, calcium stearate, talc and colloidal silica.

Preferable examples of the binder include pregelatinized starch, sucrose, gelatin, gum arabic, methylcellulose, carboxymethylcellulose, sodium carboxymethylcellulose , crystalline cellulose, sucrose, D-mannitol, trehalose, dextrin, pullulan, hydroxypropylcellulose, hydroxypropylmethylcellulose and polyvinylpyrrolidone.

Preferable examples of the disintegrant include lactose, sucrose, starch, carboxymethylcellulose, calcium carboxymethylcellulose, croscarmellose sodium, carboxymethyl starch sodium, light anhydrous silicic acid and low-substituted hydroxypropylcellulose.

Preferable examples of the solvent include water for injection, physiological brine, Ringer's solution, alcohol, propylene glycol, polyethylene glycol, sesame oil, corn oil, olive oil and cottonseed oil.

Preferable examples of the dissolution aids include polyethylene glycol, propylene glycol, D-mannitol, trehalose, benzyl benzoate, ethanol, trisaminomethane, cholesterol, triethanolamine, sodium carbonate, sodium citrate, sodium salicylate and sodium acetate.

Preferable examples of the suspending agent include surfactants such as stearyltriethanolamine, sodium lauryl sulfate, lauryl aminopropionic acid, lecithin, benzalkonium chloride, benzethonium chloride, glyceryl monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates and polyoxyethylene hydrogenated castor oil.

Preferable examples of the isotonicity agent include sodium chloride, glycerol, D-mannitol, D-sorbitol and glucose.

Preferable examples of the buffer include buffers such as phosphate, acetate, carbonate, citrate and the like.

Preferable examples of the soothing agent include benzyl alcohol.

Preferable examples of the preservative include paraoxybenzoates, chlorobutanol, benzyl alcohol, phenethyl alcohol, dehydroacetic acid and sorbic acid.

Preferable examples of the antioxidant include sulfite, ascorbate and the like.

Preferable examples of the colorant include aqueous food tar colors (e.g., food colors such as Food Red No. 2 and No. 3, Food Yellow No. 4 and No. 5, Food Blue No. 1 and No. 2, etc.), water insoluble lake dye (e.g., aluminum salt of the aforementioned aqueous food tar color), natural dye (e.g., β-carotene, chlorophyll, red iron oxide).

Preferable examples of the sweetening agent include saccharin sodium, dipotassium glycyrrhizinate, aspartame, stevia.

Examples of the dosage form of the aforementioned pharmaceutical composition include oral preparations such as tablet (including sublingual tablet, orally disintegrating tablet), capsule (including soft capsule, microcapsule), granule, powder, troche, syrup, emulsion, suspension and the like; and parenteral agents such as injections (e.g., subcutaneous injection, intravenous injection, intramuscular injection, intraperitoneal injection, drip infusion), external preparations (e.g., dermal preparation, ointment), suppositories (e.g., rectal suppository, vaginal suppository), pellet, transnasal agent, pulmonary preparation (inhalant), eye drop and the like. Each of these can be safely administered orally or parenterally.

These preparations may be release control preparations (e.g., sustained-release microcapsule) such as immediate-release preparation, sustained-release preparation and the like.

A pharmaceutical composition can be produced by a method conventionally used in the technical field of pharmaceutical preparation, for example, the method described in the Japanese Pharmacopoeia and the like.

While the content of the compound of the present invention in the pharmaceutical composition varies depending on the dosage form, dose of the compound of the present invention, and the like, it is, for example, about 0.1 to 100 wt%.

During production of an oral preparation, coating may be applied as necessary for the purpose of masking of taste, enteric property or durability.

Examples of a coating base to be used for coating include sugar coating base, water-soluble film coating base, enteric film coating base and sustained-release film coating base.

As the sugar coating base, sucrose is used. Moreover, one or more kinds selected from talc, precipitated calcium carbonate, gelatin, gum arabic, pullulan, carnauba wax and the like may be used in combination.

Examples of the water-soluble film coating base include cellulose polymers such as hydroxypropyl cellulose, hydroxypropylmethyl cellulose, hydroxyethyl cellulose, methylhydroxyethyl cellulose etc.; synthetic polymers such as polyvinylacetal diethylaminoacetate, aminoalkyl methacrylate copolymer E [Eudragit E (trade name)], polyvinylpyrrolidone etc.; polysaccharides such as pullulan etc.

Examples of the enteric film coating base include cellulose polymers such as hydroxypropylmethyl cellulose phthalate, hydroxypropylmethyl cellulose acetate succinate, carboxymethylethyl cellulose, cellulose acetate phthalate etc.; acrylic polymers such as methacrylic acid copolymer L [Eudragit L (trade name)], methacrylic acid copolymer LD [Eudragit L-30D55 (trade name)], methacrylic acid copolymer S [Eudragit S (trade name)] etc.; naturally occurring substances such as shellac etc.

Examples of the sustained-release film coating base include cellulose polymers such as ethyl cellulose etc.; acrylic polymers such as aminoalkyl methacrylate copolymer RS [Eudragit RS (trade name)], ethyl acrylate-methyl methacrylate copolymer suspension [Eudragit NE (trade name)] etc.

The aforementioned coating bases may be used after mixing with two or more kinds thereof at appropriate ratios. For coating, for example, a light shielding agent such as titanium oxide, diiron trioxide and the like can be used.

The compound of the present invention shows low toxicity (e.g., acute toxicity, chronic toxicity, genetic toxicity, reproductive toxicity, cardiotoxicity, carcinogenicity and the like) and a few side effects. Therefore, it can be used as an agent for the prophylaxis or treatment of or a diagnostic of various diseases in a mammal (e.g., human, bovine, horse, dog, cat, monkey, mouse, rat).

The compound of the present invention has a superior ACC (acetyl-CoA carboxylase) inhibitory action. Examples of ACC include liver, adipose tissue or pancreas-specific isozyme (ACC1); and muscle specific isozyme (ACC2). The compound of the present invention particularly has a selective inhibitory action on ACC2.

The compound of the present invention can be used as an agent for the prophylaxis or treatment of obesity, diabetes (e.g., type 1 diabetes, type 2 diabetes, gestational diabetes, obese diabetes), hyperlipidemia (e.g., hypertriglyceridemia, hypercholesterolemia, hypoHDL-emia, postprandial hyperlipemia), hypertension, cardiac failure, diabetic complications [e.g., neuropathy, nephropathy, retinopathy, diabetic cardiomyopathy, cataract, macroangiopathy, osteopenia, hyperosmolar diabetic coma, infections (e.g., respiratory infection, urinary tract infection, gastrointestinal infection, dermal soft tissue infections, inferior limb infection), diabetic gangrene, xerostomia hypacusis, hypacusis, cerebrovascular disorder, peripheral blood circulation disorder], metabolic syndrome (pathology having three or more selected from hypertriglyceridemia (TG), low HDL cholesterol (HDL-C), hypertension, abdomen obesity and impaired glucose tolerance), sarcopenia and the like.

For diagnostic criteria of diabetes, Japan Diabetes Society reported new diagnostic criteria in 1999.

According to this report, diabetes is a condition showing any of a fasting blood glucose level (glucose concentration of intravenous plasma) of not less than 126 mg/dl, a 75 g oral glucose tolerance test (75 g OGTT) 2 hr level (glucose concentration of intravenous plasma) of not less than 200 mg/dl, and a non-fasting blood glucose level (glucose concentration of intravenous plasma) of not less than 200 mg/dl. A condition not falling under the above-mentioned diabetes and different from "a condition showing a fasting blood glucose level (glucose concentration of intravenous plasma) of less than 110 mg/dl or a 75 g oral glucose tolerance test (75 g OGTT) 2 hr level (glucose concentration of intravenous plasma) of less than 140 mg/dl" (normal type) is called a "borderline type".

In addition, ADA (American Diabetes Association) reported new diagnostic criteria of diabetes in 1997 and WHO in 1998.

According to these reports, diabetes is a condition showing a fasting blood glucose level (glucose concentration of intravenous plasma) of not less than 126 mg/dl and a 75 g oral glucose tolerance test 2 hr level (glucose concentration of intravenous plasma) of not less than 200 mg/dl.

According to the above-mentioned reports, impaired glucose tolerance is a condition showing a fasting blood glucose level (glucose concentration of intravenous plasma) of less than 126 mg/dl and a 75 g oral glucose tolerance test 2 hr level (glucose concentration of intravenous plasma) of not less than 140 mg/dl and less than 200 mg/dl. According to the report of ADA, a condition showing a fasting blood glucose level (glucose concentration of intravenous plasma) of not less than 110 mg/dl and less than 126 mg/dl is called IFG (Impaired Fasting Glucose). According to the report of WHO, among the IFG (Impaired Fasting Glucose), a condition showing a 75 g oral glucose tolerance test 2 hr level (glucose concentration of intravenous plasma) of less than 140 mg/dl is called IFG (Impaired Fasting Glycemia).

The compound of the present invention can be also used as an agent for the prophylaxis or treatment of diabetes, borderline type, impaired glucose tolerance, IFG (Impaired Fasting Glucose) and IFG (Impaired Fasting Glycemia), as determined according to the above-mentioned new diagnostic criteria. Moreover, the compound of the present invention can prevent progress of borderline type, impaired glucose tolerance, IFG (Impaired Fasting Glucose) or IFG (Impaired Fasting Glycemia) into diabetes.

The compound of the present invention can also be used as an agent for the prophylaxis or treatment of osteoporosis, cachexia (e.g., carcinocachexia, tuberculous cachexia, diabetic cachexia, hemopathic cachexia, endocrinopathic cachexia, infectious cachexia or cachexia induced by acquired immunodeficiency syndrome), fatty liver, polycystic ovary syndrome, renal disease (e.g., diabetic nephropathy, glomerulonephritis, glomerulosclerosis, nephrosissyndrome, hypertensive nephrosclerosis, terminal renal disorder), muscular dystrophy, myocardial infarction, angina pectoris, cerebrovascular disorder (e.g., cerebral infarction, cerebral apoplexy), Alzheimer's disease, Parkinson's disease, anxiety, dementia, insulin resistance syndrome, syndrome X, hyperinsulinemia, sensory abnormality in hyperinsulinemia, tumor (e.g., leukemia, breast cancer, prostate cancer, skin cancer), irritable bowel syndrome, acute or chronic diarrhea, inflammatory disease (e.g., chronic rheumatoid arthritis, spondylitis deformans, osteoarthritis, lumbago, gout, postoperative or posttraumatic inflammation, swelling, neuralgia, pharyngolaryngitis, cystitis, hepatitis (including nonalcoholic steatohepatitis), pneumonia, pancreatitis, enteritis, inflammatory bowel disease (including inflammatory colitis), ulcerative colitis, stomach mucous membrane injury (including stomach mucous membrane injury caused by aspirin)), small intestine mucous membrane injury, malabsorption, testis dysfunction, visceral obesity syndrome or sarcopenia.

The compound of the present invention can also be used for secondary prevention or suppression of progression of the above-mentioned various diseases (e.g., cardiovascular events such as myocardial infarction and the like).

While the dose of the compound of the present invention varies depending on the subject of administration, administration route, target disease, symptom and the like, for example, for oral administration to an adult diabetic patient, it is generally about 0.01 to 100 mg/kg body weight, preferably 0.05 to 30 mg/kg body weight, more preferably 0.1 to 10 mg/kg body weight for one dose, which is desirably administered once to 3 times a day.

With the aim of enhancing the action of the compound of the present invention or decreasing the dose of the compound and the like, the compound can be used in combination with pharmaceutical agents such as therapeutic agents for diabetes, therapeutic agents for diabetic complications, therapeutic agents for hyperlipidemia, antihypertensive

agents, antiobesity agents, diuretics, antithrombotic agents and the like (hereinafter to be abbreviated as concomitant drug). The time of administration of the compound of the present invention and that of the concomitant drug are not limited, and they may be administered simultaneously or in a staggered manner to the administration subject. In addition, the compound of the present invention and the concomitant drug may be administered as two kinds of preparations containing respective active ingredients or a single preparation containing both active ingredients.

The dose of the concomitant drug can be appropriately determined based on the dose employed clinically. In addition, the mixing ratio of the compound of the present invention and the concomitant drug can be appropriately determined according to the administration subject, administration route, target disease, condition, combination, and the like. For example, when the administration subject is a human, the concomitant drug may be used in an amount of 0.01 to 100 parts by weight per 1 part by weight of the compound of the present invention.

Examples of the therapeutic agent for diabetes include insulin preparations (e.g., animal insulin preparation extracted from the pancreas of bovine or swine; human insulin preparation synthesized by genetic engineering using Escherichia coli or yeast; zinc insulin; protamine zinc insulin; insulin fragment or derivative (e.g., INS-1), oral insulin preparation), insulin sensitizers (e.g., pioglitazone or a salt thereof (preferably hydrochloride), rosiglitazone or a salt thereof (preferably maleate), netoglitazone (MCC-555), edaglitazone (BM-13-1258), rivoglitazone, FK-614, compounds described in WO01/38325, tesaglitazar, ragaglitazar, muraglitazar, metaglidasen (MBX-102), naveglitazar, MX-6054, LY-510929, AMG131 (T-131) or a salt thereof, THR-0921, balaglitazone), PPARγ agonist, PPARγ antagonist, PPARγ/α dual agonist, α-glucosidase inhibitors (e.g., voglibose, acarbose, miglitol, emiglitate), biguanides (e.g., phenformin, metformin, buformin or a salt thereof (e.g., hydrochloride, fumarate, succinate)), insulin secretagogues [sulfonylurea (e.g., tolbutamide, glibenclamide, gliclazide, chlorpropamide, tolazamide, acetohexamide, glyclopyramide, glimepiride, glipizide, glybuzole etc.), repaglinide, senaglinide, nateglinide, mitiglinide or calcium salt hydrate thereof], GPR40 agonist, GLP-1 receptor agonists [e.g., GLP-1, GLP-1MR agent, NN-2211, AC-2993 (exendin-4), BIM-51077, Aib(8,35)hGLP-1 (7,37)$NH_2$, CJC-1131], amylin agonists (e.g., pramlintide), phosphotyrosine phosphatase inhibitors (e.g., sodium vanadate), dipeptidyl peptidase IV inhibitors (e.g., NVP-DPP-278, PT-100, P32/98, vidagliptin (LAF-237), P93/01, TS-021, sitagliptin (MK-431), saxagliptin (BMS-477118), denagliptin (823093)), β3 agonists (e.g., AJ-9677, AZ40140), gluconeogenesis inhibitors (e.g., glycogen phosphorylase inhibitor, glucose-6-phosphatase inhibitor, glucagon antagonist), SGLT (sodium-glucose cotransporter) inhibitors (e.g., T-1095), 11β-hydroxysteroid dehydrogenase inhibitors (e.g., BVT-3498), adiponectin or agonist thereof, IKK inhibitors (e.g., AS-2868), leptin sensitizer, somatostatin receptor agonists (e.g., compounds described in WO01/25228, WO03/42204, WO98/44921, WO98/45285 and WO99/22735) and glucokinase activators (e.g., Ro-28-1675).

Examples of the therapeutic agent for diabetic complications include aldose reductase inhibitors (e.g., tolrestat, epalrestat, zenarestat, zopolrestat, minalrestat, fidarestat, CT-112), neurotrophic factors and increasing drugs thereof (e.g., NGF, NT-3, BDNF, neurotrophin (e.g., 4-(4-chlorophenyl)-2-(2-methyl-1-imidazolyl)-5-[3-(2-methylphenoxy)propyl]oxazole)) described in WO01/14372, nerve regeneration promoters (e.g., Y-128), PKC inhibitors (e.g., ruboxistaurin mesylate), AGE inhibitors (e.g., ALT946, pimagedine, pyratoxanthine, N-phenacylthiazolium bromide (ALT766), ALT-711, EXO-226, pyridorin, pyridoxamine), active oxygen scavengers (e.g., thioctic acid), cerebral vasodilators (e.g., tiapuride, mexiletine), somatostatin receptor agonists (e.g., BIM23190) and apoptosis signal regulating kinase-1 (ASK-1) inhibitor.

Examples of the therapeutic agent for hyperlipidemia include statin compounds (e.g., pravastatin, simvastatin, lovastatin, atorvastatin, fluvastatin, rosuvastatin, pitavastatin or a salt thereof (e.g., sodium salt, calcium salt)), squalene synthase inhibitors (e.g., compounds described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzooxazepin-3-yl]acetyl]piperidine-4-acetic acid), fibrate compounds (e.g., bezafibrate, clofibrate, simfibrate, clinofibrate), ACAT inhibitors (e.g., avasimibe, eflucimibe), anion exchange resins (e.g., colestyramine), probucol, nicotinic acid drugs (e.g., nicomol, niceritrol), ethyl icosapentate and phytosterols (e.g., soysterol, γ-oryzanol).

Examples of the antihypertensive agent include angiotensin converting enzyme inhibitors (e.g., captopril, enalapril, delapril), angiotensin II antagonists (e.g., candesartan cilexetil, losartan, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium antagonists (e.g., manidipine, nifedipine, amlodipine, efonidipine, nicardipine), potassium channel openers (e.g., levcromakalim, L-27152, AL 0671, NIP-121) and clonidine.

Examples of the antiobesity agent include central nervous system antiobesity drugs (e.g., dexfenfluramine, fenfluramine, phentermine, sibutramine, amfepramone, dexamphetamine, mazindol, phenylpropanolamine, clobenzorex; MCH receptor antagonists (e.g., SB-568849; SNAP-7941; compounds described in WO01/82925 and WO01/87834); neuropeptide Y antagonists (e.g., CP-422935); cannabinoid receptor antagonists (e.g., SR-141716, SR-147778); ghrelin antagonist; 11β-hydroxysteroid dehydrogenase inhibitors (e.g., BVT-3498)), pancreatic lipase inhibitors (e.g., orlistat, ATL-962), β3 agonists (e.g., AJ-9677, AZ40140), anorectic peptides (e.g., leptin, CNTF (ciliary neurotrophic factor)), cholecystokinin agonists (e.g., lintitript, FPL-15849) and feeding deterrents (e.g., P-57).

Examples of the diuretics include xanthine derivatives (e.g., theobromine sodium salicylate, theobromine calcium salicylate), thiazide preparations (e.g., ethiazide, cyclopenthiazide, trichloromethyazide, hydrochlorothiazide, hydroflumethiazide, benzylhydrochlorothiazide, penflutizide, polythiazide, methyclothiazide), antialdosterone preparations (e.g., spironolactone, triamterene), carbonic anhydrase inhibitors (e.g., acetazolamide), chlorobenzenesulfonamide agents (e.g., chlortalidone, mefruside, indapamide), azosemide, isosorbide, ethacrynic acid, piretanide, bumetanide and furosemide.

Examples of the antithrombotic agent include heparin (e.g., heparin sodium, heparin calcium, dalteparin sodium), warfarin (e.g., warfarin potassium), anti-thrombin drugs (e.g., aragatroban), thrombolytic agents (e.g., urokinase, tisokinase, alteplase, nateplase, monteplase, pamiteplase) and platelet aggregation inhibitors (e.g., ticlopidine hydrochloride, cilostazol, ethyl icosapentate, beraprost sodium, sarpogrelate hydrochloride).

The production method of compound (I) is explained in the following. Compound (I) can be produced by, for example, the method of Reaction Schemes 1, 2, 3, 4, 5, 6, 7 and 8 to be described in detail in the following or a method according thereto.

In the following Reaction Schemes 1, 2, 3, 4, 5, 6, 7 and 8, the compound used as a starting material compound may be each in the form of a salt. Examples of the salt include those exemplified as the salt of compound (I).

In each of the reactions in the following Reaction Schemes 1, 2, 3, 4, 5, 6, 7 and 8, the resultant product can be used directly as the reaction mixture, or as a crude product for the next reaction. In addition, it can be isolated from a reaction mixture according to a conventional method, and can be easily purified by a general separation means (e.g., recrystallization, distillation, chromatography).

When alkylation reaction, hydrolysis, amination reaction, esterification reaction, amidation reaction, esterification reaction, etherification reaction, oxidation reaction, reduction reaction and the like are to be performed in the following Reaction Schemes 1, 2, 3, 4, 5, 6, 7 and 8, these reactions are performed according to a method known per se. Examples of such method include the methods described in ORGANIC FUNCTIONAL GROUP PREPARATIONS, 2nd ed., ACADEMIC PRESS, INC., 1989; Comprehensive Organic Transformations, VCH Publishers Inc., 1989 and the like, and the like.

The solvents used in the following reactions, which are shown with generic terms, are explained in the following.

Examples of the "nitrile solvent" include acetonitrile, propionitrile and the like.

Examples of the "amide solvent" include N,N-dimethylformamide (DMF), N,N-dimethylacetamide, N-methylpyrrolidone and the like.

Examples of the "halogenated hydrocarbon solvent" include dichloromethane, chloroform, 1,2-dichloroethane, carbon tetrachloride and the like.

Examples of the "ether solvent" include diethyl ether, diisopropyl ether, tert-butyl methyl ether, tetrahydrofuran (THF), 1,4-dioxane, 1,2-dimethoxyethane and the like.

Examples of the "aromatic solvent" include benzene, toluene, xylene, pyridine and the like.

Examples of the "aliphatic hydrocarbon solvent" include hexane, pentane, cyclohexane and the like.

Examples of the "sulfoxide solvent" include dimethyl sulfoxide (DMSO) and the like.

Examples of the "alcohol solvent" include methanol, ethanol, propanol, 2-propanol, butanol, isobutanol, tert-butanol and the like.

Examples of the "ester solvent" include methyl acetate, ethyl acetate, n-butyl acetate, tert-butyl acetate and the like.

Examples of the "ketone solvent" include acetone, methylethylketone and the like.

Examples of the "organic acid solvent" include formic acid, acetic acid, propionic acid, trifluoroacetic acid, methanesulfonic acid and the like.

The bases used in the following reactions, which are shown with generic terms, are explained in the following.

Examples of the "inorganic base" include sodium hydroxide, potassium hydroxide, lithium hydroxide, barium hydroxide and the like.

Examples of the "basic salt" include sodium carbonate, potassium carbonate, cesium carbonate, sodium hydrogen carbonate, potassium hydrogen carbonate and the like.

Examples of the "aromatic amine" include pyridine, imidazole, 2,6-lutidine and the like.

Examples of the "tertiary amine" include triethylamine, diisopropylethylamine, N-methylmorpholine, DBU (1,8-diazabicyclo[5.4.0]undec-7-ene), DBN (1,5-diazabicyclo[4.3.0]non-5-ene) and the like.

Examples of the "alkali metal hydride or alkaline earth metal hydride" include lithium hydride, sodium hydride, potassium hydride, calcium hydride and the like.

Examples of the "metal amide" include lithium amide, sodium amide, lithium diisopropylamide, lithium dicyclohexylamide, lithium hexamethyldisilazide, sodium hexamethyldisilazide, potassium hexamethyldisilazide and the like.

Examples of the "alkyl metal" include n-butyl lithium, sec-butyl lithium, tert-butyl lithium, methyl magnesium bromide and the like.

Examples of the "aryl metal" include phenyl lithium, phenyl magnesium bromide and the like.

Examples of the "metal alkoxide" include sodium methoxide, sodium ethoxide, potassium tert-butoxide and the like.

<Reaction scheme 1>

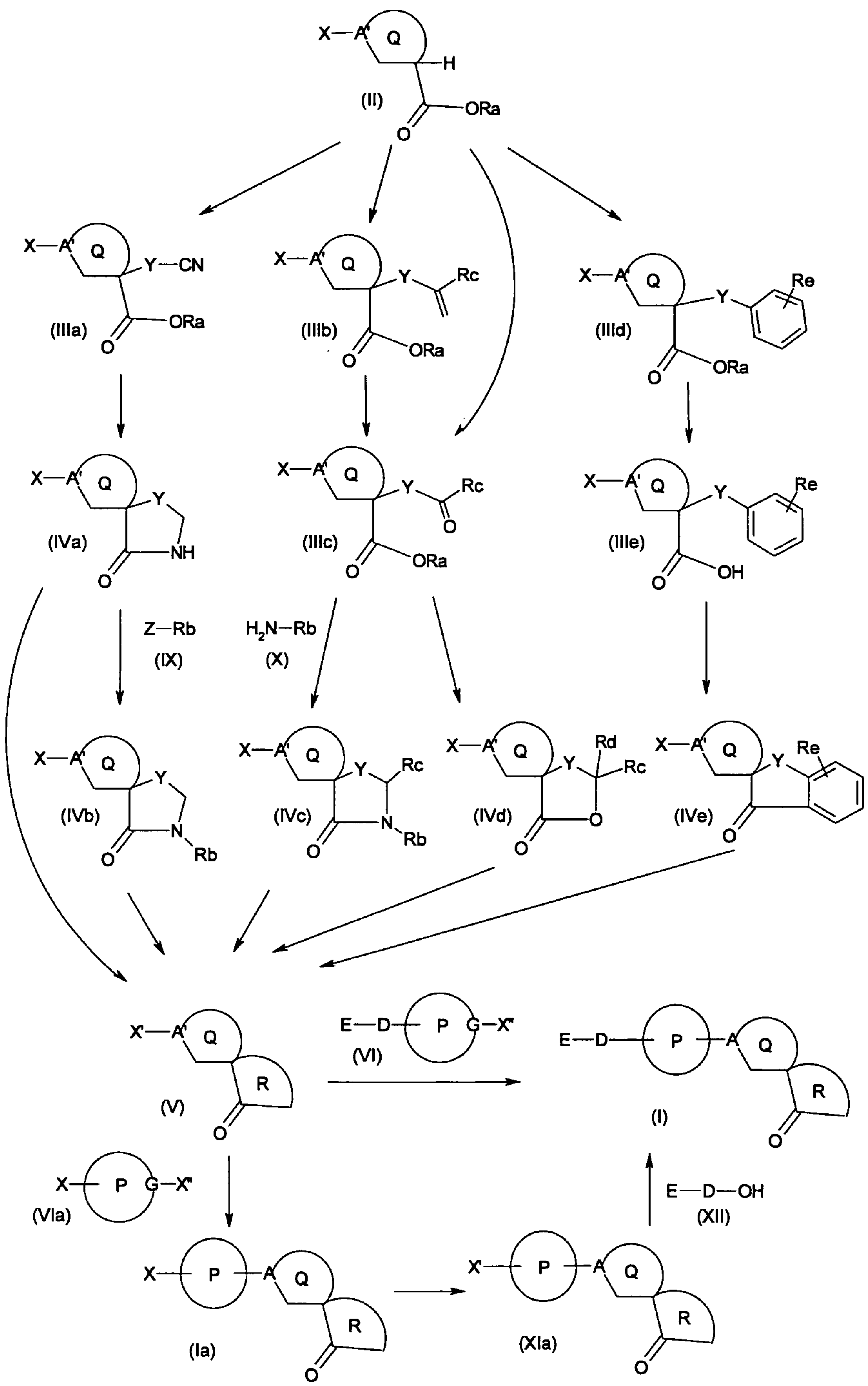

<Reaction scheme 2>

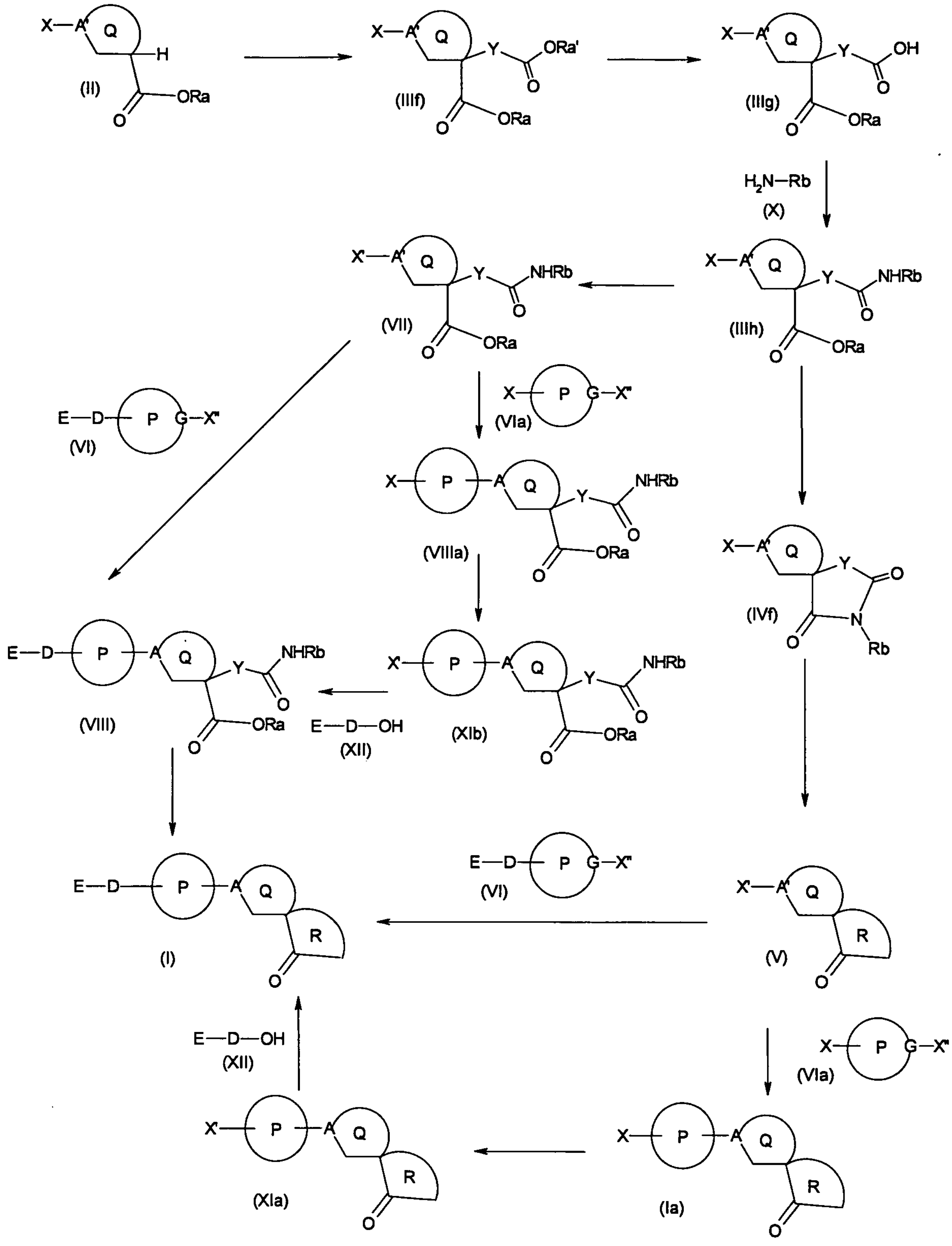

<Reaction scheme 3>

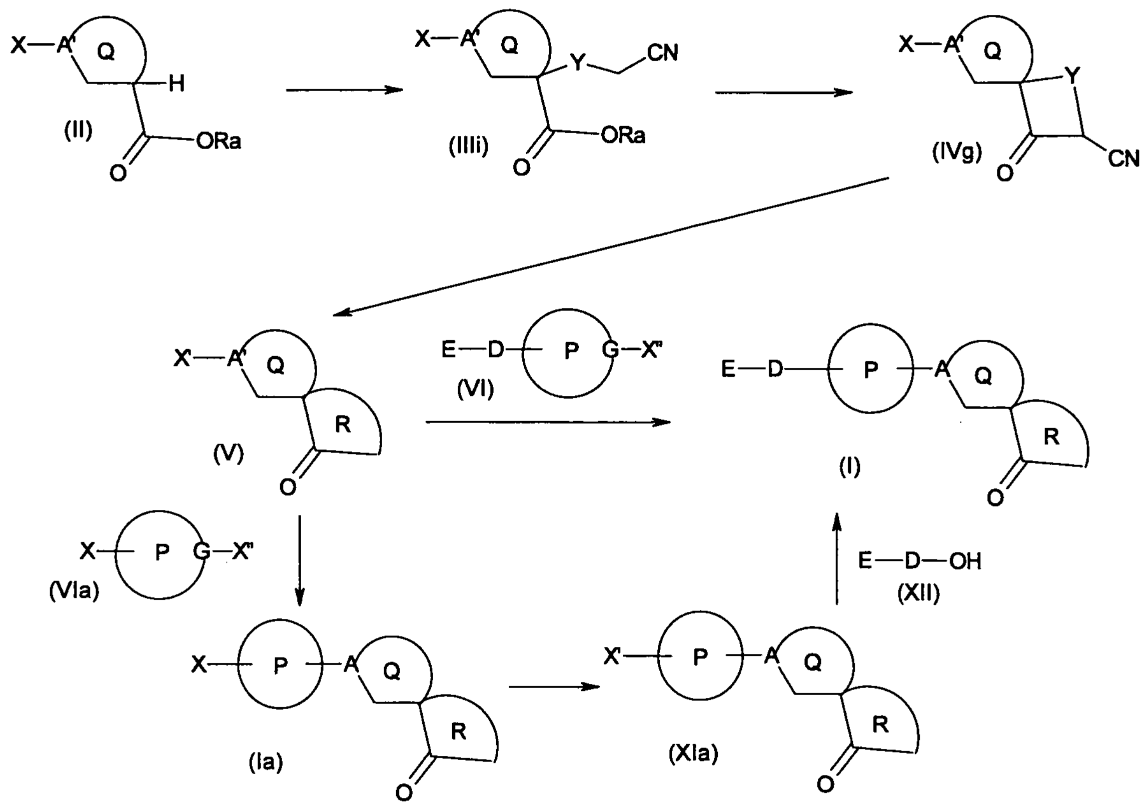

<Reaction scheme 4>

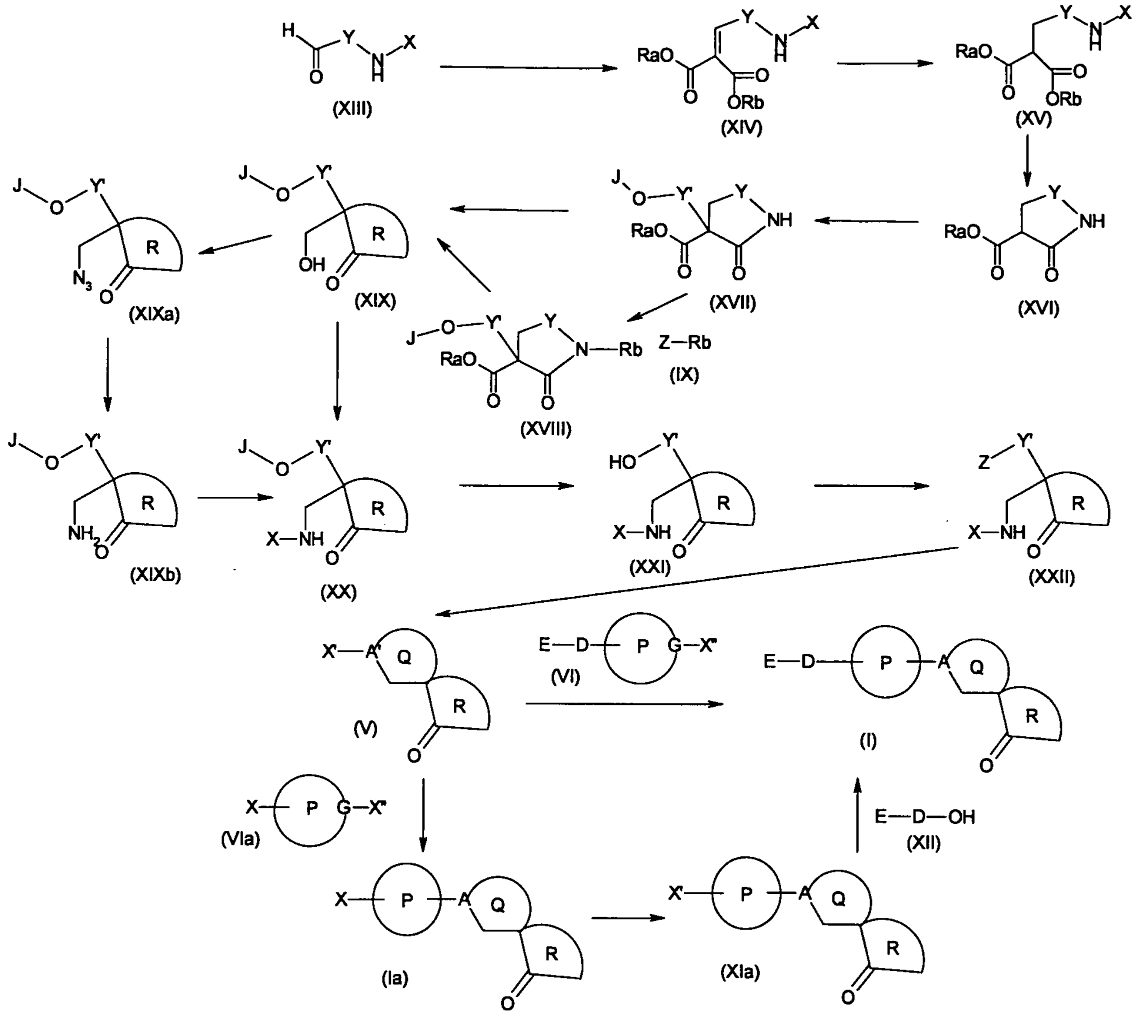

<Reaction scheme 5>

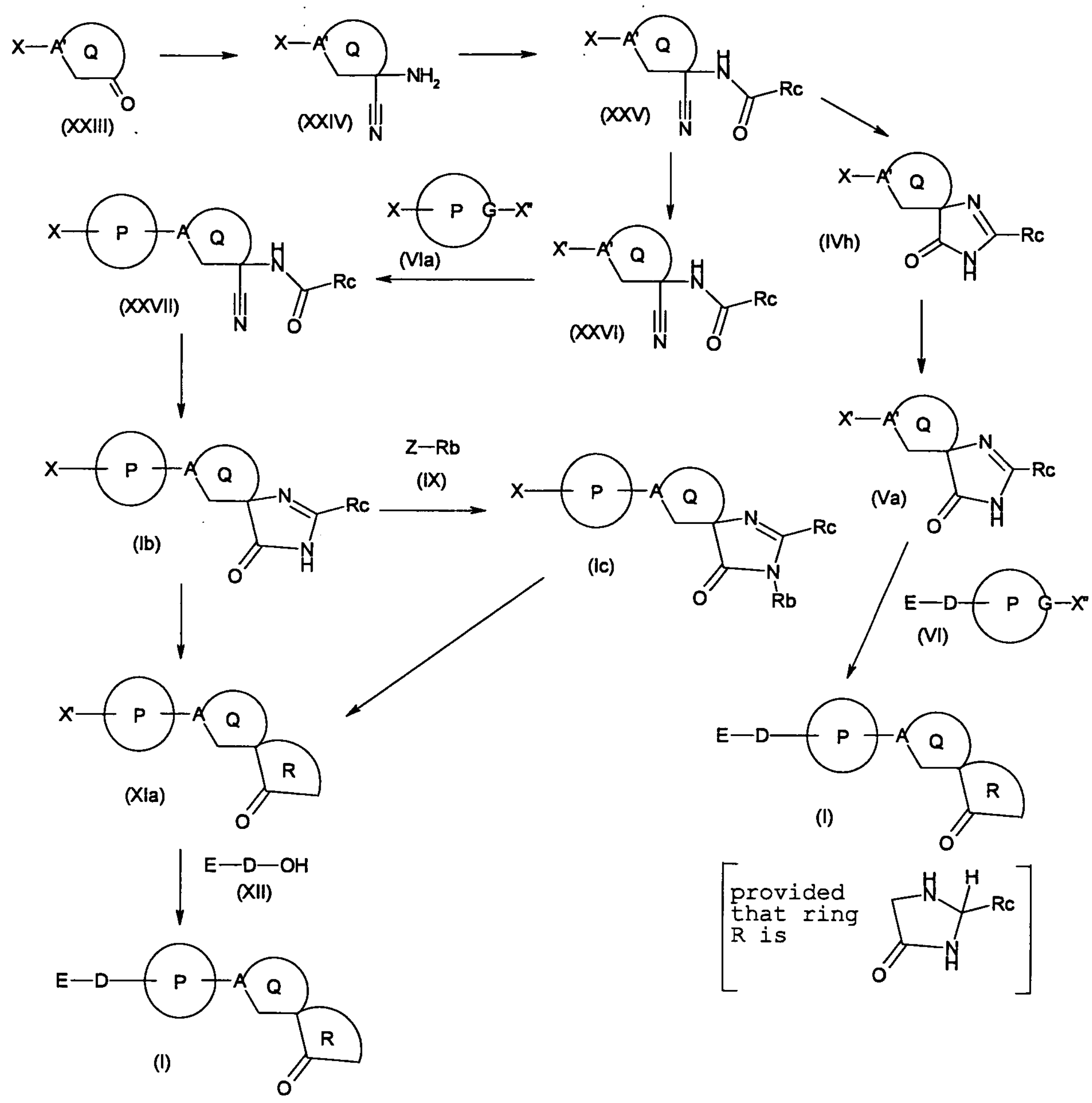

<Reaction scheme 6>

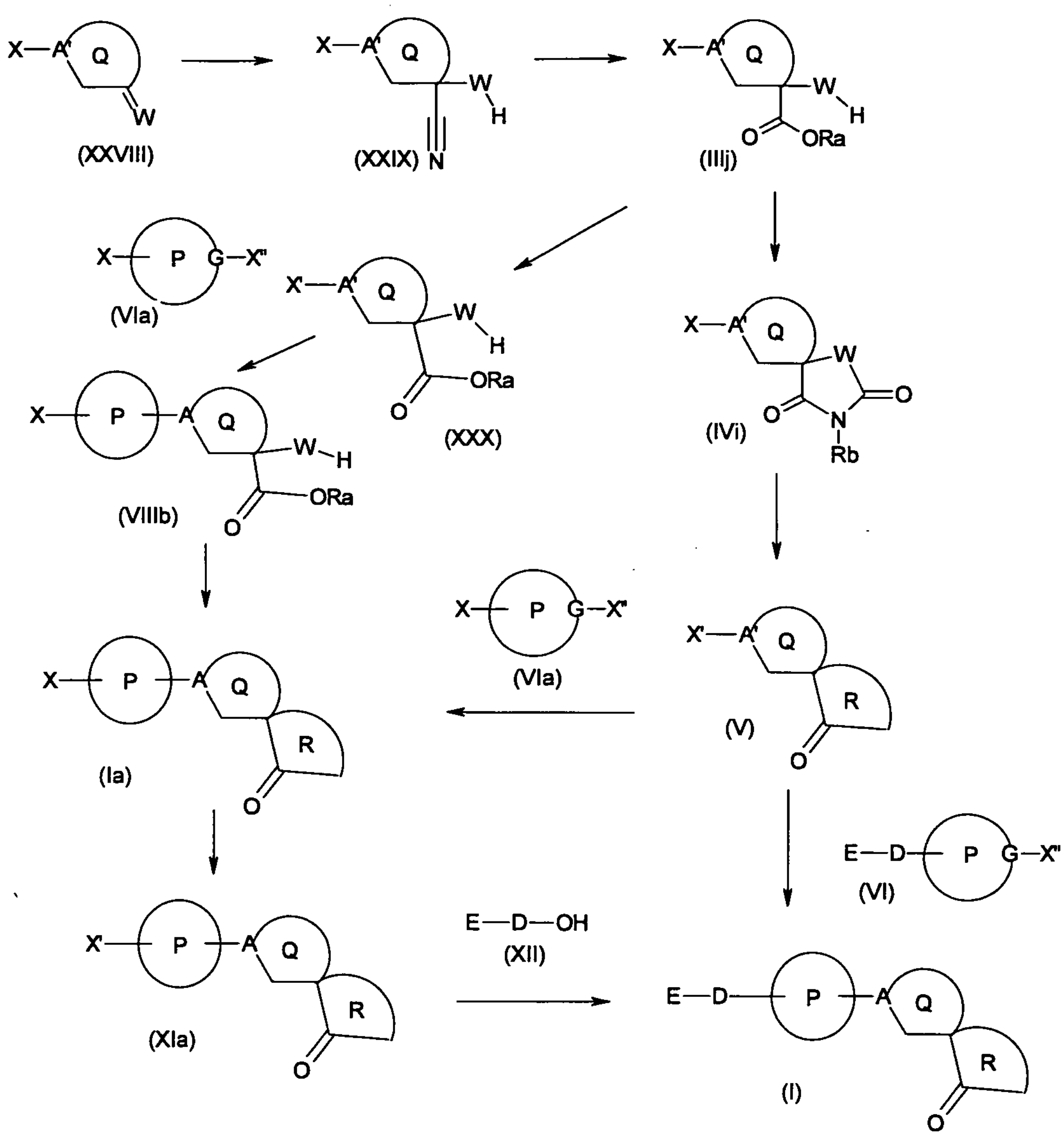

<Reaction scheme 7>

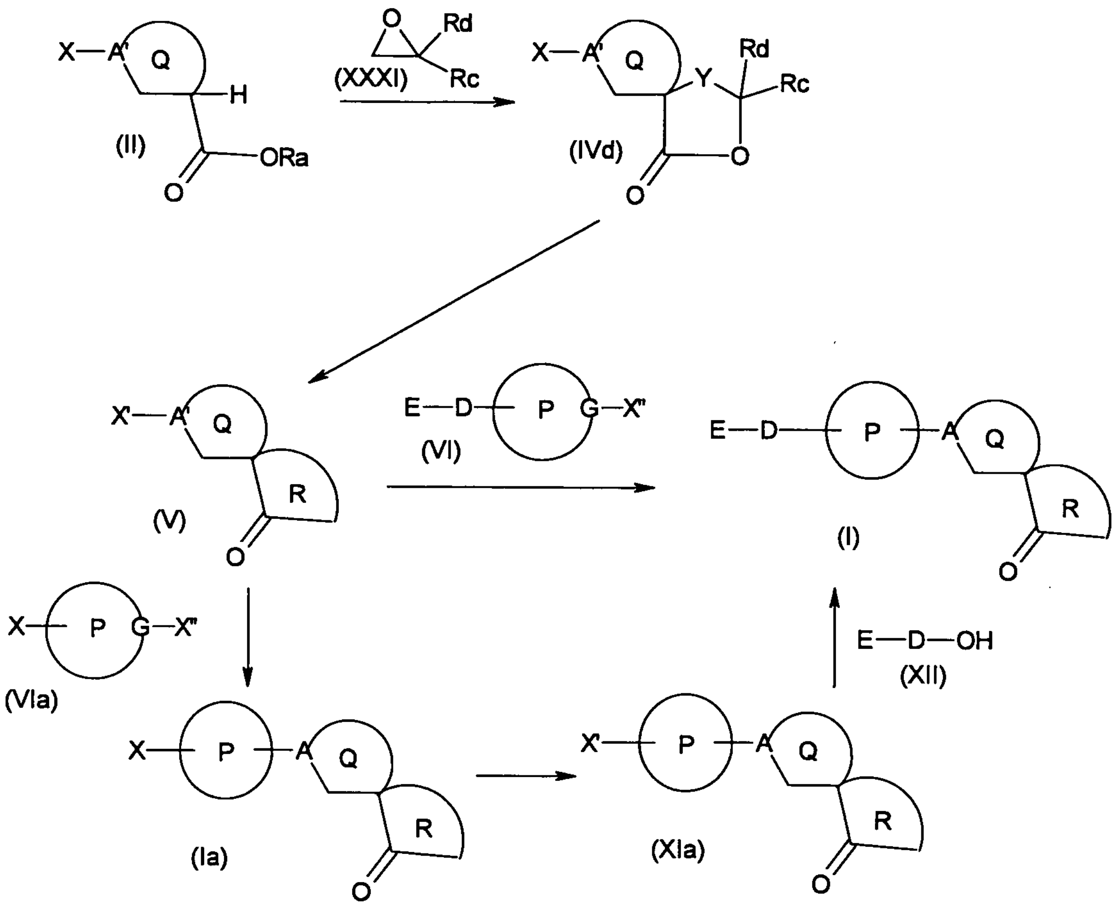

<Reaction scheme 8>

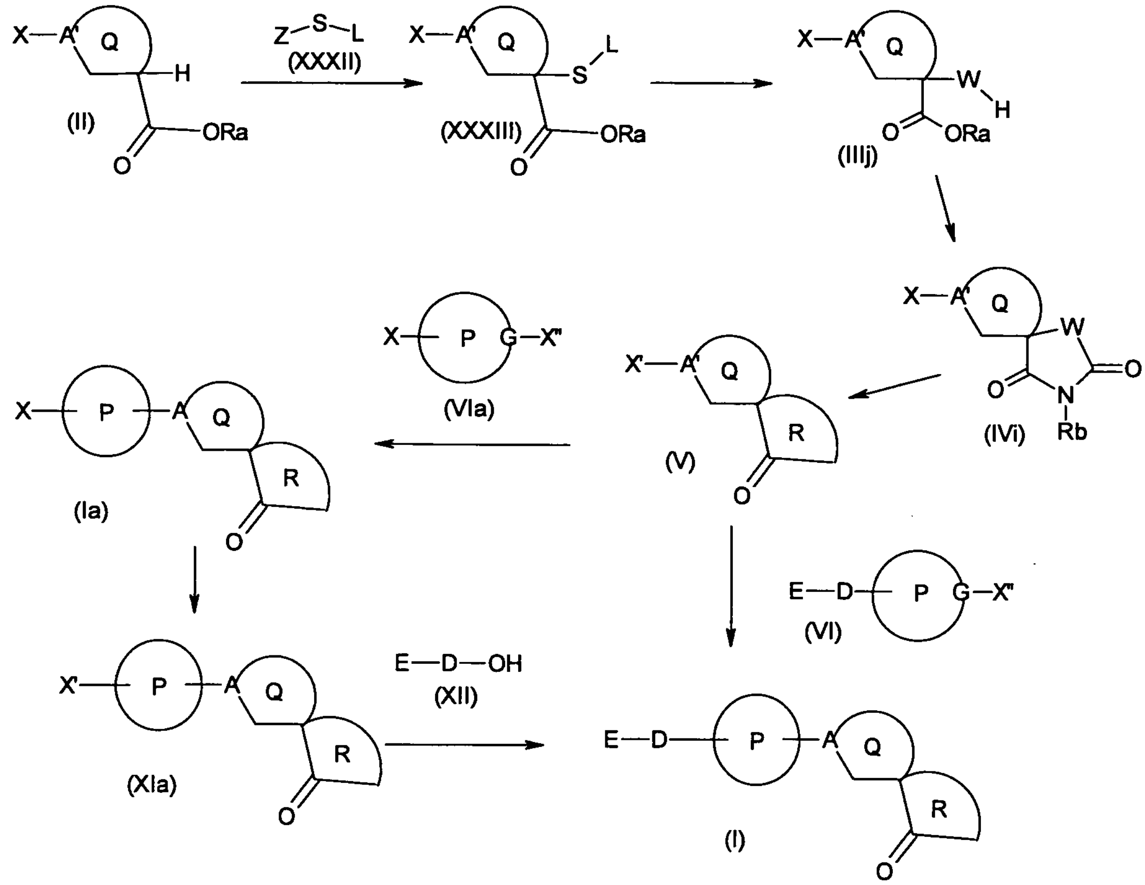

In the formulas, E, D, A, ring P, ring Q and ring R are each as mentioned above.

Ra, Ra', Rb, Rc, Rd and Re are the same or different and each is a hydrogen atom or a substituent. Examples of the "substituent" include those exemplified as the substituent for the "cyclic group" of the aforementioned "optionally substituted cyclic group" for E.

Y and Y' are each an optionally substituted linear $C_{1-3}$ alkylene. Examples of the "linear $C_{1-3}$ alkylene" include $-CH_2-$, $-CH_2CH_2-$ and $-CH_2CH_2CH_2-$. Examples of the substituent include those exemplified as the substituent for the "cyclic group" of the aforementioned "optionally substituted cyclic group" for E.

Z is a leaving group. Examples of the "leaving group" include a halogen atom, a sulfonated hydroxy group (e.g., toluenesulfonyloxy group, methanesulfonyloxy group, trifluoromethanesulfonyloxy group) and the like.
A' is N or C.

1) When A' is N,
X is, for example, an amino-protecting group generally used in the peptide chemistry and the like, from those exemplified as the substituent for the "cyclic group" of the aforementioned "optionally substituted cyclic group" for E, and the like,
X' is a hydrogen atom,
G is C,
X" is an oxo group, a halogen atom, a sulfonated hydroxy group (e.g., a toluenesulfonyloxy group, a methanesulfonyloxy group, a trifluoromethanesulfonyloxy group) and the like.
2) When A' is C,
X is an oxo-protecting group generally used in the organic synthesis, for example, cyclic acetal (e.g., 1,3-dioxane), noncyclic acetal (e.g., di-$C_{1-6}$ alkylacetal) and the like,
X' is an oxo group,
G is N, and
X" is a hydrogen atom.

J is, for example, a hydroxy-protecting group generally used in the peptide chemistry and the like, from those exemplified as the substituent for the "cyclic group" of the aforementioned "optionally substituted cyclic group" for E. L is, for example, a mercapto-protecting group generally used in the peptide chemistry and the like, from those exemplified as the substituent for the "cyclic group" of the aforementioned "optionally substituted cyclic group" for E.
W is O, NRc or S. When W is NRc, compound (IVi) can also be produced according to a method known from WO 2006/053024 A2.
Compound (IIIa) can be produced, for example, by an alkylation reaction of compound (II).
The alkylation reaction can be performed by a method known per se, for example, the method described in Journal of Medicinal Chemistry (J. Med. Chem.), pages 2439-2441, 1998 and the like, or by a method according thereto.
Compound (II) is easily commercially available, and can also be produced by a method known per se or by a method according thereto.
This reaction can be performed by reacting compound (II) with an alkylating agent in the presence of a base, in an inert solvent.
Examples of the above-mentioned "alkylating agent" include cyanoalkyl halide (e.g., bromoacetonitrile), alkenylnitrile (e.g., acrylonitrile) and the like. The amount of the "alkylating agent" to be used is generally 1 to 5 equivalents, preferably 1 to 1.5 equivalents, relative to compound (II).
Examples of the above-mentioned "inert solvent" include ether solvent, aromatic solvent, aliphatic hydrocarbon solvent and the like. Two or more kinds of these may be used in a mixture at an appropriate ratio. Of these, ether solvent and the like are preferable.
Examples of the above-mentioned "base" include "alkali metal hydride or alkaline earth metal hydride", "metal amide", "alkyl metal", "aryl metal" and the like. The amount of the "base" to be used is generally 1 to 10 equivalents, preferably 1 to 1.5 equivalents, relative to compound (II).
The reaction temperature is generally -100˚C to 150˚C, preferably -78˚C to 100˚C.
The reaction time is generally 5 min to 48 hr, preferably 30 min to 24 hr.
Compound (IIIb) can be produced, for example, by an alkylation reaction of compound (II).
The alkylation reaction can be performed by a method known per se, for example, the method described in Journal of Medicinal Chemistry (J. Med. Chem.), pages 2439-2441, 1998 and the like, or by a method according thereto.
This reaction can be performed by reacting compound (II) with an alkylating agent in the presence of a base in an inert solvent.
Examples of the above-mentioned "alkylating agent" include alkenyl halide (e.g., allyl bromide) and the like. The amount of the "alkylating agent" to be used is generally 1 to 5 equivalents, preferably 1 to 1.5 equivalents, relative to compound (II).
Examples of the above-mentioned "inert solvent" include ether solvent, aromatic solvent, aliphatic hydrocarbon solvent and the like. Two or more kinds of these may be used in a mixture at an appropriate ratio. Of these, ether solvent and the like are preferable.
Examples of the above-mentioned "base" include "alkali metal hydride or alkaline earth metal hydride", "metal amide", "alkyl metal", "aryl metal" and the like. The amount of the "base" to be used is generally 1 to 10 equivalents, preferably 1 to 1.5 equivalents, relative to compound (II).
The reaction temperature is generally -100˚C to 150˚C, preferably -78˚C to 100˚C.
The reaction time is generally 5 min to 48 hr, preferably 30 min to 24 hr.
Compound (IIIc) can be produced, for example, by an alkylation reaction of compound (II).
The alkylation reaction can be performed by a method known per se, for example, the method described in Journal of Medicinal Chemistry (J. Med. Chem.), pages 2439-2441, 1998 and the like, or by a method according thereto.
This reaction can be performed by reacting compound (II) with an alkylating agent in the presence of a base in an inert solvent.
Examples of the above-mentioned "alkylating agent" include alkenyl aldehyde (e.g., acrolein), alkenyl ketone (e.g., methyl vinyl ketone) and the like. The amount of the "alkylating agent" to be used is generally 1 to 5 equivalents, preferably 1 to 1.5 equivalents, relative to compound (II).
Examples of the above-mentioned "inert solvent" include ether solvent, aromatic solvent, aliphatic hydrocarbon solvent and the like. Two or more kinds of these may be used in a mixture at an appropriate ratio. Of these, ether solvent and the like are preferable.
Examples of the above-mentioned "base" include "alkali metal hydride or alkaline earth metal hydride", "metal amide", "alkyl metal", "aryl metal" and the like. The amount of the "base" to be used is generally 1 to 10 equivalents, preferably 1 to 1.5 equivalents, relative to compound (II).
The reaction temperature is generally -100˚C to 150˚C, preferably -78˚C to 100˚C.
The reaction time is generally 5 min to 48 hr, preferably 30 min to 24 hr.
Compound (IIIc) can also be produced, for example, by an oxidative fission reaction of compound (IIIb).
The oxidative fission reaction can be performed by a method known *per* se, for example, the method described

in Heterocycles, pages 2263-2267, 1992 and the like, or by a method according thereto.

This reaction can be performed by reacting compound (IIIb) with an oxidant in an inert solvent.

Examples of the above-mentioned "oxidant" include ozone, potassium permanganate, sodium periodate, osmium tetroxide and the like. The amount of the "oxidant" to be used is generally 1 to 5 equivalents, preferably 1 to 1.5 equivalents, relative to compound (IIIb).

Examples of the above-mentioned "inert solvent" include alcohol solvent, nitrile solvent, amide solvent, halogenated hydrocarbon solvent, ether solvent and the like. Two or more kinds of these may be used in a mixture at an appropriate ratio. Of these, halogenated hydrocarbon solvent and the like are preferable.

The reaction temperature is generally -100˚C to 50˚C, preferably -78˚C to 0˚C.

The reaction time is generally 5 min to 48 hr, preferably 30 min to 24 hr.

Compound (IIId) can be produced, for example, by an alkylation reaction of compound (II).

The alkylation reaction can be performed by a method known *per* se, for example, the method described in Journal of Medicinal Chemistry (J. Med. Chem.), pages 2439-2441, 1998 and the like, or by a method according thereto.

This reaction can be performed by reacting compound (II) with an alkylating agent in the presence of a base in an inert solvent.

Examples of the above-mentioned "alkylating agent" include aralkyl halide (e.g., benzyl bromide), aralkylaldehyde (e.g., phenylacetaldehyde) and the like. The amount of the "alkylating agent" to be used is generally 1 to 5 equivalents, preferably 1 to 1.5 equivalents, relative to compound (II).

Examples of the above-mentioned "inert solvent" include ether solvent, aromatic solvent, aliphatic hydrocarbon solvent and the like. Two or more kinds of these may be used in a mixture at an appropriate ratio. Of these, ether solvent and the like are preferable.

Examples of the above-mentioned "base" include "alkali metal hydride or alkaline earth metal hydride", "metal amide", "alkyl metal", "aryl metal" and the like. The amount of the "base" to be used is generally 1 to 10 equivalents, preferably 1 to 1.5 equivalents, relative to compound (II).

The reaction temperature is generally -100˚C to 150˚C, preferably -78˚C to 100˚C.

The reaction time is generally 5 min to 48 hr, preferably 30 min to 24 hr.

Compound (IIIe) can be produced, for example, by hydrolysis of compound (IIId).

This reaction can be performed by reacting compound (IIId) with a base in an inert solvent.

Examples of the above-mentioned "base" include "inorganic base" and the like. The amount of the "base" to be used is generally 1 to 10 equivalents, preferably 1 to 1.5 equivalents, relative to compound (IIId).

Examples of the above-mentioned "inert solvent" include alcohol solvent, nitrile solvent, aromatic solvent, aliphatic hydrocarbon solvent, ether solvent, amide solvent, halogenated hydrocarbon solvent and the like. These are preferably used in a mixture with water at an appropriate ratio. Of these, a water-containing alcohol solvent is preferable.

The reaction temperature is generally -78˚C to 150˚C, preferably -20˚C to 100˚C.

The reaction time is generally 5 min to 100 hr, preferably 30 min to 24 hr.

Compound (IIIf) can be produced, for example, by an alkylation reaction of compound (II).

The alkylation reaction can be performed by a method known per se, for example, the method described in Journal of Medicinal Chemistry (J. Med. Chem.), pages 2439-2441, 1998 and the like, or by a method according thereto.

This reaction can be performed by reacting compound (II) with an alkylating agent in the presence of a base in an inert solvent.

Examples of the above-mentioned "alkylating agent" include (alkoxycarbonyl)alkyl halide (e.g., ethyl bromoacetate), alkenyl ester (e.g., methyl acrylate) and the like. The amount of the "alkylating agent" to be used is generally 1 to 5 equivalents, preferably 1 to 1.5 equivalents, relative to compound (II).

Examples of the above-mentioned "inert solvent" include ether solvent, aromatic solvent, aliphatic hydrocarbon solvent and the like. Two or more kinds of these may be used in a mixture at an appropriate ratio. Of these, ether solvent and the like are preferable.

Examples of the above-mentioned "base" include "alkali metal hydrides or alkaline earth metal hydride", "metal amide", "alkyl metal", "aryl metal" and the like. The amount of the "base" to be used is generally 1 to 10 equivalents, preferably 1 to 1.5 equivalents, relative to compound (II).

The reaction temperature is generally -100˚C to 150˚C, preferably -78˚C to 100˚C.

The reaction time is generally 5 min to 48 hr, preferably 30 min to 24 hr.

Compound (IIIg) can be produced, for example, by hydrolysis of compound (IIIf).

This reaction can be performed by reacting compound (IIIf) with a base in an inert solvent.

Examples of the above-mentioned "base" include "inorganic base" and the like. The amount of the "base" to be used is generally 1 to 10 equivalents, preferably 1 to 1.5 equivalents, relative to compound (IIIf).

Examples of the above-mentioned "inert solvent" include alcohol solvent, nitrile solvent, aromatic solvent, aliphatic hydrocarbon solvent, ether solvent, amide solvent, halogenated hydrocarbon solvent and the like. These are preferably used in a mixture with water at an appropriate ratio. Of these, a water-containing alcohol solvent is preferable.

The reaction temperature is generally -78°C to 150°C, preferably -20°C to 100°C.
The reaction time is generally 5 min to 100 hr, preferably 30 min to 24 hr.
This reaction can also be performed by reacting compound (IIIf) in the presence of a metal catalyst and a hydrogen source in an inert solvent.
Examples of the above-mentioned "metal catalyst" include palladium-carbon, palladium black, palladium chloride, platinum oxide, platinum black, platinum-palladium, Raney nickel, Raney cobalt and the like. The amount of the "metal catalyst" to be used is generally 0.001 to 1000 equivalents, preferably 0.01 to 100 equivalents, relative to compound (IIIf).
Examples of the above-mentioned "hydrogen source" include hydrogen gas, formic acid, formic acid amine salt, phosphinic acid salt, hydrazine and the like.
Examples of the above-mentioned "inert solvent" include alcohol solvent, nitrile solvent, aromatic solvent, aliphatic hydrocarbon solvent, ether solvent, amide solvent, halogenated hydrocarbon solvents and the like. These may be used in a mixture with water at an appropriate ratio. Of these, an alcohol solvent is preferable.
The reaction temperature is generally -70°C to 150°C, preferably -20°C to 100°C.
The reaction time is generally 0.1 to 100 hr, preferably 0.1 to 40 hr.
Compound (IIIh) can be produced, for example, by an amidation reaction of compound (IIIg).
The above-mentioned "amidation reaction" includes the following "method using a dehydrating condensation agent" and "method using a reactive derivative of carboxylic acid" and the like.

i) Method using a dehydrating condensation agent

The method can be performed by reacting compound (IIIg) with compound (X) in the presence of a dehydrating condensation agent in an inert solvent. Where necessary, the reaction may be performed in the presence of a catalytic amount to 5 equivalents of 1-hydroxybenzotriazole (HOBt), a catalytic amount to 5 equivalents of a base and the like.
Compound (X) is easily commercially available, and can also be produced according to a method known per se or a method according thereto.
The amount of compound (X) to be used is generally 1 to 5 equivalents, preferably 1 to 1.5 equivalents, relative to compound (IIIg).
Examples of the above-mentioned "dehydrating condensation agent" include dicyclohexylcarbodiimide (DCC), 1-ethyl-3-(3-dimethylaminopropyl)carbodiimide hydrochloride (EDC·HCl) and the like. Of these, EDC·HCl is preferable. The amount of the "dehydrating condensation agent" to be used is generally 1 to 10 equivalents, preferably 1 to 5 equivalents, relative to compound (IIIg).
Examples of the above-mentioned "inert solvent" include nitrile solvent, amide solvent, halogenated hydrocarbon solvent, ether solvent and the like. Two or more kinds of these may be used in a mixture at an appropriate ratio. Of these, amide solvent is preferable.
Examples of the above-mentioned "base" include "aromatic amine", "tertiary amine" and the like.
The reaction temperature is generally -70°C to 150°C, preferably -20°C to 100°C.
The reaction time is generally 0.1 to 100 hr, preferably 1 to 48 hr.

ii) Method using a reactive derivative of carboxylic acid

A reactive derivative of compound (IIIg) is reacted with 1 to 5 equivalents (preferably 1 to 3 equivalents) of compound (X) in an inert solvent. Where necessary, the reaction may be performed in the presence of 1 to 10 equivalents, preferably 1 to 3 equivalents, of a base.
Examples of the "reactive derivative" of compound (IIIg) include acid halide (e.g., acid chloride, acid bromide), mixed acid anhydride (e.g., acid anhydride with $C_{1-6}$ alkyl-carboxylic acid, $C_{6-10}$ aryl-carboxylic acid, $C_{1-6}$ alkyl carbonic acid, etc.), active ester (e.g., ester with phenol optionally having substituents, HOBt, N-hydroxysuccinimide, etc.) and the like.
Examples of the "substituent" of the above-mentioned "phenol optionally having substituents" include those exemplified as the substituent for the "cyclic group" of the aforementioned "optionally substituted cyclic group" for E.
Specific examples of the "phenol optionally having substituents" include phenol, pentachlorophenol, pentafluorophenol, p-nitrophenol and the like.
The reactive derivative is preferably an acid halide.
Examples of the above-mentioned "inert solvent" include ether solvent, halogenated hydrocarbon solvent, aromatic solvent, aliphatic hydrocarbon solvent, nitrile solvent, amide solvent, ketone solvent, sulfoxide solvent, water and the like. Two or more kinds of these may be used in a mixture at an appropriate ratio. Of these, acetonitrile, THF, dichloromethane, chloroform and the like are preferable.
Preferable examples of the above-mentioned "base" include "aromatic amine", "tertiary amine" and the like.

The reaction temperature is generally -20°C to 100°C, preferably -20°C to 50°C.
The reaction time is generally 5 min to 40 hr, preferably 30 min to 18 hr.
Compound (IIIi) can be produced, for example, by an alkylation reaction of compound (II).
This reaction can be performed in the same manner as in the alkylation reaction of compound (II) for the aforementioned production of the compound (IIIa).
Compound (IIIj) can be produced, for example, by hydrolysis of compound (XXIX).
This reaction can be performed by reacting compound (XXIX) with a base or an acid in an inert solvent.
Examples of the above-mentioned "base" include "inorganic base" and the like. The amount of the "base" to be used is generally 1 to 10 equivalents, preferably 1 to 1.5 equivalents, relative to compound (XXIX).
Examples of the above-mentioned "acid" include organic acid, hydrochloric acid, sulfuric acid and the like. The amount of the "acid" to be used is generally 1 to 50 equivalents, preferably 1 to 10 equivalents, relative to compound (XXIX).
Examples of the above-mentioned "organic acid" include formic acid, acetic acid and the like.
Examples of the above-mentioned "inert solvent" include alcohol solvent, nitrile solvent, aromatic solvent, aliphatic hydrocarbon solvent, ether solvent, amide solvent, halogenated hydrocarbon solvent and the like. These are preferably used in a mixture with water at an appropriate ratio. Of these, a water-containing alcohol solvent is preferable.
The reaction temperature is generally -78°C to 150°C, preferably -20°C to 100°C.
The reaction time is generally 5 min to 100 hr, preferably 30 min to 24 hr.
Compound (IIIj) can also be produced, for example, by deprotection of compound (XXXIII).
The method for removing the protecting group is a method known per se, for example, a method according to the method described in Protective Groups in Organic Synthesis, John Wiley and Sons (1980) and the like.
Specifically, a method using an acid, a base, ultraviolet rays, hydrazine, phenylhydrazine, sodium N-methyldithiocarbamate, tetrabutylammonium fluoride, palladium acetate, trialkylsilyl halide (e.g., trimethylsilyl iodide, trimethylsilyl bromide) and the like, a reduction method and the like can be used.
Compound (IVa) can be produced, for example, a reduction reaction of compound (IIIa).
The reduction reaction can be performed by a method known *per* se, for example, the method described in Bioorganic and Medicinal Chemistry (Bioorg. Med. Chem.), pages 2945-2952, 1999 and the like, or by a method according thereto.
This reaction can be performed by reacting compound (IIIa) with a reducing agent in an inert solvent.
Examples of the above-mentioned "reducing agent" include metal hydrogen compounds (e.g., sodium bis(2-methoxyethoxy)aluminum hydride, diisobutylaluminum hydride), metal hydrogen complex compounds (e.g., sodium borohydride, sodium cyanoborohydride, lithium aluminum hydride, sodium aluminum hydride) and the like. The amount of the "reducing agent" to be used is generally 0.1 to 20 equivalents, preferably 1 to 5 equivalents, relative to compound (IIIa).
Examples of the above-mentioned "inert solvent" include alcohol solvent, aromatic solvent, aliphatic hydrocarbon solvent, ether solvent, ester solvent, amide solvents and the like. Two or more kinds of these may be used in a mixture at an appropriate ratio.
The reaction temperature is generally -70°C to 150°C, preferably -20°C to 100°C.
The reaction time is generally 0.1 to 100 hr, preferably 0.1 to 40 hr.
This reaction can also be performed by reacting compound (IIIa) in the presence of a metal catalyst and a hydrogen source in an inert solvent.
Examples of the above-mentioned "metal catalyst" include palladium-carbon, palladium black, palladium chloride, platinum oxide, platinum black, platinum-palladium, Raney nickel, Raney cobalt and the like. The amount of the "metal catalyst" to be used is generally 0.001 to 1000 equivalents, preferably 0.01 to 100 equivalents, relative to compound (IIIa).
Examples of the above-mentioned "hydrogen source" include hydrogen gas, formic acid, formic acid amine salt, phosphinic acid salt, hydrazine and the like.
Examples of the above-mentioned "inert solvent" include those exemplified for the reduction reaction using the aforementioned reducing agent.
The reaction temperature and reaction time are the same as in the aforementioned reduction reaction using a reducing agent.
Where necessary, this reaction can also be performed in the presence of ammonia (e.g., aqueous ammonia, ammonia-ethanol). A reaction in the presence of ammonia suppresses side reactions and compound (IVa) can be produced in a high yield.
Compound (IVb) can be produced, for example, by an alkylation reaction of compound (IVa).
The alkylation reaction can be performed by a method known *per se,* for example, the method described in Journal of Organic Chemistry (J. Org. Chem.), pages 2441-2450, 2004 and the like, or by a method according thereto.
This reaction can be performed by reacting compound (IVa) with compound (IX) in the presence of a base in an inert solvent.

Compound (IX) is commercially easily available, or can also be produced according to a method known per se or a method according thereto.

The amount of compound (IX) to be used is generally 1 to 5 equivalents, preferably 1 to 1.5 equivalents, relative to compound (IVa).

Examples of the above-mentioned "inert solvent" include nitrile solvent, amide solvent, halogenated hydrocarbon solvent, ether solvent and the like. Two or more kinds of these may be used in a mixture at an appropriate ratio. Of these, THF, DMF and the like are preferable.

Examples of the above-mentioned "base" include "alkali metal hydride or alkaline earth metal hydride" and the like. The amount of the "base" to be used is generally 1 to 10 equivalents, preferably 1 to 1.5 equivalents, relative to compound (IVa).

The reaction temperature is generally -100˚C to 150˚C, preferably 0˚C to 100˚C.

The reaction time is generally 5 min to 48 hr, preferably 30 min to 24 hr.

Compound (IVc) can be produced, for example, by a reductive amination reaction of compound (IIIc).

The reductive amination reaction can be performed by a method known *per se,* for example, the method described in Tetrahedron Letters (Tetrahedron Lett.), pages 8345-8349, 2001 and the like, or by a method according thereto.

This reaction can be performed by reacting compound (IIIc) with compound (X) in the presence of a reducing agent in an inert solvent. Where necessary, the reaction can also be performed in the presence of 1 to 50 equivalents of an organic acid.

The amount of compound (X) to be used is generally 1 to 5 equivalents, preferably 2 to 4 equivalents, relative to compound (IIIc).

Examples of the above-mentioned "reducing agent" include metal hydrogen compound (e.g., sodium bis(2-methoxyethoxy)aluminum hydride, diisobutyl aluminum hydride), metal hydrogen complex compound (e.g., sodium borohydride, sodium cyanoborohydride, sodium triacetoxyborohydride, lithium aluminum hydride, sodium aluminum hydride) and the like. The amount of the "reducing agent" to be used is generally 0.1 to 20 equivalents, preferably 1 to 5 equivalents, relative to compound (IIIc).

Examples of the above-mentioned "inert solvent" include alcohol solvent, nitrile solvent, amide solvent, halogenated hydrocarbon solvent, ether solvent and the like. Two or more kinds of these may be used in a mixture at an appropriate ratio. Of these, THF, dichloromethane and the like are preferable.

Examples of the above-mentioned "organic acid" include acetic acid and the like.

The reaction temperature is generally -78˚C to 100˚C, preferably 0˚C to 50˚C.

The reaction time is generally 5 min to 48 hr, preferably 30 min to 24 hr.

Of compound (IVd), a compound wherein Rd is a hydrogen atom can be produced, for example, by a reduction reaction of compound (IIIc).

This reaction can be performed by reacting compound (IIIc) with a reducing agent in an inert solvent.

Examples of the above-mentioned "reducing agent" include metal hydrogen compound (e.g., sodium bis(2-methoxyethoxy)aluminum hydride, diisobutyl aluminum hydride), metal hydrogen complex compound (e.g., sodium borohydride, sodium cyanoborohydride, lithium aluminum hydride, sodium aluminum hydride) and the like. The amount of the "reducing agent" to be used is generally 0.1 to 20 equivalents, preferably 1 to 5 equivalents, relative to compound (IIIc).

Examples of the above-mentioned "inert solvent" include nitrile solvent, aromatic solvent, hydrocarbon solvent, ether solvent, amide solvent, halogenated hydrocarbon solvent and the like. Two or more kinds of these solvents may be used in a mixture at an appropriate ratio. Of these, THF and the like are preferable.

The reaction temperature is generally -78˚C to 150˚C, preferably -20˚C to 100˚C.

The reaction time is generally 5 min to 48 hr, preferably 30 min to 24 hr.

Of compound (IVd), a compound wherein Rd is other than a hydrogen atom can be produced, for example, by an addition reaction of compound (IIIc).

This reaction can be performed by reacting compound (IIIc) with an organic metal reagent in an inert solvent.

Examples of the above-mentioned "organic metal reagent" include organic Grignard reagent (e.g., methyl magnesium bromide), organic lithium reagent (e.g., methyl lithium) and the like. The amount of the "organic metal reagent" to be used is generally 0.1 to 20 equivalents, preferably 1 to 5 equivalents, relative to compound (IIIc).

Examples of the above-mentioned "inert solvent" include nitrile solvent, aromatic solvent, hydrocarbon solvent, ether solvent, amide solvent, halogenated hydrocarbon solvent and the like. Two or more kinds of these solvents may be used in a mixture at an appropriate ratio. Of these, THF and the like are preferable.

The reaction temperature is generally -78˚C to 150˚C, preferably -20˚C to 100˚C.

The reaction time is generally 5 min to 48 hr, preferably 30 min to 24 hr.

Compound (IVd) can also be produced by reacting, for example, compound (II) with compound (XXXI).

This reaction can be performed by reacting compound (II) with compound (XXXI) in the presence of a base in an inert solvent.

Examples of the above-mentioned "base" include "alkali metal hydride or alkaline earth metal hydride", "metal amide", "alkyl metal", "aryl metal" and the like. The amount of the "base" to be used is generally 1 to 10 equivalents, preferably 1 to 1.5 equivalents, relative to compound (II).

Examples of the above-mentioned "inert solvent" include nitrile solvent, aromatic solvent, hydrocarbon solvent, ether solvent, amide solvent, halogenated hydrocarbon solvent and the like. Two or more kinds of these solvents may be used in a mixture at an appropriate ratio. Of these, THF and the like are preferable.

The reaction temperature is generally -78˚C to 150˚C, preferably -20˚C to 100˚C.

The reaction time is generally 5 min to 48 hr, preferably 30 min to 24 hr.

Compound (XXXI) is commercially easily available, or can also be produced according to a method known per se or a method according thereto.

Compound (IVe) can be produced, for example, by a Friedel-Crafts reaction of compound (IIIe).

This reaction can be performed by reacting compound (IIIe) with a carboxylic acid activator in the presence of a Lewis acid in an inert solvent.

Examples of the above-mentioned "carboxylic acid activator" include thionyl chloride, thionyl bromide, oxalyl chloride and the like.

Examples of the above-mentioned "Lewis acid" include aluminum chloride, boron trifluoride diethyl etherate and the like.

The amount of the "carboxylic acid activator" or "Lewis acid" to be used is generally 1 to 20 equivalents, preferably 1 to 10 equivalents, relative to compound (IIIe).

Examples of the above-mentioned "inert solvent" include hydrocarbon solvent, ether solvent, halogenated hydrocarbon solvent and the like. Two or more kinds of these solvents may be used in a mixture at an appropriate ratio. Of these, dichloromethane, dichloroethane and the like are preferable.

The reaction temperature is generally -78˚C to 150˚C, preferably -20˚C to 100˚C.

The reaction time is generally 5 min to 100 hr, preferably 30 min to 24 hr.

This reaction can also be performed by isolating a reactive derivative prepared from compound (IIIe) and a carboxylic acid activator, followed by reaction in the presence of a Lewis acid in an inert solvent.

Compound (IVf) can be produced, for example, a cyclization reaction of compound (IIIh).

The cyclization reaction can be performed by a method known per se, for example, by the method described in Journal of Medicinal Chemistry (J. Med. Chem.), pages 4118-4129, 1998 and the like, or by a method according thereto.

This reaction can be performed by reacting compound (IIIh) with a base in an inert solvent.

Examples of the above-mentioned "base" include "alkali metal hydride or alkaline earth metal hydride" and the like. The amount of the "base" to be used is generally 1 to 10 equivalents, preferably 1 to 1.5 equivalents, relative to compound (IIIh).

Examples of the above-mentioned "inert solvent" include nitrile solvent, aromatic solvent, aliphatic hydrocarbon solvent, ether solvent, amide solvent, halogenated hydrocarbon solvent and the like. Two or more kinds of these solvents may be used in a mixture at an appropriate ratio. Of these, amide solvent is preferable.

The reaction temperature is generally -100˚C to 150˚C, preferably 0˚C to 100˚C.

The reaction time is generally 5 min to 48 hr, preferably 30 min to 24 hr.

Compound (IVg) can be produced, for example, by a cyclization reaction of compound (IIIi).

The cyclization reaction can be performed by a method known per se, for example, the method described in Journal of Organic Chemistry (J. Org. Chem.), pages 820-826, 1990 and the like, or by a method according thereto.

This reaction can be performed by reacting compound (IIIi) with a base in an inert solvent.

Examples of the above-mentioned "base" include "alkali metal hydride or alkaline earth metal hydride" and the like. The amount of the "base" to be used is generally 1 to 10 equivalents, preferably 1 to 1.5 equivalents, relative to compound (IIIi).

Examples of the above-mentioned "inert solvent" include nitrile solvent, aromatic solvent, aliphatic hydrocarbon solvent, ether solvent, amide solvents, halogenated hydrocarbon solvent and the like. Two or more kinds of these solvents may be used in a mixture at an appropriate ratio. Of these, amide solvent is preferable.

The reaction temperature is generally -100˚C to 150˚C, preferably 0˚C to 100˚C.

The reaction time is generally 5 min to 48 hr, preferably 30 min to 24 hr.

Compound (IVh) can be produced, for example, by a cyclization reaction of compound (XXV).

This reaction can be performed by reacting compound (XXV) with a base in an inert solvent. Where necessary, the reaction can also be performed in the presence of a catalytic amount to 5 equivalents of hydrogen peroxide.

Examples of the above-mentioned "base" include "inorganic base" and the like. The amount of the "base" to be used is generally 1 to 10 equivalents, preferably 1 to 1.5 equivalents, relative to compound (XXV).

Examples of the above-mentioned "inert solvent" include alcohol solvent, nitrile solvent, aromatic solvent, aliphatic hydrocarbon solvent, ether solvent, amide solvent, halogenated hydrocarbon solvent and the like. These are preferably used in a mixture with water at an appropriate ratio. Of these, a water-containing alcohol solvent is preferable.

The reaction temperature is generally -78˚C to 150˚C, preferably -20˚C to 100˚C.

The reaction time is generally 5 min to 100 hr, preferably 30 min to 24 hr.

Compound (IVi) can be produced, for example, by a cyclization reaction of compound (IIIj).

This reaction can be performed by reacting compound (IIIj) with isocyanate (hereinafter sometimes to be referred to as isocyanic acid ester; e.g., ethyl isocyanate, isopropyl isocyanate) in the presence of a base in an inert solvent. The amount of isocyanate to be used is generally 1 to 5 equivalents, preferably 1 to 2 equivalents, relative to compound (IIIj).

Examples of the above-mentioned "base" include "alkali metal hydride or alkaline earth metal hydride" and the like. The amount of the "base" to be used is generally 0.5 to 10 equivalents, preferably 0.5 to 1.5 equivalents, relative to compound (IIIj).

Examples of the above-mentioned "inert solvent" include nitrile solvent, amide solvent, halogenated hydrocarbon solvent, ether solvent and the like. Two or more kinds of these may be used in a mixture at an appropriate ratio. Of these, THF is preferable.

The reaction temperature is generally -78˚C to 50˚C, preferably room temperature to 50˚C.

The reaction time is generally 5 min to 48 hr, preferably 30 min to 24 hr.

Compound (V) can be produced, for example, by deprotection of compound (IVa), (IVb), (IVc), (IVd), (IVe), (IVf), (IVg), (IVi) or (XXII).

The method for removing the protecting group is a method known *per* se, for example, a method according to the method described in Protective Groups in Organic Synthesis, John Wiley and Sons (1980) and the like.

Specifically, a method using an acid, a base, ultraviolet rays, hydrazine, phenylhydrazine, sodium N-methyldithiocarbamate, tetrabutylammonium fluoride, palladium acetate, trialkylsilyl halide (e.g., trimethylsilyl iodide, trimethylsilyl bromide) and the like, a reduction method and the like can be employed.

Compound (Va) can be produced, for example, by deprotection of compound (IVh).

This reaction can be performed in the same manner as in the aforementioned deprotection of the compound (IVa).

Compound (VII) can be produced, for example, by deprotection of compound (IIIh).

This reaction can be performed in the same manner as in the aforementioned deprotection of the compound (IVa).

Compound (VIIIa) can be produced, for example, by a coupling reaction of compound (VII) with compound (VIa).

This reaction can be performed by reacting compound (VII) with compound (VIa) in the presence of a reducing agent in an inert solvent. Where necessary, the reaction can also be performed in the presence of 1 to 50 equivalents of an organic acid.

Compound (VIa) is commercially easily available, or can also be produced according to a method known *per* se or a method according thereto.

The amount of compound (VIa) to be used is generally 1 to 5 equivalents, preferably 1 to 4 equivalents, relative to compound (VII).

Examples of the above-mentioned "reducing agent" include metal hydrogen compound (e.g., sodium bis(2-methoxyethoxy)aluminum hydride, diisobutylaluminum hydride), metal hydrogen complex compound (e.g., sodium borohydride, sodium cyanoborohydride, sodium triacetoxyborohydride, lithium aluminum hydride, sodium aluminum hydride) and the like. The amount of the "reducing agent" to be used is generally 0.1 to 20 equivalents, preferably 1 to 5 equivalents, relative to compound (VII).

Examples of the above-mentioned "inert solvent" include alcohol solvent, nitrile solvent, amide solvent, halogenated hydrocarbon solvent, ether solvent and the like. Two or more kinds of these may be used in a mixture at an appropriate ratio. Of these, THF, dichloromethane and the like are preferable.

Examples of the above-mentioned "organic acid" include acetic acid and the like.

The reaction temperature is generally -78˚C to 50˚C, preferably room temperature to 50˚C.

The reaction time is generally 5 min to 48 hr, preferably 30 min to 24 hr.

Compound (XIb) can be produced, for example, by deprotection of compound (VIIIa).

This reaction can be performed in the same manner as in the aforementioned deprotection of the compound (IVa).

Compound (VIII) can be produced, for example, by an acylation reaction of compound (XIb).

This reaction can be performed in the same manner as in the aforementioned amidation reaction of the compound (IIIg). Compound (XII) is commercially easily available, or can also be produced according to a method known *per se* or a method according thereto.

Compound (VIII) can also be produced, for example, by a coupling reaction of compound (VII) with compound (VI).

This reaction can be performed in the same manner as in the aforementioned coupling reaction of compound (VII) with compound (VIa). Compound (VI) is commercially easily available, or can also be produced according to a method known *per* se or a method according thereto.

Compound (XIa) can be produced, for example, by deprotection of compound (Ia), (Ib) or (Ic).

This reaction can be performed in the same manner as in the aforementioned deprotection of the compound (IVa).

Compound (XIV) can be produced, for example, by a Knoevenagel reaction of compound (XIII).

The Knoevenagel reaction can be performed by a method known per se, for example, the method described in Helvetica Chimica Acta (Hel. Chim. Acta), pages 450-465, 1983, and the like, or by a method according thereto.

Compound (XIII) is commercially easily available, or can also be produced according to a method known *per* se or a method according thereto.

This reaction is performed by reacting compound (XIII) with a malonic acid derivative in the presence of one or both of an acid and a base in an inert solvent.

Examples of the above-mentioned "acid" include organic acid and Lewis acid. The amount of the "acid" to be used is generally 1 to 10 equivalents, preferably 1 to 1.5 equivalents, relative to compound (XIII).

Examples of the above-mentioned "organic acid" include acetic acid and the like.

Examples of the above-mentioned "Lewis acid" include titanium (IV) chloride.

Preferable examples of the above-mentioned "base" include "aromatic amine", "secondary amine", "tertiary amine" and the like. The amount of the "base" to be used is generally 1 to 10 equivalents, preferably 1 to 1.5 equivalents, relative to compound (XIII).

Examples of the above-mentioned "inert solvent" include ether solvent, aromatic solvent, aliphatic hydrocarbon solvent and the like. Two or more kinds of these may be used in a mixture at an appropriate ratio. Of these, ether solvent and the like are preferable.

The reaction temperature is generally -100˚C to 150˚C, preferably -78˚C to 100˚C.

The reaction time is generally 5 min to 48 hr, preferably 30 min to 24 hr.

Compound (XV) can be produced, for example, by a reduction reaction of compound (XIV).

This reaction can be performed by reacting compound (XIV) in the presence of a metal catalyst and a hydrogen source in an inert solvent.

Examples of the above-mentioned "metal catalyst" include palladium-carbon, palladium black, palladium chloride, platinum oxide, platinum black, platinum-palladium, Raney-nickel, Raney cobalt and the like. The amount of the "metal catalyst" to be used is generally 0.001 to 1000 equivalents, preferably 0.01 to 100 equivalents, relative to compound (XIV).

Examples of the above-mentioned "hydrogen source" include hydrogen gas, formic acid, formic acid amine salt, phosphinic acid salt, hydrazine and the like.

Examples of the above-mentioned "inert solvent" include alcohol solvent, nitrile solvent, aromatic solvent, aliphatic hydrocarbon solvent, ether solvent, amide solvent, halogenated hydrocarbon solvent and the like. These may be used in a mixture with water at an appropriate ratio. Of these, alcohol solvent is preferable.

The reaction temperature is generally -70˚C to 150˚C, preferably -20˚C to 100˚C.

The reaction time is generally 0.1 to 100 hr, preferably 0.1 to 40 hr.

Compound (XVI) can be produced, for example, by deprotection of the amino-protecting group of compound (XV).

This reaction can be performed in the same manner as in the aforementioned deprotection of the compound (IVa).

Compound (XVII) can be produced, for example, by an alkylation reaction of compound (XVI).

The alkylation reaction can be performed by a method known per *se,* for example, the method described in Journal of Medicinal Chemistry (J. Med. Chem.), pages 2439-2441, 1998, and the like, or a method according thereto.

This reaction can be performed by reacting compound (XVI) with an alkylating agent in the presence of a base in an inert solvent.

Examples of the above-mentioned "alkylating agent" include alkyl halides (e.g., benzyl 3-bromopropyl ether) and the like. The amount of the "alkylating agent" to be used is generally 1 to 5 equivalents, preferably 1 to 1.5 equivalents, relative to compound (XVI).

Examples of the above-mentioned "inert solvent" include ether solvent, aromatic solvent, aliphatic hydrocarbon solvent and the like. Two or more kinds of these may be used in a mixture at an appropriate ratio. Of these, ether solvent and the like are preferable.

Examples of the above-mentioned "base" include "alkali metal hydride or alkaline earth metal hydride", "metal amide", "alkyl metal", "aryl metal" and the like. The amount of the "base" to be used is generally 1 to 10 equivalents, preferably 1 to 1.5 equivalents, relative to compound (XVI).

The reaction temperature is generally -100˚C to 150˚C, preferably -78˚C to 100˚C.

The reaction time is generally 5 min to 48 hr, preferably 30 min to 24 hr.

Compound (XVIII) can be produced, for example, by an alkylation reaction of compound (XVII).

The alkylation reaction can be performed by a method known per se, for example, the method described in

Journal of Organic Chemistry (J. Org. Chem.), pages 2441-2450, 2004, and the like, or a method according thereto.
This reaction can be performed by reacting compound (XVII) with compound (IX) in the presence of a base in an inert solvent.
The amount of compound (IX) to be used is generally 1 to 5 equivalents, preferably 1 to 1.5 equivalents, relative to compound (XVII).
Examples of the above-mentioned "inert solvent" include nitrile solvent, amide solvent, halogenated hydrocarbon solvent, ether solvent and the like. Two or more kinds of these may be used in a mixture at an appropriate ratio. Of these, THF, DMF and the like are preferable.
Examples of the above-mentioned "base" include "alkali metal hydride or alkaline earth metal hydride" and the like. The amount of the "base" to be used is generally 1 to 10 equivalents, preferably 1 to 1.5 equivalents, relative to compound (XVII).
The reaction temperature is generally -100˚C to 150˚C, preferably 0˚C to 100˚C.
The reaction time is generally 5 min to 48 hr, preferably 30 min to 24 hr.
Compound (XIX) can be produced, for example, by a reduction reaction of compound (XVII).
This reaction can be performed by reacting compound (XVII) in the presence of a reducing agent in an inert solvent.
Examples of the above-mentioned "reducing agent" include metal hydrogen compounds (e.g., sodium bis(2-methoxyethoxy)aluminum hydride, diisobutylaluminum hydride), metal hydrogen complex compounds (e.g., sodium borohydride, lithium borohydride, lithium aluminum hydride, sodium aluminum hydride) and the like. The amount of the "reducing agent" to be used is generally 0.1 to 20 equivalents, preferably 1 to 5 equivalents, relative to compound (XVII).
Examples of the above-mentioned "inert solvent" include alcohol solvent, aromatic solvent, aliphatic hydrocarbon solvent, ether solvent, ester solvent, amide solvent and the like. Two or more kinds of these may be used in a mixture at an appropriate ratio.
The reaction temperature is generally -70˚C to 150˚C, preferably -20˚C to 100˚C.
The reaction time is generally 0.1 to 100 hr, preferably 0.1 to 40 hr.
Compound (XIX) can also be produced from compound (XVIII) by a method similar to the method for producing compound (XIX) from compound (XVII).
Compound (XIXa) can be produced, for example, by a substitution reaction of compound (XIX).
This reaction is performed by converting compound (XIX) to an active derivative with a hydroxy group activator and then reacting the derivative with a nitrogen nucleophile in an inert solvent. Where necessary, this reaction may be performed in the presence of 1 to 5 equivalents of a base and the like.
Examples of the above-mentioned "hydroxy group activator" include methanesulfonyl chloride, p-toluenesulfonyl chloride and the like. The amount of the hydroxy group activator to be used is generally 1 to 10 equivalents, preferably 1 to 1.5 equivalents, relative to compound (XIX).
Examples of the above-mentioned "nitrogen nucleophile" include sodium azide, lithium azide, diphenylphosphoryl azide and the like. The amount of the "nitrogen nucleophile" to be used is generally 1 to 10 equivalents, preferably 1 to 5 equivalents, relative to compound (XIX).
Examples of the above-mentioned "base" include "aromatic amine", "tertiary amine" and the like.
Examples of the above-mentioned "inert solvent" include aromatic solvent, aliphatic hydrocarbon solvent, ether solvent, ester solvent, amide solvent and the like. Two or more kinds of these may be used in a mixture at an appropriate ratio.
The reaction temperature is generally -70˚C to 150˚C, preferably -20˚C to 100˚C, for any reaction.
The reaction time is generally 0.1 to 100 hr, preferably 0.1 to 40 hr, for any reaction.
Compound (XIXb) can be produced, for example, by a reduction reaction of compound (XIXa).
This reaction can be performed in the same manner as in the above-mentioned reduction reaction of compound (IIIa).
Compound (XX) can be produced, for example, by a substitution reaction of compound (XIX).
This reaction is performed by converting compound (XIX) to an active derivative with a hydroxy group activator and then reacting the derivative with a nitrogen nucleophile in an inert solvent. Where necessary, this reaction may be performed in the presence of 1 to 5 equivalents of a base and the like.
Examples of the above-mentioned "hydroxy group activator" include cyanomethylene tri-n-butylphosphoran, diethyl azodicarboxylate and triphenylphosphine, and the like. The amount of the "hydroxy group activator" to be used is generally 1 to 10 equivalents, preferably 1 to 1.5 equivalents, relative to compound (XIX).
Examples of the above-mentioned "nitrogen nucleophile" include nitrobenzenesulfonamide, p-toluenesulfonamide and the like. The amount of the "nitrogen nucleophile" to be used is generally 1 to 10 equivalents, preferably 1 to 5 equivalents, relative to compound (XIX).
Examples of the above-mentioned "base" include "aromatic amine", "tertiary amine" and the like.
Examples of the above-mentioned "inert solvent" include aromatic solvent, aliphatic hydrocarbon solvent, ether

solvent, ester solvent, amide solvent and the like. Two or more kinds of these may be used in a mixture at an appropriate ratio.

The reaction temperature is generally -70°C to 150°C, preferably -20°C to 100°C, for any reaction.

The reaction time is generally 0.1 to 100 hr, preferably 0.1 to 40 hr, for any reaction.

Compound (XX) can also be produced, for example, by a protection reaction of compound (XIXb).

Protection can be performed according to a method known *per se,* for example, a method according to the method described in Protective Groups in Organic Synthesis, John Wiley and Sons (1980) and the like.

Specifically, a method using di-tert-butyl dicarbonate, benzyl chloroformate and triethylamine, acetic anhydride and pyridine, and the like can be employed.

Compound (XXI) can be produced, for example, by deprotection of compound (XX).

This reaction can be performed in the same manner as in the aforementioned deprotection of the compound (IVa).

Compound (XXII) can be produced, for example, by an activation reaction of hydroxy group of compound (XXI).

This reaction is performed by reacting compound (XXI) with a hydroxy group activator in the presence of a base in an inert solvent.

Examples of the above-mentioned "hydroxy group activator" include methanesulfonyl chloride, p-toluenesulfonyl chloride and the like. The amount of the "hydroxy group activator" to be used is generally 1 to 10 equivalents, preferably 1 to 1.5 equivalents, relative to compound (XXI).

Preferable examples of the above-mentioned "base" include "aromatic amine", "tertiary amine" and the like. The amount of the "base" to be used is generally 1 to 10 equivalents, preferably 1 to 1.5 equivalents, relative to compound (XXI).

Examples of the above-mentioned "inert solvent" include aromatic solvent, aliphatic hydrocarbon solvent, ether solvent, ester solvent, amide solvent and the like. Two or more kinds of these may be used in a mixture at an appropriate ratio.

The reaction temperature is generally -70°C to 150°C, preferably -20°C to 100°C.

The reaction time is generally 0.1 to 100 hr, preferably 0.1 to 40 hr.

Compound (XXIV) can be produced, for example, by a Strecker reaction of compound (XXIII).

Compound (XXIII) is commercially easily available, or can also be produced according to a method known per se or a method according thereto.

Strecker reaction can be performed according to a method known *per* se, for example, the method described in Tetrahedron Letters (Tetrahedron Lett.), pages 3285-3288, 1986, and the like, or a method according thereto.

This reaction can be performed by reacting compound (XXXIII), ammonia and a cyanating agent in the presence of an acid in an inert solvent.

Examples of the above-mentioned "cyanating agent" include sodium cyanide, potassium cyanide, trimethylsilyl cyanide and the like. The amount of the "cyanating agent" to be used is generally 1 to 10 equivalents, preferably 1 to 5 equivalents, relative to compound (XXIII).

Preferable examples of the above-mentioned "acid" include acetic acid, ammonium chloride and the like. The amount of the "acid" to be used is generally 1 to 10 equivalents, preferably 1 to 1.5 equivalents, relative to compound (XXIII).

Examples of the above-mentioned "inert solvent" include alcohol solvent, aromatic solvent, aliphatic hydrocarbon solvent, ether solvent, ester solvent, amide solvent and the like. Two or more kinds of these may be used in a mixture at an appropriate ratio.

The reaction temperature is generally -70°C to 150°C, preferably -20°C to 100°C.

The reaction time is generally 0.1 to 100 hr, preferably 0.1 to 40 hr.

Compound (XXV) can be produced, for example, by an acylation reaction of compound (XXIV).

The above-mentioned "acylation reaction" includes the following "method using a dehydrating condensation agent", "method using a reactive derivative of carboxylic acid", and the like.

i) Method using a dehydrating condensation agent

The method can be performed by reacting compound (XXIV) with an organic acid in the presence of a dehydrating condensation agent in an inert solvent. Where necessary, the reaction may be performed in the presence of a catalytic amount to 5 equivalents of 1-hydroxybenzotriazole (HOBt), a catalytic amount to 5 equivalents of a base, and the like. The amount of the organic acid to be used is generally 1 to 5 equivalents, preferably 1 to 1.5 equivalents, relative to compound (XXIV).

Examples of the above-mentioned "organic acid" include formic acid, acetic acid, propionic acid and the like.

Examples of the above-mentioned "dehydrating condensation agent" include dicyclohexylcarbodiimide (DCC), 1-ethyl-3-(3-dimethylaminopropyl)carbodiimide hydrochloride (EDC·HCl) and the like. Of these, EDC·HCl is preferable.

The amount of the "dehydrating condensation agent" to be used is generally 1 to 10 equivalents, preferably 1 to 5 equivalents, relative to compound (XXIV).

Examples of the above-mentioned "inert solvent" include nitrile solvent, amide solvent, halogenated hydrocarbon solvent, ether solvent and the like. Two or more kinds of these may be used in a mixture at an appropriate ratio. Of these, amide solvent is preferable.

Examples of the above-mentioned "base" include "aromatic amine", "tertiary amine" and the like.

The reaction temperature is generally -70˚C to 150˚C, preferably -20˚C to 100˚C.

The reaction time is generally 0.1 to 100 hr, preferably 1 to 48 hr.

ii) Method using a reactive derivative of carboxylic acid

Compound (XXIV) is reacted with 1 to 5 equivalents (preferably 1 to 3 equivalents) of the reactive derivative in an inert solvent. Where necessary, the reaction may be performed in the presence of 1 to 10 equivalents, preferably 1 to 3 equivalents, of a base.

Examples of the above-mentioned "reactive derivative" include acid halide (e.g., acid chloride, acid bromide), mixed acid anhydride (e.g., acid anhydride with $C_{1-6}$ alkyl-carboxylic acid, $C_{6-10}$ aryl-carboxylic acid, $C_{1-6}$ alkyl carbonic acid, etc.), active ester (e.g., ester with phenol optionally having substituent(s), HOBt, N-hydroxysuccinimide, etc.) and the like.

Examples of the "substituent(s)" of the above-mentioned "phenol optionally having substituent(s)" include those exemplified as the substituent for the "cyclic group" of the above-mentioned "optionally substituted cyclic group" for E.

Specific examples of the "phenol optionally having substituent(s)" include phenol, pentachlorophenol, pentafluorophenol, p-nitrophenol and the like.

The reactive derivative is preferably an acid halide.

Examples of the above-mentioned "inert solvent" include ether solvent, halogenated hydrocarbon solvent, aromatic solvent, aliphatic hydrocarbon solvent, nitrile solvent, amide solvent, ketone solvent, sulfoxide solvent, water and the like. Two or more kinds of these may be used in a mixture at an appropriate ratio. Of these, acetonitrile, THF, dichloromethane, chloroform and the like are preferable.

Preferable examples of the above-mentioned "base" include "aromatic amine", "tertiary amine" and the like.

The reaction temperature is generally -20˚C to 100˚C, preferably -20˚C to 50˚C.

The reaction time is generally 5 min to 40 hr, preferably 30 min to 18 hr.

Compound (XXVI) can be produced, for example, by a deprotection of compound (XXV).

This reaction can be performed in the same manner as in the aforementioned deprotection of the compound (IVa).

Compound (XXVII) can be produced, for example, by a coupling reaction of compound (XXVI) with compound (VIa).

This reaction can be performed in the same manner as in the aforementioned coupling reaction of compound (VII) with compound (VIa).

Compound (XXVII) wherein A is N can also be produced by reacting compound (XXVI) wherein A' is N with compound (VIa) wherein X" is a halogen atom, a sulfonylated hydroxy group and the like in the presence of a base and a transition metal catalyst in an inert solvent.

The amount of compound (VIa) to be used is generally 1 to 3 equivalents, preferably 1 to 1.5 equivalents, relative to compound (XXVI).

Examples of the above-mentioned "base" include "inorganic base", "basic salt", "metal alkoxide" and the like. The amount of the "base" to be used is generally 0.1 to 20 equivalents, preferably 1 to 5 equivalents, relative to compound (XXVI).

Examples of the above-mentioned "transition metal catalyst" include palladium catalyst, nickel catalyst and the like. Examples of the "palladium catalyst" include tetrakis(triphenylphosphine)palladium(0), palladium acetate, bis(triphenylphosphine)palladium(II) chloride, palladium-carbon and the like. Examples of the "nickel catalyst" include tetrakis (triphenylphosphine)nickel(0) and the like. The amount of the "transition metal catalyst" to be used is generally about 0.01 to 1 equivalent, preferably about 0.01 to 0.5 equivalent, relative to compound (XXVI).

Examples of the above-mentioned "inert solvent" include water, alcohol solvent, aromatic solvent and the like. Two or more kinds of these may be used in a mixture at an appropriate ratio.

The reaction temperature is generally -78˚C to 150˚C, preferably 0˚C to 50˚C.

The reaction time is generally 5 min to 48 hr, preferably 30 min to 24 hr.

Compound (XXIX) can be produced, for example, by a cyanation reaction of compound (XXVIII).

Compound (XXVIII) is commercially easily available, or can also be produced according to a method known per se or a method according thereto.

The cyanation reaction can be performed by a method known *per se,* for example, the method described in

Journal of Medicinal Chemistry (J. Med. Chem.), pages 486-491, 1988 and the like, or a method according thereto.

This reaction is performed by reacting compound (XXVIII) with a cyanating agent in an inert solvent. Where necessary, the reaction may be performed in the presence of 1 to 10 equivalents of an acid.

Examples of the above-mentioned "cyanating agent" include sodium cyanide, potassium cyanide, trimethylsilyl cyanide and the like. The amount of the "cyanating agent" to be used is generally 1 to 10 equivalents, preferably 1 to 1.5 equivalents, relative to compound (XXVIII).

Examples of the above-mentioned "acid" include organic acid (e.g., formic acid, acetic acid) and Lewis acid (aluminum chloride, boron trifluoride diethyl etherate, zinc iodide).

Examples of the above-mentioned "inert solvent" include nitrile solvent, amide solvent, halogenated hydrocarbon solvent, ether solvent and the like. Two or more kinds of these may be used in a mixture at an appropriate ratio. Of these, halogenated hydrocarbon solvent is preferable.

The reaction temperature is generally -100˚C to 150˚C, preferably 0˚C to 100˚C.

The reaction time is generally 5 min to 48 hr, preferably 30 min to 24 hr.

Compound (XXX) can be produced, for example, by deprotection of compound (IIIj).

This reaction can be performed in the same manner as in the aforementioned deprotection of the compound (IVa).

Compound (VIIIb) can be produced, for example, by a coupling reaction of compound (XXX) with compound (VIa).

This reaction can be performed in the same manner as in the aforementioned coupling reaction of compound (VII) with compound (VIa).

Compound (XXXIII) can be produced, for example, by a reaction between compound (II) and compound (XXXII).

Compound (XXXII) is commercially easily available, or can also be produced according to a method known per se or a method according thereto.

This reaction is performed by reacting compound (II) with compound (XXXII) in the presence of a base in an inert solvent.

The amount of compound (XXXII) to be used is generally 1 to 5 equivalents, preferably 1 to 1.5 equivalents, relative to compound (II).

Examples of the above-mentioned "inert solvent" include ether solvent, aromatic solvent, aliphatic hydrocarbon solvent and the like. Two or more kinds of these may be used in a mixture at an appropriate ratio. Of these, ether solvent and the like are preferable.

Examples of the above-mentioned "base" include "alkali metal hydride or alkaline earth metal hydride", "metal amide", "alkyl metal", "aryl metal" and the like. The amount of the "base" to be used is generally 1 to 10 equivalents, preferably 1 to 1.5 equivalents, relative to compound (II).

The reaction temperature is generally -100˚C to 150˚C, preferably -78˚C to 100˚C.

The reaction time is generally 5 min to 48 hr, preferably 30 min to 24 hr.

Compound (Ia) can be produced, for example, by a coupling reaction of compound (V) with compound (VIa).

This reaction can be performed in the same manner as in the aforementioned coupling reaction of compound (VII) with compound (VIa).

Compounds (Ia) wherein A is N can also be produced by reacting compound (V) wherein A' is N with compound (VIa) wherein X" is a halogen atom, a sulfonylated hydroxy group and the like in the presence of a base and a transition metal catalyst in an inert solvent.

This reaction can be performed in the same manner as in the aforementioned reaction of the "compound (XXVI) wherein A' is N" with "compound (VIa) wherein X" is a halogen atom, a sulfonylated hydroxy group and the like".

Compound (Ia) can also be produced, for example, by cyclization reaction of compound (VIIIb).

This reaction can be performed in the same manner as in the aforementioned cyclization reaction of compound (IIIj).

Compound (Ib) can be produced, for example, by a cyclization reaction of compound (XXVII).

This reaction can be performed in the same manner as in the aforementioned cyclization reaction of compound (XXV).

Compound (Ic) can be produced, for example, by an alkylation reaction of compound (Ib).

This reaction can be performed in the same manner as in the aforementioned alkylation reaction of compound (IVa) to produce compound (IVb).

Compound (I) can be produced, for example, by an acylation reaction of compound (XIa).

This reaction can be performed in the same manner as in the aforementioned amidation reaction of compound (IIIg).

Compound (I) can be produced, for example, by a coupling reaction of compound (V) with compound (VI).

This reaction can be performed in the same manner as in the aforementioned coupling reaction of compound (VII) with compound (VIa).

Compound (I) wherein A is N can be produced by reacting compound (V) wherein A' is N with compound (VI) wherein X" is a halogen atom, a sulfonylated hydroxy group and the like in the presence of a base and a transition metal catalyst in an inert solvent.

This reaction can be performed in the same manner as in the aforementioned reaction of the "compound (XXVI) wherein A' is N" and "compound (VIa) wherein X" is a halogen atom, a sulfonylated hydroxy group and the like".

Compound (I) can also be produced, for example, by cyclization reaction of compound (VIII).

This reaction can be performed in the same manner as in the aforementioned cyclization reaction of compound (IIIh).

Compound (I) can also be produced, for example, by a coupling reaction of compound (Va) with compound (VI).

This reaction can be performed in the same manner as in the aforementioned coupling reaction of compound (VII) with compound (VIa).

In compound (I) thus obtained, a functional group within a molecule can also be converted to a desired functional group by a combination of chemical reactions known per *se.* Examples of the chemical reaction here include oxidation reaction, reduction reaction, alkylation reaction, hydrolysis reaction, amination reaction, esterification reaction, aryl coupling reaction, deprotection reaction and the like.

In the aforementioned production methods, when the starting compound has amino group, carboxyl group, hydroxy group or carbonyl group as a substituent, a protecting group generally used in peptide chemistry and the like may be introduced into these groups. By removing the protecting group as necessary after the reaction, the object compound can be obtained.

Examples of the amino-protecting group include formyl group, $C_{1-6}$ alkyl-carbonyl groups, $C_{1-6}$ alkoxy-carbonyl groups, benzoyl group, $C_{7-10}$ aralkyl-carbonyl groups (e.g., benzylcarbonyl), $C_{7-14}$ aralkyloxy-carbonyl groups (e.g., benzyloxycarbonyl, 9-fluorenylmethoxycarbonyl), trityl group, phthaloyl group, N,N-dimethylaminomethylene group, substituted silyl groups (e.g., trimethylsilyl, triethylsilyl, dimethylphenylsilyl, tert-butyldimethylsilyl, tert-butyldiethylsilyl), $C_{2-6}$ alkenyl groups (e.g., 1-allyl) and the like. These groups may be substituted with 1 to 3 substituents selected from halogen atoms, $C_{1-6}$ alkoxy groups and nitro group.

Examples of the carboxyl-protecting group include $C_{1-6}$ alkyl groups, $C_{7-11}$ aralkyl groups (e.g., benzyl), phenyl group, trityl group, substituted silyl groups (e.g., trimethylsilyl, triethylsilyl, dimethylphenylsilyl, tert-butyldimethylsilyl, tert-butyldiethylsilyl), $C_{2-6}$ alkenyl groups (e.g., 1-allyl) and the like. These groups may be substituted with 1 to 3 substituents selected from halogen atoms, $C_{1-6}$ alkoxy groups and nitro group.

Examples of the hydroxy-protecting group include $C_{1-6}$ alkyl groups, phenyl group, trityl group, $C_{7-10}$ aralkyl groups (e.g., benzyl), formyl group, $C_{l-6}$ alkyl-carbonyl groups, benzoyl group, $C_{7-10}$ aralkyl-carbonyl groups (e.g., benzylcarbonyl), 2-tetrahydropyranyl group, 2-tetrahydrofuranyl group, substitution silyl groups (e.g., trimethylsilyl, triethylsilyl, dimethylphenylsilyl, tert-butyldimethylsilyl, tert-butyldiethylsilyl), $C_{2-6}$ alkenyl group (e.g., 1-allyl) and the like. These groups may be substituted with 1 to 3 substituents selected from halogen atoms, $C_{1-6}$ alkyl groups, $C_{1-6}$ alkoxy groups and nitro group.

Examples of the carbonyl-protecting group include cyclic acetals (e.g., 1,3-dioxane), noncyclic acetals (e.g., di-$C_{1-6}$ alkylacetals) and the like.

The method for removal of the aforementioned protecting groups may be a method known per se, for example, a method according to the method described in Protective Groups in Organic Synthesis, John Wiley and Sons (1980), and the like. Specifically, a method using an acid, a base, ultraviolet rays, hydrazine, phenylhydrazine, sodium N-methyldithiocarbamate, tetrabutylammonium fluoride, palladium acetate, trialkylsilyl halide (e.g., trimethylsilyl iodide, trimethylsilyl bromide) and the like, a reduction method and the like.

Compound (I) obtained by the above-mentioned production methods can be isolated and purified by a known means, for example, solvent extraction, liquid conversion, phase transfer, crystallization, recrystallization, chromatography and the like.

When compound (I) contains an optical isomer, a stereoisomer, a regioisomer or a rotamer, these are also encompassed in compound (I), and can be obtained as a single product according to synthesis and separation methods known per *se.* For example, when compound (I) has an optical isomer, an optical isomer resolved from this compound is also encompassed in compound (I).

The optical isomer can be produced by a method known per *se*.

Compound (I) may be a crystal.

Crystals of compound (I) (hereinafter sometimes to be abbreviated as the crystals of the present invention) can be produced by crystallization according to crystallization methods known per se.

In the present specification, the melting point means that measured using, for example, a micromelting point apparatus (Yanako, MP-500D or Buchi, B-545) or a DSC (differential scanning calorimetry) device (SEIKO, EXSTAR6000) and the like.

In general, the melting points vary depending on the measurement apparatuses, the measurement conditions and the like. The crystal in the present specification may show different values from the melting point described in the

present specification, as long as they are within each of a general error range.

The crystal of the present invention is superior in physicochemical properties (e.g., melting point, solubility, stability) and biological properties (e.g., pharmacokinetics (absorption, distribution, metabolism, excretion), efficacy expression), and thus it is extremely useful as a medicament.

## Examples

The present invention is explained in more detail in the following by referring to Reference Examples, Examples, Experimental Examples and Preparation Examples, which are not to be construed as limitative, and may be modified within the range not deviated from the present invention.

In the Reference Examples and Examples, the abbreviations mean the following.

s: singlet, d: doublet, t: triplet, q: quartet, m: multiplet, br: broad, J: coupling constant

In the Reference Examples and Examples, % means wt% unless otherwise specified.

Reference Example 1

3-ethyl 1-tert-butyl 3-(cyanomethyl)piperidine-1,3-dicarboxylate

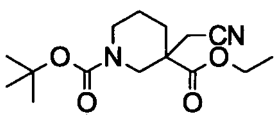

To a solution of 3-ethyl 1-tert-butyl piperidine-1,3-dicarboxylate (10.0 g, 38.9 mmol) in THF (100 mL) was added a 1.0 M lithium bis(trimethylsilyl)amide-THF solution (50.0 mL, 50.0 mmol) at -78˚C, and the mixture was stirred at the same temperature for 30 min. To this solution was added bromoacetonitrile (3.20 mL, 46.7 mmol) at -78˚C, and the mixture was warmed to room temperature and stirred for 30 min. The reaction mixture was dissolved in ethyl acetate, washed with saturated aqueous ammonium chloride solution and saturated brine, and dried over anhydrous magnesium sulfate. The solvent was evaporated under reduced pressure, and the obtained residue was purified by silica gel column chromatography (developing solvent; hexane:ethyl acetate=4:1→ 1:1) to give the title compound (3.02 g, yield 26%) as an oil. This was used in the next step without further purification.

Reference Example 2

tert-butyl 1-oxo-2,7-diazaspiro[4.5]decane-7-carboxylate

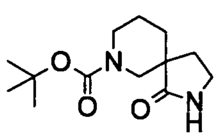

To 3-ethyl 1-tert-butyl 3-(cyanomethyl)piperidine-1,3-dicarboxylate (3.02 g, 10.2 mmol) obtained in Reference Example 1 and Raney nickel (8.30 g) were added 25% aqueous ammonia (5.0 mL) and ethanol (20 mL), and the mixture was stirred under 0.5 MPa hydrogen atmosphere at room temperature for 6 hr. The reaction mixture was filtrated, the filtrate was concentrated under reduced pressure, and the obtained residue was purified by silica gel column chromatography (developing solvent; hexane:ethyl acetate=1:9→0:1) to give the title compound (2.08 g, yield 80%) as an oil.
EI(pos) 255 $[M+H]^+$

Reference Example 3

tert-butyl 2-ethyl-1-oxo-2,7-diazaspiro[4.5]decane-7-carboxylate

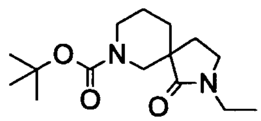

To a solution of tert-butyl 1-oxo-2,7-diazaspiro[4.5]decane-7-carboxylate (2.35 g, 9.24 mmol) obtained in Reference Example 2 in THF (30 mL) was added sodium hydride (60% in oil, 0.560 g, 13.9 mmol) and the mixture was stirred at room temperature for 30 min. To this solution was added ethyl iodide (1.50 mL, 18.5 mmol) and the mixture was stirred at 70˚C for 1 hr. The reaction mixture was diluted with ethyl acetate, washed with saturated aqueous ammonium chloride solution, and dried over anhydrous magnesium sulfate. The solvent was evaporated under reduced pressure, and the obtained residue was purified by silica gel column chromatography (developing solvent; hexane:ethyl acetate=1:3→ 0:1) to give the title compound (2.27 g, yield 87%) as an oil. EI(pos) 227 $[M-tBu]^+$

Reference Example 4

tert-butyl 4-(2-ethyl-1-oxo-2,7-diazaspiro[4.5]dec-7-yl)piperidine-1-carboxylate

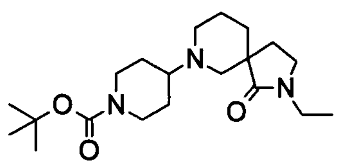

A solution (20 mL) of tert-butyl 2-ethyl-1-oxo-2,7-diazaspiro[4.5]decane-7-carboxylate (2.27 g, 8.04 mmol) obtained in Reference Example 3 in 4M hydrogen chloride-ethyl acetate was stirred at room temperature for 30 min. The solvent was evaporated under reduced pressure, and the residue was dissolved in saturated aqueous sodium hydrogencarbonate solution, and extracted 10 times with ethyl acetate-THF (1:1). After drying over anhydrous magnesium sulfate, the solvent was evaporated under reduced pressure, and a solution of the obtained residue, tert-butyl 4-oxopiperidine-1-carboxylate (1.92 g, 9.65 mmol) and sodium triacetoxyborohydride (5.11 g, 24.1 mmol) in THF (20 mL) was stirred at room temperature for 18 hr. The reaction mixture was diluted with ethyl acetate, washed with saturated aqueous sodium hydrogencarbonate solution, and dried over anhydrous magnesium sulfate. The solvent was evaporated under reduced pressure, and the obtained residue was purified by silica gel column chromatography (developing solvent; methanol:ethyl acetate=0:1 →1:4) to give the title compound (0.650 g, yield 22%) as an oil.
EI(pos) 366 $[M+H]^+$

Reference Example 5

3-ethyl 1-tert-butyl 3-(2-cyanoethyl)piperidine-1,3-dicarboxylate

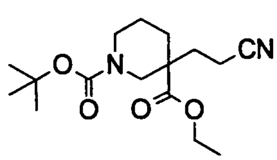

Using 3-ethyl 1-tert-butyl piperidine-1,3-dicarboxylate (10.0 g, 38.9 mmol) and 3-bromopropionitrile (4.80 mL, 58.4 mmol), the title compound (3.85 g, yield 32%) was obtained as an oil by an operation similar to that of Reference Example 1. This was used in the next step without further purification.

Reference Example 6

tert-butyl 7-oxo-2,8-diazaspiro[5.5]undecane-2-carboxylate

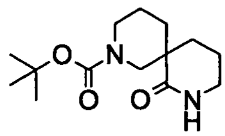

Using 3-ethyl 1-tert-butyl 3-(2-cyanoethyl)piperidine-1,3-dicarboxylate (3.85 g, 12.4 mmol) obtained in Reference Example 5, the title compound (0.230 g, yield 6.9%) was obtained as an oil by an operation similar to that of Reference Example 2.
EI(pos) 213 $[M-tBu]^+$

Reference Example 7

tert-butyl 8-ethyl-7-oxo-2,8-diazaspiro[5.5]undecane-2-carboxylate

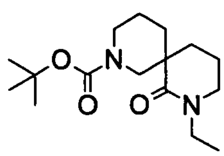

Using tert-butyl 7-oxo-2,8-diazaspiro[5.5]undecane-2-carboxylate (0.230 g, 0.858 mmol) obtained in Reference Example 6, the title compound (0.250 g, yield 98%) was obtained as an oil by an operation similar to that of Reference Example 3.
EI(pos) 241 $[M-tBu]^+$

Reference Example 8

1-(9-anthrylcarbonyl)piperidine-4-one

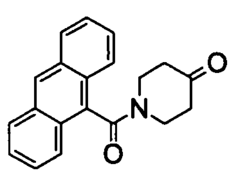

A solution of anthracene-9-carboxylic acid (3.00 g, 13.5 mmol), piperidine-4-one hydrochloride (2.20 g, 16.2 mmol), triethylamine (2.30 mL, 16.2 mmol), 1-ethyl-3-(3'-dimethylaminopropyl)carbodiimide hydrochloride (3.37 g, 17.6 mmol), 1-hydroxybenzotriazole (2.39 g, 17.6 mmol) in DMF (50 mL) was stirred at room temperature for 19 hr. The

reaction mixture was diluted with ethyl acetate, washed 3 times with saturated brine, and dried over anhydrous magnesium sulfate. The solvent was evaporated under reduced pressure, and the obtained residue was purified by silica gel column chromatography (developing solvent; hexane:ethyl acetate=4:1→ 0:1) to give the title compound (2.08 g, yield 51%) as a green solid.
EI (pos) 304 $[M+H]^+$

Reference Example 9

3-ethyl 1-tert-butyl 3-(1-cyanoethyl)piperidine-1,3-dicarboxylate

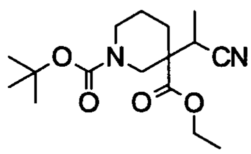

Using 3-ethyl 1-tert-butyl piperidine-1,3-dicarboxylate (10.8 g, 41.9 mmol) and 2-bromopropionitrile (5.40 mL, 62.9 mmol), the title compound (3.10 g, yield 23%) was obtained as an oil by an operation similar to that of Reference Example 1. EI(pos) 333 $[M+Na]^+$

Reference Example 10

tert-butyl 4-methyl-l-oxo-2,7-diazaspiro[4.5]decane-7-carboxylate

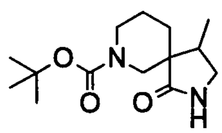

Using 3-ethyl 1-tert-butyl 3-(1-cyanoethyl)piperidine-1,3-dicarboxylate (3.05 g, 9.83 mmol) obtained in Reference Example 9, the title compound (2.15 g, yield 81%) was obtained as an oil by an operation similar to that of Reference Example 2. This was used in the next step without further purification.

Reference Example 11

tert-butyl 2-ethyl-4-methyl-1-oxo-2,7-diazaspiro[4.5]decane-7-carboxylate

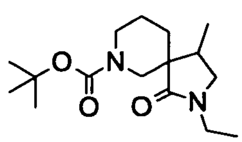

Using tert-butyl 4-methyl-1-oxo-2,7-diazaspiro[4.5]decane-7-carboxylate (1.51 g, 5.63 mmol) obtained in Reference Example 10, the title compound (1.48 g, yield 88%) was obtained as an oil by an operation similar to that of Reference Example 3.
EI(pos) 297 $[M+H]^+$

Reference Example 12

3-ethyl 1-tert-butyl 3-(2-methylprop-2-en-1-yl)piperidine-1,3-dicarboxylate

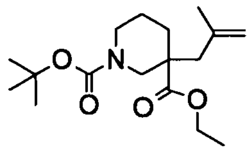

Using 3-ethyl 1-tert-butyl piperidine-1,3-dicarboxylate (10.0 g, 38.9 mmol) and 3-bromo-2-methylpropene (5.00 mL, 50.8 mmol), the title compound (12.1 g, quantitative) was obtained as an oil by an operation similar to that of Reference Example 1.
EI(pos) 334 $[M+Na]^+$

Reference Example 13

3-ethyl 1-tert-butyl 3-(2-oxopropyl)piperidine-1,3-dicarboxylate

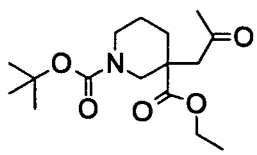

Ozone was passed through a solution of 3-ethyl 1-tert-butyl 3-(2-methylprop-2-en-1-yl)piperidine-1,3-dicarboxylate (12.1 g, 38.9 mmol) obtained in Reference Example 12 in dichloromethane-methanol (50 mL-50 mL) at -78˚C for 1 hr. After passing nitrogen therethrough at the same temperature for 10 min, dimethyl sulfide (8.60 mL, 117 mmol) was added thereto, and the mixture was warmed to room temperature. The solvent was evaporated under reduced pressure, and the obtained residue was purified by silica gel column chromatography (developing solvent; hexane:ethyl acetate=1:1) to give the title compound (12.1 g, quantitative) as an oil.
EI (pos) 314 $[M+H]^+$

Reference Example 14

tert-butyl 2-ethyl-3-methyl-1-oxo-2,7-diazaspiro[4.5]decane-7-carboxylate

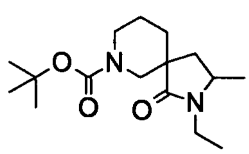

A solution of 3-ethyl 1-tert-butyl 3-(2-oxopropyl)piperidine-1,3-dicarboxylate (3.00 g, 9.58 mmol), a 2.0 M ethylamine-THF solution (14.4 mL, 28.7 mmol), sodium triacetoxyborohydride (6.08 g, 28.7 mmol) obtained in Reference Example 13 in dichloromethane-acetic acid (15 mL-5 mL) was stirred at 40˚C for 24 hr. The reaction mixture was neutralized with aqueous sodium hydroxide solution, and extracted twice with ethyl acetate. The organic layer was dried over anhydrous magnesium sulfate. The solvent was evaporated under reduced pressure, and the obtained residue

was purified by silica gel column chromatography (developing solvent; methanol:ethyl acetate=0:1→1:4) to give the title compound (0.930 g, yield 33%) as an oil.
EI(pos) 297 $[M+H]^+$

Reference Example 15

tert-butyl 3-methyl-1-oxo-2-oxa-7-azaspiro[4.5]decane-7-carboxylate

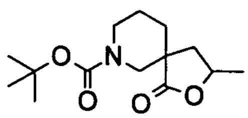

To a solution of 3-ethyl 1-tert-butyl 3-(2-oxopropyl)piperidine-1,3-dicarboxylate (1.50 g, 4.79 mmol) obtained in Reference Example 13 in THF-methanol (20 mL-5 mL) was added sodium borohydride (0.090 g, 2.39 mmol) and the mixture was stirred at room temperature for 1 hr. The reaction mixture was diluted with ethyl acetate, washed with saturated aqueous ammonium chloride solution, and dried over anhydrous magnesium sulfate. The solvent was evaporated under reduced pressure, and the obtained residue was purified by silica gel column chromatography (developing solvent; hexane:ethyl acetate=4:1→1:1) to give the title compound (0.470 g, yield 36%) as an oil.
EI(pos) 270 $[M+H]^+$

Reference Example 16

tert-butyl 2-(cyclopropylmethyl)-3-methyl-1-oxo-2,7-diazaspiro[4.5]decane-7-carboxylate

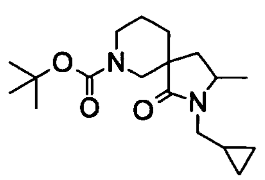

Using 3-ethyl 1-tert-butyl 3-(2-oxopropyl)piperidine-1,3-dicarboxylate (2.00 g, 6.39 mmol) obtained in Reference Example 13 and cyclopropylmethylamine (2.40 g, 33.7 mmol), the title compound (1.01 g, yield 49%) was obtained as an oil by an operation similar to that of Reference Example 14.
EI(pos) 323 $[M+H]^+$

Reference Example 17

tert-butyl 2-(cyanomethyl)-3-methyl-1-oxo-2,7-diazaspiro[4.5]decane-7-carboxylate

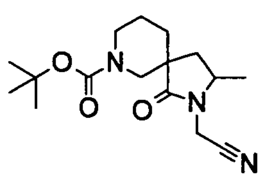

Using 3-ethyl 1-tert-butyl 3-(2-oxopropyl)piperidine-1,3-dicarboxylate (1.50 g, 4.79 mmol) obtained in Reference

Example 13 and cyanomethylamine hydrochloride (1.33 g, 14.4 mmol), the title compound (0.450 g, yield 30%) was obtained as an oil by an operation similar to that of Reference Example 14. EI(pos) 330 [M+Na]$^+$

Reference Example 18

tert-butyl 2-(2-methoxy-2-oxoethyl)-3-methyl-1-oxo-2,7-diazaspiro[4.5]decane-7-carboxylate

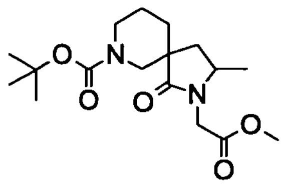

Using 3-ethyl 1-tert-butyl 3-(2-oxopropyl)piperidine-1,3-dicarboxylate (1.50 g, 4.79 mmol) obtained in Reference Example 13 and glycine methyl ester hydrochloride (1.81 g, 14.4 mmol), the title compound (0.610 g, yield 37%) was obtained as an oil by an operation similar to that of Reference Example 14.
EI(pos) 341 [M+H]$^+$

Reference Example 19

tert-butyl 3,3-dimethyl-1-oxo-2-oxa-7-azaspiro[4.5]decane-7-carboxylate

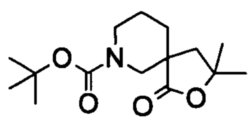

To a solution of 3-ethyl 1-tert-butyl 3-(2-oxopropyl)piperidine-1,3-dicarboxylate (2.00 g, 6.38 mmol) obtained in Reference Example 13 in THF (20 mL) was added a 3.0 M methylmagnesium bromide-THF solution (4.20 mL, 12.6 mmol) and the mixture was stirred at room temperature for 1 hr. The reaction mixture was diluted with ethyl acetate, washed with saturated aqueous ammonium chloride solution, and dried over anhydrous magnesium sulfate. The solvent was evaporated under reduced pressure, and the obtained residue was purified by silica gel column chromatography (developing solvent; hexane:ethyl acetate=7:3→1:1) to give the title compound (0.930 g, yield 51%) as an oil.
EI(pos) 306 [M+Na]$^+$

Reference Example 20

3-methyl 1-tert-butyl 3-(2-methylprop-2-en-1-yl)pyrrolidine-1,3-dicarboxylate

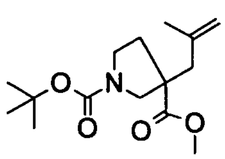

Using 3-methyl 1-tert-butyl pyrrolidine-1,3-dicarboxylate (4.82 g, 21.0 mmol) and 3-bromo-2-methylpropene (2.80 mL, 27.3 mmol), the title compound (3.94 g, yield 66%) was obtained as an oil by an operation similar to that of Reference Example 1. EI(pos) 306 [M+Na]$^+$

Reference Example 21

3-methyl 1-tert-butyl 3-(2-oxopropyl)pyrrolidine-1,3-dicarboxylate

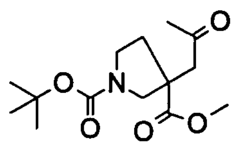

Using 3-methyl 1-tert-butyl 3-(2-methylprop-2-en-1-yl)pyrrolidine-1,3-dicarboxylate (3.94 g, 13.9 mmol) obtained in Reference Example 20, the title compound (3.96 g, quantitative) was obtained as an oil by an operation similar to that of Reference Example 13.
EI(pos) 186 $[M-Boc]^+$

Reference Example 22

tert-butyl 7-ethyl-8-methyl-6-oxo-2,7-diazaspiro[4.4]nonane-2-carboxylate

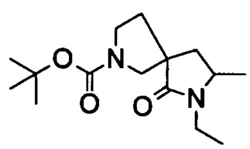

Using 3-methyl 1-tert-butyl 3-(2-oxopropyl)pyrrolidine-1,3-dicarboxylate (2.27 g, 7.96 mmol) obtained in Reference Example 21 and 2.0 M ethylamine-THF solution (12.0 mL, 24.0 mmol), the title compound (1.09 g, yield 48%) was obtained as an oil by an operation similar to that of Reference Example 14. EI(pos) 305 $[M+Na]^+$

Reference Example 23

2-ethyl 4-tert-butyl 2-(2-methylprop-2-en-1-yl)morpholine-2,4-dicarboxylate

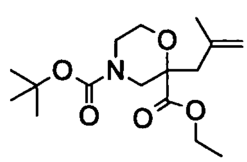

Using 2-ethyl 4-tert-butyl morpholine-2,4-dicarboxylate (3.15 g, 12.1 mmol) and 3-bromo-2-methylpropene (1.60 mL, 15.7 mmol), the title compound (2.50 g, yield 66%) was obtained as an oil by an operation similar to that of Reference Example 1. EI(pos) 336 $[M+Na]^+$

Reference Example 24

2-ethyl 4-tert-butyl 2-(2-oxopropyl)morpholine-2,4-dicarboxylate

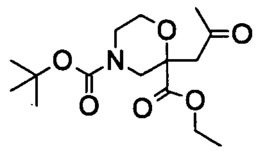

Using 2-ethyl 4-tert-butyl 2-(2-methylprop-2-en-1-yl)morpholine-2,4-dicarboxylate (2.50 g, 7.98 mmol) obtained in Reference Example 23, the title compound (2.47 g, yield 98%) was obtained as an oil by an operation similar to that of Reference Example 13.
EI(pos) 338 $[M+Na]^+$

Reference Example 25

tert-butyl 2-ethyl-3-methyl-1-oxo-6-oxa-2,9-diazaspiro[4.5]decane-9-carboxylate

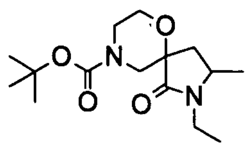

Using 2-ethyl 4-tert-butyl 2-(2-oxopropyl)morpholine-2,4-dicarboxylate (2.47 g, 7.83 mmol) obtained in Reference Example 24 and 2.0 M ethylamine-THF solution (12.0 mL, 24.0 mmol), the title compound (1.60 g, yield 68%) was obtained as an oil by an operation similar to that of Reference Example 14. EI(pos) 321 $[M+Na]^+$

Reference Example 26

tert-butyl 3-methyl-1-oxo-2,7-diazaspiro[4.5]decane-7-carboxylate

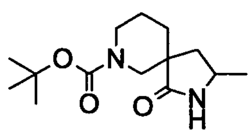

Using 3-ethyl 1-tert-butyl 3-(2-oxopropyl)piperidine-1,3-dicarboxylate (3.00 g, 9.57 mmol) obtained in Reference Example 13 and ammonium acetate (3.69 g, 47.9 mmol), the title compound (1.37 g, yield 53%) was obtained as an oil by an operation similar to that of Reference Example 14. This was used in the next step without further purification.

Reference Example 27

tert-butyl 3-methyl-1-oxo-2-(thiazol-2-ylmethyl)-2,7-diazaspiro[4.5]decane-7-carboxylate

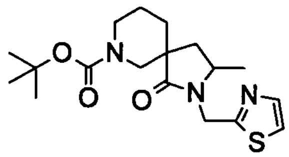

Using 3-ethyl 1-tert-butyl 3-(2-oxopropyl)piperidine-1,3-dicarboxylate (0.780 g, 2.49 mmol) obtained in Reference Example 13 and thiazol-2-ylmethylamine hydrochloride (0.750 g, 4.98 mmol), the title compound (0.250 g, yield 27%) was obtained as an oil by an operation similar to that of Reference Example 14.
EI(pos) 388 $[M+Na]^+$

Reference Example 28

tert-butyl 3-methyl-1-oxo-2-(pyridin-4-ylmethyl)-2,7-diazaspiro[4.5]decane-7-carboxylate

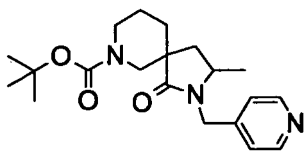

Using 3-ethyl 1-tert-butyl 3-(2-oxopropyl)piperidine-1,3-dicarboxylate (0.750 g, 2.39 mmol) obtained in Reference Example 1 and pyridin-4-ylmethylamine (0.760 mL, 7.47 mmol), the title compound (0.460 g, yield 51%) was obtained as an oil by an operation similar to that of Reference Example 14. EI(pos) 360 $[M+H]^+$

Reference Example 29

tert-butyl 2-(6-methoxy-6-oxohexyl)-3-methyl-1-oxo-2,7-diazaspiro[4.5]decane-7-carboxylate

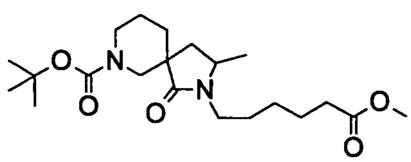

Using 3-ethyl 1-tert-butyl 3-(2-oxopropyl)piperidine-1,3-dicarboxylate (1.50 g, 4.79 mmol) obtained in Reference Example 13 and methyl 6-aminohexanoate hydrochloride (2.61 g, 14.4 mmol), the title compound (0.530 g, yield 28%) was obtained as an oil by an operation similar to that of Reference Example 14.
EI(pos) 419 $[M+Na]^+$

Reference Example 30

tert-butyl 3-methyl-1-oxo-2-[2-(pyrrolidin-1-yl)ethyl]-2,7-diazaspiro[4.5]decane-7-carboxylate

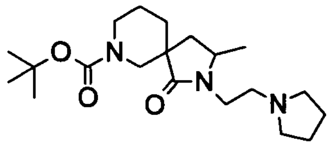

Using 3-ethyl 1-tert-butyl 3-(2-oxopropyl)piperidine-1,3-dicarboxylate (1.50 g, 4.79 mmol) obtained in Reference Example 13 and 2-(pyrrolidin-1-yl)ethylamine (1.64 g, 14.4 mmol), the title compound (0.860 g, yield 49%) was obtained as an oil by an operation similar to that of Reference Example 14. EI(pos) 310 [M-tBu]$^+$

Reference Example 31

3-ethyl 1-tert-butyl 3-[2-(benzyloxy)-2-oxoethyl]piperidine-1,3-dicarboxylate

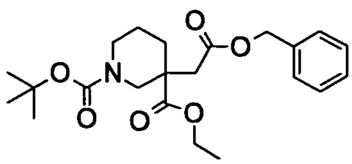

Using 3-ethyl 1-tert-butyl piperidine-1,3-dicarboxylate (10.0 g, 38.9 mmol) and benzyl bromoacetate (7.40 mL, 46.6 mmol), the title compound (12.3 g, yield 78%) was obtained as an oil by an operation similar to that of Reference Example 1. EI(pos) 428 [M+Na]$^+$

Reference Example 32

[1-(tert-butoxycarbonyl)-3-(ethoxycarbonyl)piperidin-3-yl]acetic acid

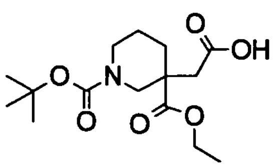

A suspension of 3-ethyl 1-tert-butyl 3-[2-(benzyloxy)-2-oxoethyl]piperidine-1,3-dicarboxylate (12.3 g, 30.3 mmol) obtained in Reference Example 31 and 10% palladium carbon (50% water-containing product, 3.40 g) in methanol (100 mL) was stirred under a hydrogen atmosphere for 45 min. After filtration, the solvent was evaporated under reduced pressure to give the title compound (9.50 g, quantitative) as an oil. EI(pos) 338 [M+Na]$^+$

Reference Example 33

3-ethyl 1-tert-butyl 3-[2-(ethylamino)-2-oxoethyl]piperidine-1,3-dicarboxylate

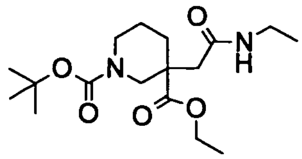

To a solution of [1-(tert-butoxycarbonyl)-3-(ethoxycarbonyl)piperidin-3-yl]acetic acid (5.00 g, 15.8 mmol) obtained in Reference Example 32 and a 2.0 M ethylamine-THF solution (10.0 mL, 20.0 mmol), 1-hydroxybenzotriazole (2.80 g, 20.7 mmol) in DMF (50 mL) was added 1-[3-(dimethylamino)propyl]-3-ethylcarbodiimide hydrochloride (3.97 g, 20.7 mmol) at room temperature, and the mixture was stirred at room temperature for 1 hr. The reaction mixture was dissolved in ethyl acetate, washed with 0.5N hydrochloric acid, aqueous potassium carbonate solution and saturated brine, and dried over anhydrous sodium sulfate. The solvent was evaporated under reduced pressure, and the obtained residue was purified by silica gel column chromatography (developing solvent; hexane:ethyl acetate=1:1→0:1) to give the title compound (2.40 g, yield 44%) as an oil.
EI(pos) 343 $[M+H]^+$

Reference Example 34

ethyl 1'-(9-anthrylcarbonyl)-3-[2-(ethylamino)-2-oxoethyl]-1,4'-bipiperidine-3-carboxylate

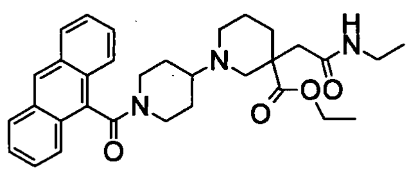

A solution (20 mL) of 3-ethyl 1-tert-butyl 3-[2-(ethylamino)-2-oxoethyl]piperidine-1,3-dicarboxylate (2.40 g, 7.01 mmol) obtained in Reference Example 33 in 4M hydrogen chloride-ethyl acetate was stirred at room temperature for 30 min. The solvent was evaporated under reduced pressure, and a solution of the residue, triethylamine (0.980 mL, 7.01 mmol), 1-(9-anthrylcarbonyl)piperidin-4-one (2.13 g, 7.01 mmol) obtained in Reference Example 8 and sodium triacetoxyborohydride (4.46 g, 21.0 mmol) in dichloromethane (10 mL) was stirred at room temperature for 19 hr. The reaction mixture was diluted with ethyl acetate, washed with saturated aqueous sodium hydrogencarbonate solution, and dried over anhydrous magnesium sulfate. The solvent was evaporated under reduced pressure, and the obtained residue was purified by silica gel column chromatography (developing solvent; methanol:ethyl acetate=0:1→1:4) to give the title compound (1.66 g, yield 44%) as an oil.
EI(pos) 530 $[M+H]^+$

Reference Example 35

2-methyl 4-tert-butyl 1-benzyl 2-(2-methylprop-2-en-1-yl)piperazine-1,2,4-tricarboxylate

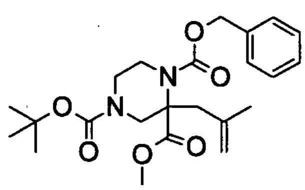

Using 2-methyl 4-tert-butyl 1-benzyl piperazine-1,2,4-tricarboxylate (4.90 g, 10.5 mmol) and 3-bromo-2-methylpropene (1.40 mL, 13.7 mmol), the title compound (1.80 g, yield 40%) was obtained as an oil by an operation similar to that of Reference Example 1.
EI(pos) 455 [M+Na]+

Reference Example 36

2-methyl 4-tert-butyl 1-benzyl 2-(2-oxopropyl)piperazine-1,2,4-tricarboxylate

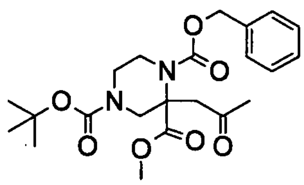

Using 2-methyl 4-tert-butyl 1-benzyl 2-(2-methylprop-2-en-1-yl)piperazine-1,2,4-tricarboxylate (1.80 g, 4.16 mmol) obtained in Reference Example 35, the title compound (1.80 g, yield 99%) was obtained as an oil by an operation similar to that of Reference Example 13.
EI(pos) 457 [M+Na]+

Reference Example 37

9-tert-butyl 6-benzyl 2-ethyl-3-methyl-1-oxo-2,6,9-triazaspiro[4.5]decane-6,9-dicarboxylate

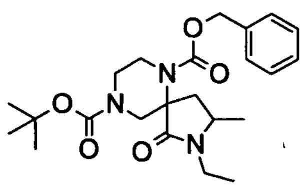

Using 2-methyl 4-tert-butyl 1-benzyl 2-(2-oxopropyl)piperazine-1,2,4-tricarboxylate (1.80 g, 4.14 mmol) obtained in Reference Example 36 and 2.0 M ethylamine-THF solution (6.30 mL, 12.6 mmol), the title compound (0.92 g, yield 51%) was obtained as an oil by an operation similar to that of Reference Example 14.
EI(pos) 432 [M+H]+

Reference Example 38

tert-butyl 2-ethyl-3-methyl-1-oxo-6-(trifluoroacetyl)-2,6,9-triazaspiro[4.5]decane-9-carboxylate

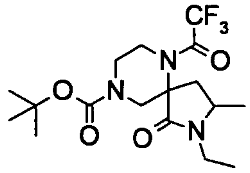

A suspension of 9-tert-butyl 6-benzyl 2-ethyl-3-methyl-1-oxo-2,6,9-triazaspiro[4.5]decane-6,9-dicarboxylate

(0.920 g, 2.13 mmol) obtained in Reference Example 37 and 10% palladium carbon (50% water-containing product, 0.23 g) in methanol (20 mL) was stirred under a hydrogen atmosphere for 30 min. After filtration, the solvent was evaporated under reduced pressure, and the obtained residue was dissolved in THF (20 mL). To this solution was added triethylamine (0.600 mL, 4.26 mmol) and trifluoroacetic anhydride (0.450 mL, 3.20 mmol), and the mixture was stirred at room temperature for 30 min. The reaction mixture was diluted with ethyl acetate, washed successively with saturated aqueous sodium hydrogencarbonate solution and brine, and dried over anhydrous magnesium sulfate. The solvent was evaporated under reduced pressure, and the obtained residue was purified by silica gel column chromatography (developing solvent; hexane:ethyl acetate=1:1→ 1:3) to give the title compound (0.780 g, yield 93%) as an oil. EI(pos) 416 $[M+Na]^+$

Reference Example 39

3-ethyl 1-tert-butyl 3-(2-amino-2-oxoethyl)piperidine-1,3-dicarboxylate

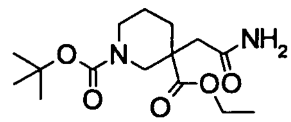

To a solution of [1-(tert-butoxycarbonyl)-3-(ethoxycarbonyl)piperidin-3-yl]acetic acid (5.00 g, 15.8 mmol) obtained in Reference Example 32 and 1-hydroxybenzotriazole ammonium salt (4. 84 g, 31. 8 mmol) in DMF (50 mL), 1-[3-(dimethylamino)propyl]-3-ethylcarbodiimide hydrochloride(6.10 g, 31.8 mmol) was added at room temperature, and the reaction mixture was stirred at room temperature for 12 hr. The reaction mixture was dissolved in ethyl acetate, washed with 0.5N hydrochloric acid, aqueous potassium carbonate solution and saturated brine, and dried over anhydrous sodium sulfate. The solvent was evaporated under reduced pressure, and the obtained residue was purified by silica gel column chromatography (developing solvent; hexane:ethyl acetate=1:3→ 0:1) to give the title compound (1.56 g, yield 31%) as an oil. EI(pos) 315 $[M+H]^+$

Reference Example 40

ethyl 3-(2-amino-2-oxoethyl)-1'-(9-anthrylcarbonyl)-1,4'-bipiperidine-3-carboxylate

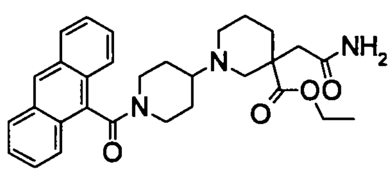

A solution (20 mL) of 3-ethyl 1-tert-butyl 3-(2-amino-2-oxoethyl)piperidine-1,3-dicarboxylate (1.30 g, 4.14 mmol) obtained in Reference Example 39 in 4M hydrogen chloride-ethyl acetate was stirred at room temperature for 30 min. The solvent was evaporated under reduced pressure, and a solution of the residue, triethylamine (0.580 mL, 4.14 mmol), 1-(9-anthrylcarbonyl)piperidine-4-one (1.00 g, 3.29 mmol) obtained in Reference Example 8 and sodium triacetoxyborohydride (2.00 g, 9.39 mmol) in dichloromethane (10 mL) was stirred at room temperature for 20 hr. The reaction mixture was diluted with ethyl acetate, washed with saturated aqueous sodium hydrogencarbonate solution, and dried over anhydrous magnesium sulfate. The solvent was evaporated under reduced pressure, and the obtained residue was purified by silica gel column chromatography (developing solvent; methanol:ethyl acetate=0:1 →1:4) to give the title compound (0.40 g, yield 19%) as an oil. EI(pos) 502 $[M+H]^+$

Reference Example 41

tert-butyl 3-ethyl-3-methyl-1-oxo-2-oxa-7-azaspiro[4.5]decane-7-carboxylate

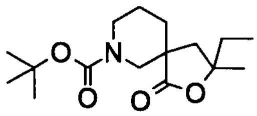

3-Ethyl 1-tert-butyl 3-(2-oxopropyl)piperidine-1,3-dicarboxylate (3.00 g, 9.57 mmol) obtained in Reference Example 13 and a 3.0 M ethyl magnesium bromide-THF solution (3.20 mL, 9.60 mmol) were mixed and stirred at room temperature for 1 hr. The reaction mixture was diluted with ethyl acetate, washed with saturated aqueous ammonium chloride solution, and dried over anhydrous magnesium sulfate. The solvent was evaporated under reduced pressure, and the obtained residue was purified by silica gel column chromatography (developing solvent; hexane:ethyl acetate=20:3→ 3:2) to give the title compound (0.260 g, yield 9.1%) as an oil.
EI(pos) 320 $[M+Na]^+$

Reference Example 42

tert-butyl 3-methyl-1-oxo-2-(pyridin-2-ylmethyl)-2,7-diazaspiro[4.5]decane-7-carboxylate

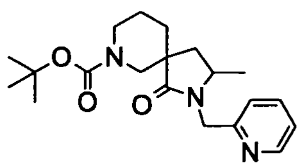

Using 3-ethyl 1-tert-butyl 3-(2-oxopropyl)piperidine-1,3-dicarboxylate (1.00 g, 3.19 mmol) obtained in Reference Example 13 and pyridin-2-ylmethylamine hydrochloride (1.04 g, 9.58 mmol), the title compound (0.700 g, yield 61%) was obtained as an oil by an operation similar to that of Reference Example 14.
EI(pos) 260 $[M-Boc]^+$

Reference Example 43

tert-butyl 3-methyl-1-oxo-2-(pyridin-3-ylmethyl)-2,7-diazaspiro[4.5]decane-7-carboxylate

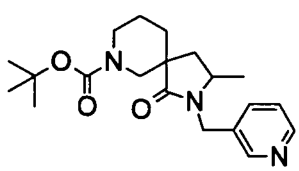

Using 3-ethyl 1-tert-butyl 3-(2-oxopropyl)piperidine-1,3-dicarboxylate (1.00 g, 3.19 mmol) obtained in Reference Example 13 and pyridin-3-ylmethylamine hydrochloride (1.04 g, 9.58 mmol), the title compound (0.510 g, yield 44%) was obtained as an oil by an operation similar to that of Reference Example 14.
EI(pos) 260 $[M-Boc]^+$

Reference Example 44

ethyl 1-benzoyl-3-(2-phenylethyl)piperidine-3-carboxylate

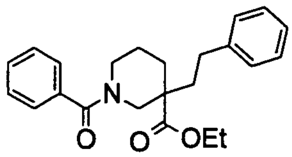

Using ethyl 1-benzoylpiperidine-3-carboxylate (4.18 g, 16.0 mmol) and (2-bromoethyl)benzene (2.40 mL, 17.6 mmol), the title compound (1.04 g, yield 18%) was obtained as an oil by an operation similar to that of Reference Example 1. $^1$H NMR ($CDCl_3$) δ1.26 (3H, t, J = 6 Hz), 1.59-1.83 (5H, m), 2.26-2.52 (3H, m), 3.20-3.47 (3H, m), 4.09-4.50 (3H, m), 7.15-7.20 (3H, m), 7.24-7.26 (2H, m), 7.39 (5H, m).

Reference Example 45

1-benzoyl-3-(2-phenylethyl)piperidine-3-carboxylic acid

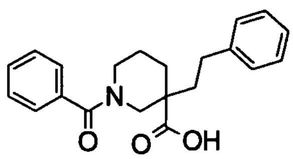

To a solution of ethyl 1-benzoyl-3-(2-phenylethyl)piperidine-3-carboxylate (1.03 g, 2.82 mmol) obtained in Reference Example 44 in ethanol (14 mL) was added 1N aqueous sodium hydroxide solution (5.64 mL, 5.64 mmol), and the mixture was stirred at 90°C for 16 hr. The solvent was evaporated under reduced pressure, the residue was acidified with 1N hydrochloric acid, and the mixture was extracted with ethyl acetate. The extract was washed with saturated brine, and dried over anhydrous sodium sulfate, and the solvent was evaporated under reduced pressure to give the title compound (951 mg, quantitative) as an oil. This was used in the next step without further purification.

Reference Example 46

1'-benzoyl-3,4-dihydro-1H-spiro[naphthalene-2,3'-piperidin]-1-one

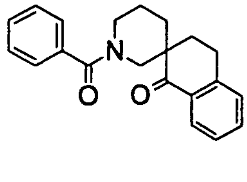

To a solution of 1-benzoyl-3-(2-phenylethyl)piperidine-3-carboxylic acid (951 mg, 2.82 mmol) obtained in Reference Example 45 in dichloroethane (30 mL) was added thionyl chloride (0.247 mL, 3.38 mmol), and the mixture was stirred at room temperature for 30 min. To this solution was added aluminum chloride (939 mg, 7.05 mmol), and the mixture was stirred at room temperature for 2 hr. The reaction mixture was diluted with ethyl acetate, washed with water, potassium carbonate aqueous solution and saturated brine, and dried over anhydrous sodium sulfate. The solvent was

evaporated under reduced pressure, and the obtained residue was purified by silica gel column chromatography (developing solvent; ethyl acetate) to give the title compound (612 mg, yield 68%) as an oil.
$^1$H NMR ($CDCl_3$) δ1.71-1.83 (4H, m), 2.05-2.26 (2H, m), 2.69-2.92 (2H, m), 3.10-3.28 (2H, m), 3.52-3.73 (1H, m), 4.50 (1H, m), 7.26-7.42 (8H, m), 8.03 (1H, m).

Reference Example 47

3,4-dihydro-1H-spiro[naphthalene-2,3'-piperidin]-1-one

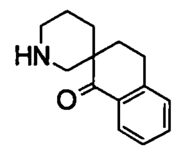

To 1'-benzoyl-3,4-dihydro-1H-spiro[naphthalene-2,3'-piperidin]-1-one (611 mg, 1.91 mmol) obtained in Reference Example 46 was added concentrated hydrochloric acid (8 mL), and the mixture was stirred at 100°C for 2 days. The mixture was diluted with water, washed with diethyl ether, basified with potassium carbonate, and extracted with ethyl acetate. The extract was washed with saturated brine, and dried over anhydrous sodium sulfate. The solvent was evaporated under reduced pressure and the obtained residue was purified by silica gel column chromatography (developing solvent; ethyl acetate) to give the title compound (335 mg, yield 81%) as an oil. This was used in the next step without further purification.

Reference Example 48

tert-butyl 4-(1-oxo-3,4-dihydro-1H,1'H-spiro[naphthalene-2,3'-piperidin]-1'-yl)piperidine-1-carboxylate

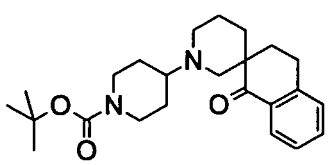

To a solution of 3,4-dihydro-1H-spiro[naphthalene-2,3'-piperidin]-1-one (335 mg, 1.56 mmol) obtained in Reference Example 47 and tert-butyl 4-oxopiperidine-1-carboxylate (310 mg, 1.56 mmol) in tetrahydrofuran (7 mL) was added sodium triacetoxyborohydride (495 mg, 2.33 mmol), and the mixture was stirred at room temperature for 2 days. To the reaction mixture was added ethyl acetate, and the mixture was washed with aqueous potassium carbonate solution and saturated brine, and dried over anhydrous sodium sulfate. The solvent was evaporated under reduced pressure and the obtained residue was purified by silica gel column chromatography (developing solvent; ethyl acetate: methanol=1:0→10:1) to give the title compound (529 mg, yield 85%) as an oil.
$^1$H NMR ($CDCl_3$) δ1.43 (9H, s), 1.43-1.70 (9H, m), 2.05-2.45 (5H, m), 2.69-2.94 (6H, m), 4.11 (1H, m), 7.21-7.26 (2H, m), 7.46 (1H, m), 7.99 (1H, d, J = 9 Hz).

Reference Example 49

1'-(piperidin-4-yl)-3,4-dihydro-1H-spiro[naphthalene-2,3'-piperidin]-1-one

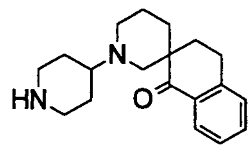

Tert-butyl 4-(1-oxo-3,4-dihydro-1H,1'H-spiro[naphthalene-2,3'-piperidin]-1'-yl)piperidine-1-carboxylate (529 mg, 1.33 mmol) obtained in Reference Example 48 was dissolved in trifluoroacetic acid (7 mL), and the mixture was stirred for 1 hr. The solvent was evaporated under reduced pressure, and the residue was dissolved in ethyl acetate, washed with aqueous potassium carbonate solution and saturated brine, and dried over anhydrous sodium sulfate. The solvent was evaporated under reduced pressure to give the title compound (386 mg, yield 98%) as an oil. This was used in the next step without further purification.

Reference Example 50

2-[(tert-butoxycarbonyl)amino]-1-benzothiophene-3-carboxylic acid

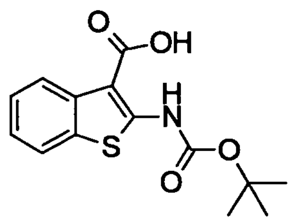

To a solution of ethyl 2-amino-1-benzothiophene-3-carboxylate (1.10 g, 5.0 mmol) in tert-butyl alcohol (10 mL) were added 4-dimethylaminopyridine (catalytic amount) and di-tert-butyl bicarbonate (1.30 g, 6.0 mmol), and the mixture was stirred at 60˚C for 24 hr. The reaction mixture was concentrated under reduced pressure, and the obtained residue was dissolved in ethyl acetate, washed with saturated brine, and dried over anhydrous sodium sulfate. The solvent was evaporated under reduced pressure, the obtained residue was dissolved in ethanol (5 mL). 1N Aqueous sodium hydroxide solution (5 mL) was added and the mixture was stirred at 60˚C for 3 hr. The reaction mixture was adjusted to pH 6 with 0.1N hydrochloric acid at 0˚C. The obtained crystals were collected by filtration to give the title compound (1.25 g, yield 78%). EI(pos) 238 $[M-tBu]^+$

Reference Example 51

2-amino-1-benzothiophene-3-carboxylic acid

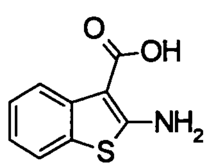

To a solution of ethyl 2-amino-1-benzothiophene-3-carboxylate (0.66 g, 3.0 mmol) in ethanol (10 mL) and tetrahydrofuran (10 mL) was added 1N aqueous sodium hydroxide solution (20 mL, 20 mmol) and the mixture was heated under reflux for 5 hr. The reaction solution was concentrated under reduced pressure, and the obtained residue was adjusted to pH 5 with 1N hydrochloric acid. The obtained crystals were collected by filtration to give the title compound (537 mg, yield 81%).
EI(pos) 194 $[M+H]^+$

Reference Example 52

tert-butyl 4-(3,3-dimethyl-1-oxo-2-oxa-7-azaspiro[4.5]dec-7-yl)piperidine-1-carboxylate

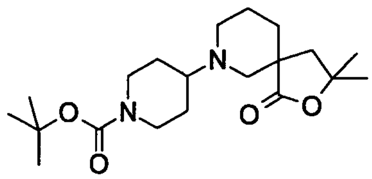

Tert-butyl 3,3-dimethyl-1-oxo-2-oxa-7-azaspiro[4.5]decane-7-carboxylate (19.1 g, 67.4 mmol) obtained in Reference Example 19 or Reference Example 140 was dissolved in 4N hydrogen chloride-ethyl acetate (200 mL), and the mixture was stirred at room temperature for 30 min. The reaction solution was concentrated under reduced pressure, and the obtained residue was dissolved in dichloromethane (200 mL). To this solution were added triethylamine (9.4 mL, 67.4 mmol), 1-(tert-butoxycarbonyl)-4-piperidone (13.5 g, 67.4 mmol) and sodium triacetoxyborohydride (42.9 g, 202 mmol) under ice-cooling, and the mixture was stirred at room temperature for 4 days. A saturated aqueous sodium hydrogencarbonate solution was added to the reaction mixture, and the mixture was extracted with ethyl acetate. The organic layer was washed with saturated brine, and dried over anhydrous magnesium sulfate. The solvent was evaporated under reduced pressure, and the obtained residue was purified by basic silica gel column chromatography (developing solvent; hexane:ethyl acetate=4:1→3:2) and then silica gel column chromatography (developing solvent; hexane:ethyl acetate=1:1→0:1) to give the title compound (15.2 g, yield 61%) as a white solid.
EI(pos) 367 $[M+H]^+$

Reference Example 53

3,3-dimethyl-7-(piperidin-4-yl)-2-oxa-7-azaspiro[4.5]decan-1-one dihydrochloride

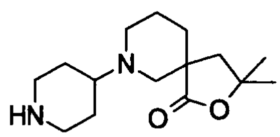

2HCl

Tert-butyl 4-(3,3-dimethyl-1-oxo-2-oxa-7-azaspiro[4.5]dec-7-yl)piperidine-1-carboxylate (13.5 g, 36.9 mmol) was dissolved in 4N hydrogen chloride - ethyl acetate (250 mL), and the mixture was stirred at room temperature for 30 min. The precipitated solid was collected by filtration and washed with diethyl ether to give the title compound (12.4 g, yield 99%) as a white solid.
EI(pos) 267 $[M+H]^+$

Reference Example 54

ethyl 2-{[(diethylamino)carbonyl]amino}-1-benzothiophene-3-carboxylate

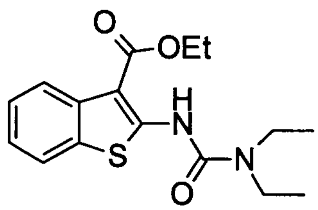

A solution of ethyl 2-amino-1-benzothiophene-3-carboxylate (0.22 g, 1.0 mmol), triethylamine (0.20 g, 2.0 mmol) in tetrahydrofuran (1 mL) was added to triphosgene (0.10 mg, 0.33 mmol) in tetrahydrofuran (2 mL) with stirring at 0°C, and then, the mixture was directly stirred for 30 min. To the reaction mixture was added a solution of diethylamine (0.07 g, 1.0 mmol) and triethylamine (0.10 g, 1.0 mmol) in tetrahydrofuran (1 mL). The mixture was warmed to room temperature and stirred for 2 hr. Water was added to the reaction mixture, and the mixture was extracted with ethyl acetate. The organic layer was washed with saturated brine, and dried over anhydrous sodium sulfate. The solvent was evaporated under reduced pressure to give the title compound (0.33 g, quantitative). This was used in the next step without further purification.
EI(pos) 321 $[M+H]^+$

Reference Example 55

2-{[(diethylamino)carbonyl]amino}-1-benzothiophene-3-carboxylic acid

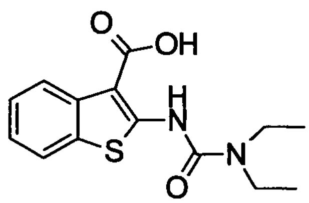

To a solution of ethyl 2-{[(diethylamino)carbonyl]amino}-1-benzothiophene-3-carboxylate (0.33 g, 1.0 mmol) obtained in Reference Example 54 in ethanol (5 mL) was added 1N aqueous sodium hydroxide solution (3 mL, 3.0 mmol) and the mixture was stirred at 60°C for 3 hr. The reaction solution was concentrated under reduced pressure, and the obtained residue was adjusted to pH 7 with 1N hydrochloric acid. The obtained crystals were collected by filtration to give the title compound (0.25 g, yield 86%).
EI(pos) 293 $[M+H]^+$

Reference Example 56

tert-butyl 3-bromo-5-(pyridin-3-yl)benzoate

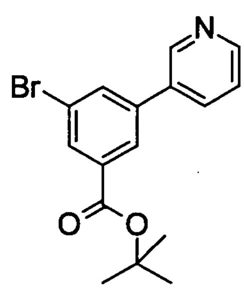

To a solution of tert-butyl 3-bromo-5-iodobenzoate (5.00 g, 13.1 mmol), 3-pyridineboronic acid (1.60 g, 13.1 mmol) and 2N aqueous sodium carbonate solution(13 mL) in tetrahydrofuran (130 mL) was added tetrakis(triphenylphosphine)palladium (453 mg, 0.392 mmol), and the mixture was stirred at 100˚C under a nitrogen atmosphere for 16 hr. After completion of the reaction, the mixture was diluted with ethyl acetate, and dried over anhydrous sodium sulfate. The solvent was evaporated under reduced pressure, and the obtained residue was purified by silica gel column chromatography (developing solvent; hexane→hexane:ethyl acetate=4:1) to give the title compound (1.53 g, yield 35%) as an oil.
$^1$H NMR ($CDCl_3$) δ1.62 (9H, s), 7.40 (1H, m), 7.87-7.90 (2H, m), 8.13 (2H, m), 8.65 (1H, dd, J = 1.5, 6.0 Hz), 8.84 (1H, d, J = 1.5 Hz).

Reference Example 57

3-bromo-5-(pyridin-3-yl)benzoic acid trifluoroacetate

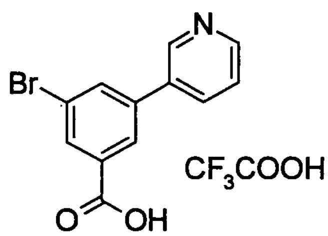

Tert-butyl 3-bromo-5-(pyridin-3-yl)benzoate (1.53 g, 4.58 mmol) obtained in Reference Example 56 was dissolved in trifluoroacetic acid (20 mL) and, after 30 min, the mixture was concentrated under reduced pressure. The residue was washed with diisopropyl ether to give the title compound (1.47 g, yield 82%).
$^1$H NMR (DMSO-$d_6$) δ7.68 (1H, m), 8.10 (1H, s), 8.23 (1H, s), 8.26 (1H, s), 8.39 (1H, m), 8.71 (1H, d, J = 6.0 Hz), 9.05 (1H, s).

Reference Example 58

tert-butyl 3-methyl-1-oxo-3-propyl-2-oxa-7-azaspiro[4.5]decane-7-carboxylate

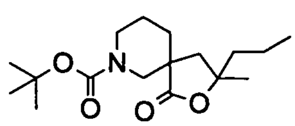

Using 3-ethyl 1-tert-butyl 3-(2-oxopropyl)piperidine-1,3-dicarboxylate (2.00 g, 6.38 mmol) obtained in Reference Example 13 and 2.0 M n-propylmagnesiumbromide-THF solution (6.4 mL, 12.8 mmol), the title compound (0.78 g, yield 39%) was obtained as an oil by an operation similar to that of Reference Example 41.
EI(pos) 334 $[M+Na]^+$

Reference Example 59

tert-butyl 2-(2-{[(benzyloxy)carbonyl]amino}ethyl)-3-methyl-1-oxo-2,7-diazaspiro[4.5]decane-7-carboxylate

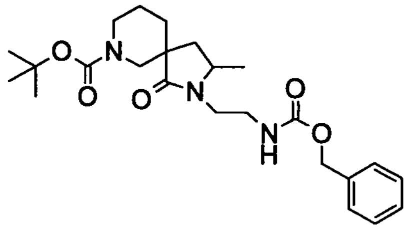

Using 3-ethyl 1-tert-butyl 3-(2-oxopropyl)piperidine-1,3-dicarboxylate (1.34 g, 3.01 mmol) obtained in Reference Example 13 and benzyl (2-aminoethyl)carbamate (5.00 g, 21.7 mmol), the title compound (1.34 g, yield 31%) was obtained as an oil by an operation similar to that of Reference Example 14. EI(pos) 468 $[M+Na]^+$

Reference Example 60

tert-butyl 3-methyl-2-{2-[(methylsulfonyl)amino]ethyl}-1-oxo-2,7-diazaspiro[4.5]decane-7-carboxylate

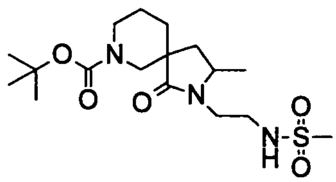

A suspension of tert-butyl 2-(2-{[(benzyloxy)carbonyl]amino}ethyl)-3-methyl-1-oxo-2,7-diazaspiro[4.5]decane-7-carboxylate (1.34 g, 3.01 mmol) obtained in Reference Example 59 and 10% palladium carbon (50% water-containing product, 0.32 g) in methanol (20 mL) was stirred under a hydrogen atmosphere for 1 hr. The reaction mixture was filtrated, and the filtrate was concentrated under reduced pressure. The obtained crude product was dissolved in tetrahydrofuran (20 mL), triethylamine (0.84 mL, 6.02 mmol) and methanesulfonyl chloride (0.28 mL, 3.61 mmol) were added and the mixture was stirred for 30 min. The reaction mixture was diluted with ethyl acetate, washed with saturated aqueous ammonium chloride solution and saturated brine, and dried over anhydrous magnesium sulfate. The solvent was evaporated under reduced pressure, and the obtained residue was purified by column chromatography (developing solvent; methanol:ethyl acetate=0:1→1:9) to give the title compound (0.24 g, yield 20%) as an oil.
EI(pos) 290 $[M-Boc]^+$

Reference Example 61

3-ethyl 1-tert-butyl 3-(3-cyanopropyl)piperidine-1,3-dicarboxylate

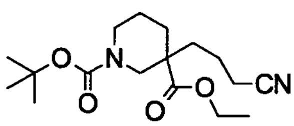

Using 3-ethyl 1-tert-butyl piperidine-1,3-dicarboxylate (5.00 g, 19.4 mmol) and 4-bromobutyronitrile (2.9 mL, 29.1 mmol), the title compound (6.30 g, yield 99%) was obtained as an oil by an operation similar to that of Reference Example 1. EI(pos) 347 $[M+Na]^+$

Reference Example 62

ethyl 1'-(9-anthrylcarbonyl)-3-(3-cyanopropyl)-1,4'-bipiperidine-3-carboxylate

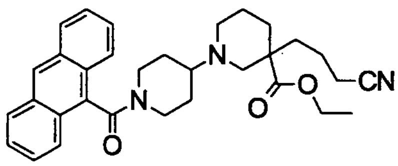

Using 3-ethyl 1-tert-butyl 3-(3-cyanopropyl)piperidine-1,3-dicarboxylate (1.14 g, 3.52 mmol) obtained in Reference Example 61, the title compound (0.31 g, yield 17%) was obtained as an oil by an operation similar to that of Reference Example 34.
EI(pos) 512 $[M+H]^+$

Reference Example 63

diethyl (2-{[(benzyloxy)carbonyl]amino}-2-methylpropylidene)malonate

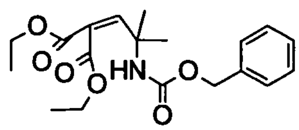

To a solution of titanium chloride(IV) (4.9 mL, 44.8 mmol) in tetrahydrofuran (200 mL) was added a solution of diethyl malonate (3.64 mL, 22.4 mmol) and benzyl (1,1-dimethyl-2-oxoethyl)carbamate(4.96 g, 22.4 mmol) in tetrahydrofuran (20 mL) at 0°C, and the mixture was stirred for 10 min. To this solution was added pyridine (7.25 mL, 89.6 mmol), and the mixture was stirred at room temperature for 5 hr. Water was added to the reaction mixture, and the mixture was extracted with ethyl acetate. The organic layer was washed with saturated brine, and dried over anhydrous magnesium sulfate. The solvent was evaporated under reduced pressure, and the obtained residue was purified by column chromatography (developing solvent; ethyl acetate:hexane=1:4→2:3) to give the title compound (1.71 g, yield 21%) as an oil.
EI(pos) 386 $[M+Na]^+$

Reference Example 64

ethyl 5,5-dimethyl-2-oxopyrrolidine-3-carboxylate

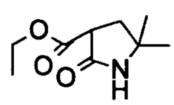

A suspension of diethyl (2-{[(benzyloxy)carbonyl]amino}-2-methylpropylidene)malonate (1.71 g, 4.71 mmol) obtained in Reference Example 63 and 10% palladium carbon (50% water-containing product, 1.00 g) in ethanol (20 mL) was stirred for 1 hr under a hydrogen atmosphere. After filtration, the filtrate was concentrated under reduced pressure to give the title compound (0.87 g, yield 99%) as an oil.
$^1H$ NMR ($CDCl_3$) δ1.29-1.33 (6H, m), 1.38 (3H, s), 2.15-2.22 (1H, m), 2.34-2.41 (1H, m), 3.51-3.77 (1H, m), 4.25 (2H,

q, 7.2 Hz), 5.66 (1H, br).

Reference Example 65

ethyl 3-[3-(benzyloxy)propyl]-5,5-dimethyl-2-oxopyrrolidine-3-carboxylate

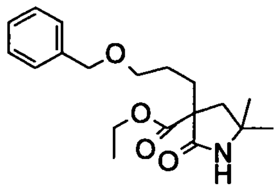

Using ethyl 5,5-dimethyl-2-oxopyrrolidine-3-carboxylate (6.30 g, 34.0 mmol) obtained in Reference Example 64 and benzyl(3-bromopropyl)ether (9.0 mL, 51 mmol), the title compound (3.93 g, yield 35%) was obtained as an oil by an operation similar to that of Reference Example 1.
EI(pos) 356 $[M+Na]^+$

Reference Example 66

3-[3-(benzyloxy)propyl]-3-(hydroxymethyl)-5,5-dimethylpyrrolidin-2-one

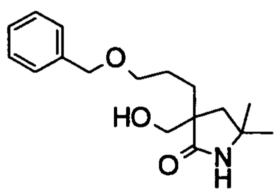

To a solution of ethyl 3-[3-(benzyloxy)propyl]-5,5-dimethyl-2-oxopyrrolidine-3-carboxylate (4.76 g, 14.3 mmol) obtained in Reference Example 65 in tetrahydrofuran (100 mL) was added lithium borohydride (311 mg, 14.3 mmol) at 0˚C, and the mixture was stirred at the same temperature for 30 min. A saturated aqueous sodium hydrogencarbonate solution was added to the reaction mixture, and the mixture was extracted 3 times with ethyl acetate. The organic layer was dried over anhydrous magnesium sulfate. The solvent was evaporated under reduced pressure, and the obtained residue was purified by column chromatography (developing solvent; methanol:ethyl acetate=0:1 →1:9) to give the title compound (2.89 g, yield 69%) as an oil. EI(pos) 314 $[M+Na]^+$

Reference Example 67

3-(azidomethyl)-3-[3-(benzyloxy)propyl]-5,5-dimethylpyrrolidin-2-one

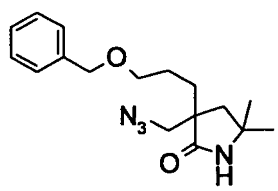

To a solution of 3-[3-(benzyloxy)propyl]-3-(hydroxymethyl)-5,5-dimethylpyrrolidin-2-one (2.87 g, 9.85 mmol)

obtained in Reference Example 66 and triethylamine (2.8 mL, 19.9 mmol) in tetrahydrofuran (100 mL) was added methanesulfonyl chloride (1.15 mL, 14.8 mmol), and the mixture was stirred at room temperature for 30 min. A saturated aqueous sodium hydrogencarbonate solution was added to the reaction mixture, and the mixture was extracted 3 times with ethyl acetate. The organic layer was dried over anhydrous magnesium sulfate. The solvent was evaporated under reduced pressure, and a suspension of the obtained residue and sodium azide (2.27 g, 49.3 mmol) in N,N-dimethylformamide was stirred at 125°C for 13 hr. The reaction mixture was diluted with ethyl acetate, washed 3 times with saturated brine, and dried over anhydrous magnesium sulfate. The solvent was evaporated under reduced pressure, and the obtained residue was purified by column chromatography (developing solvent; ethyl acetate:hexane=3:7→13:7) to give the title compound (2.16 g, yield 69%) as an oil.
EI(pos) 317 $[M+H]^+$
$^1H$ NMR ($CDCl_3$) δ1.29 (3H, s), 1.33 (3H, s), 1.53-1.73 (4H, m), 1.86 (1H, d, J = 1.5 Hz), 2.01 (1H, d, J = 1.5 Hz), 3.24 (1H, d, J = 1.2 Hz), 3.40-3.51 (2H, m), 3.59 (1H, d, J = 1.2 Hz), 4.48 (2H, s), 7.15-7.33 (5H, m).

Reference Example 68

tert-butyl 3,3-dimethyl-1-oxo-2,7-diazaspiro[4.5]decane-7-carboxylate

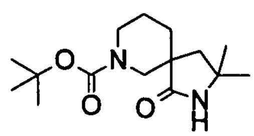

A solution (90 mL) of 3-(azidomethyl)-3-[3-(benzyloxy)propyl]-5,5-dimethylpyrrolidin-2-one (2.16 g, 6.83 mmol) obtained in Reference Example 67, di-tert-butyl bicarbonate (1.89 mL, 8.20 mmol) and 10% palladium carbon (50% water-containing product, 0.73 g) in ethanol was stirred for 30 min under a hydrogen atmosphere at atmospheric pressure. The reaction mixture was filtrated, and the filtrate was concentrated under reduced pressure. A solution of the obtained residue and 10% palladium carbon (50% water-containing product, 0.73 g) in ethanol (30 mL) was stirred under a hydrogen atmosphere (0.5 MPa) at room temperature for 5.5 hr and the reaction mixture was filtrated. To a solution of the obtained residue and triethylamine (1.9 mL, 13.7 mmol) in tetrahydrofuran (30 mL) was added methanesulfonyl chloride (0.94 mL, 12.2 mmol), and the mixture was stirred at room temperature for 15 min. The reaction mixture was diluted with ethyl acetate, washed with saturated aqueous sodium hydrogencarbonate solution, and dried over anhydrous magnesium sulfate. The solvent was evaporated under reduced pressure, and the obtained residue was dissolved in trifluoroacetic acid (10 mL), and the mixture was stirred at room temperature for 30 min. The reaction mixture was basified (about pH 10) with saturated aqueous sodium hydrogencarbonate solution. Di-tert-butyl bicarbonate (1.9 mL, 8.20 mmol) was added, and the mixture was stirred at room temperature for 1 hr. The reaction mixture was extracted twice with ethyl acetate, and the organic layer was dried over anhydrous magnesium sulfate. The solvent was evaporated under reduced pressure, and the obtained residue was purified by column chromatography (developing solvent; ethyl acetate:hexane=2:3→3:1) to give the title compound (0.64 g, yield 33%) as an oil.
EI(pos) 283 $[M+H]^+$

Reference Example 69

7-benzyl-3-ethyl-1-oxa-3,7-diazaspiro[4.5]decane-2,4-dione

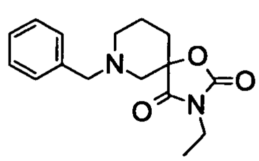

To a solution of methyl 1-benzyl-3-hydroxypiperidine-3-carboxylate (2.00 g, 8.02 mmol) and ethyl isocyanate

(1.3 mL, 16.0 mmol) in tetrahydrofuran (25 mL) was added 60% sodium hydride (0.16 g, 4.01 mmol), and the mixture was stirred at 60˚C for 1 hr. A saturated aqueous ammonium chloride solution was added to the reaction mixture, and the mixture was extracted with ethyl acetate. The organic layer was dried over anhydrous magnesium sulfate. The solvent was evaporated under reduced pressure, and the obtained residue was purified by column chromatography (developing solvent; ethyl acetate:hexane=1:9→2:3) to give the title compound (1.81 g, yield 78%) as an oil.
EI(pos) 289 $[M+H]^+$

Reference Example 70

3-ethyl-1-oxa-3,7-diazaspiro[4.5]decane-2,4-dione

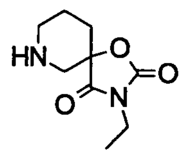

A suspension of 7-benzyl-3-ethyl-1-oxa-3,7-diazaspiro[4.5]decane-2,4-dione (1.81 g, 6.28 mmol) obtained in Reference Example 69 and 20% palladium hydroxide on carbon (0.44 g) in ethanol (30 mL) was stirred for 2 hr under a hydrogen atmosphere. After filtration, the filtrate was concentrated under reduced pressure to give the title compound (1.19 g, yield 96%) as an oil.
EI(pos) 199 $[M+H]^+$

Reference Example 71

ethyl 2-[(tert-butoxycarbonyl)amino]-4-methylthiophene-3-carboxylate

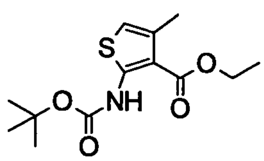

To a solution of ethyl 2-amino-4-methylthiophene-3-carboxylate (10.0 g, 53.9 mmol) in tetrahydrofuran (200 mL) were added di-tert-butyl bicarbonate (18.6 mL, 81.0 mmol) and 4-dimethylaminopyridine (0.66 g, 5.39 mmol), and the mixture was stirred at room temperature for 16 hr. The solvent was evaporated under reduced pressure, and the obtained residue was purified by column chromatography (developing solvent; ethyl acetate:hexane=1:19→3:17) to give the title compound (6.75 g, yield 44%) as an oil.
$^1H$ NMR ($CDCl_3$) δ1.39 (3H, t, J = 7.1 Hz), 1.55 (9H, s), 2.34 (3H, s), 4.34 (2H, q, 7.1 Hz), 6.30 (1H, s), 10.38 (1H, br).

Reference Example 72

ethyl 5-bromo-2-[(tert-butoxycarbonyl)amino]-4-methylthiophene-3-carboxylate

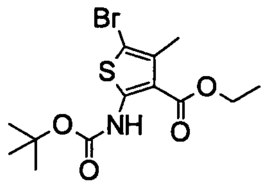

To a solution of ethyl 2-[(tert-butoxycarbonyl)amino]-4-methylthiophene-3-carboxylate (6.75 g, 23.7 mmol) obtained in Reference Example 71 in chloroform (100 mL) was added N-bromosuccinimide (4.64 g, 26.1 mmol), and the mixture was stirred at room temperature for 30 min. The reaction mixture was washed with saturated brine and dried over anhydrous magnesium sulfate. The solvent was evaporated under reduced pressure, and the obtained residue was purified by column chromatography (developing solvent; ethyl acetate:hexane=1:19→ 1:3) to give the title compound 8.63 g (quantitative) as an oil.
EI(pos) 387 $[M+Na]^+$

Reference Example 73

ethyl 2-[(tert-butoxycarbonyl)amino]-4-methyl-5-phenylthiophene-3-carboxylate

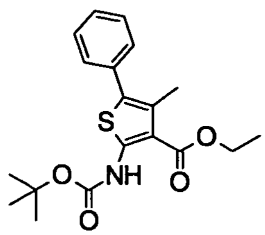

Using ethyl 5-bromo-2-[(tert-butoxycarbonyl)amino]-4-methylthiophene-3-carboxylate (6.00 g, 16.5 mmol) obtained in Reference Example 72 and phenylboronic acid (4.02 g, 33.0 mmol), the title compound (0.95 g, yield 16%) was obtained as an oil by an operation similar to that of Reference Example 56. $^1H$ NMR ($CDCl_3$) δ1.40 (3H, t, J = 7.1 Hz), 1.54 (9H, s), 2.36 (3H, s), 4.36 (2H, q, 7.2 Hz), 7.30-7.34 (1H, m), 7.39 (4H, m).

Reference Example 74

2-[(tert-butoxycarbonyl)amino]-4-methyl-5-phenylthiophene-3-carboxylic acid

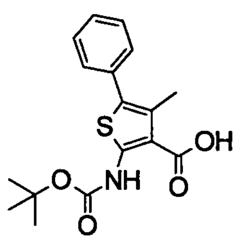

Using ethyl 2-[(tert-butoxycarbonyl)amino]-4-methyl-5-phenylthiophene-3-carboxylate (0.95 g, 2.63 mmol) obtained in Reference Example 73, the title compound (0.87 g, yield 99%) was obtained as an oil by an operation similar to that of Reference Example 45.
$^1H$ NMR ($CDCl_3$) δ1.57 (9H, s), 2.42 (3H, s), 7.33-7.42 (5H, m).

Reference Example 75

methyl 2-[(tert-butoxycarbonyl)amino]-5-phenylthiophene-3-carboxylate

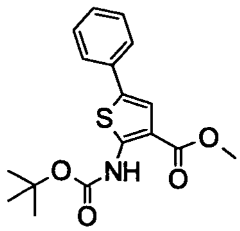

To a mixed solution of methyl 2-amino-5-phenylthiophene-3-carboxylate (10.3 g, 44.2 mmol) in tert-butanol-tetrahydrofuran (200 mL-50 mL) were added di-tert-butyl bicarbonate (12.2 mL, 53.0 mmol) and 4-dimethylaminopyridine (0.27 g, 2.21 mmol), and the mixture was stirred at room temperature for 14 hr. The solvent was evaporated under reduced pressure, and the obtained solid was washed with ethyl acetate to give the title compound (9.18 g, yield 62%) as a powder.
$^1$H NMR ($CDCl_3$) δ1.55 (9H, s), 3.89 (3H, s), 7.23-7.28 (1H, m), 7.33-7.38 (3H, m), 7.55-7.58 (2H, m), 10.06 (1H, br).
EI (pos) 334 $[M+H]^+$

Reference Example 76

2-[(tert-butoxycarbonyl)amino]-5-phenylthiophene-3-carboxylic acid

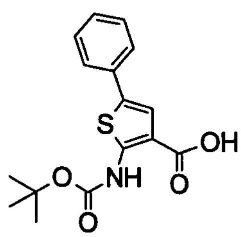

Using methyl 2-[(tert-butoxycarbonyl)amino]-5-phenylthiophene-3-carboxylate (2.00 g, 6.00 mmol) obtained in Reference Example 75, the title compound (1.73 g, yield 90%) was obtained as a yellow solid by an operation similar to that of Reference Example 45.
$^1$H NMR ($CDCl_3$) δ1.58 (9H, s), 7.27-7.30 (1H, m), 7.35-7.49 (2H, m), 7.43 (1H, m), 7.56-7.59 (2H, m), 9.88 (1H, br).

Reference Example 77

9-tert-butyl 6-benzyl 3,3-dimethyl-1-oxo-2-oxa-6,9-diazaspiro[4.5]decane-6,9-dicarboxylate

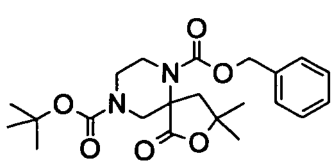

Using 2-methyl 4-tert-butyl 1-benzyl 2-(2-oxopropyl)piperazine-1,2,4-tricarboxylate (3.31 g, 7.61 mmol) obtained in Reference Example 36, the title compound (2.51 g, yield 79%) was obtained as an oil by an operation similar to that of Reference Example 19.
EI(pos) 363 [M-tBu]$^+$

Reference Example 78

tert-butyl 3,3-dimethyl-1-oxo-6-(trifluoroacetyl)-2-oxa-6,9-diazaspiro[4.5]decane-9-carboxylate

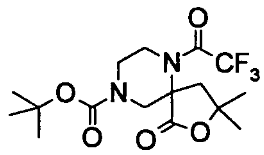

Using 9-tert-butyl 6-benzyl 3,3-dimethyl-1-oxo-2-oxa-6,9-diazaspiro[4.5]decane-6,9-dicarboxylate (2.51 g, 6.00 mmol) obtained in Reference Example 77, the title compound (1.51 g, yield 66%) was obtained as an oil by an operation similar to that of Reference Example 38.
EI(pos) 325 [M-Boc]$^+$

Reference Example 79

tert-butyl 3-acetamido-3-cyanopiperidine-1-carboxylate

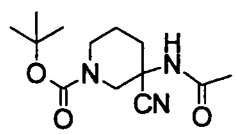

To a mixed solution of tert-butyl 3-oxopiperidine-1-carboxylate (5.00 g, 25.1 mmol) and ammonium chloride (5.35 g, 100 mmol) in isopropyl alcohol-25% aqueous ammonia (30 mL-62 mL) was added potassium cyanide (6.54 g, 100 mmol), and the mixture was stirred for 14 hr. The reaction mixture was extracted 3 times with ethyl acetate, and the organic layer was dried over anhydrous magnesium sulfate. The solvent was evaporated under reduced pressure. To a solution of the obtained residue and triethylamine (5.3 mL, 37.7 mmol) in tetrahydrofuran (100 mL) was added acetyl chloride (2.32 mL, 32.6 mmol), and the mixture was stirred at room temperature for 30 min. A saturated aqueous sodium hydrogencarbonate solution was added to the reaction mixture, and the mixture was extracted twice with ethyl acetate. The organic layer was dried over anhydrous magnesium sulfate. The solvent was evaporated under reduced pressure, and the obtained residue was purified by column chromatography (developing solvent; methanol:ethyl acetate=0:1→3,1:9) to give the title compound (5.26 g, yield 78%) as an oil.
EI(pos) 168 [M-Boc]$^+$

Reference Example 80

tert-butyl 2-methyl-4-oxo-1,3,7-triazaspiro[4.5]dec-1-en-7-carboxylate

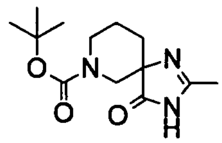

To a solution of tert-butyl 3-acetamido-3-cyanopiperidine-1-carboxylate (1.08 g, 4.04 mmol) obtained in Reference Example 79 in ethanol (10 mL) were added 8N aqueous sodium hydroxide solution (1.14 mL, 9.09 mmol) and 30% aqueous hydrogen peroxide (0.5 mL), and the mixture was stirred for 2 hr with heating under reflux. Water was added to the reaction mixture. The mixture was extracted twice with ethyl acetate-tetrahydrofuran mixture (1:1), and the organic layer was dried over anhydrous magnesium sulfate. The solvent was evaporated under reduced pressure to give the title compound (1.07 g, yield 99%) as a solid.
EI(pos) 268 $[M+H]^+$

Reference Example 81

tert-butyl 2-(3-ethoxy-3-oxopropyl)-3-methyl-1-oxo-2,7-diazaspiro[4.5]decane-7-carboxylate

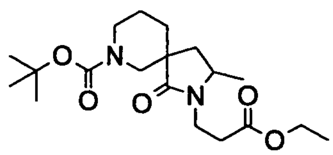

Using 3-ethyl 1-tert-butyl 3-(2-oxopropyl)piperidine-1,3-dicarboxylate (2.00 g, 6.39 mmol) obtained in Reference Example 13 and β-alanine ethyl ester hydrochloride (2.94 g, 19.2 mmol), the title compound (0.75 g, yield 32%) was obtained as an oil by an operation similar to that of Reference Example 14.
EI(pos) 369 $[M+H]^+$

Reference Example 82

N-[1'-(9-anthrylcarbonyl)-3-cyano-1,4'-bipiperidin-3-yl]acetamide

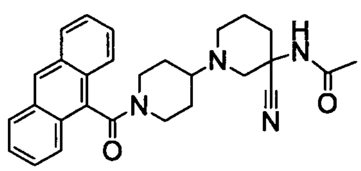

Using tert-butyl 3-acetamido-3-cyanopiperidine-1-carboxylate (4.18 g, 15.6 mmol) obtained in Reference Example 79, the title compound (3.83 g, yield 54%) was obtained as an oil by an operation similar to that of Reference Example 34. EI(pos) 455 $[M+H]^+$

Reference Example 83

tert-butyl 2-(2-acetoxyethyl)-3-methyl-1-oxo-2,7-diazaspiro[4.5]decane-7-carboxylate

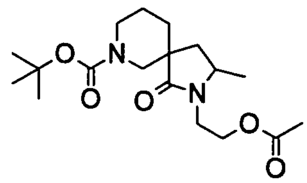

To a mixed solution of tert-butyl 2-(2-methoxy-2-oxoethyl)-3-methyl-1-oxo-2,7-diazaspiro[4.5]decane-7-carboxylate (1.62 g, 4.76 mmol) obtained in Reference Example 18 in tetrahydrofuran-methanol (15 mL-10 mL) was added 1N aqueous sodium hydroxide solution (10 mL, 10 mmol), and the mixture was stirred at room temperature for 15 min. The reaction mixture was neutralized with 1N hydrochloric acid (10 mL), extracted 3 times with ethyl acetate, and dried over anhydrous magnesium sulfate. The solvent was evaporated under reduced pressure. To a solution of the obtained residue and triethylamine (0.83 mL, 5.95 mmol) in tetrahydrofuran (30 mL) was added ethyl chloroformate (0.55 mL, 5.71 mmol) at 0˚C, and the mixture was stirred at the same temperature for 30 min. A solution of sodium borohydride (0.27 g, 7.14 mmol) in methanol (30 mL) was added to the reaction mixture, and the mixture was stirred at room temperature for 30 min. Saturated aqueous ammonium chloride was added to the reaction mixture, and the mixture was extracted twice with ethyl acetate, and dried over anhydrous magnesium sulfate. The solvent was evaporated under reduced pressure. To the obtained residue were added acetic anhydride (10 mL) and pyridine (10 mL), and the mixture was stirred at room temperature for 30 min. The solvent was evaporated under reduced pressure, and the obtained residue was purified by column chromatography (developing solvent; ethyl acetate:hexane=3:1→1:0) to give the title compound (1.05 g, yield 62%) as an oil.
EI(pos) 355 $[M+H]^+$

Reference Example 84

methyl 2-[(tert-butoxycarbonyl)amino]-5-(pyridin-4-yl)thiophene-3-carboxylate

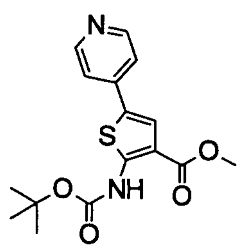

To a solution of methyl 5-bromo-2-[(tert-butoxycarbonyl)amino]thiophene-3-carboxylate (5.77 g, 17.2 mmol), 4-pyridineboronic acid (4.22 g, 34.3 mmol) and 2N aqueous sodium carbonate solution (17 mL) in N,N-dimethylformamide (50 mL) was added 1,1'-bis(diphenylphosphino)ferrocenepalladium dichloride (702 mg, 0.860 mmol), and the mixture was stirred at 80˚C for 15 hr under a nitrogen atmosphere. After completion of the reaction, the mixture was diluted with ethyl acetate, washed 3 times with saturated brine, and dried over anhydrous sodium sulfate. The solvent was evaporated under reduced pressure, and the obtained residue was purified by silica gel column chromatography (developing solvent; hexane:ethyl acetate=3:2→ 0:1) to give the title compound (2.72 g, yield 47%) as a yellow solid.
$^1H$ NMR ($CDCl_3$) δ1.56 (9H, s), 3.91 (3H, s), 7.41-7.43 (2H, m), 7.60 (1H, s), 8.55-8.57 (2H, m), 10.12 (1H, br).
EI(pos) 335 $[M+H]^+$

Reference Example 85

2-[(tert-butoxycarbonyl)amino]-5-(pyridin-4-yl)thiophene-3-carboxylic acid

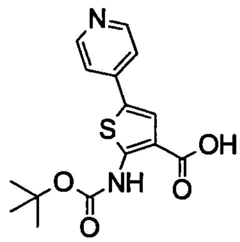

Using methyl 2-[(tert-butoxycarbonyl)amino]-5-(pyridin-4-yl)thiophene-3-carboxylate (2.11 g, 6.31 mmol) obtained in Reference Example 84, the title compound (1.65 g, yield 81%) was obtained as a yellow solid by an operation similar to that of Reference Example 45.
$^1$H NMR (DMSO-$d_6$) δ1.52 (9H, s), 7.61-7.63 (2H, m), 7.82 (1H, s), 8.51-8.53 (2H, m), 10.38 (1H, br).

Reference Example 86

3-amino-1-phenyl-1H-pyrazole-4-carboxylic acid

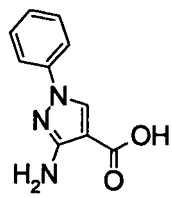

Using ethyl 3-amino-1-phenyl-1H-pyrazole-4-carboxylate (4.80 g, 20.8 mmol), the title compound (3.42 g, yield 81%) was obtained as a yellow solid by an operation similar to that of Reference Example 45.
$^1$H NMR (DMSO-$d_6$) δ5.65 (2H, br), 7.25 (1H, m), 7.44 (2H, m), 7.77-7.80 (2H, m), 8.67 (1H, s).

Reference Example 87

tert-butyl 2-(3-acetoxypropyl)-3-methyl-1-oxo-2,7-diazaspiro[4.5]decane-7-carboxylate

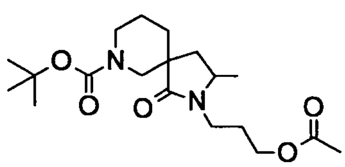

Using tert-butyl 2-(3-ethoxy-3-oxopropyl)-3-methyl-1-oxo-2,7-diazaspiro[4.5]decane-7-carboxylate (1.88 g, 5.10 mmol) obtained in Reference Example 81, the title compound (0.97 g, yield 52%) was obtained as an oil by an operation similar to that of Reference Example 83.
EI(pos) 369 $[M+H]^+$

Reference Example 88

tert-butyl 3,3-dimethyl-1-oxo-2,6-dioxa-9-azaspiro[4.5]decane-9-carboxylate

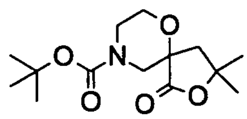

Using 2-ethyl 4-tert-butyl 2-(2-oxopropyl)morpholine-2,4-dicarboxylate (6.00 g, 19.0 mmol) obtained in Reference Example 24, the title compound (1.76 g, yield 32%) was obtained as an oil by an operation similar to that of Reference Example 19.
EI(pos) 308 $[M+Na]^+$

Reference Example 89

5-bromo-2-[(tert-butoxycarbonyl)amino]thiophene-3-carboxylic acid

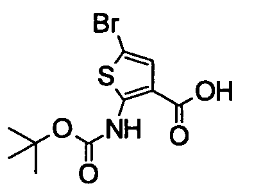

Using methyl 5-bromo-2-[(tert-butoxycarbonyl)amino]thiophene-3-carboxylate (2.00 g, 5.95 mmol), the title compound (1.05 g, yield 55%) was obtained as a yellow solid by an operation similar to that of Reference Example 45.

$^1$H NMR (DMSO-$d_6$) δ1.50 (9H, s), 7.20 (1H, s), 10.20 (1H, br). Reference Example 90

benzyl 9-[1-(tert-butoxycarbonyl)piperidin-4-yl]-3,3-dimethyl-1-oxo-2-oxa-6,9-diazaspiro[4.5]decane-6-carboxylate

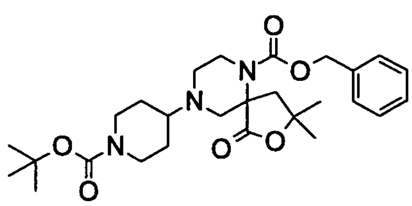

Using 9-tert-butyl 6-benzyl 3,3-dimethyl-1-oxo-2-oxa-6,9-diazaspiro[4.5]decane-6,9-dicarboxylate (0.87 g, 2.08 mmol) obtained in Reference Example 77, the title compound (0.89 g, yield 85%) was obtained as an oil by an operation similar to that of Reference Example 52.
EI(pos) 502 $[M+H]^+$

Reference Example 91

tert-butyl 4-[2-(2-acetoxyethyl)-3-methyl-1-oxo-2,7-diazaspiro[4.5]dec-7-yl]piperidine-1-carboxylate

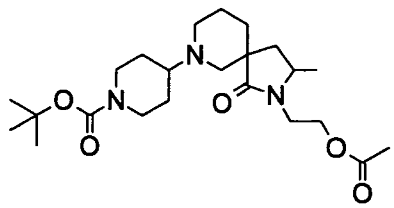

Using tert-butyl 2-(2-acetoxyethyl)-3-methyl-1-oxo-2,7-diazaspiro[4.5]decane-7-carboxylate (2.75 g, 7.76 mmol) obtained in Reference Example 83, the title compound (1.87 g, yield 55%) was obtained as an oil by an operation similar to that of Reference Example 52.
EI(pos) 438 $[M+H]^+$

Reference Example 92

methyl 2-[(tert-butoxycarbonyl)amino]-5-(3-hydroxyprop-1-yn-1-yl)thiophene-3-carboxylate

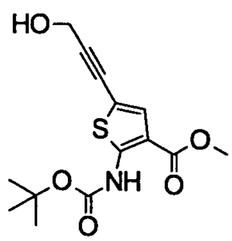

A mixture of methyl 5-bromo-2-[(tert-butoxycarbonyl)amino]thiophene-3-carboxylate (2.00 g, 5.95 mmol), 2-propyn-1-ol (1.6 mL, 26.8 mmol), dichlorobis(triphenylphosphine)palladium (314 mg, 0.447 mmol) and copper iodide (171 mg, 0.893 mmol) in tetrahydrofuran-triethylamine (30 mL-10 mL) was stirred at 70°C for 13.5 hr under an argon atmosphere. The reaction mixture was diluted with ethyl acetate, washed with saturated brine, and dried over anhydrous magnesium sulfate. The solvent was evaporated under reduced pressure, and the obtained residue was purified by column chromatography (developing solvent; ethyl acetate:hexane=1:4→1:1) to give the title compound (1.70 g, yield 61%) as an oil.
$^1H$ NMR ($CDCl_3$) δ1.53 (9H, s), 3.86 (3H, s), 4.48 (2H, d, J = 6.1 Hz), 7.27 (1H, s), 10.10 (1H, br).
EI(pos) 312 $[M+H]^+$

Reference Example 93

2-[(tert-butoxycarbonyl)amino]-5-(3-hydroxypropyl)thiophene-3-carboxylic acid

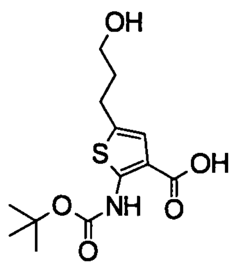

A suspension of methyl 2-[(tert-butoxycarbonyl)amino]-5-(3-hydroxyprop-1-yn-1-yl)thiophene-3-carboxylate (1.80 g, 5.78 mmol) obtained in Reference Example 92 and 10% palladium carbon (50% water-containing product, 616

mg) in ethanol (50 mL) was stirred at room temperature for 1.7 hr under a hydrogen atmosphere. After filtration, the filtrate was concentrated under reduced pressure. Using the obtained residue, the title compound (0.62 g, yield 36%) was obtained as a yellow solid by an operation similar to that of Reference Example 45.
$^1H$ NMR (DMSO-$d_6$) δ1.49 (9H, s), 1.66-1.75 (2H, m), 2.70 (2H, t, J = 7.4 Hz), 3.42 (2H, t, J = 6.1 Hz), 6.80 (1H, s), 10.12 (1H, br).
EI(pos) 302 $[M+H]^+$

Reference Example 94

methyl N-[(cyanoimino)(phenyl)methyl]glycinate

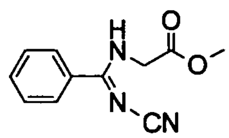

A solution of methyl N-cyanobenzenecarboximidate (18.5 g, 116 mmol), glycine methyl ester hydrochloride (15.1 g, 120 mmol) and triethylamine (28 mL, 200 mmol) in methanol (100 mL) was stirred at room temperature for 30 min. Water was added to the reaction mixture, and the mixture was extracted twice with ethyl acetate, and dried over anhydrous magnesium sulfate. The solvent was evaporated, and the obtained residue was purified by column chromatography (developing solvent; ethyl acetate) to give the title compound (7.89 g, yield 31%) as a yellow solid.
EI(pos) 218 $[M+H]^+$

Reference Example 95

methyl 4-amino-1-benzyl-2-phenyl-1H-imidazole-5-carboxylate

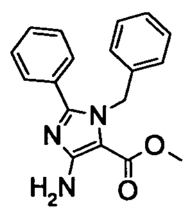

To a solution of methyl N-[(cyanoimino)(phenyl)methyl]glycinate (1.00 g, 4.60 mmol) obtained in Reference Example 94 in N,N-dimethylformamide (30 mL) was added 60% sodium hydride (203 mg, 5.07 mmol), and the mixture was stirred at room temperature for 15 min. To the solution was added benzyl bromide (0.60 mL, 5.07 mmol), and the mixture was stirred for 2 hr. The reaction mixture was diluted with ethyl acetate, washed 3 times with saturated brine, and dried over anhydrous magnesium sulfate. The solvent was evaporated under reduced pressure, and the obtained residue was purified by column chromatography (developing solvent; ethyl acetate:hexane=2:3→4:1) to give the title compound (1.15 g, yield 81%) as an oil.
EI(pos) 308 $[M+H]^+$
$^1H$ NMR ($CDCl_3$) δ3.74 (3H, s), 4.98 (2H, br), 5.47 (2H, s), 6.99-7.02 (2H, m), 7.21-7.32 (3H, m), 7.35-7.45 (3H, m), 7.50-7.53 (2H, m).

Reference Example 96

methyl 1-benzyl-4-[bis(tert-butoxycarbonyl)amino]-2-phenyl-1H-imidazole-5-carboxylate

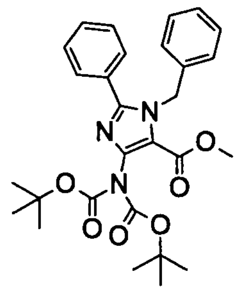

A solution of methyl 4-amino-1-benzyl-2-phenyl-1H-imidazole-5-carboxylate (1.15 g, 3.75 mmol) obtained in Reference Example 95, di-tert-butyl bicarbonate (1.30 mL, 5.63 mmol), triethylamine (0.53 mL, 5.63 mmol) and 4-dimethylaminopyridine (45.8 mg, 0.375 mmol) in tert-butanol (10 mL) was stirred at 60˚C for 4 hr. The solvent was evaporated under reduced pressure, and the obtained residue was purified by column chromatography (developing solvent; ethyl acetate:hexane=1:9→1:1) to give the title compound (1.43 g, yield 75%) as an oil.
EI(pos) 508 $[M+H]^+$
$^1$H NMR (DMSO-$d_6$) δ1.45 (18H, s), 3.76 (3H, s), 5.67 (2H, s), 6.94-6.97 (2H, m), 7.25-7.29 (3H, m), 7.39-7.44 (3H, m), 7.50-7.54 (2H, m).

Reference Example 97

1-benzyl-4-[(tert-butoxycarbonyl)amino]-2-phenyl-1H-imidazole-5-carboxylic acid

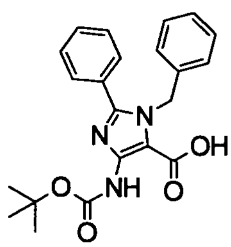

Using methyl 1-benzyl-4-[bis(tert-butoxycarbonyl)amino]-2-phenyl-1H-imidazole-5-carboxylate (1.43 g, 2.82 mmol) obtained in Reference Example 96, the title compound (0.88 g, yield 79%) was obtained as an oil by an operation similar to that of Reference Example 45.
$^1$H NMR (DMSO-$d_6$) δ1.44 (9H, s), 5.62 (2H, s), 6.87 (2H, d, J = 6.8 Hz), 7.18-7.30 (3H, m), 7.44-7.52 (5H, m).

Reference Example 98

tert-butyl 3-acetamido-3-cyano-1,4'-bipiperidine-1'-carboxylate

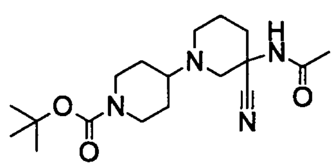

Using tert-butyl 3-acetamido-3-cyanopiperidine-1-carboxylate (10.7 g, 40.0 mmol) obtained in Reference Example 79, the title compound (6.04 g, yield 43%) was obtained as an oil by an operation similar to that of Reference

Example 52. EI(pos) 351 [M+H]+

Reference Example 99

tert-butyl 4-(2-methyl-4-oxo-1,3,7-triazaspiro[4.5]dec-1-en-7-yl)piperidine-1-carboxylate

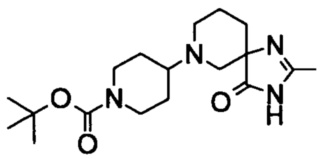

Using tert-butyl 3-acetamido-3-cyano-1,4'-bipiperidine-1'-carboxylate (3.00 g, 8.56 mmol) obtained in Reference Example 98, the title compound (2.31 g, yield 77%) was obtained as an oil by an operation similar to that of Reference Example 80.
EI(pos) 351 [M+H]+

Reference Example 100

tert-butyl 4-[3-(2-acetoxyethyl)-2-methyl-4-oxo-1,3,7-triazaspiro[4.5]dec-1-en-7-yl]piperidine-1-carboxylate

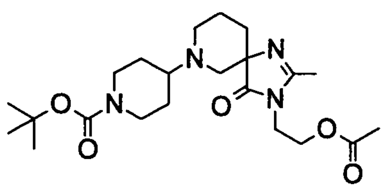

To a solution of tert-butyl 4-(2-methyl-4-oxo-1,3,7-triazaspiro[4.5]dec-1-en-7-yl)piperidine-1-carboxylate (0.50 g, 1.43 mmol) obtained in Reference Example 99 in N,N-dimethylformamide (10 mL) was added 60% sodium hydride (68.7 mg, 1.72 mmol), and the mixture was stirred at room temperature for 30 min. To the solution was added 2-bromoethyl acetate (0.316 mL, 2.86 mmol), and the mixture was stirred at 60°C for 1 hr. The reaction mixture was diluted with ethyl acetate, washed 3 times with brine, and dried over anhydrous magnesium sulfate. The solvent was evaporated under reduced pressure, and the obtained residue was purified by basic silica gel column chromatography (developing solvent; methanol:ethyl acetate=0:1→1:9) to give the title compound (0.41 g, yield 66%) as an oil.
EI(pos) 437 [M+H]+

Reference Example 101

tert-butyl 4-(3,3-dimethyl-1-oxo-2,6-dioxa-9-azaspiro[4.5]dec-9-yl)piperidine-1-carboxylate

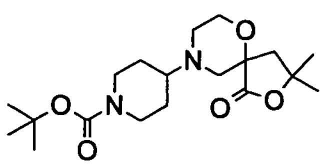

Using tert-butyl 3,3-dimethyl-1-oxo-2,6-dioxa-9-azaspiro[4.5]decane-9-carboxylate (1.67 g, 5.86 mmol) obtained in Reference Example 88, the title compound (1.37 g, yield 63%) was obtained as an oil by an operation similar to that of Reference Example 52.
EI(pos) 369 $[M+H]^+$

Reference Example 102

methyl 2-[(tert-butoxycarbonyl)amino]-5-(pyridin-2-yl)thiophene-3-carboxylate

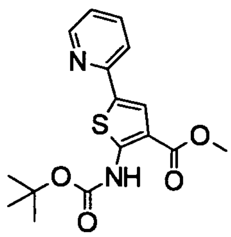

A solution of methyl 5-bromo-2-[(tert-butoxycarbonyl)amino]thiophene-3-carboxylate (2.25 g, 6.70 mmol), 2-(tributylstannyl)pyridine (4.93 g, 13.4 mmol) and tetrakis(triphenylphosphine)palladium (388 mg, 0.335 mmol) in 1,4-dioxane (50 mL) was stirred at 110˚C for 19 hr under an argon atmosphere. The solvent was evaporated under reduced pressure, and the obtained residue was purified by column chromatography (developing solvent; ethyl acetate:hexane=1:19→ 1:3) to give the title compound (2.00 g, yield 89%) as an oil. EI(pos) 335 $[M+H]^+$
$^1$H NMR (DMSO-$d_6$) δ1.55 (9H, s), 3.89 (3H, s), 7.08-7.12 (1H, m), 7.55-7.58 (1H, m), 7.63 (1H, dd, J = 7.3, 1.8 Hz), 7.66 (1H, s), 8.49-8.52 (1H, m), 10.08 (1H, br).

Reference Example 103

2-[(tert-butoxycarbonyl)amino]-5-(pyridin-2-yl)thiophene-3-carboxylic acid

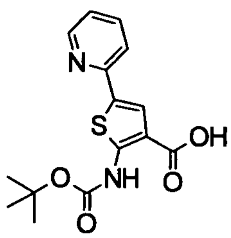

Using methyl 2-[(tert-butoxycarbonyl)amino]-5-(pyridin-2-yl)thiophene-3-carboxylate (2.00 g, 5.98 mmol) obtained in Reference Example 102, the title compound (1.27 g, yield 66%) was obtained as a yellow solid by an operation similar to that of Reference Example 45.
$^1$H NMR (DMSO-$d_6$) δ1.52 (9H, s), 7.19-7.24 (1H, m), 7.74-7.80 (2H, m), 7.91 (1H, d, J = 7.9 Hz), 8.46-8.47 (1H, m).
EI (pos) 321 $[M+H]^+$

Reference Example 104

tert-butyl 4-(1-oxo-2,7-diazaspiro[4.5]dec-7-yl)piperidine-1-carboxylate

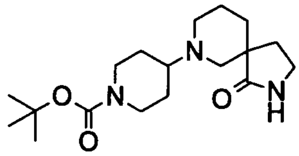

Using tert-butyl 1-oxo-2,7-diazaspiro[4.5]decane-7-carboxylate (1.20 g, 4.72 mmol) obtained in Reference Example 2, the title compound (1.01 g, yield 63%) was obtained as an oil by an operation similar to that of Reference Example 52. EI(pos) 338 $[M+H]^+$

Reference Example 105

methyl 2-iodo-5-phenylthiophene-3-carboxylate

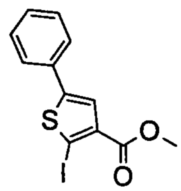

To a mixed solution of methyl 2-amino-5-phenylthiophene-3-carboxylate (1.00 g, 4.29 mmol) in acetonitrile-water (15 mL-15 mL) was added sulfuric acid (0.5 g), and the mixture was cooled to 0˚C. An aqueous solution (5 mL) of sodium nitrite (444 mg, 6.43 mmol) was added, and the mixture was stirred at the same temperature for 30 min. An aqueous solution (5 mL) of potassium iodide (1.43 g, 8.58 mmol) was added to the reaction mixture, and the mixture was stirred at room temperature for 1 hr. The reaction mixture was diluted with ethyl acetate, washed with saturated brine, and dried over anhydrous magnesium sulfate. The solvent was evaporated under reduced pressure, and the obtained residue was purified by column chromatography (developing solvent; ethyl acetate:hexane=1:19→ 3:7) to give the title compound (0.35 g, yield 23%) as a yellow solid.
$^1H$ NMR ($CDCl_3$) δ3.91 (3H, s), 7.31-7.43 (3H, m), 7.51-7.55 (3H, m).

Reference Example 106

methyl 2-[(1E)-3-ethoxy-3-oxoprop-1-en-1-yl]-5-phenylthiophene-3-carboxylate

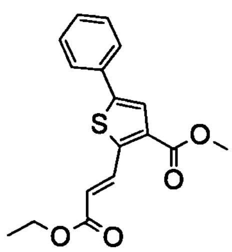

A solution of methyl 2-iodo-5-phenylthiophene-3-carboxylate (0.35 g, 1.02 mmol) obtained in Reference Example 105, ethyl acrylate (0.33 mL, 3.05 mmol), tetrabutylammonium chloride (284 mg, 1.02 mmol), triethylamine (0.29 mL, 2.04 mmol) and palladium acetate (11.5 mg, 0.0510 mmol) in N,N-dimethylformamide (10 mL) was stirred at 90˚C for 3 hr under an argon atmosphere. The reaction mixture was diluted with ethyl acetate, washed 3 times with saturated brine, and dried over anhydrous magnesium sulfate. The solvent was evaporated under reduced pressure, and the obtained residue was purified by column chromatography (developing solvent; ethyl acetate:hexane=1:19→3:7) to give

the title compound (0.30 g, yield 93%) as an oil.
$^1$H NMR ($CDCl_3$) δ1.35 (3H, t, J = 7.1 Hz), 3.93 (3H, s), 4.28 (2H, q, J = 7.3 Hz), 6.37 (1H, d, J = 15.8 Hz), 7.35-7.45 (3H, m), 7.60-7.63 (2H, m), 7.69 (1H, s), 8.60 (1H, d, J = 16.0 Hz). EI(pos) 317 [M+H]$^+$

Reference Example 107

methyl 2-(3-ethoxy-3-oxopropyl)-5-phenylthiophene-3-carboxylate

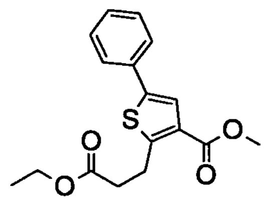

A suspension of methyl 2-[(1E)-3-ethoxy-3-oxoprop-1-en-1-yl]-5-phenylthiophene-3-carboxylate (0.30 g, 0.949 mmol) obtained in Reference Example 106 and 10% palladium carbon (50% water-containing product, 0.10 g) in ethanol (10 mL) was stirred at room temperature for 1 hr under a hydrogen atmosphere. After filtration, the filtrate was concentrated under reduced pressure, and the obtained residue was purified by column chromatography (developing solvent; ethyl acetate) to give the title compound (0.23 g, yield 76%) as an oil.
$^1$H NMR ($CDCl_3$) δ1.26 (3H, t, J = 7.2 Hz), 2.75 (2H, t, J = 7.5 Hz), 3.50 (2H, t, J =7.4 Hz), 3.88 (3H, s), 4.16 (2H, q, J = 7.2 Hz), 7.28-7.41 (3H, m), 7.53-7.57 (2H, m), 7.60 (1H, s).

Reference Example 108

methyl 2-{2-[(tert-butoxycarbonyl)amino]ethyl}-5-phenylthiophene-3-carboxylate

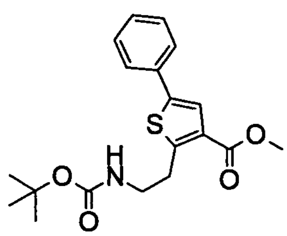

To a mixed solution of methyl 2-(3-ethoxy-3-oxopropyl)-5-phenylthiophene-3-carboxylate (0.23 g, 0.722 mmol) obtained in Reference Example 107 in tetrahydrofuran-methanol (5 mL-2.5 mL) was added 1N aqueous sodium hydroxide solution (1.4 mL, 1.40 mmol), and the mixture was stirred at room temperature for 30 min. The reaction mixture was acidified with 1N hydrochloric acid, and the mixture was extracted with ethyl acetate. The organic layer was washed with saturated brine, and dried over anhydrous magnesium sulfate. The solvent was evaporated under reduced pressure, and the obtained yellow solid and triethylamine (0.12 mL, 0.867 mmol) were dissolved in toluene (10 mL) and diphenylphosphoryl azide (0.187 mL, 0.867 mmol) was added thereto. Tert-butanol (10 mL) was added to the mixture, and the mixture was stirred at 100˚C for 2 hr. The reaction mixture was diluted with ethyl acetate, washed with saturated brine, and dried over anhydrous magnesium sulfate. The solvent was evaporated under reduced pressure, and the obtained residue was purified by column chromatography (developing solvent; ethyl acetate:hexane=1:19→3:7) to give the title compound (90 mg, yield 34%) as an oil.
EI(pos) 262 [M-Boc]$^+$

Reference Example 109

2-{2-[(tert-butoxycarbonyl)amino]ethyl}-5-phenylthiophene-3-carboxylic acid

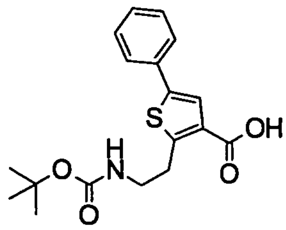

Using methyl 2-{2-[(tert-butoxycarbonyl)amino]ethyl}-5-phenylthiophene-3-carboxylate (2.19 g, 6.06 mmol) obtained in Reference Example 108, the title compound (1.61 g, yield 76%) was obtained as a yellow solid by an operation similar to that of Reference Example 45.
$^1$H NMR (DMSO-$d_6$) δ1.36 (9H, s), 3.21-3.30 (4H, m), 6.96-7.03 (1H, m), 7.29-7.34 (1H, m), 7.39-7.45 (2H, m), 7.62 (2H, d, J = 7.7 Hz), 7.66 (1H, s).
EI(pos) 348 $[M+H]^+$

Reference Example 110

tert-butyl 4-(3-ethyl-2,4-dioxo-1-oxa-3,7-diazaspiro[4.5]dec-7-yl)piperidine-1-carboxylate

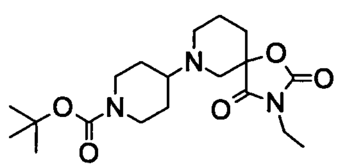

Using 3-ethyl-1-oxa-3,7-diazaspiro[4.5]decane-2,4-dione (1.28 g, 6.45 mmol) obtained in Reference Example 70, the title compound (2.17 g, yield 88%) was obtained as an oil by an operation similar to that of Reference Example 52.
EI(pos) 382 $[M+H]^+$

Reference Example 111

3-ethyl-7-(piperidin-4-yl)-1-oxa-3,7-diazaspiro[4.5]decane-2,4-dione dihydrochloride

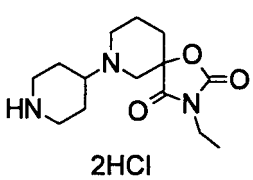

2HCl

Using tert-butyl 4-(3-ethyl-2,4-dioxo-1-oxa-3,7-diazaspiro[4.5]dec-7-yl)piperidine-1-carboxylate (2.17 g, 5.69 mmol) obtained in Reference Example 110, the title compound (1.98 g, yield 98%) was obtained as a white solid by an operation similar to that of Reference Example 53.
EI(pos) 282 $[M+H]^+$

Reference Example 112

tert-butyl 4-(3-ethyl-2-methyl-4-oxo-1,3,7-triazaspiro[4.5]dec-1-en-7-yl)piperidine-1-carboxylate

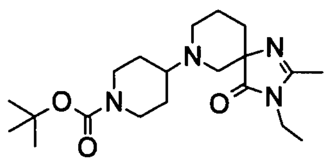

Using tert-butyl 4-(2-methyl-4-oxo-1,3,7-triazaspiro[4.5]dec-1-en-7-yl)piperidine-1-carboxylate (1.23 g, 3.51 mmol) obtained in Reference Example 99 and iodoethane (0.42 mL, 5.27 mmol), the title compound (1.13 g, yield 85%) was obtained as an oil by an operation similar to that of Reference Example 100.
EI(pos) 379 $[M+H]^+$

Reference Example 113

methyl 5-phenyl-2-(pyridin-3-yl)thiophene-3-carboxylate

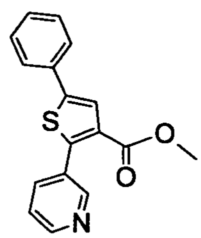

Using methyl 2-iodo-5-phenylthiophene-3-carboxylate (1.22 g, 3.55 mmol) obtained in Reference Example 105 and 3-pyridineboronic acid (872 mg, 7.09 mmol), the title compound (0.73 g, yield 70%) was obtained as an oil by an operation similar to that of Reference Example 84.
$^1H$ NMR ($CDCl_3$) δ3.77 (3H, s), 7.32-7.45 (4H, m), 7.60-7.64 (2H, m), 7.76 (1H, s), 7.87-7.91 (1H, m), 8.64 (1H, dd, J = 4.8, 1.6 Hz), 8.76 (1H, d, J = 2.4 Hz).
EI(pos) 296 $[M+H]^+$

Reference Example 114

5-phenyl-2-(pyridin-3-yl)thiophene-3-carboxylic acid

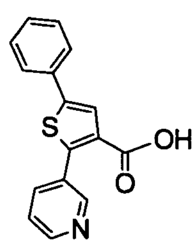

Using methyl 5-phenyl-2-(pyridin-3-yl)thiophene-3-carboxylate (0.73 g, 2.47 mmol) obtained in Reference Example 113, the title compound (0.69 g, quantitative) was obtained as a yellow solid by an operation similar to that of Reference Example 45.
$^1$H NMR (DMSO-$d_6$) δ7.35-7.41 (1H, m), 7.44-7.51 (3H, m), 7.72-7.75 (2H, m), 7.86 (1H, s), 7.96-8.00 (1H, m), 8.61 (1H, dd, J = 4.8, 1.6 Hz), 8.74 (1H, d, J = 1.7 Hz), 12.91 (1H, br). EI(pos) 282 $[M+H]^+$

Reference Example 115

3-ethyl 1'-tert-butyl 3-[2-(ethylamino)-2-oxoethyl]-1,4'-bipiperidine-1',3-dicarboxylate

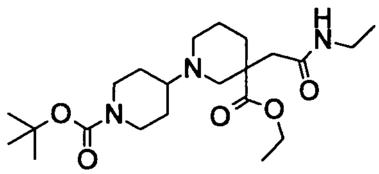

Using 3-ethyl 1-tert-butyl 3-[2-(ethylamino)-2-oxoethyl]piperidine-1,3-dicarboxylate (3.69 g, 10.8 mmol) obtained in Reference Example 33, the title compound (1.32 g, yield 29%) was obtained as an oil by an operation similar to that of Reference Example 52.
EI(pos) 426 $[M+H]^+$

Reference Example 116

ethyl 1'-({2-[(tert-butoxycarbonyl)amino]-1-benzothien-3-yl}carbonyl)-3-[2-(ethylamino)-2-oxoethyl]-1,4'-bipiperidine-3-carboxylate

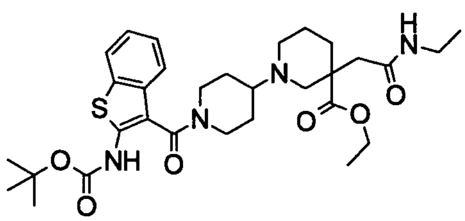

Using 3-ethyl 1'-tert-butyl 3-[2-(ethylamino)-2-oxoethyl]-1,4'-bipiperidine-1',3-dicarboxylate (1.32 g, 3.10 mmol) obtained in Reference Example 115, the title compound (0.57 g, yield 31%) was obtained as an oil by successively performing operations similar to those of Reference Examples 53 and 8.
EI(pos) 601 $[M+H]^+$

Reference Example 117

ethyl 1'-{[2-(carbamoylamino)-1-benzothien-3-yl]carbonyl}-3-[2-(ethylamino)-2-oxoethyl]-1,4'-bipiperidine-3-carboxylate

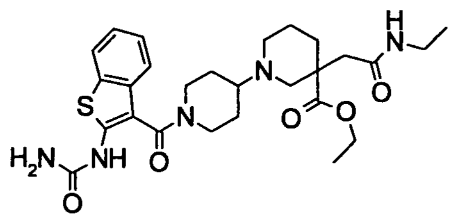

A solution of ethyl 1'-({2-[(tert-butoxycarbonyl)amino]-1-benzothien-3-yl}carbonyl)-3-[2-(ethylamino)-2-oxoethyl]-1,4'-bipiperidine-3-carboxylate (0.32 g, 0.533 mmol) obtained in Reference Example 116 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure. To a solution of the obtained crude product in tetrahydrofuran (10 mL) was added trichloroacetyl isocyanate (0.13 mL, 1.07 mmol) at 0˚C, and the mixture was stirred at room temperature for 10 min. A 7N ammonia methanol solution (3 mL) was added thereto, and the mixture was stirred at room temperature for 2 hr. The solvent was evaporated under reduced pressure, and the obtained residue was purified by basic silica gel column chromatography (developing solvent; methanol:ethyl acetate=0:1 →1:9) to give the title compound (270 mg, yield 93%) as an oil. EI(pos) 544 $[M+H]^+$

Reference Example 118

2-iodo-5-phenylthiophene-3-carboxylic acid

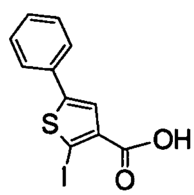

Using methyl 2-iodo-5-phenylthiophene-3-carboxylate (1.37 g, 3.98 mmol) obtained in Reference Example 105, the title compound (1.01 g, yield 77%) was obtained as a yellow solid by an operation similar to that of Reference Example 45.
$^1$H NMR (DMSO-$d_6$) δ7.33-7.46 (3H, m), 7.60 (1H, s), 7.64-7.67 (2H, m), 13.01 (1H, br).
EI(pos) 331 $[M+H]^+$

Reference Example 119

ethyl 1-benzoyl-3-(3-phenylpropyl)piperidine-3-carboxylate

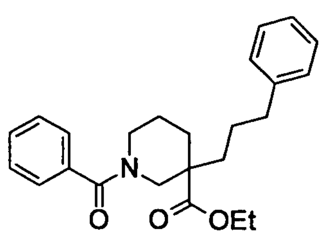

Using ethyl 1-benzoylpiperidine-3-carboxylate (5.00 g, 19.1 mmol) and (3-bromopropyl)benzene (3.20 mL, 21.0 mmol), the title compound (2.94 g, yield 41%) was obtained as an oil by an operation similar to that of Reference

Example 1.
EI(pos) 380 [M+H]+

Reference Example 120

1-benzoyl-3-(3-phenylpropyl)piperidine-3-carboxylic acid

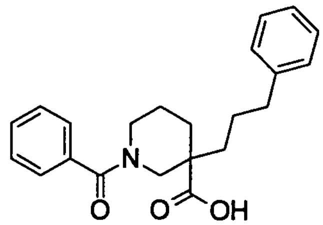

Using ethyl 1-benzoyl-3-(3-phenylpropyl)piperidine-3-carboxylate (2.94 g, 7.75 mmol) obtained in Reference Example 119, the title compound (2.36 g, yield 87%) was obtained as an oil by an operation similar to that of Reference Example 45. This was used in the next step without further purification.

Reference Example 121

1'-benzoyl-8,9-dihydrospiro[benzo[7]annulene-6,3'-piperidin]-5(7H)-one

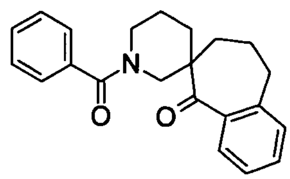

Using 1-benzoyl-3-(3-phenylpropyl)piperidine-3-carboxylic acid (2.36 g, 6.72 mmol) obtained in Reference Example 120, the title compound (1.25 g, yield 56%) was obtained as an oil by an operation similar to that of Reference Example 46. EI(pos) 334 [M+H]+

Reference Example 122

8,9-dihydrospiro[benzo[7]annulene-6,3'-piperidin]-5(7H)-one

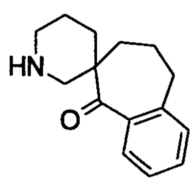

Using 1'-benzoyl-8,9-dihydrospiro[benzo[7]annulene-6,3'-piperidin]-5(7H)-one (1.25 g, 3.75 mmol) obtained in Reference Example 121, the title compound (674 mg, yield 78%) was obtained as an oil by an operation similar to that of Reference Example 47. This was used in the next step without further purification.

Reference Example 123

2-amino-6-(tert-butoxycarbonyl)-4,5,6,7-tetrahydrothieno[2,3-c]pyridine-3-carboxylic acid

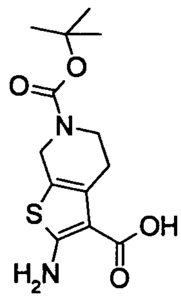

To a solution of 6-tert-butyl 3-ethyl 2-amino-4,7-dihydrothieno[2,3-c]pyridine-3,6(5H)-dicarboxylate (5.00 g, 15.3 mmol) in ethanol (80 mL) was added 2N aqueous sodium hydroxide solution (38.3 mL, 76.6 mmol), and the mixture was stirred at 80˚C for 5 hr. The solvent was evaporated under reduced pressure, ad the residue was adjusted to pH 3 with 0.5N hydrochloric acid. The mixture was extracted with ethyl acetate, and the extract was washed with saturated brine and dried over anhydrous sodium sulfate. The solvent was evaporated under reduced pressure, and the obtained residue was purified by silica gel column chromatography (developing solvent; ethyl acetate) and triturated with diisopropyl ether to give the title compound (3.59 g, yield 79%). This was used in the next step without further purification.

Reference Example 124

2-amino-5-bromonicotinic acid

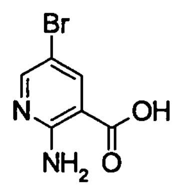

To a solution of methyl 2-amino-5-bromonicotinate (3.00 g, 13.0 mmol) in methanol (65 mL) was added 2N aqueous sodium hydroxide solution (32.5 mL, 65.0 mmol), and the mixture was stirred at 80˚C for 3 hr. The solvent was evaporated under reduced pressure, and 1N hydrochloric acid (65.0 mL) was added to the residue. The obtained precipitate was washed with water, acetone and diisopropyl ether to give the title compound (1.38 g, yield 49%). This was used in the next step without further purification.

Reference Example 125

tert-butyl 3-aminoisoquinoline-4-carboxylate

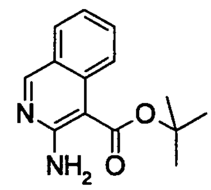

To a solution of 1-(2-iodophenyl)methanamine (2.90 g, 12.4 mmol), tert-butyl cyanoacetate (3.51 g, 24.9 mmol) and diisopropylethylamine (4.34 mL, 24.9 mmol) in dimethyl sulfide (60 mL) was added copper bromide (I) (3.57 g, 24.9 mmol) under a nitrogen atmosphere, and the mixture was stirred at room temperature for 3 hr. A 10% aqueous ammonia solution and diethyl ether were added, and the mixture was stirred for 16 hr. The mixture was extracted with diethyl ether, washed with saturated brine, and dried over anhydrous sodium sulfate. The solvent was evaporated under reduced pressure, and the obtained residue was purified by silica gel column chromatography (developing solvent; hexane:ethyl acetate=3:1→ 1:1) and triturated with a small amount of hexane to give the title compound (282 mg, yield 9%).
$^1$H NMR (DMSO-$d_6$) δ1.69 (9H, s), 6.50 (2H, s), 7.28 (1H, m), 7.58 (1H, m), 7.74 (1H, d, J = 8.4 Hz), 8.56 (1H, d, J = 8.7 Hz), 8.85 (1H, s).

Reference Example 126

3-aminoisoquinoline-4-carboxylic acid trifluoroacetate

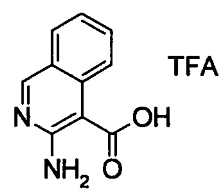

tert-Butyl 3-aminoisoquinoline-4-carboxylate (270 mg, 1.11 mmol) obtained in Reference Example 125 was dissolved in trifluoroacetic acid (5 mL), and concentrated under reduced pressure 3 hr later. The obtained residue was triturated with diisopropyl ether to give the title compound (310 mg, yield 93%). This was used in the next step without further purification.

Reference Example 127

2-amino-5-(tert-butoxycarbonyl)-4-methylthiophene-3-carboxylic acid

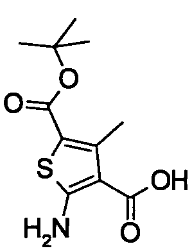

Using 2-tert-butyl 4-ethyl 5-amino-3-methylthiophene-2,4-dicarboxylate (500 mg, 1.75 mmol), the title compound (354 mg, yield 78%) was obtained by an operation similar to that of Reference Example 45 and triturated with hexane. This was used in the next step without further purification.

Reference Example 128

2-amino-4-(4-fluorophenyl)thiophene-3-carboxylic acid

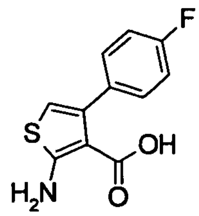

Using ethyl 2-amino-4-(4-fluorophenyl)thiophene-3-carboxylate (982 mg, 3.70 mmol), the title compound (354 mg, yield 26%) was obtained by an operation similar to that of Reference Example 45 and triturated with diisopropyl ether:hexane=1:1. This was used in the next step without further purification.

Reference Example 129

3-tert-butyl 6-ethyl 2-amino-4,5,6,7-tetrahydro-1-benzothiophene-3,6-dicarboxylate

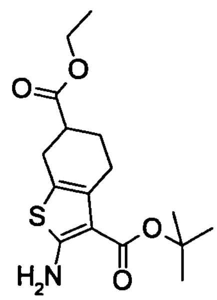

A solution of ethyl 4-oxocyclohexanecarboxylate (25.7 g, 151 mmol), tert-butyl cyanoacetate (25.7 g, 151 mmol), ammonium chloride (25.7 g, 151 mmol) and acetic acid (25.7 g, 151 mmol) in toluene (50 mL) was heated under reflux with a Dean-Stark trap. After cooling to room temperature, the mixture was concentrated under reduced pressure. A saturated aqueous sodium hydrogencarbonate solution was added, and the mixture was extracted with ethyl acetate. The extract was washed 3 times with saturated brine and dried over anhydrous magnesium sulfate. The solvent was evaporated under reduced pressure to give an oil (42.6 g, 145 mmol). To a solution of the oil and sulfur (4.66 g, 145 mmol) in ethanol (150 mL) was added diethylamine (15 mL, 145 mmol), and the mixture was stirred at 60°C for 3 hr. After cooling to room temperature, the mixture was concentrated under reduced pressure. A saturated aqueous sodium hydrogencarbonate solution was added, and the mixture was extracted with ethyl acetate. The extract was washed 3 times with saturated brine and dried over anhydrous magnesium sulfate. The solvent was evaporated under reduced pressure, and the obtained residue was purified by silica gel column chromatography (developing solvent; hexane: chloroform =3:7→0:1) and recrystallized from ether-hexane to give the title compound (22.8 g, yield 46%).
$^1H$ NMR ($CDCl_3$) δ1.27 (3H, t, J = 7.2 Hz), 1.54 (9H, s), 1.72-1.85 (1H, m), 2.14-2.21 (1H, m), 2.57-2.77 (4H, m), 2.87-2.96 (1H, m), 4.16 (2H, q, J = 7.2 Hz), 5.89 (2H, s).

Reference Example 130

2-amino-6-(ethoxycarbonyl)-4,5,6,7-tetrahydro-1-benzothiophene-3-carboxylic acid

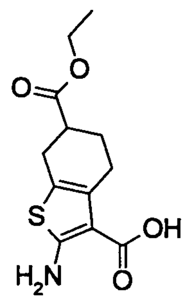

Using 3-tert-butyl 6-ethyl 2-amino-4,5,6,7-tetrahydro-1-benzothiophene-3,6-dicarboxylate (1.50 g, 4.61 mmol) obtained in Reference Example 129, the title compound (1.02 g, yield 82%) was obtained by an operation similar to that of Reference Example 57 and trituration with hexane:ethyl acetate=3:1. This was used in the next step without further purification.

Reference Example 131

2-amino-4,7-dihydro-5H-thieno[2,3-c]pyran-3-carboxylic acid

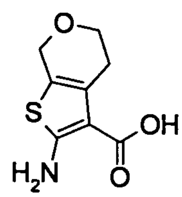

Using ethyl 2-amino-4,7-dihydro-5H-thieno[2,3-c]pyran-3-carboxylate (2.00 g, 8.80 mmol), the title compound (907 mg, yield 52%) was obtained by an operation similar to that of Reference Example 45 and trituration with ethyl acetate. This was used in the next step without further purification.

Reference Example 132

2-amino-4,7-dihydro-5H-thieno[2,3-c]thiopyran-3-carboxylic acid

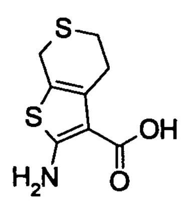

Using ethyl 2-amino-4,7-dihydro-5H-thieno [2,3-c]thiopyran-3-carboxylate (3.00 g, 12.3 mmol), the title compound (2.23 g, yield 84%) was obtained by an operation similar to that of Reference Example 45 and trituration with ethyl acetate. This was used in the next step without further purification.

Reference Example 133

2-amino-7-oxo-4,5,6,7-tetrahydro-1-benzothiophene-3-carboxylic acid

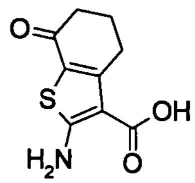

Using ethyl 2-amino-7-oxo-4,5,6,7-tetrahydro-1-benzothiophene-3-carboxylate (774 mg, 3.23 mmol), the title compound (116 mg, yield 17%) was obtained by an operation similar to that of Reference Example 45 and trituration with diisopropyl ether.
$^1$H NMR ($CDCl_3$) δ1.99 (2H, m), 2.37 (2H, t, J = 6.3 Hz), 2.93 (2H, t, J = 6.0 Hz), 8.24 (2H, s), 12.53 (1H, s).

Reference Example 134

3-amino-5-phenylthiophene-2-carboxylic acid

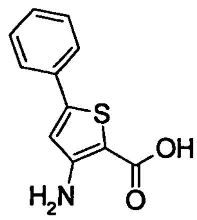

Using ethyl 3-amino-5-phenylthiophene-2-carboxylate (1.00 g, 4.29 mmol), the title compound (745 mg, yield 79%) was obtained by an operation similar to that of Reference Example 45 and trituration with diisopropyl ether. This was used in the next step without further purification.

Reference Example 135

5-amino-2-phenyl-1,3-thiazole-4-carboxylic acid

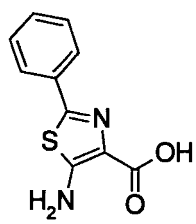

Using ethyl 5-amino-2-phenyl-1,3-thiazole-4-carboxylate (480 mg, 1.93 mmol), the title compound (338 mg, yield 79%) was obtained by an operation similar to that of Reference Example 45 and trituration with diisopropyl ether.
$^1$H NMR (DMSO-$d_6$) δ7.35-7.46 (3H, m), 7.50 (2H, s), 7.73 (2H, m), 12.17 (1H, s).

Reference Example 136

5-(acetyloxy)-2-{[(benzyloxy)carbonyl]amino}benzoic acid

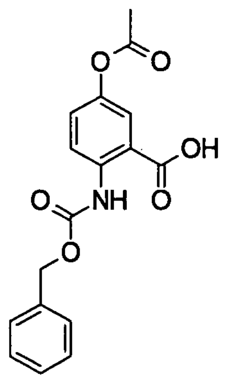

2-{[(Benzyloxy)carbonyl]amino}-5-hydroxybenzoic acid (6.71 g, 23.4 mmol) was dissolved in pyridine (60 mL), acetic anhydride (2.65 mL, 28.0 mmol) was added, and the mixture was stirred at room temperature for 16 hr. The solvent was evaporated under reduced pressure, and the obtained residue was dissolved in ethyl acetate. The mixture was washed with 1N hydrochloric acid and saturated brine and dried over anhydrous sodium sulfate. The solvent was evaporated under reduced pressure, and the obtained residue was triturated with diisopropyl ether to give the title compound (4.04 g, yield 53%).
$^1$H NMR (DMSO-$d_6$) δ2.27 (3H, s), 5.19 (2H, s), 7.37-7.42 (6H, m), 7.70 (1H, d, J = 3.0 Hz), 8.28 (1H, d, J = 9.3 Hz), 10.71 (1H, s) .

Reference Example 137

4-benzyl 1-tert-butyl 4-hydroxypiperidine-1,4-dicarboxylate

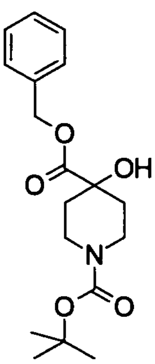

To methyl 1-benzyl-4-hydroxypiperidine-4-carboxylate (6.49 g, 26.0 mmol) and 20% palladium hydroxide (2.0 g) was added methanol (200 mL), and the mixture was stirred at room temperature for 16 hr. Palladium hydroxide was filtered through celite, and the filtrate was concentrated under reduced pressure. The obtained residue was dissolved in tetrahydrofuran (100 mL), and di-tert-butyl bicarbonate (5.98 mL, 26.0 mmol) was added. The mixture was stirred at room temperature for 3 hr and concentrated under reduced pressure. The obtained residue was purified by basic silica gel column chromatography (developing solvent; ethyl acetate) and silica gel column chromatography (developing solvent; ethyl acetate) to give 1-tert-butyl 4-methyl 4-hydroxypiperidine-1,4-dicarboxylate (6.75 g, quantitative). The residue was dissolved in methanol (65 mL), and 2N aqueous sodium hydroxide solution (15.6 mL) was added. After stirring for 16 hr, benzyl bromide (3.72 mL, 31.2 mmol) was added at 0˚C, and the mixture was stirred at room temperature for 16 hr. The solvent was evaporated under reduced pressure, and the obtained residue was dissolved in ethyl acetate. The residue was washed with aqueous potassium carbonate solution and saturated brine and dried over anhydrous sodium sulfate. The solvent was evaporated under reduced pressure and the residue was purified by silica gel column chromatography (developing solvent; hexane:ethyl acetate=9:1→2:1) to give the title compound (2.81 g, yield 32%).
$^1$H NMR ($CDCl_3$) δ1.46 (9H, s), 1.57-1.61 (2H, m), 1.92-2.02 (2H, m), 3.04 (1H, br), 3.10-3.18 (2H, m), 3.96 (2H, m), 5.21 (2H, s), 7.32-7.42 (5H, m).

Reference Example 138

benzyl 4-hydroxypiperidine-4-carboxylate hydrochloride

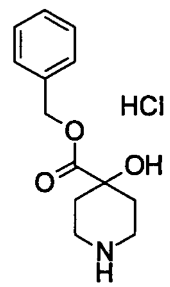

To 4-benzyl 1-tert-butyl 4-hydroxypiperidine-1,4-dicarboxylate (2.81 g, 8.38 mmol) obtained in Reference Example 137 was added 4N hydrogen chloride-ethyl acetate (30 mL), and the mixture was stirred for 1 hr. The reaction mixture was concentrated under reduced pressure, and triturated with diisopropyl ether to give the title compound (2.04 g, yield 90%). This was used in the next step without further purification.

Reference Example 139

3,3-dimethyl-7-(piperidin-4-yl)-2-oxa-7-azaspiro[4.5]decan-1-one ditrifluoroacetate

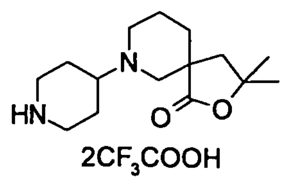

To tert-butyl 4-(3,3-dimethyl-1-oxo-2-oxa-7-azaspiro[4.5]dec-7-yl)piperidine-1-carboxylate (2.20 g, 6.0 mmol) obtained in Reference Example 52 was added trifluoroacetic acid (10 mL), and the mixture was stirred at room temperature for 0.5 hr. The reaction mixture was concentrated under reduced pressure to give the title compound (2.20 g, yield 74%) as white crystals. This was used in the next step without further purification.
$^1$H-NMR (DMSO-$d_6$) δ1.42 (3H, s), 1.45 (3H, s), 1.83-2.03 (6H, m), 2.18-2.34 (3H, m), 2.86-3.05 (5H, m), 3.36-3.50 (5H, m).

Reference Example 140

tert-butyl 3,3-dimethyl-1-oxo-2-oxa-7-azaspiro[4.5]decane-7-carboxylate

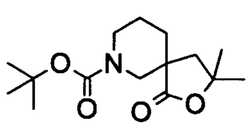

To a solution of 3-ethyl 1-tert-butyl piperidine-1,3-dicarboxylate (32.4 g, 126 mmol) in THF (150 mL) was added a 1.0 M lithium bis(trimethylsilyl)amide-THF solution (164 mL, 164 mmol) at -78°C, and the mixture was stirred at the same temperature for 30 min. Isobutylene oxide (16.3 mL, 189 mmol) was added to the solution at -78°C. After heating

to room temperature, the mixture was stirred for 3.5 hr. The reaction mixture was dissolved in ethyl acetate, washed with saturated aqueous ammonium chloride solution and saturated brine, and dried over anhydrous magnesium sulfate. The solvent was evaporated under reduced pressure, and the obtained residue was purified by silica gel column chromatography (developing solvent; hexane:ethyl acetate=4:1→1:1) to give the title compound (24.76 g, yield 69%) as an oil.
EI(pos) 306 $[M+Na]^+$

Reference Example 141

methyl 2-[(tert-butoxycarbonyl)amino]-5-(pyridin-3-yl)thiophene-3-carboxylate

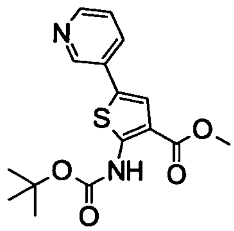

Using methyl 5-bromo-2-[(tert-butoxycarbonyl)amino]thiophene-3-carboxylate (3.50 g, 10.9 mmol) and 3-pyridineboronic acid (2.00 g, 16.3 mmol), the title compound (2.16 g, yield 59%) was obtained as a yellow solid by an operation similar to that of Reference Example 84. $^1H$ NMR ($CDCl_3$) δ1.56 (9H, s), 3.91 (3H, s), 7.28-7.31 (1H, m), 7.44 (1H, s), 7.79-7.83 (1H, m), 8.49 (1H, dd, J = 4.9, 1.5 Hz), 8.84 (1H, d, J = 1.9 Hz), 10.09 (1H, br).
EI(pos) 335 $[M+H]^+$

Reference Example 142

2-[(tert-butoxycarbonyl)amino]-5-(pyridin-3-yl)thiophene-3-carboxylic acid

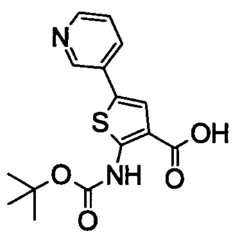

Using methyl 2-[(tert-butoxycarbonyl)amino]-5-(pyridin-3-yl)thiophene-3-carboxylate (2.16 g, 6.46 mmol) obtained in Reference Example 141, the title compound (0.55 g, yield 27%) was obtained as a yellow solid by an operation similar to that of Reference Example 45.
EI(pos) 321 $[M+H]^+$

Reference Example 143

1'-tert-butyl 3-methyl 3-hydroxy-1,4'-bipiperidine-1',3-dicarboxylate

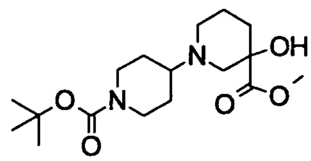

A suspension of methyl 1-benzyl-3-hydroxypiperidine-3-carboxylate (4.00 g, 16.0 mmol) and 20% palladium hydroxide on carbon (1.13 g) in ethanol (50 mL) was stirred for 2 hr under a hydrogen atmosphere. After filtration, the filtrate was concentrated under reduced pressure, and the obtained residue, 1-(tert-butoxycarbonyl)-4-piperidone (3.04 g, 15.3 mmol) and sodium triacetoxyborohydride (9.73 g, 45.9 mmol) were added under ice-cooling, and the mixture was stirred at room temperature for 1 day. A saturated aqueous sodium hydrogencarbonate solution was added to the reaction mixture, and the mixture was extracted with ethyl acetate. The organic layer was washed with saturated brine, and dried over anhydrous magnesium sulfate. The solvent was evaporated under reduced pressure, and the obtained residue was purified by silica gel column chromatography (developing solvent; methanol:ethyl acetate=0:1→1:9) to give the title compound (2.73 g, yield 52%) as a yellow oil.
EI(pos) 343 $[M+H]^+$

Reference Example 144

tert-butyl 4-(3-isopropyl-2,4-dioxo-1-oxa-3,7-diazaspiro[4.5]dec-7-yl)piperidine-1-carboxylate

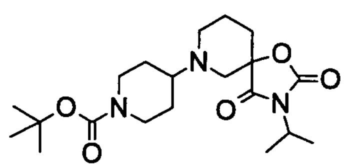

Using 1'-tert-butyl 3-methyl 3-hydroxy-1,4'-bipiperidine-1',3-dicarboxylate (1.47 g, 4.29 mmol) obtained in Reference Example 143 and isopropyl isocyanate (0.84 mL, 8.59 mmol), the title compound (1.28 g, yield 75%) was obtained as a yellow oil by an operation similar to that of Reference Example 69. EI(pos) 396 $[M+H]^+$

Reference Example 145

3-isopropyl-7-(piperidin-4-yl)-1-oxa-3,7-diazaspiro[4.5]decane-2,4-dione dihydrochloride

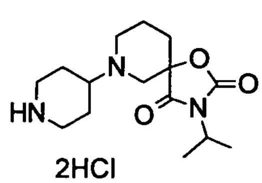

Using tert-butyl 4-(3-isopropyl-2,4-dioxo-1-oxa-3,7-diazaspiro[4.5]dec-7-yl)piperidine-1-carboxylate (1.28 g, 3.24 mmol) obtained in Reference Example 144, the title compound (1.19 g, yield 99%) was obtained as a white solid by an operation similar to that of Reference Example 53.
EI(pos) 296 $[M+H]^+$

Reference Example 146

1'-tert-butyl 3-ethyl 3-(2-amino-2-oxoethyl)-1,4'-bipiperidine-1',3-dicarboxylate

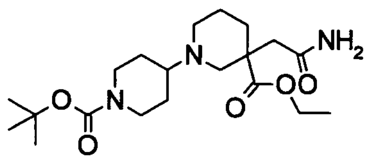

Using 3-ethyl 1-tert-butyl 3-(2-amino-2-oxoethyl)piperidine-1,3-dicarboxylate (2.76 g, 8.78 mmol) obtained in Reference Example 39, the title compound (2.26 g, yield 64%) was obtained as an oil by an operation similar to that of Reference Example 52.
EI(pos) 398 $[M+H]^+$

Reference Example 147

ethyl 3-(2-amino-2-oxoethyl)-1,4'-bipiperidine-3-carboxylate dihydrochloride

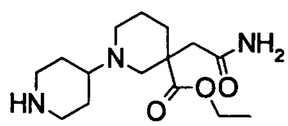

2HCl

Using 1'-tert-butyl 3-ethyl 3-(2-amino-2-oxoethyl)-1,4'-bipiperidine-1',3-dicarboxylate (2.26 g, 5.69 mmol) obtained in Reference Example 146, the title compound (2.11 g, yield 99%) was obtained as a white solid by an operation similar to that of Reference Example 53.
EI(pos) 298 $[M+H]^+$

Reference Example 148

ethyl 1'-[(2-amino-1-benzothien-3-yl)carbonyl]-3-(2-amino-2-oxoethyl)-1,4'-bipiperidine-3-carboxylate

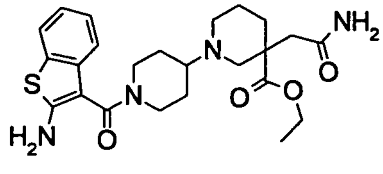

A solution of ethyl 3-(2-amino-2-oxoethyl)-1,4'-bipiperidine-3-carboxylate dihydrochloride (1.10 g, 2.97 mmol) obtained in Reference Example 147, 2-amino-1-benzothiophene-3-carboxylic acid (0.57 g, 2.97 mmol) obtained in Reference Example 51, 1-hydroxybenzotriazole (603 mg, 4.46 mmol) and 1-ethyl-3-(3'-dimethylaminopropyl)carbodiimide hydrochloride (855 mg, 4.46 mmol) in DMF (10 mL) was stirred at room temperature for 6 hr. The reaction mixture was diluted with ethyl acetate, washed with aqueous sodium hydrogencarbonate solution and saturated brine, and dried over anhydrous sodium sulfate. The solvent was evaporated under reduced pressure, and the obtained residue was

purified by basic silica gel column chromatography (developing solvent; ethyl acetate) to give the title compound (1.29 g, yield 92%) as a yellow oil. EI(pos) 473 $[M+H]^+$

Reference Example 149

ethyl 3-(2-amino-2-oxoethyl)-1'-[(2-{[(ethylamino)carbonyl]amino}-l-benzothien-3-yl)carbonyl]-1,4'-bipiperidine-3-carboxylate

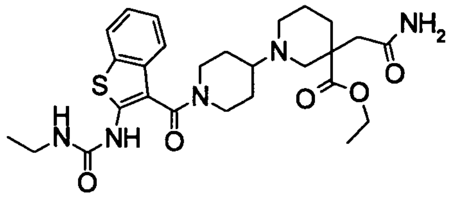

To a solution of ethyl 1'-[(2-amino-1-benzothien-3-yl)carbonyl]-3-(2-amino-2-oxoethyl)-1,4'-bipiperidine-3-carboxylate (1.29 g, 2.73 mmol) obtained in Reference Example 148 in pyridine (15 mL) was added ethyl isocyanate (0.43 mL, 5.46 mmol), and the mixture was stirred at 80°C for 13 hr. The solvent was evaporated under reduced pressure, and the obtained solid was washed with diethyl ether to give the title compound (0.74 g, yield 50%) as a white solid.
EI(pos) 544 $[M+H]^+$

Reference Example 150

benzyl 4-(3,3-dimethyl-1-oxo-2-oxa-7-azaspiro[4.5]dec-7-yl)piperidine-1-carboxylate

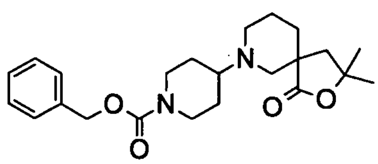

tert-Butyl 3,3-dimethyl-1-oxo-2-oxa-7-azaspiro[4.5]decane-7-carboxylate (13.2 g, 46.7 mmol) obtained in Reference Example 19 or Reference Example 140 was dissolved in 4N hydrogen chloride-ethyl acetate (200 mL), and the mixture was stirred at room temperature for 30 min. The reaction solution was concentrated under reduced pressure, and the obtained residue was dissolved in dichloromethane (150 mL). To the solution were added triethylamine (6.5 mL, 46.7 mmol), N-benzyloxycarbonyl-4-piperidone (10.9 g, 46.7 mmol) and sodium triacetoxyborohydride (29.7 g, 140 mmol) under ice-cooling, and the mixture was stirred at room temperature for 3 days. A saturated aqueous sodium hydrogencarbonate solution was added to the reaction mixture, and the mixture was extracted with ethyl acetate. The organic layer was washed with saturated brine, and dried over anhydrous magnesium sulfate. The solvent was evaporated under reduced pressure, and the obtained residue was purified by basic silica gel column chromatography (developing solvent; hexane:ethyl acetate=4:1→3:2) to give the title compound (13.4 g, yield 71%) as a yellow oil.
EI(pos) 401 $[M+H]^+$

Reference Example 151

Optically active forms (two kinds) of benzyl 4-(3,3-dimethyl-1-oxo-2-oxa-7-azaspiro[4.5]dec-7-yl)piperidine-1-carboxylate

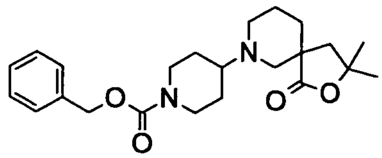

Benzyl 4-(3,3-dimethyl-1-oxo-2-oxa-7-azaspiro[4.5]dec-7-yl)piperidine-1-carboxylate (racemate, 13 g) obtained in Reference Example 150 was separated by high performance liquid chromatography (column; CHIRALPAK AS (50 mmID × 500 mmL), temperature; 25°C, mobile phase; hexane:ethanol=9:1, flow rate; 60 mL/min to 90 mL/min, detection wavelength; 220 nm) to give the two title compounds [retention time, short (5.91 g) and retention time, long (6.01 g)].

Reference Example 152

3,3-dimethyl-7-(piperidin-4-yl)-2-oxa-7-azaspiro[4.5]decan-1-one dihydrochloride

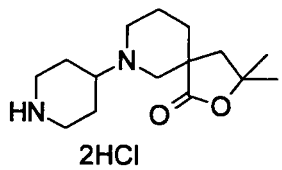

A suspension of benzyl 4-(3,3-dimethyl-1-oxo-2-oxa-7-azaspiro[4.5]dec-7-yl)piperidine-1-carboxylate (retention time, short: 5.91 g, 14.8 mmol) obtained in Reference Example 151 and 10% palladium carbon (50% water-containing product, 1.58 g) in ethanol (50 mL) was stirred at room temperature for 1.5 hr under a hydrogen atmosphere. After filtration, the filtrate was concentrated under reduced pressure. The obtained residue was dissolved in 4N hydrogen chloride-ethyl acetate (20 mL), and the mixture was stirred at room temperature for 30 min. The precipitated solid was collected by filtration and washed with diethyl ether to give the title compound (4.80 g, yield 95%) as a white solid.
EI(pos) 267 $[M+H]^+$

Reference Example 153

3,3-dimethyl-7-(piperidin-4-yl)-2-oxa-7-azaspiro[4.5]decan-1-one dihydrochloride

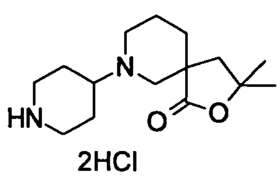

A suspension of benzyl 4-(3,3-dimethyl-1-oxo-2-oxa-7-azaspiro[4.5]dec-7-yl)piperidine-1-carboxylate (retention time, long: 6.01 g, 15.0 mmol) obtained in Reference Example 151 and 10% palladium carbon (50% water-containing product, 1.60 g) in ethanol (50 mL) was stirred at room temperature for 1.5 hr under a hydrogen atmosphere. After filtration, the filtrate was concentrated under reduced pressure. The obtained residue was dissolved in 4N hydrogen chloride-ethyl acetate (20 mL), and the mixture was stirred at room temperature for 30 min. The precipitated solid was collected by filtration and washed with diethyl ether to give the title compound (4.72 g, yield 93%) as a white solid.
EI(pos) 267 $[M+H]^+$

Reference Example 154

tert-butyl 3-ethyl-2,4-dioxo-1,3,7-triazaspiro[4.5]decane-7-carboxylate

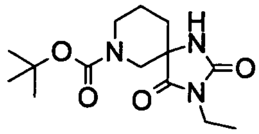

To a solution of tert-butyl 2,4-dioxo-1,3,7-triazaspiro[4.5]decane-7-carboxylate (6.75 g, 25.1 mmol) and potassium carbonate (6.9 g, 50.2 mmol) in DMF (75 mL) was added iodoethane (3.4 mL, 42.2 mmol), and the mixture was stirred at room temperature for 1 hr. The reaction mixture was diluted with ethyl acetate, washed with aqueous sodium hydrogencarbonate solution and saturated brine, and dried over anhydrous sodium sulfate. The solvent was evaporated under reduced pressure, and the obtained solid was washed with ethyl acetate to give the title compound (2.39 g, yield 32%) as a white solid.
EI(pos) 198 $[M-Boc]^+$

Reference Example 155

tert-butyl 4-(3-ethyl-2,4-dioxo-1,3,7-triazaspiro[4.5]dec-7-yl)piperidine-1-carboxylate

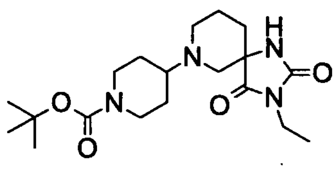

Using tert-butyl 3-ethyl-2,4-dioxo-1,3,7-triazaspiro[4.5]decane-7-carboxylate (2.39 g, 8.04 mmol) obtained in Reference Example 154, the title compound (2.26 g, yield 73%) was obtained as an oil by an operation similar to that of Reference Example 52.
EI(pos) 381 $[M+H]^+$

Reference Example 156

3-ethyl-7-(piperidin-4-yl)-1,3,7-triazaspiro[4.5]decane-2,4-dione dihydrochloride

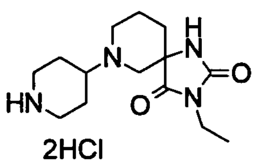

Using tert-butyl 4-(3-ethyl-2,4-dioxo-1,3,7-triazaspiro[4.5]dec-7-yl)piperidine-1-carboxylate (2.26 g, 5.94 mmol) obtained in Reference Example 155, the title compound (2.09 g, yield 99%) was obtained as a white solid by an operation similar to that of Reference Example 53.
EI(pos) 281 $[M+H]^+$

Reference Example 157

Optically active forms (two kinds) of 1'-tert-butyl 3-methyl 3-hydroxy-1,4'-bipiperidine-1',3-dicarboxylate

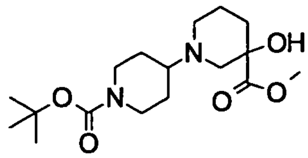

1'-tert-Butyl 3-methyl 3-hydroxy-1,4'-bipiperidine-1',3-dicarboxylate (racemate, 7.0 g) obtained in Reference Example 143 was separated by high performance liquid chromatography (column; CHIRALPAK AD (50 mmID × 500 mmL), temperature; 30˚C, mobile phase; hexane:ethanol=9:1, flow rate; 80 mL/min, detection wavelength; 220 nm) to give the two title compounds [retention time, short (2.93 g) and retention time, long (2.98 g)].

Reference Example 158

tert-butyl 4-(3-ethyl-2,4-dioxo-1-oxa-3,7-diazaspiro[4.5]dec-7-yl)piperidine-1-carboxylate

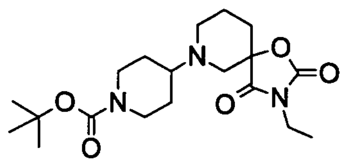

Using 1'-tert-butyl 3-methyl 3-hydroxy-1,4'-bipiperidine-1',3-dicarboxylate (retention time, short: 1.00 g, 2.92 mmol) obtained in Reference Example 157, the title compound (1.11 g, yield 99%) was obtained as a yellow oil by an operation similar to that of Reference Example 69.
EI(pos) 382 $[M+H]^+$

Reference Example 159

3-ethyl-7-(piperidin-4-yl)-1-oxa-3,7-diazaspiro[4.5]decane-2,4-dione dihydrochloride

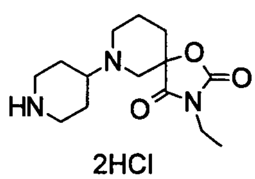

2HCl

Using tert-butyl 4-(3-ethyl-2,4-dioxo-1-oxa-3,7-diazaspiro[4.5]dec-7-yl)piperidine-1-carboxylate (1.11 g, 2.91 mmol) obtained in Reference Example 158, the title compound (0.95 g, yield 80%) was obtained as a white solid by an operation similar to that of Reference Example 53.
EI(pos) 282 $[M+H]^+$

Reference Example 160

tert-butyl 4-(3-ethyl-2,4-dioxo-1-oxa-3,7-diazaspiro[4.5]dec-7-yl)piperidine-1-carboxylate

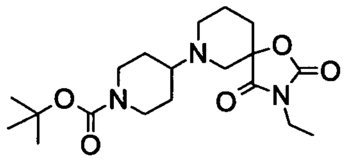

Using 1'-tert-butyl 3-methyl 3-hydroxy-1,4'-bipiperidine-1',3-dicarboxylate (retention time, long: 1.00 g, 2.92 mmol) obtained in Reference Example 157, the title compound (1.11 g, yield 99%) was obtained as a yellow oil by an operation similar to that of Reference Example 69.
EI(pos) 382 $[M+H]^+$

Reference Example 161

3-ethyl-7-(piperidin-4-yl)-1-oxa-3,7-diazaspiro[4.5]decane-2,4-dione dihydrochloride

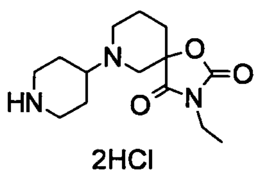

2HCl

Using tert-butyl 4-(3-ethyl-2,4-dioxo-1-oxa-3,7-diazaspiro[4.5]dec-7-yl)piperidine-1-carboxylate (1.11 g, 2.91 mmol) obtained in Reference Example 160, the title compound (0.88 g, yield 85%) was obtained as a white solid by an operation similar to that of Reference Example 53.
EI(pos) 282 $[M+H]^+$

Reference Example 162

tert-butyl 4-(2,4-dioxo-3-propyl-1-oxa-3,7-diazaspiro[4.5]dec-7-yl)piperidine-1-carboxylate

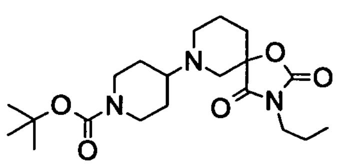

Using 1'-tert-butyl 3-methyl 3-hydroxy-1,4'-bipiperidine-1',3-dicarboxylate (1.50 g, 4.38 mmol) obtained in Reference Example 143 and propyl isocyanate (0.82 mL, 8.77 mmol), the title compound (1.26 g, yield 73%) was obtained as a yellow oil by an operation similar to that of Reference Example 69. EI(pos) 396 $[M+H]^+$

Reference Example 163

7-(piperidin-4-yl)-3-propyl-1-oxa-3,7-diazaspiro[4.5]decane-2,4-dione dihydrochloride

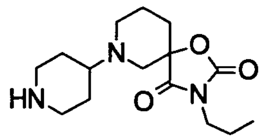

2HCl

Using tert-butyl 4-(2,4-dioxo-3-propyl-1-oxa-3,7-diazaspiro[4.5]dec-7-yl)piperidine-1-carboxylate (1.26 g, 3.18 mmol) obtained in Reference Example 162, the title compound (1.17 g, yield 99%) was obtained as a white solid by an operation similar to that of Reference Example 53. This was used in the next step without further purification.

Reference Example 164

tert-butyl 2-methyl-1-oxo-2,7-diazaspiro[4.5]decane-7-carboxylate

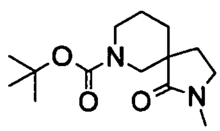

Using tert-butyl 1-oxo-2,7-diazaspiro[4.5]decane-7-carboxylate (1.53 g, 6.04 mmol) obtained in Reference Example 2 and methyl iodide (0.75 mL, 12.1 mmol), the title compound (1.61 g, yield 99%) was obtained as a yellow oil by an operation similar to that of Reference Example 3.
EI(pos) 291 $[M+Na]^+$

Reference Example 165

tert-butyl 4-(2-methyl-1-oxo-2,7-diazaspiro[4.5]dec-7-yl)piperidine-1-carboxylate

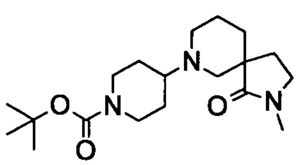

Using tert-butyl 2-methyl-1-oxo-2,7-diazaspiro[4.5]decane-7-carboxylate (1.61 g, 6.02 mmol) obtained in Reference Example 164, the title compound (1.63 g, yield 76%) was obtained as a yellow oil by an operation similar to that of Reference Example 52.
EI(pos) 352 $[M+H]^+$

Reference Example 166

2-methyl-7-(piperidin-4-yl)-2,7-diazaspiro[4.5]decan-1-one dihydrochloride

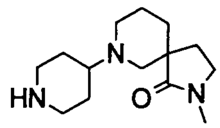

2HCl

Using tert-butyl 4-(2-methyl-1-oxo-2,7-diazaspiro[4.5]dec-7-yl)piperidine-1-carboxylate (1.63 g, 4.64 mmol) obtained in Reference Example 165, the title compound (1.43 g, yield 95%) was obtained as a white solid by an operation similar to that of Reference Example 53.
EI(pos) 252 $[M+H]^+$

Reference Example 167

tert-butyl 4-(3-tert-butyl-2,4-dioxo-1-oxa-3,7-diazaspiro[4.5]dec-7-yl)piperidine-1-carboxylate

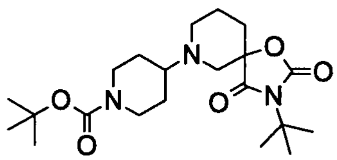

Using 1'-tert-butyl 3-methyl 3-hydroxy-1,4'-bipiperidine-1',3-dicarboxylate (retention time, long: 0.97 g, 2.84 mmol) obtained in Reference Example 157 and tert-butyl isocyanate (0.65 mL, 5.67 mmol), the title compound (1.05 g, yield 90%) was obtained as a yellow oil by an operation similar to that of Reference Example 69.
EI(pos) 410 $[M+H]^+$

Reference Example 168

tert-butyl 1-oxo-2-propyl-2,7-diazaspiro[4.5]decane-7-carboxylate

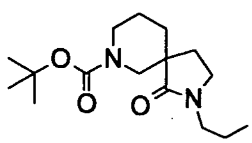

Using tert-butyl 1-oxo-2,7-diazaspiro[4.5]decane-7-carboxylate (1.59 g, 6.28 mmol) obtained in Reference Example 2 and propyl iodide (1.25 mL, 12.5 mmol), the title compound (1.86 g, yield 99%) was obtained as a yellow oil by an operation similar to that of Reference Example 3.
EI(pos) 319 $[M+Na]^+$

Reference Example 169

tert-butyl 4-(l-oxo-2-propyl-2,7-diazaspiro[4.5]dec-7-yl)piperidine-1-carboxylate

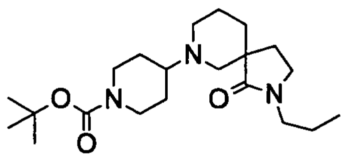

Using tert-butyl 1-oxo-2-propyl-2,7-diazaspiro[4.5]decane-7-carboxylate (1.86 g, 6.27 mmol) obtained in Reference Example 168, the title compound (1.90 g, yield 79%) was obtained as a yellow oil by an operation similar to that of Reference Example 52.
EI(pos) 380 $[M+H]^+$

Reference Example 170

tert-butyl 3-cyano-3-(propionylamino)piperidine-1-carboxylate

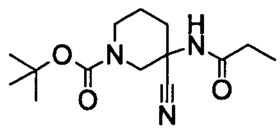

To a mixed solution of tert-butyl 3-oxopiperidine-1-carboxylate (11.5 g, 57.7 mmol) and ammonium chloride (10.7 g, 200 mmol) in isopropyl alcohol-25% aqueous ammonia (50 mL-126 mL) was added potassium cyanide (13.1 g, 200 mmol), and the mixture was stirred for 3 days. The reaction mixture was extracted 3 times with ethyl acetate, and the organic layer was dried over anhydrous magnesium sulfate. The solvent was evaporated under reduced pressure to give tert-butyl 3-amino-3-cyanopiperidine-1-carboxylate (12.95 g) as a crude product. To a solution of the crude product (5.00 g, 22.2 mmol) and triethylamine (4.7 mL, 33.3 mmol) in tetrahydrofuran (100 mL) was added propionyl chloride (2.5 mL, 28.9 mmol), and the mixture was stirred at room temperature for 30 min. A saturated aqueous sodium hydrogencarbonate solution was added to the reaction mixture. The mixture was extracted twice with ethyl acetate, and the organic layer was dried over anhydrous magnesium sulfate. The solvent was evaporated under reduced pressure, and the obtained residue was purified by column chromatography (developing solvent; hexane:ethyl acetate=7:13→ 0: 1) to give the title compound (6.24 g, yield 99%) as an oil. EI(pos) 226 $[M-tBu]^+$

Reference Example 171

tert-butyl 3-cyano-3-(propionylamino)-1,4'-bipiperidine-1'-carboxylate

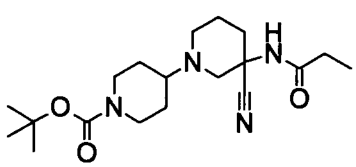

Using tert-butyl 3-cyano-3-(propionylamino)piperidine-1-carboxylate (6.24 g, 22.1 mmol) obtained in Reference Example 170, the title compound (6.45 g, yield 80%) was obtained as an oil by an operation similar to that of Reference Example 52. EI(pos) 365 $[M+H]^+$

Reference Example 172

tert-butyl 4-(2-ethyl-4-oxo-1,3,7-triazaspiro[4.5]dec-1-en-7-yl)piperidine-1-carboxylate

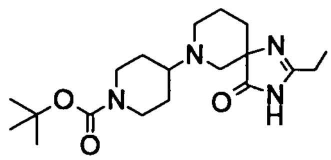

Using tert-butyl 3-cyano-3-(propionylamino)-1,4'-bipiperidine-1'-carboxylate (6.45 g, 17.7 mmol) obtained in Reference Example 171, the title compound (3.25 g, yield 50%) was obtained as an oil by an operation similar to that of Reference Example 80.
EI(pos) 365 $[M+H]^+$

Reference Example 173

tert-butyl 4-(2,3-diethyl-4-oxo-1,3,7-triazaspiro[4.5]dec-1-en-7-yl)piperidine-1-carboxylate

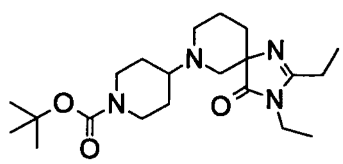

Using tert-butyl 4-(2-ethyl-4-oxo-1,3,7-triazaspiro[4.5]dec-1-en-7-yl)piperidine-1-carboxylate (1.22 g, 3.35 mmol) obtained in Reference Example 172 and iodoethane (0.41 mL, 5.03 mmol), the title compound (0.93 g, yield 70%) was obtained as an oil by an operation similar to that of Reference Example 100.
EI(pos) 393 $[M+H]^+$

Reference Example 174

tert-butyl 3-cyano-3-(isobutyrylamino)piperidine-1-carboxylate

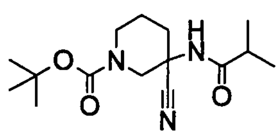

To a mixed solution of tert-butyl 3-oxopiperidine-1-carboxylate (11.5 g, 57.7 mmol) and ammonium chloride (10.7 g, 200 mmol) in isopropyl alcohol-25% aqueous ammonia (50 mL-126 mL) was added potassium cyanide (13.1 g, 200 mmol), and the mixture was stirred for 3 days. The reaction mixture was extracted 3 times with ethyl acetate, and the organic layer was dried over anhydrous magnesium sulfate. The solvent was evaporated under reduced pressure

to give tert-butyl 3-amino-3-cyanopiperidine-1-carboxylate (12.95 g) as a crude product. To a solution of the crude product (7.95 g, 35.3 mmol) and triethylamine (7.4 mL, 52.9 mmol) in tetrahydrofuran (100 mL) was added isobutyryl chloride (4.9 mL, 45.9 mmol), and the mixture was stirred at room temperature for 30 min. A saturated aqueous sodium hydrogencarbonate solution was added to the reaction mixture and extracted twice with ethyl acetate. The organic layer was dried over anhydrous magnesium sulfate. The solvent was evaporated under reduced pressure, and the obtained solid was washed with ethyl acetate to give the title compound (8.73 g, yield 83%) as an oil.
EI(pos) 196 [M-Boc]$^+$

Reference Example 175

tert-butyl 3-cyano-3-(isobutyrylamino)-1,4'-bipiperidine-1'-carboxylate

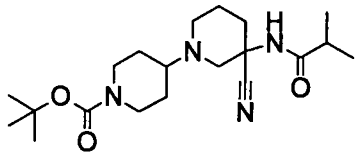

Using tert-butyl 3-cyano-3-(isobutyrylamino)piperidine-1-carboxylate (8.73 g, 29.6 mmol) obtained in Reference Example 174, the title compound (7.15 g, yield 64%) was obtained as an oil by an operation similar to that of Reference Example 52. EI(pos) 379 [M+H]$^+$

Reference Example 176

tert-butyl 4-(2-isopropyl-4-oxo-1,3,7-triazaspiro[4.5]dec-1-en-7-yl)piperidine-1-carboxylate

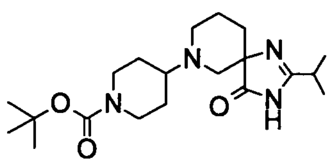

Using tert-butyl 3-cyano-3-(isobutyrylamino)-1,4'-bipiperidine-1'-carboxylate (7.15 g, 18.9 mmol) obtained in Reference Example 175, the title compound (3.95 g, yield 55%) was obtained as an oil by an operation similar to that of Reference Example 80.
EI(pos) 379 [M+H]$^+$

Reference Example 177

tert-butyl 4-(3-ethyl-2-isopropyl-4-oxo-1,3,7-triazaspiro[4.5]dec-l-en-7-yl)piperidine-1-carboxylate

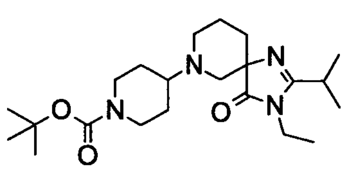

Using tert-butyl 4-(2-isopropyl-4-oxo-1,3,7-triazaspiro[4.5]dec-1-en-7-yl)piperidine-1-carboxylate (1.50 g, 3.96 mmol) obtained in Reference Example 176 and iodoethane (0.48 mL, 5.94 mmol), the title compound (0.48 g, yield 30%) was obtained as an oil by an operation similar to that of Reference Example 100.
EI(pos) 407 $[M+H]^+$

Reference Example 178

tert-butyl 2-isopropyl-1-oxo-2,7-diazaspiro[4.5]decane-7-carboxylate

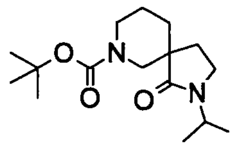

Using tert-butyl 1-oxo-2,7-diazaspiro[4.5]decane-7-carboxylate (1.21 g, 4.78 mmol) obtained in Reference Example 2 and 2-iodopropane (0.96 mL, 9.56 mmol), the title compound (0.91 g, yield 64%) was obtained as a yellow oil by an operation similar to that of Reference Example 3.
EI(pos) 241 $[M-tBu]^+$

Reference Example 179

tert-butyl 4-(2-isopropyl-1-oxo-2,7-diazaspiro[4.5]dec-7-yl)piperidine-1-carboxylate

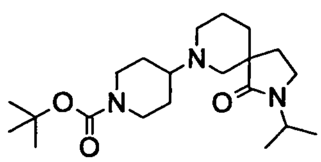

Using tert-butyl 2-isopropyl-1-oxo-2,7-diazaspiro[4.5]decane-7-carboxylate (0.91 g, 3.08 mmol) obtained in Reference Example 178, the title compound (0.69 g, yield 59%) was obtained as a yellow oil by an operation similar to that of Reference Example 52.
EI(pos) 380 $[M+H]^+$

Reference Example 180

1'-tert-butyl 3-ethyl 3-[2-(benzyloxy)-2-oxoethyl]-1,4'-bipiperidine-1',3-dicarboxylate

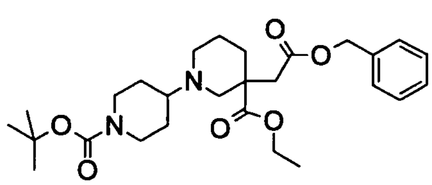

To 3-ethyl 1-tert-butyl 3-[2-(benzyloxy)-2-oxoethyl]piperidine-1,3-dicarboxylate (16.4 g, 40.4 mmol) obtained in Reference Example 31 was added 4N hydrogen chloride-ethyl acetate (200 mL), and the mixture was stirred for 1 hr. The solvent was evaporated under reduced pressure, toluene (50 mL) was added to the obtained residue, and an

operation of concentration under reduced pressure was performed 3 times. The obtained residue was dissolved in tetrahydrofuran (200 mL), and acetic acid (20 mL) and triethylamine (8.44 mL, 60.7 mmol) were added. tert-Butyl 4-oxopiperidine-1-carboxylate (8.86 g, 44.5 mmol) and sodium triacetoxyborohydride (12.9 g, 60.7 mmol) were successively added under ice-cooling, and the mixture was stirred at room temperature for 16 hr. The solvent was evaporated under reduced pressure, and the residue was diluted with ethyl acetate. The mixture was washed with aqueous potassium carbonate solution and saturated brine, and dried over anhydrous sodium sulfate. The solvent was evaporated under reduced pressure, and the obtained residue was purified by silica gel column chromatography (developing solvent; hexane:ethyl acetate=9:1→1:1) to give the title compound (13.8 g, yield 70%) as an oil.
$^1H$ NMR ($CDCl_3$) δ1.17 (3H, t, J = 6.9 Hz), 1.29-1.39 (2H, m), 1.45 (9H, s), 1.52-1.66 (5H, m), 1.82-1.87 (1H, m), 2.32-2.44 (2H, m), 2.53-2.95 (7H, m), 4.03-4.15 (4H, m), 5.05 (2H, m), 7.31 (5H, m).

Reference Example 181

[1'-(tert-butoxycarbonyl)-3-(ethoxycarbonyl)-1,4'-bipiperidin-3-yl]acetic acid

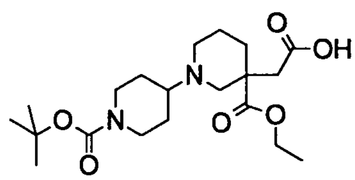

Using 1'-tert-butyl 3-ethyl 3-[2-(benzyloxy)-2-oxoethyl]-1,4'-bipiperidine-1',3-dicarboxylate (13.8 g, 28.2 mmol) obtained in Reference Example 180, the title compound (11.3 g, quantitative) was obtained as an oil by an operation similar to that of Reference Example 32. This was used in the next step without further purification.

Reference Example 182

1'-tert-butyl 3-ethyl 3-[2-(ethylamino)-2-oxoethyl]-1,4'-bipiperidine-1',3-dicarboxylate

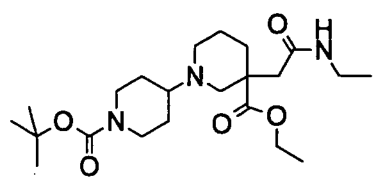

To a solution of [1'-(tert-butoxycarbonyl)-3-(ethoxycarbonyl)-1,4'-bipiperidin-3-yl]acetic acid (2.00 g, 5.02 mmol) obtained in Reference Example 181, 2.0 M ethylamine-THF solution (3.0 mL, 6.02 mmol) and 1-hydroxybenzotriazole (814 mg, 6.02 mmol) in DMF (12 mL) was added 1-[3-(dimethylamino)propyl]-3-ethylcarbodiimide hydrochloride (1.15 g, 6.02 mmol) at room temperature, and the mixture was stirred at room temperature for 2 days. The reaction mixture was dissolved in ethyl acetate, washed with aqueous potassium carbonate solution and saturated brine, and dried over anhydrous sodium sulfate. The solvent was evaporated under reduced pressure, and the obtained residue was purified by silica gel column chromatography (developing solvent; ethyl acetate:methanol=1:0→7:1) to give the title compound (1.71 g, yield 80%) as an oil.
EI(pos) 426 $[M+H]^+$

Reference Example 183

tert-butyl 4-(2-ethyl-1,3-dioxo-2,7-diazaspiro[4.5]dec-7-yl)piperidine-1-carboxylate

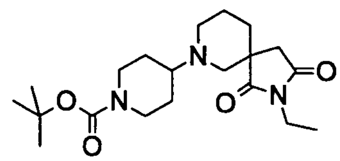

To a solution (10 mL) of 1'-tert-butyl 3-ethyl 3-[2-(ethylamino)-2-oxoethyl]-1,4'-bipiperidine-1',3-dicarboxylate (1.71 g, 4.02 mmol) obtained in Reference Example 182 in DMF was added sodium hydride (60% in oil, 161 mg, 4.02 mmol), and the mixture was stirred at room temperature for 20 min. The reaction mixture was diluted with ethyl acetate, washed 3 times with saturated brine, and dried over anhydrous magnesium sulfate. The solvent was evaporated under reduced pressure, and the obtained residue was purified by silica gel column chromatography (developing solvent; hexane:ethyl acetate=3:1→ 0:1) to give the title compound (1.15 g, yield 75%) as an oil. EI(pos) 380 $[M+H]^+$

Reference Example 184

2-ethyl-7-(piperidin-4-yl)-2,7-diazaspiro[4.5]decane-1,3-dione dihydrochloride

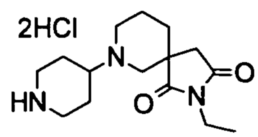

Using tert-butyl 4-(2-ethyl-1,3-dioxo-2,7-diazaspiro[4.5]dec-7-yl)piperidine-1-carboxylate (1.14 g, 3.00 mmol) obtained in Reference Example 183, the title compound (973 mg, yield 92%) was obtained by an operation similar to that of Reference Example 53 and trituration with diisopropyl ether. This was used in the next step without further purification.

Reference Example 185

1'-tert-butyl 3-ethyl 3-[2-(isopropylamino)-2-oxoethyl]-1,4'-bipiperidine-1',3-dicarboxylate

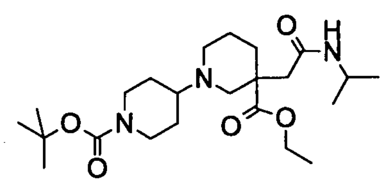

Using [1'-(tert-butoxycarbonyl)-3-(ethoxycarbonyl)-1,4'-bipiperidin-3-yl]acetic acid (3.00 g, 7.53 mmol) obtained in Reference Example 181 and isopropylamine (0.769 mL, 9.03 mmol), the title compound (3.42 g, quantitative) was obtained as an oil by an operation similar to that of Reference Example 182. EI(pos) 440 $[M+H]^+$

Reference Example 186

tert-butyl 4-(2-isopropyl-1,3-dioxo-2,7-diazaspiro[4.5]dec-7-yl)piperidine-1-carboxylate

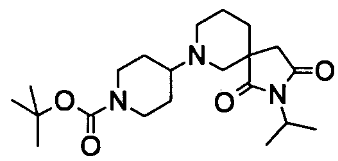

Using 1'-tert-butyl 3-ethyl 3-[2-(isopropylamino)-2-oxoethyl]-1,4'-bipiperidine-1',3-dicarboxylate (3.31 g, 7.53 mmol) obtained in Reference Example 185, the title compound (2.05 g, quantitative) was obtained as an oil by an operation similar to that of Reference Example 183.
EI(pos) 394 $[M+H]^+$

Reference Example 187

2-isopropyl-7-(piperidin-4-yl)-2,7-diazaspiro[4.5]decane-1,3-dione dihydrochloride

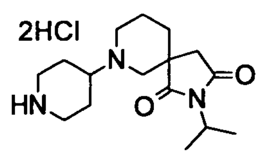

Using tert-butyl 4-(2-isopropyl-1,3-dioxo-2,7-diazaspiro[4.5]dec-7-yl)piperidine-1-carboxylate (2.05 g, 5.21 mmol) obtained in Reference Example 186, the title compound (1.91 g, quantitative) was obtained by an operation similar to that of Reference Example 53 and trituration with diisopropyl ether. This was used in the next step without further purification.

Reference Example 188

tert-butyl 4-(2-methoxy-1,3-dioxo-2,7-diazaspiro[4.5]dec-7-yl)piperidine-1-carboxylate

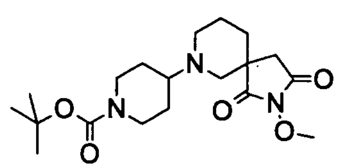

Using [1'-(tert-butoxycarbonyl)-3-(ethoxycarbonyl)-1,4'-bipiperidin-3-yl]acetic acid (2.00 g, 5.02 mmol) obtained in Reference Example 181 and (aminooxy)methane hydrochloride (503 mg, 6.02 mmol), an operation similar to that of Reference Example 182 was performed up to the ring closure reaction to give the title compound (1.71 g, yield 90%) as an oil.
EI(pos) 382 $[M+H]^+$

Reference Example 189

2-methoxy-7-(piperidin-4-yl)-2,7-diazaspiro[4.5]decane-1,3-dione dihydrochloride

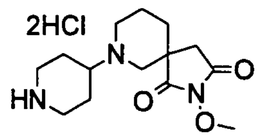

Using tert-butyl 4-(2-methoxy-1,3-dioxo-2,7-diazaspiro[4.5]dec-7-yl)piperidine-1-carboxylate (1.71 g, 4.48 mmol) obtained in Reference Example 188, the title compound (1.34 g, yield 84%) was obtained by an operation similar to that of Reference Example 53 and trituration with diisopropyl ether. This was used in the next step without further purification.

Reference Example 190

1'-tert-butyl 3-ethyl 3-[2-(cyclopropylamino)-2-oxoethyl]-1,4'-bipiperidine-1',3-dicarboxylate

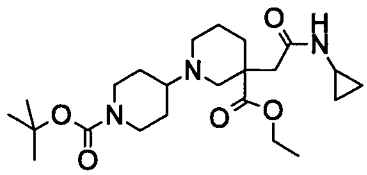

Using [1'-(tert-butoxycarbonyl)-3-(ethoxycarbonyl)-1,4'-bipiperidin-3-yl]acetic acid (1.00 g, 2.51 mmol) obtained in Reference Example 181 and cyclopropylamine (0.209 mL, 3.01 mmol), the title compound (1.10 g, quantitative) was obtained as an oil by an operation similar to that of Reference Example 182. This was used in the next step without further purification.

Reference Example 191

tert-butyl 4-(2-cyclopropyl-1,3-dioxo-2,7-diazaspiro[4.5]dec-7-yl)piperidine-1-carboxylate

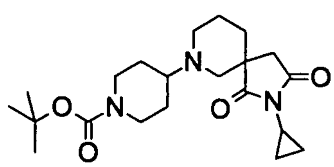

Using 1'-tert-butyl 3-ethyl 3-[2-(cyclopropylamino)-2-oxoethyl]-1,4'-bipiperidine-1',3-dicarboxylate (1.10 g, 2.51 mmol) obtained in Reference Example 190, the title compound (432 mg, yield 44%) was obtained as an oil by an operation similar to that of Reference Example 183.
EI(pos) 392 $[M+H]^+$

Reference Example 192

2-cyclopropyl-7-(piperidin-4-yl)-2,7-diazaspiro[4.5]decane-1,3-dione dihydrochloride

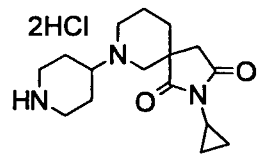

Using tert-butyl 4-(2-cyclopropyl-1,3-dioxo-2,7-diazaspiro[4.5]dec-7-yl)piperidine-1-carboxylate (432 mg, 1.10 mmol) obtained in Reference Example 191, the title compound (381 mg, yield 95%) was obtained by an operation similar to that of Reference Example 53 and trituration with diisopropyl ether. This was used in the next step without further purification.

Reference Example 193

1'-tert-butyl 3-ethyl 3-[2-(cyclobutylamino)-2-oxoethyl]-1,4'-bipiperidine-1',3-dicarboxylate

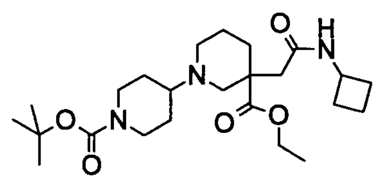

Using [1'-(tert-butoxycarbonyl)-3-(ethoxycarbonyl)-1,4'-bipiperidin-3-yl]acetic acid (1.00 g, 2.51 mmol) obtained in Reference Example 181 and cyclobutylamine (0.257 mL, 3.01 mmol), the title compound (1.13 g, quantitative) was obtained as an oil by an operation similar to that of Reference Example 182. This was used in the next step without further purification.

Reference Example 194

tert-butyl 4-(2-cyclobutyl-1,3-dioxo-2,7-diazaspiro[4.5]dec-7-yl)piperidine-1-carboxylate

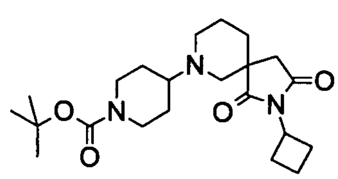

Using 1'-tert-butyl 3-ethyl 3-[2-(cyclobutylamino)-2-oxoethyl]-1,4'-bipiperidine-1',3-dicarboxylate (1.13 g, 2.51 mmol) obtained in Reference Example 193, the title compound (454 mg, yield 45%) was obtained as an oil by an operation similar to that of Reference Example 183.
EI(pos) 406 $[M+H]^+$

Reference Example 195

2-cyclobutyl-7-(piperidin-4-yl)-2,7-diazaspiro[4.5]decane-1,3-dione dihydrochloride

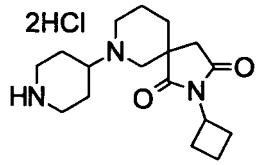

Using tert-butyl 4-(2-cyclobutyl-1,3-dioxo-2,7-diazaspiro[4.5]dec-7-yl)piperidine-1-carboxylate (454 mg, 1.12 mmol) obtained in Reference Example 194, the title compound (395 mg, yield 93%) was obtained by an operation similar to that of Reference Example 53 and trituration with diisopropyl ether. This was used in the next step without further purification.

Reference Example 196

1'-tert-butyl 3-ethyl 3-[2-(methylamino)-2-oxoethyl]-1,4'-bipiperidine-1',3-dicarboxylate

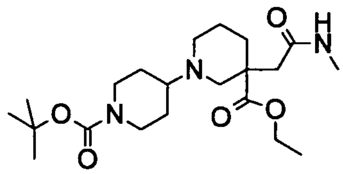

Using [1'-(tert-butoxycarbonyl)-3-(ethoxycarbonyl)-1,4'-bipiperidin-3-yl]acetic acid (1.27 g, 3.19 mmol) obtained in Reference Example 181 and 2.0 M methylamine-THF solution (1.91 mL, 3.82 mmol), the title compound (991 g, yield 76%) was obtained as an oil by an operation similar to that of Reference Example 182. This was used in the next step without further purification.

Reference Example 197

tert-butyl 4-(2-methyl-1,3-dioxo-2,7-diazaspiro[4.5]dec-7-yl)piperidine-1-carboxylate

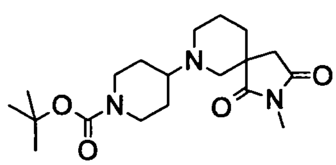

Using 1'-tert-butyl 3-ethyl 3-[2-(methylamino)-2-oxoethyl]-1,4'-bipiperidine-1',3-dicarboxylate (991 mg, 2.41 mmol) obtained in Reference Example 196, the title compound (318 mg, yield 36%) was obtained as a solid by an operation similar to that of Reference Example 183.
EI(pos) 366 $[M+H]^+$

Reference Example 198

2-methyl-7-(piperidin-4-yl)-2,7-diazaspiro[4.5]decane-1,3-dione dihydrochloride

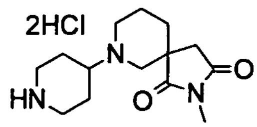

Using tert-butyl 4-(2-methyl-1,3-dioxo-2,7-diazaspiro[4.5]dec-7-yl)piperidine-1-carboxylate (318 mg, 0.870 mmol) obtained in Reference Example 197, the title compound (286 mg, yield 97%) was obtained by an operation similar to that of Reference Example 53 and trituration with diisopropyl ether. This was used in the next step without further purification.

Reference Example 199.

ethyl 3-(2-amino-2-oxoethyl)-1'-[(2-{[(isopropylamino)carbonyl]amino}-1-benzothien-3-yl)carbonyl]-1,4'-bipiperidine-3-carboxylate

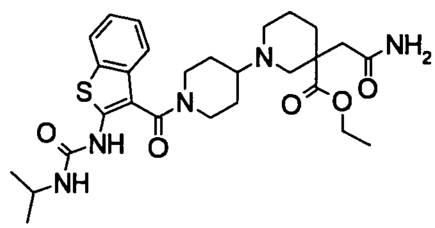

Using ethyl 1'-[(2-amino-1-benzothien-3-yl)carbonyl]-3-(2-amino-2-oxoethyl)-1,4'-bipiperidine-3-carboxylate (230 mg, 0.487 mmol) obtained in Reference Example 148 and isopropyl isocyanate (0.143 mL, 1.46 mmol), the title compound (167 mg, yield 62%) was obtained by an operation similar to that of Reference Example 166 and trituration with diisopropyl ether. EI(pos) 558 $[M+H]^+$

Reference Example 200

1-benzyl 3-ethyl 3-[(methoxycarbonyl)thio]piperidine-1,3-dicarboxylate

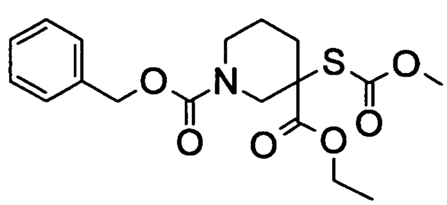

To a solution of 1-benzyl 3-ethyl piperidine-1,3-dicarboxylate (6.00 g, 20.6 mmol) in THF (100 mL) was added a 1.0 M lithium bis(trimethylsilyl)amide-THF solution (22.7 mL, 22.7 mmol) at -78°C, and the mixture was stirred at the same temperature for 30 min. To the solution was added dropwise (chlorothio)(methoxy)oxomethane (2.79 mL, 30.9 mmol) in THF (10 mL) at -78°C over 10 min. After warmed to room temperature, the mixture was stirred for 30 min. The reaction mixture was dissolved in ethyl acetate, washed with water, 1N hydrochloric acid and saturated brine, and dried over anhydrous magnesium sulfate. The solvent was evaporated under reduced pressure, and the obtained residue was purified by silica gel column chromatography (developing solvent; hexane:ethyl acetate=1:0→ 3:1) to give the title compound (3.09 g, yield 39%) as an oil. EI(pos) 382 $[M+H]^+$

Reference Example 201

1-benzyl 3-ethyl 3-mercaptopiperidine-1,3-dicarboxylate

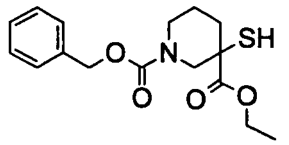

To a solution of 1-benzyl 3-ethyl 3-[(methoxycarbonyl)thio]piperidine-1,3-dicarboxylate (2.99 g, 7.84 mmol) obtained in Reference Example 200 in ethanol (40 mL) was added sodium ethoxide (533 mg, 11.8 mmol), and the mixture was stirred at room temperature for 1 hr. The solvent was evaporated under reduced pressure. To the obtained residue was added ethyl acetate, and the mixture was washed with 1N hydrochloric acid and saturated brine, and dried over anhydrous magnesium sulfate. The solvent was evaporated under reduced pressure to give the title compound (2.54 g, quantitative) as an oil.
EI(pos) 324 $[M+H]^+$

Reference Example 202

benzyl 3-ethyl-2,4-dioxo-1-thia-3,7-diazaspiro[4.5]decane-7-carboxylate

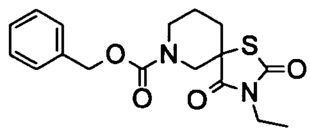

To a solution of 1-benzyl 3-ethyl 3-mercaptopiperidine-1,3-dicarboxylate (2.54 g, 7.84 mmol) obtained in Reference Example 201 and ethyl isocyanate (1.24 mL, 15.7 mmol) in THF (30 mL) was added 60% sodium hydride (157 mg, 3.92 mmol), and the mixture was stirred at room temperature for 1 hr. The reaction mixture was diluted with ethyl acetate, washed with saturated brine, and dried over anhydrous sodium sulfate. The solvent was evaporated under reduced pressure, and the obtained residue was purified by column chromatography (developing solvent; ethyl acetate: hexane=1:9→1:3) to give the title compound (2.07 g, yield 76%) as an oil.
$^1$H NMR ($CDCl_3$) δ1.19 (3H, t, J = 7.2 Hz), 1.60 (1H, m), 1.87-1.91 (1H, m), 2.01-2.07 (1H, m), 2.27-2.37 (1H, m), 2.90 (1H, m), 3.45 (1H, m), 3.64 (2H, q, J = 7.2 Hz), 4.25 (2H, m), 5.14 (2H, s), 7.34 (5H, m).
EI(pos) 349 $[M+H]^+$

Reference Example 203

3-ethyl-1-thia-3,7-diazaspiro[4.5]decane-2,4-dione

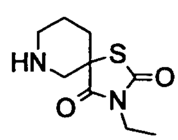

A suspension of benzyl 3-ethyl-2,4-dioxo-1-thia-3,7-diazaspiro[4.5]decane-7-carboxylate (2.07 g, 5.94 mmol) obtained in Reference Example 202 and 10% palladium carbon (50% water-containing product, 2.0 g) in THF (30 mL)

was stirred for 1 day under a hydrogen atmosphere. After filtration, the solvent was evaporated under reduced pressure, and the obtained residue was purified by column chromatography (developing solvent; ethyl acetate:hexane=1:3→1:0) to give the title compound (180 mg, yield 14%) as an oil. This was used in the next step without further purification.

Reference Example 204

tert-butyl 4-(3-ethyl-2,4-dioxo-1-thia-3,7-diazaspiro[4.5]dec-7-yl)piperidine-1-carboxylate

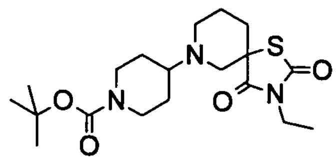

Using 3-ethyl-l-thia-3,7-diazaspiro[4.5]decane-2,4-dione (179 mg, 0.835 mmol) obtained in Reference Example 203 and tert-butyl 4-oxopiperidine-1-carboxylate (183 mg, 0.919 mmol), the title compound (279 mg, yield 84%) was obtained as an oil by an operation similar to that of Reference Example 48.
EI(pos) 398 $[M+H]^+$

Reference Example 205

3-ethyl-7-(piperidin-4-yl)-1-thia-3,7-diazaspiro[4.5]decane-2,4-dione dihydrochloride

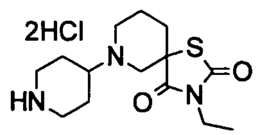

Using tert-butyl 4-(3-ethyl-2,4-dioxo-l-thia-3,7-diazaspiro[4.5]dec-7-yl)piperidine-1-carboxylate (278 mg, 0.699 mmol) obtained in Reference Example 204, the title compound (244 mg, yield 94%) was obtained by an operation similar to that of Reference Example 53 and trituration with diisopropyl ether. This was used in the next step without further purification.

Reference Example 206

2-chloro-6-(4-hydroxypiperidin-1-yl)isonicotinic acid

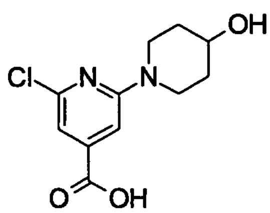

To 2,6-dichloroisonicotinic acid (2.24 g, 11.7 mmol), piperidin-4-ol (3.54 g, 35.0 mmol) and cesium carbonate (7.60 g, 23.3 mmol) was added DMF (45 mL), and the mixture was stirred with heating at 120°C for 3 days. After completion of the reaction, the mixture was diluted with ethyl acetate, washed with 1N hydrochloric acid and saturated

brine, and dried over anhydrous sodium sulfate. The solvent was evaporated under reduced pressure, and the residue was triturated with ethyl acetate to give the title compound (1.70 g, yield 57%). This was used in the next step without further purification.

Reference Example 207

ethyl 3-(2-amino-2-oxoethyl)-1'-[(2-{[(propylamino)carbonyl]amino}-1-benzothien-3-yl)carbonyl]-1,4'-bipiperidine-3-carboxylate

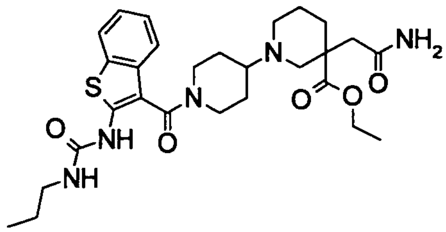

Using ethyl 1'-[(2-amino-1-benzothien-3-yl)carbonyl]-3-(2-amino-2-oxoethyl)-1,4'-bipiperidine-3-carboxylate (361 mg, 0.764 mmol) obtained in Reference Example 148 and propyl isocyanate (0.215 mL, 2.29 mmol), the title compound (355 mg, yield 83%) was obtained by an operation similar to that of Reference Example 166 and trituration with diisopropyl ether.
EI(pos) 558 $[M+H]^+$

Reference Example 208

tert-butyl 4-[3-(3-methoxypropyl)-2-methyl-4-oxo-1,3,7-triazaspiro[4.5]dec-1-en-7-yl]piperidine-1-carboxylate

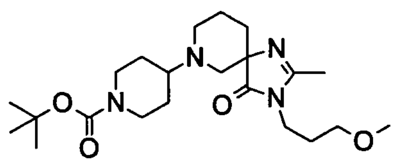

Using tert-butyl 4-(2-methyl-4-oxo-1,3,7-triazaspiro[4.5]dec-1-en-7-yl)piperidine-1-carboxylate (0.96 g, 2.74 mmol) obtained in Reference Example 99 and 1-bromo-3-methoxypropane (1.22 mL, 7.66 mmol), the title compound (1.03 g, yield 89%) was obtained as an oil by an operation similar to that of Reference Example 100.
EI(pos) 423 $[M+H]^+$

Reference Example 209

tert-butyl 4-(3-methyl-2,4-dioxo-1-oxa-3,7-diazaspiro[4.5]dec-7-yl)piperidine-1-carboxylate

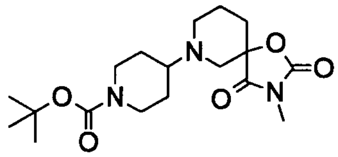

Using 1'-tert-butyl 3-methyl 3-hydroxy-1,4'-bipiperidine-1',3-dicarboxylate (1.22 g, 3.56 mmol) obtained in Reference Example 143 and methyl isocyanate (0.42 mL, 7.13 mmol), the title compound (0.87 g, yield 66%) was obtained as a yellow oil by an operation similar to that of Reference Example 69.
EI(pos) 368 $[M+H]^+$

Reference Example 210

3-methyl-7-(piperidin-4-yl)-1-oxa-3,7-diazaspiro[4.5]decane-2,4-dione dihydrochloride

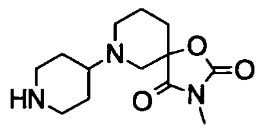

2HCl

Using tert-butyl 4-(3-methyl-2,4-dioxo-1-oxa-3,7-diazaspiro[4.5]dec-7-yl)piperidine-1-carboxylate (0.87 g, 2.38 mmol) obtained in Reference Example 209, the title compound (0.72 g, yield 89%) was obtained as a white solid by an operation similar to that of Reference Example 53.
EI(pos) 268 $[M+H]^+$

Reference Example 211

tert-butyl 4-(3-ethyl-1-methyl-2,4-dioxo-1,3,7-triazaspiro[4.5]dec-7-yl)piperidine-1-carboxylate

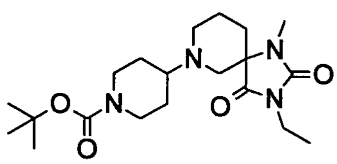

To a solution of tert-butyl 4-(3-ethyl-2,4-dioxo-1,3,7-triazaspiro[4.5]dec-7-yl)piperidine-1-carboxylate (1.18 g, 3.10 mmol) obtained in Reference Example 155 in DMF (10 mL) was added 60% sodium hydride (0.19 g, 4.65 mmol), and the mixture was stirred at room temperature for 15 min. Methyl iodide (0.39 mL, 6.20 mmol) was added to the reaction mixture, and the mixture was stirred at 70°C for 30 min. The reaction mixture was diluted with ethyl acetate, washed 3 times with brine, and dried over anhydrous magnesium sulfate. The solvent was evaporated under reduced pressure, and the obtained residue was purified by silica gel column chromatography (developing solvent; hexane:ethyl acetate=2:3→0:1) to give the title compound (1.22 g, yield 99%) as an oil.
EI(pos) 395 $[M+H]^+$

Reference Example 212

3-ethyl-1-methyl-7-(piperidin-4-yl)-1,3,7-triazaspiro[4.5]decane-2,4-dione dihydrochloride

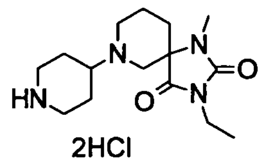

Using tert-butyl 4-(3-ethyl-1-methyl-2,4-dioxo-1,3,7-triazaspiro[4.5]dec-7-yl)piperidine-1-carboxylate (1.22 g, 3.09 mmol) obtained in Reference Example 211, the title compound (1.13 g, yield 99%) was obtained as a white solid by an operation similar to that of Reference Example 53.
EI(pos) 295 $[M+H]^+$

Reference Example 213

3-tert-butyl-7-(piperidin-4-yl)-1-oxa-3,7-diazaspiro[4.5]decane-2,4-dione dihydrochloride

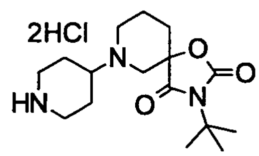

Using tert-butyl 4-(3-tert-butyl-2,4-dioxo-1-oxa-3,7-diazaspiro[4.5]dec-7-yl)piperidine-1-carboxylate (525 mg, 1.28 mmol) obtained in Reference Example 167, the title compound (389 mg, yield 79%) was obtained by an operation similar to that of Reference Example 53 and trituration with diisopropyl ether. This was used in the next step without further purification.

Reference Example 214

tert-butyl 4-(2,3-dimethyl-4-oxo-1,3,7-triazaspiro[4.5]dec-1-en-7-yl)piperidine-1-carboxylate

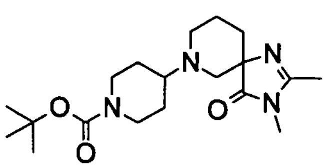

Using tert-butyl 4-(2-methyl-4-oxo-1,3,7-triazaspiro[4.5]dec-1-en-7-yl)piperidine-1-carboxylate (1.07 g, 3.05 mmol) obtained in Reference Example 99 and iodomethane (0.21 mL, 3.36 mmol), the title compound (0.49 g, yield 44%) was obtained as an oil by an operation similar to that of Reference Example 100.
EI(pos) 365 $[M+H]^+$

Example 1

7-[1-(9-anthrylcarbonyl)piperidin-4-yl]-2-ethyl-2,7-diazaspiro[4.5]decan-1-one

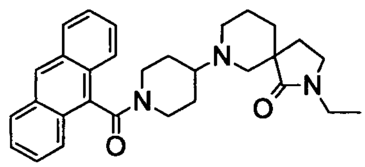

A solution of tert-butyl 4-(2-ethyl-1-oxo-2,7-diazaspiro[4.5]dec-7-yl)piperidine-1-carboxylate (0.650 g, 1.78 mmol) obtained in Reference Example 4 in 4M hydrogen chloride-ethyl acetate (20 mL) was stirred at room temperature for 30 min. The solvent was evaporated under reduced pressure, and a solution of the residue, anthracene-9-carboxylic acid (0.590 g, 2.67 mmol), triethylamine (0.500 mL, 3.56 mmol), 1-ethyl-3-(3'-dimethylaminopropyl)carbodiimide hydrochloride (0.510 g, 2.67 mmol) and 1-hydroxybenzotriazole (0.360 g, 2.67 mmol) in DMF (10 mL) was stirred at room temperature for 1 hr. The reaction mixture was diluted with ethyl acetate, washed 3 times with saturated brine, and dried over anhydrous magnesium sulfate. The solvent was evaporated under reduced pressure, and the obtained residue was purified by silica gel column chromatography (developing solvent; methanol:ethyl acetate=0:1 →1:4) to give the title compound (0.120 g, yield 14%) as an oil.
EI(pos) 470 $[M+H]^+$

Example 2

8-[1-(9-anthrylcarbonyl)piperidin-4-yl]-2-ethyl-2,8-diazaspiro[5.5]undecan-1-one

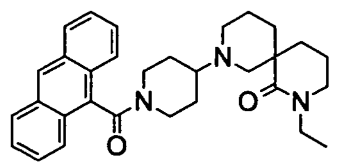

A solution of tert-butyl 8-ethyl-7-oxo-2,8-diazaspiro[5.5]undecane-2-carboxylate (0.250 g, 0.843 mmol) obtained in Reference Example 7 in 4M hydrogen chloride-ethyl acetate (20 mL) was stirred at room temperature for 30 min. The solvent was evaporated under reduced pressure, and a solution of the residue, triethylamine (0.120 mL, 0.843 mmol), 1-(9-anthrylcarbonyl)piperidine-4-one (0.280 g, 0.928 mmol) obtained in Reference Example 8, sodium triacetoxyborohydride (0.540 g, 2.53 mmol) in dichloromethane (10 mL) was stirred at room temperature for 19 hr. The reaction mixture was diluted with ethyl acetate, washed with saturated aqueous sodium hydrogencarbonate solution, and dried over anhydrous magnesium sulfate. The solvent was evaporated under reduced pressure, and the obtained residue was purified by silica gel column chromatography (developing solvent; methanol:ethyl acetate=0:1 →1:4) to give the title compound (0.160 g, yield 39%) as an oil.
EI(pos) 484 $[M+H]^+$

Example 3

7-[1-(9-anthrylcarbonyl)piperidin-4-yl]-2-ethyl-4-methyl-2,7-diazaspiro[4.5]decan-1-one

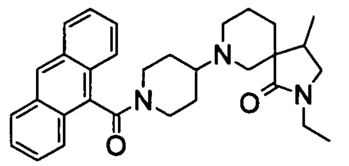

Using tert-butyl 2-ethyl-4-methyl-1-oxo-2,7-diazaspiro[4.5]decane-7-carboxylate (0.480 g, 1.62 mmol) obtained in Reference Example 11, the title compound (0.240 g, yield 30%) was obtained as an oil by an operation similar to that of Example 2.
EI(pos) 484 $[M+H]^+$

Example 4

7-[1-(9-anthrylcarbonyl)piperidin-4-yl]-2-ethyl-3-methyl-2,7-diazaspiro[4.5]decan-1-one

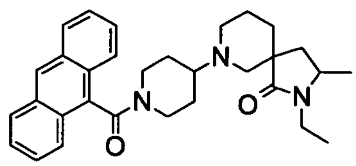

Using tert-butyl 2-ethyl-3-methyl-l-oxo-2,7-diazaspiro[4.5]decane-7-carboxylate (0.380 g, 1.28 mmol) obtained in Reference Example 14, an operation similar to that of Example 2 was performed to give the title compounds (120 mg, less polar, yield 20% and 86.0 mg, highly-polar, yield 14%) each as an oil.
EI(pos) 484 $[M+H]^+$

Example 5

7-[1-(9-anthrylcarbonyl)piperidin-4-yl]-3-methyl-2-oxa-7-azaspiro[4.5]decan-1-one

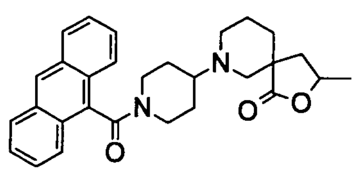

Using tert-butyl 3-methyl-1-oxo-2-oxa-7-azaspiro[4.5]decane-7-carboxylate (0.470 g, 1.75 mmol) obtained in Reference Example 15, the title compound (0.150 g, yield 19%) was obtained as an oil by an operation similar to that of Example 2.
EI(pos) 457 $[M+H]^+$

Example 6

7-[1-(9-anthrylcarbonyl)piperidin-4-yl]-2-(cyclopropylmethyl)-3-methyl-2,7-diazaspiro[4.5]decan-1-one

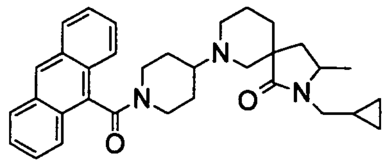

Using tert-butyl 2-(cyclopropylmethyl)-3-methyl-1-oxo-2,7-diazaspiro[4.5]decane-7-carboxylate (0.550 g, 1.71 mmol) obtained in Reference Example 16, an operation similar to that of Example 2 was performed to give the title compounds (0.150 g, less polar, yield 17% and 0.100 g, highly-polar, yield 12%) each as an oil.
EI(pos) 510 $[M+H]^+$

Example 7

{7-[1-(9-anthrylcarbonyl)piperidin-4-yl]-3-methyl-1-oxo-2,7-diazaspiro[4.5]dec-2-yl}acetonitrile

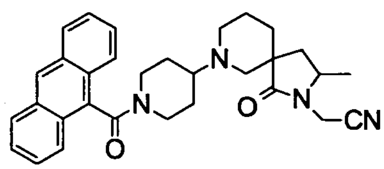

Using tert-butyl 2-(cyanomethyl)-3-methyl-1-oxo-2,7-diazaspiro[4.5]decane-7-carboxylate (0.450 g, 1.47 mmol) obtained in Reference Example 17, the title compound (0.150 g, yield 21%) was obtained as an oil by an operation similar to that of Example 2.
EI(pos) 495 $[M+H]^+$

Example 8

methyl {7-[1-(9-anthrylcarbonyl)piperidin-4-yl]-3-methyl-1-oxo-2,7-diazaspiro[4.5]dec-2-yl}acetate

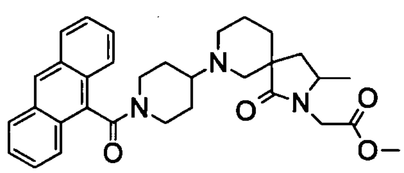

Using tert-butyl 2-(2-methoxy-2-oxoethyl)-3-methyl-1-oxo-2,7-diazaspiro[4.5]decane-7-carboxylate (0.610 g, 1.79 mmol) obtained in Reference Example 18, the title compound (0.200 g, yield 21%) was obtained as an oil by an operation similar to that of Example 2.
EI(pos) 528 $[M+H]^+$

Example 9

{7-[1-(9-anthrylcarbonyl)piperidin-4-yl]-3-methyl-1-oxo-2,7-diazaspiro[4.5]dec-2-yl}acetic acid

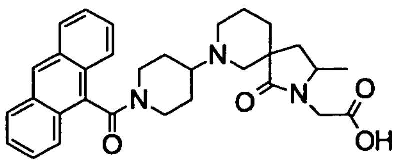

To a solution of methyl {7-[1-(9-anthrylcarbonyl)piperidin-4-yl]-3-methyl-1-oxo-2,7-diazaspiro[4.5]dec-2-yl}acetate (0.190 g, 0.366 mmol) obtained in Example 8 in THF-methanol (5 mL-1 mL) was added 1N aqueous sodium hydroxide solution (1.10 mL, 1.10 mmol), and the mixture was stirred at room temperature for 30 min. The reaction mixture was neutralized with 1N hydrochloric acid (1.10 mL, 1.10 mmol), extracted 3 times with ethyl acetate, and dried over anhydrous magnesium sulfate. The solvent was evaporated under reduced pressure, and the obtained white solid was washed with diisopropyl ether to give the title compound (25.0 mg, yield 13%) as a white solid.
EI(pos) 514 $[M+H]^+$

Example 10

7-[1-(9-anthrylcarbonyl)piperidin-4-yl]-3,3-dimethyl-2-oxa-7-azaspiro[4.5]decan-1-one

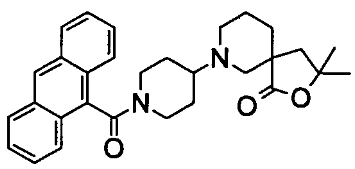

Using tert-butyl 3,3-dimethyl-1-oxo-2-oxa-7-azaspiro[4.5]decane-7-carboxylate (0.600 g, 2.12 mmol) obtained in Reference Example 19 or 140, the title compound (0.400 g, yield 40%) was obtained as an oil by an operation similar to that of Example 2.
EI(pos) 471 $[M+H]^+$

Example 11

7-[1-(9-anthrylcarbonyl)piperidin-4-yl]-2-ethyl=3-methyl-2,7-diazaspiro[4.4]nonan-1-one

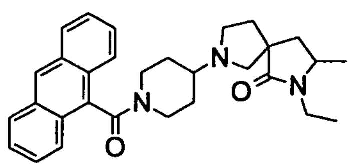

Using tert-butyl 7-ethyl-8-methyl-6-oxo-2,7-diazaspiro[4.4]nonane-2-carboxylate (0.570 g, 2.02 mmol) obtained in Reference Example 22, the title compound (0.700 g, yield 73%) was obtained as an oil by an operation similar to that of Example 2.
EI(pos) 470 $[M+H]^+$

Example 12

9-[1-(9-anthrylcarbonyl)piperidin-4-yl]-2-ethyl-3-methyl-6-oxa-2,9-diazaspiro[4.5]decan-1-one

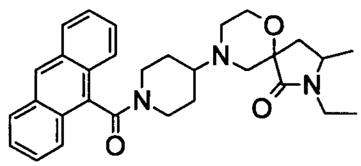

Using tert-butyl 2-ethyl-3-methyl-1-oxo-6-oxa-2,9-diazaspiro[4.5]decane-9-carboxylate (0.510 g, 1.71 mmol) obtained in Reference Example 25, the title compound (0.250 g, yield 30%) was obtained as an oil by an operation similar to that of Example 2.
EI(pos) 486 $[M+H]^+$

Example 13

7-[1-(9-anthrylcarbonyl)piperidin-4-yl]-3-methyl-2,7-diazaspiro[4.5]decan-1-one

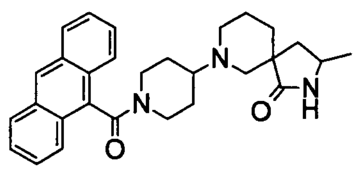

Using tert-butyl 3-methyl-1-oxo-2,7-diazaspiro[4.5]decane-7-carboxylate (1.37 g, 5.11 mmol) obtained in Reference Example 26, an operation similar to that of Example 2 was performed to give the title compounds (0.410 g, less polar, yield 17% and 0.180 g, highly-polar, yield 7.7%) each as an oil.
EI(pos) 456 $[M+H]^+$

Example 14

7-[1-(9-anthrylcarbonyl)piperidin-4-yl]-3-methyl-2-(thiazol-2-ylmethyl)-2,7-diazaspiro[4.5]decan-1-one

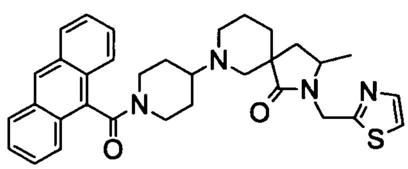

Using tert-butyl 3-methyl-1-oxo-2-(thiazol-2-ylmethyl)-2,7-diazaspiro[4.5]decane-7-carboxylate (0.250 g, 0.684 mmol) obtained in Reference Example 27, the title compound (15.0 mg, yield 3.9%) was obtained as an oil by an operation similar to that of Example 2.
EI(pos) 553 $[M+H]^+$

Example 15

7-[1-(9-anthrylcarbonyl)piperidin-4-yl]-3-methyl-2-(pyridin-4-ylmethyl)-2,7-diazaspiro[4.5]decan-1-one

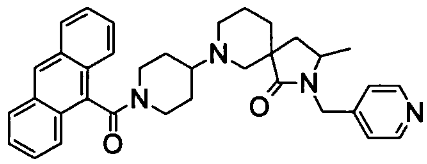

Using tert-butyl 3-methyl-1-oxo-2-(pyridin-4-ylmethyl)-2,7-diazaspiro[4.5]decane-7-carboxylate (0.460 g, 1.28 mmol) obtained in Reference Example 28, an operation similar to that of Example 2 was performed to give the title compounds (87.0 mg, less polar, yield 12% and 59.0 mg, highly-polar, yield 8.4%) each as an oil.
EI(pos) 547 $[M+H]^+$

Example 16

methyl 6-{7-[1-(9-anthrylcarbonyl)piperidin-4-yl]-3-methyl-1-oxo-2,7-diazaspiro[4.5]dec-2-yl}hexanoate

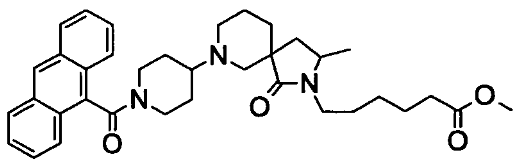

Using tert-butyl 2-(6-methoxy-6-oxohexyl)-3-methyl-1-oxo-2,7-diazaspiro[4.5]decane-7-carboxylate (0.520 g, 1.32 mmol) obtained in Reference Example 29, the title compound (0.240 g, yield 31%) was obtained as an oil by an operation similar to that of Example 2.
EI(pos) 584 $[M+H]^+$

Example 17

6-{7-[1-(9-anthrylcarbonyl)piperidin-4-yl]-3-methyl-1-oxo-2,7-diazaspiro[4.5]dec-2-yl}hexanoic acid

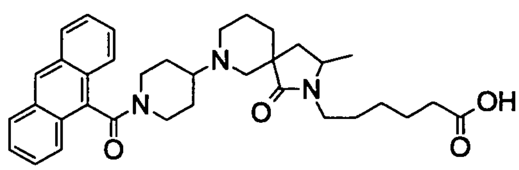

Using methyl 6-{7-[1-(9-anthrylcarbonyl)piperidin-4-yl]-3-methyl-1-oxo-2,7-diazaspiro[4.5]dec-2-yl}hexanoate (0.230 g, 0.394 mmol) obtained in Reference Example 16, the title compound (88.0 mg, yield 39%) was obtained as a white solid by an operation similar to that of Example 9.
EI(pos) 570 $[M+H]^+$

Example 18

7-[1-(9-anthrylcarbonyl)piperidin-4-yl]-2-(3-methoxypropyl)-3-methyl-2,7-diazaspiro[4.5]decan-1-one

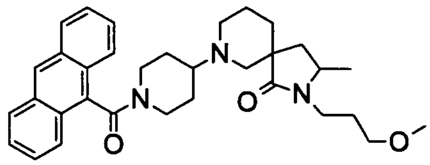

To a solution of 7-[1-(9-anthrylcarbonyl)piperidin-4-yl]-3-methyl-2,7-diazaspiro[4.5]decan-1-one in less polar compound (0.190 g, 0.417 mmol) obtained in Example 13 in DMF (5 mL) was added sodium hydride (60% in oil, 25.0 mg, 0.626 mmol), and the mixture was stirred at room temperature for 30 min. 1-Bromo-3-methoxypropane (0.130 g, 0.834 mmol) was added to the solution, and the mixture was stirred at 70°C for 1 hr. The reaction mixture was diluted with ethyl acetate, washed successively with saturated aqueous ammonium chloride solution and saturated brine, and dried over anhydrous magnesium sulfate. The solvent was evaporated under reduced pressure, and the obtained residue was purified by silica gel column chromatography (developing solvent; methanol:ethyl acetate=0:1 →1:4) to give the title compound (87.0 mg, yield 39%) as an oil.
EI(pos) 528 $[M+H]^+$

Example 19

7-[1-(9-anthrylcarbonyl)piperidin-4-yl]-3-methyl-2-[2-(pyrrolidin-1-yl)ethyl]-2,7-diazaspiro[4.5]decan-1-one

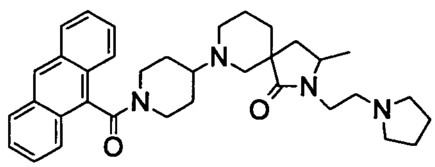

Using tert-butyl 3-methyl-1-oxo-2-[2-(pyrrolidin-1-yl)ethyl]-2,7-diazaspiro[4.5]decane-7-carboxylate (0.860 g, 2.35 mmol) obtained in Reference Example 30, the title compound (0.160 g, yield 12%) was obtained as an oil by an operation similar to that of Example 2.
EI(pos) 553 $[M+H]^+$

Example 20

7-[1-(9-anthrylcarbonyl)piperidin-4-yl]-2-ethyl-2,7-diazaspiro[4.5]decane-1,3-dione

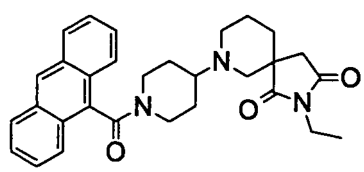

To a solution of ethyl 1'-(9-anthrylcarbonyl)-3-[2-(ethylamino)-2-oxoethyl]-1,4'-bipiperidine-3-carboxylate (0.580 g, 1.09 mmol) obtained in Reference Example 34 in DMF (10 mL) was added sodium hydride (60% in oil, 44.0 mg, 1.09 mmol) under ice-cooling, and the mixture was stirred at room temperature for 20 min. The reaction mixture was diluted with ethyl acetate, washed 3 times with saturated brine, and dried over anhydrous magnesium sulfate. The solvent was evaporated under reduced pressure, and the obtained residue was purified by silica gel column chromatography (developing solvent; hexane:ethyl acetate=1:3→0:1) to give the title compound (0.520 g, quantitative) as an oil.
EI(pos) 484 $[M+H]^+$

Example 21

9-[1-(9-anthrylcarbonyl)piperidin-4-yl]-2-ethyl-3-methyl-6-(trifluoroacetyl)-2,6,9-triazaspiro[4.5]decan-1-one

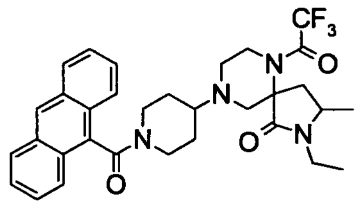

Using tert-butyl 2-ethyl-3-methyl-1-oxo-6-(trifluoroacetyl)-2,6,9-triazaspiro[4.5]decane-9-carboxylate (0.780 g, 1.98 mmol) obtained in Reference Example 38, the title compound (0.550 g, yield 47%) was obtained as an oil by an operation similar to that of Example 2.
EI(pos) 581 $[M+H]^+$

Example 22

9-[1-(9-anthrylcarbonyl)piperidin-4-yl]-2-ethyl-3-methyl-2,6,9-triazaspiro[4.5]decan-1-one

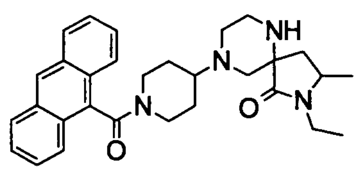

To 9-[1-(9-Anthrylcarbonyl)piperidin-4-yl]-2-ethyl-3-methyl-6-(trifluoroacetyl)-2,6,9-triazaspiro[4.5]decan-1-one (0.550 g, 0.947 mmol) obtained in Example 21 and potassium carbonate (0.260 g, 1.90 mmol) were added methanol (5 mL) and water (1 mL), and the mixture was stirred at 60˚C for 2 hr. Ethyl acetate was added to the reaction mixture. The mixture was washed with aqueous potassium carbonate solution and saturated brine, and dried over anhydrous sodium sulfate. The solvent was evaporated under reduced pressure, and the obtained residue was purified by basic silica gel column chromatography (developing solvent; hexane:ethyl acetate=1:3→ 0:1) to give the title compound (0.450 g, yield 98%) as an oil. EI(pos) 485 $[M+H]^+$

Example 23

9-[1-(9-anthrylcarbonyl)piperidin-4-yl]-2-ethyl-3,6-dimethyl-2,6,9-triazaspiro[4.5]decan-1-one

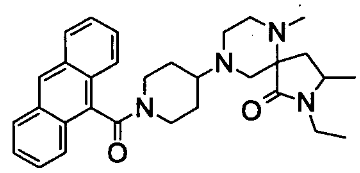

To a suspension of 9-[1-(9-anthrylcarbonyl)piperidin-4-yl]-2-ethyl-3-methyl-2,6,9-triazaspiro[4.5]decan-1-one (0.450 g, 0.929 mmol) obtained in Example 22, 37% aqueous formaldehyde solution (1.00 mL) and anhydrous sodium

sulfate(3.00 g) in dichloromethane (10 mL) was added sodium triacetoxyborohydride (0.930 g, 4.64 mmol), and the mixture was stirred at room temperature for 15 hr. Ethyl acetate was added to the reaction mixture, and the mixture was washed with saturated aqueous sodium hydrogencarbonate solution, and dried over anhydrous magnesium sulfate. The solvent was evaporated under reduced pressure, and the obtained residue was purified by basic silica gel column chromatography (developing solvent; methanol:ethyl acetate=0:1→1:9) to give the title compound (0.300 g, yield 64%) as an oil.
EI(pos) 499 $[M+H]^+$

Example 24

7-[1-(9-anthrylcarbonyl)piperidin-4-yl]-2,7-diazaspiro[4.5]decane-1,3-dione

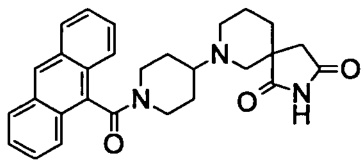

Using ethyl 3-(2-amino-2-oxoethyl)-1'-(9-anthrylcarbonyl)-1,4'-bipiperidine-3-carboxylate (0.400 g, 0.797 mmol) obtained in Reference Example 40, the title compound (88.0 mg, yield 24%) was obtained as an oil by an operation similar to that of Example 20.
EI(pos) 456 $[M+H]^+$

Example 25

7-[1-(9-anthrylcarbonyl)piperidin-4-yl]-3-ethyl-3-methyl-2-oxa-7-azaspiro[4.5]decan-1-one

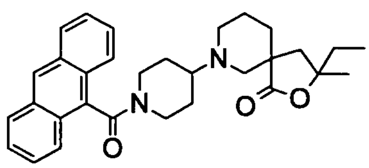

Using tert-butyl 3-ethyl-3-methyl-1-oxo-2-oxa-7-azaspiro[4.5]decane-7-carboxylate (0.260 g, 0.874 mmol) obtained in Reference Example 41, the title compound (0.250 g, yield 59%) was obtained as an oil by an operation similar to that of Example 2.
EI(pos) 485 $[M+H]^+$

Example 26

7-[1-(9-anthrylcarbonyl)piperidin-4-yl]-3-methyl-2-(pyridin-2-ylmethyl)-2,7-diazaspiro[4.5]decan-1-one

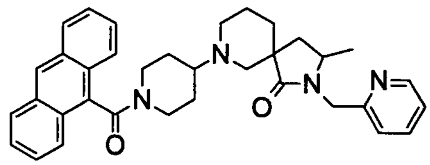

Using tert-butyl 3-methyl-1-oxo-2-(pyridin-2-ylmethyl)-2,7-diazaspiro[4.5]decane-7-carboxylate (0.700 g, 1.95 mmol) obtained in Reference Example 42, the title compound (49.0 mg, yield 4.6%) was obtained as an oil by an operation similar to that of Example 2.
EI(pos) 547 $[M+H]^+$

Example 27

7-[1-(9-anthrylcarbonyl)piperidin-4-yl]-3-methyl-2-(pyridin-3-ylmethyl)-2,7-diazaspiro[4.5]decan-1-one

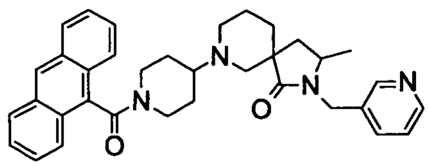

Using tert-butyl 3-methyl-1-oxo-2-(pyridin-3-ylmethyl)-2,7-diazaspiro[4.5]decane-7-carboxylate (0.510 g, 1.42 mmol) obtained in Reference Example 43, an operation similar to that of Example 2 was performed to give the title compounds (85.0 mg, less polar, yield 11% and 61.0 mg, highly-polar, yield 7.8%) each as an oil.
EI(pos) 547 $[M+H]^+$

Example 28

1'-[1-(9-anthrylcarbonyl)piperidin-4-yl]spiro[inden-2,3'-piperidin]-1(3H)-one

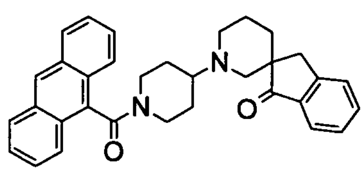

A solution of spiro[inden-2,3'-piperidine]-1(3H)-one (121 mg, 0.601 mmol), 1-(9-anthrylcarbonyl)piperidin-4-one (182 mg, 0.601 mmol) obtained in Reference Example 8 and sodium triacetoxyborohydride (191 mg, 0.902 mmol) in tetrahydrofuran (3 mL) was stirred at room temperature for 16 hr. The reaction mixture was diluted with ethyl acetate, washed with saturated aqueous sodium hydrogencarbonate solution, and dried over anhydrous magnesium sulfate. The solvent was evaporated under reduced pressure, and the obtained residue was purified by basic silica gel column chromatography (developing solvent; hexane:ethyl acetate=3:1→1:1) to give the title compound (90.2 mg, yield 31%) as an oil.
EI(pos) 489 $[M+H]^+$

Example 29

1'-[1-(9-anthrylcarbonyl)piperidin-4-yl]-3,4-dihydro-1H-spiro[naphthalene-2,3'-piperidin]-1-one hydrochloride

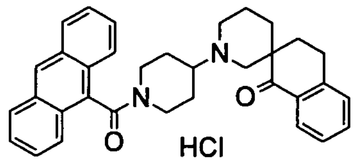

A solution of 1'-(piperidin-4-yl)-3,4-dihydro-1H-spiro[naphthalene-2,3'-piperidin]-1-one (386 mg, 1.29 mmol) obtained in Reference Example 49, anthracene-9-carboxylic acid (316 mg, 1.42 mmol), 1-hydroxybenzotriazole (192 mg, 1.42 mmol) and 1-ethyl-3-(3'-dimethylaminopropyl)carbodiimide hydrochloride (273 mg, 1.42 mmol) in DMF (6 mL) was stirred at room temperature for 16 hr. The reaction mixture was diluted with ethyl acetate, washed with aqueous potassium carbonate solution and saturated brine, and dried over anhydrous sodium sulfate. The solvent was evaporated under reduced pressure, and to the obtained residue (621 mg) was added 4N hydrogen chloride-ethyl acetate (0.31 mL) to give the title compound (527 mg, yield 76%) as a solid.
EI(pos) 503 $[M+H]^+$

Example 30

7-{1-[(2-amino-1-benzothien-3-yl)carbonyl]piperidin-4-yl}-3,3-dimethyl-2-oxa-7-azaspiro[4.5]decan-1-one hydrochloride

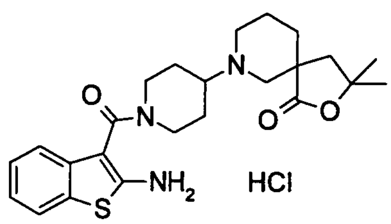

To a suspension of 3,3-dimethyl-7-(piperidin-4-yl)-2-oxa-7-azaspiro[4.5]decan-1-one ditrifluoroacetate (1.4 g, 2.8 mmol) obtained in Reference Example 139, 2-[(tert-butoxycarbonyl)amino]-1-benzothiophene-3-carboxylic acid (0.64 g, 2.0 mmol) obtained in Reference Example 50, 1-ethyl-3-(3-dimethylaminopropyl)carbodiimide hydrochloride (0.38 g, 2.0 mmol) and 1-hydroxybenzotriazole (0.27 g, 2.0 mmol) in N,N-dimethylformamide (5 mL) was added triethylamine (0.40 g, 4.0 mmol), and the mixture was stirred at room temperature for 24 hr. Water was added to the reaction mixture, and the mixture was extracted with ethyl acetate. The organic layer was washed with saturated brine, and dried over anhydrous sodium sulfate. The solvent was evaporated under reduced pressure, and the obtained residue was purified by silica gel column chromatography (developing solvent; hexane:ethyl acetate=7:3→ 3:7). A 4N hydrogen chloride-ethyl acetate solution (2 mL, 8 mmol) was added to the obtained residue. After standing the mixture for 10 min, the residue was concentrated under reduced pressure to give the title compound (0.55 g, yield 62%) as pale-brown crystals.
EI(pos) 442 $[M+H]^+$

Example 31

7-{1-[(2-amino-1-benzothien-3-yl)carbonyl]piperidin-4-yl}-3,3-dimethyl-2-oxa-7-azaspiro[4.5]decan-1-one

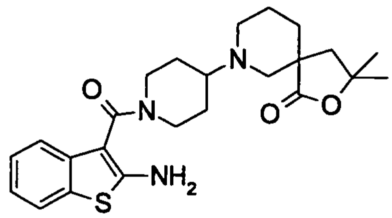

To a suspension of 2-amino-1-benzothiophene-3-carboxylic acid (0.87 g, 4.5 mmol) obtained in Reference Example 51, 3,3-dimethyl-7-(piperidin-4-yl)-2-oxa-7-azaspiro[4.5]decan-1-one dihydrochloride (1.33 g, 4.0 mmol) obtained in Reference Example 53, 1-ethyl-3-(3-dimethylaminopropyl)carbodiimide hydrochloride (1.15 g, 6.0 mmol) and 1-hydroxybenzotriazole (0.54 g, 4.0 mmol) in N,N-dimethylformamide (15 mL) was added triethylamine (3.33 g, 33.0 mmol), and the mixture was stirred at room temperature for 24 hr. Water was added to the reaction mixture, and the mixture was extracted with ethyl acetate. The organic layer was washed with saturated brine, and dried over anhydrous sodium sulfate. The solvent was evaporated under reduced pressure, and the obtained residue was purified by silica gel column chromatography (developing solvent; hexane:ethyl acetate=3:1→0:1) to give the title compound (1.05 g, yield 60%) as colorless amorphous crystals.
EI(pos) 442 $[M+H]^+$

Example 32

N'-(3-{[4-(3,3-dimethyl-1-oxo-2-oxa-7-azaspiro[4.5]dec-7-yl)piperidin-1-yl]carbonyl}-1-benzothien-2-yl)-N,N-diethylurea

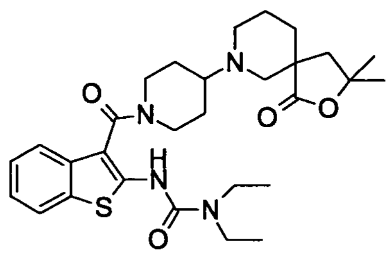

To a suspension of 2-{[(diethylamino)carbonyl]amino}-1-benzothiophene-3-carboxylic acid (0.12 g, 0.5 mmol) obtained in Reference Example 55, 3,3-dimethyl-7-(piperidin-4-yl)-2-oxa-7-azaspiro[4.5]decan-1-one dihydrochloride (0.11 g, 0.33 mmol) obtained in Reference Example 53, 1-ethyl-3-(3-dimethylaminopropyl)carbodiimide hydrochloride (0.19 g, 1.0 mmol) and 1-hydroxybenzotriazole (0.07 g, 0.05mmol) in N,N-dimethylformamide (10 mL) was added triethylamine (0.10 g, 1.0 mmol), and the mixture was stirred at room temperature for 24 hr. Water was added to the reaction mixture, and the mixture was extracted with ethyl acetate. The organic layer was washed with saturated brine, and dried over anhydrous sodium sulfate. The solvent was evaporated under reduced pressure, and the obtained residue was dissolved in N,N-dimethylformamide (1 mL). An eluate containing the object compound purified by preparative HPLC was neutralized with saturated aqueous sodium hydrogencarbonate solution, and the precipitate was collected by filtration to give the title compound (0.07 g, yield 37%) as white crystals.
EI(pos) 541 $[M+H]^+$

Example 33

7-{1-[(2-chloroquinolin-4-yl)carbonyl]piperidin-4-yl}-3,3-dimethyl-2-oxa-7-azaspiro[4.5]decan-1-one

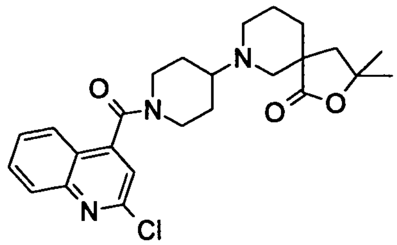

To a solution of 2-bromoquinoline-4-carboxylic acid (1.10 g, 4.2 mmol) in tetrahydrofuran (20 mL) were added oxalyl chloride (1.26 g, 10.0 mmol) and N,N-dimethylformamide (1 drop), and the mixture was stirred at 60˚C for 2 hr. The reaction solution was concentrated under reduced pressure, and the obtained residue was dissolved in tetrahydrofuran (5 mL). To a solution of 3,3-dimethyl-7-(piperidin-4-yl)-2-oxa-7-azaspiro[4.5]decan-1-one dihydrochloride (1.0 g, 3.0 mmol) obtained in Reference Example 53 and triethylamine (1.0 g, 10.0 mmol) in tetrahydrofuran (20 mL) was added at 0˚C with stirring. The mixture was allowed to warm to room temperature and stirred for 2 hr. Water was added to the reaction mixture, and the mixture was extracted with ethyl acetate. The organic layer was washed with saturated brine, and dried over anhydrous sodium sulfate. The solvent was evaporated under reduced pressure, and the obtained residue was purified by silica gel column chromatography (developing solvent; hexane:ethyl acetate=1:1→1:9) to give the title compound (0.72 g, yield 53%) as white crystals.
EI(pos) 456 $[M+H]^+$

Examples 34 - 37

7-{1-[(2-Amino-1-benzothien-3-yl)carbonyl]piperidin-4-yl}-3,3-dimethyl-2-oxa-7-azaspiro[4.5]decan-1-one hydrochloride (0.035 mg, 0.06 mmol) obtained in Example 30 and triethylamine (0.10 mg, 1.0 mmol) were dissolved in a mixture of tetrahydrofuran (1 mL) and dichloromethane (1 mL), each of various acid chlorides or isocyanic acid esters (1.0 mmol) was added at room temperature and the mixture was stirred for 24 hr. Water (2 mL) and ethyl acetate (2 mL) were added, and the mixture was stirred vigorously. Using phase Sep (manufactured by Wako Pure Chemical Industries, Ltd.), the aqueous layer was removed and the organic layer was concentrated. The residue was dissolved in N,N-dimethylformamide (0.5 mL), and purified by preparative HPLC to give the object compound at purity 80% or above (LC/MS analysis).

Examples 38 - 55

To a solution of 7-{1-[(2-amino-1-benzothien-3-yl)carbonyl]piperidin-4-yl}-3,3-dimethyl-2-oxa-7-azaspiro[4.5]decan-1-one (0.044 mg, 0.1 mmol) obtained in Example 31 in pyridine(1 mL) was added each of various acid chlorides, isocyanic acid esters or sulfonyl chlorides (0.5 mmol) at room temperature, and the mixture was stirred at 55˚C for 3 days. Water (2 mL) and ethyl acetate (3 mL) were added, and the mixture was stirred vigorously. Using phase Sep (manufactured by Wako Pure Chemical Industries, Ltd.), the aqueous layer was removed and the organic layer was concentrated. The residue was dissolved in N,N-dimethylformamide (0.5 mL), and purified by preparative HPLC to give the object compound at purity 80% or above (LC/MS analysis).

Examples 56 - 64

To a solution of 7-{1-[(2-chloroquinolin-4-yl)carbonyl]piperidin-4-yl}-3,3-dimethyl-2-oxa-7-azaspiro[4.5]decan-1-one (0.027 g, 0.06 mmol) obtained in Example 33 in N,N-dimethylacetamide (2 mL) were added each of various hetero rings (0.18 mmol) and potassium carbonate (0.14 g, 0.1 mmol), and the mixture was tightly sealed, exposed to microwave (Personal Chemistry, manufactured by Emrys Optimizer) irradiation when the mixture was stirred at 180˚C for 5 min. Water (2 mL) and ethyl acetate (3 mL) were added, and the mixture was stirred vigorously. Using phase Sep (manufactured by Wako Pure Chemical Industries, Ltd.), the aqueous layer was removed and the organic layer was concentrated. The residue was dissolved in N,N-dimethylformamide (0.5 mL), and purified by preparative HPLC to give the object compound at purity 80% or above (LC/MS analysis).

The purification in Examples 34 - 64 by preparative HPLC was performed under the following conditions.
instrument: High Through-put Purification System, Gilson Company, Inc.
column: YMC CombiPrep ODS-A S-5 μm, 50×20 mm
solvent: SOLUTION A; 0.1% trifluoroacetic acid aqueous solution, SOLUTION B; 0.1% trifluoroacetic acid acetonitrile solution

gradient cycle: 0 min (SOLUTION A/SOLUTION B =95/5), 1.00 min (SOLUTION A/SOLUTION B =95/5), 5.20 min (SOLUTION A/SOLUTION B =5/95), 6.40 min (SOLUTION A/SOLUTION B =5/95), 6.50 min (SOLUTION A/SOLUTION B =95/5), 6.60 min (SOLUTION A/SOLUTION B =95/5)
flow rate :20 mL/min, detection method: UV 220 nm

The structural formulas and mass spectrum data of the compounds obtained in Examples 34 - 64 are shown in Table 1 and Table 2.

Table 1

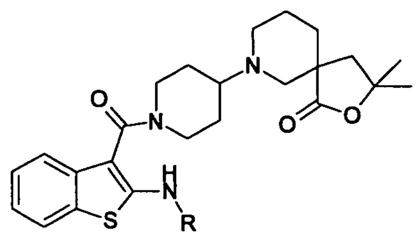

| Example No. | R | MS (m/Z) |
|---|---|---|
| 34 | Ac | 484 |
| 35 | BnCO | 560 |
| 36 | EtNHCO | 513 |
| 37 | nPrNHCO | 527 |
| 38 | 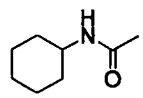 | 567 |
| 39 | 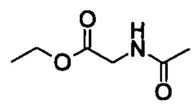 | 571 |
| 40 | 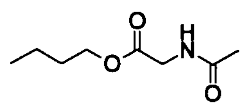 | 599 |
| 41 | 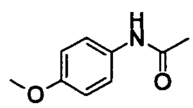 | 591 |
| 42 | 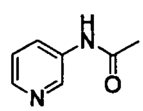 | 562 |
| 43 | 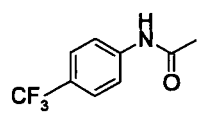 | 629 |
| 44 | 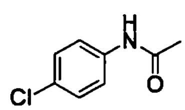 | 595 |

(continued)

| Example No. | R | MS (m/Z) |
|---|---|---|
| 45 | 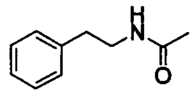 | 589 |
| 46 | EtCO | 498 |
| 47 | $EtO_2C(CH_2)_2CO$ | 570 |
| 48 | $MeOCH_2CO$ | 514 |
| 49 | 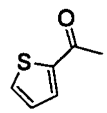 | 552 |
| 50 | $MeO(CH_2)_2OCO$ | 544 |
| 51 | $CH_2CH(CH_2)_2CO$ | 524 |
| 52 | 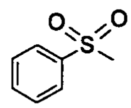 | 582 |
| 53 | 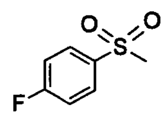 | 600 |
| 54 | 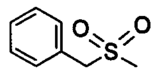 | 596 |
| 55 | 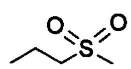 | 548 |

Table 2

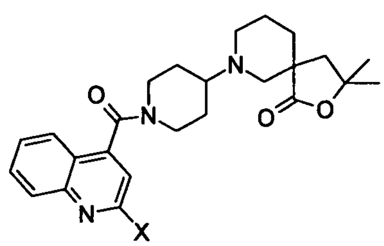

| Example No. | X | MS (m/Z) |
|---|---|---|
| 56 | 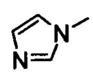 | 488 |
| 57 | 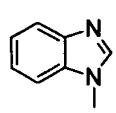 | 538 |

(continued)

| Example No. | X | MS (m/Z) |
|---|---|---|
| 58 | 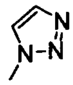 | 489 |
| 59 | 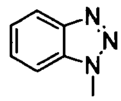 | 539 |
| 60 | 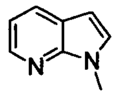 | 538 |
| 61 | 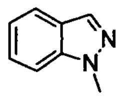 | 538 |
| 62 | 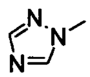 | 489 |
| 63 | 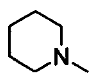 | 505 |
| 64 | 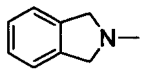 | 539 |

Example 65

7-[1-(5-bromo-2-methoxybenzoyl)piperidin-4-yl]-3,3-dimethyl-2-oxa-7-azaspiro[4.5]decan-1-one

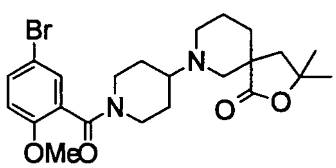

To a suspension of 5-bromo-2-methoxybenzoic acid (0.62 g, 2.7 mmol), 3,3-dimethyl-7-(piperidin-4-yl)-2-oxa-7-azaspiro[4.5]decan-1-one dihydrochloride (0.82 g, 2.4 mmol) obtained in Reference Example 53, 1-ethyl-3-(3-dimethylaminopropyl)carbodiimide hydrochloride (0.70 g, 3.6 mmol) and 1-hydroxybenzotriazole monohydrate (0.57 g, 3.6 mmol) in N,N-dimethylformamide (24 mL) was added triethylamine (0.73 g, 7.26 mmol), and the mixture was stirred at room temperature for 3.5 hr. Water was added to the reaction mixture, and the mixture was extracted with ethyl acetate. The organic layer was washed with water and saturated brine, and dried over anhydrous sodium sulfate. The solvent was evaporated under reduced pressure, and the obtained residue was purified by silica gel column chromatography (developing solvent; hexane:ethyl acetate=1:1→0:1) to give the title compound (1.1 g, yield 95%) as white crystals.
EI(pos) 480 $[M+H]^+$

Example 66

7-[1-(3-bromo-5-(pyridin-3-yl)benzoyl)piperidin-4-yl]-3,3-dimethyl-2-oxa-7-azaspiro[4.5]decan-1-one

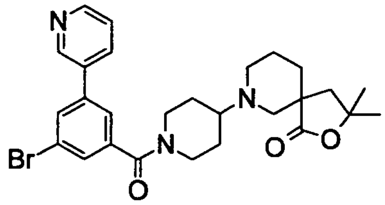

To a suspension of 3-bromo-5-(pyridin-3-yl)benzoic acid trifluoroacetate (0.58 g, 1.5 mmol) obtained in Reference Example 57, 3,3-dimethyl-7-(piperidin-4-yl)-2-oxa-7-azaspiro[4.5]decan-1-one dihydrochloride (0.50 g, 1.5 mmol) obtained in Reference Example 53, 1-ethyl-3-(3-dimethylaminopropyl)carbodiimide hydrochloride (0.43 g, 2.2 mmol) and 1-hydroxybenzotriazole monohydrate (0.34 g, 2.2 mmol) in N,N-dimethylformamide (10 mL) was added triethylamine (1.12 g, 11.1 mmol), and the mixture was stirred at room temperature overnight. Water was added to the reaction mixture, and the mixture was extracted with ethyl acetate. The organic layer was washed with water and saturated brine, and dried over anhydrous sodium sulfate. The solvent was evaporated under reduced pressure, and the obtained residue was purified by silica gel column chromatography (developing solvent; hexane:ethyl acetate=1:1 to ethyl acetate:methanol=5:1) to give the title compound (0.48 g, yield 61%) as colorless amorphous crystals.
EI(pos) 527 $[M+H]^+$

Examples 67 - 100

To a solution of 3,3-dimethyl-7-(piperidin-4-yl)-2-oxa-7-azaspiro[4.5]decan-1-one ditrifluoroacetate (0.054 g, 0.10 mmol) obtained in Reference Example 139, 1-ethyl-3-(3-dimethylaminopropyl)carbodiimide hydrochloride (0.029 g, 0.15 mmol), 1-hydroxybenzotriazole monohydrate (0.023 g, 0.15 mmol) and triethylamine (0.030 g, 0.3 mmol) in N, N-dimethylformamide (2 mL) was added each of various carboxylic acids (0.15 mmol), and the mixture was stirred at room temperature overnight. Water (2 mL) and dichloromethane (2 mL) were added, and the mixture was stirred vigorously. Using phase Sep (Whatman), the aqueous layer was removed and the organic layer was concentrated. The residue was dissolved in dimethyl sulfoxide (0.5 mL), and purified by preparative HPLC to give the object compound at purity 80% or above (LC/MS analysis) (purification by HPLC in Examples 69 and 71 was performed by Method B to be described below, and that in the other Examples by Method A to be described below).

Example 101

3,3-dimethyl-7-(1-{[2-(1H-pyrrol-1-yl)-4,5,6,7-tetrahydro-1-benzothien-3-yl]carbonyl}piperidin-4-yl)-2-oxa-7-azaspiro[4.5]decan-1-one

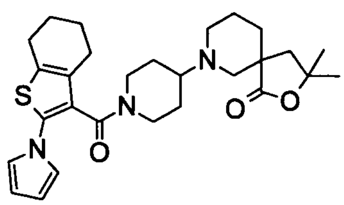

To a suspension of 3,3-dimethyl-7-(piperidin-4-yl)-2-oxa-7-azaspiro[4.5]decan-1-one dihydrochloride (0.034 g, 0.10 mmol) obtained in Reference Example 53, 2-(1H-pyrrol-1-yl)-4,5,6,7-tetrahydro-1-benzothiophene-3-carboxylic acid(0.030 g, 0.12 mmol), 1-ethyl-3-(3-dimethylaminopropyl)carbodiimide hydrochloride (0.029 g, 0.15 mmol) and 1-hydroxybenzotriazole monohydrate (0.023 g, 0.15 mmol) in N,N-dimethylformamide (2 mL) was added triethylamine (0.030 g, 0.30 mmol), and the mixture was stirred at room temperature for 3 hr. Water was added to the reaction mixture, and the mixture was extracted with ethyl acetate. The organic layer was washed with water and saturated brine, and dried over anhydrous sodium sulfate. The solvent was evaporated under reduced pressure, and the obtained residue was purified by silica gel column chromatography (developing solvent; hexane:ethyl acetate=5:1→ 2:1) to give the title compound (42.7 mg, yield 86%) as a colorless oil.
EI(pos) 496 $[M+H]^+$

Example 102

7-{1-[5-methoxy-2-(1,3,5-trimethyl-1H-pyrazol-4-yl)benzoyl]piperidin-4-yl}-3,3-dimethyl-2-oxa-7-azaspiro[4.5]decan-1-one

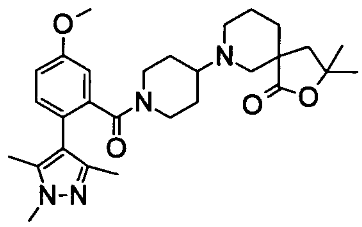

To a suspension of 3,3-dimethyl-7-(piperidin-4-yl)-2-oxa-7-azaspiro[4.5]decan-1-one dihydrochloride (0.034 g, 0.10 mmol) obtained in Reference Example 53, 5-methoxy-2-(1,3,5-trimethyl-1H-pyrazol-4-yl)benzoic acid (0.031 g, 0.12 mmol), 1-ethyl-3-(3-dimethylaminopropyl)carbodiimide hydrochloride (0.029 g, 0.15 mmol) and 1-hydroxybenzotriazole monohydrate (0.023 g, 0.15 mmol) in N,N-dimethylformamide (2 mL) was added triethylamine (0.030 g, 0.30 mmol), and the mixture was stirred at room temperature for 6 hr. Water was added to the reaction mixture, and the mixture was extracted with ethyl acetate. The organic layer was washed with water and saturated brine, and dried over anhydrous sodium sulfate. The solvent was evaporated under reduced pressure, and the residue was dissolved in N, N-dimethyl sulfoxide (0.5 mL), and purified by preparative HPLC to give the title compound (0.052 g, yield 83%) as a brown oil.
EI(pos) 509 $[M+H]^+$

Example 103

7-{1-[5-(3-furyl)-2-methoxybenzoyl]piperidin-4-yl}-3,3-dimethyl-2-oxa-7-azaspiro[4.5]decan-1-one

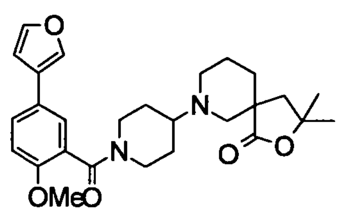

7-[1-(5-Bromo-2-methoxybenzoyl)piperidin-4-yl]-3,3-dimethyl-2-oxa-7-azaspiro[4.5]decan-1-one (0.038 g, 0.08 mmol) obtained in Example 65 was dissolved in a mixture of 1,2-dimethoxyethane (1.5 mL) and water (0.5 mL). 3-Furylboronic acid (0.010 g, 0.09 mmol), sodium carbonate (0.025 g, 0.24 mmol) and tetrakis(triphenylphosphine)palladium (0.005 g, 0.004 mmol) were added thereto, and the mixture was tightly sealed, exposed to microwave (Personal Chemistry, manufactured by Emrys Optimizer) irradiation when the mixture was stirred at 150°C for 5 min. Water (2 mL) and ethyl acetate (2 mL) were added, and the mixture was stirred vigorously. Using phase Sep (manufactured by Wako Pure Chemical Industries, Ltd.), the aqueous layer was removed and the organic layer was concentrated. The residue was dissolved in dimethyl sulfoxide (0.5 mL), and purified by preparative HPLC to give the title compound (0.010 g, yield 28%) as colorless amorphous crystals. EI(pos) 467 $[M+H]^+$

Examples 104 - 125

7-[1-(5-Bromo-2-methoxybenzoyl)piperidin-4-yl]-3,3-dimethyl-2-oxa-7-azaspiro[4.5]decan-1-one (0.029 g, 0.06 mmol) obtained in Example 65 was dissolved in a mixture of 1,2-dimethoxyethane (1.5 mL) and water (0.5 mL). Each of various boronic acids (0.06 mmol), sodium carbonate (0.02 g, 0.18 mmol) and a catalytic amount of tetrakis(triphenylphosphine)palladium were added thereto, and the mixture was tightly sealed, exposed to microwave (Personal Chemistry, manufactured by Emrys Optimizer) irradiation when the mixture was stirred at 150°C for 5 min. Water (2 mL) and ethyl acetate (2 mL) were added, and the mixture was stirred vigorously. Using phase Sep (manufactured by Wako Pure Chemical Industries, Ltd.), the aqueous layer was removed and the organic layer was concentrated. The residue was dissolved in dimethyl sulfoxide (0.5 mL), and purified by preparative HPLC to give the object compound at purity 80% or above (LC/MS analysis) (purification by HPLC in Examples 105, 106 and 124 was performed by Method B to be described below, and that in the other Examples by Method A to be described below).

Example 126

3,3-dimethyl-7-{1-[(5-(pyridin-3-yl)biphenyl-3-yl)carbonyl]piperidin-4-yl}-2-oxa-7-azaspiro[4.5]decan-1-one

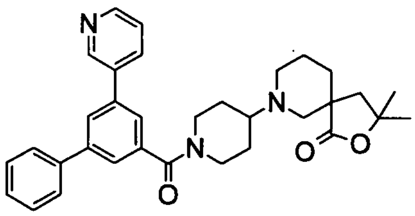

7-[1-(3-Bromo-5-(pyridin-3-yl)benzoyl)piperidin-4-yl]-3,3-dimethyl-2-oxa-7-azaspiro[4.5]decan-1-one (0.100 g, 0.19 mmol) obtained in Example 66 was dissolved in a mixture of 1,2-dimethoxyethane (1.5 mL) and water (0.5 mL). Phenylboronic acid (0.026 g, 0.21 mmol), sodium carbonate (0.06 g, 0.57 mmol) and tetrakis(triphenylphosphine)palladium (0.01 g, 0.01 mmol) were added thereto, and the mixture was tightly sealed, exposed to microwave (Emrys Optimizer manufactured by Personal Chemistry) irradiation, and stirred at 150°C for 5 min. Water (2 mL) and ethyl acetate (3 mL) were added, and the mixture was stirred vigorously and extracted with ethyl acetate. The organic layer was washed with water and saturated brine, and dried over anhydrous magnesium sulfate. The solvent was evaporated under reduced pressure, and the obtained residue was purified by silica gel column chromatography (developing solvent; hexane:ethyl acetate=5:1→0:1) to give the title compound (0.041 g, yield 41%) as white crystals.
EI(pos) 524 $[M+H]^+$

Example 127 - 139

7-[1-(3-Bromo-5-(pyridin-3-yl)benzoyl)piperidin-4-yl]-3,3-dimethyl-2-oxa-7-azaspiro[4.5]decan-1-one (0.032 g, 0.06 mmol) obtained in Example 66 was dissolved in a mixture of 1,2-dimethoxyethane (1.5 mL) and water (0.5 mL). Each of various boronic acids (0.06 mmol), sodium carbonate (0.02 g, 0.18 mmol) and a catalytic amount of tetrakis (triphenylphosphine)palladium were added thereto, and the mixture was tightly sealed, exposed to microwave (Personal Chemistry, manufactured by Emrys Optimizer) irradiation when the mixture was stirred at 150°C for 5 min. Water (2 mL) and ethyl acetate (2 mL) were added, and the mixture was stirred vigorously. Using phase Sep (manufactured by Wako Pure Chemical Industries, Ltd.), the aqueous layer was removed and the organic layer was concentrated. The residue was dissolved in dimethyl sulfoxide (0.5 mL), and purified by preparative HPLC to give the object compound at purity 80% or above (LC/MS analysis). (purification by HPLC in Examples 128, 129, 134, 138 and 139 was performed by Method B, and that in the other Examples by Method A)

The purification in Examples 67 - 100, 104 - 125 and 127 - 139 by preparative HPLC was performed under the following method A or method B.

<Method A>

instrument: High Through-put Purification System, Gilson Company, Inc.
column: YMC CombiPrep ODS-A S-5 μm, 50×20 mm gradient cycle: 0 min (SOLUTION A/SOLUTION B =95/5), 1.00 min (SOLUTION A/SOLUTION B =95/5), 5.20 min (SOLUTION A/SOLUTION B =5/95), 6.40 min (SOLUTION A/SOLUTION B =5/95), 6.50 min (SOLUTION A/SOLUTION B =95/5), 6.60 min (SOLUTION A/SOLUTION B =95/5)
solvent: SOLUTION A; 0.1% trifluoroacetic acid aqueous solution, SOLUTION B; 0.1% trifluoroacetic acid acetonitrile solution

flow rate: 20 mL/min, detection method: UV 220 nm

<Method B>

instrument: High Through-put Purification System, Gilson Company, Inc.
column: CombiPrep Hydrosphere C18 S-5 μm, 50x20 mm gradient cycle: 0 min (SOLUTION A/SOLUTION B =98/2), 1.00 min (SOLUTION A/SOLUTION B =98/2), 5.00 min (SOLUTION A/SOLUTION B =0/100), 6.40 min (SOLUTION A/SOLUTION B =0/100), 6.50 min (SOLUTION A/SOLUTION B =98/2), 6.60 min (SOLUTION A/SOLUTION B =98/2)
solvent: SOLUTION A; 0.1% trifluoroacetic acid aqueous solution, SOLUTION B; 0.1% trifluoroacetic acid acetonitrile solution
flow rate: 20 mL/min, detection method: UV 220 nm

The structural formulas and mass spectrum data of the compounds obtained in Examples 67 - 100, 104 - 125 and 127 - 139 are shown in Tables 3 - Table 7

Table 3

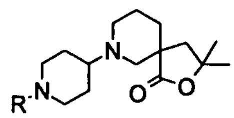

| Example No. | R | MS (m/Z) |
|---|---|---|
| 67 | 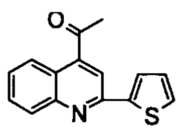 | 504 |
| 68 | 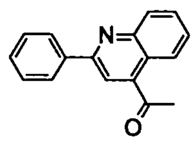 | 498 |
| 69 | 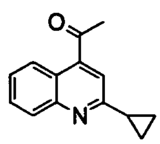 | 462 |
| 70 | 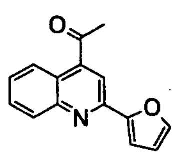 | 488 |
| 71 | 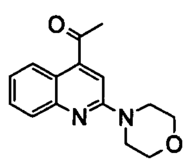 | 507 |
| 72 | 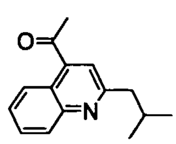 | 478 |

(continued)

| Example No. | R | MS (m/Z) |
|---|---|---|
| 73 | 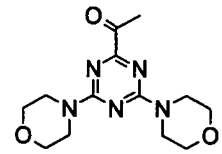 | 544 |
| 74 | 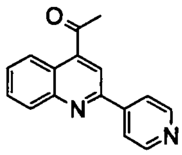 | 499 |
| 75 | 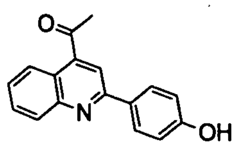 | 514 |
| 76 | 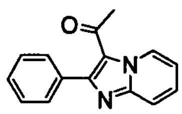 | 487 |
| 77 | 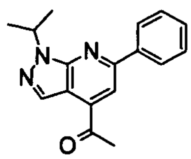 | 530 |
| 78 | 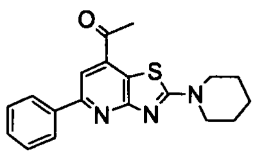 | 588 |
| 79 | 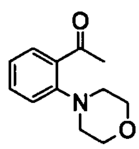 | 456 |
| 80 | 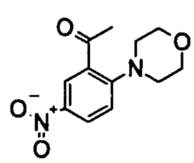 | 501 |

Table 4

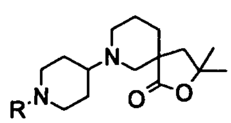

(continued)

| Example No. | R | MS (m/Z) |
|---|---|---|
| 81 | 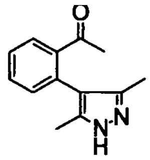 | 465 |
| 82 | 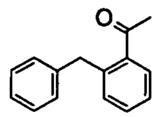 | 461 |
| 83 | 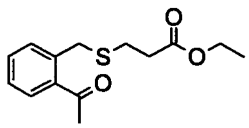 | 517 |
| 84 | 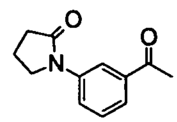 | 454 |
| 85 | 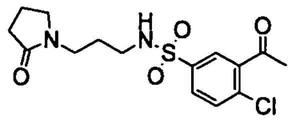 | 609 |
| 86 | 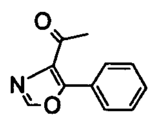 | 438 |
| 87 | 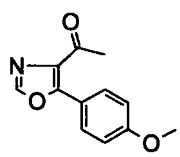 | 468 |
| 88 | 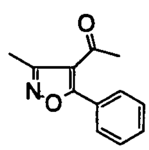 | 452 |
| 89 | 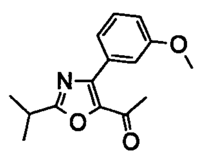 | 510 |
| 90 | 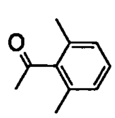 | 399 |

(continued)

| Example No. | R | MS (m/Z) |
|---|---|---|
| 91 | 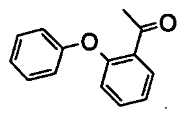 | 463 |
| 92 | 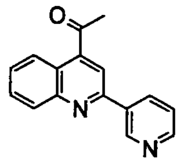 | 499 |
| 93 | 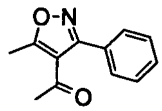 | 452 |
| 94 | 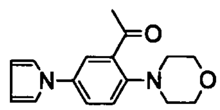 | 521 |

Table 5

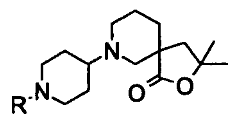

| Example No. | R | MS (m/Z) |
|---|---|---|
| 95 | 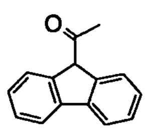 | 459 |
| 96 | 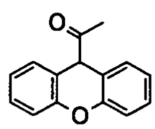 | 475 |
| 97 | 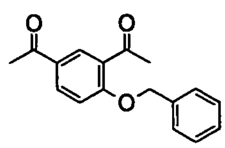 | 519 |
| 98 | 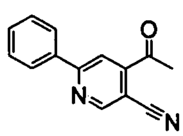 | 473 |

(continued)

| Example No. | R | MS (m/Z) |
|---|---|---|
| 99 | 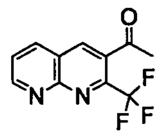 | 491 |
| 100 | 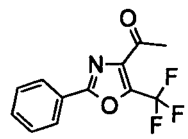 | 506 |

Table 6

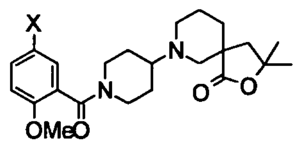

| Example No. | X | MS (m/Z) |
|---|---|---|
| 104 | 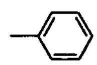 | 477 |
| 105 | 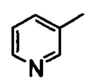 | 478 |
| 106 | 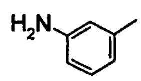 | 492 |
| 107 | 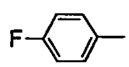 | 495 |
| 108 | 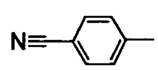 | 502 |
| 109 | 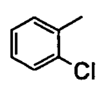 | 511 |
| 110 | 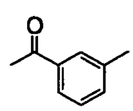 | 519 |
| 111 | 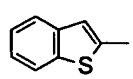 | 533 |
| 112 | 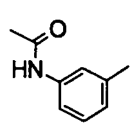 | 534 |

(continued)

| Example No. | X | MS (m/Z) |
|---|---|---|
| 113 | 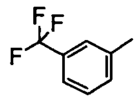 | 545 |
| 114 | 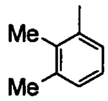 | 505 |
| 115 | 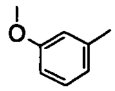 | 507 |
| 116 | 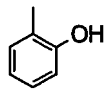 | 493 |
| 117 | 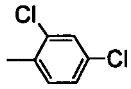 | 545 |
| 118 | 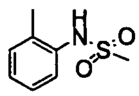 | 570 |
| 119 | 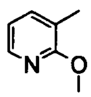 | 508 |
| 120 | 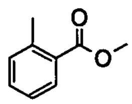 | 535 |
| 121 | 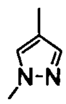 | 481 |
| 122 | 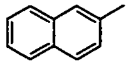 | 527 |
| 123 | 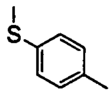 | 523 |
| 124 | 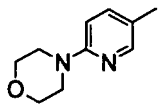 | 563 |
| 125 | 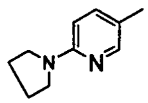 | 546 |

Table 7

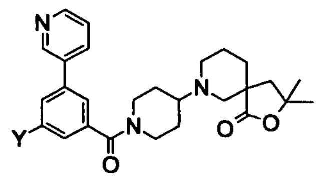

| Example No. | Y | MS (m/Z) |
|---|---|---|
| 127 | 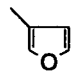 | 514 |
| 128 | 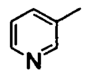 | 525 |
| 129 | 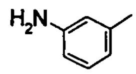 | 538 |
| 130 | 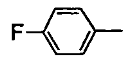 | 542 |
| 131 | 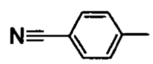 | 549 |
| 132 | 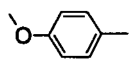 | 554 |
| 133 | 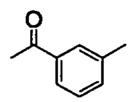 | 566 |
| 134 | 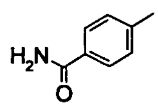 | 567 |
| 135 | 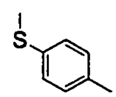 | 570 |
| 136 | 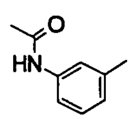 | 581 |
| 137 | 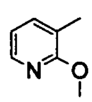 | 554 |
| 138 | 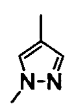 | 528 |
| 139 | 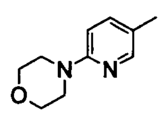 | 610 |

Example 140

7-[1-(9-anthrylcarbonyl)piperidin-4-yl]-3-methyl-3-propyl-2-oxa-7-azaspiro[4.5]decan-1-one

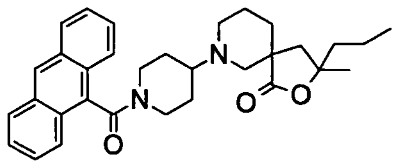

Using tert-butyl 3-methyl-1-oxo-3-propyl-2-oxa-7-azaspiro[4.5]decane-7-carboxylate (0.78 g, 2.50 mmol) obtained in Reference Example 58, the title compound (0.67 g, yield 53%) was obtained as an oil by an operation similar to that of Example 2.
EI(pos) 499 $[M+H]^+$

Example 141

N-(2-{7-[1-(9-anthrylcarbonyl)piperidin-4-yl]-3-methyl-1-oxo-2,7-diazaspiro[4.5]dec-2-yl}ethyl)methanesulfonamide

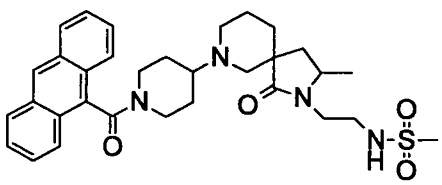

Using tert-butyl 3-methyl-2-{2-[(methylsulfonyl)amino]ethyl}-1-oxo-2,7-diazaspiro[4.5]decane-7-carboxylate (240 mg, 0.616 mmol) obtained in Reference Example 60, the title compound (74.5 mg, yield 21%) was obtained as an oil by an operation similar to that of Example 2.
EI(pos) 577 $[M+H]^+$

Example 142

7-[1-(9-anthrylcarbonyl)piperidin-4-yl]-1-oxo-7-azaspiro[4.5]decane-2-carbonitrile

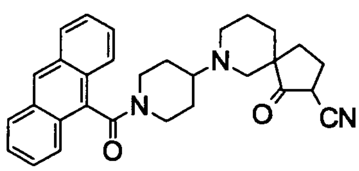

To a solution of ethyl 1'-(9-anthrylcarbonyl)-3-(3-cyanopropyl)-1,4'-bipiperidine-3-carboxylate (0.30 g, 0.587 mmol) obtained in Reference Example 62 in tetrahydrofuran (10 mL) was added potassium tert-butoxide (99 mg, 0.880 mmol), and the mixture was stirred at 70°C for 2 hr. A saturated aqueous ammonium chloride solution was added to the reaction mixture. The mixture was extracted twice with ethyl acetate, and the organic layer was dried over magnesium sulfate. The solvent was evaporated under reduced pressure, and the obtained residue was purified by column chromatography (developing solvent; ethyl acetate:hexane=3:1→1:0) to give the title compound (0.18 g, yield 65%) as an oil.
EI(pos) 466 $[M+H]^+$

Example 143

7-[1-(9-anthrylcarbonyl)piperidin-4-yl]-3,3-dimethyl-2,7-diazaspiro[4.5]decan-1-one

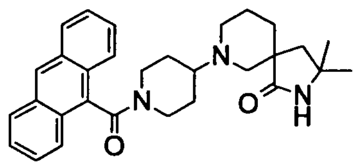

Using tert-butyl 3,3-dimethyl-1-oxo-2,7-diazaspiro[4.5]decane-7-carboxylate (0.64 g, 2.27 mmol) obtained in Reference Example 68, the title compound (30.0 mg, yield 2.8%) was obtained as an oil by an operation similar to that of Example 2.
EI(pos) 470 $[M+H]^+$

Example 144

7-[1-(9-anthrylcarbonyl)piperidin-4-yl]-3-ethyl-1-oxa-3,7-diazaspiro[4.5]decane-2,4-dione

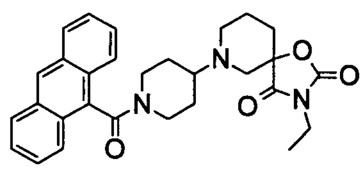

A solution of 3-ethyl-1-oxa-3,7-diazaspiro[4.5]decane-2,4-dione (0.50 g, 2.52 mmol) obtained in Reference Example 70, 1-(9-anthrylcarbonyl)piperidin-4-one (0.77 g, 2.52 mmol) obtained in Reference Example 8 and sodium triacetoxyborohydride (1.60 g, 7.56 mmol) in dichloromethane (20 mL) was stirred at room temperature for 20 hr. The reaction mixture was diluted with ethyl acetate, washed with saturated aqueous sodium hydrogencarbonate solution, and dried over anhydrous magnesium sulfate. The solvent was evaporated under reduced pressure, and the obtained residue was purified by basic silica gel column chromatography (developing solvent; ethyl acetate:hexane=7:13→3:1) to give the title compound (0.69 g, yield 56%) as an oil.
EI(pos) 486 $[M+H]^+$

Example 145

7-[1-(2-amino-5-bromobenzoyl)piperidin-4-yl]-3,3-dimethyl-2-oxa-7-azaspiro[4.5]decan-1-one

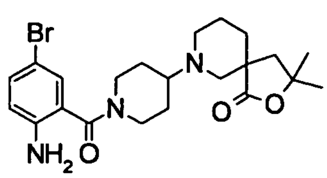

Using 3,3-dimethyl-7-(piperidin-4-yl)-2-oxa-7-azaspiro[4.5]decan-1-one dihydrochloride (0.34 g, 1.00 mmol) obtained in Reference Example 53 and 2-amino-5-bromobenzoic acid (324 mg, 1.50 mmol), the title compound (0.40

g, yield 86%) was obtained as an oil by an operation similar to that of Example 31.
EI(pos) 464 [M]+

Example 146

7-{1-[(4-aminobiphenyl-3-yl)carbonyl]piperidin-4-yl}-3,3-dimethyl-2-oxa-7-azaspiro[4.5]decan-1-one

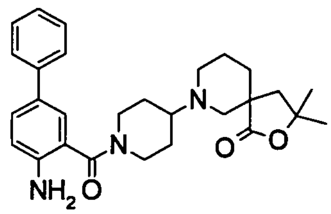

Using 7-[1-(2-amino-5-bromobenzoyl)piperidin-4-yl]-3,3-dimethyl-2-oxa-7-azaspiro[4.5]decan-1-one (0.36 g, 0.775 mmol) obtained in Reference Example 145 and phenylboronic acid (189 mg, 1.55 mmol), the title compound (0.20 g, yield 56%) was obtained as an oil by an operation similar to that of Reference Example 56.
EI(pos) 462 [M+H]+

Example 147

1-(3-{[4-(3,3-dimethyl-1-oxo-2-oxa-7-azaspiro[4.5]dec-7-yl)piperidin-1-yl]carbonyl}biphenyl-4-yl)-3-ethylurea

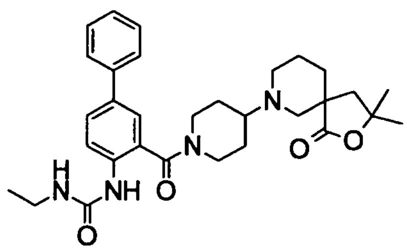

A solution of 7-{1-[(4-aminobiphenyl-3-yl)carbonyl]piperidin-4-yl}-3,3-dimethyl-2-oxa-7-azaspiro[4.5]decan-1-one (0.20 g, 0.433 mmol) obtained in Example 146, ethyl isocyanate (0.052 mL, 0.650 mmol) and triethylamine (0.091 mL, 0.650 mmol) in tetrahydrofuran (10 mL) was stirred at 60°C for 16 hr. The solvent was evaporated under reduced pressure, and the obtained residue was purified by basic silica gel column chromatography (developing solvent; methanol: ethyl acetate=0:1→1:9) to give the title compound (61.8 mg, yield 26%) as an oil.
EI(pos) 533 [M+H]+

Example 148

tert-butyl (3-{[4-(3,3-dimethyl-1-oxo-2-oxa-7-azaspiro[4.5]dec-7-yl)piperidin-1-yl]carbonyl}-4-methyl-5-phenyl-2-thienyl)carbamate

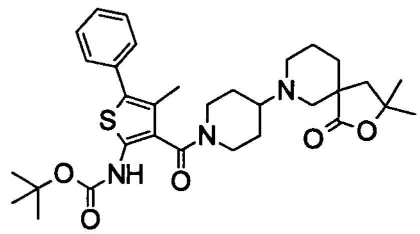

Using 3,3-dimethyl-7-(piperidin-4-yl)-2-oxa-7-azaspiro[4.5]decan-1-one dihydrochloride (1.07 g, 3.16 mmol) obtained in Reference Example 53 and 2-[(tert-butoxycarbonyl)amino]-4-methyl-5-phenylthiophene-3-carboxylic acid (877 mg, 2.63 mmol) obtained in Reference Example 74, the title compound (1.20 g, yield 78%) was obtained as an oil by an operation similar to that of Example 31.
EI(pos) 582 $[M+H]^+$

Example 149

1-(3-{[4-(3,3-dimethyl-1-oxo-2-oxa-7-azaspiro[4.5]dec-7-yl)piperidin-1-yl]carbonyl}-4-methyl-5-phenyl-2-thienyl)-3-ethylurea

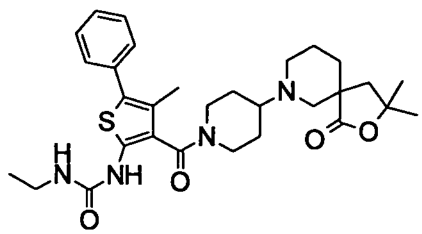

A solution of tert-butyl (3-{[4-(3,3-dimethyl-1-oxo-2-oxa-7-azaspiro[4.5]dec-7-yl)piperidin-1-yl]carbonyl}-4-methyl-5-phenyl-2-thienyl)carbamate (1.20 g, 2.06 mmol) obtained in Example 148 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 7-{1-[(2-amino-4-methyl-5-phenyl-3-thienyl)carbonyl]piperidin-4-yl}-3,3-dimethyl-2-oxa-7-azaspiro[4.5]decan-1-one (EI(pos) 482 $[M+H]^+$) as a crude product. The crude product was dissolved in pyridine (15 mL), ethyl isocyanate (0.32 mL, 4.13 mmol) was added, and the mixture was stirred at 60˚C for 2 hr. The solvent was evaporated under reduced pressure, and the obtained residue was purified by column chromatography (developing solvent; methanol:ethyl acetate=0:1→1:9) to give the title compound (165 mg, yield 14%) as an oil.
EI(pos) 553 $[M+H]^+$

Example 150

tert-butyl (3-{[4-(3,3-dimethyl-1-oxo-2-oxa-7-azaspiro[4.5]dec-7-yl)piperidin-1-yl]carbonyl}-5-phenyl-2-thienyl)carbamate

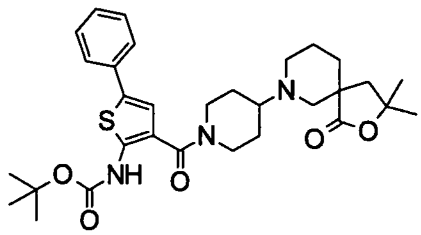

Using 3,3-dimethyl-7-(piperidin-4-yl)-2-oxa-7-azaspiro[4.5]decan-1-one dihydrochloride (0.50 g, 1.47 mmol) obtained in Reference Example 53 and 2-[(tert-butoxycarbonyl)amino]-5-phenylthiophene-3-carboxylic acid (565 mg, 1.77 mmol) obtained in Reference Example 76, the title compound (0.84 g, quantitative) was obtained as an oil by an operation similar to that of Example 31.
EI(pos) 568 $[M+H]^+$

Example 151

1-(3-{[4-(3,3-dimethyl-1-oxo-2-oxa-7-azaspiro[4.5]dec-7-yl)piperidin-1-yl]carbonyl}-5-phenyl-2-thienyl)-3-ethylurea

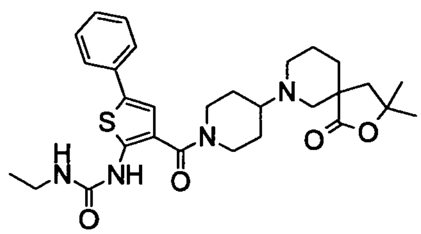

A solution of tert-butyl (3-{[4-(3,3-dimethyl-1-oxo-2-oxa-7-azaspiro[4.5]dec-7-yl)piperidin-1-yl]carbonyl}-5-phenyl-2-thienyl)carbamate (0.84 g, 1.48 mmol) obtained in Example 150 in trifluoroacetic acid (5 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 7-{1-[(2-amino-5-phenyl-3-thienyl)carbonyl]piperidin-4-yl}-3,3-dimethyl-2-oxa-7-azaspiro[4.5]decan-1-one as a crude product. The crude product was dissolved in pyridine (10 mL), ethyl isocyanate (0.24 mL, 2.96 mmol) was added, and the mixture was stirred at 60˚C for 4 hr. The solvent was evaporated under reduced pressure, and the obtained residue was purified by column chromatography (developing solvent; ethyl acetate:hexane=2:3→ 4:1) to give the title compound (397 mg, yield 50%) as a white solid.
EI(pos) 539 $[M+H]^+$

Example 152

9-[1-(9-anthrylcarbonyl)piperidin-4-yl]-3,3-dimethyl-6-(trifluoroacetyl)-2-oxa-6,9-diazaspiro[4.5]decan-1-one

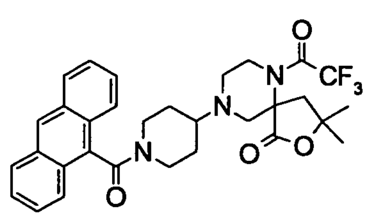

Using tert-butyl 3,3-dimethyl-1-oxo-6-(trifluoroacetyl)-2-oxa-6,9-diazaspiro[4.5]decane-9-carboxylate (1.51 g, 3.84 mmol) obtained in Reference Example 78, the title compound (0.73 g, yield 33%) was obtained as an oil by an operation similar to that of Example 2.
EI(pos) 568 $[M+H]^+$

Example 153

9-[1-(9-anthrylcarbonyl)piperidin-4-yl]-3,3-dimethyl-2-oxa-6,9-diazaspiro[4.5]decan-1-one

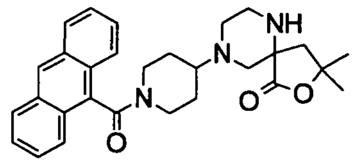

To a solution of 9-[1-(9-anthrylcarbonyl)piperidin-4-yl]-3,3-dimethyl-6-(trifluoroacetyl)-2-oxa-6,9-diazaspiro [4.5]decan-1-one (0.73 g, 1.29 mmol) obtained in Example 152 in methanol (20 mL) was added potassium carbonate (356 mg, 2.58 mmol), and the mixture was stirred at 60˚C for 3 hr. The mixture was allowed to cool to room temperature, 1N hydrochloric acid (10 mL) was added, and the mixture was stirred for 1 hr. The reaction mixture was neutralized with saturated aqueous sodium hydrogencarbonate solution. The mixture was extracted 3 times with ethyl acetate-tetrahydrofuran (1:1) mixture, and the organic layer was dried over anhydrous magnesium sulfate. The solvent was evaporated under reduced pressure, and the obtained residue was purified by basic silica gel column chromatography (developing solvent; methanol:ethyl acetate=0:1→1:9) to give the title compound (0.25 g, yield 41%) as an oil.
EI(pos) 472 $[M+H]^+$

Example 154

tert-butyl {9-[1-(9-anthrylcarbonyl)piperidin-4-yl]-3,3-dimethyl-1-oxo-2-oxa-6,9-diazaspiro[4.5]dec-6-yl}acetate

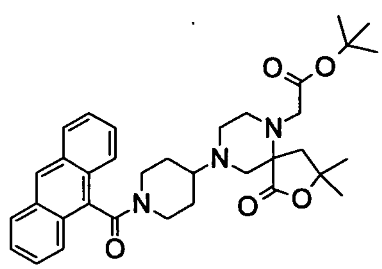

A solution of 9-[1-(9-anthrylcarbonyl)piperidin-4-yl]-3,3-dimethyl-2-oxa-6,9-diazaspiro[4.5]decan-1-one (0.22 g, 0.467 mmol) obtained in Example 153, tert-butyl bromoacetate (0.10 mL, 0.700 mmol) and triethylamine (0.20 mL, 1.40 mmol) in tetrahydrofuran (10 mL) was stirred at 60˚C for 1 hr. The solvent was evaporated under reduced pressure, and the obtained residue was purified by basic silica gel column chromatography (developing solvent; ethyl acetate: hexane=1:1→ 4:1) to give the title compound (0.10 g, yield 37%) as an oil. EI(pos) 586 $[M+H]^+$

Example 155

{9-[1-(9-anthrylcarbonyl)piperidin-4-yl]-3,3-dimethyl-1-oxo-2-oxa-6,9-diazaspiro[4.5]dec-6-yl}acetic acid dihydrochloride

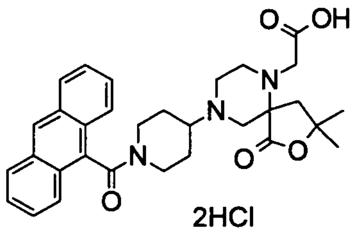

2HCl

A solution of tert-butyl {9-[1-(9-anthrylcarbonyl)piperidin-4-yl]-3,3-dimethyl-1-oxo-2-oxa-6,9-diazaspiro[4.5]dec-6-yl}acetate (0.10 g, 0.171 mmol) obtained in Example 154 in trifluoroacetic acid (5 mL) was stirred at room temperature for 1 hr. The solvent was evaporated under reduced pressure, 4N hydrogen chloride-ethyl acetate solution (5 mL) was added to the residue, and the solvent was evaporated under reduced pressure. The obtained yellow solid was washed with diethyl ether to give the title compound (84.9 mg, yield 82%).
EI(pos) 530 $[M+H]^+$

Example 156

7-[1-(9-anthrylcarbonyl)piperidin-4-yl]-2-methyl-1,3,7-triazaspiro[4.5]decan-4-one

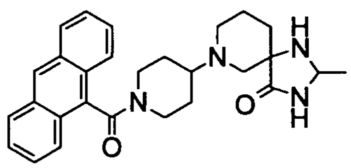

Using tert-butyl 2-methyl-4-oxo-1,3,7-triazaspiro[4.5]dec-1-en-7-carboxylate (1.07 g, 4.00 mmol) obtained in Reference Example 80, the title compound (51.7 mg, yield 2.8%) was obtained as an oil by an operation similar to that of Example 2.
EI(pos) 457 $[M+H]^+$

Example 157

ethyl 3-{7-[1-(9-anthrylcarbonyl)piperidin-4-yl]-3-methyl-1-oxo-2,7-diazaspiro[4.5]dec-2-yl}propanoate

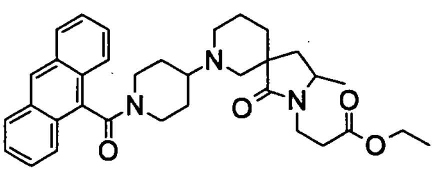

Using tert-butyl 2-(3-ethoxy-3-oxopropyl)-3-methyl-1-oxo-2,7-diazaspiro[4.5]decane-7-carboxylate (0.75 g, 2.04 mmol) obtained in Reference Example 81, the title compound (0.44 g, yield 39%) was obtained as an oil by an operation similar to that of Example 2.
EI(pos) 556 $[M+H]^+$

Example 158

3-{7-[1-(9-anthrylcarbonyl)piperidin-4-yl]-3-methyl-1-oxo-2,7-diazaspiro[4.5]dec-2-yl}propanoic acid

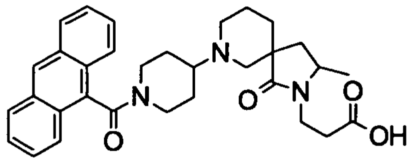

Using ethyl 3-{7-[1-(9-anthrylcarbonyl)piperidin-4-yl]-3-methyl-1-oxo-2,7-diazaspiro[4.5]dec-2-yl}propanoate (0.42 g, 0.758 mmol) obtained in Example 157, the title compound (65 mg, yield 16%) was obtained as a white solid by an operation similar to that of Example 9.
EI(pos) 528 $[M+H]^+$

Example 159

7-[1-(9-anthrylcarbonyl)piperidin-4-yl]-2-methyl-1,3,7-triazaspiro[4.5]dec-1-en-4-one

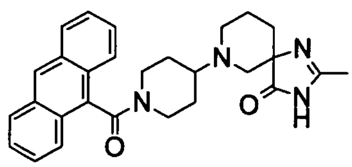

Using N-[1'-(9-anthrylcarbonyl)-3-cyano-1,4'-bipiperidin-3-yl]acetamide (0.50 g, 1.10 mmol) obtained in Reference Example 82, the title compound (182 mg, yield 36%) was obtained as an oil by an operation similar to that of Reference Example 80.
EI(pos) 455 $[M+H]^+$

Example 160

2-{7-[1-(9-anthrylcarbonyl)piperidin-4-yl]-3-methyl-1-oxo-2,7-diazaspiro[4.5]dec-2-yl}ethyl acetate

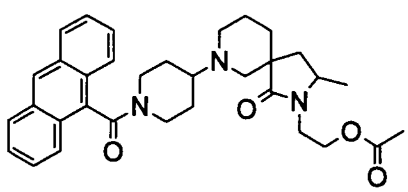

Using tert-butyl 2-(2-acetoxyethyl)-3-methyl-1-oxo-2,7-diazaspiro[4.5]decane-7-carboxylate (1.05 g, 2.96 mmol) obtained in Reference Example 83, an operation similar to that of Example 2 was performed to give the title compounds (339 mg, less polar, yield 21% and 292 mg, highly-polar, yield 18%) each as an oil.
EI(pos) 542 $[M+H]^+$

Example 161

7-[1-(9-anthrylcarbonyl)piperidin-4-yl]-2-(2-hydroxyethyl)-3-methyl-2,7-diazaspiro[4.5]decan-1-one

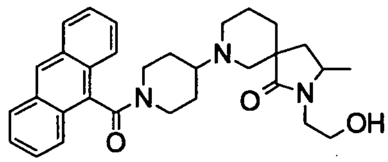

To a solution of 2-{7-[1-(9-anthrylcarbonyl)piperidin-4-yl]-3-methyl-1-oxo-2,7-diazaspiro[4.5]dec-2-yl}ethyl acetate (less polar component: 0.31 g, 0.574 mmol) obtained in Example 160 in tetrahydrofuran-methanol (5 mL-5 mL) was added potassium carbonate (0.16 g, 1.15 mmol), and the mixture was stirred at room temperature for 1 hr. Water was added to the reaction mixture. The mixture was extracted 3 times with ethyl acetate, and the organic layer was dried over anhydrous magnesium sulfate. The solvent was evaporated under reduced pressure, and the obtained residue was purified by basic silica gel column chromatography (developing solvent; methanol:ethyl acetate=0:1→1:9) to give the title compound (251 mg, yield 87%) as an oil.
EI(pos) 500 $[M+H]^+$

In the same manner, the title compound (200 mg, yield 82%) was obtained as an oil from 2-{7-[1-(9-anthrylcarbonyl)piperidin-4-yl]-3-methyl-1-oxo-2,7-diazaspiro[4.5]dec-2-yl}ethyl acetate (highly-polar component:0.26 g, 0.4894 mmol) obtained in Example 160.
EI(pos) 500 $[M+H]^+$

Example 162

tert-butyl (3-{[4-(3,3-dimethyl-1-oxo-2-oxa-7-azaspiro[4.5]dec-7-yl)piperidin-1-yl]carbonyl}-5-(pyridin-4-yl)-2-thienyl) carbamate

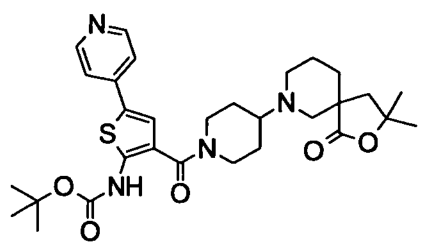

Using 3,3-dimethyl-7-(piperidin-4-yl)-2-oxa-7-azaspiro[4.5]decan-1-one dihydrochloride (0.50 g, 1.47 mmol) obtained in Reference Example 53 and 2-[(tert-butoxycarbonyl)amino]-5-(pyridin-4-yl)thiophene-3-carboxylic acid (614 mg, 1.92 mmol) obtained in Reference Example 85, the title compound (0.85 g, quantitative) was obtained as an oil by an operation similar to that of Example 31.
EI(pos) 569 $[M+H]^+$

Example 163

1-(3-{[4-(3,3-dimethyl-1-oxo-2-oxa-7-azaspiro[4.5]dec-7-yl)piperidin-1-yl]carbonyl}-5-(pyridin-4-yl)-2-thienyl)-3-ethylurea

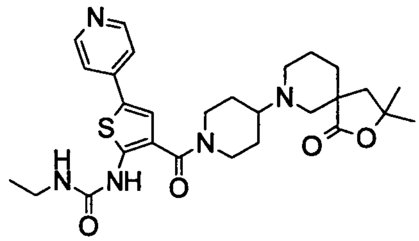

A solution of tert-butyl (3-{[4-(3,3-dimethyl-1-oxo-2-oxa-7-azaspiro[4.5]dec-7-yl)piperidin-1-yl]carbonyl}-5-(pyridin-4-yl)-2-thienyl)carbamate (0.85 g, 1.49 mmol) obtained in Example 162 in trifluoroacetic acid (5 mL) was stirred at room temperature for 30 min. The reaction mixture was neutralized with saturated aqueous sodium hydrogencarbonate solution and extracted twice with ethyl acetate. The organic layer was dried over anhydrous magnesium sulfate and the solvent was evaporated under reduced pressure to give 7-{1-[(2-amino-5-(pyridin-4-yl)-3-thienyl)carbonyl]piperidin-4-yl}-3,3-dimethyl-2-oxa-7-azaspiro[4.5]decan-1-one (EI(pos) 469 $[M+H]^+$) as a crude product. The crude product was dissolved in pyridine (10 mL), ethyl isocyanate (0.24 mL, 2.96 mmol) was added, and the mixture was stirred at 60°C for 4 hr. The solvent was evaporated under reduced pressure, and the obtained residue was purified by preparative HPLC to give the title compound (322 mg, yield 40%) as an oil.
EI(pos) 540 $[M+H]^+$

Example 164

7-[1-(9-anthrylcarbonyl)piperidin-4-yl]-2-[2-(1H-imidazol-1-yl)ethyl]-3-methyl-2,7-diazaspiro[4.5]decan-1-one

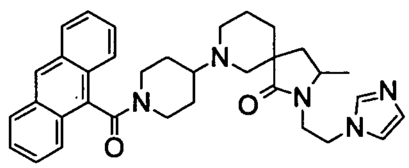

To a solution of 7-[1-(9-anthrylcarbonyl)piperidin-4-yl]-2-(2-hydroxyethyl)-3-methyl-2,7-diazaspiro[4.5]decan-1-one (less polar component:0.22 g, 0.440 mmol) obtained in Example 161 and triethylamine (0.123 mL, 0.880 mmol) in tetrahydrofuran (10 mL) was added methanesulfonyl chloride (0.052 mL, 0.660 mmol), and the mixture was stirred at room temperature for 30 min. A saturated aqueous sodium hydrogencarbonate solution was added to the reaction mixture. The mixture was extracted 3 times with ethyl acetate, and dried over anhydrous magnesium sulfate. The solvent was evaporated under reduced pressure, and a solution of the obtained residue and imidazole (150 mg, 2.20 mmol) in tetrahydrofuran (10 mL) was stirred at 60°C for 16 hr. The solvent was evaporated under reduced pressure, and the obtained residue was purified by basic silica gel column chromatography (developing solvent; methanol:ethyl acetate=0:1 →1:9) to give the title compound (95.1 mg, yield 39%) as an oil.
EI(pos) 550 $[M+H]^+$

Example 165

7-{1-[(3-amino-1-phenyl-1H-pyrazol-4-yl)carbonyl]piperidin-4-yl}-3,3-dimethyl-2-oxa-7-azaspiro[4.5]decan-1-one

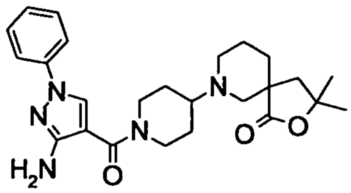

Using 3,3-dimethyl-7-(piperidin-4-yl)-2-oxa-7-azaspiro[4.5]decan-1-one dihydrochloride (0.50 g, 1.47 mmol) obtained in Reference Example 53 and 3-amino-1-phenyl-1H-pyrazole-4-carboxylic acid (0.36 g, 1.77 mmol) obtained in Reference Example 86, the title compound (0.47 g, yield 70%) was obtained as an oil by an operation similar to that of Example 31.
EI(pos) 452 $[M+H]^+$

Example 166

1-(4-{[4-(3,3-dimethyl-1-oxo-2-oxa-7-azaspiro[4.5]dec-7-yl)piperidin-1-yl]carbonyl}-1-phenyl-1H-pyrazol-3-yl)-3-ethylurea

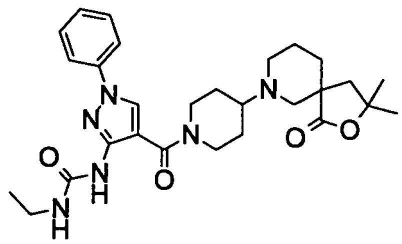

To a solution of 7-{1-[(3-amino-1-phenyl-1H-pyrazol-4-yl)carbonyl]piperidin-4-yl}-3,3-dimethyl-2-oxa-7-azaspiro[4.5]decan-1-one (0.47 g, 1.04 mmol) obtained in Example 165 in pyridine (5 mL) was added ethyl isocyanate (0.124 mL, 1.56 mmol), and the mixture was stirred at 60˚C for 4.5 hr. The solvent was evaporated under reduced pressure, and the obtained residue was purified by basic silica gel column chromatography (developing solvent; ethyl acetate:hexane=3:2→ 1:0) to give the title compound (192 mg, yield 35%) as a white solid.
EI(pos) 523 $[M+H]^+$

Example 167

3-{7-[1-(9-anthrylcarbonyl)piperidin-4-yl]-3-methyl-1-oxo-2,7-diazaspiro[4.5]dec-2-yl}propyl acetate

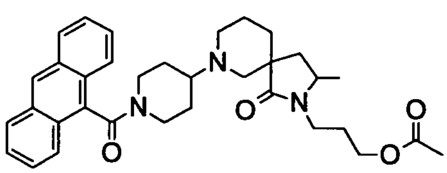

Using tert-butyl 2-(3-acetoxypropyl)-3-methyl-1-oxo-2,7-diazaspiro[4.5]decane-7-carboxylate (0.97 g, 2.64 mmol) obtained in Reference Example 87, an operation similar to that of Example 2 was performed to give the title compounds (256 mg, less polar, yield 17% and 339 mg, highly-polar, yield 23%) each as an oil.
EI(pos) 556 $[M+H]^+$

Example 168

7-[1-(9-anthrylcarbonyl)piperidin-4-yl]-2-(3-hydroxypropyl)-3-methyl-2,7-diazaspiro[4.5]decan-1-one

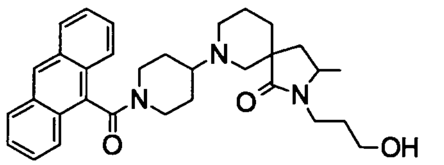

Using 3-{7-[1-(9-anthrylcarbonyl)piperidin-4-yl]-3-methyl-1-oxo-2,7-diazaspiro[4.5]dec-2-yl}propyl acetate (highly-polar component, 315 mg, 0.567 mmol) obtained in Example 167, the title compound (258 mg, yield 88%) was obtained as an oil by an operation similar to that of Example 161.
EI(pos) 514 $[M+H]^+$

Example 169

9-[1-(9-anthrylcarbonyl)piperidin-4-yl]-3,3-dimethyl-2,6-dioxa-9-azaspiro[4.5]decan-1-one

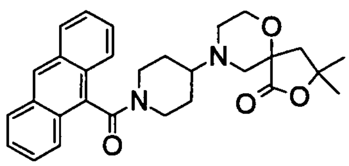

Using tert-butyl 3,3-dimethyl-1-oxo-2,6-dioxa-9-azaspiro[4.5]decane-9-carboxylate (0.88 g, 3.08 mmol) obtained in Reference Example 88, the title compound (0.80 g, yield 55%) was obtained as an oil by an operation similar to that of Example 2.
EI(pos) 473 $[M+H]^+$

Example 170

tert-butyl (5-bromo-3-{[4-(3,3-dimethyl-1-oxo-2-oxa-7-azaspiro[4.5]dec-7-yl)piperidin-1-yl]carbonyl}-2-thienyl)carbamate

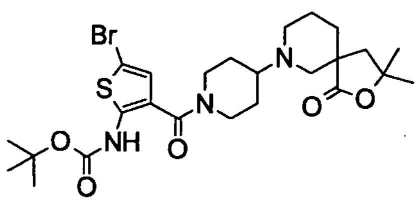

Using 3,3-dimethyl-7-(piperidin-4-yl)-2-oxa-7-azaspiro[4.5]decan-1-one dihydrochloride (0.50 g, 1.47 mmol) obtained in Reference Example 53 and 5-bromo-2-[(tert-butoxycarbonyl)amino]thiophene-3-carboxylic acid (1.05 g, 3.26 mmol) obtained in Reference Example 89, the title compound (1.78 g, yield 96%) was obtained as an oil by an operation similar to that of Example 31.

EI(pos) 571 [M+H]$^+$

Example 171

methyl 4-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)benzoate

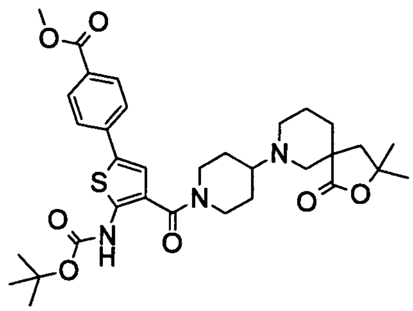

Using tert-butyl (5-bromo-3-{[4-(3,3-dimethyl-1-oxo-2-oxa-7-azaspiro[4.5]dec-7-yl)piperidin-1-yl]carbonyl}-2-thienyl)carbamate (0.89 g, 1.56 mmol) obtained in Example 170 and 4-methoxycarbonylphenylboronic acid (562 mg, 3.12 mmol), the title compound (0.97 g, yield 99%) was obtained as an oil by an operation similar to that of Reference Example 84. EI(pos) 626 [M+H]$^+$

Example 172

methyl 4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)benzoate

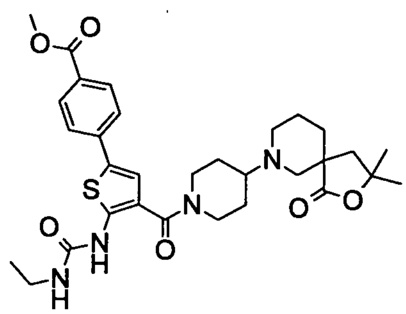

A solution of methyl 4-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)benzoate (1.05 g, 1.68 mmol) obtained in Example 171 in trifluoroacetic acid (20 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give methyl 4-(5-amino-4-{[4-(3,3-dimethyl-1-oxo-2-oxa-7-azaspiro[4.5]dec-7-yl)piperidin-1-yl]carbonyl}-2-thienyl)benzoate (EI(pos) 526 [M+H]$^+$) as a crude product. Using the crude product, the title compound (0.85 g, yield 85%) was obtained as an oil by an operation similar to that of Example 166.
EI(pos) 597 [M+H]$^+$

Example 173

methyl 3-[4-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)phenyl]propanoate

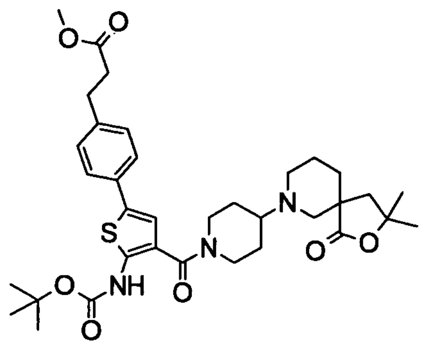

Using tert-butyl (5-bromo-3-{[4-(3,3-dimethyl-1-oxo-2-oxa-7-azaspiro[4.5]dec-7-yl)piperidin-1-yl]carbonyl}-2-thienyl)carbamate (0.89 g, 1.56 mmol) obtained in Example 170 and [4-(3-methoxy-3-oxopropyl)phenyl]boronic acid (649 mg, 3.12 mmol), the title compound (0.95 g, yield 93%) was obtained as an oil by an operation similar to that of Reference Example 84.
EI(pos) 654 $[M+H]^+$

Example 174

methyl 3-[4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)phenyl]propanoate

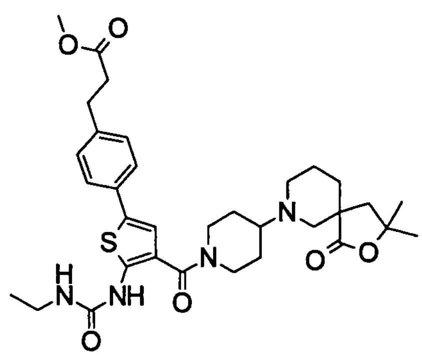

A solution of methyl 3-[4-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)phenyl]propanoate (0.95 g, 1.45 mmol) obtained in Example 173 in trifluoroacetic acid (20 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give methyl 3-[4-(5-amino-4-{[4-(3,3-dimethyl-1-oxo-2-oxa-7-azaspiro[4.5]dec-7-yl)piperidin-1-yl]carbonyl}-2-thienyl)phenyl]propanoate (EI(pos) 554 $[M+H]^+$) as a crude product. Using the crude product, the title compound (0.72 g, yield 79%) was obtained as an oil by an operation similar to that of Example 166.
EI(pos) 625 $[M+H]^+$

Example 175

3-[4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)phenyl]propanoic acid

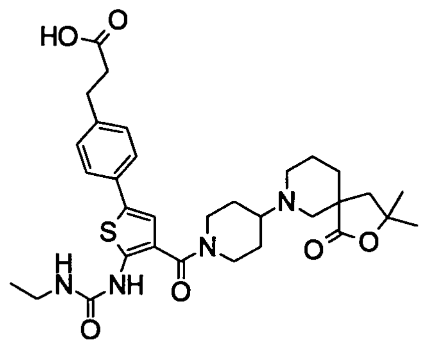

Using methyl 3-[4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)phenyl]propanoate (0.66 g, 1.06 mmol) obtained in Example 171, the title compound (55 mg, yield 8.5%) was obtained as a white solid by an operation similar to that of Example 9.
EI(pos) 611 $[M+H]^+$

Example 176

tert-butyl [5-(4-cyanophenyl)-3-{[4-(3,3-dimethyl-1-oxo-2-oxa-7-azaspiro[4.5]dec-7-yl)piperidin-1-yl]carbonyl}-2-thienyl] carbamate

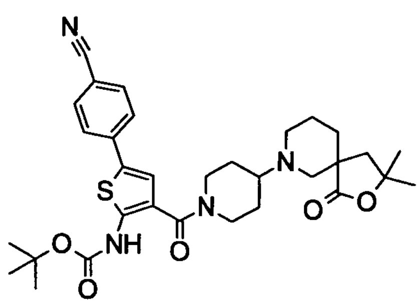

Using tert-butyl (5-bromo-3-{[4-(3,3-dimethyl-1-oxo-2-oxa-7-azaspiro[4.5]dec-7-yl)piperidin-1-yl]carbonyl}-2-thienyl)carbamate (0.50 g, 0.877 mmol) obtained in Example 170 and (4-cyanophenyl)boronic acid (258 mg, 1.76 mmol), the title compound (0.52 g, quantitative) was obtained as an oil by an operation similar to that of Reference Example 84.
EI(pos) 593 $[M+H]^+$

Example 177

1-[5-(4-cyanophenyl)-3-{[4-(3,3-dimethyl-1-oxo-2-oxa-7-azaspiro[4.5]dec-7-yl)piperidin-1-yl]carbonyl}-2-thienyl]-3-ethylurea

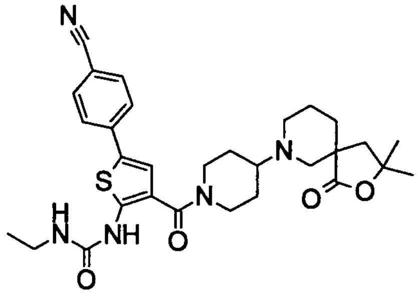

A solution of tert-butyl [5-(4-cyanophenyl)-3-{[4-(3,3-dimethyl-1-oxo-2-oxa-7-azaspiro[4.5]dec-7-yl)piperidin-1-yl]carbonyl}-2-thienyl]carbamate (0.66 g, 1.12 mmol) obtained in Example 176 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 4-(5-amino-4-{[4-(3,3-dimethyl-1-oxo-2-oxa-7-azaspiro[4.5]dec-7-yl)piperidin-1-yl]carbonyl}-2-thienyl)benzonitrile(EI(pos) 493 $[M+H]^+$) as a crude product. Using the crude product, the title compound (0.42 g, yield 66%) was obtained as an oil by an operation similar to that of Example 166.
EI(pos) 564 $[M+H]^+$

Example 178

tert-butyl (3-{[4-(3,3-dimethyl-1-oxo-2-oxa-7-azaspiro[4.5]dec-7-yl)piperidin-1-yl]carbonyl}-5-(pyridin-2-yl)-2-thienyl) carbamate

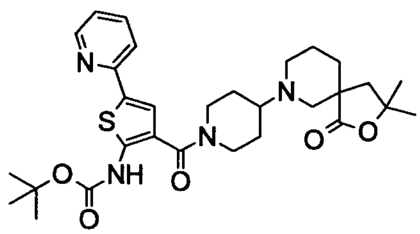

A solution of tert-butyl (5-bromo-3-{[4-(3,3-dimethyl-1-oxo-2-oxa-7-azaspiro[4.5]dec-7-yl)piperidin-1-yl]carbonyl}-2-thienyl)carbamate (0.80 g, 1.40 mmol) obtained in Example 170, 2-(tributylstannyl)pyridine (1.15 g, 2.82 mmol) and tetrakis(triphenylphosphine)palladium (81.1 mg, 0.0702 mmol) in 1,4-dioxane (20 mL) was stirred at 110˚C for 12 hr under an argon atmosphere. The solvent was evaporated under reduced pressure, and the obtained residue was purified by basic silica gel column chromatography (developing solvent; ethyl acetate:hexane=3:7→7:3) to give the title compound (0.65 g, yield 81%) as an oil.
EI(pos) 569 $[M+H]^+$

Example 179

1-(3-{[4-(3,3-dimethyl-1-oxo-2-oxa-7-azaspiro[4.5]dec-7-yl)piperidin-1-yl]carbonyl}-5-(pyridin-2-yl)-2-thienyl)-3-ethylurea

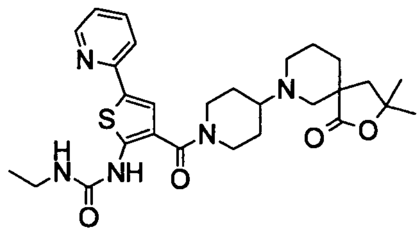

A solution of tert-butyl (3-{[4-(3,3-dimethyl-1-oxo-2-oxa-7-azaspiro[4.5]dec-7-yl)piperidin-1-yl]carbonyl}-5-(pyridin-2-yl)-2-thienyl)carbamate (0.65 g, 1.15 mmol) obtained in Example 178 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure to give 7-{1-[(2-amino-5-(pyridin-2-yl)-3-thienyl)carbonyl]piperidin-4-yl}-3,3-dimethyl-2-oxa-7-azaspiro[4.5]decan-1-one (EI(pos) 469 $[M+H]^+$) as a crude product. Using the crude product, the title compound (0.57 g, yield 92%) was obtained as an oil by an operation similar to that of Example 166.
EI(pos) 540 $[M+H]^+$

Example 180

7-[1-(9-anthrylcarbonyl)piperidin-4-yl]-3-(2-{[tert-butyl(dimethyl)silyl]oxy}ethyl)-2-methyl-1,3,7-triazaspiro[4.5]dec-1-en-4-one

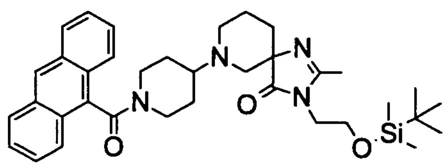

To a solution of 7-[1-(9-anthrylcarbonyl)piperidin-4-yl]-2-methyl-1,3,7-triazaspiro[4.5]dec-1-en-4-one (141 mg, 0.310 mmol) obtained in Example 159 in N,N-dimethylformamide (10 mL) was added 60% sodium hydride (16.9 mg, 0.419 mmol), and the mixture was stirred at room temperature for 30 min. To the solution was added (2-bromoethoxy)(tert-butyl)dimethylsilane (0.134 mL, 0.622 mmol), and the mixture was stirred at room temperature for 1 hr. The reaction mixture was diluted with ethyl acetate, washed 3 times with brine, and dried over anhydrous magnesium sulfate. The solvent was evaporated under reduced pressure, and the obtained residue was purified by basic silica gel column chromatography (developing solvent; ethyl acetate:hexane=3:1→1:0) to give the title compound (92.2 mg, yield 49%) as an oil.
EI(pos) 613 $[M+H]^+$

Example 181

7-[1-(9-anthrylcarbonyl)piperidin-4-yl]-3-(2-hydroxyethyl)-2-methyl-1,3,7-triazaspiro[4.5]dec-1-en-4-one

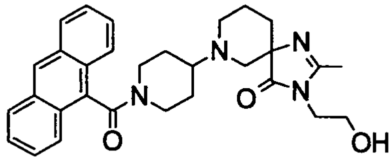

To a solution of 7-[1-(9-anthrylcarbonyl)piperidin-4-yl]-3-(2-{[tert-butyl(dimethyl)silyl]oxy}ethyl)-2-methyl-1,3,7-triazaspiro[4.5]dec-1-en-4-one (92.2 mg, 0.150 mmol) obtained in Example 180 in tetrahydrofuran (10 mL) was added 1N tetrabutylammonium fluoride-tetrahydrofuran solution (0.3 mL, 0.300 mmol), and the mixture was stirred at room temperature for 30 min. Water was added to the reaction mixture and the mixture was extracted 3 times with ethyl acetate. The organic layer was dried over anhydrous magnesium sulfate. The solvent was evaporated under reduced pressure, and the obtained residue was purified by preparative HPLC to give the title compound (65.8 mg, yield 88%) as an oil.
EI(pos) 499 $[M+H]^+$

Example 182

benzyl 9-[1-({2-[(tert-butoxycarbonyl)amino]-1-benzothien-3-yl}carbonyl)piperidin-4-yl]-3,3-dimethyl-1-oxo-2-oxa-6,9-diazaspiro[4.5]decane-6-carboxylate

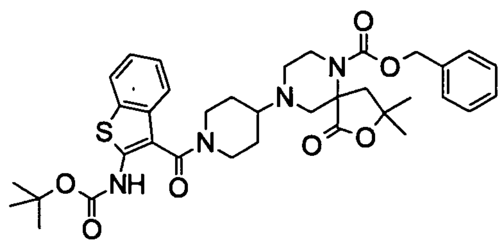

To benzyl 9-[1-(tert-butoxycarbonyl)piperidin-4-yl]-3,3-dimethyl-1-oxo-2-oxa-6,9-diazaspiro[4.5]decane-6-carboxylate (0.89 g, 1.78 mmol) obtained in Reference Example 90 was added 4N hydrogen chloride-ethyl acetate solution (10 mL), and the mixture was stirred at room temperature for 30 min. The solvent was evaporated under reduced pressure, and the obtained white solid was washed with diethyl ether. Using the white solid and 2-[(tert-butoxycarbonyl) amino]-1-benzothiophene-3-carboxylic acid (575 mg, 1.96 mmol) obtained in Reference Example 50, the title compound (1.05 g, yield 87%) was obtained as an oil by an operation similar to that of Example 31.
EI(pos) 677 $[M+H]^+$

Example 183

benzyl 9-[1-({2-[(ethylcarbamoyl)amino]-1-benzothien-3-yl}carbonyl)piperidin-4-yl]-3,3-dimethyl-1-oxo-2-oxa-6,9-diazaspiro[4.5]decane-6-carboxylate

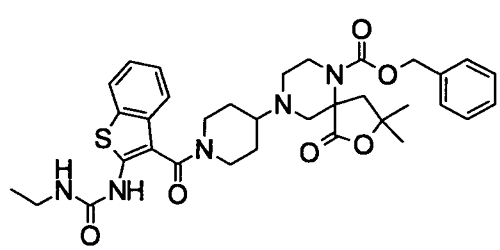

A solution of benzyl 9-[1-({2-[(tert-butoxycarbonyl)amino]-1-benzothien-3-yl}carbonyl)piperidin-4-yl]-3,3-dimethyl-1-oxo-2-oxa-6,9-diazaspiro[4.5]decane-6-carboxylate (1.05 g, 1.55 mmol) obtained in Example 182 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure to give benzyl 9-{1-[(2-amino-1-benzothien-3-yl)carbonyl]piperidin-4-yl}-3,3-dimethyl-1-oxo-2-oxa-6,9-diazaspiro[4.5]decane-6-carboxylate (EI(pos) 577 $[M+H]^+$) as a crude product. Using the crude product, the title compound (0.90 g, yield 90%) was obtained as an oil by an operation

similar to that of Example 166.
EI(pos) 648 $[M+H]^+$

Example 184

1-(3-{[4-(3,3-dimethyl-1-oxo-2-oxa-6,9-diazaspiro[4.5]dec-9-yl)piperidin-1-yl]carbonyl}-1-benzothien-2-yl)-3-ethylurea

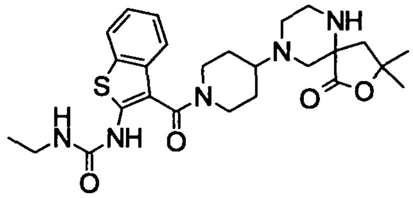

A suspension of benzyl 9-[1-({2-[(ethylcarbamoyl)amino]-1-benzothien-3-yl}carbonyl)piperidin-4-yl]-3,3-dimethyl-1-oxo-2-oxa-6,9-diazaspiro[4.5]decane-6-carboxylate (0.90 g, 1.19 mmol) obtained in Example 183 and 10% palladium carbon (50% water-containing product, 127 mg) in ethanol (20 mL) was stirred for 1 hr under a hydrogen atmosphere. After filtration, the filtrate was concentrated under reduced pressure, and the obtained residue was purified by basic silica gel column chromatography (developing solvent; methanol:ethyl acetate=0:1 →1:9) to give the title compound (492 mg, yield 69%) as an oil. EI(pos) 514 $[M+H]^+$

Example 185

2-{7-[1-({2-[(tert-butoxycarbonyl)amino]-1-benzothien-3-yl}carbonyl)piperidin-4-yl]-3-methyl-1-oxo-2,7-diazaspiro[4.5]dec-2-yl}ethyl acetate

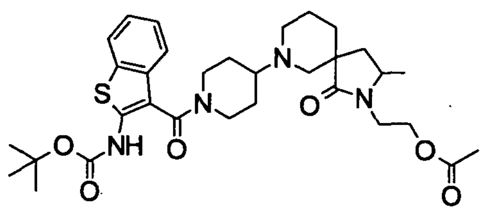

Using tert-butyl 4-[2-(2-acetoxyethyl)-3-methyl-1-oxo-2,7-diazaspiro[4.5]dec-7-yl]piperidine-1-carboxylate (935 mg, 2.14 mmol) obtained in Reference Example 91, the title compound (0.55 g, yield 42%) was obtained as an oil by an operation similar to that of Example 182.
EI(pos) 613 $[M+H]^+$

Example 186

2-{7-[1-({2-[(ethylcarbamoyl)amino]-1-benzothien-3-yl}carbonyl)piperidin-4-yl]-3-methyl-1-oxo-2,7-diazaspiro[4.5]dec-2-yl}ethyl acetate

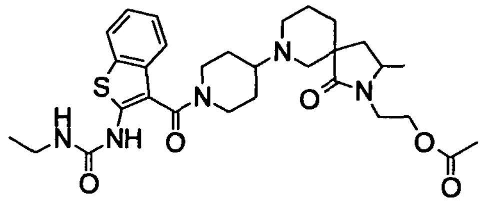

A solution of 2-{7-[1-({2-[(tert-butoxycarbonyl)amino]-1-benzothien-3-yl}carbonyl)piperidin-4-yl]-3-methyl-1-oxo-2,7-diazaspiro[4.5]dec-2-yl}ethyl acetate (0.55 g, 0.898 mmol) obtained in Example 185 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure to give 2-(7-{1-[(2-amino-1-benzothien-3-yl) carbonyl]piperidin-4-yl}-3-methyl-1-oxo-2,7-diazaspiro[4.5]dec-2-yl)ethyl acetate (EI(pos) 513 $[M+H]^+$) as a crude product. Using the crude product, the title compound (0.37 g, yield 70%) was obtained as an oil by an operation similar to that of Example 166.
EI(pos) 584 $[M+H]^+$

Example 187

1-ethyl-3-[3-({4-[2-(2-hydroxyethyl)-3-methyl-1-oxo-2,7-diazaspiro[4.5]dec-7-yl]piperidin-1-yl}carbonyl)-1-benzothien-2-yl]urea

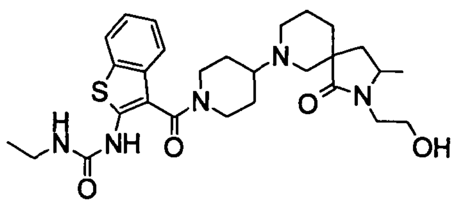

Using 2-{7-[1-({2-[(ethylcarbamoyl)amino]-1-benzothien-3-yl}carbonyl)piperidin-4-yl]-3-methyl-1-oxo-2,7-diazaspiro[4.5]dec-2-yl}ethyl acetate (0.37 g, 0.634 mmol) obtained in Example 186, the title compound (265 mg, yield 77%) was obtained as an oil by an operation similar to that of Example 161.
EI(pos) 542 $[M+H]^+$

Example 188

benzyl 4-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)benzoate

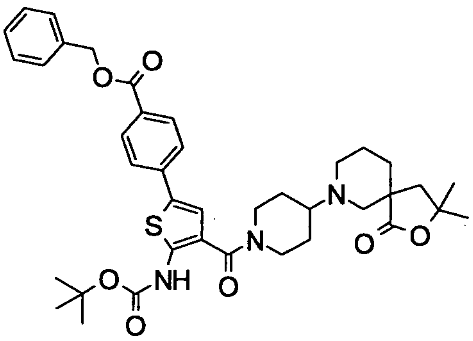

Using tert-butyl (5-bromo-3-{[4-(3,3-dimethyl-1-oxo-2-oxa-7-azaspiro[4.5]dec-7-yl)piperidin-1-yl]carbonyl}-2-thienyl)carbamate (0.89 g, 1.56 mmol) obtained in Example 170 and (4-benzyloxycarbonylphenyl)boronic acid (800 mg, 3.12 mmol), the title compound (1.09 g, quantitative) was obtained as an oil by an operation similar to that of Reference Example 84.
EI(pos) 702 $[M+H]^+$

Example 189

benzyl 4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)benzoate

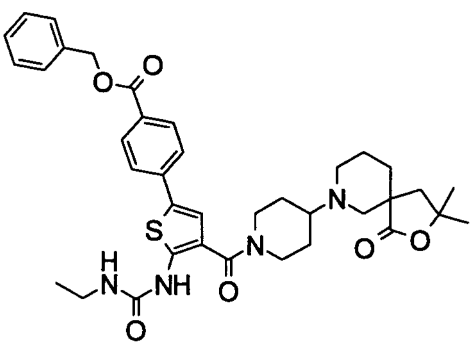

A solution of benzyl 4-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)benzoate (1.12 g, 1.60 mmol) obtained in Example 188 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give benzyl 4-(5-amino-4-{[4-(3,3-dimethyl-1-oxo-2-oxa-7-azaspiro[4.5]dec-7-yl)piperidin-1-yl]carbonyl}-2-thienyl)benzoate (EI(pos) 602$[M+H]^+$) as a crude product. Using the crude product, the title compound (0.61 g, yield 57%) was obtained as an oil by an operation similar to that of Example 166.
EI(pos) 673 $[M+H]^+$

Example 190

4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)benzoic acid

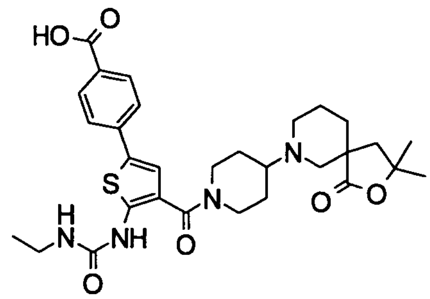

A suspension of benzyl 4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)benzoate (0.30 g, 0.446 mmol) obtained in Example 189 and 10% palladium carbon (50% water-containing product, 48 mg) in ethanol (10 mL) was stirred for 2.5 hr under a hydrogen atmosphere. After filtration, the filtrate was concentrated under reduced pressure, and the obtained solid was washed with ethyl acetate to give the title compound (12.5 mg, yield 4.8%) as a white solid.
EI(pos) 583 $[M+H]^+$

Example 191

ethyl (2E)-3-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)acrylate

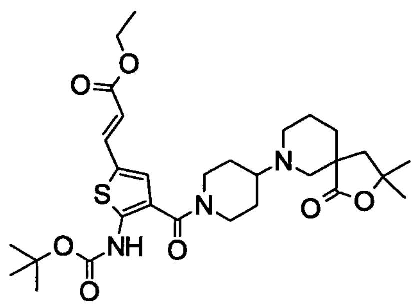

A solution of tert-butyl (5-bromo-3-{[4-(3,3-dimethyl-1-oxo-2-oxa-7-azaspiro[4.5]dec-7-yl)piperidin-1-yl]carbonyl}-2-thienyl)carbamate (1.00 g, 1.75 mmol) obtained in Example 170, ethyl acrylate (0.38 mL, 3.51 mmol), tetrabutylammonium chloride (487 mg, 1.75 mmol), triethylamine (0.49 mL, 3.51 mmol) and palladium acetate (19.7 mg, 0.0875 mmol) in N,N-dimethylformamide (10 mL) was stirred at 90°C for 12 hr. The reaction mixture was diluted with ethyl acetate, washed 3 times with saturated brine, and dried over anhydrous magnesium sulfate. The solvent was evaporated under reduced pressure, and the obtained residue was purified by column chromatography (developing solvent; ethyl acetate:hexane=7:3→1:0) to give the title compound (1.00 g, yield 96%) as an oil.
EI(pos) 590 $[M+H]^+$

Example 192

ethyl (2E)-3-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)acrylate

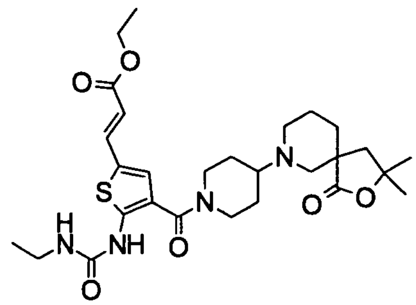

Using ethyl (2E)-3-(5-[(tert-butoxycarbonyl)amino]-4-{[4-(3,3-dimethyl-1-oxo-2-oxa-7-azaspiro[4.5]dec-7-yl) piperidin-1-yl]carbonyl}-2-thienyl)acrylate (1.00 g, 1.70 mmol) obtained in Example 191, the title compound (331 mg, yield 34%) was obtained as an oil by an operation similar to that of Example 149.
EI(pos) 561 $[M+H]^+$

Example 193

3-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)propyl acetate

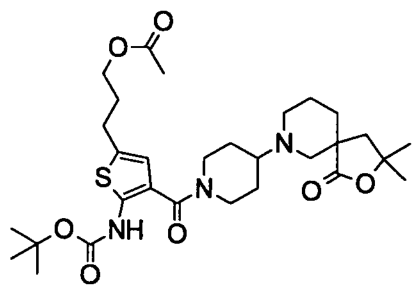

A suspension of 3,3-dimethyl-7-(piperidin-4-yl)-2-oxa-7-azaspiro[4.5]decan-1-one dihydrochloride (0.50 g, 1.47 mmol) obtained in Reference Example 53, 2-[(tert-butoxycarbonyl)amino]-5-(3-hydroxypropyl)thiophene-3-carboxylic acid (768 mg, 2.26 mmol) obtained in Reference Example 93, 1-ethyl-3-(3-dimethylaminopropyl)carbodiimide hydrochloride (593 mg, 3.09 mmol), 1-hydroxybenzotriazole (418 mg, 3.09 mmol) and triethylamine (0.60 mL, 4.74 mmol) in N,N-dimethylformamide (15 mL) was stirred at room temperature for 24 hr. The reaction mixture was diluted with ethyl acetate, washed 3 times with saturated brine, and dried over anhydrous magnesium sulfate. The solvent was evaporated under reduced pressure, and the obtained residue was dissolved in acetic anhydride (10 mL) and pyridine (10 mL), and the mixture was stirred at room temperature for 30 min. The solvent was evaporated under reduced pressure, and the obtained residue was purified by basic silica gel column chromatography (developing solvent; ethyl acetate:hexane=1:4→3:2) to give the title compound (0.57 g, yield 47%) as an oil.
EI(pos) 592 $[M+H]^+$

Example 194

3-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)propyl acetate

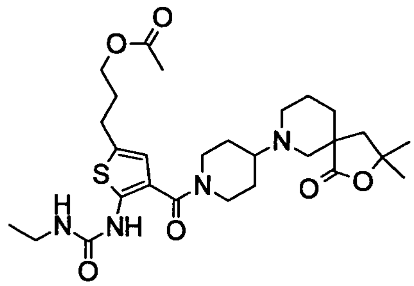

A solution of 3-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)propyl acetate (0.57 g, 0.963 mmol) obtained in Example 193 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure to give 3-(5-amino-4-{[4-(3,3-dimethyl-1-oxo-2-oxa-7-azaspiro[4.5]dec-7-yl)piperidin-1-yl]carbonyl}-2-thienyl)propyl acetate (EI(pos) 492[M+H]$^+$) as a crude product. Using the crude product, the title compound (465 mg, yield 85%) was obtained as an oil by an operation similar to that of Example 166.
EI(pos) 563 [M+H]$^+$

Example 195

1-[3-{[4-(3,3-dimethyl-1-oxo-2-oxa-7-azaspiro[4.5]dec-7-yl)piperidin-1-yl]carbonyl}-5-(3-hydroxypropyl)-2-thienyl]-3-ethylurea

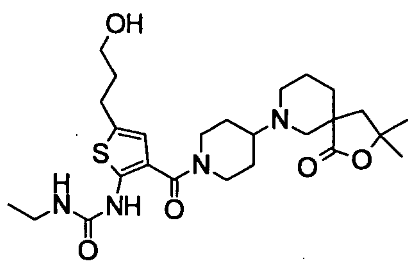

Using 3-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)propyl acetate (0.44 g, 0.845 mmol) obtained in Example 194, the title compound (249 mg, yield 56%) was obtained as an oil by an operation similar to that of Example 161.
EI(pos) 521 [M+H]$^+$

Example 196

ethyl 3-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)propanoate

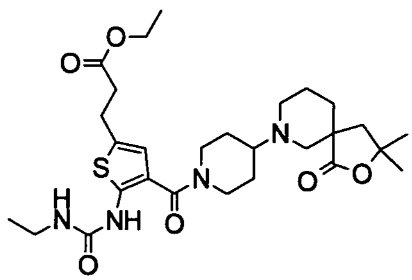

A suspension of ethyl (2E)-3-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)acrylate (0.63 g, 1.07 mmol) obtained in Example 191 and 10% palladium carbon (50% water-containing product, 114 mg) in ethanol (20 mL) was stirred at room temperature for 1.7 hr under a hydrogen atmosphere. After filtration, the filtrate was concentrated under reduced pressure to give ethyl 3-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)propanoate (EI(pos) 592 $[M+H]^+$) as a crude product. A solution of the crude product in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure to give ethyl 3-(5-amino-4-{[4-(3,3-dimethyl-l-oxo-2-oxa-7-azaspiro[4.5]dec-7-yl)piperidin-1-yl]carbonyl}-2-thienyl)propanoate as a crude product. Using the crude product, the title compound (160 mg, yield 27%) was obtained as an oil by an operation similar to that of Example 166.
EI(pos) 563 $[M+H]^+$

Example 197

1-(3-{[4-(3,3-dimethyl-1-oxo-2-oxa-7-azaspiro[4.5]dec-7-yl)piperidin-1-yl]carbonyl}-2-thienyl)-3-ethylurea

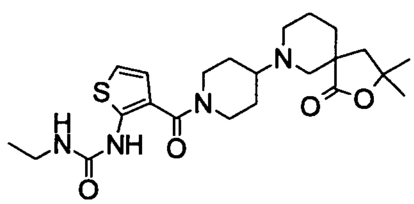

A solution of tert-butyl (5-bromo-3-{[4-(3,3-dimethyl-1-oxo-2-oxa-7-azaspiro[4.5]dec-7-yl)piperidin-1-yl]carbonyl}-2-thienyl)carbamate (0.93 g, 1.63 mmol) obtained in Example 170, ethyl acrylate (0.36 mL, 3.26 mmol), tetrabutylammonium chloride (454 mg, 1.63 mmol), triethylamine (0.46 mL, 3.26 mmol) and palladium acetate (18.3 mg, 0.0815 mmol) in N,N-dimethylformamide (10 mL) was stirred at 90˚C for 14 hr under an argon atmosphere. The reaction mixture was diluted with ethyl acetate, washed 3 times with saturated brine, and dried over anhydrous magnesium sulfate. The solvent was evaporated under reduced pressure, and the obtained residue was purified by column chromatography (developing solvent; ethyl acetate:hexane=7:3→1:0). The obtained oil was dissolved in ethanol (20 mL), 10% palladium carbon (50% water-containing product, 114 mg) was added, and the mixture was stirred at room temperature for 1.7 hr under a hydrogen atmosphere. After filtration, the filtrate was concentrated under reduced pressure. The obtained residue was dissolved in trifluoroacetic acid (10 mL), and the mixture was stirred at room temperature for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure. The obtained residue was dissolved in pyridine (5 mL), ethyl isocyanate (0.170 mL, 2.14 mmol) was added thereto, and the mixture was stirred at 60˚C for 4.5 hr. The solvent was evaporated under reduced pressure, and the obtained residue was purified by preparative HPLC to give the title compound (45.2 mg, yield 6.0%) as an oil.
EI(pos) 463 $[M+H]^+$

Example 198

3-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)propanoic acid

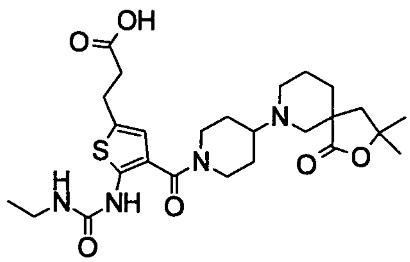

Using ethyl 3-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)propanoate (0.14 g, 0.249 mmol) obtained in Example 196, the title compound (7.5 mg, yield 5.6%) was obtained as a white solid by an operation similar to that of Example 9.
EI(pos) 535 $[M+H]^+$

Example 199

tert-butyl (1-benzyl-5-{[4-(3,3-dimethyl-1-oxo-2-oxa-7-azaspiro[4.5]dec-7-yl)piperidin-1-yl]carbonyl}-2-phenyl-1H-imidazol-4-yl)carbamate

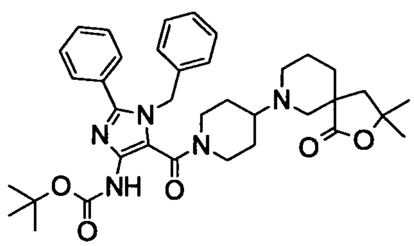

Using 3,3-dimethyl-7-(piperidin-4-yl)-2-oxa-7-azaspiro[4.5]decan-1-one dihydrochloride (0.43 g, 1.27 mmol) obtained in Reference Example 53 and 1-benzyl-4-[(tert-butoxycarbonyl)amino]-2-phenyl-1H-imidazole-5-carboxylic acid (0.50 g, 1.27 mmol) obtained in Reference Example 97, the title compound (0.73 g, yield 90%) was obtained as an oil by an operation similar to that of Example 31.
EI(pos) 642 $[M+H]^+$

Example 200

1-(1-benzyl-5-{[4-(3,3-dimethyl-1-oxo-2-oxa-7-azaspiro[4.5]dec-7-yl)piperidin-1-yl]carbonyl}-2-phenyl-1H-imidazol-4-yl)-3-ethylurea

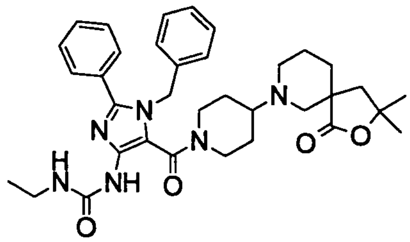

A solution of tert-butyl (1-benzyl-5-{[4-(3,3-dimethyl-1-oxo-2-oxa-7-azaspiro[4.5]dec-7-yl)piperidin-1-yl]carbonyl}-2-phenyl-1H-imidazol-4-yl)carbamate (0.73 g, 1.14 mmol) obtained in Example 199 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure to give 7-{1-[(4-amino-1-benzyl-2-phenyl-1H-imidazol-5-yl)carbonyl]piperidin-4-yl}-3,3-dimethyl-2-oxa-7-azaspiro[4.5]decan-1-one (EI(pos) 542 $[M+H]^+$) as a crude product. Using the crude product, the title compound (446 mg, yield 64%) was obtained as an oil by an operation similar to that of Example 166.
EI(pos) 613 $[M+H]^+$

Example 201

1-(4-{[4-(3,3-dimethyl-1-oxo-2-oxa-7-azaspiro[4.5]dec-7-yl)piperidin-1-yl]carbonyl}-2-phenyl-1H-imidazol-5-yl)-3-ethylurea

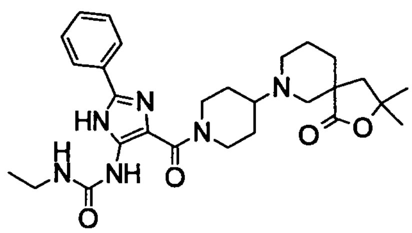

A suspension of 1-(1-benzyl-5-{[4-(3,3-dimethyl-1-oxo-2-oxa-7-azaspiro[4.5]dec-7-yl)piperidin-1-yl]carbonyl}-2-phenyl-1H-imidazol-4-yl)-3-ethylurea (0.20 g, 0.327 mmol) obtained in Example 200 and 20% palladium hydroxide on carbon (0.20 g) in ethanol (10 mL) was stirred at 70°C for 2 hr under a hydrogen atmosphere. After filtration, the filtrate was concentrated under reduced pressure, and the obtained residue was purified by basic silica gel column chromatography (developing solvent; ethyl acetate:hexane=7:3→1:0) to give the title compound (132 mg, yield 77%) as an oil.
EI(pos) 523 $[M+H]^+$

Example 202

tert-butyl [3-{[4-(3,3-dimethyl-1-oxo-2-oxa-7-azaspiro[4.5]dec-7-yl)piperidin-1-yl]carbonyl}-5-(4-hydroxyphenyl)-2-thienyl]carbamate

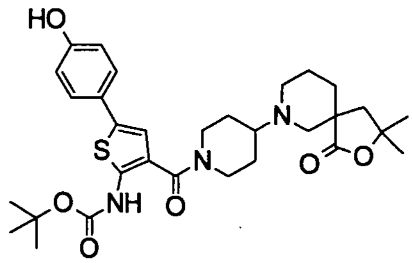

Using tert-butyl (5-bromo-3-{[4-(3,3-dimethyl-1-oxo-2-oxa-7-azaspiro[4.5]dec-7-yl)piperidin-1-yl]carbonyl}-2-thienyl)carbamate (0.50 g, 0.877 mmol) obtained in Example 170 and (4-hydroxyphenyl)boronic acid (484 mg, 3.51 mmol), the title compound (1.02 g, quantitative) was obtained as an oil by an operation similar to that of Reference Example 84. EI(pos) 584 $[M+H]^+$

Example 203

benzyl [4-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)phenoxy]acetate

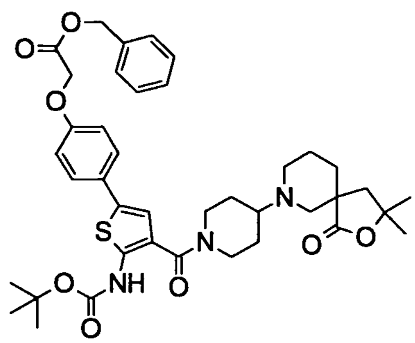

A suspension of tert-butyl [3-{[4-(3,3-dimethyl-1-oxo-2-oxa-7-azaspiro[4.5]dec-7-yl)piperidin-1-yl]carbonyl}-5-(4-hydroxyphenyl)-2-thienyl]carbamate (1.10 g, 1.89 mmol) obtained in Example 202, benzyl bromoacetate (0.45 mL, 2.83 mmol) and potassium carbonate (392 mg, 2.83 mmol) in N,N-dimethylformamide (10 mL) was stirred at room temperature for 1 hr. The reaction mixture was diluted with ethyl acetate, washed 3 times with saturated brine, and dried over anhydrous magnesium sulfate. The solvent was evaporated under reduced pressure, and the obtained residue was purified by basic silica gel column chromatography (developing solvent; ethyl acetate:hexane=3:7→3:2) to give the title compound (0.35 g, yield 25%) as an oil.
EI(pos) 732 $[M+H]^+$

Example 204

benzyl [4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)phenoxy]acetate

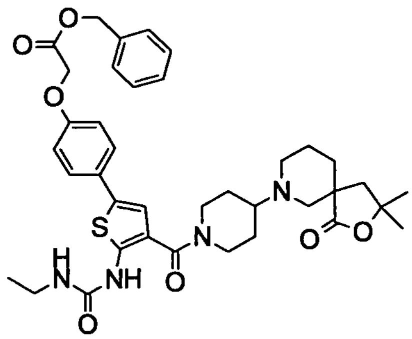

A solution of benzyl [4-(5-[(tert-butoxycarbonyl)amino]-4-{[4-(3,3-dimethyl-1-oxo-2-oxa-7-azaspiro[4.5]dec-7-yl)piperidin-1-yl]carbonyl}-2-thienyl)phenoxy]acetate (0.35 g, 0.478 mmol) obtained in Example 203 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure to give benzyl [4-(5-amino-4-{[4-(3,3-dimethyl-1-oxo-2-oxa-7-azaspiro[4.5]dec-7-yl)piperidin-1-yl]carbonyl}-2-thienyl)phenoxy]acetate (EI(pos) 632 $[M+H]^+$) as a crude product. Using the crude product, the title compound (0.27 g, yield 80%) was obtained as an oil by an operation similar to that of Example 166.
EI(pos) 703 $[M+H]^+$

Example 205

[4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)phenoxy]acetic acid

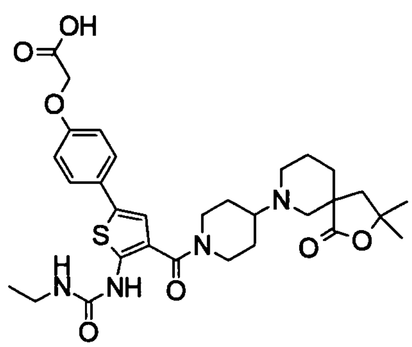

Using benzyl [4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)phenoxy]acetate (0.27 g, 0.384 mmol) obtained in Example 204, the title compound (86 mg, yield 37%) was obtained as a white solid by an operation similar to that of Example 190.
EI(pos) 613 $[M+H]^+$

Example 206

2-{7-[1-({2-[(ethylcarbamoyl)amino]-1-benzothien-3-yl}carbonyl)piperidin-4-yl]-2-methyl-4-oxo-1,3,7-triazaspiro[4.5]dec-1-en-3-yl}ethyl acetate

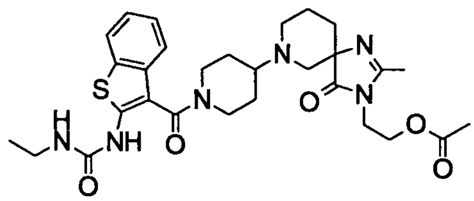

Using tert-butyl 4-[3-(2-acetoxyethyl)-2-methyl-4-oxo-1,3,7-triazaspiro[4.5]dec-1-en-7-yl]piperidine-1-carboxylate (0.94 g, 2.15 mmol) obtained in Reference Example 100, 2-{7-[1-({2-[(tert-butoxycarbonyl)amino]-1-benzothien-3-yl}carbonyl)piperidin-4-yl]-2-methyl-4-oxo-1,3,7-triazaspiro[4.5]dec-1-en-3-yl}ethyl acetate (EI(pos) 612 $[M+H]^+$) was obtained as an oil by an operation similar to that of Example 182. A solution of the obtained oil in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 2-(7-{1-[(2-amino-1-benzothien-3-yl)carbonyl]piperidin-4-yl}-2-methyl-4-oxo-1,3,7-triazaspiro[4.5]dec-1-en-3-yl)ethyl acetate (EI(pos) 512 $[M+H]^+$) as a crude product. Using the crude product, the title compound (0.40 g, yield 32%) was obtained as an oil by an operation similar to that of Example 166.
EI(pos) 583 $[M+H]^+$

Example 207

1-ethyl-3-[3-({4-[3-(2-hydroxyethyl)-2-methyl-4-oxo-1,3,7-triazaspiro[4.5]dec-1-en-7-yl]piperidin-1-yl}carbonyl)-1-benzothien-2-yl]urea

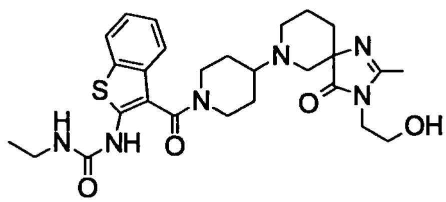

Using 2-{7-[1-({2-[(ethylcarbamoyl)amino]-1-benzothien-3-yl}carbonyl)piperidin-4-yl]-2-methyl-4-oxo-1,3,7-triazaspiro[4.5]dec-1-en-3-yl}ethyl acetate (0.37 g, 0.635 mmol) obtained in Example 206, the title compound (249 mg, yield 73%) was obtained as an oil by an operation similar to that of Example 161.
EI(pos) 541 $[M+H]^+$

Example 208

benzyl 9-{1-[(2-amino-7-oxo-4,5,6,7-tetrahydro-1-benzothien-3-yl)carbonyl]piperidin-4-yl}-3,3-dimethyl-1-oxo-2-oxa-6,9-diazaspiro[4.5]decane-6-carboxylate

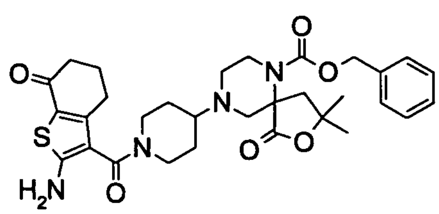

Using benzyl 9-[1-(tert-butoxycarbonyl)piperidin-4-yl]-3,3-dimethyl-1-oxo-2-oxa-6,9-diazaspiro[4.5]decane-6-carboxylate (1.10 g, 2.22 mmol) obtained in Reference Example 90, benzyl 3,3-dimethyl-1-oxo-9-(piperidin-4-yl)-2-oxa-6,9-diazaspiro[4.5]decane-6-carboxylate dihydrochloride (1.05 g, quantitative) was obtained as a white solid by an operation similar to that of Reference Example 53. Using benzyl 3,3-dimethyl-1-oxo-9-(piperidin-4-yl)-2-oxa-6,9-diazaspiro[4.5]decane-6-carboxylate dihydrochloride (0.50 g, 1.06 mmol) and 2-amino-7-oxo-4,5,6,7-tetrahydro-1-benzothiophene-3-carboxylic acid (232 mg, 1.11 mmol) obtained in Reference Example 133, the title compound (0.33 g, yield 52%) was obtained as an oil by an operation similar to that of Example 31.
EI(pos) 595 $[M+H]^+$

Example 209

benzyl 9-[1-({2-[(ethylcarbamoyl)amino]-7-oxo-4,5,6,7-tetrahydro-1-benzothien-3-yl}carbonyl)piperidin-4-yl]-3,3-dimethyl-1-oxo-2-oxa-6,9-diazaspiro[4.5]decane-6-carboxylate

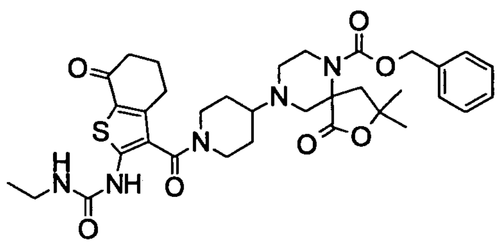

Using benzyl 9-{1-[(2-amino-7-oxo-4,5,6,7-tetrahydro-1-benzothien-3-yl)carbonyl]piperidin-4-yl}-3,3-dimethyl-1-oxo-2-oxa-6,9-diazaspiro[4.5]decane-6-carboxylate (0.33 g, 0.541 mmol) obtained in Example 208, the title compound (0.34 g, yield 94%) was obtained as an oil by an operation similar to that of Example 166.
EI(pos) 666 $[M+H]^+$

Example 210

1-(3-{[4-(3,3-dimethyl-1-oxo-2-oxa-6,9-diazaspiro[4.5]dec-9-yl)piperidin-1-yl]carbonyl}-7-oxo-4,5,6,7-tetrahydro-1-benzothien-2-yl)-3-ethylurea

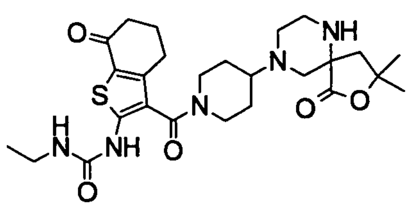

Benzyl 9-[1-({2-[(ethylcarbamoyl)amino]-7-oxo-4,5,6,7-tetrahydro-1-benzothien-3-yl}carbonyl)piperidin-4-yl]-3,3-dimethyl-1-oxo-2-oxa-6,9-diazaspiro[4.5]decane-6-carboxylate (0.34 g, 0.511 mmol) obtained in Example 209 was dissolved in 25% hydrobromide-acetic acid (10 mL), and the mixture was stirred at room temperature for 30 min. The reaction mixture was neutralized with saturated aqueous sodium hydrogencarbonate solution, and extracted 3 times with ethyl acetate-tetrahydrofuran (1:1) mixture. The organic layer was dried over anhydrous magnesium sulfate. The solvent was evaporated under reduced pressure, and the obtained residue was purified by basic silica gel column chromatography (developing solvent; methanol:ethyl acetate=0:1→3:17) to give the title compound (119 mg, yield 43%) as an oil.
EI(pos) 532 $[M+H]^+$

Example 211

7-[1-(9-anthrylcarbonyl)piperidin-4-yl]-3-ethyl-2-methyl-1,3,7-triazaspiro[4.5]dec-1-en-4-one

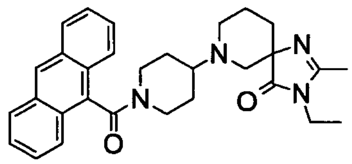

Using 7-[1-(9-anthrylcarbonyl)piperidin-4-yl]-2-methyl-1,3,7-triazaspiro[4.5]dec-1-en-4-one (122 mg, 0.269 mmol) obtained in Example 159 and iodoethane (0.044 mL, 0.538 mmol), the title compound (79.6 mg, yield 61%) was obtained as an oil by an operation similar to that of Reference Example 100. EI(pos) 483 $[M+H]^+$

Example 212

tert-butyl (3-{[4-(3,3-dimethyl-1-oxo-2,6-dioxa-9-azaspiro[4.5]dec-9-yl)piperidin-1-yl]carbonyl}-1-benzothien-2-yl)carbamate

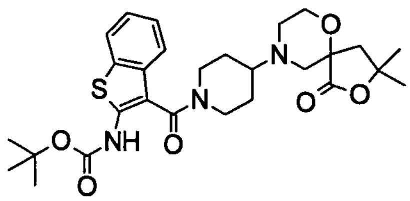

Using tert-butyl 4-(3,3-dimethyl-1-oxo-2,6-dioxa-9-azaspiro[4.5]dec-9-yl)piperidine-1-carboxylate (1.37 g, 3.72 mmol) obtained in Reference Example 101, 3,3-dimethyl-9-(piperidin-4-yl)-2,6-dioxa-9-azaspiro[4.5]decan-1-one dihydrochloride (1.27 g, quantitative) was obtained as a white solid by an operation similar to that of Reference Example 53. Using 3,3-dimethyl-9-(piperidin-4-yl)-2,6-dioxa-9-azaspiro[4.5]decan-1-one dihydrochloride (0.65 g, 1.91 mmol) and 2-[(tert-butoxycarbonyl)amino]-1-benzothiophene-3-carboxylic acid (559 mg, 1.91 mmol) obtained in Reference Example 50, the title compound (0.66 g, yield 64%) was obtained as an oil by an operation similar to that of Example 31.
EI(pos) 544 $[M+H]^+$

Example 213

1-(3-{[4-(3,3-dimethyl-1-oxo-2,6-dioxa-9-azaspiro[4.5]dec-9-yl)piperidin-1-yl]carbonyl}-1-benzothien-2-yl)-3-ethylurea

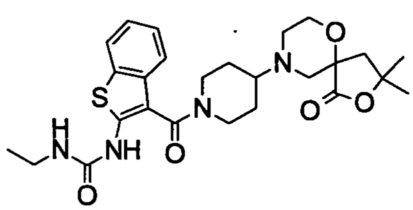

A solution of tert-butyl (3-{[4-(3,3-dimethyl-1-oxo-2,6-dioxa-9-azaspiro[4.5]dec-9-yl)piperidin-1-yl]carbonyl}-1-benzothien-2-yl)carbamate (0.66 g, 1.22 mmol) obtained in Example 212 in trifluoroacetic acid (10 mL) was stirred at room temperature for 30 min. The reaction mixture was neutralized with saturated aqueous sodium hydrogencarbonate solution and extracted twice with ethyl acetate. The organic layer was dried over anhydrous magnesium sulfate and the solvent was evaporated under reduced pressure to give 9-{1-[(2-amino-1-benzothien-3-yl)carbonyl]piperidin-4-yl}-3,3-dimethyl-2,6-dioxa-9-azaspiro[4.5]decan-1-one (EI(pos) 444 $[M+H]^+$) as a crude product. Using the crude product, the title compound (338 mg, yield 54%) was obtained as an oil by an operation similar to that of Example 166.
EI(pos) 515 $[M+H]^+$

Example 214

2-(7-{1-[(2-amino-7-oxo-4,5,6,7-tetrahydro-1-benzothien-3-yl)carbonyl]piperidin-4-yl}-3-methyl-1-oxo-2,7-diazaspiro[4.5]dec-2-yl)ethyl acetate

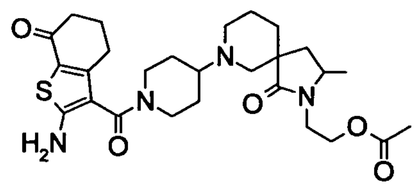

A solution of tert-butyl 4-[2-(2-acetoxyethyl)-3-methyl-1-oxo-2,7-diazaspiro[4.5]dec-7-yl]piperidine-1-carboxylate (935 mg, 2.14 mmol) obtained in Reference Example 91 in 4N hydrogen chloride-ethyl acetate (10 mL) was stirred at room temperature for 30 min. The solvent was evaporated under reduced pressure to give 2-(3-methyl-1-oxo-7-(piperidin-4-yl)-2,7-diazaspiro[4.5]dec-2-yl)ethyl acetate dihydrochloride as an oil. Using the oil and 2-amino-7-oxo-4,5,6,7-tetrahydro-1-benzothiophene-3-carboxylic acid (444 mg, 2.10 mmol) obtained in Reference Example 133, the title compound (166 mg, yield 14%) was obtained as an oil by an operation similar to that of Example 31.
EI(pos) 531 $[M+H]^+$

Example 215

2-{7-[1-({2-[(ethylcarbamoyl)amino]-7-oxo-4,5,6,7-tetrahydro-1-benzothien-3-yl}carbonyl)piperidin-4-yl]-3-methyl-1-oxo-2,7-diazaspiro[4.5]dec-2-yl}ethyl acetate

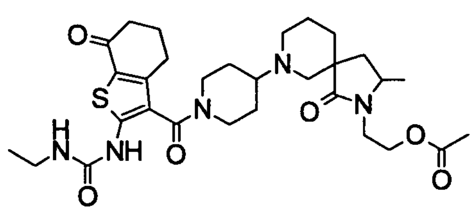

Using 2-(7-{1-[(2-amino-7-oxo-4,5,6,7-tetrahydro-1-benzothien-3-yl)carbonyl]piperidin-4-yl}-3-methyl-1-oxo-2,7-diazaspiro[4.5]dec-2-yl)ethyl acetate (0.16 g, 0.302 mmol) obtained in Example 214, the title compound (0.10 g, yield 55%) was obtained as an oil by an operation similar to that of Example 166.
EI(pos) 602 $[M+H]^+$

Example 216

1-ethyl-3-[3-({4-[2-(2-hydroxyethyl)-3-methyl-1-oxo-2,7-diazaspiro[4.5]dec-7-yl]piperidin-1-yl}carbonyl)-7-oxo-4,5,6,7-tetrahydro-1-benzothien-2-yl]urea

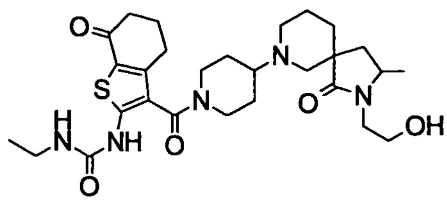

Using 2-{7-[1-({2-[(ethylcarbamoyl)amino]-7-oxo-4,5,6,7-tetrahydro-1-benzothien-3-yl}carbonyl)piperidin-4-yl]-3-methyl-1-oxo-2,7-diazaspiro[4.5]dec-2-yl}ethyl acetate (0.10 g, 0.167 mmol) obtained in Example 215, the title compound (67 mg, yield 72%) was obtained as an oil by an operation similar to that of Example 161.
EI(pos) 560 $[M+H]^+$

Example 217

benzyl 9-[1-({2-[(ethylcarbamoyl)amino]-5-(pyridin-2-yl)-3-thienyl}carbonyl)piperidin-4-yl]-3,3-dimethyl-1-oxo-2-oxa-6,9-diazaspiro[4.5]decane-6-carboxylate

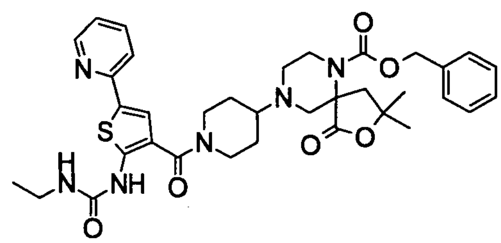

Using benzyl 9-[1-(tert-butoxycarbonyl)piperidin-4-yl]-3,3-dimethyl-1-oxo-2-oxa-6,9-diazaspiro[4.5]decane-6-carboxylate (1.10 g, 2.22 mmol) obtained in Reference Example 90, benzyl 3,3-dimethyl-1-oxo-9-(piperidin-4-yl)-2-oxa-6,9-diazaspiro[4.5]decane-6-carboxylate dihydrochloride (1.05 g, quantitative) was obtained as a white solid by an operation similar to that of Reference Example 53. Using benzyl 3,3-dimethyl-1-oxo-9-(piperidin-4-yl)-2-oxa-6,9-diazaspiro[4.5]decane-6-carboxylate dihydrochloride (0.47 g, 0.990 mmol) and 2-[(tert-butoxycarbonyl)amino]-5-(pyridin-2-yl)thiophene-3-carboxylic acid (0.35 g, 1.09 mmol) obtained in Reference Example 103, benzyl 9-[1-({2-[(tert-butoxycarbonyl)amino]-5-(pyridin-2-yl)-3-thienyl}carbonyl)piperidin-4-yl]-3,3-dimethyl-1-oxo-2-oxa-6,9-diazaspiro[4.5]decane-6-carboxylate (0.70 g, quantitative) was obtained as an oil by an operation similar to that of Example 31. A solution of benzyl 9-[1-({2-[(tert-butoxycarbonyl)amino]-5-(pyridin-2-yl)-3-thienyl}carbonyl)piperidin-4-yl]-3,3-dimethyl-1-oxo-2-oxa-6,9-diazaspiro[4.5]decane-6-carboxylate (0.72 g, 1.03 mmol) in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure to give benzyl 9-{1-[(2-amino-5-(pyridin-2-yl)-3-thienyl)carbonyl]piperidin-4-yl}-3,3-dimethyl-1-oxo-2-oxa-6,9-diazaspiro[4.5]decane-6-carboxylate (EI(pos) 604 $[M+H]^+$) as a crude product. Using the crude product, the title compound (0.39 g, yield 56%) was obtained as an oil by an operation similar to that of Example 166. EI(pos) 675 $[M+H]^+$

Example 218

1-(3-{[4-(3,3-dimethyl-1-oxo-2-oxa-6,9-diazaspiro[4.5]dec-9-yl)piperidin-1-yl]carbonyl}-5-(pyridin-2-yl)-2-thienyl)-3-ethylurea

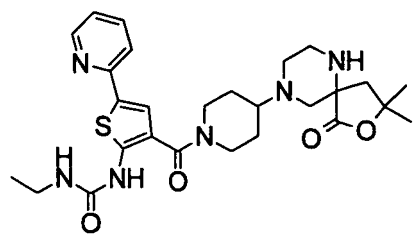

A suspension (10 mL) of benzyl 9-[1-({2-[(ethylcarbamoyl)amino]-5-(pyridin-2-yl)-3-thienyl}carbonyl)piperidin-4-yl]-3,3-dimethyl-1-oxo-2-oxa-6,9-diazaspiro[4.5]decane-6-carboxylate (0.39 g, 0.578 mmol) obtained in Example 217 and 10% palladium carbon (50% water-containing product, 62 mg) in ethanol was stirred for 1 hr under a hydrogen atmosphere. After filtration, the filtrate was concentrated under reduced pressure, and the obtained residue was purified by basic silica gel column chromatography (developing solvent; methanol:ethyl acetate=0:1→1:9) to give the title compound (207 mg, yield 66%) as an oil.
EI(pos) 541 $[M+H]^+$

Example 219

tert-butyl (3-{[4-(1-oxo-2,7-diazaspiro[4.5]dec-7-yl)piperidin-1-yl]carbonyl}-1-benzothien-2-yl)carbamate

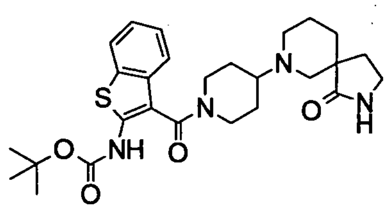

Using tert-butyl 4-(1-oxo-2,7-diazaspiro[4.5]dec-7-yl)piperidine-1-carboxylate (0.78 g, 2.32 mmol) obtained in Reference Example 104, the title compound (0.71 g, yield 60%) was obtained as an oil by an operation similar to that of Example 182.
EI(pos) 513 $[M+H]^+$

Example 220

1-ethyl-3-(3-{[4-(1-oxo-2,7-diazaspiro[4.5]dec-7-yl)piperidin-1-yl]carbonyl}-1-benzothien-2-yl)urea

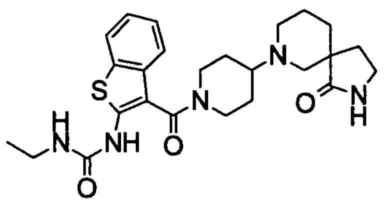

A solution of tert-butyl (3-{[4-(1-oxo-2,7-diazaspiro[4.5]dec-7-yl)piperidin-1-yl]carbonyl}-1-benzothien-2-yl)carbamate (0.71 g, 1.39 mmol) obtained in Example 219 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure to give 7-{1-[(2-amino-1-benzothien-3-yl)carbonyl]piperidin-4-yl}-2,7-diazaspiro[4.5]decan-1-one (EI(pos) 413 $[M+H]^+$) as a crude product. Using the crude product, the title compound (202 mg, yield 30%) was obtained as an oil by an operation similar to that of Example 166.
EI(pos) 484 $[M+H]^+$

Example 221

tert-butyl (3-{[4-(3,3-dimethyl-1-oxo-2-oxa-7-azaspiro[4.5]dec-7-yl)piperidin-1-yl]carbonyl}-5-(pyridin-3-yl)-2-thienyl) carbamate

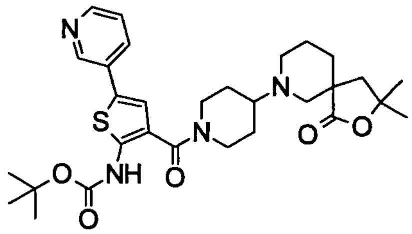

Using tert-butyl (5-bromo-3-{[4-(3,3-dimethyl-1-oxo-2-oxa-7-azaspiro[4.5]dec-7-yl)piperidin-1-yl]carbonyl}-2-thienyl)carbamate (0.89 g, 1.56 mmol) obtained in Example 170 and 3-pyridineboronic acid (242 mg, 1.97 mmol), the title compound (0.47 g, yield 84%) was obtained as an oil by an operation similar to that of Reference Example 84.
EI(pos) 569 $[M+H]^+$

Example 222

1-(3-{[4-(3,3-dimethyl-1-oxo-2-oxa-7-azaspiro[4.5]dec-7-yl)piperidin-1-yl]carbonyl}-5-(pyridin-3-yl)-2-thienyl)-3-ethylurea

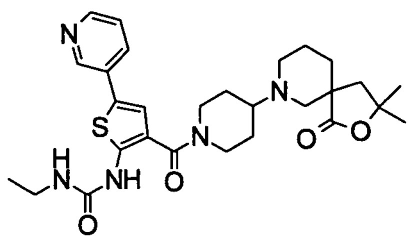

A solution of tert-butyl (3-{[4-(3,3-dimethyl-1-oxo-2-oxa-7-azaspiro[4.5]dec-7-yl)piperidin-1-yl]carbonyl}-5-(pyridin-3-yl)-2-thienyl)carbamate (0.47 g, 0.827 mmol) obtained in Example 221 in trifluoroacetic acid (10 mL) was stirred

at room temperature for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure to give 7-{1-[(2-amino-5-(pyridin-3-yl)-3-thienyl)carbonyl]piperidin-4-yl}-3,3-dimethyl-2-oxa-7-azaspiro[4.5]decan-1-one (EI(pos) 469 [M+H]$^+$) as a crude product. Using the crude product, the title compound (265 mg, yield 59%) was obtained as a white solid by an operation similar to that of Example 166.
EI(pos) 540 [M+H]$^+$

Example 223

tert-butyl [2-(3-{[4-(3,3-dimethyl-l-oxo-2-oxa-7-azaspiro[4.5]dec-7-yl)piperidin-1-yl]carbonyl}-5-phenyl-2-thienyl)ethyl] carbamate

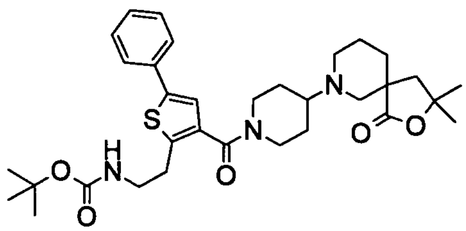

Using 3,3-dimethyl-7-(piperidin-4-yl)-2-oxa-7-azaspiro[4.5]decan-1-one dihydrochloride (1.40 g, 4.13 mmol) obtained in Reference Example 53 and 2-{2-[(tert-butoxycarbonyl)amino]ethyl}-5-phenylthiophene-3-carboxylic acid (1.61 g, 4.63 mmol) obtained in Reference Example 109, the title compound (1.79 g, yield 73%) was obtained as an oil by an operation similar to that of Example 31.
EI(pos) 596 [M+H]$^+$

Example 224

7-(1-{[2-(2-aminoethyl)-5-phenyl-3-thienyl]carbonyl}piperidin-4-yl)-3,3-dimethyl-2-oxa-7-azaspiro[4.5]decan-1-one dihydrochloride

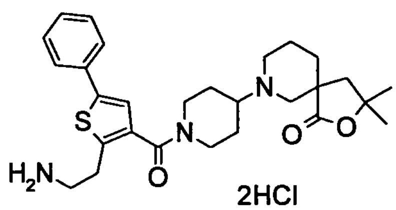

Using tert-butyl [2-(3-{[4-(3,3-dimethyl-1-oxo-2-oxa-7-azaspiro[4.5]dec-7-yl)piperidin-1-yl]carbonyl}-5-phenyl-2-thienyl)ethyl]carbamate (215 mg, 0.361 mmol) obtained in Example 223, the title compound (194 mg, yield 95%) was obtained as a white solid by an operation similar to that of Reference Example 53.
EI(pos) 496 [M+H]$^+$

Example 225

N-[2-(3-{[4-(3,3-dimethyl-1-oxo-2-oxa-7-azaspiro[4.5]dec-7-yl)piperidin-1-yl]carbonyl}-5-phenyl-2-thienyl)ethyl]acetamide

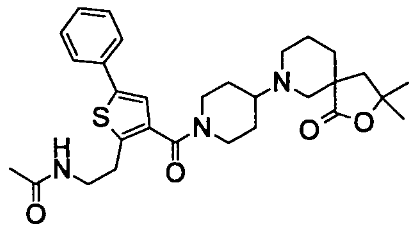

Using tert-butyl [2-(3-{[4-(3,3-dimethyl-1-oxo-2-oxa-7-azaspiro[4.5]dec-7-yl)piperidin-1-yl]carbonyl}-5-phenyl-2-thienyl)ethyl]carbamate (0.50 g, 0.839 mmol) obtained in Example 223, 7-(1-{[2-(2-aminoethyl)-5-phenyl-3-thienyl] carbonyl}piperidin-4-yl)-3,3-dimethyl-2-oxa-7-azaspiro[4.5]decan-1-one dihydrochloride was obtained as a white solid by an operation similar to that of Reference Example 53. The white solid was dissolved in acetic anhydride (5 mL) and pyridine (5 mL), and the mixture was stirred at room temperature for 30 min. The solvent was evaporated under reduced pressure, and the obtained residue was purified by basic silica gel column chromatography (developing solvent; ethyl acetate:hexane=3:2→1:0) to give the title compound (224 mg, yield 50%) as an oil.
EI(pos) 538 $[M+H]^+$

Example 226

N-[2-(3-{[4-(3,3-dimethyl-1-oxo-2-oxa-7-azaspiro[4.5]dec-7-yl)piperidin-1-yl]carbonyl}-5-phenyl-2-thienyl)ethyl]methanesulfonamide

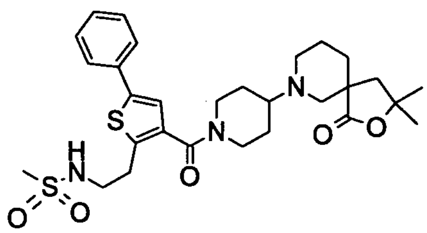

Using tert-butyl [2-(3-{[4-(3,3-dimethyl-1-oxo-2-oxa-7-azaspiro[4.5]dec-7-yl)piperidin-1-yl]carbonyl}-5-phenyl-2-thienyl)ethyl]carbamate (0.42 g, 0.705 mmol) obtained in Example 223, 7-(1-{[2-(2-aminoethyl)-5-phenyl-3-thienyl] carbonyl}piperidin-4-yl)-3,3-dimethyl-2-oxa-7-azaspiro[4.5]decan-1-one dihydrochloride was obtained as a white solid by an operation similar to that of Reference Example 53. To a solution of the white solid and triethylamine (0.39 mL, 2.82 mmol) in tetrahydrofuran (10 mL) was added methanesulfonyl chloride (0.11 mL, 1.41 mmol), and the mixture was stirred at room temperature for 30 min. The reaction mixture was diluted with ethyl acetate, washed with saturated aqueous sodium hydrogencarbonate solution, and dried over anhydrous magnesium sulfate. The solvent was evaporated under reduced pressure, and the obtained residue was purified by basic silica gel column chromatography (developing solvent; ethyl acetate:hexane=7:3→1:0) to give the title compound (247 mg, yield 61%) as an oil.
EI(pos) 574 $[M+H]^+$

Example 227

tert-butyl (3-{[4-(3-ethyl-2,4-dioxo-1-oxa-3,7-diazaspiro[4.5]dec-7-yl)piperidin-1-yl]carbonyl}-1-benzothien-2-yl)carbamate

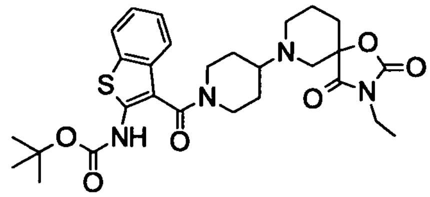

Using 3-ethyl-7-(piperidin-4-yl)-1-oxa-3,7-diazaspiro[4.5]decane-2,4-dione dihydrochloride (1.00 g, 2.83 mmol) obtained in Reference Example 111 and 2-[(tert-butoxycarbonyl)amino]-1-benzothiophene-3-carboxylic acid (0.91 g, 3.11 mmol) obtained in Reference Example 50, the title compound (0.98 g, yield 62%) was obtained as an oil by an operation similar to that of Example 31.
EI(pos) 557 $[M+H]^+$

Example 228

1-ethyl-3-(3-{[4-(3-ethyl-2,4-dioxo-1-oxa-3,7-diazaspiro[4.5]dec-7-yl)piperidin-1-yl]carbonyl}-1-benzothien-2-yl)urea

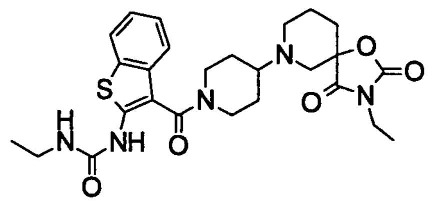

A solution of tert-butyl (3-{[4-(3-ethyl-2,4-dioxo-1-oxa-3,7-diazaspiro[4.5]dec-7-yl)piperidin-1-yl]carbonyl}-1-benzothien-2-yl)carbamate (0.98 g, 1.76 mmol) obtained in Example 227 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 7-{1-[(2-amino-1-benzothien-3-yl)carbonyl]piperidin-4-yl}-3-ethyl-1-oxa-3,7-diazaspiro[4.5]decane-2,4-dione (EI(pos) 457 $[M+H]^+$) as a crude product. Using a half of the crude product (402 mg, 0.880 mmol), the title compound (301 mg, yield 65%) was obtained as an oil by an operation similar to that of Example 166.
EI(pos) 528 $[M+H]^+$

Example 229

1-(3-{[4-(3-ethyl-2,4-dioxo-1-oxa-3,7-diazaspiro[4.5]dec-7-yl)piperidin-1-yl]carbonyl}-1-benzothien-2-yl)urea

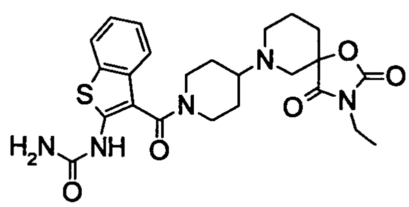

A solution of tert-butyl (3-{[4-(3-ethyl-2,4-dioxo-1-oxa-3,7-diazaspiro[4.5]dec-7-yl)piperidin-1-yl]carbonyl}-1-benzothien-2-yl)carbamate (0.98 g, 1.76 mmol) obtained in Example 227 in trifluoroacetic acid (10 mL) was stirred at room temperature for 30 min. The reaction mixture was neutralized with saturated aqueous sodium hydrogencarbonate

solution, and extracted twice with ethyl acetate. The organic layer was dried over anhydrous magnesium sulfate and the solvent was evaporated under reduced pressure to give 7-{1-[(2-amino-1-benzothien-3-yl)carbonyl]piperidin-4-yl}-3-ethyl-1-oxa-3,7-diazaspiro[4.5]decane-2,4-dione (EI(pos) 457 $[M+H]^+$) as a crude product. To a solution of a half of the crude product (402 mg, 0.880 mmol) in tetrahydrofuran (10 mL) was added trichloroacetyl isocyanate (0.21 mL, 1.76 mmol) at 0˚C, and the mixture was stirred at room temperature for 10 min. To the solution was added 7M ammonia-methanol solution (3 mL), and the mixture was stirred at room temperature for 2 hr. The solvent was evaporated under reduced pressure, and the obtained residue was purified by basic silica gel column chromatography (developing solvent; methanol:ethyl acetate=0:1 →1:9) to give the title compound (346 mg, yield 79%) as an oil. EI(pos) 500 $[M+H]^+$

Example 230

tert-butyl (3-{[4-(3-ethyl-2,4-dioxo-1-oxa-3,7-diazaspiro[4.5]dec-7-yl)piperidin-1-yl]carbonyl}-5-phenyl-2-thienyl)carbamate

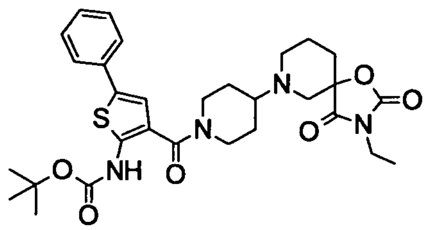

Using 3-ethyl-7-(piperidin-4-yl)-1-oxa-3,7-diazaspiro[4.5]decane-2,4-dione dihydrochloride (0.50 g, 1.41 mmol) obtained in Reference Example 111 and 2-[(tert-butoxycarbonyl)amino]-5-phenylthiophene-3-carboxylic acid (0.54 g, 1.69 mmol) obtained in Reference Example 76, the title compound (0.52 g, yield 63%) was obtained as an oil by an operation similar to that of Example 31.
EI(pos) 583 $[M+H]^+$

Example 231

1-(3-{[4-(3-ethyl-2,4-dioxo-1-oxa-3,7-diazaspiro[4.5]dec-7-yl)piperidin-1-yl]carbonyl}-5-phenyl-2-thienyl)urea

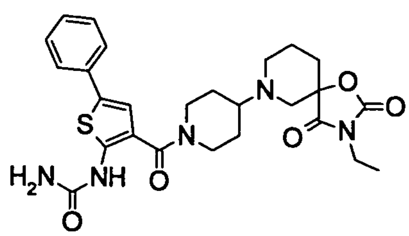

A solution of tert-butyl (3-{[4-(3-ethyl-2,4-dioxo-1-oxa-3,7-diazaspiro[4.5]dec-7-yl)piperidin-1-yl]carbonyl}-5-phenyl-2-thienyl)carbamate (0.52 g, 0.894 mmol) obtained in Example 230 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure to give 7-{1-[(2-amino-5-phenyl-3-thienyl)carbonyl]piperidin-4-yl}-3-ethyl-1-oxa-3,7-diazaspiro[4.5]decane-2,4-dione (EI(pos) 483 $[M+H]^+$) as a crude product. To a solution of the crude product in tetrahydrofuran (10 mL) was added trichloroacetyl isocyanate (0.22 mL, 1.79 mmol) at 0˚C, and the mixture was stirred at room temperature for 10 min. To the solution was added 7M ammonia-methanol solution (3 mL), and the mixture was stirred at room temperature for 2 hr. The solvent was evaporated under reduced pressure, and the obtained residue was purified by basic silica gel column chromatography (developing solvent; methanol:ethyl acetate=0:16→1:9) to give the title compound (25.3 mg, yield 5.4%) as an oil.

EI(pos) 526 [M+H]+

Example 232

tert-butyl (3-{[4-(3-ethyl-2-methyl-4-oxo-1,3,7-triazaspiro[4.5]dec-1-en-7-yl)piperidin-1-yl]carbonyl}-1-benzothien-2-yl) carbamate

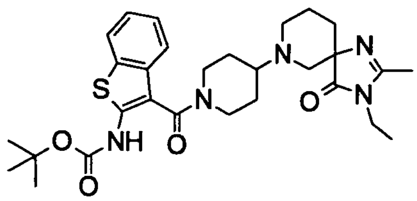

Using tert-butyl 4-(3-ethyl-2-methyl-4-oxo-1,3,7-triazaspiro[4.5]dec-1-en-7-yl)piperidine-1-carboxylate (1.13 g, 2.99 mmol) obtained in Reference Example 112, the title compound (0.89 g, yield 53%) was obtained as an oil by an operation similar to that of Example 182.
EI(pos) 554 [M+H]+

Example 233

1-ethyl-3-(3-{[4-(3-ethyl-2-methyl-4-oxo-1,3,7-triazaspiro[4.5]dec-1-en-7-yl)piperidin-1-yl]carbonyl}-1-benzothien-2-yl) urea

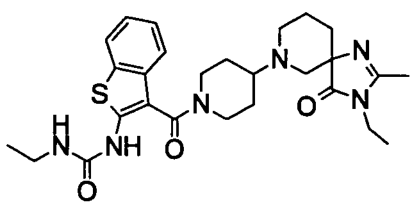

A solution (10 mL) of tert-butyl (3-{[4-(3-ethyl-2-methyl-4-oxo-1,3,7-triazaspiro[4.5]dec-1-en-7-yl)piperidin-1-yl]carbonyl}-1-benzothien-2-yl)carbamate (0.89 g, 1.61 mmol) obtained in Example 232 in trifluoroacetic acid was stirred at room temperature for 30 min. The reaction mixture was neutralized with saturated aqueous sodium hydrogencarbonate solution and extracted twice with ethyl acetate. The organic layer was dried over anhydrous magnesium sulfate and the solvent was evaporated under reduced pressure to give 7-{1-[(2-amino-1-benzothien-3-yl)carbonyl]piperidin-4-yl}-3-ethyl-2-methyl-1,3,7-triazaspiro[4.5]dec-1-en-4-one (EI(pos) 454 [M+H]+) as a crude product. Using a half of the crude product (365 mg, 0.805 mmol), the title compound (257 mg, yield 61%) was obtained as an oil by an operation similar to that of Example 166.
EI(pos) 525 [M+H]+

Example 234

1-(3-{[4-(3-ethyl-2-methyl-4-oxo-1,3,7-triazaspiro[4.5]dec-1-en-7-yl)piperidin-1-yl]carbonyl}-1-benzothien-2-yl)urea

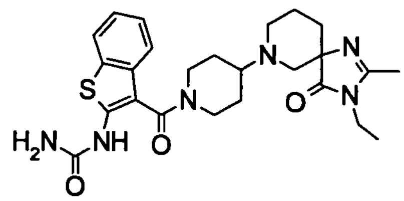

A solution (10 mL) of tert-butyl (3-{[4-(3-ethyl-2-methyl-4-oxo-1,3,7-triazaspiro[4.5]dec-1-en-7-yl)piperidin-1-yl]carbonyl}-1-benzothien-2-yl)carbamate (0.89 g, 1.61 mmol) obtained in Example 232 in trifluoroacetic acid was stirred at room temperature for 30 min. The reaction mixture was neutralized with saturated aqueous sodium hydrogencarbonate solution and extracted twice with ethyl acetate. The organic layer was dried over anhydrous magnesium sulfate and the solvent was evaporated under reduced pressure to give 7-{1-[(2-amino-1-benzothien-3-yl)carbonyl]piperidin-4-yl}-3-ethyl-2-methyl-1,3,7-triazaspiro[4.5]dec-1-en-4-one (EI(pos) 454 $[M+H]^+$) as a crude product. To a solution of a half of the crude product (365 mg, 0.805 mmol) in tetrahydrofuran (10 mL) was added trichloroacetyl isocyanate (0.192 mL, 1.61 mmol) at 0°C, and the mixture was stirred at room temperature for 10 min. To the solution was added 7M ammonia-methanol solution (3 mL), and the mixture was stirred at room temperature for 2 hr. The solvent was evaporated under reduced pressure, and the obtained residue was purified by basic silica gel column chromatography (developing solvent; methanol:ethyl acetate=0:1 →1:9) to give the title compound (399 mg, yield 99%) as an oil. EI(pos) 497 $[M+H]^+$

Example 235

tert-butyl (3-{[4-(2-ethyl-1-oxo-2,7-diazaspiro[4.5]dec-7-yl)piperidin-1-yl]carbonyl}-1-benzothien-2-yl)carbamate

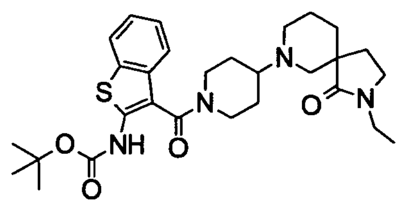

Using tert-butyl 4-(2-ethyl-1-oxo-2,7-diazaspiro[4.5]dec-7-yl)piperidine-1-carboxylate (0.63 g, 1.73 mmol) obtained in Reference Example 4, the title compound (0.62 g, yield 66%) was obtained as an oil by an operation similar to that of Example 182.
EI(pos) 541 $[M+H]^+$

Example 236

1-(3-{[4-(2-ethyl-1-oxo-2,7-diazaspiro[4.5]dec-7-yl)piperidin-1-yl]carbonyl}-1-benzothien-2-yl)urea

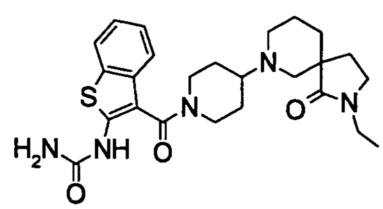

A solution of tert-butyl (3-{[4-(2-ethyl-1-oxo-2,7-diazaspiro[4.5]dec-7-yl)piperidin-1-yl]carbonyl}-1-benzothien-2-yl)carbamate (0.62 g, 1.15 mmol) obtained in Example 235 in trifluoroacetic acid (10 mL) was stirred at room temperature

for 30 min. The reaction mixture was neutralized with saturated aqueous sodium hydrogencarbonate solution, and extracted twice with ethyl acetate. The organic layer was dried over anhydrous magnesium sulfate and the solvent was evaporated under reduced pressure to give 7-{1-[(2-amino-1-benzothien-3-yl)carbonyl]piperidin-4-yl}-2-ethyl-2,7-diazaspiro[4.5]decan-1-one as a crude product. Using the crude product, the title compound (445 mg, yield 80%) was obtained as an oil by an operation similar to that of Example 231. EI(pos) 484 $[M+H]^+$

Example 237

3,3-dimethyl-7-{1-[(5-phenyl-2-(pyridin-3-yl)-3-thienyl)carbonyl]piperidin-4-yl}-2-oxa-7-azaspiro[4.5]decan-1-one

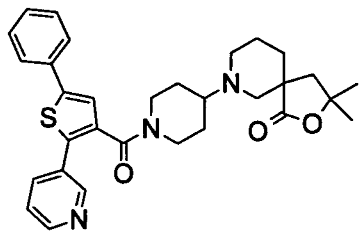

Using 3,3-dimethyl-7-(piperidin-4-yl)-2-oxa-7-azaspiro[4.5]decan-1-one dihydrochloride (0.50 g, 1.47 mmol) obtained in Reference Example 53 and 5-phenyl-2-(pyridin-3-yl)thiophene-3-carboxylic acid (0.40 g, 1.42 mmol) obtained in Reference Example 114, the title compound (109 mg, yield 14%) was obtained as an oil by an operation similar to that of Example 31.
EI(pos) 530 $[M+H]^+$

Example 238

tert-butyl (3-{[4-(3,3-dimethyl-1-oxo-2,6-dioxa-9-azaspiro[4.5]dec-9-yl)piperidin-1-yl]carbonyl}-5-phenyl-2-thienyl)carbamate

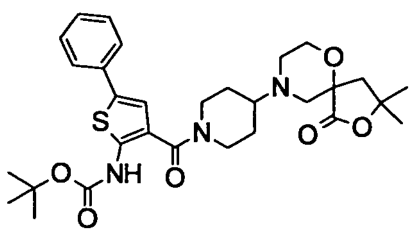

Using 3,3-dimethyl-9-(piperidin-4-yl)-2,6-dioxa-9-azaspiro[4.5]decan-1-one dihydrochloride (0.71 g, 2.08 mmol) obtained in Example 212 and 2-[(tert-butoxycarbonyl)amino]-5-phenylthiophene-3-carboxylic acid (665 mg, 2.08 mmol) obtained in Reference Example 76, the title compound (0.81 g, yield 68%) was obtained as an oil by an operation similar to that of Example 31.
EI(pos) 570 $[M+H]^+$

Example 239

1-(3-{[4-(3,3-dimethyl-1-oxo-2,6-dioxa-9-azaspiro[4.5]dec-9-yl)piperidin-1-yl]carbonyl}-5-phenyl-2-thienyl)urea

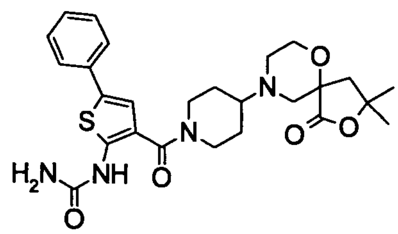

A solution of tert-butyl (3-{[4-(3,3-dimethyl-1-oxo-2,6-dioxa-9-azaspiro[4.5]dec-9-yl)piperidin-1-yl]carbonyl}-5-phenyl-2-thienyl)carbamate (0.81 g, 1.43 mmol) obtained in Example 238 in trifluoroacetic acid (10 mL) was stirred at room temperature for 30 min. The reaction mixture was neutralized with saturated aqueous sodium hydrogencarbonate solution and extracted twice with ethyl acetate. The organic layer was dried over anhydrous magnesium sulfate and the solvent was evaporated under reduced pressure to give 9-{1-[(2-amino-5-phenyl-3-thienyl)carbonyl]piperidin-4-yl}-3,3-dimethyl-2,6-dioxa-9-azaspiro[4.5]decan-1-one as a crude product. To a solution of the crude product in tetrahydrofuran (10 mL) was added trichloroacetyl isocyanate (0.34 mL, 2.85 mmol) at 0˚C, and the mixture was stirred at room temperature for 10 min. To the solution was added 7M ammonia-methanol solution (3 mL), and the mixture was stirred at room temperature for 2 hr. The solvent was evaporated under reduced pressure, and the obtained residue was purified by basic silica gel column chromatography (developing solvent; methanol:ethyl acetate=0:1→1:9) to give the title compound (298 mg, yield 41%) as a white solid.
EI(pos) 512 $[M+H]^+$

<u>Example 240</u>

1-(3-{[4-(2-ethyl-1,3-dioxo-2,7-diazaspiro[4.5]dec-7-yl)piperidin-1-yl]carbonyl}-1-benzothien-2-yl)urea

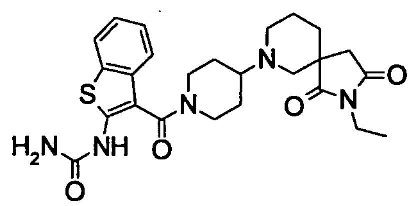

Using ethyl 1'-{[2-(carbamoylamino)-1-benzothien-3-yl]carbonyl}-3-[2-(ethylamino)-2-oxoethyl]-1,4'-bipiperidine-3-carboxylate (0.27 g, 0.497 mmol) obtained in Reference Example 117, the title compound (134 mg, yield 54%) was obtained as an oil by an operation similar to that of Example 20.
EI(pos) 498 $[M+H]^+$

<u>Example 241</u>

7-{1-[(2-iodo-5-phenyl-3-thienyl)carbonyl]piperidin-4-yl}-3,3-dimethyl-2-oxa-7-azaspiro[4.5]decan-1-one

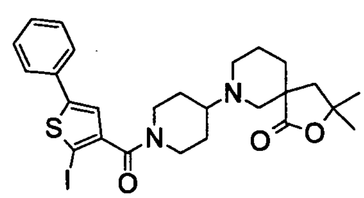

Using 3,3-dimethyl-7-(piperidin-4-yl)-2-oxa-7-azaspiro[4.5]decan-1-one dihydrochloride (944 mg, 2.78 mmol)

obtained in Reference Example 53 and 2-iodo-5-phenylthiophene-3-carboxylic acid (1.01 g, 2.78 mmol) obtained in Reference Example 118, the title compound (1.21 g, yield 68%) was obtained as an oil by an operation similar to that of Example 31.
EI(pos) 579 [M+H]$^+$

Example 242

3,3-dimethyl-7-(1-{[5-phenyl-2-(1H-pyrrol-2-yl)-3-thienyl]carbonyl}piperidin-4-yl)-2-oxa-7-azaspiro[4.5]decan-1-one

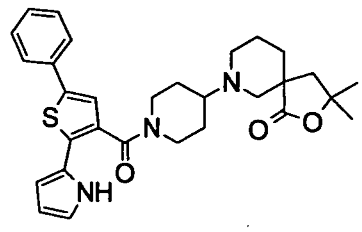

Using 7-{1-[(2-iodo-5-phenyl-3-thienyl)carbonyl]piperidin-4-yl}-3,3-dimethyl-2-oxa-7-azaspiro[4.5]decan-1-one (0.81 g, 1.40 mmol) obtained in Example 241 and 1-(tert-butoxycarbonyl)-1H-pyrrol-2-yl-2-boronic acid (591 mg, 2.80 mmol), a mixture (0.93 g) of tert-butyl 2-(3-{[4-(3,3-dimethyl-1-oxo-2-oxa-7-azaspiro[4.5]dec-7-yl)piperidin-1-yl]carbonyl}-5-phenyl-2-thienyl)-1H-pyrrole-1-carboxylate and 3,3-dimethyl-7-(1-{[5-phenyl-2-(1H-pyrrol-2-yl)-3-thienyl]carbonyl}piperidin-4-yl)-2-oxa-7-azaspiro[4.5]decan-1-one at 3:2 was obtained as an oil by an operation similar to that of Reference Example 84. A solution of this mixture in trifluoroacetic acid (5 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate. The solvent was evaporated under reduced pressure, and the obtained residue was purified by basic silica gel column chromatography (developing solvent; ethyl acetate:hexane=1:1→1:0) to give the title compound (553 mg, yield 71%) as an oil.
EI(pos) 518 [M+H]$^+$

Example 243

di-tert-butyl {(Z)-[(3-{[4-(3,3-dimethyl-1-oxo-2-oxa-7-azaspiro[4.5]dec-7-yl)piperidin-1-yl]carbonyl}-5-phenyl-2-thienyl)amino]methylidene}biscarbamate

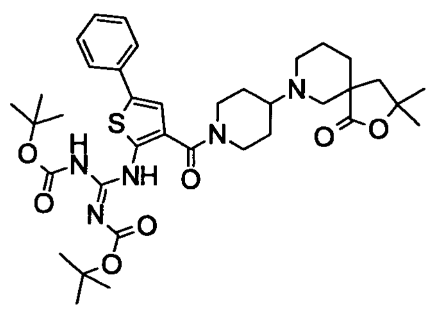

A solution of tert-butyl (3-{[4-(3,3-dimethyl-1-oxo-2-oxa-7-azaspiro[4.5]dec-7-yl)piperidin-1-yl]carbonyl}-5-phenyl-2-thienyl)carbamate (0.42 g, 0.740 mmol) obtained in Example 150 in trifluoroacetic acid (5 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 7-{1-[(2-amino-5-phenyl-3-thienyl)carbonyl]piperidin-4-yl}-3,3-dimethyl-2-oxa-7-azaspiro[4.5]decan-1-one as a crude product. A solution of the crude product, 1,3-bis(tert-butoxycarbonyl)-2-methyl-2-thiopseudourea (0.43 g, 1.48 mmol) and silver nitrate (252 mg, 1.48 mmol) in acetonitrile (10 mL)

was stirred at room temperature for 12 hr. After filtration, the filtrate was concentrated under reduced pressure, and the obtained residue was purified by basic silica gel column chromatography (developing solvent; ethyl acetate:hexane=1:1→1:0) to give the title compound (138 mg, yield 26%) as a white solid.
EI(pos) 710 $[M+H]^+$

Example 244

tert-butyl (3-{[4-(3-ethyl-2,4-dioxo-1-oxa-3,7-diazaspiro[4.5]dec-7-yl)piperidin-1-yl]carbonyl}-5-(pyridin-2-yl)-2-thienyl) carbamate

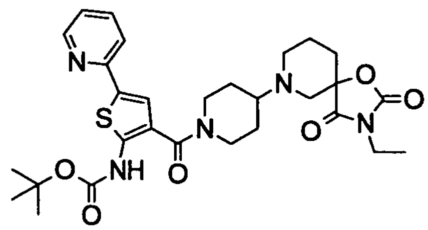

Using 3-ethyl-7-(piperidin-4-yl)-1-oxa-3,7-diazaspiro[4.5]decane-2,4-dione dihydrochloride (444 mg, 1.25 mmol) obtained in Reference Example 111 and 2-[(tert-butoxycarbonyl)amino]-5-(pyridin-2-yl)thiophene-3-carboxylic acid (402 mg, 1.25 mmol) obtained in Reference Example 103, the title compound (0.63 g, yield 86%) was obtained as an oil by an operation similar to that of Example 31.
EI(pos) 584 $[M+H]^+$

Example 245

1-ethyl-3-(3-{[4-(3-ethyl-2,4-dioxo-1-oxa-3,7-diazaspiro[4.5]dec-7-yl)piperidin-1-yl]carbonyl}-5-(pyridin-2-yl)-2-thienyl) urea

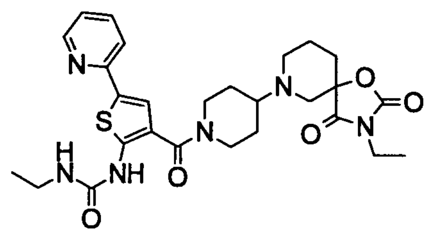

A solution of tert-butyl (3-{[4-(3-ethyl-2,4-dioxo-1-oxa-3,7-diazaspiro[4.5]dec-7-yl)piperidin-1-yl]carbonyl}-5-(pyridin-2-yl)-2-thienyl)carbamate (0.63 g, 1.08 mmol) obtained in Example 244 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 7-{1-[(2-amino-5-(pyridin-2-yl)-3-thienyl)carbonyl]piperidin-4-yl}-3-ethyl-1-oxa-3,7-diazaspiro[4.5]decane-2,4-dione as a crude product. Using the crude product, the title compound (456 mg, yield 76%) was obtained as an oil by an operation similar to that of Example 166.
EI(pos) 555 $[M+H]^+$

Example 246

1'-[1-(9-anthrylcarbonyl)piperidin-4-yl]-8,9-dihydrospiro[benzo[7]annulene-6,3'-piperidin]-5(7H)-one

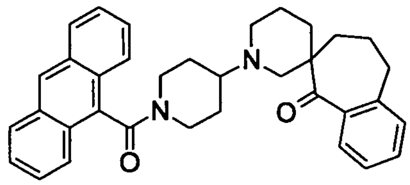

Using 8,9-dihydrospiro[benzo[7]annulene-6,3'-piperidin]-5(7H)-one(674 mg, 2.94 mmol) obtained in Reference Example 122 and 1-(9-anthrylcarbonyl)piperidin-4-one (892 mg, 2.94 mmol) obtained in Reference Example 8, the title compound (1.02 g, yield 67%) was obtained as an oil by an operation similar to that of Example 28.
EI(pos) 517 $[M+H]^+$

Example 247

tert-butyl 3-{[4-(3,3-dimethyl-1-oxo-2-oxa-7-azaspiro[4.5]dec-7-yl)piperidin-1-yl]carbonyl}-2-{[(ethylamino)carbonyl] amino}-4,7-dihydrothieno[2,3-c]pyridine-6(5H)-carboxylate

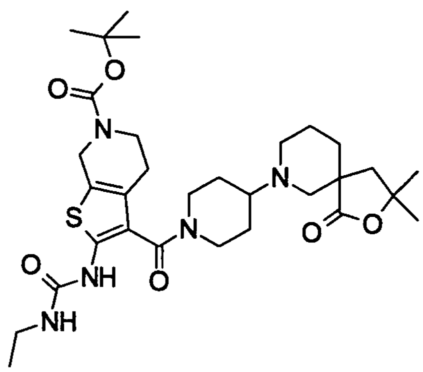

Using 2-amino-6-(tert-butoxycarbonyl)-4,5,6,7-tetrahydrothieno[2,3-c]pyridine-3-carboxylic acid (967 mg, 15.3 mmol) obtained in Reference Example 123 and 3,3-dimethyl-7-(piperidin-4-yl)-2-oxa-7-azaspiro[4.5]decan-1-one dihydrochloride (1.00 g, 2.95 mmol) obtained in Reference Example 53, tert-butyl 2-amino-3-{[4-(3,3-dimethyl-1-oxo-2-oxa-7-azaspiro[4.5]dec-7-yl)piperidin-1-yl]carbonyl}-4,7-dihydrothieno[2,3-c]pyridine-6(5H)-carboxylate (1.06 g, yield 66%) was obtained as an oil by an operation similar to that of Example 31. Since this compound was somewhat unstable, the next reaction was successively performed. That is, using the obtained amine form (1.05 g, 1.92 mmol) and ethyl isocyanate (0.228 mL, 2.88 mmol), the title compound (638 mg, yield 54%) was obtained as an oil by an operation similar to that of Example 166.
EI(pos) 618 $[M+H]^+$

Example 248

N-(3-{[4-(3,3-dimethyl-1-oxo-2-oxa-7-azaspiro[4.5]dec-7-yl)piperidin-1-yl]carbonyl}-4,5,6,7-tetrahydrothieno[2,3-c]pyridin-2-yl)-N'-ethylurea

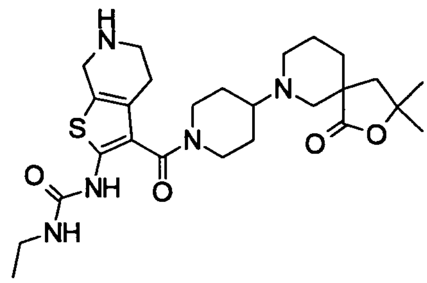

Using tert-butyl 3-{[4-(3,3-dimethyl-1-oxo-2-oxa-7-azaspiro[4.5]dec-7-yl)piperidin-1-yl]carbonyl}-2-{[(ethylamino)carbonyl]amino}-4,7-dihydrothieno[2,3-c]pyridine-6(5H)-carboxylate (525 mg, 0.850 mmol) obtained in Example 247, the title compound (389 mg, yield 88%) was obtained as an oil by an operation similar to that of Reference Example 49. EI(pos) 518 $[M+H]^+$

Example 249

N-(6-acetyl-3-{[4-(3,3-dimethyl-1-oxo-2-oxa-7-azaspiro[4.5]dec-7-yl)piperidin-1-yl]carbonyl}-4,5,6,7-tetrahydrothieno [2,3-c]pyridin-2-yl)-N'-ethylurea

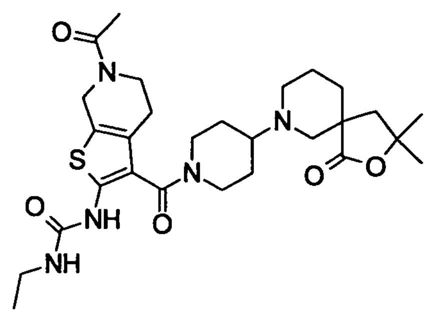

N-(3-{[4-(3,3-dimethyl-1-oxo-2-oxa-7-azaspiro[4.5]dec-7-yl)piperidin-1-yl]carbonyl}-4,5,6,7-tetrahydrothieno [2,3-c]pyridin-2-yl)-N'-ethylurea (150 mg, 0.290 mmol) obtained in Example 248 was dissolved in pyridine (2 mL), and acetic anhydride (0.0548 mL, 0.579 mmol) was added. The mixture was stirred at room temperature for 30 min, and the solvent was evaporated under reduced pressure. The obtained residue was purified by basic silica gel column chromatography (developing solvent; hexane:ethyl acetate=1:1→0:1) and triturated with diisopropyl ether to give the title compound (115 mg, yield 71%).
EI(pos) 560 $[M+H]^+$

Example 250

7-{1-[(2-amino-5-bromopyridin-3-yl)carbonyl]piperidin-4-yl}-3,3-dimethyl-2-oxa-7-azaspiro[4.5]decan-1-one

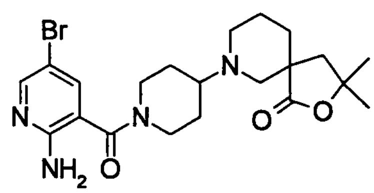

Using 2-amino-5-bromonicotinic acid (352 mg, 1.62 mmol) obtained in Reference Example 124 and 3,3-dimethyl-7-(piperidin-4-yl)-2-oxa-7-azaspiro[4.5]decan-1-one dihydrochloride (500 mg, 1.47 mmol) obtained in Reference Example 53, the title compound (686 mg, yield 86%) was obtained by an operation similar to that of Example 31 and trituration with diisopropyl ether.
EI(pos) 465 $[M]^+$

Example 251

N-(5-bromo-3-{[4-(3,3-dimethyl-1-oxo-2-oxa-7-azaspiro[4.5]dec-7-yl)piperidin-1-yl]carbonyl}pyridin-2-yl)-N'-ethylurea

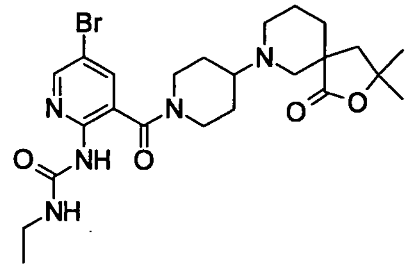

Using 7-{1-[(2-amino-5-bromopyridin-3-yl)carbonyl]piperidin-4-yl}-3,3-dimethyl-2-oxa-7-azaspiro[4.5]decan-1-one (313 mg, 0.673 mmol) obtained in Example 250 and ethyl isocyanate (0.080 mL, 1.01 mmol), the title compound (238 mg, yield 66%) was obtained by an operation similar to that of Example 166 and trituration with hexane.
EI(pos) 536 $[M]^+$

Example 252

7-{1-[(2-amino-5-phenylpyridin-3-yl)carbonyl]piperidin-4-yl}-3,3-dimethyl-2-oxa-7-azaspiro[4.5]decan-1-one

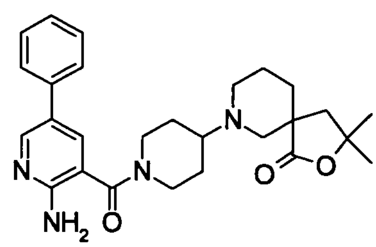

Using 7-{1-[(2-amino-5-bromopyridin-3-yl)carbonyl]piperidin-4-yl}-3,3-dimethyl-2-oxa-7-azaspiro[4.5]decan-1-one (500 mg, 1.07 mmol) obtained in Example 250 and phenylboronic acid (262 mg, 2.15 mmol), the title compound (412 mg, yield 83%) was obtained by an operation similar to that of Reference Example 56 and trituration with diisopropyl ether.
EI(pos) 463 $[M+H]^+$

Example 253

N-(3-{[4-(3,3-dimethyl-1-oxo-2-oxa-7-azaspiro[4.5]dec-7-yl)piperidin-1-yl]carbonyl}-5-phenylpyridin-2-yl)-N'-ethylurea

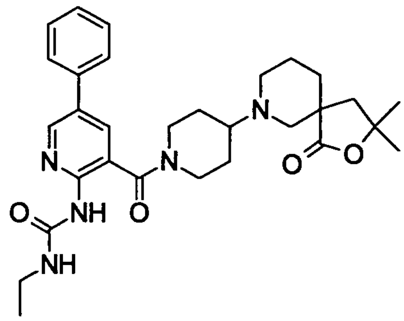

Using 7-{1-[(2-amino-5-phenylpyridin-3-yl)carbonyl]piperidin-4-yl}-3,3-dimethyl-2-oxa-7-azaspiro[4.5]decan-1-one (200 mg, 0.432 mmol) obtained in Example 252 and ethyl isocyanate (0.068 mL, 0.865 mmol), the title compound (40.3 mg, yield 17%) was obtained by an operation similar to that of Example 166 and trituration with diisopropyl ether.
EI(pos) 534 $[M+H]^+$

Example 254

7-{1-[(3-aminoisoquinolin-4-yl)carbonyl]piperidin-4-yl}-3,3-dimethyl-2-oxa-7-azaspiro[4.5]decan-1-one

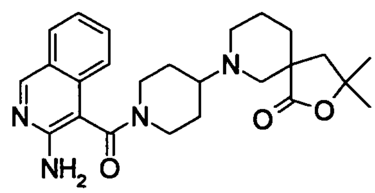

Using 3-aminoisoquinoline-4-carboxylic acid trifluoroacetate (300 mg, 0.993 mmol) obtained in Reference Example 126 and 3,3-dimethyl-7-(piperidin-4-yl)-2-oxa-7-azaspiro[4.5]decan-1-one dihydrochloride (337 mg, 0.993 mmol) obtained in Reference Example 53, the title compound (323 mg, yield 75%) was obtained by an operation similar to that of Example 31 and trituration with diisopropyl ether.
EI(pos) 437 $[M+H]^+$

Example 255

N-(4-{[4-(3,3-dimethyl-1-oxo-2-oxa-7-azaspiro[4.5]dec-7-yl)piperidin-1-yl]carbonyl}isoquinolin-3-yl)-N'-ethylurea

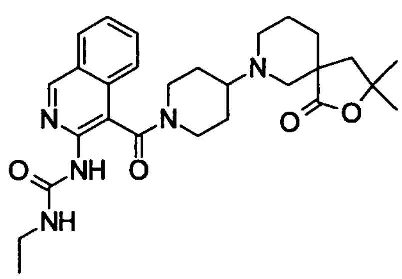

Using 7-{1-[(3-aminoisoquinolin-4-yl)carbonyl]piperidin-4-yl}-3,3-dimethyl-2-oxa-7-azaspiro[4.5]decan-1-one (150 mg, 0.344 mmol) obtained in Example 254 and ethyl isocyanate (0.054 mL, 0.687 mmol), the title compound (54.7 mg, yield 31%) was obtained by an operation similar to that of Example 166 and trituration with diisopropyl ether.
EI(pos) 508 $[M+H]^+$

Example 256

tert-butyl 5-amino-4-{[4-(3,3-dimethyl-1-oxo-2-oxa-7-azaspiro[4.5]dec-7-yl)piperidin-1-yl]carbonyl}-3-methylthiophene-2-carboxylate

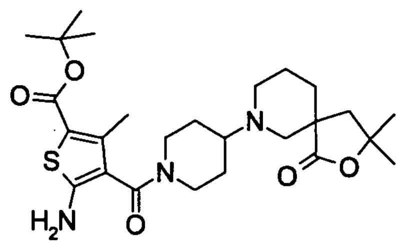

Using 2-amino-5-(tert-butoxycarbonyl)-4-methylthiophene-3-carboxylic acid (319 mg, 1.24 mmol) obtained in Reference Example 127 and 3,3-dimethyl-7-(piperidin-4-yl)-2-oxa-7-azaspiro[4.5]decan-1-one dihydrochloride (382 mg, 1.13 mmol) obtained in Reference Example 53, the title compound (451 mg, yield 79%) was obtained by an operation similar to that of Example 31 and trituration with hexane.
EI(pos) 506 $[M+H]^+$

Example 257

tert-butyl 4-{[4-(3,3-dimethyl-1-oxo-2-oxa-7-azaspiro[4.5]dec-7-yl)piperidin-1-yl]carbonyl}-5-{[(ethylamino)carbonyl] amino}-3-methylthiophene-2-carboxylate

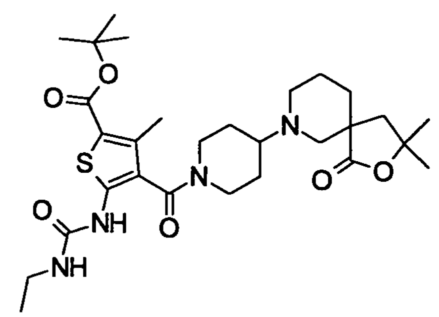

Using tert-butyl 5-amino-4-{[4-(3,3-dimethyl-1-oxo-2-oxa-7-azaspiro[4.5]dec-7-yl)piperidin-1-yl]carbonyl}-3-methylthiophene-2-carboxylate (200 mg, 0.396 mmol) obtained in Example 256 and ethyl isocyanate (0.047 mL, 0.593 mmol), the title compound (119 mg, yield 52%) was obtained by an operation similar to that of Example 166 and trituration with diisopropyl ether.
EI(pos) 577 $[M+H]^+$

Example 258

7-{1-[(2-amino-4-methyl-3-thienyl)carbonyl]piperidin-4-yl}-3,3-dimethyl-2-oxa-7-azaspiro[4.5]decan-1-one trifluoroacetate

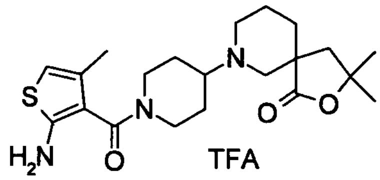

TFA

Using tert-butyl 5-amino-4-{[4-(3,3-dimethyl-1-oxo-2-oxa-7-azaspiro[4.5]dec-7-yl)piperidin-1-yl]carbonyl}-3-methylthiophene-2-carboxylate (200 mg, 0.396 mmol) obtained in Example 256, the title compound (127 mg, quantitative) was obtained by an operation similar to that of Reference Example 57 and trituration with diisopropyl ether.
EI(pos) 406 $[M+H]^+$

Example 259

N-(3-{[4-(3,3-dimethyl-1-oxo-2-oxa-7-azaspiro[4.5]dec-7-yl)piperidin-1-yl]carbonyl}-4-methyl-2-thienyl)-N'-ethylurea

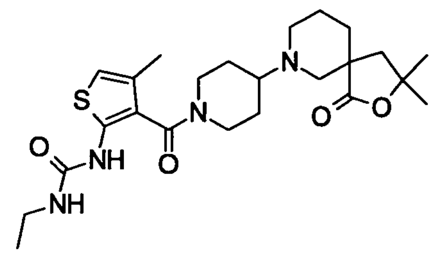

Using tert-butyl 4-{[4-(3,3-dimethyl-1-oxo-2-oxa-7-azaspiro[4.5]dec-7-yl)piperidin-1-yl]carbonyl}-5-{[(ethylamino)carbonyl]amino}-3-methylthiophene-2-carboxylate (80 mg, 0.139 mmol) obtained in Example 257, the title compound (87 mg, quantitative) was obtained by an operation similar to that of Reference Example 57 and trituration with diisopropyl ether.
EI(pos) 477 $[M+H]^+$

Example 260

7-{1-[(2,6-dimorpholin-4-ylpyrimidin-4-yl)carbonyl]piperidin-4-yl}-3,3-dimethyl-2-oxa-7-azaspiro[4.5]decan-1-one

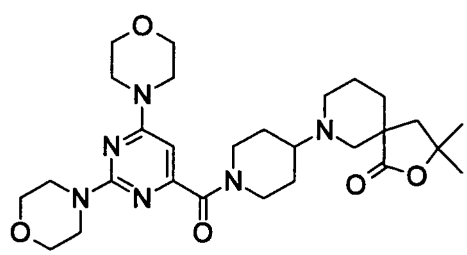

Using 2,6-di(morpholin-4-yl)pyrimidine-4-carboxylic acid (191 mg, 0.648 mmol) and 3,3-dimethyl-7-(piperidin-4-yl)-2-oxa-7-azaspiro[4.5]decan-1-one dihydrochloride (200 mg, 0.589 mmol) obtained in Reference Example 53, the title compound (213 mg, yield 67%) was obtained by an operation similar to that of Example 31 and trituration with diisopropyl ether.
EI (pos) 543 $[M+H]^+$

Example 261

N-(3-{[4-(3,3-dimethyl-1-oxo-2-oxa-7-azaspiro[4.5]dec-7-yl)piperidin-1-yl]carbonyl}-4-(4-fluorophenyl)-2-thienyl)-N'-ethylurea

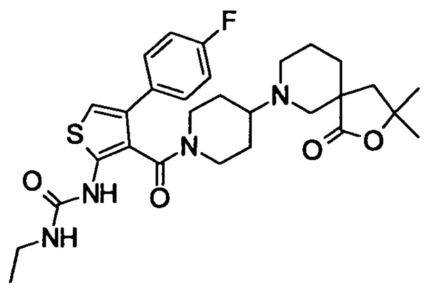

Using 2-amino-4-(4-fluorophenyl)thiophene-3-carboxylic acid (154 mg, 0.648 mmol) obtained in Reference Example 128 and 3,3-dimethyl-7-(piperidin-4-yl)-2-oxa-7-azaspiro[4.5]decan-1-one dihydrochloride (200 mg, 0.589 mmol) obtained in Reference Example 53, 7-(1-{[2-amino-4-(4-fluorophenyl)-3-thienyl]carbonyl}piperidin-4-yl)-3,3-dimethyl-2-oxa-7-azaspiro[4.5]decan-1-one (147 mg, yield 51%) was obtained by an operation similar to that of Example 31 and trituration with diisopropyl ether. Since this compound was somewhat unstable, the next reaction was successively performed. That is, using the obtained amine form (144 mg, 0.297 mmol) and ethyl isocyanate (0.035 mL, 0.445 mmol), the title compound (50.8 mg, yield 31%) was obtained by an operation similar to that of Example 166 and trituration with diisopropyl ether.
EI(pos) 557 $[M+H]^+$

Example 262

ethyl 3-{[4-(3,3-dimethyl-1-oxo-2-oxa-7-azaspiro[4.5]dec-7-yl)piperidin-1-yl]carbonyl}-2-{[(ethylamino)carbonyl]amino}-4,5,6,7-tetrahydro-1-benzothiophene-6-carboxylate

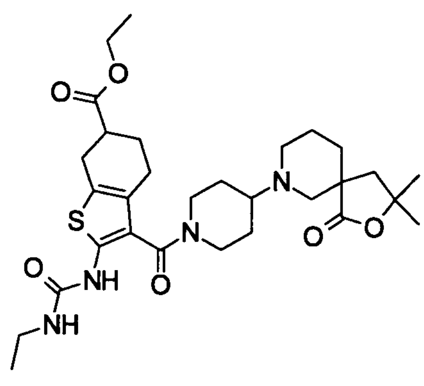

Using 2-amino-6-(ethoxycarbonyl)-4,5,6,7-tetrahydro-1-benzothiophene-3-carboxylic acid (175 mg, 0.648 mmol) obtained in Reference Example 130 and 3,3-dimethyl-7-(piperidin-4-yl)-2-oxa-7-azaspiro[4.5]decan-1-one dihydrochloride (200 mg, 0.589 mmol) obtained in Reference Example 53, ethyl 2-amino-3-{[4-(3,3-dimethyl-1-oxo-2-oxa-7-azaspiro[4.5]dec-7-yl)piperidin-1-yl]carbonyl}-4,5,6,7-tetrahydro-1-benzothiophene-6-carboxylate (112 mg, yield 37%) was obtained by an operation similar to that of Example 31 and trituration with diisopropyl ether. Since this compound was somewhat unstable, the next reaction was successively performed. That is, using the obtained amine form (109 mg, 0.211 mmol) and ethyl isocyanate (0.025 mL, 0.316 mmol), the title compound (30.8 mg, yield 25%) was obtained by an operation similar to that of Example 166 and trituration with diisopropyl ether.
EI(pos) 589 $[M+H]^+$

Example 263

N-(3-{[4-(3,3-dimethyl-1-oxo-2-oxa-7-azaspiro[4.5]dec-7-yl)piperidin-1-yl]carbonyl}-4,7-dihydro-5H-thieno[2,3-c]pyran-2-yl)-N'-ethylurea

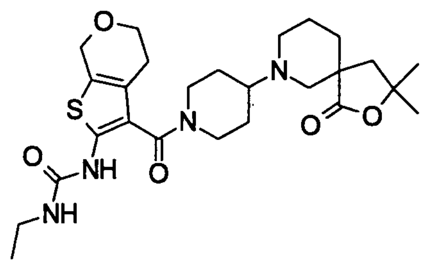

Using 2-amino-4,7-dihydro-5H-thieno[2,3-c]pyran-3-carboxylic acid (129 mg, 0.648 mmol) obtained in Reference Example 131 and 3,3-dimethyl-7-(piperidin-4-yl)-2-oxa-7-azaspiro[4.5]decan-1-one dihydrochloride (200 mg, 0.589 mmol) obtained in Reference Example 53, 7-{1-[(2-amino-4,7-dihydro-5H-thieno[2,3-c]pyran-3-yl)carbonyl]piperidin-4-yl}-3,3-dimethyl-2-oxa-7-azaspiro[4.5]decan-1-one (114 mg, yield 43%) was obtained by an operation similar to that of Example 31 and trituration with diisopropyl ether. Since this compound was somewhat unstable, the next reaction was successively performed. That is, using the obtained amine form (111 mg, 0.248 mmol) and ethyl isocyanate (0.029 mL, 0.372 mmol), the title compound (36.1 mg, yield 28%) was obtained by an operation similar to that of Example 166 and trituration with diisopropyl ether.
EI(pos) 519 $[M+H]^+$

Example 264

N-(3-{[4-(3,3-dimethyl-1-oxo-2-oxa-7-azaspiro[4.5]dec-7-yl)piperidin-1-yl]carbonyl}-4,5,6,7-tetrahydro-1-benzothien-2-yl)-N'-ethylurea

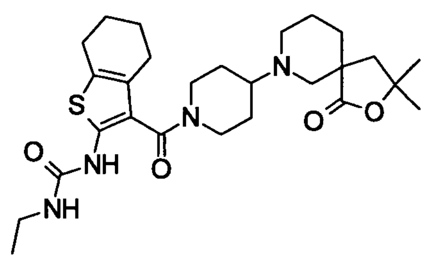

Using 2-amino-4,5,6,7-tetrahydro-1-benzothiophene-3-carboxylic acid (128 mg, 0.648 mmol) and 3,3-dimethyl-7-(piperidin-4-yl)-2-oxa-7-azaspiro[4.5]decan-1-one dihydrochloride (200 mg, 0.589 mmol) obtained in Reference Example 53, 7-{1-[(2-amino-4,5,6,7-tetrahydro-1-benzothien-3-yl)carbonyl]piperidin-4-yl}-3,3-dimethyl-2-oxa-7-azaspiro[4.5]decan-1-one (70 mg, yield 13%) was obtained by an operation similar to that of Example 31 and trituration with diisopropyl ether. Since this compound was somewhat unstable, the next reaction was successively performed. That is, using the obtained amine form (68 mg, 0.153 mmol) and ethyl isocyanate (0.018 mL, 0.229 mmol), the title compound (7 mg, yield 9%) was obtained by an operation similar to that of Example 166 and trituration with diisopropyl ether.
EI(pos) 517 $[M+H]^+$

Example 265

N-(3-{[4-(3,3-dimethyl-1-oxo-2-oxa-7-azaspiro[4.5]dec-7-yl)piperidin-1-yl]carbonyl}-4,5-dimethyl-2-thienyl)-N'-ethylurea

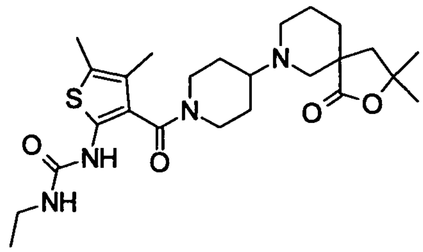

Using 2-amino-4,5-dimethylthiophene-3-carboxylic acid (111 mg, 0.648 mmol) and 3,3-dimethyl-7-(piperidin-4-yl)-2-oxa-7-azaspiro[4.5]decan-1-one dihydrochloride (200 mg, 0.589 mmol) obtained in Reference Example 53, 7-{1-[(2-amino-4,5-dimethyl-3-thienyl)carbonyl]piperidin-4-yl}-3,3-dimethyl-2-oxa-7-azaspiro[4.5]decan-1-one (95.6 mg, yield 39%) was obtained by an operation similar to that of Example 31 and trituration with diisopropyl ether. Since this compound was somewhat unstable, the next reaction was successively performed. That is, using the obtained amine form (95.6 mg, 0.228 mmol) and ethyl isocyanate (0.027 mL, 0.342 mmol), the title compound (23 mg, yield 21%) was obtained by an operation similar to that of Example 166 and trituration with diisopropyl ether.
EI(pos) 491 $[M+H]^+$

Example 266

N-(3-{[4-(3,3-dimethyl-1-oxo-2-oxa-7-azaspiro[4.5]dec-7-yl)piperidin-1-yl]carbonyl}-4,7-dihydro-5H-thieno[2,3-c]thiopyran-2-yl)-N'-ethylurea

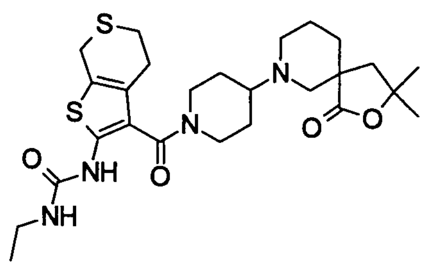

Using 2-amino-4,7-dihydro-5H-thieno[2,3-c]thiopyran-3-carboxylic acid (750 mg, 3.48 mmol) obtained in Reference Example 132 and 3,3-dimethyl-7-(piperidin-4-yl)-2-oxa-7-azaspiro[4.5]decan-1-one dihydrochloride (1.07 g, 3.17 mmol) obtained in Reference Example 53, 7-{1-[(2-amino-4,7-dihydro-5H-thieno[2,3-c]thiopyran-3-yl)carbonyl]piperidin-4-yl}-3,3-dimethyl-2-oxa-7-azaspiro[4.5]decan-1-one (823 mg, yield 56%) was obtained by an operation similar to that of Example 31 and trituration with diisopropyl ether. Since this compound was somewhat unstable, the next reaction was successively performed. That is, using the obtained amine form (822 mg, 1.77 mmol) and ethyl isocyanate (0.210 mL, 2.66 mmol), the title compound (517 mg, yield 55%) was obtained by an operation similar to that of Example 166 and trituration with diisopropyl ether.
EI(pos) 535 $[M+H]^+$

Example 267

3,3-dimethyl-7-{1-[(2-methyl-1-benzothien-3-yl)carbonyl]piperidin-4-yl}-2-oxa-7-azaspiro[4.5]decan-1-one

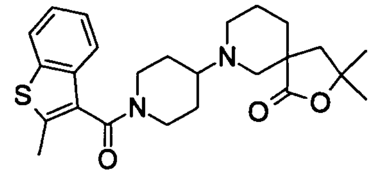

Using 2-methyl-1-benzothiophene-3-carboxylic acid (588 mg, 3.06 mmol) and 3,3-dimethyl-7-(piperidin-4-yl)-2-oxa-7-azaspiro[4.5]decan-1-one dihydrochloride (1.04 g, 3.06 mmol) obtained in Reference Example 53, the title compound (1.22 g, yield 91%) was obtained as an oil by an operation similar to that of Example 31.
EI(pos) 441 $[M+H]^+$

Example 268

7-[1-(2-amino-6-methoxybenzoyl)piperidin-4-yl]-3,3-dimethyl-2-oxa-7-azaspiro[4.5]decan-1-one

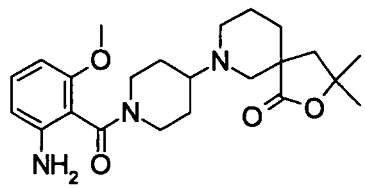

Using 2-amino-6-methoxybenzoic acid (163 mg, 0.973 mmol) and 3,3-dimethyl-7-(piperidin-4-yl)-2-oxa-7-azaspiro[4.5]decan-1-one dihydrochloride (300 mg, 0.884 mmol) obtained in Reference Example 53, the title compound (152 mg, yield 41%) was obtained by an operation similar to that of Example 31 and trituration with diisopropyl ether and hexane.
EI(pos) 416 $[M+H]^+$

Example 269

N-(2-{[4-(3,3-dimethyl-1-oxo-2-oxa-7-azaspiro[4.5]dec-7-yl)piperidin-1-yl]carbonyl}-3-methoxyphenyl)-N'-ethylurea

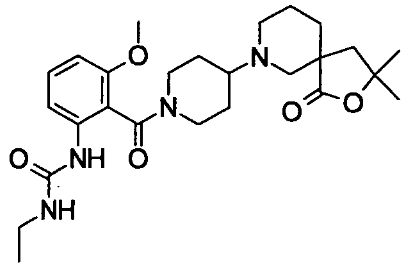

Using 7-[1-(2-amino-6-methoxybenzoyl)piperidin-4-yl]-3,3-dimethyl-2-oxa-7-azaspiro[4.5]decan-1-one (133

mg, 0.320 mmol) obtained in Example 268 and ethyl isocyanate (0.038 mL, 0.480 mmol), the title compound (114 mg, yield 73%) was obtained by an operation similar to that of Example 166 and trituration with diisopropyl ether.
EI(pos) 487 $[M+H]^+$

Example 270

7-[1-(2-hydroxy-1-naphthoyl)piperidin-4-yl]-3,3-dimethyl-2-oxa-7-azaspiro[4.5]decan-1-one

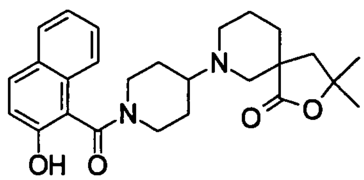

Using 2-hydroxy-1-naphtoic acid (244 mg, 1.30 mmol) and 3,3-dimethyl-7-(piperidin-4-yl)-2-oxa-7-azaspiro[4.5]decan-1-one dihydrochloride (400 mg, 1.18 mmol) obtained in Reference Example 53, the title compound (221 mg, yield 43%) was obtained by an operation similar to that of Example 31 and trituration with diisopropyl ether.
EI(pos) 437 $[M+H]^+$

Example 271

1-{[4-(3,3-dimethyl-1-oxo-2-oxa-7-azaspiro[4.5]dec-7-yl)piperidin-1-yl]carbonyl}-2-naphthyl ethylcarbamate

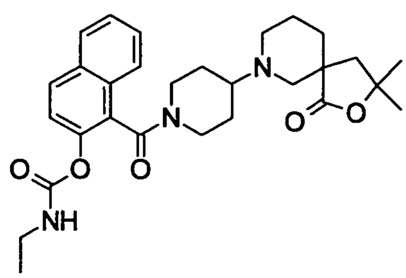

Using 7-[1-(2-hydroxy-1-naphthoyl)piperidin-4-yl]-3,3-dimethyl-2-oxa-7-azaspiro[4.5]decan-1-one (183 mg, 0.419 mmol) obtained in Example 270 and ethyl isocyanate (0.050 mL, 0.629 mmol), the title compound (2.9 mg, yield 1%) was obtained by an operation similar to that of Example 166 and trituration with diisopropyl ether.
EI(pos) 508 $[M+H]^+$

Example 272

7-{1- [(5-ethoxy-2-phenyl-1, 3-oxazol-4-yl) carbonyl]piperidin-4-yl}-3,3-dimethyl-2-oxa-7-azaspiro[4.5]decan-1-one

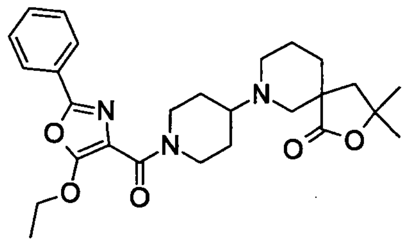

Using 5-ethoxy-2-phenyl-1,3-oxazole-4-carboxylic acid (151 mg, 0.648 mmol) and 3,3-dimethyl-7-(piperidin-4-yl)-2-oxa-7-azaspiro[4.5]decan-1-one dihydrochloride (200 mg, 0.589 mmol) obtained in Reference Example 53, the title compound (185 mg, yield 65%) was obtained as an oil by an operation similar to that of Example 31.
EI(pos) 482 $[M+H]^+$

Example 273

7-{1-[(2-amino-7-oxo-4,5,6,7-tetrahydro-1-benzothien-3-yl)carbonyl]piperidin-4-yl}-3,3-dimethyl-2-oxa-7-azaspiro[4.5]decan-1-one

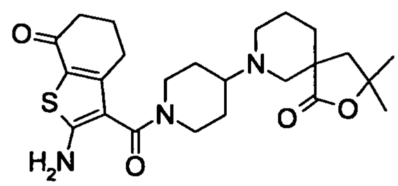

Using 2-amino-7-oxo-4,5,6,7-tetrahydro-1-benzothiophene-3-carboxylic acid (115 mg, 0.544 mmol) obtained in Reference Example 133 and 3,3-dimethyl-7-(piperidin-4-yl)-2-oxa-7-azaspiro[4.5]decan-1-one dihydrochloride (194 mg, 0.572 mmol) obtained in Reference Example 53, the title compound (177 mg, yield 71%) was obtained by an operation similar to that of Example 31 and trituration with diisopropyl ether.
EI (pos) 460 $[M+H]^+$

Example 274

N-(3-{[4-(3,3-dimethyl-1-oxo-2-oxa-7-azaspiro[4.5]dec-7-yl)piperidin-1-yl]carbonyl}-7-oxo-4,5,6,7-tetrahydro-1-benzothien-2-yl)-N'-ethylurea

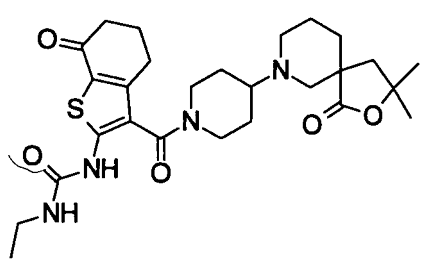

Using 7-{1-[(2-amino-7-oxo-4,5,6,7-tetrahydro-1-benzothien-3-yl)carbonyl]piperidin-4-yl}-3,3-dimethyl-2-oxa-7-azaspiro[4.5]decan-1-one (150 mg, 0.326 mmol) obtained in Example 273 and ethyl isocyanate (0.039 mL, 0.490 mmol), the title compound (111 mg, yield 64%) was obtained by an operation similar to that of Example 166 and trituration

with diisopropyl ether.
EI(pos) 531 [M+H]+

Example 275

7-{1-[(3-amino-5-phenyl-2-thienyl)carbonyl]piperidin-4-yl}-3,3-dimethyl-2-oxa-7-azaspiro[4.5]decan-1-one

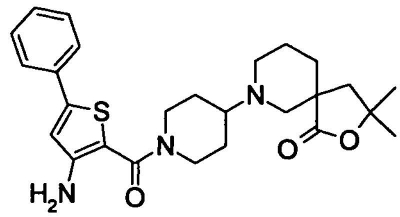

Using 3-amino-5-phenylthiophene-2-carboxylic acid (136 mg, 0.619 mmol) obtained in Reference Example 134 and 3,3-dimethyl-7-(piperidin-4-yl)-2-oxa-7-azaspiro[4.5]decan-1-one dihydrochloride (200 mg, 0.589 mmol) obtained in Reference Example 53, the title compound (236 mg, yield 85%) was obtained by an operation similar to that of Example 31 and trituration with diisopropyl ether.
EI(pos) 468 [M+H]+

Example 276

N-(2-{[4-(3,3-dimethyl-1-oxo-2-oxa-7-azaspiro[4.5]dec-7-yl)piperidin-1-yl]carbonyl}-5-phenyl-3-thienyl)-N'-ethylurea

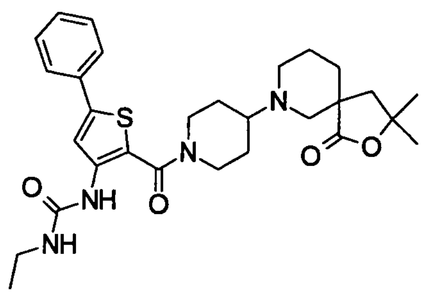

Using 7-{1-[(3-amino-5-phenyl-2-thienyl)carbonyl]piperidin-4-yl}-3,3-dimethyl-2-oxa-7-azaspiro[4.5]decan-1-one (200 mg, 0.428 mmol) obtained in Example 275 and ethyl isocyanate (0.051 mL, 0.642 mmol), the title compound (161 mg, yield 70%) was obtained by an operation similar to that of Example 166 and trituration with diisopropyl ether.
EI(pos) 539 [M+H]+

Example 277

3,3-dimethyl-7-(1-{[2-(1-methyl-1H-pyrazol-3-yl)quinolin-4-yl]carbonyl}piperidin-4-yl)-2-oxa-7-azaspiro[4.5]decan-1-one

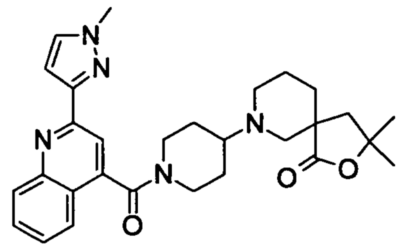

Using 2-(1-methyl-1H-pyrazol-3-yl)quinoline-4-carboxylic acid (164 mg, 0.648 mmol) and 3,3-dimethyl-7-(piperidin-4-yl)-2-oxa-7-azaspiro[4.5]decan-1-one dihydrochloride (200 mg, 0.589 mmol) obtained in Reference Example 53, the title compound (162 mg, yield 55%) was obtained by an operation similar to that of Example 31 and trituration with diisopropyl ether.
EI(pos) 502 $[M+H]^+$

Example 278

7-[1-(2-amino-5-hydroxybenzoyl)piperidin-4-yl]-3,3-dimethyl-2-oxa-7-azaspiro[4.5]decan-1-one

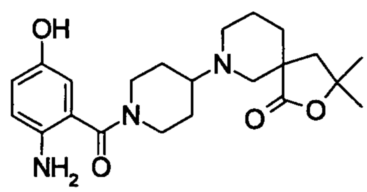

Using 2-amino-5-hydroxybenzoic acid (142 mg, 0.928 mmol) and 3,3-dimethyl-7-(piperidin-4-yl)-2-oxa-7-azaspiro[4.5]decan-1-one dihydrochloride (300 mg, 0.884 mmol) obtained in Reference Example 53, the title compound (41.8 mg, yield 12%) was obtained by an operation similar to that of Example 31 and trituration with diisopropyl ether.
EI(pos) 402 $[M+H]^+$

Example 279

3-{[4-(3,3-dimethyl-1-oxo-2-oxa-7-azaspiro[4.5]dec-7-yl)piperidin-1-yl]carbonyl}-4-{[(ethylamino)carbonyl]amino}phenyl ethylcarbamate

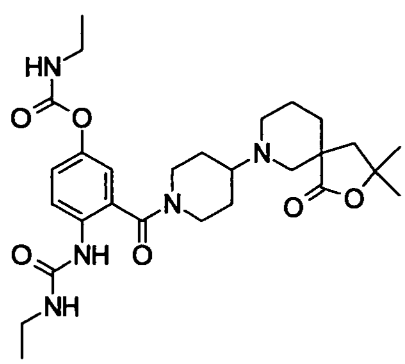

Using 7-[1-(2-amino-5-hydroxybenzoyl)piperidin-4-yl]-3,3-dimethyl-2-oxa-7-azaspiro[4.5]decan-1-one (38.2 mg, 0.095 mmol) obtained in Example 278 and ethyl isocyanate (0.023 mL, 0.285 mmol), the title compound (24.7 mg,

yield 48%) was obtained by an operation similar to that of Example 166 and trituration with diisopropyl ether.
EI(pos) 544 [M+H] +

Example 280

methyl 4-amino-3-{[4-(3,3-dimethyl-1-oxo-2-oxa-7-azaspiro[4.5]dec-7-yl)piperidin-1-yl]carbonyl}benzoate

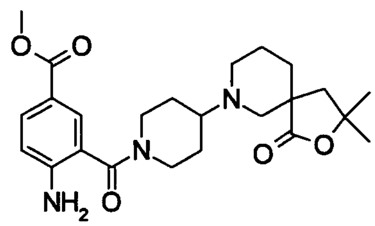

Using 2-amino-5-(methoxycarbonyl)benzoic acid (173 mg, 0.884 mmol) and 3,3-dimethyl-7-(piperidin-4-yl)-2-oxa-7-azaspiro[4.5]decan-1-one dihydrochloride (300 mg, 0.884 mmol) obtained in Reference Example 53, the title compound (282 mg, yield 72%) was obtained by an operation similar to that of Example 31 and trituration with diisopropyl ether.
EI(pos) 444 [M+H]+

Example 281

methyl 3-{[4-(3,3-dimethyl-1-oxo-2-oxa-7-azaspiro[4.5]dec-7-yl)piperidin-1-yl]carbonyl}-4-{[(ethylamino)carbonyl]amino}benzoate

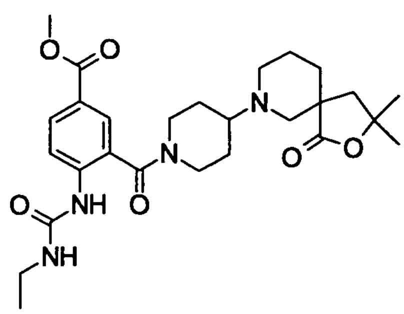

Using methyl 4-amino-3-{[4-(3,3-dimethyl-1-oxo-2-oxa-7-azaspiro[4.5]dec-7-yl)piperidin-1-yl]carbonyl}benzoate (156 mg, 0.352 mmol) obtained in Example 280 and ethyl isocyanate (0.042 mL, 0.528 mmol), the title compound (71.8 mg, yield 40%) was obtained by an operation similar to that of Example 166 and trituration with diisopropyl ether.
EI(pos) 515 [M+H]+

Example 282

7-{1-[(5-amino-2-phenyl-1,3-thiazol-4-yl)carbonyl]piperidin-4-yl}-3,3-dimethyl-2-oxa-7-azaspiro[4.5]decan-1-one

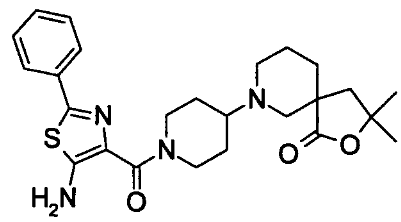

Using 5-amino-2-phenyl-1,3-thiazole-4-carboxylic acid (130 mg, 0.589 mmol) obtained in Reference Example 135 and 3,3-dimethyl-7-(piperidin-4-yl)-2-oxa-7-azaspiro[4.5]decan-1-one dihydrochloride (200 mg, 0.589 mmol) obtained in Reference Example 53, the title compound (201 mg, yield 73%) was obtained by an operation similar to that of Example 31 and trituration with diisopropyl ether and hexane.
EI(pos) 469 $[M+H]^+$

Example 283

N-(4-{[4-(3,3-dimethyl-1-oxo-2-oxa-7-azaspiro[4.5]dec-7-yl)piperidin-1-yl]carbonyl}-2-phenyl-1,3-thiazol-5-yl)-N'-ethylurea

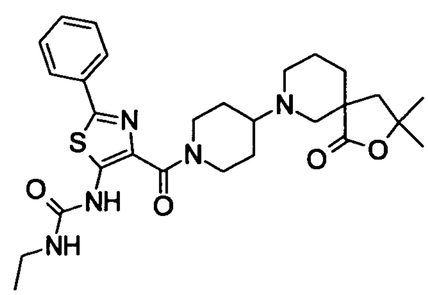

Using 7-{1-[(5-amino-2-phenyl-1,3-thiazol-4-yl)carbonyl]piperidin-4-yl}-3,3-dimethyl-2-oxa-7-azaspiro[4.5]decan-1-one (150 mg, 0.320 mmol) obtained in Example 282 and ethyl isocyanate (0.038 mL, 0.480 mmol), the title compound (143 mg, yield 83%) was obtained by an operation similar to that of Example 166 and trituration with diisopropyl ether and hexane.
EI(pos) 540 $[M+H]^+$

Example 284

benzyl (2-{[4-(3,3-dimethyl-1-oxo-2-oxa-7-azaspiro[4.5]dec-7-yl)piperidin-1-yl]carbonyl}-4-hydroxyphenyl)carbamate

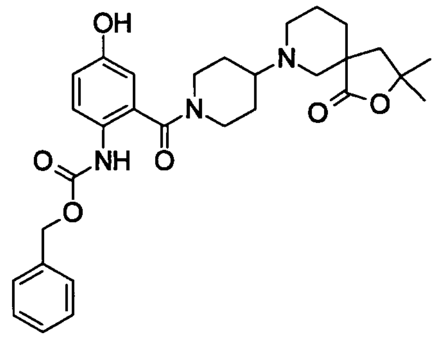

Using 5-(acetyloxy)-2-{[(benzyloxy)carbonyl]amino}benzoic acid (1.60 g, 4.86 mmol) obtained in Reference Example 136 and 3,3-dimethyl-7-(piperidin-4-yl)-2-oxa-7-azaspiro[4.5]decan-1-one dihydrochloride (1.50 g, 4.42 mmol) obtained in Reference Example 53, 4-{[(benzyloxy)carbonyl]amino}-3-{[4-(3,3-dimethyl-1-oxo-2-oxa-7-azaspiro[4.5]dec-7-yl)piperidin-1-yl]carbonyl}phenyl acetate (1.74 g, yield 68%) was obtained by an operation similar to that of Example 31. The obtained acetate form (1.74 g, 3.01 mmol) was dissolved in methanol (30 mL), 1N aqueous sodium hydroxide solution (3.16 mL) was added, and the mixture was stirred at room temperature for 16 hr. The solvent was evaporated under reduced pressure, 1N hydrochloric acid (3.16 mL) was added, and the resultant precipitate was collected by filtration. The obtained precipitate was dissolved in ethyl acetate, and dried over anhydrous sodium sulfate. The solvent was evaporated under reduced pressure, and the obtained residue was purified by basic silica gel column chromatography (developing solvent; hexane:ethyl acetate=1:1→ethyl acetate:methanol=5:1) and triturated with diisopropyl ether:hexane=1:1 to give the title compound (1.19 g, yield 74%).
EI(pos) 536 $[M+H]^+$

Example 285

tert-butyl (4-{[(benzyloxy)carbonyl]amino}-3-{[4-(3,3-dimethyl-1-oxo-2-oxa-7-azaspiro[4.5]dec-7-yl)piperidin-1-yl]carbonyl}phenoxy)acetate

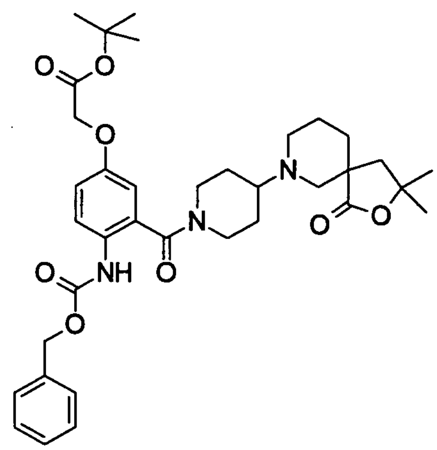

Benzyl (2-{[4-(3,3-dimethyl-1-oxo-2-oxa-7-azaspiro[4.5]dec-7-yl)piperidin-1-yl]carbonyl}-4-hydroxyphenyl) carbamate (500 mg, 0.933 mmol) obtained in Example 284 and potassium carbonate (258 mg, 1.87 mmol) were dissolved in N,N-dimethylformamide (2.5 mL), tert-butyl bromoacetate (0.145 mL, 0.980 mmol) was added, and the mixture was stirred at room temperature for 16 hr. The reaction mixture was diluted with ethyl acetate, washed with aqueous potassium carbonate solution and saturated brine, and dried over anhydrous sodium sulfate. The solvent was evaporated under reduced pressure, and the obtained residue was purified by silica gel column chromatography (developing solvent; hexane:ethyl acetate=3:1→0:1) to give the title compound (577 mg, yield 95%) as an oil.
EI(pos) 650 $[M+H]^+$

Example 286

tert-butyl (4-amino-3-{[4-(3,3-dimethyl-1-oxo-2-oxa-7-azaspiro[4.5]dec-7-yl)piperidin-1-yl]carbonyl}phenoxy)acetate

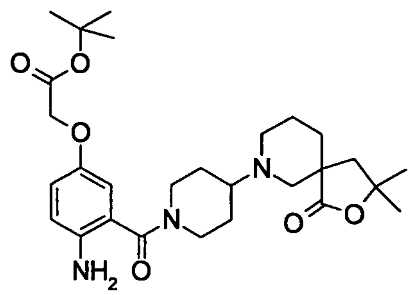

To tert-butyl (4-{[(benzyloxy)carbonyl]amino}-3-{[4-(3,3-dimethyl-1-oxo-2-oxa-7-azaspiro[4.5]dec-7-yl)piperidin-1-yl]carbonyl}phenoxy)acetate (560 mg, 0.862 mmol) obtained in Example 285 and 10% palladium carbon (100 mg) was added tetrahydrofuran (8 mL), and the mixture was stirred at room temperature for 3 hr under a hydrogen atmosphere. The catalyst was filtrated off through celite, and the filtrate was concentrated under reduced pressure. The obtained residue was purified by silica gel column chromatography (developing solvent; hexane:ethyl acetate=1:3→0:1) to give the title compound (316 mg, yield 71%) as an oil.
EI(pos) 516 $[M+H]^+$

Example 287

tert-butyl (3-{[4-(3,3-dimethyl-1-oxo-2-oxa-7-azaspiro[4.5]dec-7-yl)piperidin-1-yl]carbonyl}-4-{[(ethylamino)carbonyl]amino}phenoxy)acetate

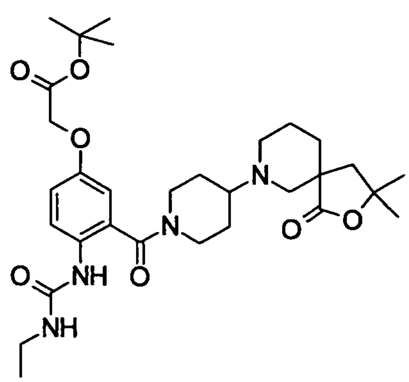

Using tert-butyl (4-amino-3-{[4-(3,3-dimethyl-1-oxo-2-oxa-7-azaspiro[4.5]dec-7-yl)piperidin-1-yl]carbonyl}phenoxy)acetate (208 mg, 0.403 mmol) obtained in Example 286 and ethyl isocyanate (0.064 mL, 0.807 mmol), the title compound (253 mg, quantitative) was obtained by an operation similar to that of Example 166 and trituration with diisopropyl ether and hexane.
EI(pos) 587 $[M+H]^+$

Example 288

ethyl (3-{[4-(3,3-dimethyl-1-oxo-2-oxa-7-azaspiro[4.5]dec-7-yl)piperidin-1-yl]carbonyl}-4-{[(ethylamino)carbonyl]amino}phenoxy)acetate

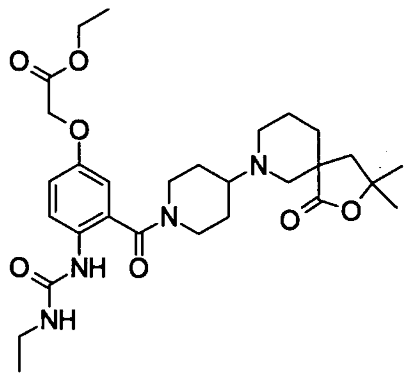

Using benzyl (2-{[4-(3,3-dimethyl-1-oxo-2-oxa-7-azaspiro[4.5]dec-7-yl)piperidin-1-yl]carbonyl}-4-hydroxyphenyl)carbamate (620 mg, 1.16 mmol) obtained in Example 284, the title compound (427 mg, yield of 3 steps 66%) was obtained by successively performing operations similar to those of Examples 285, 286 and 166 and trituration with diisopropyl ether and hexane.
EI(pos) 559 $[M+H]^+$

Example 289

(3-{[4-(3,3-dimethyl-1-oxo-2-oxa-7-azaspiro[4.5]dec-7-yl)piperidin-1-yl]carbonyl}-4-{[(ethylamino)carbonyl]amino}phenoxy)acetic acid sodium salt

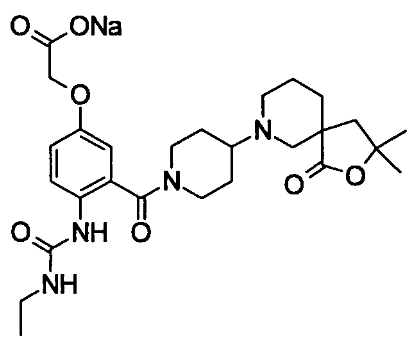

Ethyl (3-{[4-(3,3-dimethyl-1-oxo-2-oxa-7-azaspiro[4.5]dec-7-yl)piperidin-1-yl]carbonyl}-4-{[(ethylamino)carbonyl]amino}phenoxy)acetate (400 mg, 0.716 mmol) obtained in Example 288 was dissolved in methanol (8 mL), 1N aqueous sodium hydroxide solution (0.788 mL) was added, and the mixture was stirred at room temperature for 16 hr. The solvent was evaporated under reduced pressure, and the residue was triturated with tetrahydrofuran. This was washed with tetrahydrofuran, isopropyl alcohol and diisopropyl ether to give the title compound (161 mg, yield 41%) as a powder.
EI(pos) 531 $[M+H]^+$

Example 290

N-(3-{[4-(3,3-dimethyl-1-oxo-2-oxa-7-azaspiro[4.5]dec-7-yl)piperidin-1-yl]carbonyl}-1-benzothien-2-yl)urea

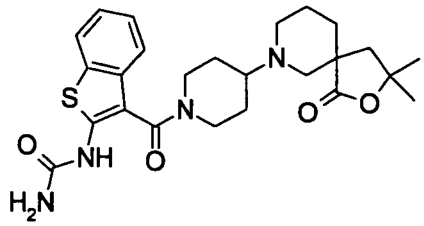

To a solution of 7-{1-[(2-amino-1-benzothien-3-yl)carbonyl]piperidin-4-yl}-3,3-dimethyl-2-oxa-7-azaspiro[4.5] decan-1-one (300 mg, 0.679 mmol) obtained in Example 31 in tetrahydrofuran (7 mL) was added trichloroacetyl isocyanate (0.162 mL, 1.36 mmol) at 0˚C, and the mixture was stirred for 2 hr. A 7M ammonia-methanol solution was added, and the mixture was stirred at room temperature for 1 day. The reaction mixture was concentrated under reduced pressure, purified by basic silica gel column chromatography, and triturated with diisopropyl ether to give the title compound (151 mg, yield 46%).
EI(pos) 485 $[M+H]^+$

Example 291

tert-butyl 5-[(aminocarbonyl)amino]-4-{[4-(3,3-dimethyl-1-oxo-2-oxa-7-azaspiro[4.5]dec-7-yl)piperidin-1-yl]carbonyl}-3-methylthiophene-2-carboxylate

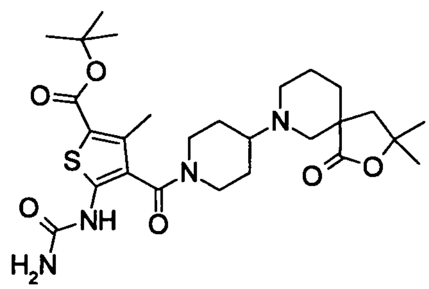

Using tert-butyl 5-amino-4-{[4-(3,3-dimethyl-1-oxo-2-oxa-7-azaspiro[4.5]dec-7-yl)piperidin-1-yl]carbonyl}-3-methylthiophene-2-carboxylate (95 mg, 0.188 mmol) obtained in Example 256 and trichloroacetyl isocyanate (0.045 mL, 0.376 mmol), the title compound (21 mg, yield 20%) was obtained by an operation similar to that of Example 290 and trituration with diisopropyl ether.
EI(pos) 549 $[M+H]^+$

Example 292

N-(4-{[4-(3,3-dimethyl-1-oxo-2-oxa-7-azaspiro[4.5]dec-7-yl)piperidin-1-yl]carbonyl}-2-phenyl-1,3-thiazol-5-yl)urea

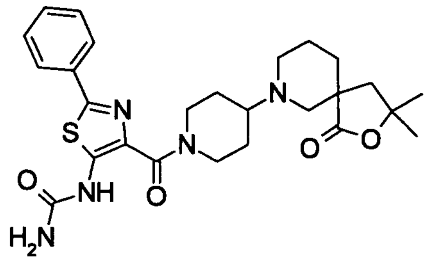

Using 7-{1-[(5-amino-2-phenyl-1,3-thiazol-4-yl)carbonyl]piperidin-4-yl}-3,3-dimethyl-2-oxa-7-azaspiro[4.5]decan-1-one (42.3 mg, 0.090 mmol) obtained in Example 282 and trichloroacetyl isocyanate (0.022 mL, 0.181 mmol), the title compound (28 mg, yield 61%) was obtained by an operation similar to that of Example 290 and trituration with diisopropyl ether.
EI(pos) 512 $[M+H]^+$

Example 293

7-{1-[(2-bromoquinolin-4-yl)carbonyl]piperidin-4-yl}-3,3-dimethyl-2-oxa-7-azaspiro[4.5]decan-1-one

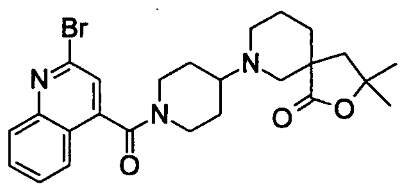

To a solution of 2-bromoquinoline-4-carboxylic acid (1.56 g, 6.19 mmol) and 3,3-dimethyl-7-(piperidin-4-yl)-2-oxa-7-azaspiro[4.5]decan-1-one dihydrochloride (2.00 g, 5.89 mmol) obtained in Reference Example 53 in N,N-dimethylformamide (15 mL) were added triethylamine (1.97 mL, 14.1 mmol) and 1-ethyl-3-(3-dimethylaminopropyl)carbodiimide hydrochloride (1.19 g, 6.19 mmol), and the mixture was stirred at room temperature for 16 hr. The reaction mixture was diluted with ethyl acetate, washed with aqueous potassium carbonate solution and saturated brine, and dried over anhydrous sodium sulfate. The solvent was evaporated under reduced pressure, and the obtained residue was purified by silica gel column chromatography (developing solvent; ethyl acetate) and triturated with diisopropyl ether to give the title compound (461 mg, yield 16%).
EI(pos) 500 $[M]^+$

Example 294

ethyl 1-(4-{[4-(3,3-dimethyl-1-oxo-2-oxa-7-azaspiro[4.5]dec-7-yl)piperidin-1-yl]carbonyl}quinolin-2-yl)piperidine-4-carboxylate

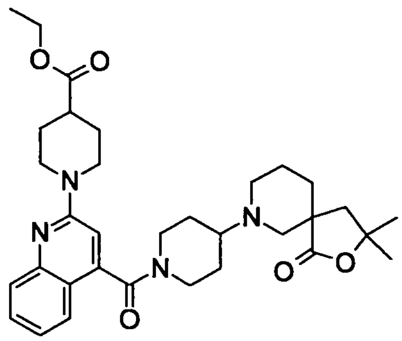

To 7-{1-[(2-bromoquinolin-4-yl)carbonyl]piperidin-4-yl}-3,3-dimethyl-2-oxa-7-azaspiro[4.5]decan-1-one (200 mg, 0.400 mmol) obtained in Example 293, ethyl piperidine-4-carboxylate (190 mg, 1.20 mmol) and potassium carbonate (166 mg, 1.20 mmol) was added dimethyl sulfoxide (2 mL), and the mixture was stirred at 80˚C for 16 hr. The reaction mixture was diluted with ethyl acetate, washed with aqueous potassium carbonate solution and saturated brine, and dried over anhydrous sodium sulfate. The solvent was evaporated under reduced pressure, and the obtained residue was purified by silica gel column chromatography (developing solvent; hexane:ethyl acetate=1:1→ 0:1) to give the title compound (145 mg, yield 63%) as an oil. EI(pos) 577 $[M+H]^+$

Example 295

methyl 1-(4-{[4-(3,3-dimethyl-1-oxo-2-oxa-7-azaspiro[4.5]dec-7-yl)piperidin-1-yl]carbonyl}quinolin-2-yl)piperidine-4-carboxylate

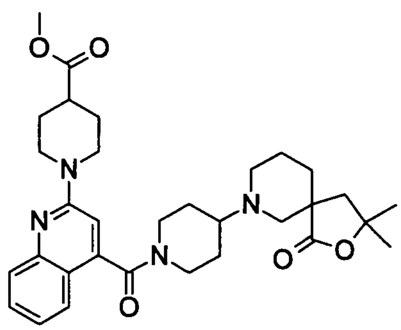

To a solution of ethyl 1-(4-{[4-(3,3-dimethyl-1-oxo-2-oxa-7-azaspiro[4.5]dec-7-yl)piperidin-1-yl]carbonyl}quinolin-2-yl)piperidine-4-carboxylate (130 mg, 0.225 mmol) obtained in Example 294 in methanol (1 mL) was added 1N aqueous sodium hydroxide solution (0.236 mL), and the mixture was stirred at room temperature for 16 hr. The reaction mixture was concentrated under reduced pressure, and 0.1% aqueous trifluoroacetic acid solution was added to the obtained aqueous solution. The resultant precipitate was collected and washed well with water to give the title compound (38 mg, yield 30%) as a powder.
EI(pos) 563 $[M+H]^+$

Example 296

7-{1-[(5-acetyl-2-amino-4-methyl-3-thienyl)carbonyl]piperidin-4-yl}-3,3-dimethyl-2-oxa-7-azaspiro[4.5]decan-1-one

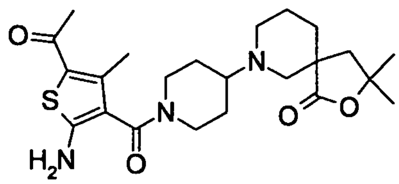

Using 5-acetyl-2-amino-4-methylthiophene-3-carboxylic acid (98.2 mg, 0.493 mmol) and 3,3-dimethyl-7-(piperidin-4-yl)-2-oxa-7-azaspiro[4.5]decan-1-one dihydrochloride (152 mg, 0.448 mmol) obtained in Reference Example 53, the title compound (175 mg, yield 87%) was obtained by an operation similar to that of Example 31 and trituration with diisopropyl ether.
EI(pos) 448 $[M+H]^+$

Example 297

N-(5-acetyl-3-{[4-(3,3-dimethyl-l-oxo-2-oxa-7-azaspiro[4.5]dec-7-yl)piperidin-1-yl]carbonyl}-4-methyl-2-thienyl)-N'-ethylurea

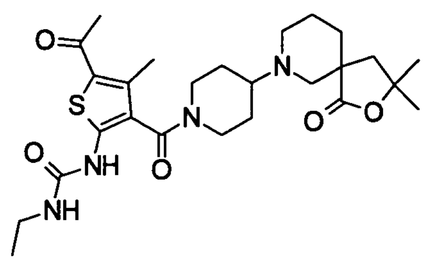

Using 7-{1-[(5-acetyl-2-amino-4-methyl-3-thienyl)carbonyl]piperidin-4-yl}-3,3-dimethyl-2-oxa-7-azaspiro[4.5]decan-1-one (155 mg, 0.346 mmol) obtained in Example 296 and ethyl isocyanate (0.082 mL, 1.04 mmol), the title compound (108 mg, yield 60%) was obtained by an operation similar to that of Example 166 and trituration with diisopropyl ether.
EI(pos) 519 $[M+H]^+$

Example 298

tert-butyl (6-chloro-4-{[4-(3,3-dimethyl-1-oxo-2-oxa-7-azaspiro[4.5]dec-7-yl)piperidin-1-yl]carbonyl}pyridin-3-yl)carbamate

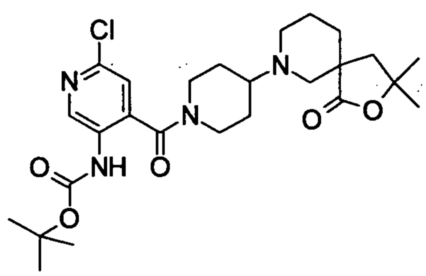

Using 5-[(tert-butoxycarbonyl)amino]-2-chloroisonicotinic acid (804 mg, 2.95 mmol) and 3,3-dimethyl-7-(piperidin-4-yl)-2-oxa-7-azaspiro[4.5]decan-1-one dihydrochloride (1.00 g, 2.95 mmol) obtained in Reference Example 53, the title compound (896 mg, yield 58%) was obtained by an operation similar to that of Example 31 and trituration with

diisopropyl ether. EI(pos) 521 [M+H]+

Example 299

tert-butyl (4-{[4-(3,3-dimethyl-1-oxo-2-oxa-7-azaspiro[4.5]dec-7-yl)piperidin-1-yl]carbonyl}-6-phenylpyridin-3-yl)carbamate

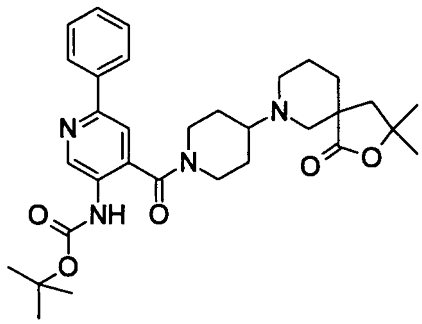

Using tert-butyl (6-chloro-4-{[4-(3,3-dimethyl-1-oxo-2-oxa-7-azaspiro[4.5]dec-7-yl)piperidin-1-yl]carbonyl}pyridin-3-yl)carbamate (250 mg, 0.480 mmol) obtained in Example 298 and phenylboronic acid (117 mg, 0.960 mmol), the title compound (270 mg, quantitative) was obtained by an operation similar to that of Reference Example 56 and trituration with diisopropyl ether.
EI(pos) 563 [M+H]+

Example 300

7-[1-(5-amino-2-phenylisonicotinoyl)piperidin-4-yl]-3,3-dimethyl-2-oxa-7-azaspiro[4.5]decan-1-one

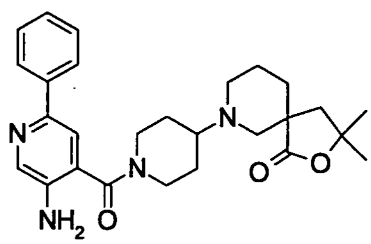

Using tert-butyl (4-{[4-(3,3-dimethyl-1-oxo-2-oxa-7-azaspiro[4.5]dec-7-yl)piperidin-1-yl]carbonyl}-6-phenylpyridin-3-yl)carbamate (270 mg, 0.480 mmol) obtained in Example 299, the title compound (198 mg, yield 89%) was obtained by an operation similar to that of Reference Example 49 and trituration with diisopropyl ether.
EI(pos) 463 [M+H]+

Example 301

N-(4-{[4-(3,3-dimethyl-1-oxo-2-oxa-7-azaspiro[4.5]dec-7-yl)piperidin-1-yl]carbonyl}-6-phenylpyridin-3-yl)-N'-ethylurea

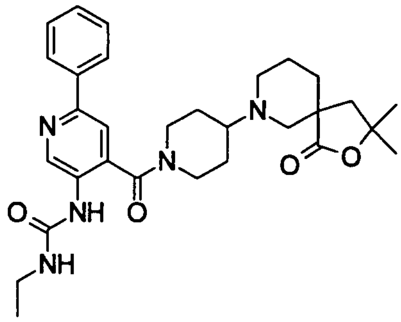

Using 7-[1-(5-amino-2-phenylisonicotinoyl)piperidin-4-yl]-3,3-dimethyl-2-oxa-7-azaspiro[4.5]decan-1-one (90 mg, 0.195 mmol) obtained in Example 300 and ethyl isocyanate (0.046 mL, 0.584 mmol), the title compound (57 mg, yield 55%) was obtained by an operation similar to that of Example 166 and trituration with diisopropyl ether.
EI(pos) 534 $[M+H]^+$

Example 302

benzyl 1-(4-{[4-(3,3-dimethyl-1-oxo-2-oxa-7-azaspiro[4.5]dec-7-yl)piperidin-1-yl]carbonyl}quinolin-2-yl)piperidine-4-carboxylate

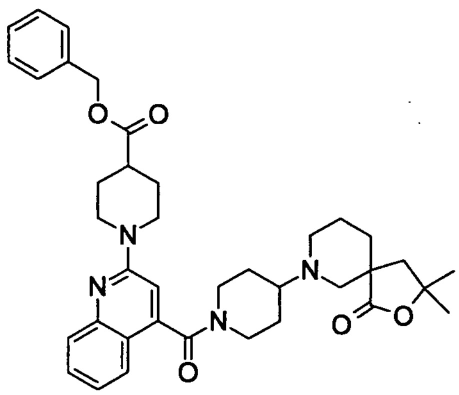

To 7-{1-[(2-chloroquinolin-4-yl)carbonyl]piperidin-4-yl}-3,3-dimethyl-2-oxa-7-azaspiro[4.5]decan-1-one (200 mg, 0.439 mmol) obtained in Example 33, benzyl piperidine-4-carboxylate hydrochloride (224 mg, 0.877 mmol) and potassium carbonate (182 mg, 1.32 mmol) was added dimethyl sulfoxide (2 mL), and the mixture was stirred at 80°C for 16 hr. The reaction mixture was diluted with ethyl acetate, washed with aqueous potassium carbonate solution and saturated brine, and dried over anhydrous sodium sulfate. The solvent was evaporated under reduced pressure, and the obtained residue was purified by silica gel column chromatography (developing solvent; hexane:ethyl acetate=1:1 → 0:1) to give the title compound (232 mg, yield 83%) as an oil.
EI(pos) 639 $[M+H]^+$

Example 303

1-(4-{[4-(3,3-dimethyl-1-oxo-2-oxa-7-azaspiro[4.5]dec-7-yl)piperidin-1-yl]carbonyl}quinolin-2-yl)piperidine-4-carboxylic acid

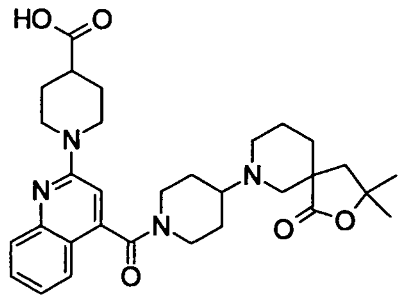

To benzyl 1-(4-{[4-(3,3-dimethyl-1-oxo-2-oxa-7-azaspiro[4.5]dec-7-yl)piperidin-1-yl]carbonyl}quinolin-2-yl)piperidine-4-carboxylate (232 mg, 0.363 mmol) obtained in Example 302 and 10% palladium carbon (500 mg) was added methanol (2 mL), and the mixture was stirred at room temperature for 3 hr under a hydrogen atmosphere. The catalyst was filtrated off through celite, and the filtrate was concentrated under reduced pressure. The obtained residue was triturated with diisopropyl ether to give the title compound (150 mg, yield 75%).
EI(pos) 549 $[M+H]^+$

Example 304

benzyl 1-(4-{[4-(3,3-dimethyl-1-oxo-2-oxa-7-azaspiro[4.5]dec-7-yl)piperidin-1-yl]carbonyl}quinolin-2-yl)-4-hydroxypiperidine-4-carboxylate

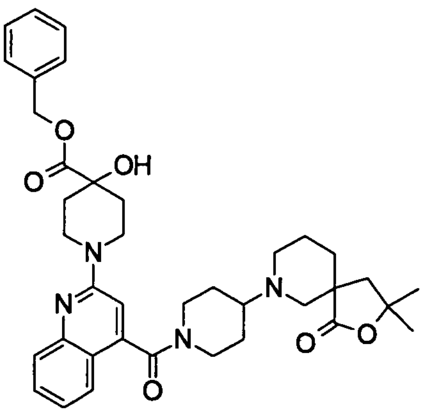

Using 7-{1-[(2-chloroquinolin-4-yl)carbonyl]piperidin-4-yl}-3,3-dimethyl-2-oxa-7-azaspiro[4.5]decan-1-one (200 mg, 0.439 mmol) obtained in Example 33 and benzyl 4-hydroxypiperidine-4-carboxylate hydrochloride (238 mg, 0.877 mmol) obtained in Reference Example 138, the title compound (212 mg, yield 74%) was obtained as an oil by an operation similar to that of Example 302.
EI(pos) 655 $[M+H]^+$

Example 305

1-(4-{[4-(3,3-dimethyl-1-oxo-2-oxa-7-azaspiro[4.5]dec-7-yl)piperidin-1-yl]carbonyl}quinolin-2-yl)-4-hydroxypiperidine-4-carboxylic acid

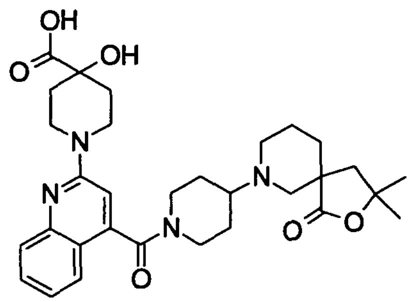

Using benzyl 1-(4-{ [4-(3,3-dimethyl-l-oxo-2-oxa-7-azaspiro[4.5]dec-7-yl)piperidin-1-yl]carbonyl}quinolin-2-yl)-4-hydroxypiperidine-4-carboxylate (212 mg, 0.324 mmol) obtained in Example 304, the title compound (133 mg, yield 73%) was obtained by an operation similar to that of Example 303 and trituration with diisopropyl ether.
EI(pos) 565 $[M+H]^+$

Example 306

7-(1-{[2-(4-hydroxypiperidin-1-yl)quinolin-4-yl]carbonyl}piperidin-4-yl)-3,3-dimethyl-2-oxa-7-azaspiro[4.5]decan-1-one

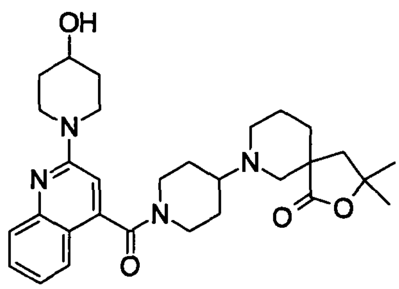

Using 7-{1-[(2-chloroquinolin-4-yl)carbonyl]piperidin-4-yl}-3,3-dimethyl-2-oxa-7-azaspiro[4.5]decan-1-one (97 mg, 0.213 mmol) obtained in Example 33 and piperidine-4-ol (43 mg, 0.425 mmol), the title compound (58 mg, yield 52%) was obtained by an operation similar to that of Example 302 and trituration with diisopropyl ether.
EI(pos) 521 $[M+H]^+$

Example 307

tert-butyl (3-{[4-(3-ethyl-2,4-dioxo-1-oxa-3,7-diazaspiro[4.5]dec-7-yl)piperidin-1-yl]carbonyl}-5-(pyridin-3-yl)-2-thienyl) carbamate

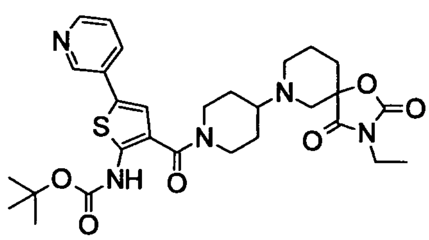

Using 3-ethyl-7-(piperidin-4-yl)-1-oxa-3,7-diazaspiro[4.5]decane-2,4-dione dihydrochloride (0.60 g, 1.69 mmol)

obtained in Reference Example 111 and 2-[(tert-butoxycarbonyl)amino]-5-(pyridin-3-yl)thiophene-3-carboxylic acid (543 mg, 1.69 mmol) obtained in Reference Example 142, the title compound (0.82 g, yield 83%) was obtained as an oil by an operation similar to that of Example 31.
EI(pos) 584 $[M+H]^+$

Example 308

N-ethyl-N'-(3-{[4-(3-ethyl-2,4-dioxo-1-oxa-3,7-diazaspiro[4.5]dec-7-yl)piperidin-1-yl]carbonyl}-5-(pyridin-3-yl)-2-thienyl) urea

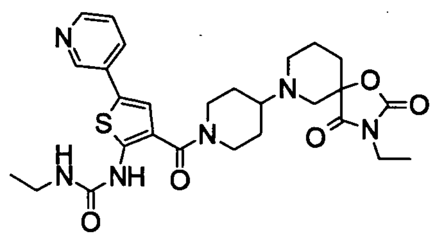

A solution of tert-butyl (3-{[4-(3-ethyl-2,4-dioxo-1-oxa-3,7-diazaspiro[4.5]dec-7-yl)piperidin-1-yl]carbonyl}-5-(pyridin-3-yl)-2-thienyl)carbamate (0.82 g, 1.41 mmol) obtained in Example 307 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 7-{1-[(2-amino-5-(pyridin-3-yl)-3-thienyl)carbonyl]piperidin-4-yl}-3-ethyl-1-oxa-3,7-diazaspiro[4.5]decane-2,4-dione as a crude product. Using the crude product, the title compound (0.47 g, yield 60%) was obtained as an oil by an operation similar to that of Example 166.
EI(pos) 555 $[M+H]^+$

Example 309

7-{1-[(2-amino-1-benzothien-3-yl)carbonyl]piperidin-4-yl}-3-isopropyl-1-oxa-3,7-diazaspiro[4.5]decane-2,4-dione

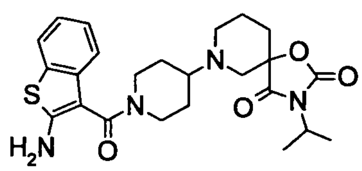

Using 3-isopropyl-7-(piperidin-4-yl)-1-oxa-3,7-diazaspiro[4.5]decane-2,4-dione dihydrochloride (0.60 g, 1.63 mmol) obtained in Reference Example 145 and 2-amino-1-benzothiophene-3-carboxylic acid (0.31 g, 1.63 mmol) obtained in Reference Example 51, the title compound (0.68 g, yield 88%) was obtained as an oil by an operation similar to that of Example 31.
EI(pos) 471 $[M+H]^+$

Example 310

N-ethyl-N'-(3-{[4-(3-isopropyl-2,4-dioxo-1-oxa-3,7-diazaspiro[4.5]dec-7-yl)piperidin-1-yl]carbonyl}-1-benzothien-2-yl) urea

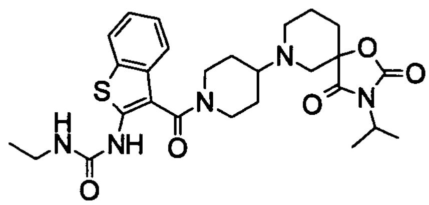

Using 7-{1-[(2-amino-1-benzothien-3-yl)carbonyl]piperidin-4-yl}-3-isopropyl-1-oxa-3,7-diazaspiro[4.5]decane-2,4-dione (0.68 g, 1.45 mmol) obtained in Example 309, the title compound (0.20 g, yield 37%) was obtained as an oil by an operation similar to that of Example 166.
EI(pos) 542 $[M+H]^+$

Example 311

N-(3-{[4-(1,3-dioxo-2,7-diazaspiro[4.5]dec-7-yl)piperidin-1-yl]carbonyl}-1-benzothien-2-yl)-N'-ethylurea

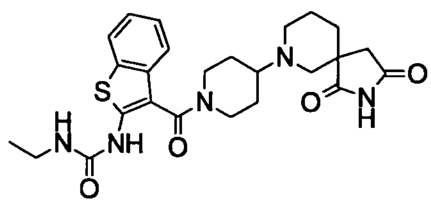

Using ethyl 3-(2-amino-2-oxoethyl)-1'-[(2-{[(ethylamino)carbonyl]amino}-1-benzothien-3-yl)carbonyl]-1,4'-bipiperidine-3-carboxylate (0.74 g, 1.36 mmol) obtained in Reference Example 149, the title compound (109 mg, yield 16%) was obtained as a yellow solid by an operation similar to that of Reference Example 20.
EI(pos) 498 $[M+H]^+$

Example 312

7-[1-(2-amino-5-(pyridin-2-yl)benzoyl)piperidin-4-yl]-3,3-dimethyl-2-oxa-7-azaspiro[4.5]decan-1-one

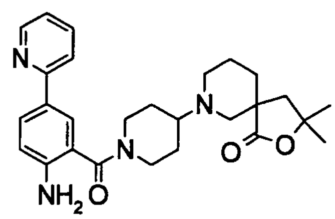

Using 7-[1-(2-amino-5-bromobenzoyl)piperidin-4-yl]-3,3-dimethyl-2-oxa-7-azaspiro[4.5]decan-1-one (1.31 g, 2.82 mmol) obtained in Example 145, the title compound (0.30 g, yield 23%) was obtained as an oil by an operation similar to that of Example 178.
EI(pos) 463 $[M+H]^+$

Example 313

N-(2-{[4-(3,3-dimethyl-1-oxo-2-oxa-7-azaspiro[4.5]dec-7-yl)piperidin-1-yl]carbonyl}-4-(pyridin-2-yl)phenyl)-N'-ethylu-

rea

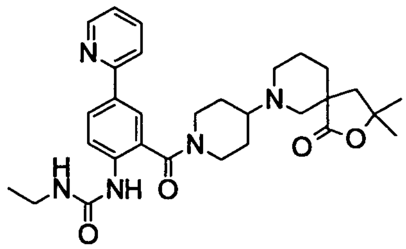

Using 7-[1-(2-amino-5-(pyridin-2-yl)benzoyl)piperidin-4-yl]-3,3-dimethyl-2-oxa-7-azaspiro[4.5]decan-1-one (0.30 g, 0.562 mmol) obtained in Example 312, the title compound (205 mg, yield 68%) was obtained as an oil by an operation similar to that of Example 166.
EI(pos) 534 $[M+H]^+$

Example 314

7-{1-[(2-amino-1-benzothien-3-yl)carbonyl]piperidin-4-yl}-3,3-dimethyl-2-oxa-7-azaspiro[4.5]decan-1-one

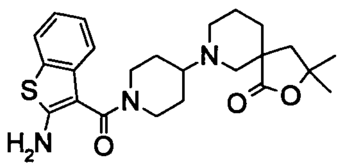

Using 3,3-dimethyl-7-(piperidin-4-yl)-2-oxa-7-azaspiro[4.5]decan-1-one dihydrochloride (0.80 g, 2.36 mmol) obtained in Reference Example 152, the title compound (1.04 g, yield 99%) was obtained as a yellow oil by an operation similar to that of Example 31.
EI(pos) 442 $[M+H]^+$

Example 315

N-(3-{[4-(3,3-dimethyl-1-oxo-2-oxa-7-azaspiro[4.5]dec-7-yl)piperidin-1-yl]carbonyl}-1-benzothien-2-yl)-N'-ethylurea

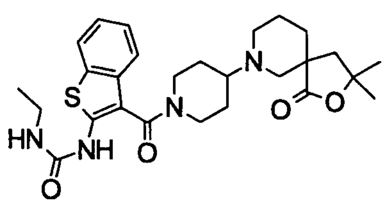

Using 7-{1-[(2-amino-1-benzothien-3-yl)carbonyl]piperidin-4-yl}-3,3-dimethyl-2-oxa-7-azaspiro[4.5]decan-1-one (1.04 g, 2.35 mmol) obtained in Example 314, the title compound (1.01 g, yield 83%) was obtained as a yellow oil by an operation similar to that of Example 166.
EI(pos) 513 $[M+H]^+$

Example 316

(-)-N-(3-{[4-(3,3-dimethyl-1-oxo-2-oxa-7-azaspiro[4.5]dec-7-yl)piperidin-1-yl]carbonyl}-1-benzothien-2-yl)-N'-ethylurea hydrochloride

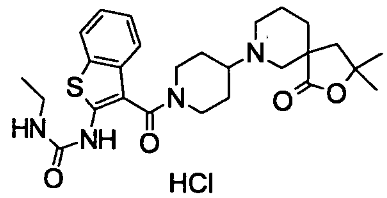

N-(3-{[4-(3,3-dimethyl-1-oxo-2-oxa-7-azaspiro[4.5]dec-7-yl)piperidin-1-yl]carbonyl}-1-benzothien-2-yl)-N'-ethylurea (112 mg, 0.218 mmol) obtained in Example 315 was dissolved in 4N hydrogen chloride-ethyl acetate (10 mL), and the mixture was stirred at room temperature for 30 min. The precipitated solid was collected by filtration, and washed with diethyl ether to give the title compound (99 mg, yield 83%) as a white solid.
EI(pos) 513 $[M+H]^+$
$[\alpha]_{D20}$ -9.9° (c1.04, MeOH)

Example 317

7-{1-[(2-amino-1-benzothien-3-yl)carbonyl]piperidin-4-yl}-3,3-dimethyl-2-oxa-7-azaspiro[4.5]decan-1-one

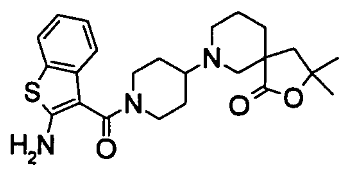

Using 3,3-dimethyl-7-(piperidin-4-yl)-2-oxa-7-azaspiro[4.5]decan-1-one dihydrochloride (0.72 g, 2.12 mmol) obtained in Reference Example 153, the title compound (0.93 g, yield 99%) was obtained as a yellow oil by an operation similar to that of Example 31.
EI(pos) 442 $[M+H]^+$

Example 318

N-(3-{[4-(3,3-dimethyl-1-oxo-2-oxa-7-azaspiro[4.5]dec-7-yl)piperidin-1-yl]carbonyl}-1-benzothien-2-yl)-N'-ethylurea

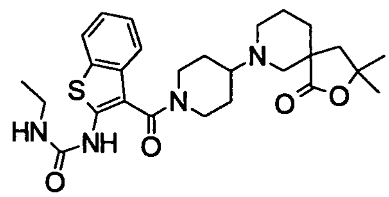

Using 7-{1-[(2-amino-1-benzothien-3-yl)carbonyl]piperidin-4-yl}-3,3-dimethyl-2-oxa-7-azaspiro[4.5]decan-1-one (0.93 g, 1.81 mmol) obtained in Example 317, the title compound (0.90 g, yield 97%) was obtained as a yellow oil

by an operation similar to that of Example 166.
EI (pos) 513 $[M+H]^+$

Example 319

(+)-N-(3-{[4-(3,3-dimethyl-1-oxo-2-oxa-7-azaspiro[4.5]dec-7-yl)piperidin-1-yl]carbonyl}-1-benzothien-2-yl)-N'-ethylurea hydrochloride

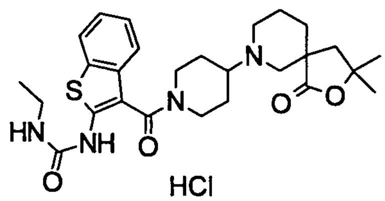

N-(3-{[4-(3,3-dimethyl-1-oxo-2-oxa-7-azaspiro[4.5]dec-7-yl)piperidin-1-yl]carbonyl}-1-benzothien-2-yl)-N'-ethylurea (114 mg, 0.222 mmol) obtained in Example 318 was dissolved in 4N hydrogen chloride-ethyl acetate (10 mL), and the mixture was stirred at room temperature for 30 min. The precipitated solid was collected by filtration, and washed with diethyl ether to give the title compound (116 mg, yield 95%) as a white solid.
EI(pos) 513 $[M+H]^+$
$[\alpha]_{D20}$ +9.2° (c0.95, MeOH)

Example 320

7-{1-[(2-amino-1-benzothien-3-yl)carbonyl]piperidin-4-yl}-2-ethyl-2,7-diazaspiro[4.5]decan-1-one

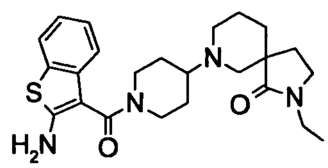

A solution (10 mL) of tert-butyl 4-(2-ethyl-1-oxo-2,7-diazaspiro[4.5]dec-7-yl)piperidine-1-carboxylate (1.57 g, 4.64 mmol) obtained in Reference Example 4 in 4N hydrogen chloride-ethyl acetate was stirred at room temperature for 30 min. The solvent was evaporated under reduced pressure, and the obtained solid was washed with diethyl ether to give a white solid (1.30 g). Using the white solid (0.65 g, 1.92 mmol), the title compound (0.77 g, yield 91%) was obtained as a yellow oil by an operation similar to that of Example 31.
EI(pos) 441 $[M+H]^+$

Example 321

N-ethyl-N'-(3-{[4-(2-ethyl-1-oxo-2,7-diazaspiro[4.5]dec-7-yl)piperidin-1-yl]carbonyl}-1-benzothien-2-yl)urea

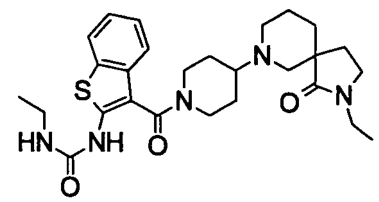

Using 7-{1-[(2-amino-1-benzothien-3-yl)carbonyl]piperidin-4-yl}-2-ethyl-2,7-diazaspiro[4.5]decan-1-one (0.77 g, 1.75 mmol) obtained in Example 320, the title compound (0.36 g, yield 40%) was obtained as a yellow oil by an operation similar to that of Example 166.
EI (pos) 512 $[M+H]^+$

Example 322

N-isopropyl-N'-(3-{[4-(3-isopropyl-2,4-dioxo-1-oxa-3,7-diazaspiro[4.5]dec-7-yl)piperidin-1-yl]carbonyl}-1-benzothien-2-yl)urea

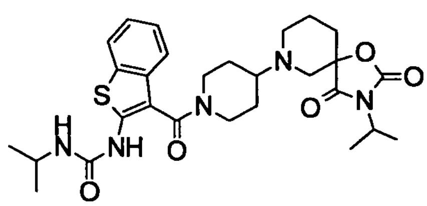

Using 7-{1-[(2-amino-1-benzothien-3-yl)carbonyl]piperidin-4-yl}-3-isopropyl-1-oxa-3,7-diazaspiro[4.5]decane-2,4-dione (0.68 g, 1.45 mmol) obtained in Example 309 and isopropyl isocyanate (0.16 mL, 1.62 mmol), the title compound (0.30 g, yield 54%) was obtained as an oil by an operation similar to that of Example 166.
EI (pos) 556 $[M+H]^+$

Example 323

N-ethyl-N'-(3-{[4-(3-isopropyl-2,4-dioxo-1-oxa-3,7-diazaspiro[4.5]dec-7-yl)piperidin-1-yl]carbonyl}-1-benzothien-2-yl) thiourea

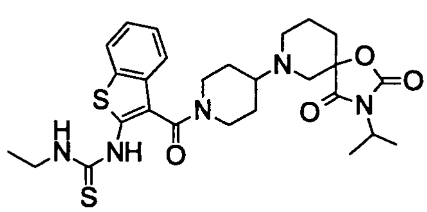

Using 7-{1-[(2-amino-1-benzothien-3-yl)carbonyl]piperidin-4-yl}-3-isopropyl-1-oxa-3,7-diazaspiro[4.5]decane-2,4-dione (0.68 g, 1.45 mmol) obtained in Example 309 and ethyl isothiocyanate (0.16 mL, 1.83 mmol), the title compound (83 mg, yield 16%) was obtained as an oil by an operation similar to that of Example 166.
EI(pos) 558 $[M+H]^+$

Example 324

N-(3-{[4-(3,3-dimethyl-1-oxo-2-oxa-7-azaspiro[4.5]dec-7-yl)piperidin-1-yl]carbonyl}-1-benzothien-2-yl)-N'-methylurea

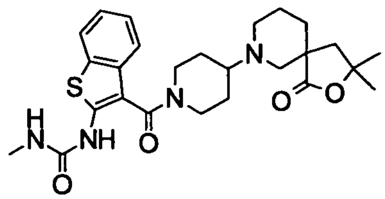

To a solution of 7-{1-[(2-amino-1-benzothien-3-yl)carbonyl]piperidin-4-yl}-3,3-dimethyl-2-oxa-7-azaspiro[4.5]decan-1-one (0.85 g, 1.93 mmol) obtained in Example 31 and triethylamine (0.32 mL, 2.31 mmol) in tetrahydrofuran (10 mL) was added 4-nitrophenyl chloroformate (0.47 g, 2.31 mmol) at room temperature, and the mixture was stirred for 30 min. A 2N methylamine-tetrahydrofuran solution (1.5 mL) was added, and the mixture was stirred at room temperature for 15 min. A saturated aqueous sodium hydrogencarbonate solution was added to the reaction mixture, and the mixture was extracted with ethyl acetate. The organic layer was washed with saturated brine, and dried over anhydrous magnesium sulfate. The solvent was evaporated under reduced pressure, and the obtained residue was purified by basic silica gel column chromatography (developing solvent; hexane:ethyl acetate=7:13→0:1) to give the title compound (172 mg, yield 18%).
EI(pos) 499 $[M+H]^+$

Example 325

tert-butyl [3-{[4-(3,3-dimethyl-1-oxo-2-oxa-7-azaspiro[4.5]dec-7-yl)piperidin-1-yl]carbonyl}-5-(3-methoxyphenyl)-2-thienyl]carbamate

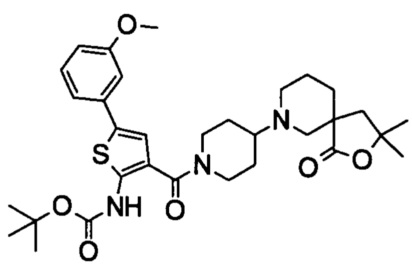

Using tert-butyl (5-bromo-3-{[4-(3,3-dimethyl-1-oxo-2-oxa-7-azaspiro[4.5]dec-7-yl)piperidin-1-yl]carbonyl}-2-thienyl)carbamate (0.77 g, 1.35 mmol) obtained in Example 170 and 3-methoxyphenylboronic acid (0.41 g, 2.70 mmol), the title compound (0.80 g, yield 99%) was obtained as an oil by an operation similar to that of Reference Example 84.
EI(pos) 598 $[M+H]^+$

Example 326

N-[3-{[4-(3,3-dimethyl-1-oxo-2-oxa-7-azaspiro[4.5]dec-7-yl)piperidin-1-yl]carbonyl}-5-(3-methoxyphenyl)-2-thienyl]-N'-ethylurea

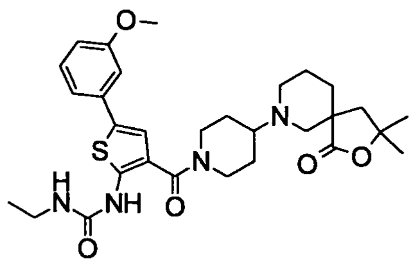

A solution of tert-butyl [3-{[4-(3,3-dimethyl-1-oxo-2-oxa-7-azaspiro[4.5]dec-7-yl)piperidin-1-yl]carbonyl}-5-(3-methoxyphenyl)-2-thienyl]carbamate (0.80 g, 1.34 mmol) obtained in Example 325 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 7-(1-{[2-amino-5-(3-methoxyphenyl)-3-thienyl]carbonyl}piperidin-4-yl)-3,3-dimethyl-2-oxa-7-azaspiro[4.5]decan-1-one as a crude product. Using the crude product, the title compound (0.49 g, yield 58%) was obtained as an oil by an operation similar to that of Example 166.
EI(pos) 569 $[M+H]^+$

Example 327

Optically active forms (two kinds) of N-ethyl-N'-(3-{[4-(3-ethyl-2,4-dioxo-1-oxa-3,7-diazaspiro[4.5]dec-7-yl)piperidin-1-yl]carbonyl}-1-benzothien-2-yl)urea

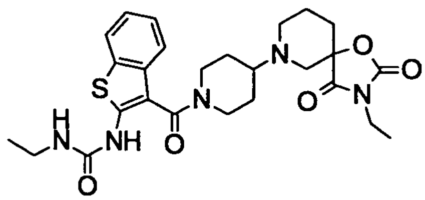

N-ethyl-N'-(3-{[4-(3-ethyl-2,4-dioxo-1-oxa-3,7-diazaspiro[4.5]dec-7-yl)piperidin-1-yl]carbonyl}-1-benzothien-2-yl)urea (racemate, 92 mg) obtained in Example 228 was separated by high performance liquid chromatography (column; CHIRALPAK AD (50 mmID × 500 mmL), temperature; 30°C, mobile phase; hexane:ethanol=1:4, flow rate; 60 mL/min, detection wavelength; 220 nm) to give the two title compounds [retention time, short (36 mg) and retention time, long (44 mg)].

Example 328

7-{1-[(2-amino-1-benzothien-3-yl)carbonyl]piperidin-4-yl}-3-ethyl-1,3,7-triazaspiro[4.5]decane-2,4-dione

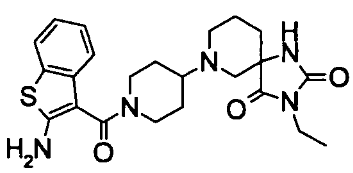

Using 3-ethyl-7-(piperidin-4-yl)-1,3,7-triazaspiro[4.5]decane-2,4-dione dihydrochloride (0.50 g, 1.42 mmol) obtained in Reference Example 156, the title compound (0.64 g, yield 99%) was obtained as a yellow oil by an operation

similar to that of Example 31.
EI(pos) 456 [M+H]$^+$

Example 329

N-ethyl-N'-(3-{[4-(3-ethyl-2,4-dioxo-1,3,7-triazaspiro[4.5]dec-7-yl)piperidin-1-yl]carbonyl}-1-benzothien-2-yl)urea

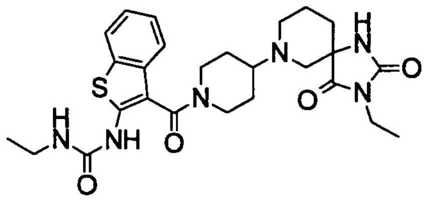

Using 7-{1-[(2-amino-1-benzothien-3-yl)carbonyl]piperidin-4-yl}-3-ethyl-1,3,7-triazaspiro[4.5]decane-2,4-dione (0.64 g, 1.40 mmol) obtained in Example 328, the title compound (0.62 g, yield 84%) was obtained as a yellow oil by an operation similar to that of Example 166.
EI(pos) 527 [M+H]$^+$

Example 330

tert-butyl (3-{[4-(3-ethyl-2,4-dioxo-1-oxa-3,7-diazaspiro[4.5]dec-7-yl)piperidin-1-yl]carbonyl}-5-(pyridin-2-yl)-2-thienyl) carbamate

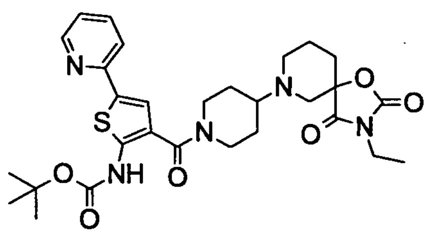

Using 3-ethyl-7-(piperidin-4-yl)-1-oxa-3,7-diazaspiro[4.5]decane-2,4-dione dihydrochloride (0.24 g, 0.687 mmol) obtained in Reference Example 159 and 2-[(tert-butoxycarbonyl)amino]-5-(pyridin-2-yl)thiophene-3-carboxylic acid (0.20 g, 0.629 mmol) obtained in Reference Example 103, the title compound (0.40 g, yield 99%) was obtained as an oil by an operation similar to that of Example 31.
EI(pos) 584 [M+H]$^+$.

Example 331

N-ethyl-N'-(3-{[4-(3-ethyl-2,4-dioxo-1-oxa-3,7-diazaspiro[4.5]dec-7-yl)piperidin-1-yl]carbonyl}-5-(pyridin-2-yl)-2-thienyl) urea

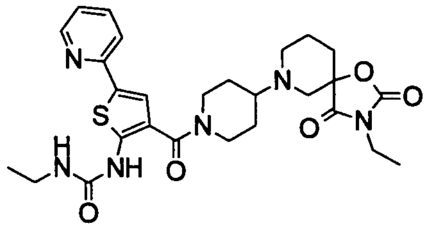

A solution of tert-butyl (3-{[4-(3-ethyl-2,4-dioxo-1-oxa-3,7-diazaspiro[4.5]dec-7-yl)piperidin-1-yl]carbonyl}-5-(pyridin-2-yl)-2-thienyl)carbamate (0.40 g, 0.686 mmol) obtained in Example 330 in trifluoroacetic acid (5 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 7-{1-[(2-amino-5-(pyridin-2-yl)-3-thienyl)carbonyl]piperidin-4-yl}-3-ethyl-1-oxa-3,7-diazaspiro[4.5]decane-2,4-dione as a crude product. Using the crude product, the title compound (380 mg, yield 99%) was obtained as an oil by an operation similar to that of Example 166.
EI(pos) 555 $[M+H]^+$

Example 332

tert-butyl (3-{[4-(3-ethyl-2,4-dioxo-1-oxa-3,7-diazaspiro[4.5]dec-7-yl)piperidin-1-yl]carbonyl}-5-(pyridin-2-yl)-2-thienyl) carbamate

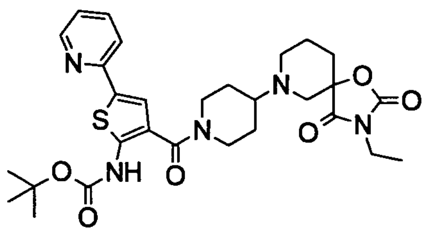

Using 3-ethyl-7-(piperidin-4-yl)-1-oxa-3,7-diazaspiro[4.5]decane-2,4-dione dihydrochloride (0.24 g, 0.687 mmol) obtained in Reference Example 161 and 2-[(tert-butoxycarbonyl)amino]-5-(pyridin-2-yl)thiophene-3-carboxylic acid (0.20 g, 0.629 mmol) obtained in Reference Example 103, the title compound (0.37 g, yield 92%) was obtained as an oil by an operation similar to that of Example 31.
EI(pos) 584 $[M+H]^+$

Example 333

N-ethyl-N'-(3-{[4-(3-ethyl-2,4-dioxo-1-oxa-3,7-diazaspiro[4.5]dec-7-yl)piperidin-1-yl]carbonyl}-5-(pyridin-2-yl)-2-thienyl) urea

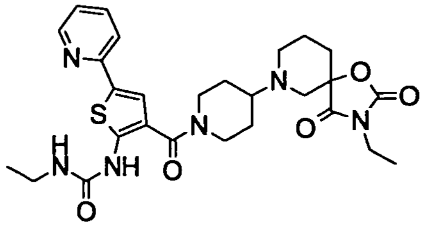

A solution of tert-butyl (3-{[4-(3-ethyl-2,4-dioxo-1-oxa-3,7-diazaspiro[4.5]dec-7-yl)piperidin-1-yl]carbonyl}-5-(pyridin-2-yl)-2-thienyl)carbamate (0.37 g, 0.634 mmol) obtained in Example 332 in trifluoroacetic acid (5 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 7-{1-[(2-amino-5-(pyridin-2-yl)-3-thienyl)carbonyl]piperidin-4-yl}-3-ethyl-1-oxa-3,7-diazaspiro[4.5]decane-2,4-dione as a crude product. Using the crude product, the title compound (305 mg, yield 99%) was obtained as an oil by an operation similar to that of Example 166.
EI(pos) 555 $[M+H]^+$

Example 334

7-[1-(9-anthrylcarbonyl)piperidin-4-yl]-3-cyclobutylmethyl-2-methyl-1,3,7-triazaspiro[4.5]dec-1-en-4-one

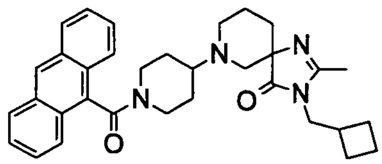

Using 7-[1-(9-anthrylcarbonyl)piperidin-4-yl]-2-methyl-1,3,7-triazaspiro[4.5]dec-1-en-4-one (295 mg, 0.649 mmol) obtained in Example 159 and (bromomethyl)cyclobutane (0.146 mL, 1.30 mmol), the title compound (171 mg, yield 50%) was obtained as an oil by an operation similar to that of Reference Example 100.
EI(pos) 523 $[M+H]^+$

Example 335

N-(3-{[4-(3-isopropyl-2,4-dioxo-1-oxa-3,7-diazaspiro[4.5] dec-7-yl)piperidin-1-yl]carbonyl}-1-benzothien-2-yl)urea

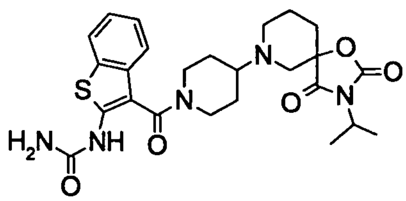

Using 7-{1-[(2-amino-1-benzothien-3-yl)carbonyl]piperidin-4-yl}-3-isopropyl-1-oxa-3,7-diazaspiro[4.5]decane-2,4-dione (0.58 g, 1.23 mmol) obtained in Example 309, the title compound (0.33 g, yield 52%) was obtained as an oil by an operation similar to that of Example 290.
EI(pos) 514 $[M+H]^+$

Example 336

tert-butyl (3-{[4-(3-isopropyl-2,4-dioxo-1-oxa-3,7-diazaspiro[4.5]dec-7-yl)piperidin-1-yl]carbonyl}-5-(pyridin-2-yl)-2-thienyl)carbamate

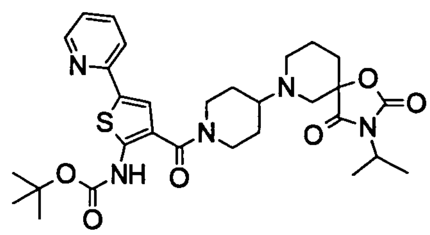

Using 3-isopropyl-7-(piperidin-4-yl)-1-oxa-3,7-diazaspiro[4.5]decane-2,4-dione dihydrochloride (0.40 g, 1.09 mmol) obtained in Reference Example 145 and 2-[(tert-butoxycarbonyl)amino]-5-(pyridin-2-yl)thiophene-3-carboxylic acid (0.20 g, 0.629 mmol) obtained in Reference Example 103, the title compound (0.65 g, yield 99%) was obtained as an oil by an operation similar to that of Example 31.
EI(pos) 598 $[M+H]^+$

Example 337

N-ethyl-N'-(3-{[4-(3-isopropyl-2,4-dioxo-1-oxa-3,7-diazaspiro[4.5]dec-7-yl)piperidin-1-yl]carbonyl}-5-(pyridin-2-yl)-2-thienyl)urea

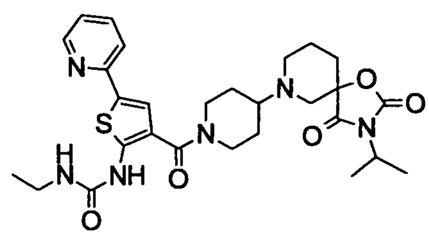

A solution of tert-butyl (3-{[4-(3-isopropyl-2,4-dioxo-1-oxa-3,7-diazaspiro[4.5]dec-7-yl)piperidin-1-yl]carbonyl}-5-(pyridin-2-yl)-2-thienyl)carbamate (0.65 g, 1.08 mmol) obtained in Example 336 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 7-{1-[(2-amino-5-(pyridin-2-yl)-3-thienyl)carbonyl]piperidin-4-yl}-3-isopropyl-1-oxa-3,7-diazaspiro[4.5]decane-2,4-dione as a crude product. Using the crude product, the title compound (0.55 g, yield 89%) was obtained as an oil by an operation similar to that of Example 166.
EI(pos) 569 $[M+H]^+$

Example 338

N-(3-{[4-(3-isopropyl-2,4-dioxo-1-oxa-3,7-diazaspiro[4.5]dec-7-yl)piperidin-1-yl]carbonyl}-1-benzothien-2-yl)-N'-methylurea

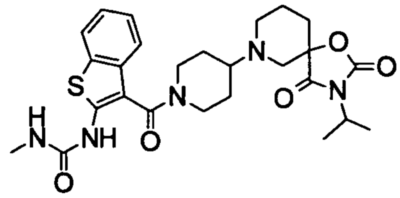

Using 7-{1-[(2-amino-1-benzothien-3-yl)carbonyl]piperidin-4-yl}-3-isopropyl-1-oxa-3,7-diazaspiro[4.5]decane-2,4-dione (0.28 g, 0.595 mmol) obtained in Example 309, the title compound (131 mg, yield 41%) was obtained as an oil by an operation similar to that of Example 324.
EI(pos) 528 $[M+H]^+$

Example 339

N-(3-{[4-(2-ethyl-1-oxo-2,7-diazaspiro[4.5]dec-7-yl)piperidin-1-yl]carbonyl}-1-benzothien-2-yl)-N'-methylurea

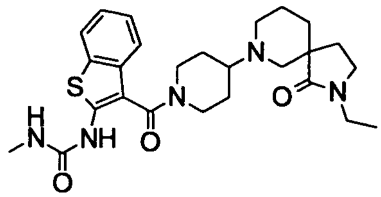

Using 7-{1-[(2-amino-1-benzothien-3-yl)carbonyl]piperidin-4-yl}-2-ethyl-2,7-diazaspiro[4.5]decan-1-one (0.52 g, 1.18 mmol) obtained in Example 320, the title compound (111 mg, yield 18%) was obtained as a yellow oil by an operation similar to that of Example 324.
EI(pos) 498 $[M+H]^+$

Example 340

Optically active forms (two kinds) of 1-(3-{[4-(2-ethyl-1,3-dioxo-2,7-diazaspiro[4.5]dec-7-yl)piperidin-1-yl]carbonyl}-1-benzothien-2-yl)urea

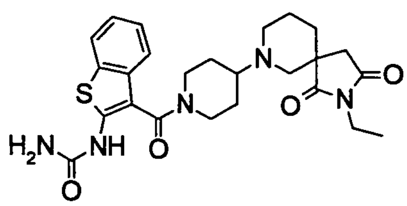

1-(3-{[4-(2-Ethyl-1,3-dioxo-2,7-diazaspiro[4.5]dec-7-yl)piperidin-1-yl]carbonyl}-1-benzothien-2-yl)urea (racemate, 250 mg) obtained in Example 240 was separated by high performance liquid chromatography (column; CHIRALCEL OD (50 mmID × 500 mmL), temperature; 30°C, mobile phase; hexane:ethanol=1:1, flow rate; 60 mL/min, detection wavelength; 220 nm) to give the two title compounds [retention time, short (90 mg) and retention time, long (91 mg)].

Example 341

7-{1-[(2-amino-1-benzothien-3-yl)carbonyl]piperidin-4-yl}-3-propyl-1-oxa-3,7-diazaspiro[4.5]decane-2,4-dione

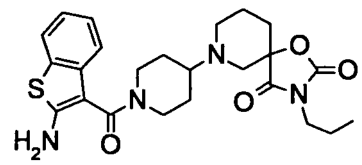

Using 7-(piperidin-4-yl)-3-propyl-1-oxa-3,7-diazaspiro[4.5]decane-2,4-dione dihydrochloride (0.80 g, 2.17 mmol) obtained in Reference Example 163 and 2-amino-1-benzothiophene-3-carboxylic acid (0.42 g, 2.17 mmol) obtained in Reference Example 51, the title compound (0.67 g, yield 65%) was obtained as an oil by an operation similar to that of Example 31.
EI(pos) 471 [M+H]$^+$

Example 342

N-(3-{[4-(2,4-dioxo-3-propyl-1-oxa-3,7-diazaspiro[4.5]dec-7-yl)piperidin-1-yl]carbonyl}-1-benzothien-2-yl)-N'-methylurea

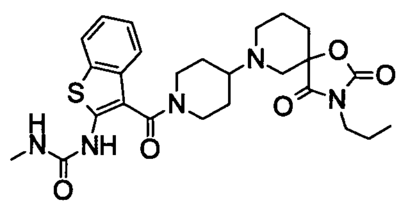

Using 7-{1-[(2-amino-1-benzothien-3-yl)carbonyl]piperidin-4-yl}-3-propyl-1-oxa-3,7-diazaspiro[4.5]decane-2,4-dione (335 mg, 0.712 mmol) obtained in Example 341, the title compound (146 mg, yield 39%) was obtained as a yellow oil by an operation similar to that of Example 324.
EI(pos) 528 [M+H]$^+$

Example 343

N-(3-{[4-(2,4-dioxo-3-propyl-1-oxa-3,7-diazaspiro[4.5]dec-7-yl)piperidin-1-yl]carbonyl}-1-benzothien-2-yl)urea

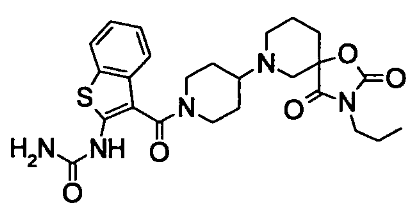

Using 7-{1-[(2-amino-1-benzothien-3-yl)carbonyl]piperidin-4-yl}-3-propyl-1-oxa-3,7-diazaspiro[4.5]decane-2,4-dione (0.55 g, 1.17 mmol) obtained in Example 341, the title compound (112 mg, yield 19%) was obtained as an oil by an operation similar to that of Example 290.
EI(pos) 514 [M+H]$^+$

Example 344

7-{1-[(2-amino-1-benzothien-3-yl)carbonyl]piperidin-4-yl}-2-methyl-2,7-diazaspiro[4.5]decan-1-one

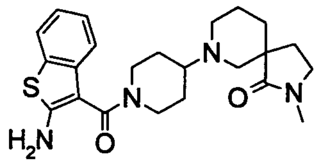

Using 2-methyl-7-(piperidin-4-yl)-2,7-diazaspiro[4.5]decan-1-one dihydrochloride (0.80 g, 2.47 mmol) obtained in Reference Example 166 and 2-amino-1-benzothiophene-3-carboxylic acid (0.48 g, 2.47 mmol) obtained in Reference Example 51, the title compound (0.99 g, yield 94%) was obtained as an oil by an operation similar to that of Example 31.
EI(pos) 427 $[M+H]^+$

Example 345

N-methyl-N'-(3-{[4-(2-methyl-1-oxo-2,7-diazaspiro[4.5]dec-7-yl)piperidin-1-yl]carbonyl}-1-benzothien-2-yl)urea

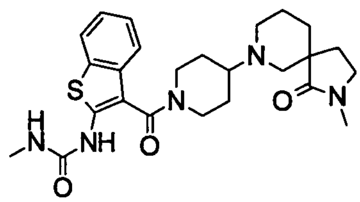

Using 7-{1-[(2-amino-1-benzothien-3-yl)carbonyl]piperidin-4-yl}-2-methyl-2,7-diazaspiro[4.5]decan-1-one (445 mg, 1.04 mmol) obtained in Example 344, the title compound (68 mg, yield 13%) was obtained as a yellow oil by an operation similar to that of Example 324.
EI(pos) 484 $[M+H]^+$

Example 346

7-{1-[(2-amino-1-benzothien-3-yl)carbonyl]piperidin-4-yl}-3-ethyl-1-oxa-3,7-diazaspiro[4.5]decane-2,4-dione

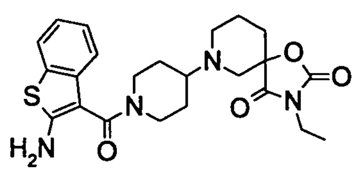

Using 3-ethyl-7-(piperidin-4-yl)-1-oxa-3,7-diazaspiro[4.5]decane-2,4-dione dihydrochloride (0.40 g, 1.13 mmol) obtained in Reference Example 161 and 2-amino-1-benzothiophene-3-carboxylic acid (0.22 g, 1.13 mmol) obtained in Reference Example 51, the title compound (435 mg, yield 84%) was obtained as an oil by an operation similar to that of Example 31.
EI(pos) 457 $[M+H]^+$

Example 347

N-(3-{[4-(3-ethyl-2,4-dioxo-1-oxa-3,7-diazaspiro[4.5]dec-7-yl)piperidin-1-yl]carbonyl}-1-benzothien-2-yl)-N'-methylurea

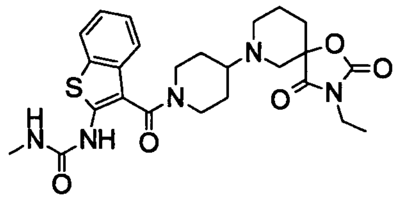

Using 7-{1-[(2-amino-1-benzothien-3-yl)carbonyl]piperidin-4-yl}-3-ethyl-1-oxa-3,7-diazaspiro[4.5]decane-2,4-dione (435 mg, 0.953 mmol) obtained in Example 346, the title compound (181 mg, yield 37%) was obtained as a yellow oil by an operation similar to that of Example 324.
EI(pos) 514 $[M+H]^+$

Example 348

N-ethyl-N'-(3-{[4-(2-methyl-1-oxo-2,7-diazaspiro[4.5]dec-7-yl)piperidin-1-yl]carbonyl}-1-benzothien-2-yl)urea

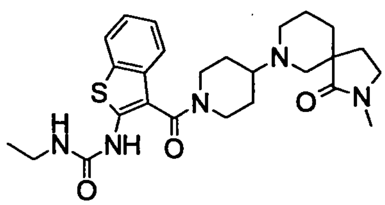

Using 7-{1-[(2-amino-1-benzothien-3-yl)carbonyl]piperidin-4-yl}-2-methyl-2,7-diazaspiro[4.5]decan-1-one (0.51 g, 1.20 mmol) obtained in Example 344, the title compound (313 mg, yield 52%) was obtained as a yellow oil by an operation similar to that of Example 166.
EI(pos) 498 $[M+H]^+$

Example 349

7-{1-[(2-amino-1-benzothien-3-yl)carbonyl]piperidin-4-yl}-3-tert-butyl-1-oxa-3,7-diazaspiro[4.5]decane-2,4-dione

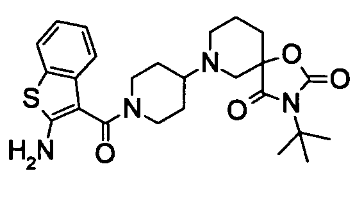

Using tert-butyl 4-(3-tert-butyl-2,4-dioxo-1-oxa-3,7-diazaspiro[4.5]dec-7-yl)piperidine-1-carboxylate (525 mg, 1.28 mmol) obtained in Reference Example 167, the title compound (0.59 g, yield 95%) was obtained as an oil by successively performing operations similar to those of Reference Example 53 and Example 31.
EI(pos) 485 $[M+H]^+$

Example 350

N-(3-{[4-(3-tert-butyl-2,4-dioxo-1-oxa-3,7-diazaspiro[4.5]dec-7-yl)piperidin-1-yl]carbonyl}-1-benzothien-2-yl)-N'-ethylurea

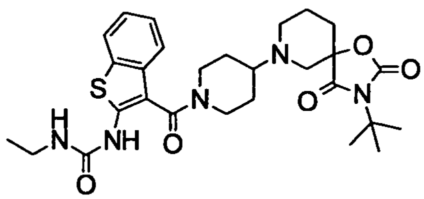

Using 7-{1-[(2-amino-1-benzothien-3-yl)carbonyl]piperidin-4-yl}-3-tert-butyl-1-oxa-3,7-diazaspiro[4.5]decane-2,4-dione (0.59 g, 1.22 mmol) obtained in Example 349, the title compound (524 mg, yield 77%) was obtained as an oil by an operation similar to that of Example 166.
EI(pos) 556 $[M+H]^+$

Example 351

7-{1-[(2-amino-1-benzothien-3-yl)carbonyl]piperidin-4-yl}-2-propyl-2,7-diazaspiro[4.5]decan-1-one

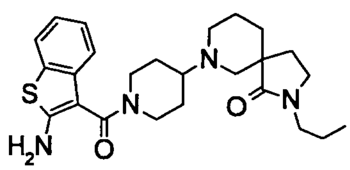

Using tert-butyl 4-(1-oxo-2-propyl-2,7-diazaspiro[4.5]dec-7-yl)piperidine-1-carboxylate (0.95 g, 2.51 mmol) obtained in Reference Example 169, the title compound (0.54 g, yield 47%) was obtained as an oil by successively performing operations similar to those of Reference Example 53 and Example 31.
EI(pos) 455 $[M+H]^+$

Example 352

N-(3-{[4-(1-oxo-2-propyl-2,7-diazaspiro[4.5]dec-7-yl)piperidin-1-yl]carbonyl}-1-benzothien-2-yl)urea

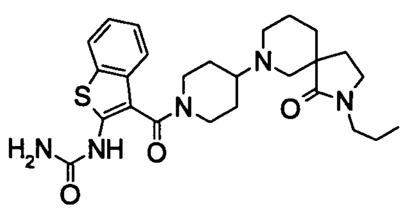

Using 7-{1-[(2-amino-1-benzothien-3-yl)carbonyl]piperidin-4-yl}-2-propyl-2,7-diazaspiro[4.5]decan-1-one (0.54 g, 1.19 mmol) obtained in Example 351, the title compound (305 mg, yield 51%) was obtained as an oil by an operation similar to that of Example 290.
EI(pos) 498 $[M+H]^+$

Example 353

7-{1-[(2-amino-1-benzothien-3-yl)carbonyl]piperidin-4-yl}-2,3-diethyl-1,3,7-triazaspiro[4.5]dec-1-en-4-one

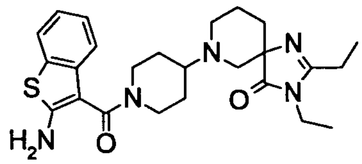

Using tert-butyl 4-(2,3-diethyl-4-oxo-1,3,7-triazaspiro[4.5]dec-1-en-7-yl)piperidine-1-carboxylate (0.93 g, 2.37 mmol) obtained in Reference Example 173, the title compound (0.48 g, yield 43%) was obtained as an oil by successively performing operations similar to those of Reference Example 53 and Example 31.
EI(pos) 468 $[M+H]^+$

Example 354

N-(3-{[4-(2,3-diethyl-4-oxo-1,3,7-triazaspiro[4.5]dec-1-en-7-yl)piperidin-1-yl]carbonyl}-1-benzothien-2-yl)-N'-ethylurea

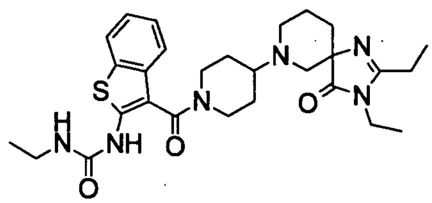

Using 7-{1-[(2-amino-1-benzothien-3-yl)carbonyl]piperidin-4-yl}-2,3-diethyl-1,3,7-triazaspiro[4.5]dec-1-en-4-one (0.35 g, 0.748 mmol) obtained in Example 353, the title compound (284 mg, yield 70%) was obtained as an oil by an operation similar to that of Example 166.
EI(pos) 539 $[M+H]^+$

Example 355

N-(3-{[4-(2,3-diethyl-4-oxo-1,3,7-triazaspiro[4.5]dec-1-en-7-yl)piperidin-1-yl]carbonyl}-1-benzothien-2-yl)urea

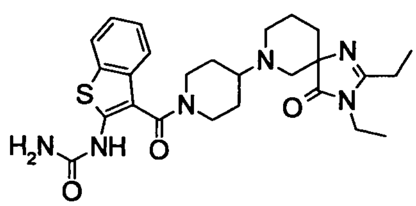

Using 7-{1-[(2-amino-1-benzothien-3-yl)carbonyl]piperidin-4-yl}-2,3-diethyl-1,3,7-triazaspiro[4.5]dec-1-en-4-one (0.13 g, 0.278 mmol) obtained in Example 353, the title compound (110 mg, yield 77%) was obtained as an oil by an operation similar to that of Example 290.
EI (pos) 512 $[M+H]^+$

Example 356

7-{1-[(2-amino-1-benzothien-3-yl)carbonyl]piperidin-4-yl}-3-ethyl-2-isopropyl-1,3,7-triazaspiro[4.5]dec-1-en-4-one

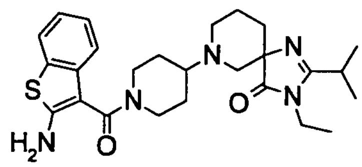

Using tert-butyl 4-(3-ethyl-2-isopropyl-4-oxo-1,3,7-triazaspiro[4.5]dec-1-en-7-yl)piperidine-1-carboxylate (0.74 g, 1.82 mmol) obtained in Reference Example 177, the title compound (0.70 g, yield 80%) was obtained as an oil by successively performing operations similar to those of Reference Example 53 and Example 31.
EI(pos) 482 $[M+H]^+$

Example 357

N-(3-{[4-(3-ethyl-2-isopropyl-4-oxo-1,3,7-triazaspiro[4.5]dec-1-en-7-yl)piperidin-1-yl]carbonyl}-1-benzothien-2-yl)urea

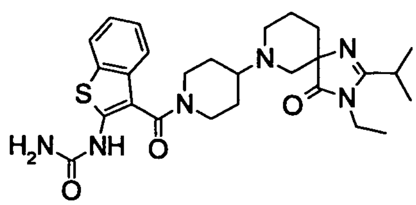

Using 7-{1-[(2-amino-1-benzothien-3-yl)carbonyl]piperidin-4-yl}-3-ethyl-2-isopropyl-1,3,7-triazaspiro[4.5]dec-1-en-4-one (0.70 g, 1.46 mmol) obtained in Example 356, the title compound (109 mg, yield 14%) was obtained as an oil by an operation similar to that of Example 290.
EI(pos) 525 $[M+H]^+$

Example 358

7-{1-[(2-amino-1-benzothien-3-yl)carbonyl]piperidin-4-yl}-2-isopropyl-2,7-diazaspiro[4.5]decan-1-one

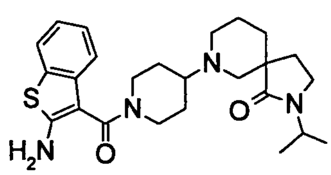

Using tert-butyl 4-(2-isopropyl-1-oxo-2,7-diazaspiro[4.5]dec-7-yl)piperidine-1-carboxylate (0.69 g, 1.82 mmol) obtained in Reference Example 179, the title compound (414 mg, yield 50%) was obtained as an oil by successively performing operations similar to those of Reference Example 53 and Example 31.
EI(pos) 455 $[M+H]^+$

Example 359

N-(3-{[4-(2-isopropyl-1-oxo-2,7-diazaspiro[4.5]dec-7-yl)piperidin-1-yl]carbonyl}-1-benzothien-2-yl)urea

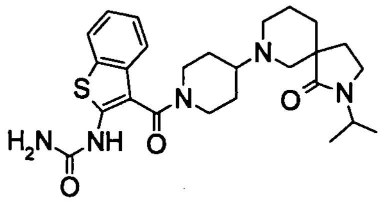

Using 7-{1-[(2-amino-1-benzothien-3-yl)carbonyl]piperidin-4-yl}-2-isopropyl-2,7-diazaspiro[4.5]decan-1-one (0.28 g, 0.616 mmol) obtained in Example 358, the title compound (215 mg, yield 70%) was obtained as an oil by an operation similar to that of Example 290.
EI(pos) 498 $[M+H]^+$

Example 360

N-(3-{[4-(2-isopropyl-1-oxo-2,7-diazaspiro[4.5]dec-7-yl)piperidin-1-yl]carbonyl}-1-benzothien-2-yl)-N'-methylurea

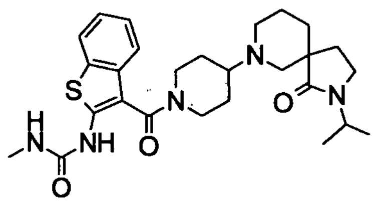

Using 7-{1-[(2-amino-1-benzothien-3-yl)carbonyl]piperidin-4-yl}-2-isopropyl-2,7-diazaspiro[4.5]decan-1-one (134 mg, 0.295 mmol) obtained in Example 358 and methyl isocyanate (0.035 mL, 0.587 mmol), the title compound (82 mg, yield 70%) was obtained as an oil by an operation similar to that of Example 166.
EI(pos) 512 $[M+H]^+$

Example 361

7-{1-[(2-amino-1-benzothien-3-yl)carbonyl]piperidin-4-y1}-2-ethyl-2,7-diazaspiro[4.5]decane-1,3-dione

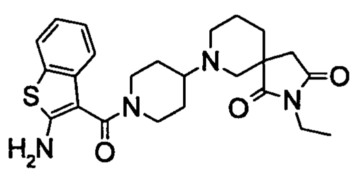

Using 2-amino-1-benzothiophene-3-carboxylic acid (305 mg, 1.58 mmol) obtained in Reference Example 51 and 2-ethyl-7-(piperidin-4-yl)-2,7-diazaspiro[4.5]decane-1,3-dione dihydrochloride (530 mg, 1.50 mmol) obtained in Reference Example 184, the title compound (501 mg, yield 73%) was obtained by an operation similar to that of Example 31 and trituration with diisopropyl ether.

EI(pos) 455 $[M+H]^+$

Example 362

N-ethyl-N'-(3-{[4-(2-ethyl-1,3-dioxo-2,7-diazaspiro[4.5]dec-7-yl)piperidin-1-yl]carbonyl}-1-benzothien-2-yl)urea

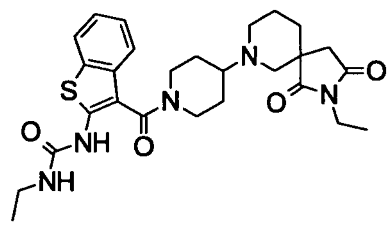

Using 7-{1-[(2-amino-1-benzothien-3-yl)carbonyl]piperidin-4-yl}-2-ethyl-2,7-diazaspiro[4.5]decane-1,3-dione (100 mg, 0.220 mmol) obtained in Example 361 and ethyl isocyanate (0.052 mL, 0.660 mmol), the title compound (88.0 mg, yield 76%) was obtained by an operation similar to that of Example 166 and trituration with diisopropyl ether. EI (pos) 526 $[M+H]^+$

Example 363

N-(3-{[4-(2-ethyl-1,3-dioxo-2,7-diazaspiro[4.5]dec-7-yl)piperidin-1-yl]carbonyl}-1-benzothien-2-yl)-N'-methylurea

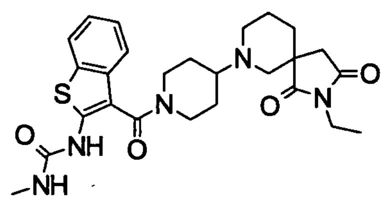

Using 7-{1-[(2-amino-1-benzothien-3-yl)carbonyl]piperidin-4-yl}-2-ethyl-2,7-diazaspiro[4.5]decane-1,3-dione (92.0 mg, 0.202 mmol) obtained in Example 361 and methyl isocyanate (0.036 mL, 0.606 mmol), the title compound (77.5 mg, yield 75%) was obtained by an operation similar to that of Example 166 and trituration with diisopropyl ether. EI (pos) 512 $[M+H]^+$

Example 364

7-{1-[(2-amino-1-benzothien-3-yl)carbonyl]piperidin-4-yl}-2-isopropyl-2,7-diazaspiro[4.5]decane-1,3-dione

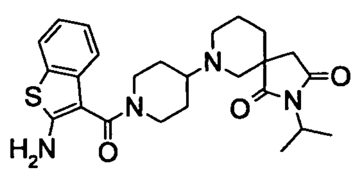

Using 2-amino-1-benzothiophene-3-carboxylic acid (323 mg, 1.67 mmol) obtained in Reference Example 51 and 2-isopropyl-7-(piperidin-4-yl)-2,7-diazaspiro[4.5]decane-1,3-dione dihydrochloride (583 mg, 1.59 mmol) obtained

in Reference Example 187, the title compound (541 mg, yield 73%) was obtained by an operation similar to that of Example 31 and trituration with diisopropyl ether and hexane.
EI(pos) 469 [M+H]$^+$

Example 365

N-ethyl-N'-(3-{[4-(2-isopropyl-1,3-dioxo-2,7-diazaspiro[4.5]dec-7-yl)piperidin-1-yl]carbonyl}-1-benzothien-2-yl)urea

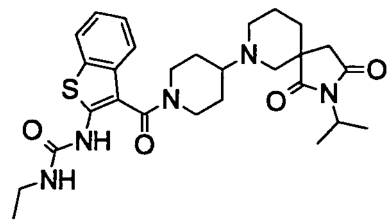

Using 7-{1-[(2-amino-1-benzothien-3-yl)carbonyl]piperidin-4-yl}-2-isopropyl-2,7-diazaspiro[4.5]decane-1,3-dione (100 mg, 0.213 mmol) obtained in Example 364 and ethyl isocyanate (0.051 mL, 0.640 mmol), the title compound (73.7 mg, yield 64%) was obtained by an operation similar to that of Example 166 and trituration with diisopropyl ether.
EI(pos) 540 [M+H]$^+$

Example 366

N-(3-{[4-(2-isopropyl-1,3-dioxo-2,7-diazaspiro[4.5]dec-7-yl)piperidin-1-yl]carbonyl}-1-benzothien-2-yl)urea

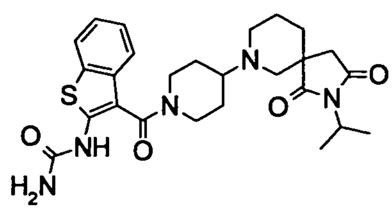

Using 7-{1-[(2-amino-1-benzothien-3-yl)carbonyl]piperidin-4-yl}-2-isopropyl-2,7-diazaspiro[4.5]decane-1,3-dione (297 mg, 0.634 mmol) obtained in Example 364 and trichloroacetyl isocyanate (0.151 mL, 1.27 mmol), the title compound (227 mg, yield 70%) was obtained by an operation similar to that of Example 290 and trituration with diisopropyl ether.
EI(pos) 512 [M+H]$^+$

Example 367

7-{1-[(2-amino-1-benzothien-3-yl)carbonyl]piperidin-4-yl}-2-methoxy-2,7-diazaspiro[4.5]decane-1,3-dione

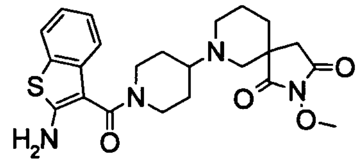

Using 2-amino-1-benzothiophene-3-carboxylic acid (172 mg, 0.889 mmol) obtained in Reference Example 51 and 2-methoxy-7-(piperidin-4-yl)-2,7-diazaspiro[4.5]decane-1,3-dione dihydrochloride (300 mg, 0.847 mmol) obtained in Reference Example 189, the title compound (301 mg, yield 78%) was obtained by an operation similar to that of Example 31 and trituration with diisopropyl ether and hexane.
EI(pos) 457 [M+H]$^+$

Example 368

N-ethyl-N'-(3-{[4-(2-methoxy-1,3-dioxo-2,7-diazaspiro[4.5]dec-7-yl)piperidin-1-yl]carbonyl}-1-benzothien-2-yl)urea

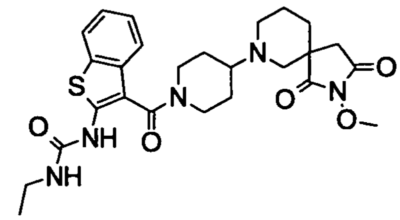

Using 7-{1-[(2-amino-1-benzothien-3-yl)carbonyl]piperidin-4-yl}-2-methoxy-2,7-diazaspiro[4.5]decane-1,3-dione (150 mg, 0.329 mmol) obtained in Example 367 and ethyl isocyanate (0.078 mL, 0.986 mmol), the title compound (134 mg, yield 78%) was obtained by an operation similar to that of Example 166 and trituration with diisopropyl ether.
EI(pos) 528 [M+H]$^+$

Example 369

tert-butyl (3-{[4-(2-isopropyl-1,3-dioxo-2,7-diazaspiro[4.5]dec-7-yl)piperidin-1-yl]carbonyl}-5-(pyridin-2-yl)-2-thienyl) carbamate

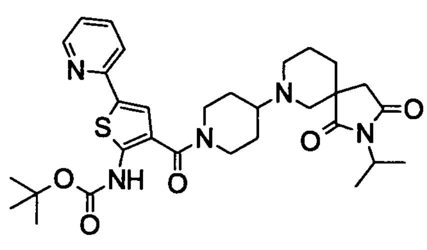

Using 2-isopropyl-7-(piperidin-4-yl)-2,7-diazaspiro[4.5]decane-1,3-dione dihydrochloride (191 mg, 0.521 mmol) obtained in Reference Example 187 and 2-[(tert-butoxycarbonyl)amino]-5-(pyridin-2-yl)thiophene-3-carboxylic acid (167 mg, 0.521 mmol) obtained in Reference Example 103, the title compound (271 mg, yield 87%) was obtained as an oil by an operation similar to that of Example 31.
EI(pos) 596 [M+H]$^+$

Example 370

N-(3-{[4-(2-isopropyl-1,3-dioxo-2,7-diazaspiro[4.5]dec-7-yl)piperidin-1-yl]carbonyl}-5-(pyridin-2-yl)-2-thienyl)urea

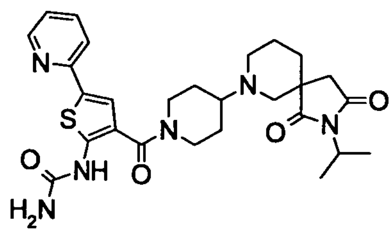

A solution of tert-butyl (3-{[4-(2-isopropyl-1,3-dioxo-2,7-diazaspiro[4.5]dec-7-yl)piperidin-1-yl]carbonyl}-5-(pyridin-2-yl)-2-thienyl)carbamate (271 mg, 0.455 mmol) obtained in Example 369 in trifluoroacetic acid (2 mL) was stirred at room temperature for 1 hr. The reaction mixture was neutralized with aqueous potassium carbonate solution, extracted twice with ethyl acetate, and the organic layer was dried over anhydrous sodium sulfate. The solvent was evaporated under reduced pressure to give 7-{1-[(2-amino-5-(pyridin-2-yl)-3-thienyl)carbonyl]piperidin-4-yl}-2-isopropyl-2,7-diazaspiro[4.5]decane-1,3-dione (198 mg, 88%) as a crude product. Using the crude product (198 mg, 0.399 mmol) and trichloroacetyl isocyanate (0.095 mL, 0.799 mmol), the title compound (148 mg, yield 69%) was obtained by an operation similar to that of Example 290 and trituration with diisopropyl ether.
EI(pos) 539 $[M+H]^+$

Example 371

tert-butyl (3-{[4-(2-cyclopropyl-1,3-dioxo-2,7-diazaspiro[4.5]dec-7-yl)piperidin-1-yl]carbonyl}-5-(pyridin-2-yl)-2-thienyl) carbamate

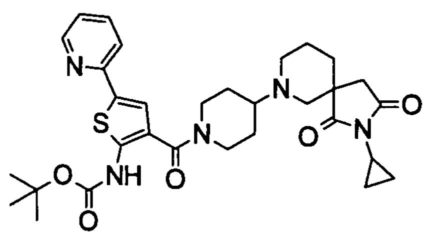

Using 2-cyclopropyl-7-(piperidin-4-yl)-2,7-diazaspiro[4.5]decane-1,3-dione dihydrochloride (167 mg, 0.458 mmol) obtained in Reference Example 192 and 2-[(tert-butoxycarbonyl)amino]-5-(pyridin-2-yl)thiophene-3-carboxylic acid (147 mg, 0.458 mmol) obtained in Reference Example 103, the title compound (265 mg, yield 97%) was obtained as an oil by an operation similar to that of Example 31.
EI(pos) 594 $[M+H]^+$

Example 372

N-(3-{[4-(2-cyclopropyl-1,3-dioxo-2,7-diazaspiro[4.5]dec-7-yl)piperidin-1-yl]carbonyl}-5-(pyridin-2-yl)-2-thienyl)urea

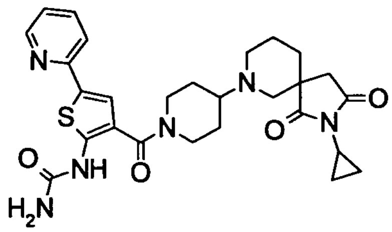

A solution of tert-butyl (3-{[4-(2-cyclopropyl-1,3-dioxo-2,7-diazaspiro[4.5]dec-7-yl)piperidin-1-yl]carbonyl}-5-(pyridin-2-yl)-2-thienyl)carbamate (264 mg, 0.445 mmol) obtained in Example 371 in trifluoroacetic acid (2 mL) was stirred at room temperature for 1 hr. The reaction mixture was neutralized with aqueous potassium carbonate solution, extracted twice with ethyl acetate, and the organic layer was dried over anhydrous sodium sulfate. The solvent was evaporated under reduced pressure to give 7-{1-[(2-amino-5-(pyridin-2-yl)-3-thienyl)carbonyl]piperidin-4-yl}-2-cyclopropyl-2,7-diazaspiro[4.5]decane-1,3-dione (192 mg, 88%) as a crude product. Using the crude product (192 mg, 0.389 mmol) and trichloroacetyl isocyanate (0.093 mL, 0.778 mmol), the title compound (114 mg, yield 55%) was obtained by an operation similar to that of Example 290 and trituration with diisopropyl ether.
EI(pos) 537 $[M+H]^+$

Example 373

7-{1-[(2-amino-1-benzothien-3-yl)carbonyl]piperidin-4-yl}-2-cyclopropyl-2,7-diazaspiro[4.5]decane-1,3-dione

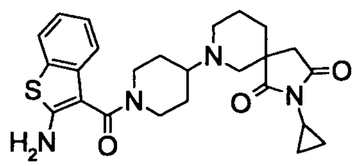

Using 2-amino-1-benzothiophene-3-carboxylic acid (85.9 mg, 0.445 mmol) obtained in Reference Example 51 and 2-cyclopropyl-7-(piperidin-4-yl)-2,7-diazaspiro[4.5]decane-1,3-dione dihydrochloride (162 mg, 0.445 mmol) obtained in Reference Example 192, the title compound (155 mg, yield 75%) was obtained by an operation similar to that of Example 31 and trituration with diisopropyl ether and hexane.
EI(pos) 467 $[M+H]^+$

Example 374

N-(3-{[4-(2-cyclopropyl-1,3-dioxo-2,7-diazaspiro[4.5]dec-7-yl)piperidin-1-yl]carbonyl}-1-benzothien-2-yl)urea

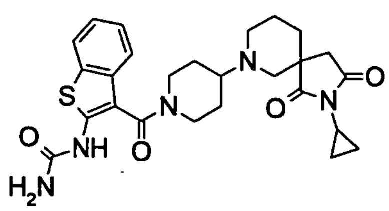

Using 7-{1-[(2-amino-1-benzothien-3-yl)carbonyl]piperidin-4-yl}-2-cyclopropyl-2,7-diazaspiro[4.5]decane-1,3-dione (155 mg, 0.332 mmol) obtained in Example 373 and trichloroacetyl isocyanate (0.079 mL, 0.664 mmol), the title compound (105 mg, yield 62%) was obtained by an operation similar to that of Example 290 and trituration with diisopropyl

ether.
EI(pos) 510 [M+H]+

Example 375

tert-butyl (3-{[4-(2-cyclobutyl-1,3-dioxo-2,7-diazaspiro[4.5]dec-7-yl)piperidin-1-yl]carbonyl}-5-(pyridin-2-yl)-2-thienyl) carbamate

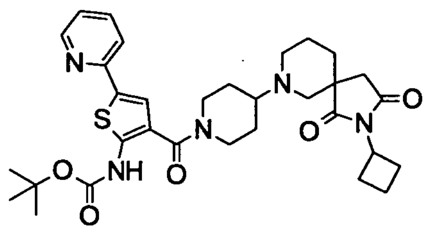

Using 2-cyclobutyl-7-(piperidin-4-yl)-2,7-diazaspiro[4.5]decane-1,3-dione dihydrochloride (168 mg, 0.444 mmol) obtained in Reference Example 195 and 2-[(tert-butoxycarbonyl)amino]-5-(pyridin-2-yl)thiophene-3-carboxylic acid (142 mg, 0.444 mmol) obtained in Reference Example 103, the title compound (270 mg, quantitative) was obtained as an oil by an operation similar to that of Example 31.
EI(pos) 608 [M+H]+

Example 376

N-(3-{[4-(2-cyclobutyl-1,3-dioxo-2,7-diazaspiro[4.5]dec-7-yl)piperidin-1-yl]carbonyl}-5-(pyridin-2-yl)-2-thienyl)urea

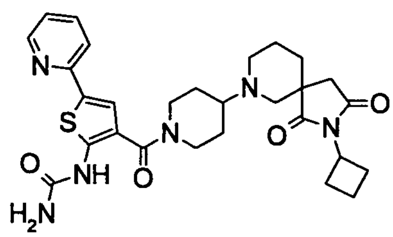

A solution of tert-butyl (3-{[4-(2-cyclobutyl-1,3-dioxo-2,7-diazaspiro[4.5]dec-7-yl)piperidin-1-yl]carbonyl}-5-(pyridin-2-yl)-2-thienyl)carbamate (270 mg, 0.444 mmol) obtained in Example 375 in trifluoroacetic acid (2 mL) was stirred at room temperature for 1 hr. The reaction mixture was neutralized with aqueous potassium carbonate solution, extracted twice with ethyl acetate, and the organic layer was dried over anhydrous sodium sulfate. The solvent was evaporated under reduced pressure to give 7-{1-[(2-amino-5-(pyridin-2-yl)-3-thienyl)carbonyl]piperidin-4-yl}-2-cyclobutyl-2,7-diazaspiro[4.5]decane-1,3-dione (178 mg, 79%) as a crude product. Using the crude product (178 mg, 0.351 mmol) and trichloroacetyl isocyanate (0.084 mL, 0.701 mmol), the title compound (107 mg, yield 55%) was obtained by an operation similar to that of Example 290 and trituration with diisopropyl ether.
EI(pos) 551 [M+H]+

Example 377

tert-butyl (3-{[4-(2-methyl-1,3-dioxo-2,7-diazaspiro[4.5]dec-7-yl)piperidin-1-yl]carbonyl}-5-(pyridin-2-yl)-2-thienyl)carbamate

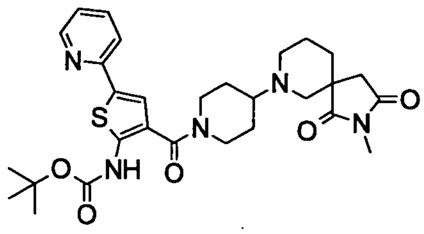

Using 2-methyl-7-(piperidin-4-yl)-2,7-diazaspiro[4.5]decane-1,3-dione dihydrochloride (146 mg, 0.432 mmol) obtained in Reference Example 198 and 2-[(tert-butoxycarbonyl)amino]-5-(pyridin-2-yl)thiophene-3-carboxylic acid (138 mg, 0.432 mmol) obtained in Reference Example 103, the title compound (211 mg, yield 86%) was obtained as an oil by an operation similar to that of Example 31.
EI(pos) 568 $[M+H]^+$

Example 378

N-ethyl-N'-(3-{[4-(2-methyl-1,3-dioxo-2,7-diazaspiro[4.5]dec-7-yl)piperidin-1-yl]carbonyl}-5-(pyridin-2-yl)-2-thienyl)urea

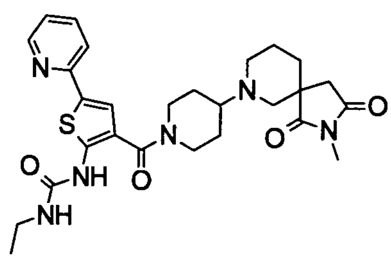

A solution of tert-butyl (3-{[4-(2-methyl-1,3-dioxo-2,7-diazaspiro[4.5]dec-7-yl)piperidin-1-yl]carbonyl}-5-(pyridin-2-yl)-2-thienyl)carbamate(211 mg, 0.370 mmol) obtained in Example 377 in trifluoroacetic acid (2 mL) was stirred at room temperature for 1 hr. The reaction mixture was neutralized with aqueous potassium carbonate solution, extracted twice with ethyl acetate, and the organic layer was dried over anhydrous sodium sulfate. The solvent was evaporated under reduced pressure to give 7-{1-[(2-amino-5-(pyridin-2-yl)-3-thienyl)carbonyl]piperidin-4-yl}-2-methyl-2,7-diazaspiro[4.5]decane-1,3-dione (151 mg, 87%) as a crude product. Using the crude product (151 mg, 0.323 mmol) and ethyl isocyanate (0.077 mL, 0.969 mmol), the title compound (132 mg, yield 76%) was obtained by an operation similar to that of Example 166 and trituration with diisopropyl ether. EI(pos) 539 $[M+H]^+$

Example 379

7-{1-[(2-amino-1-benzothien-3-yl)carbonyl]piperidin-4-yl}-2-methyl-2,7-diazaspiro[4.5]decane-1,3-dione

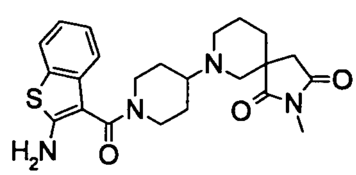

Using 2-amino-1-benzothiophene-3-carboxylic acid (77.7 mg, 0.402 mmol) obtained in Reference Example 51 and 2-methyl-7-(piperidin-4-yl)-2,7-diazaspiro[4.5]decane-1,3-dione dihydrochloride (136 mg, 0.402 mmol) obtained in Reference Example 198, the title compound (158 mg, yield 90%) was obtained as an oil by an operation similar to that

of Example 31.
EI(pos) 441 [M+H]$^+$

Example 380

N-ethyl-N'-(3-{[4-(2-methyl-1,3-dioxo-2,7-diazaspiro[4.5]dec-7-yl)piperidin-1-yl]carbonyl}-1-benzothien-2-yl)urea

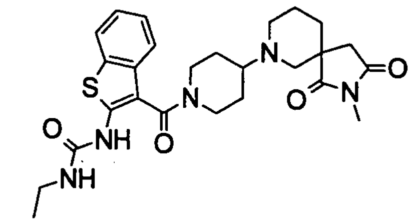

Using 7-{1-[(2-amino-1-benzothien-3-yl)carbonyl]piperidin-4-yl}-2-methyl-2,7-diazaspiro[4.5]decane-1,3-dione (158 mg, 0.359 mmol) obtained in Example 379 and ethyl isocyanate (0.085 mL, 1.08 mmol), the title compound (124 mg, yield 68%) was obtained by an operation similar to that of Example 166 and trituration with diisopropyl ether.
EI(pos) 512 [M+H]$^+$

Example 381

7-{1-[(2-amino-1-benzothien-3-yl)carbonyl]piperidin-4-yl}-3-ethyl-1-thia-3,7-diazaspiro[4.5]decane-2,4-dione

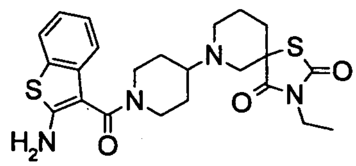

Using 2-amino-1-benzothiophene-3-carboxylic acid (127 mg, 0.656 mmol) obtained in Reference Example 51 and 3-ethyl-7-(piperidin-4-yl)-1-thia-3,7-diazaspiro[4.5]decane-2,4-dione dihydrochloride (243 mg, 0.656 mmol) obtained in Reference Example 205, the title compound (282 mg, yield 91%) was obtained by an operation similar to that of Example 31 and trituration with diisopropyl ether and hexane.
EI(pos) 473 [M+H]$^+$

Example 382

N-ethyl-N'-(3-{[4-(3-ethyl-2,4-dioxo-1-thia-3,7-diazaspiro[4.5]dec-7-yl)piperidin-1-yl]carbonyl}-1-benzothien-2-yl)urea

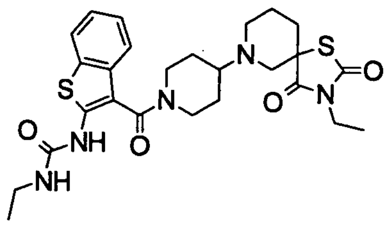

Using 7-{1-[(2-amino-1-benzothien-3-yl)carbonyl]piperidin-4-yl}-3-ethyl-1-thia-3,7-diazaspiro[4.5]decane-2,4-dione (132 mg, 0.279 mmol) obtained in Example 381 and ethyl isocyanate (0.066 mL, 0.838 mmol), the title compound (136 mg, yield 89%) was obtained by an operation similar to that of Example 166 and trituration with diisopropyl ether.
EI(pos) 544 $[M+H]^+$

Example 383

N-(3-{[4-(3-ethyl-2,4-dioxo-1-thia-3,7-diazaspiro[4.5]dec-7-yl)piperidin-1-yl]carbonyl}-1-benzothien-2-yl)urea

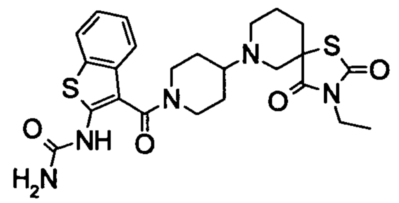

Using 7-{1-[(2-amino-1-benzothien-3-yl)carbonyl]piperidin-4-yl}-3-ethyl-1-thia-3,7-diazaspiro[4.5]decane-2,4-dione (143 mg, 0.303 mmol) obtained in Example 381 and trichloroacetyl isocyanate (0.072 mL, 0.605 mmol), the title compound (124 mg, yield 80%) was obtained by an operation similar to that of Example 290 and trituration with diisopropyl ether.
EI(pos) 516 $[M+H]^+$

Example 384

7-{1-[2-chloro-6-(4-hydroxypiperidin-1-yl)isonicotinoyl]piperidin-4-yl}-3,3-dimethyl-2-oxa-7-azaspiro[4.5]decan-1-one

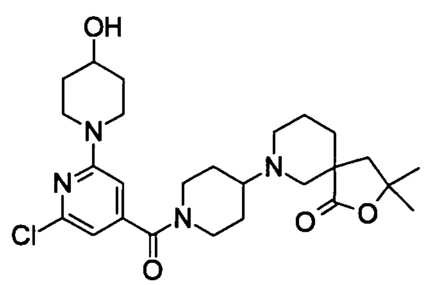

Using 2-chloro-6-(4-hydroxypiperidin-1-yl)isonicotinic acid (500 mg, 1.95 mmol) obtained in Reference Example 206 and 3,3-dimethyl-7-(piperidin-4-yl)-2-oxa-7-azaspiro[4.5]decan-1-one dihydrochloride (661 mg, 1.95 mmol) obtained in Reference Example 53, the title compound (585 mg, yield 60%) was obtained by an operation similar to that of Example 31 and trituration with diisopropyl ether.
EI(pos) 505 $[M+H]^+$

Example 385

7-{1-[2-(4-hydroxypiperidin-1-yl)-6-phenylisonicotinoyl]piperidin-4-yl}-3,3-dimethyl-2-oxa-7-azaspiro[4.5]decan-1-one

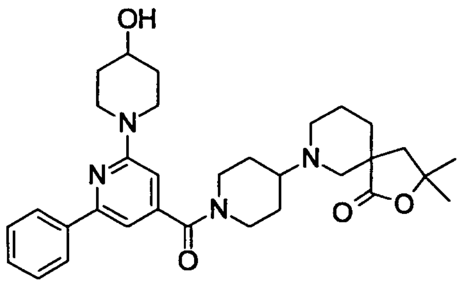

Using 7-{1-[2-chloro-6-(4-hydroxypiperidin-1-yl)isonicotinoyl]piperidin-4-yl}-3,3-dimethyl-2-oxa-7-azaspiro [4.5]decan-1-one (180 mg, 0.356 mmol) obtained in Example 384 and phenylboronic acid (86.9 mg, 0.713 mmol), the title compound (174 mg, yield 89%) was obtained by an operation similar to that of Reference Example 56 and trituration with diisopropyl ether.
EI(pos) 547 $[M+H]^+$

Example 386

7-(1-{[6-(4-hydroxypiperidin-1-yl)-2,3'-bipyridin-4-yl]carbonyl}piperidin-4-yl)-3,3-dimethyl-2-oxa-7-azaspiro[4.5]decan-1-one

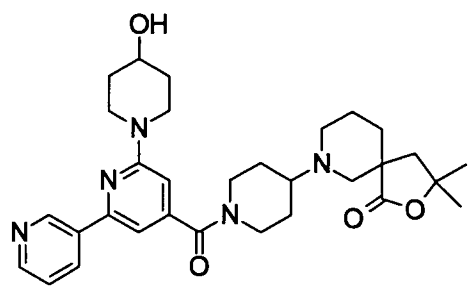

Using 7-{1-[2-chloro-6-(4-hydroxypiperidin-1-yl)isonicotinoyl]piperidin-4-yl}-3,3-dimethyl-2-oxa-7-azaspiro [4.5]decan-1-one (150 mg, 0.297 mmol) obtained in Example 384 and 3-pyridineboronic acid (73.0 mg, 0.594 mmol), the title compound (112 mg, yield 69%) was obtained by an operation similar to that of Reference Example 56 and trituration with diisopropyl ether.
EI(pos) 548 $[M+H]^+$

Example 387

7-(1-{[2-(4-methoxypiperidin-1-yl)quinolin-4-yl]carbonyl}piperidin-4-yl)-3,3-dimethyl-2-oxa-7-azaspiro[4.5]decan-1-one

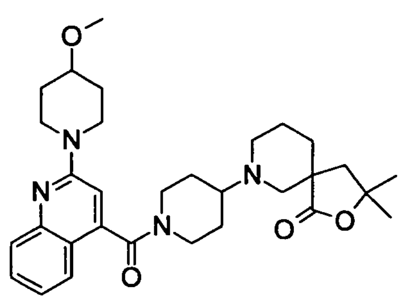

Using 7-{1-[(2-bromoquinolin-4-yl)carbonyl]piperidin-4-yl}-3,3-dimethyl-2-oxa-7-azaspiro[4.5]decan-1-one (118 mg, 0.236 mmol) obtained in Example 293 and 4-methoxypiperidinehydrochloride (71.5 mg, 0.472 mmol), the title compound (96.8 mg, yield 77%) was obtained by an operation similar to that of Example 302 and trituration with diisopropyl ether and hexane.
EI(pos) 535 [M+H]$^+$

Example 388

7-(1-{[2-(1,4-dioxa-8-azaspiro[4.5]deca-8-yl)quinolin-4-yl]carbonyl}piperidin-4-yl)-3,3-dimethyl-2-oxa-7-azaspiro[4.5]decan-1-one

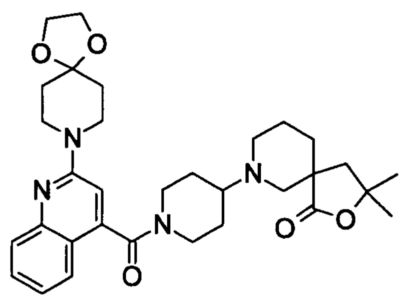

Using 7-{1-[(2-bromoquinolin-4-yl)carbonyl]piperidin-4-yl}-3,3-dimethyl-2-oxa-7-azaspiro[4.5]decan-1-one (138 mg, 0.276 mmol) obtained in Example 293 and 1,4-dioxa-8-azaspiro[4.5]decane (0.071 mL, 0.552 mmol), the title compound (155 mg, quantitative) was obtained as an oil by an operation similar to that of Example 302.
EI(pos) 563 [M+H]$^+$

Example 389

3,3-dimethyl-7-(1-{[2-(4-oxopiperidin-1-yl)quinolin-4-yl]carbonyl}piperidin-4-yl)-2-oxa-7-azaspiro[4.5]decan-1-one

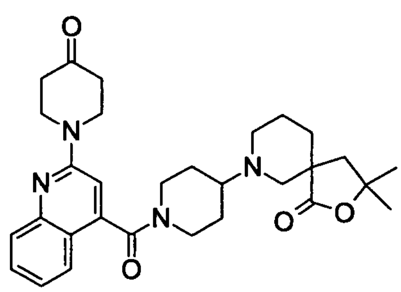

To 7-(1-{[2-(1,4-dioxa-8-azaspiro[4.5]deca-8-yl)quinolin-4-yl]carbonyl}piperidin-4-yl)-3,3-dimethyl-2-oxa-7-azaspiro[4.5]decan-1-one (155 mg, 0.276 mmol) obtained in Example 388 was added 1N hydrochloric acid (2 mL), and the mixture was stirred at 40°C for 16 hr. The reaction mixture was diluted with ethyl acetate, washed with aqueous potassium carbonate solution and saturated brine, and dried over anhydrous sodium sulfate. The solvent was evaporated under reduced pressure, and the obtained residue was purified by silica gel column chromatography (developing solvent; hexane:ethyl acetate=1:1→0:1) and triturated with diisopropyl ether to give the title compound (95.5 mg, yield 67%).
EI(pos) 519 [M+H] $^+$

Example 390

3,3-dimethyl-7-[1-(1-naphthoyl)piperidin-4-yl]-2-oxa-7-azaspiro[4.5]decan-1-one

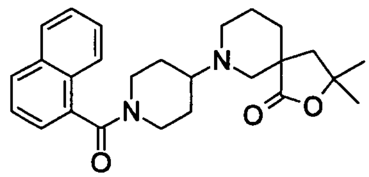

To a solution of 3,3-dimethyl-7-(piperidin-4-yl)-2-oxa-7-azaspiro[4.5]decan-1-one dihydrochloride(167 mg, 0.492 mmol) obtained in Reference Example 53 and triethylamine (0.24 mL, 1.72 mmol) in THF (5 mL) was added 1-naphthoyl chloride (103 mg, 0.541 mmol), and the mixture was stirred at room temperature for 2 hr. The reaction mixture was diluted with ethyl acetate, washed with aqueous potassium carbonate solution and saturated brine, and dried over anhydrous sodium sulfate. The solvent was evaporated under reduced pressure, and the obtained residue was purified by basic silica gel column chromatography (developing solvent; ethyl acetate), and purified by silica gel column chromatography (developing solvent; hexane:ethyl acetate=1:1→0:1) to give the title compound (150 mg, yield 73%).
EI(pos) 421 $[M+H]^+$

Example 391

7-[1-(1-benzothien-3-ylcarbonyl)piperidin-4-yl]-3,3-dimethyl-2-oxa-7-azaspiro[4.5]decan-1-one hydrochloride

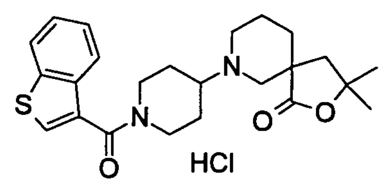

Using 1-benzothiophene-3-carboxylic acid (78.7 mg, 0.442 mmol) and 3,3-dimethyl-7-(piperidin-4-yl)-2-oxa-7-azaspiro[4.5]decan-1-one dihydrochloride (150 mg, 0.442 mmol) obtained in Reference Example 53, a free form of the title compound was obtained by an operation similar to that of Reference Example 31. To the obtained free form was added 4N hydrogen chloride-ethyl acetate (0.2 mL), followed by trituration with diisopropyl ether to give the title compound (167 mg, yield 81%).
EI (pos) 427 $[M+H]^+$

Example 392

3,3-dimethyl-7-[1-(1-naphthylsulfonyl)piperidin-4-yl]-2-oxa-7-azaspiro[4.5]decan-1-one

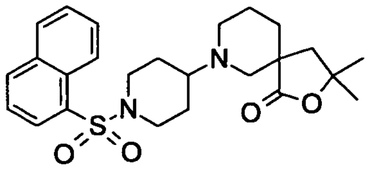

To a solution of 3,3-dimethyl-7-(piperidin-4-yl)-2-oxa-7-azaspiro[4.5]decan-1-one dihydrochloride(128 mg, 0.377 mmol) obtained in Reference Example 53 and triethylamine (0.184 mL, 1.32 mmol) in THF (5 mL) was added naphthalene-1-sulfonyl chloride (94.1 mg, 0.415 mmol), and the mixture was stirred at room temperature for 2 hr. The reaction mixture was diluted with ethyl acetate, washed with aqueous potassium carbonate solution and saturated brine, and dried over anhydrous sodium sulfate. The solvent was evaporated under reduced pressure, and the obtained residue was purified by basic silica gel column chromatography (developing solvent; ethyl acetate), and purified by silica gel column chromatography (developing solvent; hexane:ethyl acetate=3:1→0:1) to give the title compound (98.3 mg, yield 57%).
EI(pos) 457 $[M+H]^+$

Example 393

7-[1-(1-benzothien-3-ylsulfonyl)piperidin-4-yl]-3,3-dimethyl-2-oxa-7-azaspiro[4.5]decan-1-one

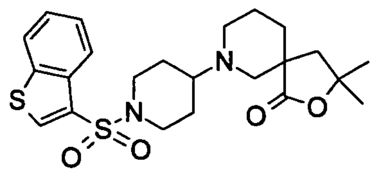

To a solution of 3,3-dimethyl-7-(piperidin-4-yl)-2-oxa-7-azaspiro[4.5]decan-1-one dihydrochloride (150 mg, 0.442 mmol) obtained in Reference Example 53 and triethylamine (0.215 mL, 1.55 mmol) in THF (2 mL) was added 1-benzothiophene-3-sulfonyl chloride (103 mg, 0.442 mmol), and the mixture was stirred at room temperature for 3 hr. The reaction mixture was diluted with ethyl acetate, washed with aqueous potassium carbonate solution and saturated brine, and dried over anhydrous sodium sulfate. The solvent was evaporated under reduced pressure, and the obtained residue was purified by basic silica gel column chromatography (developing solvent; ethyl acetate) to give the title compound (180 mg, yield 88%).
EI(pos) 463 $[M+H]^+$

Example 394

7-[1-(9-anthrylsulfonyl)piperidin-4-yl]-3,3-dimethyl-2-oxa-7-azaspiro[4.5]decan-1-one hydrochloride

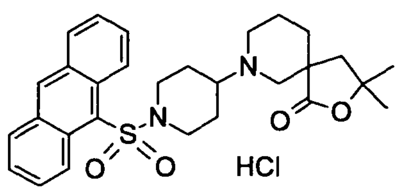

To a solution of 3,3-dimethyl-7-(piperidin-4-yl)-2-oxa-7-azaspiro[4.5]decan-1-one dihydrochloride (153 mg, 0.451 mmol) obtained in Reference Example 53 and triethylamine (0.220 mL, 1.58 mmol) in THF (2 mL) was added

anthracene-9-sulfonyl chloride (125 mg, 0.441 mmol), and the mixture was stirred at room temperature for 16 hr. The reaction mixture was diluted with ethyl acetate, washed with aqueous potassium carbonate solution and saturated brine, and dried over anhydrous sodium sulfate. The solvent was evaporated under reduced pressure, and the obtained residue was purified by basic silica gel column chromatography (developing solvent; ethyl acetate), and purified by silica gel column chromatography (developing solvent; hexane:ethyl acetate=3:1→1:1). To the obtained oil was added 4N hydrogen chloride-ethyl acetate (0.2 mL), and triturated with diisopropyl ether to give the title compound (27.7 mg, yield 11%).
EI(pos) 507 $[M+H]^+$

Example 395

7-{1-[(2-amino-5-(pyridin-2-yl)-3-thienyl)carbonyl]piperidin-4-yl}-3,3-dimethyl-2-oxa-7-azaspiro[4.5]decan-1-one

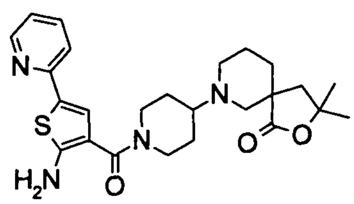

A solution of tert-butyl (3-{[4-(3,3-dimethyl-1-oxo-2-oxa-7-azaspiro[4.5]dec-7-yl)piperidin-1-yl]carbonyl}-5-(pyridin-2-yl)-2-thienyl)carbamate (737 mg, 1.30 mmol) obtained in Example 178 in trifluoroacetic acid (6 mL) was stirred at room temperature for 1 hr. The reaction mixture was concentrated, neutralized with saturated aqueous sodium hydrogencarbonate solution, and extracted twice with ethyl acetate. The organic layer was dried over anhydrous magnesium sulfate. The solvent was evaporated under reduced pressure, and triturated with diisopropyl ether to give the title compound (607 mg, quantitative).
EI(pos) 469 $[M+H]^+$

Example 396

N-(3-{[4-(3,3-dimethyl-1-oxo-2-oxa-7-azaspiro[4.5]dec-7-yl)piperidin-1-yl]carbonyl}-5-(pyridin-2-yl)-2-thienyl)urea

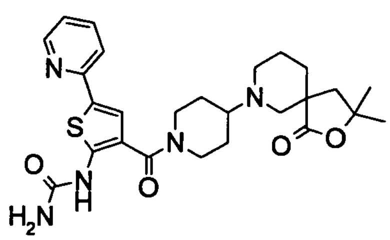

Using 7-{1-[(2-amino-5-(pyridin-2-yl)-3-thienyl)carbonyl]piperidin-4-yl}-3,3-dimethyl-2-oxa-7-azaspiro[4.5]decan-1-one (150 mg, 0.320 mmol) obtained in Example 395 and trichloroacetyl isocyanate (0.076 mL, 0.640 mmol), the title compound (124 mg, yield 76%) was obtained by an operation similar to that of Example 290 and trituration with diisopropyl ether.
EI(pos) 512 $[M+H]^+$

Example 397

N-(3-{[4-(1,3-dioxo-2,7-diazaspiro[4.5]dec-7-yl)piperidin-1-yl]carbonyl}-1-benzothien-2-yl)-N'-isopropylurea

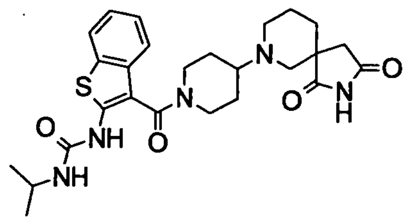

Using ethyl 3-(2-amino-2-oxoethyl)-1'-[(2-{[(isopropylamino)carbonyl]amino}-1-benzothien-3-yl)carbonyl]-1,4'-bipiperidine-3-carboxylate (157 mg, 0.282 mmol) obtained in Reference Example 199, the title compound (62 mg, yield 43%) was obtained by an operation similar to that of Example 20 and trituration with diisopropyl ether.
EI(pos) 512 $[M+H]^+$

Example 398

7-{1-[(2-bromo-1-benzothien-3-yl)carbonyl]piperidin-4-yl}-3,3-dimethyl-2-oxa-7-azaspiro[4.5]decan-1-one

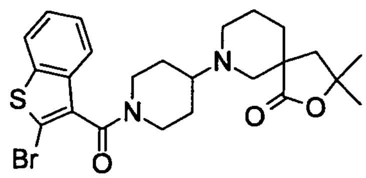

Using 2-bromo-1-benzothiophene-3-carboxylic acid (306 mg, 1.19 mmol) and 3,3-dimethyl-7-(piperidin-4-yl)-2-oxa-7-azaspiro[4.5]decan-1-one dihydrochloride (404 mg, 1.19 mmol) obtained in Reference Example 53, the title compound (413 mg, yield 69%) was obtained as an oil by an operation similar to that of Example 31.
EI(pos) 505 $[M+H]^+$

Example 399

3,3-dimethyl-7-{1-[(2-(pyridin-3-yl)-1-benzothien-3-yl)carbonyl]piperidin-4-yl}-2-oxa-7-azaspiro[4.5]decan-1-one

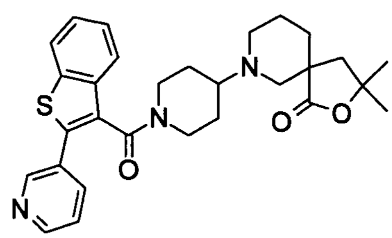

Using 7-{1-[(2-bromo-1-benzothien-3-yl)carbonyl]piperidin-4-yl}-3,3-dimethyl-2-oxa-7-azaspiro[4.5]decan-1-one (100 mg, 0.198 mmol) obtained in Example 398 and 3-pyridineboronic acid (48.6 mg, 0.396 mmol), the title compound (65.7 mg, yield 66%) was obtained by an operation similar to that of Reference Example 56 and trituration with diisopropyl ether.
EI(pos) 504 $[M+H]^+$

Example 400

Optically active forms (two kinds) of N-ethyl-N'-(3-{[4-(2-ethyl-1-oxo-2,7-diazaspiro[4.5]dec-7-yl)piperidin-1-yl]carbonyl}-1-benzothien-2-yl)urea

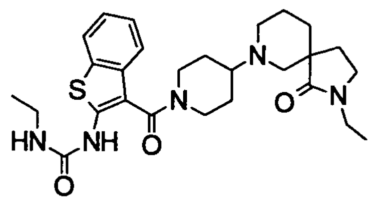

N-ethyl-N'-(3-{[4-(2-ethyl-1-oxo-2,7-diazaspiro[4.5]dec-7-yl)piperidin-1-yl]carbonyl}-1-benzothien-2-yl)urea (racemate, 380 mg) obtained in Example 321 was separated by high performance liquid chromatography (column; CHIRALPAK AD (50 mmID × 500 mmL), temperature; 30°C, mobile phase; hexane:ethanol=1:1→1:4, flow rate; 60 mL/min, detection wavelength; 220 nm) to give the two title compounds [retention time, short (139 mg) and retention time, long (137 mg)].

Example 401

Optically active forms (two kinds) of 1-ethyl-3-(3-{[4-(3-ethyl-2-methyl-4-oxo-1,3,7-triazaspiro[4.5]dec-1-en-7-yl)piperidin-1-yl]carbonyl}-1-benzothien-2-yl)urea

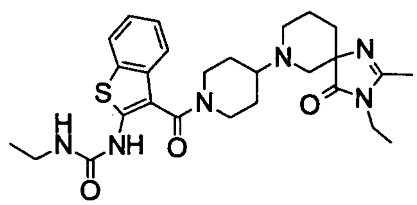

1-Ethyl-3-(3-{[4-(3-ethyl-2-methyl-4-oxo-1,3,7-triazaspiro[4.5]dec-1-en-7-yl)piperidin-1-yl]carbonyl}-1-benzothien-2-yl)urea (racemate, 125 mg) obtained in Example 233 was separated by high performance liquid chromatography (column; CHIRALCEL OD (50 mmID × 500 mmL), temperature; 30°C, mobile phase; hexane:ethanol=9:1, flow rate; 60 mL/min, detection wavelength; 220 nm) to give the two title compounds [retention time, short (45 mg) and retention time, long (50 mg)].

Example 402

N-(3-{[4-(1,3-dioxo-2,7-diazaspiro[4.5]dec-7-yl)piperidin-1-yl]carbonyl}-1-benzothien-2-yl)-N'-propylurea

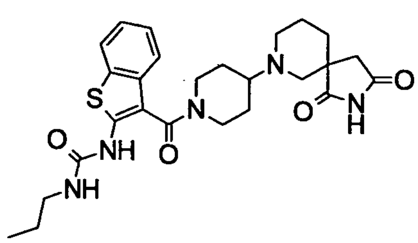

Using ethyl 3-(2-amino-2-oxoethyl)-1'-[(2-{[(propylamino)carbonyl]amino}-1-benzothien-3-yl)carbonyl]-1,4'-bipiperidine-3-carboxylate (350 mg, 0.628 mmol) obtained in Reference Example 207, the title compound (95 mg, yield 30%) was obtained by an operation similar to that of Example 20 and trituration with diisopropyl ether.
EI (pos) 512 [M+H] $^+$

Example 403

7-{1-[(2-amino-7-oxo-4,5,6,7-tetrahydro-1-benzothien-3-yl)carbonyl]piperidin-4-yl}-2-isopropyl-2,7-diazaspiro[4.5]decane-1,3-dione

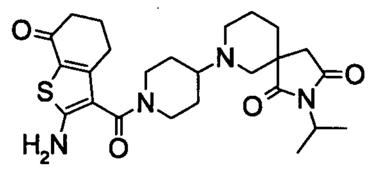

Using 2-isopropyl-7-(piperidin-4-yl)-2,7-diazaspiro[4.5]decane-1,3-dione dihydrochloride (585 mg, 1.60 mmol) obtained in Reference Example 187 and 2-amino-7-oxo-4,5,6,7-tetrahydro-1-benzothiophene-3-carboxylic acid (337 mg, 1.60 mmol) obtained in Reference Example 133, the title compound (538 mg, yield 69%) was obtained as an oil by an operation similar to that of Example 31.
EI(pos) 487 [M+H]$^+$

Example 404

N-isopropyl-N'-(3-{[4-(2-isopropyl-1,3-dioxo-2,7-diazaspiro[4.5]dec-7-yl)piperidin-1-yl]carbonyl}-7-oxo-4,5,6,7-tetrahydro-1-benzothien-2-yl)urea

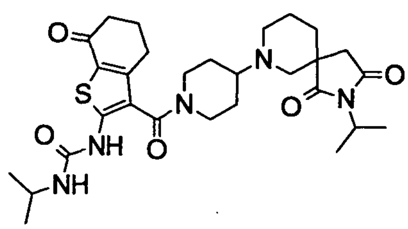

Using 7-{1-[(2-amino-7-oxo-4,5,6,7-tetrahydro-1-benzothien-3-yl)carbonyl]piperidin-4-yl}-2-isopropyl-2,7-diazaspiro[4.5]decane-1,3-dione (150 mg, 0.308 mmol) obtained in Example 403 and isopropyl isocyanate (0.091 mL, 0.925 mmol), the title compound (137 mg, yield 78%) was obtained by an operation similar to that of Example 166 and trituration with diisopropyl ether.
EI(pos) 572 [M+H]$^+$

Example 405

7-{1-[(5-acetyl-2-amino-4-methyl-3-thienyl)carbonyl]piperidin-4-yl}-2-isopropyl-2,7-diazaspiro[4.5]decane-1,3-dione

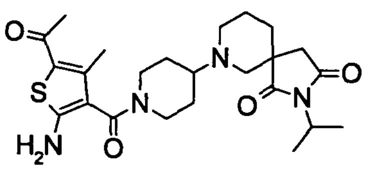

Using 2-isopropyl-7-(piperidin-4-yl)-2,7-diazaspiro[4.5]decane-1,3-dione dihydrochloride (364 mg, 0.994 mmol) obtained in Reference Example 187 and 5-acetyl-2-amino-4-methylthiophene-3-carboxylic acid (198 mg, 0.994 mmol), the title compound (348 mg, yield 74%) was obtained as an oil by an operation similar to that of Example 31.
EI(pos) 475 $[M+H]^+$

Example 406

N-(5-acetyl-3-{[4-(2-isopropyl-1,3-dioxo-2,7-diazaspiro[4.5]dec-7-yl)piperidin-1-yl]carbonyl}-4-methyl-2-thienyl)-N'-ethylurea

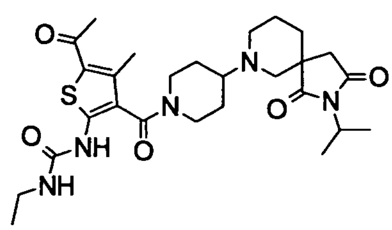

Using 7-{1-[(5-acetyl-2-amino-4-methyl-3-thienyl)carbonyl]piperidin-4-yl}-2-isopropyl-2,7-diazaspiro[4.5]decane-1,3-dione (132 mg, 0.278 mmol) obtained in Example 405 and ethyl isocyanate (0.066 mL, 0.834 mmol), the title compound (95 mg, yield 62%) was obtained by an operation similar to that of Example 166 and trituration with diisopropyl ether.
EI(pos) 546 $[M+H]^+$

Example 407

7-{1-[(2-amino-1-benzothien-3-yl)carbonyl]piperidin-4-yl}-3-(3-methoxypropyl)-2-methyl-1,3,7-triazaspiro[4.5]dec-1-en-4-one

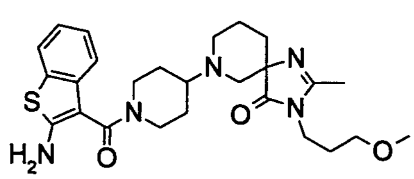

Using tert-butyl 4-[3-(3-methoxypropyl)-2-methyl-4-oxo-1,3,7-triazaspiro[4.5]dec-1-en-7-yl]piperidine-1-carboxylate (1.03 g, 2.44 mmol) obtained in Reference Example 208, the title compound (0.47 g, yield 39%) was obtained as an oil by successively performing operations similar to those of Reference Example 53 and Example 31.
EI(pos) 498 $[M+H]^+$

Example 408

N-ethyl-N'-[3-({4-[3-(3-methoxypropyl)-2-methyl-4-oxo-1,3,7-triazaspiro[4.5]dec-1-en-7-yl]piperidin-1-yl}carbonyl)-1-benzothien-2-yl]urea

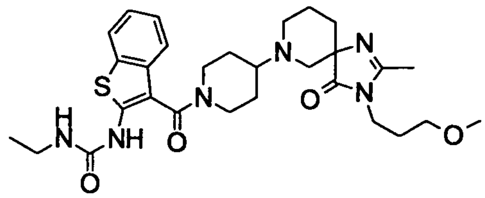

Using 7-{1-[(2-amino-1-benzothien-3-yl)carbonyl]piperidin-4-yl}-3-(3-methoxypropyl)-2-methyl-1,3,7-triazaspiro[4.5]dec-1-en-4-one (0.47 g, 0.945 mmol) obtained in Example 407, the title compound (326 mg, yield 60%) was obtained as an oil by an operation similar to that of Example 166.
EI(pos) 569 $[M+H]^+$

Example 409

N-isopropyl-N'-(3-{[4-(1-oxo-2,7-diazaspiro[4.5]dec-7-yl)piperidin-1-yl]carbonyl}-1-benzothien-2-yl)urea

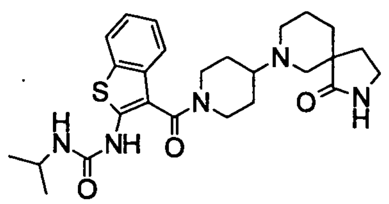

A solution of tert-butyl (3-{[4-(1-oxo-2,7-diazaspiro[4.5]dec-7-yl)piperidin-1-yl]carbonyl}-1-benzothien-2-yl)carbamate (423 mg, 0.825 mmol) obtained in Example 219 in trifluoroacetic acid (4 mL) was stirred at room temperature for 1 hr. The reaction mixture was neutralized with aqueous potassium carbonate solution, extracted twice with ethyl acetate, and the organic layer was dried over anhydrous sodium sulfate. The solvent was evaporated under reduced pressure to give 7-{1-[(2-amino-1-benzothien-3-yl)carbonyl]piperidin-4-yl}-2,7-diazaspiro[4.5]decan-1-one (0.34 g, 0.825 mmol) as a crude product. Using the crude product (0.34 g, 0.825 mmol) and isopropyl isocyanate (0.16 mL, 1.65 mmol), the title compound (251 mg, yield 61%) was obtained as an oil by an operation similar to that of Example 166.
EI(pos) 498 $[M+H]^+$

Example 410

7-{1-[(2-amino-1-benzothien-3-yl)carbonyl]piperidin-4-yl}-3-methyl-1-oxa-3,7-diazaspiro[4.5]decane-2,4-dione

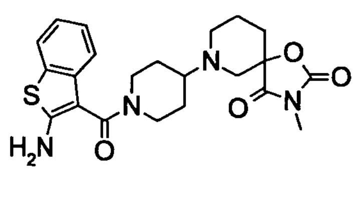

Using 3-methyl-7-(piperidin-4-yl)-1-oxa-3,7-diazaspiro[4.5]decane-2,4-dione dihydrochloride (0.72 g, 2.12 mmol) obtained in Reference Example 210 and 2-amino-1-benzothiophene-3-carboxylic acid (0.41 g, 2.12 mmol) obtained in Reference Example 51, the title compound (0.79 g, yield 84%) was obtained as an oil by an operation similar to that of Example 31.
EI(pos) 443 $[M+H]^+$

Example 411

N-ethyl-N'-(3-{[4-(3-methyl-2,4-dioxo-1-oxa-3,7-diazaspiro[4.5]dec-7-yl)piperidin-1-yl]carbonyl}-1-benzothien-2-yl)urea

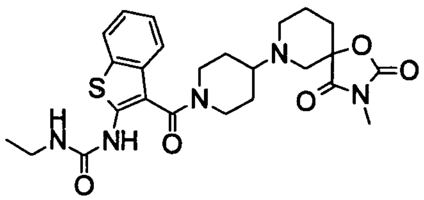

Using 7-{1-[(2-amino-1-benzothien-3-yl)carbonyl]piperidin-4-yl}-3-methyl-1-oxa-3,7-diazaspiro[4.5]decane-2,4-dione (395 mg, 0.893 mmol) obtained in Example 410, the title compound (322 mg, yield 70%) was obtained as an oil by an operation similar to that of Example 166.
EI(pos) 514 $[M+H]^+$

Example 412

N-isopropyl-N'-(3-{[4-(3-methyl-2,4-dioxo-1-oxa-3,7-diazaspiro[4.5]dec-7-yl)piperidin-1-yl]carbonyl}-1-benzothien-2-yl) urea

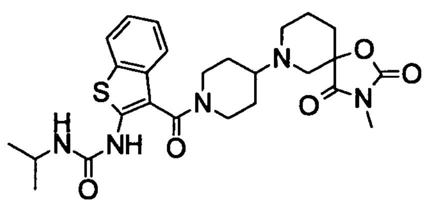

Using 7-{1-[(2-amino-1-benzothien-3-yl)carbonyl]piperidin-4-yl}-3-methyl-1-oxa-3,7-diazaspiro[4.5]decane-2,4-dione (395 mg, 0.893 mmol) obtained in Example 410 and isopropyl isocyanate (0.15 mL, 1.79 mmol), the title compound (274 mg, yield 58%) was obtained as an oil by an operation similar to that of Example 166.
EI(pos) 528 $[M+H]^+$

Example 413

N-(3-{[4-(3,3-dimethyl-1-oxo-2-oxa-7-azaspiro[4.5]dec-7-yl)piperidin-1-yl]carbonyl}-1-benzothien-2-yl)-N'-(2-isopropoxyethyl)urea

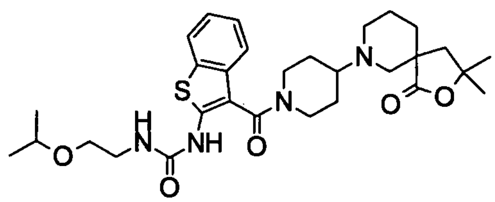

To a solution of 7-{1-[(2-amino-1-benzothien-3-yl)carbonyl]piperidin-4-yl}-3,3-dimethyl-2-oxa-7-azaspiro[4.5]decan-1-one (0.54 g, 1.23 mmol) obtained in Example 31 and triethylamine (0.26 mL, 1.84 mmol) in tetrahydrofuran (10 mL) was added 4-nitrophenyl chloroformate (0.37 g, 1.84 mmol) at room temperature, and the mixture was stirred for 30 min. 2-Isopropoxyethaneamine (0.31 mL, 2.46 mmol) was added, and the mixture was stirred at room temperature for 15 min. A saturated aqueous sodium hydrogencarbonate solution was added to the reaction mixture, and the mixture was extracted with ethyl acetate. The organic layer was washed with saturated brine, and dried over anhydrous magnesium sulfate. The solvent was evaporated under reduced pressure, and the obtained residue was purified by basic silica gel column chromatography (developing solvent; hexane:ethyl acetate=2:3→1:3) to give the title compound (144 mg, yield 20%).
EI(pos) 571 $[M+H]^+$

Example 414

7-{1-[(2-amino-1-benzothien-3-yl)carbonyl]piperidin-4-yl}-3-ethyl-1-methyl-1,3,7-triazaspiro[4.5]decane-2,4-dione

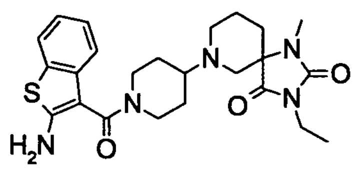

Using 3-ethyl-1-methyl-7-(piperidin-4-yl)-1,3,7-triazaspiro[4.5]decane-2,4-dione dihydrochloride (0.50 g, 1.37 mmol) obtained in Reference Example 212 and 2-amino-1-benzothiophene-3-carboxylic acid (264 mg, 1.37 mmol) obtained in Reference Example 51, the title compound (0.57 g, yield 88%) was obtained as an oil by an operation similar to that of Example 31.
EI(pos) 470 $[M+H]^+$

Example 415

N-ethyl-N'-(3-{[4-(3-ethyl-1-methyl-2,4-dioxo-1,3,7-triazaspiro[4.5]dec-7-yl)piperidin-1-yl]carbonyl}-1-benzothien-2-yl)urea

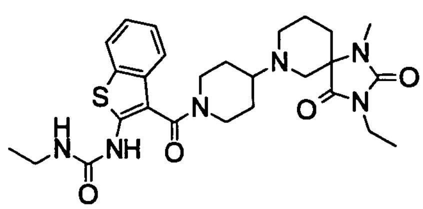

Using 7-{1-[(2-amino-1-benzothien-3-yl)carbonyl]piperidin-4-yl}-3-ethyl-1-methyl-1,3,7-triazaspiro[4.5]decane-2,4-dione (0.57 g, 1.21 mmol) obtained in Example 414, the title compound (461 mg, yield 70%) was obtained as

an oil by an operation similar to that of Example 166.
EI(pos) 541 [M+H]$^+$

Example 416

N-(3-{[4-(3-ethyl-2-methyl-4-oxo-1,3,7-triazaspiro[4.5]dec-1-en-7-yl)piperidin-1-yl]carbonyl}-1-benzothien-2-yl)-N'-methylurea

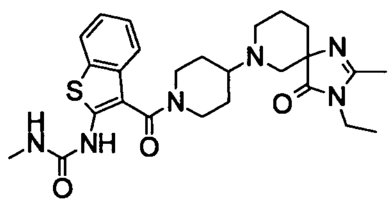

A solution of tert-butyl (3-{[4-(3-ethyl-2-methyl-4-oxo-1,3,7-triazaspiro[4.5]dec-1-en-7-yl)piperidin-1-yl]carbonyl}-1-benzothien-2-yl)carbamate (0.87 g, 1.57 mmol) obtained in Example 232 in trifluoroacetic acid (10 mL) was stirred at room temperature for 30 min. The reaction mixture was neutralized with saturated aqueous sodium hydrogencarbonate solution, and extracted twice with ethyl acetate. The organic layer was dried over anhydrous magnesium sulfate, and the solvent was evaporated under reduced pressure to give 7-{1-[(2-amino-1-benzothien-3-yl)carbonyl]piperidin-4-yl}-3-ethyl-2-methyl-1,3;7-triazaspiro[4.5]dec-1-en-4-one (0.71 g, 1.57 mmol) as a crude product. (EI(pos) 454 [M+H]$^+$) Using the crude product (0.71 g, 1.57 mmol) and methyl isocyanate (0.18 mL, 3.14 mmol), the title compound (426 mg, yield 53%) was obtained as an oil by an operation similar to that of Example 166.
EI(pos) 511 [M+H] $^+$

Example 417

7-{1-[(2-amino-7-oxo-4,5,6,7-tetrahydro-1-benzothien-3-yl)carbonyl]piperidin-4-yl}-3-ethyl-1-thia-3,7-diazaspiro[4.5]decane-2,4-dione

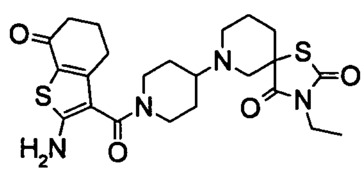

Using 2-amino-7-oxo-4,5,6,7-tetrahydro-1-benzothiophene-3-carboxylic acid (143 mg, 0.675 mmol) obtained in Reference Example 133 and 3-ethyl-7-(piperidin-4-yl)-1-thia-3,7-diazaspiro[4.5]decane-2,4-dione dihydrochloride (250 mg, 0.675 mmol) obtained in Reference Example 205, the title compound (265 mg, yield 80%) was obtained by an operation similar to that of Example 31 and trituration with diisopropyl ether and hexane.
EI(pos) 491 [M+H]$^+$

Example 418

N-ethyl-N'-(3-{[4-(3-ethyl-2,4-dioxo-1-thia-3,7-diazaspiro[4.5]dec-7-yl)piperidin-1-yl]carbonyl}-7-oxo-4,5,6,7-tetrahydro-1-benzothien-2-yl)urea

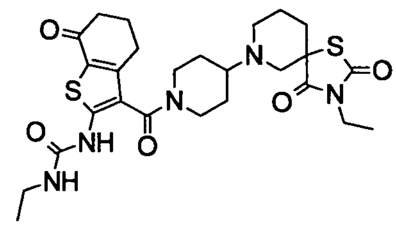

Using 7-{1-[(2-amino-7-oxo-4,5,6,7-tetrahydro-1-benzothien-3-yl)carbonyl]piperidin-4-yl}-3-ethyl-1-thia-3,7-diazaspiro[4.5]decane-2,4-dione (150 mg, 0.306 mmol) obtained in Example 417 and ethyl isocyanate (0.121 mL, 1.53 mmol), the title compound (90 mg, yield 53%) was obtained by an operation similar to that of Example 166 and trituration with diisopropyl ether.
EI(pos) 562 $[M+H]^+$

Example 419

7-{1-[(2-amino-7-oxo-4,5,6,7-tetrahydro-1-benzothien-3-yl)carbonyl]piperidin-4-yl}-3-tert-butyl-1-oxa-3,7-diazaspiro [4.5]decane-2,4-dione

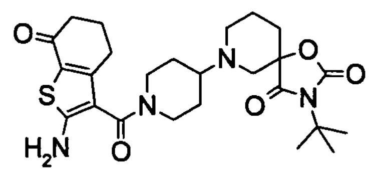

Using 2-amino-7-oxo-4,5,6,7-tetrahydro-1-benzothiophene-3-carboxylic acid (212 mg, 1.00 mmol) obtained in Reference Example 133 and 3-tert-butyl-7-(piperidin-4-yl)-1-oxa-3,7-diazaspiro[4.5]decane-2,4-dione dihydrochloride (383 mg, 1.00 mmol) obtained in Reference Example 213, the title compound (373 mg, yield 74%) was obtained by an operation similar to that of Example 31 and trituration with diisopropyl ether and hexane.
EI(pos) 503 $[M+H]^+$

Example 420

N-(3-{[4-(3-tert-butyl-2,4-dioxo-1-oxa-3,7-diazaspiro[4.5]dec-7-yl)piperidin-1-yl]carbonyl}-7-oxo-4,5,6,7-tetrahydro-1-benzothien-2-yl)-N'-ethylurea

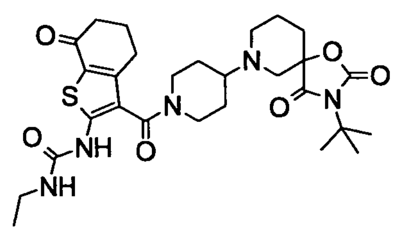

Using 7-{1-[(2-amino-7-oxo-4,5,6,7-tetrahydro-1-benzothien-3-yl)carbonyl]piperidin-4-yl}-3-tert-butyl-1-oxa-3,7-diazaspiro[4.5]decane-2,4-dione (200 mg, 0.398 mmol) obtained in Example 419 and ethyl isocyanate (0.157 mL, 1.99 mmol), the title compound (124 mg, yield 54%) was obtained by an operation similar to that of Example 166 and trituration with diisopropyl ether.
EI(pos) 574 $[M+H]^+$

Example 421

7-{1-[(2-amino-1-benzothien-3-yl)carbonyl]piperidin-4-yl}-2,3-dimethyl-1,3,7-triazaspiro[4.5]dec-1-en-4-one

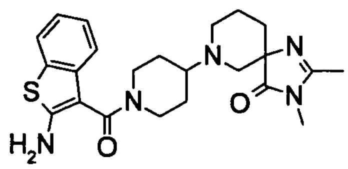

Using tert-butyl 4-(2,3-dimethyl-4-oxo-1,3,7-triazaspiro[4.5]dec-1-en-7-yl)piperidine-1-carboxylate (0.49 g, 1.35 mmol) obtained in Reference Example 214, the title compound (0.25 g, yield 42%) was obtained as an oil by successively performing operations similar to those of Reference Example 53 and Example 31.
EI(pos) 440 $[M+H]^+$

Example 422

N-(3-{[4-(2,3-dimethyl-4-oxo-1,3,7-triazaspiro[4.5]dec-1-en-7-yl)piperidin-1-yl]carbonyl}-1-benzothien-2-yl)-N'-ethylurea

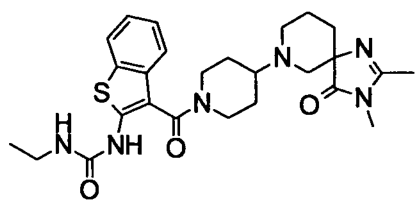

Using 7-{1-[(2-amino-1-benzothien-3-yl)carbonyl]piperidin-4-yl}-2,3-dimethyl-1,3,7-triazaspiro[4.5]dec-1-en-4-one (0.25 g, 0.569 mmol) obtained in Example 421, the title compound (0.23 g, yield 80%) was obtained as an oil by an operation similar to that of Example 166.
EI(pos) 511 $[M+H]^+$

Experimental Example 1

The ACC1 inhibitory activity of the compound of the present invention was evaluated by the following method.

(1) Cloning of human ACC1 gene and preparation of recombinant Baculovirus

Human ACC1 gene was cloned by PCR using human liver cDNA library (Clontech) as a template, and Primer 1 and Primer 2 shown below. Primer 1 and Primer 2 were prepared by adding recognition sequence of restriction enzymes SalI and NotI based on the information of the base sequence (Genbank Accession U19822) of human ACC1 gene.

Primer 1 5'AAAAGTCGACCCACCATGGATGAACCTTCTCCCTTGGCCC
Primer 2 5'AAAAGCGGCCGCCTACGTAGAAGGGGAGTCCATAGTG

PCR was performed using Pyrobest DNA polymerase (TAKARA BIO INC.). The obtained PCR product was cloned to pT7 Blue vector (Novagen) which was, after confirmation of the base sequence, digested with restriction enzymes SalI and NotI. The obtained DNA fragment was inserted into pFAST-BacHTc (Invitrogen) digested with restriction enzymes SalI and NotI to give expression plasmid ACCl/pFAST-BacHTc.

Using the expression plasmid and BAC-TO-BAC Baculovirus Expression System (Invitrogen), virus stock BAC-ACC1 of recombinant Baculovirus was prepared.

(2) Preparation of ACC1 protein

SF-9 cells (Invitrogen) were sown at $1\times10^6$ cells/mL to insect cell culture medium (1 L, Sf-900II SFM medium (Invitrogen) containing 10% fetal bovine serum (Trace), 50 mg/L gentamicin (Invitrogen) and 0.1% Pluronic F-68 (Invitrogen)), and shaking culture was performed using a 2 L volume Erlenmeyer flask at 27˚C, 100 rpm.

After culturing for 24 hr, the recombinant Baculovirus BAC-ACC1 (10 mL) was added, and the mixture was further cultured for 3 days. The culture medium was centrifuged at 1000xg for 5 min to give virus-infected cells. The infected cells were washed with phosphate buffered saline (Invitrogen) and centrifuged under the same conditions, and the obtained cells were cryopreserved at -80˚C.

The cryopreserved cells were thawed in ice, suspended in 25 mM HEPES buffer (100 mL, pH 7.5) containing 10% Glycerol, 0.13 M NaCl, 1 mM EDTA, 25 mM sodium β-glycerophosphate, 1 mM sodium orthovanadate, added with Complete Protease Inhibitor (Boehringer). The obtained suspension was homogenized 3 times with a Polytron homogenizer (Kinematica) at 20,000 rpm for 30 seconds. The obtained cell disrupt solution was clarified by centrifugation at 185700xg for 50 min and filtrated using a 0.45 μm filter. The filtrate was passed through a column packed with Ni-NTA Super Flow Gel (QIAGEN, 12 mL) at a flow rate of about 5 mL/min. The column was washed with buffer A (50 mM HEPES containing 0.3M NaCl, pH 7.5), further washed with buffer A containing 20 mM Imidazole, and eluted with buffer A containing 100 mM Imidazole. The eluate was concentrated with Vivaspin 20 (Vivascience) having a molecular weight cut off of 30 K. The obtained concentrate was dialyzed using Sephadex G-25 (Amersham Bioscience K.K., 358 mL) equilibrated with 50 mM HEPES (pH7.5) containing 10 mM $MgCl_2$, 2 mM dithiothreitol, 10 mM tripotassium citrate and 0.3M NaCl. The dialysate was concentrated with Vivaspin 20 (Vivascience) having a molecular weight cut off of 30 K and the concentrate was filtered through a 0.22 μm filter to give ACC1. The obtained ACC1 was cryopreserved at -80˚C.

(3) Measurement of ACC1 inhibitory activity

ACC1 (0.93 mg/ml) obtained in (2) above was diluted to a concentration of 8 μg/ml with an enzyme reaction buffer (50 mM HEPES (pH7.5), 10 mM $MgCl_2$, 10 mM tripotassium citrate, 2 mM dithiothreitol, 0.75 mg/ml fatty acid free BSA), and added to each well of a 384 well assay plate (Nunc 265196) by 10 μl.

Hereafter, the ACC1 inhibitory rate (%) was determined in the same manner as in Experimental Example 2-(3) to be mentioned below, and $IC_{50}$ value was calculated.

The results are shown in Table 8.

Table 8

| test compound | |
|---|---|
| Example Nos. | $IC_{50}$ (μM) |
| 1 | 1.9 |
| 36 | 0.079 |
| 92 | 4.8 |
| 133 | 3.3 |
| 153 | 1.6 |
| 179 | 0.14 |
| 190 | 0.08 |
| 228 | 0.19 |
| 229 | 0.29 |
| 233 | 0.11 |
| 240 | 0.35 |
| 245 | 0.98 |
| 301 | 1.4 |
| 311 | 0.40 |

(continued)

| test compound | |
|---|---|
| Example Nos. | $IC_{50}$ (μM) |
| 319 | 0.03 |
| 321 | 0.36 |
| 327 | 0.20 |
| 335 | 0.26 |
| 339 | 0.30 |
| 340 | 0.13 |
| 343 | 0.34 |
| 347 | 0.31 |
| 352 | 0.29 |
| 360 | 0.11 |
| 363 | 0.34 |
| 366 | 0.18 |
| 380 | 0.21 |
| 397 | 0.22 |
| 400 | 0.097 |
| 401 | 0.073 |

As shown in Table 8, the compound of the present invention has a superior ACC1 inhibitory activity.

Experimental Example 2

The ACC2 inhibitory activity of the compound of the present invention was evaluated by the following method.

(1) Cloning of human ACC2 gene and preparation of recombinant Baculovirus

Human ACC2 gene was cloned by PCR using human skeletal muscle cDNA library (Clontech) as a template, and Primer 1 and Primer 2 shown below. Primer 1 and Primer 2 were prepared by adding recognition sequence of restriction enzymes SalI and XbaI based on the information of the base sequence (Genbank Accession U89344) of human ACC2 gene.

Primer 1 5'AAAAGTCGACCCACCATGGTCTTGCTTCTTTGTCTATCTTG
Primer 2 5'TTTTTCTAGATCAGGTAGAGGCCGGGCTGTCCATG

PCR was performed using Pyrobest DNA polymerase (TAKARA BIO INC.). The obtained PCR product was cloned to pT7 Blue vector (Novagen) which was, after confirmation of the base sequence, digested with restriction enzymes SalI and XbaI. The obtained DNA fragment was inserted into pFAST-BacHTa (Invitrogen) digested with restriction enzymes SalI and XbaI to give expression plasmid ACC2/pFAST-BacHTa.

A plasmid for expression of ACC2 free of mitochondrial targeting sequence was prepared by PCR using the expression plasmid as a template, and Primer 3 and Primer 4 shown below.

Primer 3 5'CCAGGTCGACCCGCCAACGGGACTGGGACACAAGG
Primer 4 5'CGCACTCTCAGTTTCCCGGATTCCC

PCR was performed using Pyrobest DNA polymerase (TAKARA BIO INC.). The obtained PCR product was cloned to pT7 Blue vector (Novagen) which was, after confirmation of the base sequence, digested with restriction enzymes SalI and AflII. The obtained DNA fragment was inserted into pFAST-BacHTa (Invitrogen) digested with restriction enzymes SalI and AflII to give expression plasmid ACC2mito7/pFAST-BacHTa.

Using the expression plasmid and BAC-TO-BAC Baculovirus Expression System (Invitrogen), virus stock BAC-ACC2 of recombinant Baculovirus (N terminal deletion (hereinafter Nd)) was prepared.

(2) Preparation of ACC2(Nd) protein

SF-9 cells (Invitrogen) were sown at $0.5\times10^6$ cells/mL to insect cell culture medium (2 L, Sf-900II SFM medium (Invitrogen) containing 10% fetal bovine serum (Trace), 50 mg/L gentamicin (Invitrogen) and 0.1% Pluronic F-68 (Invitrogen)), and shaking culture was performed using a Wave Bioreactor (Wave) under the conditions of 27°C, 20 rpm, rocking angle of 6° and oxygen concentration of 30%.

On day 4 of the culture, 3 L of an insect cell culture medium was added, the rocking angle was set to 8°, and the cells were further cultured. On day 5 of the culture, the recombinant Baculovirus BAC-ACC2(Nd) (100 mL) was added, 5 L of the insect cell culture medium was further added, the rocking angle was set to 11°, and the cells were cultured for 3 days. The culture medium was centrifuged at 1000xg for 10 min to give virus-infected cells. The infected cells were washed with phosphate buffered saline (Invitrogen) and centrifuged under the same conditions, and the obtained cells were cryopreserved at -80°C.

The cryopreserved cells were thawed in ice, suspended in 25 mM HEPES buffer (900 mL, pH 7.5) containing 10% Glycerol, 0.13 M NaCl, 1 mM EDTA, 25 mM sodium β-glycerophosphate, 1 mM sodium orthovanadate, added with Complete Protease Inhibitor (Boehringer). The obtained suspension was homogenized 3 times with a Polytron homogenizer (Kinematica) at 20,000 rpm for 30 seconds. The obtained cell disrupt solution was clarified by centrifugation at 31000×g for 60 min and filtrated using a 0.45 μm filter. The filtrate was passed through a column packed with Ni-NTA Super Flow Gel (QIAGEN, 60 mL) at a flow rate of about 5 mL/min. The column was washed with buffer A (50 mM HEPES containing 0.3M NaCl, pH 7.5), further washed with buffer A containing 20 mM Imidazole, and eluted with buffer A containing 100 mM Imidazole. The eluate was concentrated with Vivaspin 20 (Vivascience) having a molecular weight cut off of 30 K. The obtained concentrate was dialyzed against 50 mM HEPES (pH7.5) containing 10 mM $MgCl_2$, 2 mM dithiothreitol, 10 mM tripotassium citrate and 0.3M NaCl. The dialysate was filtered through a 0.22 μm filter to give ACC2 (Nd). The obtained ACC2(Nd) was cryopreserved at -80°C.

(3) Measurement of ACC2 inhibitory activity

ACC2(Nd) (1.1 mg/ml) obtained in (2) above was diluted to a concentration of 6.4 μg/ml with an enzyme reaction buffer (50 mM HEPES (pH7.5), 10 mM $MgCl_2$, 10 mM tripotassium citrate, 2 mM dithiothreitol, 0.75 mg/ml fatty acid free BSA), and added to each well of a 384 well assay plate (Nunc 265196) by 10 μl. Then, a solution (5 μl) obtained by diluting a test compound dissolved in dimethyl sulfoxide (DMSO) with a buffer for enzyme reaction was added to each well, and the mixture was incubated at 30°C for 60 min. A substrate solution (5 μl each, 50 mM $KHCO_3$, 200 μM ATP and 200 μM Acetyl-CoA) was added to each well, and the mixture was reacted at 30°C for 20 min (test compound addition group).

In addition, a reaction in the same manner as above was performed except addition of a test compound (test compound non-addition group). Furthermore, a reaction in the same manner as above was performed except addition of a test compound and Acetyl-CoA (control group).

A malachite green solution (5 μl) was added to the respective reaction mixtures obtained in this manner and the mixtures were stirred to quench the reaction. The obtained reaction mixtures were left standing at room temperature for 20 min, and the absorbance (620 nm) was measured using wallac1420 (Perkin Elmer). The aforementioned malachite green solution was prepared by mixing SOLUTION A (0.12% malachite green solution, prepared with 5N $H_2SO_4$, shaded and preserved at 4°C), SOLUTION B (7.5% aqueous ammonium molybdate solution, prepared when in use) and SOLUTION C (11% aqueous Tween 20 solution, preserved at room temperature) at a ratio of SOLUTION A:SOLUTION B:SOLUTION C=100:25:2 (volume ratio).

Then, the ACC2 inhibitory rate (%) was determined by the calculation formula:

$$(1-(\text{absorbance of test compound addition group} - \text{absorbance of control group}) \div (\text{absorbance of test compound non-addition group} - \text{absorbance of control group}))\times 100$$

and $IC_{50}$ value was calculated.

The results are shown in Table 9.

Table 9

| test compound | |
|---|---|
| Example Nos. | $IC_{50}$ (μM) |
| 1 | 0.14 |
| 36 | 0.008 |
| 92 | 0.11 |
| 133 | 0.12 |
| 153 | 0.076 |
| 179 | 0.009 |
| 190 | 0.009 |
| 228 | 0.041 |
| 229 | 0.049 |
| 233 | 0.017 |
| 240 | 0.023 |
| 245 | 0.046 |
| 301 | 0.089 |
| 311 | 0.031 |
| 319 | 0.005 |
| 321 | 0.034 |
| 327 | 0.019 |
| 335 | 0.031 |
| 339 | 0.045 |
| 340 | 0.022 |
| 343 | 0.030 |
| 347 | 0.034 |
| 352 | 0.041 |
| 360 | 0.022 |
| 363 | 0.056 |
| 366 | 0.018 |
| 380 | 0.045 |
| 397 | 0.039 |
| 400 | 0.013 |
| 401 | 0.015 |

As shown in Table 9, the compound of the present invention has a superior ACC2 inhibitory activity.

Experimental Example 3

In the same manner as in Experimental Example 1, the ACC1 inhibitory activity of the compound of the present invention was evaluated. As a result, the compounds of Examples 36, 179, 184, 187, 190, 290, 291, 318, 319, 324, 350, 354, 357, 400, 401 and 420 showed $IC_{50}$ values of 100 nM or below.

Experimental Example 4

In the same manner as in Experimental Example 2, the ACC2 inhibitory activity of the compound of the present invention was evaluated. As a result, the compounds of Examples 36, 179, 190, 318, 319 and 420 showed $IC_{50}$ values of 10 nM or below.

Formulation Example 1 (Production of capsule)

| | |
|---|---|
| 1) compound of Example 1 | 30 mg |
| 2) finely divided powder cellulose | 10 mg |
| 3) lactose | 19 mg |
| 4) magnesium stearate | 1 mg |
| Total | 60 mg |

1), 2), 3) and 4) are mixed, and filled in a gelatin capsule.

Formulation Example 2 (Production of tablet)

| | |
|---|---|
| 1) compound of Example 1 | 30 g |
| 2) lactose | 50 g |
| 3) corn starch | 15 g |
| 4) calcium carboxymethyl cellulose | 44 g |
| 5) magnesium stearate | 1 g |
| 1000 tablets total | 140 g |

The entire amount of 1), 2) and 3) and 30 g of 4) are kneaded with water, dried in vacuo and granulated.

The sized powder is mixed with 14 g of 4) and 1 g of 5), and the mixture is tableted by a tabletting machine. In this way, tablets (1000 tablets) containing 30 mg of the compound of Example 1 per tablet are obtained.

**Industrial Applicability**

The compound of the present invention has an ACC (acetyl-CoA carboxylase) inhibitory activity, and is useful for the prophylaxis or treatment of obesity, diabetes, hypertension, hyperlipidemia, cardiac failure, diabetic complications, metabolic syndrome, sarcopenia and the like.

The present invention is based on patent application Nos. 2005-221959 and 2006-159117 filed in Japan, the contents of which are incorporated in full herein by this reference.

## Claims

1. A compound represented by the formula (I):

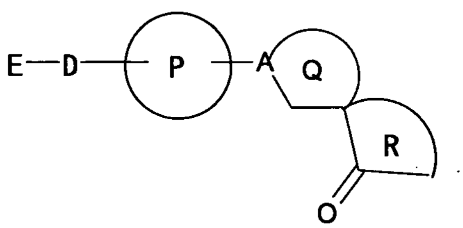

(I)

wherein

E is an optionally substituted cyclic group;
D is a carbonyl group or a sulfonyl group;
A is CH or N;
ring P is an optionally further substituted 5- to 7-membered ring;
ring Q is an optionally further substituted 5- to 7-membered nonaromatic ring; and
ring R is an optionally further substituted and optionally condensed 5- to 7-membered nonaromatic ring,

or a salt thereof.

2. The compound of claim 1, wherein ring P is a 5- to 7-membered nitrogen-containing non-aromatic heterocycle optionally further substituted.

3. The compound of claim 2, wherein the 5- to 7-membered nitrogen-containing non-aromatic heterocycle is a piperidine.

4. The compound of claim 1, wherein E is an optionally substituted aromatic hydrocarbon group.

5. The compound of claim 4, wherein the aromatic hydrocarbon group is an anthryl.

6. The compound of claim 1, wherein E is an optionally substituted aromatic heterocyclic group.

7. The compound of claim 6, wherein the aromatic heterocyclic group is a thienyl, a benzothiophenyl, a pyridyl or a quinolyl.

8. The compound of claim 1, wherein D is a carbonyl group.

9. The compound of claim 1, wherein A is N.

10. The compound of claim 1, wherein ring Q is an optionally further substituted 6-membered monocyclic non-aromatic heterocycle.

11. The compound of claim 1, wherein ring R is an optionally further substituted 5-membered monocyclic non-aromatic heterocycle.

12. The compound of claim 1, which is selected from N-(3-{[4-(3,3-dimethyl-1-oxo-2-oxa-7-azaspiro[4.5]dec-7-yl)piperidin-1-yl]carbonyl}-1-benzothien-2-yl)-N'-ethylurea, 3,3-dimethyl-7-{1-[(2-(pyridin-3-yl)quinolin-4-yl)carbonyl]piperidin-4-yl}-2-oxa-7-azaspiro[4.5]decan-1-one, 7-{1-[(3'-acetyl-5-(pyridin-3-yl)biphenyl-3-yl)carbonyl]piperidin-4-yl}-3,3-dimethyl-2-oxa-7-azaspiro[4.5]decan-1-one, 9-[1-(9-anthrylcarbonyl)piperidin-4-yl]-3,3-dimethyl-2-oxa-6,9-diazaspiro[4.5]decan-1-one, 1-(3-{[4-(3,3-dimethyl-1-oxo-2-oxa-7-azaspiro[4.5]dec-7-yl)piperidin-1-yl]carbonyl}-5-(pyridin-2-yl)-2-thienyl)-3-ethylurea, 4-(4-{[4-(3,3-dimethyl-1-oxo-2-oxa-7-azaspiro[4.5]dec-7-yl)piperidin-1-yl]carbonyl}-5-[(ethylcarbamoyl)amino]-2-thienyl)benzoic acid, N-ethyl-N'-(3-{[4-(3-ethyl-2,4-dioxo-1-oxa-3,7-diazaspiro[4.5]dec-7-yl)piperidin-1-yl]carbonyl}-1-benzothien-2-yl)urea, 1-(3-{[4-(3-ethyl-2,4-dioxo-1-oxa-3,7-diazaspiro[4.5]dec-7-yl)piperidin-1-yl]carbonyl}-1-benzothien-2-yl)urea, 1-ethyl-3-(3-{[4-(3-ethyl-2-methyl-4-oxo-1,3,7-triazaspiro[4.5]dec-1-en-7-yl)piperidin-1-yl]carbonyl}-1-benzothien-2-yl)urea, 1-(3-{[4-(2-ethyl-1,3-dioxo-2,7-diazaspiro[4.5]dec-7-yl)piperidin-1-yl]carbonyl}-1-benzothien-2-yl)urea, 1-ethyl-3-(3-{[4-(3-ethyl-2,4-dioxo-1-oxa-3,7-diazaspiro[4.5]dec-7-yl)piperidin-1-yl]carbonyl}-5-(pyridin-2-yl)-2-thienyl)urea, N-(4-{[4-(3,3-dimethyl-1-oxo-2-oxa-7-azaspiro[4.5]dec-7-yl)piperidin-1-yl]carbonyl}-6-phenylpyridin-3-yl)-N'-ethylurea, N-(3-{[4-(1,3-dioxo-2,7-diazaspiro[4.5]dec-7-yl)piperidin-1-yl]carbonyl}-1-benzothien-2-yl)-N'-ethylurea, N-ethyl-N'-(3-{[4-(2-ethyl-1-oxo-2,7-diazaspiro[4.5]dec-7-yl)piperidin-1-yl]carbonyl}-1-benzothien-2-yl)urea N-(3-{[4-(3-isopropyl-2,4-dioxo-1-oxa-3,7-diazaspiro[4.5]dec-7-yl)piperidin-1-yl]carbonyl}-1-benzothien-2-yl)urea, N-(3-{[4-(2-ethyl-1-oxo-2,7-diazaspiro[4.5]dec-7-yl)piperidin-1-yl]carbonyl}-1-benzothien-2-yl)-N'-methylurea, N-(3-{[4-(2,4-dioxo-3-propyl-1-oxa-3,7-diazaspiro[4.5]dec-7-yl)piperidin-1-yl]carbonyl}-1-benzothien-2-yl)urea, N-(3-{[4-(3-ethyl-2,4-dioxo-1-oxa-3,7-diazaspiro[4.5]dec-7-yl)piperidin-1-yl]carbonyl}-1-benzothien-2-yl)-N'-methylurea, N-(3-{[4-(1-oxo-2-propyl-2,7-diazaspiro[4.5]dec-7-yl)piperidin-1-yl]carbonyl}-1-benzothien-2-yl)urea, N-(3-{[4-(2-isopropyl-1-oxo-2,7-diazaspiro[4.5]dec-7-yl)piperidin-1-yl]carbonyl}-1-benzothien-2-yl)-N'-methylurea, N-(3-{[4-(2-ethyl-1,3-dioxo-2,7-diazaspiro[4.5]dec-7-yl)piperidin-1-yl]carbonyl}-1-benzothien-2-yl)-N'-methylurea, N-(3-{[4-(2-isopropyl-1,3-dioxo-2,7-diazaspiro[4.5]dec-7-yl)piperidin-1-yl]carbonyl}-1-benzothien-2-yl)urea, N-ethyl-N'-(3-{[4-(2-methyl-1,3-dioxo-2,7-diazaspiro[4.5]dec-7-yl)piperidin-1-yl]carbonyl}-1-benzothien-2-yl)urea, N-(3-{[4-(1,3-dioxo-2,7-diazaspiro[4.5]dec-7-yl)piperidin-1-yl]carbonyl}-1-benzothien-2-yl)-N'-isopropylurea, and an optically active form thereof and a salt thereof.

13. A prodrug of the compound of claim 1.

14. A pharmaceutical agent comprising the compound of claim 1, or a prodrug thereof.

15. An acetyl-CoA carboxylase inhibitor comprising the compound of claim 1, or a prodrug thereof.

16. A pharmaceutical agent of claim 14, which is an agent for the prophylaxis or treatment of obesity, diabetes, hypertension, hyperlipidemia, cardiac failure, diabetic complications, metabolic syndrome or sarcopenia.

17. Use of a compound represented by the formula (I):

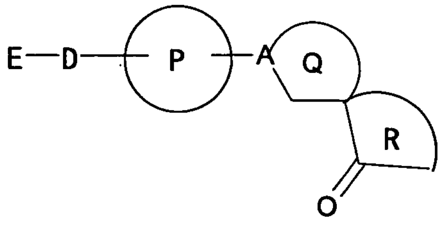

(I)

wherein

E is an optionally substituted cyclic group;
D is a carbonyl group or a sulfonyl group;
A is CH or N;
ring P is an optionally further substituted 5- to 7-membered ring;
ring Q is an optionally further substituted 5- to 7-membered nonaromatic ring; and
ring R is an optionally further substituted and optionally condensed 5- to 7-membered nonaromatic ring,
or a salt thereof, or a prodrug thereof for the production of an acetyl CoA carboxylase inhibitor.

**18.** Use of a compound represented by the formula (I):

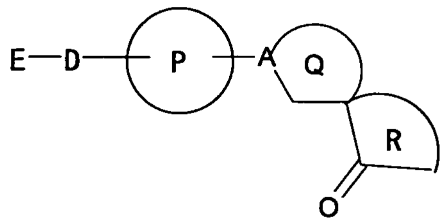

(I)

wherein

E is an optionally substituted cyclic group;
D is a carbonyl group or a sulfonyl group;
A is CH or N;
ring P is an optionally further substituted 5- to 7-membered ring;
ring Q is an optionally further substituted 5- to 7-membered nonaromatic ring; and
ring R is an optionally further substituted and optionally condensed 5- to 7-membered nonaromatic ring,
or a salt thereof, or a prodrug thereof for the production of an agent for the prophylaxis or treatment of obesity, diabetes, hypertension, hyperlipidemia, cardiac failure, diabetic complications, metabolic syndrome or sarcopenia.

**19.** A method for inhibiting acetyl CoA carboxylase in a mammal, which comprises administering a compound represented by the formula (I):

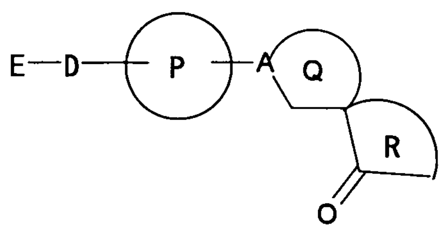

(I)

wherein

E is an optionally substituted cyclic group;
D is a carbonyl group or a sulfonyl group;
A is CH or N;
ring P is an optionally further substituted 5- to 7-membered ring;
ring Q is an optionally further substituted 5- to 7-membered nonaromatic ring; and
ring R is an optionally further substituted and optionally condensed 5- to 7-membered nonaromatic ring,
or a salt thereof or a prodrug thereof to the mammal.

**20.** A method for the prophylaxis or treatment of obesity, diabetes, hypertension, hyperlipidemia, cardiac failure, diabetic

complications, metabolic syndrome or sarcopenia in a mammal, which comprises administering a compound represented by the formula (I):

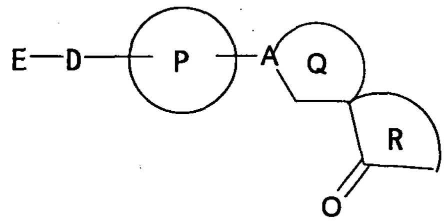

(I)

wherein

E is an optionally substituted cyclic group;
D is a carbonyl group or a sulfonyl group;
A is CH or N;
ring P is an optionally further substituted 5- to 7-membered ring;
ring Q is an optionally further substituted 5- to 7-membered nonaromatic ring; and
ring R is an optionally further substituted and optionally condensed 5- to 7-membered nonaromatic ring,
or a salt thereof, or a prodrug thereof to the mammal.

**INTERNATIONAL SEARCH REPORT**

International application No.
PCT/JP2006/315447

A. CLASSIFICATION OF SUBJECT MATTER
*C07D401/04*(2006.01)i, *A61K31/454*(2006.01)i, *A61K31/4545*(2006.01)i, *A61K31/4709*(2006.01)i, *A61K31/499*(2006.01)i, *A61K31/5386*(2006.01)i, *A61P3/00*(2006.01)i, *A61P3/04*(2006.01)i, *A61P3/06*(2006.01)i,
According to International Patent Classification (IPC) or to both national classification and IPC

B. FIELDS SEARCHED
Minimum documentation searched (classification system followed by classification symbols)
C07D401/04, A61K31/454, A61K31/4545, A61K31/4709, A61K31/499, A61K31/5386, A61P3/00, A61P3/04, A61P3/06, A61P3/10, A61P9/04, A61P9/12, A61P21/00, A61P43/00, C07D471/10, C07D487/10, C07D491/107, C07D498/10, C07D513/10,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,A | JP 2006-131559 A (Takeda Chemical Industries, Ltd.), 25 May, 2006 (25.05.06), Full text (Family: none) | 1-18 |
| A | WO 2003/072197 A1 (PFIZER PRODUCTS INC.), 04 September, 2003 (04.09.03), Full text & US 2003/187254 A1 & EP 1478437 A1 & CN 1642599 A | 1-18 |
| A | JP 2005-119987 A (Ajinomoto Co., Inc.), 12 May, 2005 (12.05.05), Full text (Family: none) | 1-18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 September, 2006 (05.09.06) | 12 September, 2006 (12.09.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/315447

C (Continuation). DOCUMENTS CONSIDERED TO BE RELEVANT

| Category* | Citation of document, with indication, where appropriate, of the relevant passages | Relevant to claim No. |
|---|---|---|
| A | JP 2003-533509 A (Merck Sharp & Dohme Ltd.), 11 November, 2003 (11.11.03), Example 127, compounds & WO 2001/087838 A1 & CA 2408849 A & EP 1286967 A1 & US 2003/236250 A1 | 1-12 |
| A | JP 2003-505388 A (Astra Zeneca AB.), 12 February, 2003 (12.02.03), Example 28, compounds & WO 2001/005790 A1 & CA 2378202 A & AU 2000063309 A & EP 1202994 A1 & EP 1426375 A2 & US 6774132 A & US 2004/087800 A1 | 1-12 |

Form PCT/ISA/210 (continuation of second sheet) (April 2005)

**INTERNATIONAL SEARCH REPORT**

International application No.
PCT/JP2006/315447

Continuation of A. CLASSIFICATION OF SUBJECT MATTER
(International Patent Classification (IPC))

*A61P3/10*(2006.01)i, *A61P9/04*(2006.01)i, *A61P9/12*(2006.01)i, *A61P21/00*(2006.01)i, *A61P43/00*(2006.01)i, *C07D471/10*(2006.01)i, *C07D487/10*(2006.01)i, *C07D491/107*(2006.01)i, *C07D498/10*(2006.01)i, *C07D513/10*(2006.01)i, *C07D519/00*(2006.01)i, *C07F7/18*(2006.01)i

(According to International Patent Classification (IPC) or to both national classification and IPC)

Continuation of B. FIELDS SEARCHED
Minimum documentation searched (International Patent Classification (IPC))

C07D519/00, C07F7/18

Minimum documentation searched (classification system followed by classification symbols)

Form PCT/ISA/210 (extra sheet) (April 2005)

**INTERNATIONAL SEARCH REPORT**

International application No.
PCT/JP2006/315447

**Box No. II Observations where certain claims were found unsearchable (Continuation of item 2 of first sheet)**

This international search report has not been established in respect of certain claims under Article 17(2)(a) for the following reasons:

1. [X] Claims Nos.: 19, 20
because they relate to subject matter not required to be searched by this Authority, namely:
Claims 19 and 20 pertain to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

Form PCT/ISA/210 (continuation of first sheet (2)) (April 2005)

**INTERNATIONAL SEARCH REPORT**

International application No.
PCT/JP2006/315447

<Subject of search>

The compound referred in claims 1-11, the prodrug referred in claim 13 and the active ingredient of pharmaceutical inventions of claims 14-18 include great many compounds having a wide variety of structures. However, those compounds which are disclosed in embodiments are limited to the compounds of the formula (I) wherein D is a carbonyl group, the ring P is a piperidine, the ring Q is a piperidine, piperadine or morpholine, and the ring R is a compound having a 5-membered ring or a 7-membered ring fused with a benzene ring.

Thus, claims 1-11 and 13-18 are not fully supported by the description (PCT Article 6) and the description is not clearly or fully disclosed with respect to claims 1-11 and 13-18 (PCT Article 5).

In addition, since it is unclear what types of compounds are included in the term "prodrug" referred in claims 13-18, claims 13-18 do not comply with the requirement of clearness under PCT Article 6, too.

Such being the case, the search was made mainly on the compounds specifically disclosed in the embodiments, i.e., compounds of the formula (I) wherein the ring Q is a piperidine, piperadine or morpholine and the ring R is a compound having a 5-membered ring or a 7-membered ring fused with a benzene ring. With respect to other alternatives, complete search was not made.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03029245 A
- WO 03072197 A
- WO 0138325 A
- WO 0125228 A
- WO 0342204 A
- WO 9844921 A
- WO 9845285 A
- WO 9922735 A
- WO 0114372 A
- WO 9710224 A
- WO 0182925 A
- WO 0187834 A
- WO 2006053024 A2
- WO 2005221959 A
- WO 2006159117 A


### Non-patent literature cited in the description


- Design of Molecules. IYAKUHIN no KAIHATSU, Development of Pharmaceuticals. HIROKAWA SHOTEN, 1990, vol. 7, 163-198
- ORGANIC FUNCTIONAL GROUP PREPARATIONS. ACADEMIC PRESS, INC, 1989
- Comprehensive Organic Transformations. VCH Publishers Inc, 1989
- *Journal of Medicinal Chemistry (J. Med. Chem.,* 1998, 2439-2441
- *Heterocycles,* 1992, 2263-2267
- Protective Groups in Organic Synthesis. John Wiley and Sons, 1980
- *Bioorganic and Medicinal Chemistry (Bioorg. Med. Chem.,* 1999, 2945-2952
- *Journal of Organic Chemistry (J. Org. Chem.,* 2004, 2441-2450
- *Tetrahedron Letters (Tetrahedron Lett.,* 2001, 8345-8349
- *Journal of Medicinal Chemistry (J. Med. Chem.,* 1998, 4118-4129
- *Journal of Organic Chemistry (J. Org. Chem.,* 1990, 820-826
- *Helvetica Chimica Acta (Hel. Chim. Acta,* 1983, 450-465
- *Tetrahedron Letters (Tetrahedron Lett.,* 1986, 3285-3288
- *Journal of Medicinal Chemistry (J. Med. Chem.,* 1988, 486-491

SEQUENCE LISTING

<110> Takeda Pharmaceutical Company Limited

<120> spiro-cyclic compound

<130> 09948

<150> JP2005-221959

<151> 2005-07-29

<150> JP2006-159117

<151> 2006-06-07

<160> 6

<170> PatentIn version 3.1

<210> 1
<211> 40
<212> DNA
<213> Artificial

<220>
<223> primer for cloning human ACC1 gene

<400> 1
aaaagtcgac ccaccatgga tgaaccttct cccttggccc 40

<210> 2
<211> 37
<212> DNA
<213> Artificial

<220>
<223> primer for cloning human ACC1 gene

<400> 2
aaaagcggcc gcctacgtag aaggggagtc catagtg 37

<210> 3
<211> 41
<212> DNA
<213> Artificial

<220>
<223> primer for cloning human ACC2 gene

<400> 3
aaaagtcgac ccaccatggt cttgcttctt tgtctatctt g 41

<210> 4
<211> 35
<212> DNA
<213> Artificial

<220>
<223> primer for cloning human ACC2 gene

<400> 4
tttttctaga tcaggtagag gccgggctgt ccatg 35

<210> 5
<211> 35
<212> DNA
<213> Artificial

<220>

<223> primer for cloning human ACC2 gene

<400> 5
ccaggtcgac ccgccaacgg gactgggaca caagg 35

<210> 6
<211> 25
<212> DNA
<213> Artificial

<220>
<223> primer for cloning human ACC2 gene

<400> 6
cgcactctca gtttcccgga ttccc 25